# EUROPEAN PATENT APPLICATION

(11) **EP 2 434 019 A1**
(43) Date of publication of application: **28.03.2012**
(21) Application number: 11160902.0
(22) Date of filing: 21.07.2004
(51) Int. Cl.: C12N 15/82, C07K 14/395, C12N 5/10, G01N 33/50, C07K 16/14, A01H 5/00, C07K 14/39

(54) **Process for the production of fine chemicals**

(30) Priority: 01.08.2003 EP 03016672; 15.04.2004 WO PCT/US2004/011887
(62) Divisional of application: 04741185.5
(71) Applicant: BASF Plant Science GmbH, 67056 Ludwigshafen (DE)
(72) Inventor: Plesch, Gunnar, 14482, Potsdam (DE); Puzio, Piotr, 9030, Mariakerke (Gent) (BE); Blau, Astrid, 14532, Stahnsdorf (DE); Looser, Ralf, 13158, Berlin (DE); Wendel, Birgit, 13437, Berlin (DE); Kamlage, Beate, 12161, Berlin (DE); Taman-Chardonnens, Agnes A., 1611, DS Bovenkarspel (NL); Shirley, Amber, Durham, NC 27703 (US); Wang, Xi-Qing, Chapel Hill, NC 27516 (US); Sarria-Millan, Rodrigo, West Lafayette, IN 47906 (US); McKersie, Bryan D, Cary, NC 27519 (US); Chen, Ruoying, Duluth, GA 30096 (US)
(74) Representative: Heistracher, Elisabeth

(57) **Abstract**

The present invention relates to a process for the production of the fine chemical in a microorgansm, a plant cell, a plant, a plant tissue or in one or more parts thereof. The invention furthermore relates to nucleic acid molecules, polypeptides, nucleic acid constructs, vectors, antisense molecules, antibodies, host cells, plant tissue, propagtion material, harvested material, plants, microorganisms as well as agricultural compositions and to their use.

SEQUENCE LISTING

<110>  BASF Plant Science GmbH

<120>  Process for the production of fine chemicals

<130>  PF55740

<140>  2004_299

<141>  2004-08-10

<160>  400

<170>  PatentIn version 3.1

<210>  1
<211>  579
<212>  DNA
<213>  Saccharomyces cerevisiae

<220>

<221>  CDS

<222>  (1)..(579)

<223>

<400>  1
atg tct gaa aag gcc gtt aga agg aaa ctt gtt att att ggt gat ggt      48
Met Ser Glu Lys Ala Val Arg Arg Lys Leu Val Ile Ile Gly Asp Gly
1               5                   10                  15

gct tgt ggc aag acc tct tta cta tat gta ttt aca tta gga aaa ttc      96
Ala Cys Gly Lys Thr Ser Leu Leu Tyr Val Phe Thr Leu Gly Lys Phe
            20                  25                  30

cct gaa caa tat cat ccg aca gtg ttc gag aat tat gtc act gat tgc      144
Pro Glu Gln Tyr His Pro Thr Val Phe Glu Asn Tyr Val Thr Asp Cys
        35                  40                  45

aga gtt gac gga ata aaa gtg tcc tta acg ctc tgg gat aca gcg gga      192
Arg Val Asp Gly Ile Lys Val Ser Leu Thr Leu Trp Asp Thr Ala Gly
    50                  55                  60

caa gag gaa tat gaa cgt tta cgt cca ttc tca tat tca aaa gca gat      240
Gln Glu Glu Tyr Glu Arg Leu Arg Pro Phe Ser Tyr Ser Lys Ala Asp
65                  70                  75                  80

ata ata tta att ggg ttt gct gta gac aat ttt gaa tca cta att aac      288
Ile Ile Leu Ile Gly Phe Ala Val Asp Asn Phe Glu Ser Leu Ile Asn
                85                  90                  95

gca agg acg aaa tgg gcg gat gag gca tta cga tat tgt cct gac gca      336

Ala Arg Thr Lys Trp Ala Asp Glu Ala Leu Arg Tyr Cys Pro Asp Ala
            100                 105                 110

cca atc gtt ctt gta ggc ttg aaa aaa gat ttg agg caa gaa gcc cat        384
Pro Ile Val Leu Val Gly Leu Lys Lys Asp Leu Arg Gln Glu Ala His
        115                 120                 125

ttt aaa gag aat gct acc gat gaa atg gtt ccc att gaa gat gca aaa        432
Phe Lys Glu Asn Ala Thr Asp Glu Met Val Pro Ile Glu Asp Ala Lys
    130                 135                 140

caa gtt gca agg gcc att ggg gcc aag aaa tac atg gaa tgt agt gca        480
Gln Val Ala Arg Ala Ile Gly Ala Lys Lys Tyr Met Glu Cys Ser Ala
145                 150                 155                 160

ctg act ggt gag ggt gtg gat gat gtc ttt gaa gta gct aca aga acc        528
Leu Thr Gly Glu Gly Val Asp Asp Val Phe Glu Val Ala Thr Arg Thr
                165                 170                 175

agt ttg ctt atg aag aag gaa cca ggg gct aac tgt tgc ata att tta        576
Ser Leu Leu Met Lys Lys Glu Pro Gly Ala Asn Cys Cys Ile Ile Leu
            180                 185                 190

taa                                                                    579

<210>  2

<211>  192

<212>  PRT

<213>  Saccharomyces cerevisiae

<400>  2

Met Ser Glu Lys Ala Val Arg Arg Lys Leu Val Ile Ile Gly Asp Gly
1               5                   10                  15

Ala Cys Gly Lys Thr Ser Leu Leu Tyr Val Phe Thr Leu Gly Lys Phe
            20                  25                  30

Pro Glu Gln Tyr His Pro Thr Val Phe Glu Asn Tyr Val Thr Asp Cys
        35                  40                  45

Arg Val Asp Gly Ile Lys Val Ser Leu Thr Leu Trp Asp Thr Ala Gly
    50                  55                  60

Gln Glu Glu Tyr Glu Arg Leu Arg Pro Phe Ser Tyr Ser Lys Ala Asp
65                  70                  75                  80

Ile Ile Leu Ile Gly Phe Ala Val Asp Asn Phe Glu Ser Leu Ile Asn
                85                  90                  95

Ala Arg Thr Lys Trp Ala Asp Glu Ala Leu Arg Tyr Cys Pro Asp Ala
            100                 105                 110

Pro Ile Val Leu Val Gly Leu Lys Lys Asp Leu Arg Gln Glu Ala His
        115                 120                 125

Phe Lys Glu Asn Ala Thr Asp Glu Met Val Pro Ile Glu Asp Ala Lys
    130                 135                 140

Gln Val Ala Arg Ala Ile Gly Ala Lys Lys Tyr Met Glu Cys Ser Ala
145                 150                 155                 160

Leu Thr Gly Glu Gly Val Asp Asp Val Phe Glu Val Ala Thr Arg Thr
                165                 170                 175

Ser Leu Leu Met Lys Lys Glu Pro Gly Ala Asn Cys Cys Ile Ile Leu
            180                 185                 190

<210>  3

<211>  600

<212>  DNA

<213>  Oryza sativa

<220>

<221>  CDS

<222>  (1)..(600)

<223>

<400>  3
atg ggt tgc tcc tcc tcc gtg cca gct cga agc act gga ggg ttg aac       48
Met Gly Cys Ser Ser Ser Val Pro Ala Arg Ser Thr Gly Gly Leu Asn
1               5                   10                  15

aat att agc aac gat aac tcc gct act gat tca aag gac ttg cgt gct       96
Asn Ile Ser Asn Asp Asn Ser Ala Thr Asp Ser Lys Asp Leu Arg Ala
            20                  25                  30

aag ttg gta ttg ctt ggt gac tct ggt gta ggg aaa agt tgc att gtt      144
Lys Leu Val Leu Leu Gly Asp Ser Gly Val Gly Lys Ser Cys Ile Val
        35                  40                  45

ctt cgc ttt gtt cgt ggt cag ttt gat ccc act tcc aag gta act gtc      192
Leu Arg Phe Val Arg Gly Gln Phe Asp Pro Thr Ser Lys Val Thr Val
    50                  55                  60

ggt gca tca ttt tta tca caa aca ttg gct ttg gag gac tca aca ata      240
Gly Ala Ser Phe Leu Ser Gln Thr Leu Ala Leu Glu Asp Ser Thr Ile
65                  70                  75                  80

gtg aaa ttt gaa ata tgg gat acc gct gga caa gag agg tat gct gcc      288
Val Lys Phe Glu Ile Trp Asp Thr Ala Gly Gln Glu Arg Tyr Ala Ala
                85                  90                  95

ttg gca cct ctt tac tac aga gga gct gct gct gca gtt gtt gtc tac      336
Leu Ala Pro Leu Tyr Tyr Arg Gly Ala Ala Ala Ala Val Val Val Tyr
            100                 105                 110

gac ata act agt cca gaa tca ttt agc aaa gca caa tac tgg gtg aag      384

Asp Ile Thr Ser Pro Glu Ser Phe Ser Lys Ala Gln Tyr Trp Val Lys
        115                 120                 125

gaa ctt caa aaa cat ggt agt cct gat att atc atg gtt ttg gtt ggt 432
Glu Leu Gln Lys His Gly Ser Pro Asp Ile Ile Met Val Leu Val Gly
    130                 135                 140

aat aaa gct gat cta cat gaa aat cga cat gta tct tct cag gaa gca 480
Asn Lys Ala Asp Leu His Glu Asn Arg His Val Ser Ser Gln Glu Ala
145                 150                 155                 160

caa gag tat gca gag aag aat aat atg gtt ttc atc gag aca tca gca 528
Gln Glu Tyr Ala Glu Lys Asn Asn Met Val Phe Ile Glu Thr Ser Ala
                165                 170                 175

aag aca gct gat aat ata aac caa gta ttt gag gaa att gcg aag agg 576
Lys Thr Ala Asp Asn Ile Asn Gln Val Phe Glu Glu Ile Ala Lys Arg
            180                 185                 190

ttg ccc agg cca acg gcg tct tga 600
Leu Pro Arg Pro Thr Ala Ser
        195

<210> 4

<211> 199

<212> PRT

<213> Oryza sativa

<400> 4

Met Gly Cys Ser Ser Ser Val Pro Ala Arg Ser Thr Gly Gly Leu Asn
1               5                   10                  15

Asn Ile Ser Asn Asp Asn Ser Ala Thr Asp Ser Lys Asp Leu Arg Ala
            20                  25                  30

Lys Leu Val Leu Leu Gly Asp Ser Gly Val Gly Lys Ser Cys Ile Val
        35                  40                  45

Leu Arg Phe Val Arg Gly Gln Phe Asp Pro Thr Ser Lys Val Thr Val
    50                  55                  60

Gly Ala Ser Phe Leu Ser Gln Thr Leu Ala Leu Glu Asp Ser Thr Ile
65                  70                  75                  80

Val Lys Phe Glu Ile Trp Asp Thr Ala Gly Gln Glu Arg Tyr Ala Ala
                85                  90                  95

Leu Ala Pro Leu Tyr Tyr Arg Gly Ala Ala Ala Ala Val Val Val Tyr
            100                 105                 110

Asp Ile Thr Ser Pro Glu Ser Phe Ser Lys Ala Gln Tyr Trp Val Lys
        115                 120                 125

Glu Leu Gln Lys His Gly Ser Pro Asp Ile Ile Met Val Leu Val Gly
    130                 135                 140

Asn Lys Ala Asp Leu His Glu Asn Arg His Val Ser Ser Gln Glu Ala
145                 150                 155                 160

Gln Glu Tyr Ala Glu Lys Asn Asn Met Val Phe Ile Glu Thr Ser Ala
                165                 170                 175

Lys Thr Ala Asp Asn Ile Asn Gln Val Phe Glu Glu Ile Ala Lys Arg
            180                 185                 190

Leu Pro Arg Pro Thr Ala Ser
        195

<210>  5

<211>  648

<212>  DNA

<213>  Oryza sativa

<220>

<221>  CDS

<222>  (1)..(648)

<223>

<400>  5
atg gcg tcc agc gcg tcg cgg ttc atc aag tgc gtc acg gtc ggg gac      48
Met Ala Ser Ser Ala Ser Arg Phe Ile Lys Cys Val Thr Val Gly Asp
1               5                   10                  15

ggc gcc gtc ggc aag acc tgc atg ctc atc tgc tac acc agc aac aag      96
Gly Ala Val Gly Lys Thr Cys Met Leu Ile Cys Tyr Thr Ser Asn Lys
            20                  25                  30

ttc ccc act gat tac gta ccc act gtt ttt gac aat ttc agt gca aac      144
Phe Pro Thr Asp Tyr Val Pro Thr Val Phe Asp Asn Phe Ser Ala Asn
        35                  40                  45

gtg gtg gtc gac ggc acc acg gtg aat ttg ggt ctc tgg gat act gca      192
Val Val Val Asp Gly Thr Thr Val Asn Leu Gly Leu Trp Asp Thr Ala
    50                  55                  60

ggg cag gaa gat tac aac aga ttg agg ccg cta agc tac cgt ggc gcc      240
Gly Gln Glu Asp Tyr Asn Arg Leu Arg Pro Leu Ser Tyr Arg Gly Ala
65                  70                  75                  80

gat gtc ttt gtg ctt gcc ttc tcc cta gtg agc cga gct agc tat gag      288

Asp Val Phe Val Leu Ala Phe Ser Leu Val Ser Arg Ala Ser Tyr Glu
                85                  90                  95

aat gtc atg aag aag tgg tta cca gag ctt cag cat tat gca cca ggg      336
Asn Val Met Lys Lys Trp Leu Pro Glu Leu Gln His Tyr Ala Pro Gly

            100                 105                 110

gtg cca att gtg ttg gtt ggg acc aaa ttg gat ctt cgt gaa gat aaa 384
Val Pro Ile Val Leu Val Gly Thr Lys Leu Asp Leu Arg Glu Asp Lys
        115                 120                 125

cac tac tta ctt gac cat cct agc ttg gtg cct gtg act aca gca cag 432
His Tyr Leu Leu Asp His Pro Ser Leu Val Pro Val Thr Thr Ala Gln
    130                 135                 140

gga gag gaa ctc cgc aag cac att ggc gca acg tgt tac atc gaa tgc 480
Gly Glu Glu Leu Arg Lys His Ile Gly Ala Thr Cys Tyr Ile Glu Cys
145                 150                 155                 160

agc tca aag aca cag cag aat gta aaa gct gtg ttt gat gct gcc atc 528
Ser Ser Lys Thr Gln Gln Asn Val Lys Ala Val Phe Asp Ala Ala Ile
                165                 170                 175

aag gta gta atc aag cct cca aca aag cag agg gac agg aag aag aag 576
Lys Val Val Ile Lys Pro Pro Thr Lys Gln Arg Asp Arg Lys Lys Lys
            180                 185                 190

aaa aca cgg cgc gga tgt tct ttc ttc tgc aag ggt gtc atg tcc aga 624
Lys Thr Arg Arg Gly Cys Ser Phe Phe Cys Lys Gly Val Met Ser Arg
        195                 200                 205

aga agg cta gta tgc ttc aag tga 648

Arg Arg Leu Val Cys Phe Lys
    210                 215

<210> 6

<211> 215

<212> PRT

<213> Oryza sativa

<400> 6

Met Ala Ser Ser Ala Ser Arg Phe Ile Lys Cys Val Thr Val Gly Asp
1               5                   10                  15

Gly Ala Val Gly Lys Thr Cys Met Leu Ile Cys Tyr Thr Ser Asn Lys
            20                  25                  30

Phe Pro Thr Asp Tyr Val Pro Thr Val Phe Asp Asn Phe Ser Ala Asn
        35                  40                  45

Val Val Val Asp Gly Thr Thr Val Asn Leu Gly Leu Trp Asp Thr Ala
    50                  55                  60

Gly Gln Glu Asp Tyr Asn Arg Leu Arg Pro Leu Ser Tyr Arg Gly Ala
65                  70                  75                  80

Asp Val Phe Val Leu Ala Phe Ser Leu Val Ser Arg Ala Ser Tyr Glu
                85                  90                  95

Asn Val Met Lys Lys Trp Leu Pro Glu Leu Gln His Tyr Ala Pro Gly
            100                 105                 110

Val Pro Ile Val Leu Val Gly Thr Lys Leu Asp Leu Arg Glu Asp Lys
        115                 120                 125

His Tyr Leu Leu Asp His Pro Ser Leu Val Pro Val Thr Thr Ala Gln
    130                 135                 140

Gly Glu Glu Leu Arg Lys His Ile Gly Ala Thr Cys Tyr Ile Glu Cys
145                 150                 155                 160

Ser Ser Lys Thr Gln Gln Asn Val Lys Ala Val Phe Asp Ala Ala Ile
                165                 170                 175

Lys Val Val Ile Lys Pro Pro Thr Lys Gln Arg Asp Arg Lys Lys Lys
            180                 185                 190

Lys Thr Arg Arg Gly Cys Ser Phe Phe Cys Lys Gly Val Met Ser Arg
        195                 200                 205

Arg Arg Leu Val Cys Phe Lys
    210                 215

<210> 7

<211> 591

<212> DNA

<213> Brassica napus

<220>

<221> CDS

<222> (1)..(591)

<223>

<400> 7
atg agc gca tcg agg ttc ata aag tgt gtt aca gtc ggc gat ggt gcc 48
Met Ser Ala Ser Arg Phe Ile Lys Cys Val Thr Val Gly Asp Gly Ala
1               5                   10                  15

gtc gga aaa acc tgt atg ctg atc tct tac acc agc aac act ttc cct 96
Val Gly Lys Thr Cys Met Leu Ile Ser Tyr Thr Ser Asn Thr Phe Pro
            20                  25                  30

acg gac tat gtt cca act gtt ttc gac aac ttc agt gct aac gtg gtt 144
Thr Asp Tyr Val Pro Thr Val Phe Asp Asn Phe Ser Ala Asn Val Val
        35                  40                  45

gtt gat ggg aac act gtg aat ctt gga ttg tgg gat aca gct ggt caa 192
Val Asp Gly Asn Thr Val Asn Leu Gly Leu Trp Asp Thr Ala Gly Gln

50                  55                  60

gaa gac tat aac agg tta aga cca ttg agt tac cgt ggt gca gat gtc        240
Glu Asp Tyr Asn Arg Leu Arg Pro Leu Ser Tyr Arg Gly Ala Asp Val
65                  70                  75                  80

ttc att ctt gct ttc tct ctt att agc aaa gct agc tac gag aac ata        288
Phe Ile Leu Ala Phe Ser Leu Ile Ser Lys Ala Ser Tyr Glu Asn Ile
                85                  90                  95

gcc aag aag tgg att cct gag ctc agg cat tat gcc cct gga gtt cct        336
Ala Lys Lys Trp Ile Pro Glu Leu Arg His Tyr Ala Pro Gly Val Pro
            100                 105                 110

atc att ctc gtg ggg aca aaa ctc gat ctt cga gat gac aag cag ttc        384
Ile Ile Leu Val Gly Thr Lys Leu Asp Leu Arg Asp Asp Lys Gln Phe
        115                 120                 125

ttc ata gac cat ccc ggt gca gtg cca atc act aca aac cag gga gag        432
Phe Ile Asp His Pro Gly Ala Val Pro Ile Thr Thr Asn Gln Gly Glu
    130                 135                 140

gaa cta aag aaa ctc ata gga tct cca gtt tac att gaa tgt agt tca        480
Glu Leu Lys Lys Leu Ile Gly Ser Pro Val Tyr Ile Glu Cys Ser Ser
145                 150                 155                 160

aag acg cag cag aat gtc aaa gca gtc ttt gac gca gct att aaa gtg        528
Lys Thr Gln Gln Asn Val Lys Ala Val Phe Asp Ala Ala Ile Lys Val
                165                 170                 175

gtg ctt cag cca cca aaa tca aag aag aag aaa aag aac aag aat cgt        576
Val Leu Gln Pro Pro Lys Ser Lys Lys Lys Lys Lys Asn Lys Asn Arg
            180                 185                 190

tgc gtt ttc ttg tga                                                    591
Cys Val Phe Leu
        195

<210>  8

<211>  196

<212>  PRT

<213>  Brassica napus

<400>  8

Met Ser Ala Ser Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Asn Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val

65                  70                  75                  80

Phe Ile Leu Ala Phe Ser Leu Ile Ser Lys Ala Ser Tyr Glu Asn Ile
                85                  90                  95

Ala Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Ile Asp His Pro Gly Ala Val Pro Ile Thr Thr Asn Gln Gly Glu
    130                 135                 140

Glu Leu Lys Lys Leu Ile Gly Ser Pro Val Tyr Ile Glu Cys Ser Ser
145                 150                 155                 160

Lys Thr Gln Gln Asn Val Lys Ala Val Phe Asp Ala Ala Ile Lys Val
                165                 170                 175

Val Leu Gln Pro Pro Lys Ser Lys Lys Lys Lys Lys Asn Lys Asn Arg
            180                 185                 190

Cys Val Phe Leu
        195

<210>  9

<211>  597

<212>  DNA

<213>  Brassica napus

<220>

<221>  CDS

<222>  (1)..(597)

<223>

<400>  9
atg agt gct tcg agg ttt atc aag tgt gtc acc gtc ggc gac ggc gct       48
Met Ser Ala Ser Arg Phe Ile Lys Cys Val Thr Val Gly Asp Gly Ala
1               5                   10                  15

gtc gga aag act tgt ctg ctc atc tcc tac act agc aac act ttc ccc       96
Val Gly Lys Thr Cys Leu Leu Ile Ser Tyr Thr Ser Asn Thr Phe Pro
            20                  25                  30

acg gac tat gtg cca act gtg ttt gat aat ttc agc gcg aat gtg att      144
Thr Asp Tyr Val Pro Thr Val Phe Asp Asn Phe Ser Ala Asn Val Ile
        35                  40                  45

gtg gat ggg aac act atc aac ttg gga ttg tgg gat act gca ggg caa 192
Val Asp Gly Asn Thr Ile Asn Leu Gly Leu Trp Asp Thr Ala Gly Gln
    50                  55                  60

gag gac tac aat aga cta aga cca ttg agc tat cgc ggc gca gat gtc 240
Glu Asp Tyr Asn Arg Leu Arg Pro Leu Ser Tyr Arg Gly Ala Asp Val
65                  70                  75                  80

ttc tta ctc gct ttc tcc ctt gtc agc aaa gct agc tat gaa aat gtt 288
Phe Leu Leu Ala Phe Ser Leu Val Ser Lys Ala Ser Tyr Glu Asn Val
                85                  90                  95

tct aaa aag tgg gta cct gaa ctg aga cat tat gct cct ggt gtt cca 336
Ser Lys Lys Trp Val Pro Glu Leu Arg His Tyr Ala Pro Gly Val Pro
            100                 105                 110

atc atc ctc gtc gga acc aag ctt gat ctt cga gat gac aag caa ttc 384
Ile Ile Leu Val Gly Thr Lys Leu Asp Leu Arg Asp Asp Lys Gln Phe
        115                 120                 125

ttt gtt gag cac cct ggt gct gtg cct atc tct act gct cag ggt gaa 432
Phe Val Glu His Pro Gly Ala Val Pro Ile Ser Thr Ala Gln Gly Glu
    130                 135                 140

gaa ctg aag aag gtg att ggg gca cct gct tat att gaa tgc agt gca 480
Glu Leu Lys Lys Val Ile Gly Ala Pro Ala Tyr Ile Glu Cys Ser Ala
145                 150                 155                 160

aaa aca caa cag aat gta aaa gcg gtg ttt gat gcg gct atc aag gta 528
Lys Thr Gln Gln Asn Val Lys Ala Val Phe Asp Ala Ala Ile Lys Val
                165                 170                 175

gtt ctc caa cca ccc aaa aac aag aag agg aag aag aga aag tct cag 576
Val Leu Gln Pro Pro Lys Asn Lys Lys Arg Lys Lys Arg Lys Ser Gln
            180                 185                 190

aaa gct tgt tct ata ttg tga 597
Lys Ala Cys Ser Ile Leu
        195

<210> 10

<211> 198

<212> PRT

<213> Brassica napus

<400> 10

Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Ile
        35                  40                  45

Val Asp Gly Asn Thr Ile Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Val Ser Lys Ala Ser Tyr Glu Asn Val
                85                  90                  95

Ser Lys Lys Trp Val Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Val Glu His Pro Gly Ala Val Pro Ile Ser Thr Ala Gln Gly Glu
    130                 135                 140

Glu Leu Lys Lys Val Ile Gly Ala Pro Ala Tyr Ile Glu Cys Ser Ala
145                 150                 155                 160

Lys Thr Gln Gln Asn Val Lys Ala Val Phe Asp Ala Ala Ile Lys Val
                165                 170                 175

Val Leu Gln Pro Pro Lys Asn Lys Lys Arg Lys Lys Arg Lys Ser Gln
            180                 185                 190

Lys Ala Cys Ser Ile Leu
        195

<210>  11

<211>  591

<212>  DNA

<213>  Glycine max


<220>

<221>  CDS

<222>  (1)..(591)

<223>


<400>  11
atg agt acg gca agg ttt atc aag tgt gta aca gtt gga gat ggt gct      48
Met Ser Thr Ala Arg Phe Ile Lys Cys Val Thr Val Gly Asp Gly Ala
1               5                   10                  15

gtg gga aag aca tgc atg ctt ata tcc tat acc agc aat acc ttt ccc      96
Val Gly Lys Thr Cys Met Leu Ile Ser Tyr Thr Ser Asn Thr Phe Pro
            20                  25                  30

acg gat tat gtt cca aca gtg ttt gac aat ttc agt gct aat gta acg 144
Thr Asp Tyr Val Pro Thr Val Phe Asp Asn Phe Ser Ala Asn Val Thr
        35                  40                  45

gtg gat ggt agt act gtt aat ctt ggt tta tgg gac act gca gga caa 192
Val Asp Gly Ser Thr Val Asn Leu Gly Leu Trp Asp Thr Ala Gly Gln
    50                  55                  60

gaa gat tac aac agg cta agg cct tta agc tat aga gga gct gat gtg 240
Glu Asp Tyr Asn Arg Leu Arg Pro Leu Ser Tyr Arg Gly Ala Asp Val
65                  70                  75                  80

ttt ttg ttg tgc tat tct ctc atc agc aaa gcc agt tat gag aac atc 288
Phe Leu Leu Cys Tyr Ser Leu Ile Ser Lys Ala Ser Tyr Glu Asn Ile
                85                  90                  95

tcc aaa aag tgg ata cct gag cta aga cat tat gct cca aat gtg cct 336
Ser Lys Lys Trp Ile Pro Glu Leu Arg His Tyr Ala Pro Asn Val Pro
            100                 105                 110

ata gtg ctg gtg gga aca aaa cta gat ttg cga gat gac aag caa ttt 384
Ile Val Leu Val Gly Thr Lys Leu Asp Leu Arg Asp Asp Lys Gln Phe
        115                 120                 125

ctg att gat cat ccg gga tcc gca cga ata aca act gct cag ggt gaa 432
Leu Ile Asp His Pro Gly Ser Ala Arg Ile Thr Thr Ala Gln Gly Glu
    130                 135                 140

gaa [?]tg aag aaa atg att ggt gca gtc act tat att gag tgc agc tcc 480
Glu Leu Lys Lys Met Ile Gly Ala Val Thr Tyr Ile Glu Cys Ser Ser
145                 150                 155                 160

aaa aca cag ctg aat gtg aag aca gtt ttt gat gct gca ata aag gtt 528
Lys Thr Gln Leu Asn Val Lys Thr Val Phe Asp Ala Ala Ile Lys Val
                165                 170                 175

gca ttg aag cca cca aag cca aag aag aaa cca cgc aag aaa agg acc 576
Ala Leu Lys Pro Pro Lys Pro Lys Lys Lys Pro Arg Lys Lys Arg Thr
            180                 185                 190

tgt act ttc ctc tga 591
Cys Thr Phe Leu
        195

<210> 12

<211> 196

<212> PRT

<213> Glycine max

<400> 12

Met Ser Thr Ala Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Thr
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Cys Tyr Ser Leu Ile Ser Lys Ala Ser Tyr Glu Asn Ile
                85                  90                  95

Ser Lys Lys Trp Ile Pro Glu Leu Arg His Tyr Ala Pro Asn Val Pro
            100                 105                 110

Ile Val Leu Val Gly Thr Lys Leu Asp Leu Arg Asp Asp Lys Gln Phe
        115                 120                 125

Leu Ile Asp His Pro Gly Ser Ala Arg Ile Thr Thr Ala Gln Gly Glu
    130                 135                 140

Glu Leu Lys Lys Met Ile Gly Ala Val Thr Tyr Ile Glu Cys Ser Ser
145                 150                 155                 160

Lys Thr Gln Leu Asn Val Lys Thr Val Phe Asp Ala Ala Ile Lys Val
                165                 170                 175

Ala Leu Lys Pro Pro Lys Pro Lys Lys Lys Pro Arg Lys Lys Arg Thr
            180                 185                 190

Cys Thr Phe Leu
        195

<210>  13

<211>  594

<212>  DNA

<213>  Glycine max

<220>

<221>  CDS

<222>  (1)..(594)

<223>

<400>  13
atg agc aca aca cgg ttt att aag tgt gtc aca gtt ggt gat ggg gct       48
Met Ser Thr Thr Arg Phe Ile Lys Cys Val Thr Val Gly Asp Gly Ala
1               5                   10                  15

gtg gga aag act tgc atg ctt atc tct tac act agc aac act ttc ccc       96

14

Val Gly Lys Thr Cys Met Leu Ile Ser Tyr Thr Ser Asn Thr Phe Pro
            20                  25                  30

acg gat tat gtt cct aca gtt ttc gac aat ttc agt gca aat gtt gtg      144
Thr Asp Tyr Val Pro Thr Val Phe Asp Asn Phe Ser Ala Asn Val Val
        35                  40                  45

gtt gat ggc agc aca gtt aac ctg gga ttg tgg gac act gct gga cag      192
Val Asp Gly Ser Thr Val Asn Leu Gly Leu Trp Asp Thr Ala Gly Gln
    50                  55                  60

gaa gat tac aac agg ctt agg cca ttg agt tac aga gga gca gat gtg      240
Glu Asp Tyr Asn Arg Leu Arg Pro Leu Ser Tyr Arg Gly Ala Asp Val
65                  70                  75                  80

ttc ttg ctg gcc ttt tcc ctc atc agc aaa gcc agc tat gaa aat ata      288
Phe Leu Leu Ala Phe Ser Leu Ile Ser Lys Ala Ser Tyr Glu Asn Ile
                85                  90                  95

tct aaa aag tgg att cct gaa ttg aga cat tat gcc cca act gtg cct      336
Ser Lys Lys Trp Ile Pro Glu Leu Arg His Tyr Ala Pro Thr Val Pro
            100                 105                 110

att gta ctg gtt gga act aaa ctt gat ttg agg gaa gac agg caa tat      384
Ile Val Leu Val Gly Thr Lys Leu Asp Leu Arg Glu Asp Arg Gln Tyr
        115                 120                 125

ttg att gat cat cct gga acc aca gct ata gct act gcc cag gga gaa      432
Leu Ile Asp His Pro Gly Thr Thr Ala Ile Ala Thr Ala Gln Gly Glu
    130                 135                 140

gaa ctg aag aag gca att ggt gct gct gtg tac ata gag tgc agc tca      480
Glu Leu Lys Lys Ala Ile Gly Ala Ala Val Tyr Ile Glu Cys Ser Ser
145                 150                 155                 160

aag act cag cag aat gtg aag gcc gtg ttt gat gct gca atc aag gtt      528
Lys Thr Gln Gln Asn Val Lys Ala Val Phe Asp Ala Ala Ile Lys Val
                165                 170                 175

gtt ttg caa cca cct aag tcc aag aaa aaa gga aag aag aag aac acg      576
Val Leu Gln Pro Pro Lys Ser Lys Lys Lys Gly Lys Lys Lys Asn Thr
            180                 185                 190

cct tgt gtt ttc ctc tga                                              594
Pro Cys Val Phe Leu
        195

<210>  14

<211>  197

<212>  PRT

<213>  Glycine max

<400>  14

Met Ser Thr Thr Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Lys Ala Ser Tyr Glu Asn Ile
                85                  90                  95

Ser Lys Lys Trp Ile Pro Glu Leu Arg His Tyr Ala Pro Thr Val Pro
            100                 105                 110

Ile Val Leu Val Gly Thr Lys Leu Asp Leu Arg Glu Asp Arg Gln Tyr
        115                 120                 125

Leu Ile Asp His Pro Gly Thr Thr Ala Ile Ala Thr Ala Gln Gly Glu
    130                 135                 140

Glu Leu Lys Lys Ala Ile Gly Ala Ala Val Tyr Ile Glu Cys Ser Ser
145                 150                 155                 160

Lys Thr Gln Gln Asn Val Lys Ala Val Phe Asp Ala Ala Ile Lys Val
                165                 170                 175

Val Leu Gln Pro Pro Lys Ser Lys Lys Lys Gly Lys Lys Lys Asn Thr
            180                 185                 190

Pro Cys Val Phe Leu
        195

<210>  15

<211>  591

<212>  DNA

<213>  Glycine max

<220>

<221>  CDS

<222>  (1)..(591)

<223>

<400>  15
atg agt gcg tcg agg ttc att aag tgc gtc acc gtc ggc gac ggt gct        48
Met Ser Ala Ser Arg Phe Ile Lys Cys Val Thr Val Gly Asp Gly Ala
1               5                   10                  15

gtc ggc aaa acc tgc ttg ttg att tcc tac acc agc aac act ttt ccc     96
Val Gly Lys Thr Cys Leu Leu Ile Ser Tyr Thr Ser Asn Thr Phe Pro
            20                  25                  30

acg gac tat gtg ccc acc gtt ttt gac aat ttc agt gct aat gtg gtg     144
Thr Asp Tyr Val Pro Thr Val Phe Asp Asn Phe Ser Ala Asn Val Val
        35                  40                  45

gtg gat gga agc acc gta aac cta gga ttg tgg gat aca gct ggt cag     192
Val Asp Gly Ser Thr Val Asn Leu Gly Leu Trp Asp Thr Ala Gly Gln
    50                  55                  60

gag gat tac aat aga tta aga ccc ttg agc tat cga gga gct gat gtc     240
Glu Asp Tyr Asn Arg Leu Arg Pro Leu Ser Tyr Arg Gly Ala Asp Val
65                  70                  75                  80

ttc ata ctt gcc ttt tct ctc ata agc aag gct agc tat gaa aat att     288
Phe Ile Leu Ala Phe Ser Leu Ile Ser Lys Ala Ser Tyr Glu Asn Ile
                85                  90                  95

gca aag aag tgg atc cct gaa cta agg cat tat gcc cct ggt gtt cca     336
Ala Lys Lys Trp Ile Pro Glu Leu Arg His Tyr Ala Pro Gly Val Pro
            100                 105                 110

ata att ctc gtt gga aca aag tta gat ctt cgg gat gat aag caa ttt     384
Ile Ile Leu Val Gly Thr Lys Leu Asp Leu Arg Asp Asp Lys Gln Phe
        115                 120                 125

ttt atg gac cat cct ggt gca gtg cca att act aca gca cag gga gaa     432
Phe Met Asp His Pro Gly Ala Val Pro Ile Thr Thr Ala Gln Gly Glu
    130                 135                 140

gaa ttg aga aag ctg att ggt gct ccg gcc tac att gag tgt agt tcc     480
Glu Leu Arg Lys Leu Ile Gly Ala Pro Ala Tyr Ile Glu Cys Ser Ser
145                 150                 155                 160

aaa acg caa cag aac gtg aaa gct gtc ttt gac gcg gca atc aaa gtg     528
Lys Thr Gln Gln Asn Val Lys Ala Val Phe Asp Ala Ala Ile Lys Val
                165                 170                 175

gtt atc caa cca cca aag cta aag aaa aag aga aaa aca cag aaa gct     576
Val Ile Gln Pro Pro Lys Leu Lys Lys Lys Arg Lys Thr Gln Lys Ala
            180                 185                 190

tgc tcc ata tta tga                                                 591
Cys Ser Ile Leu
        195

<210>  16

<211>  196

<212>  PRT

<213>  Glycine max

<400>  16

Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Ile
                85                  90                  95

Ala Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Met Asp His Pro Gly Ala Val Pro Ile Thr Thr Ala Gln Gly Glu
    130                 135                 140

Glu Leu Arg Lys Leu Ile Gly Ala Pro Ala Tyr Ile Glu Cys Ser Ser
145                 150                 155                 160

Lys Thr Gln Gln Asn Val Lys Ala Val Phe Asp Ala Ala Ile Lys Val
                165                 170                 175

Val Ile Gln Pro Pro Lys Leu Lys Lys Lys Arg Lys Thr Gln Lys Ala
            180                 185                 190

Cys Ser Ile Leu
        195

<210>  17

<211>  594

<212>  DNA

<213>  Glycine max

<220>

<221>  CDS

<222>  (1)..(594)

<223>

<400>  17
atg agc gct tct agg ttc atc aag tgc gtc act gtt ggg gat ggt gct        48

Met Ser Ala Ser Arg Phe Ile Lys Cys Val Thr Val Gly Asp Gly Ala
1               5                   10                  15

gtg ggc aaa acc tgt ttg ctt att tcc tac acc agc aac act ttt ccc      96
Val Gly Lys Thr Cys Leu Leu Ile Ser Tyr Thr Ser Asn Thr Phe Pro
            20                  25                  30

acc gat tat gtg ccg act gtt ttt gac aat ttc agc gca aat gtg gtt      144
Thr Asp Tyr Val Pro Thr Val Phe Asp Asn Phe Ser Ala Asn Val Val
        35                  40                  45

gtc aat ggg agc att gtg aat ctg ggt ttg tgg gat act gct gga caa      192
Val Asn Gly Ser Ile Val Asn Leu Gly Leu Trp Asp Thr Ala Gly Gln
    50                  55                  60

gag gat tat aac aga tta aga cct ttg agt tac cgt ggt gcc gat gtt      240
Glu Asp Tyr Asn Arg Leu Arg Pro Leu Ser Tyr Arg Gly Ala Asp Val
65                  70                  75                  80

ttc ata ctg gct ttc tct ctc ata agc aag gcc agt tat gaa aat gtc      288
Phe Ile Leu Ala Phe Ser Leu Ile Ser Lys Ala Ser Tyr Glu Asn Val
                85                  90                  95

tct aaa aag tgg att ccg gag ttg aag cat tat gct cct ggt gtc ccc      336
Ser Lys Lys Trp Ile Pro Glu Leu Lys His Tyr Ala Pro Gly Val Pro
            100                 105                 110

att att ctg gtt ggc aca aag ctt gac ctt cgg gat gat aag cag ttc      384
Ile Ile Leu Val Gly Thr Lys Leu Asp Leu Arg Asp Asp Lys Gln Phe
        115                 120                 125

tgc att gac cat cct ggt gcc gta cct att acc aca gct cag gga gaa      432
Cys Ile Asp His Pro Gly Ala Val Pro Ile Thr Thr Ala Gln Gly Glu
    130                 135                 140

gag ctt agg aag ctg att aat gcg cca gct tac att gaa tgc agt tca      480
Glu Leu Arg Lys Leu Ile Asn Ala Pro Ala Tyr Ile Glu Cys Ser Ser
145                 150                 155                 160

aaa aca cag gag aac gtg aag gca gtc ttt gat gca gcc ata aga gtt      528
Lys Thr Gln Glu Asn Val Lys Ala Val Phe Asp Ala Ala Ile Arg Val
                165                 170                 175

gtc ctt caa cca cct aag cag aag aaa aag aag ggt aaa gca caa aag      576
Val Leu Gln Pro Pro Lys Gln Lys Lys Lys Lys Gly Lys Ala Gln Lys
            180                 185                 190

gcc tgt tcg ata ttg tga                                              594
Ala Cys Ser Ile Leu
        195

<210>  18

<211>  197

<212>  PRT

<213>  Glycine max

<400>  18

Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asn Gly Ser Ile Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Val
                85                  90                  95

Ser Lys Lys Trp Ile Pro Glu Leu Lys His Tyr Ala Pro Gly Val Pro
            100                 105                 110

Ile Ile Leu Val Gly Thr Lys Leu Asp Leu Arg Asp Asp Lys Gln Phe
        115                 120                 125

Cys Ile Asp His Pro Gly Ala Val Pro Ile Thr Thr Ala Gln Gly Glu
    130                 135                 140

Glu Leu Arg Lys Leu Ile Asn Ala Pro Ala Tyr Ile Glu Cys Ser Ser
145                 150                 155                 160

Lys Thr Gln Glu Asn Val Lys Ala Val Phe Asp Ala Ala Ile Arg Val
                165                 170                 175

Val Leu Gln Pro Pro Lys Gln Lys Lys Lys Lys Gly Lys Ala Gln Lys
            180                 185                 190

Ala Cys Ser Ile Leu
        195

<210>  19

<211>  594

<212>  DNA

<213>  Glycine max

<220>

<221>  CDS

<222>  (1)..(594)

<223>

<400> 19
atg agt gcg tcc agg ttc atc aag tgt gtc act gtg ggt gac ggt gcc 48
Met Ser Ala Ser Arg Phe Ile Lys Cys Val Thr Val Gly Asp Gly Ala
1               5                   10                  15

gtt ggc aag act tgc atg ctc atc tcc tac acc agc aac act ttt cct 96
Val Gly Lys Thr Cys Met Leu Ile Ser Tyr Thr Ser Asn Thr Phe Pro
            20                  25                  30

acg gac tac gtg cca act gtc ttt gac aat ttc agt gca aat gtc gtt 144
Thr Asp Tyr Val Pro Thr Val Phe Asp Asn Phe Ser Ala Asn Val Val
        35                  40                  45

gtg gat gga agc act gtg aat ctt ggg ttg tgg gat act gct ggc caa 192
Val Asp Gly Ser Thr Val Asn Leu Gly Leu Trp Asp Thr Ala Gly Gln
    50                  55                  60

gaa gat tac aat aga ttg aga ccc tta agc tat cgt gga gca gat gta 240
Glu Asp Tyr Asn Arg Leu Arg Pro Leu Ser Tyr Arg Gly Ala Asp Val
65                  70                  75                  80

ttc ctg ctt gct ttc tct ctc ata agc agg gcc agc tat gaa aat gtt 288
Phe Leu Leu Ala Phe Ser Leu Ile Ser Arg Ala Ser Tyr Glu Asn Val
                85                  90                  95

gcc aag aaa tgg att cct gag ttg agg cat tat gct cct ggt gtt cca 336
Ala Lys Lys Trp Ile Pro Glu Leu Arg His Tyr Ala Pro Gly Val Pro
            100                 105                 110

att att ctt gtt gga aca aaa ctt gat ctt cgg gat gat aag cag ttc 384
Ile Ile Leu Val Gly Thr Lys Leu Asp Leu Arg Asp Asp Lys Gln Phe
        115                 120                 125

ttt caa gac cat cct ggt gca gtg cct atc acc aca gca cag ggt gag 432
Phe Gln Asp His Pro Gly Ala Val Pro Ile Thr Thr Ala Gln Gly Glu
    130                 135                 140

gaa ctg aga aag ctt atc ggt gct cca att tac att gaa tgt agt tca 480
Glu Leu Arg Lys Leu Ile Gly Ala Pro Ile Tyr Ile Glu Cys Ser Ser
145                 150                 155                 160

aaa aca caa cag aat gtg aag gct gtt ttt gat gca gcc atc aag gta 528
Lys Thr Gln Gln Asn Val Lys Ala Val Phe Asp Ala Ala Ile Lys Val
                165                 170                 175

gtt ctc cag ccc cca aag cag aag aaa aag aag aga aag gga caa aag 576
Val Leu Gln Pro Pro Lys Gln Lys Lys Lys Lys Arg Lys Gly Gln Lys
            180                 185                 190

gcc tgt tcc att ttg tga 594
Ala Cys Ser Ile Leu
        195

<210> 20

<211> 197

<212> PRT

<213> Glycine max

<400> 20

Met Ser Ala Ser Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Arg Ala Ser Tyr Glu Asn Val
                85                  90                  95

Ala Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Gln Asp His Pro Gly Ala Val Pro Ile Thr Thr Ala Gln Gly Glu
    130                 135                 140

Glu Leu Arg Lys Leu Ile Gly Ala Pro Ile Tyr Ile Glu Cys Ser Ser
145                 150                 155                 160

Lys Thr Gln Gln Asn Val Lys Ala Val Phe Asp Ala Ala Ile Lys Val
                165                 170                 175

Val Leu Gln Pro Pro Lys Gln Lys Lys Lys Lys Arg Lys Gly Gln Lys
            180                 185                 190

Ala Cys Ser Ile Leu
        195

<210>  21

<211>  594

<212>  DNA

<213>  Glycine max


<220>

<221>  CDS

<222>  (1)..(594)

<223>

<400> 21
atg agt gcg tcc agg ttc atc aag tgt gtc act gtg ggt gac ggt gcc      48
Met Ser Ala Ser Arg Phe Ile Lys Cys Val Thr Val Gly Asp Gly Ala
1               5                   10                  15

gtt ggc aag act tgc atg ctc atc tcc tac acc agc aac act ttt cct      96
Val Gly Lys Thr Cys Met Leu Ile Ser Tyr Thr Ser Asn Thr Phe Pro
            20                  25                  30

acg gac tac gtg cca act gtc ttt gac aat ttc agt gca aat gtc gtt      144
Thr Asp Tyr Val Pro Thr Val Phe Asp Asn Phe Ser Ala Asn Val Val
        35                  40                  45

gtg gat gga agc act gtg aat ctt ggg ttg tgg gat act gct ggc caa      192
Val Asp Gly Ser Thr Val Asn Leu Gly Leu Trp Asp Thr Ala Gly Gln
    50                  55                  60

gaa gat tac aat aga ttg aga ccc tta agc tat cgt gga gca gat gta      240
Glu Asp Tyr Asn Arg Leu Arg Pro Leu Ser Tyr Arg Gly Ala Asp Val
65                  70                  75                  80

ttc ctg ctt gct ttc tct ctc ata agc agg gcc agc tat gaa aat gtt      288
Phe Leu Leu Ala Phe Ser Leu Ile Ser Arg Ala Ser Tyr Glu Asn Val
                85                  90                  95

gcc aag aaa tgg att cct gag ttg agg cat tat gct cct ggt gtt cca      336
Ala Lys Lys Trp Ile Pro Glu Leu Arg His Tyr Ala Pro Gly Val Pro
            100                 105                 110

att att ctt gtt gga aca aaa ctt gat ctt cgg gat gat aag cag ttc      384
Ile Ile Leu Val Gly Thr Lys Leu Asp Leu Arg Asp Asp Lys Gln Phe
        115                 120                 125

ttt caa gac cat cct ggt gca gtg cct atc acc aca gca cag ggt gag      432
Phe Gln Asp His Pro Gly Ala Val Pro Ile Thr Thr Ala Gln Gly Glu
    130                 135                 140

gaa ctg aga aag ctt atc ggt gct cca att tac att gaa tgt agt tca      480
Glu Leu Arg Lys Leu Ile Gly Ala Pro Ile Tyr Ile Glu Cys Ser Ser
145                 150                 155                 160

aaa aca caa cag aat gtg aag gct gtt ttt gat gca gcc atc aag gta      528
Lys Thr Gln Gln Asn Val Lys Ala Val Phe Asp Ala Ala Ile Lys Val
                165                 170                 175

gtt ctc cag ccc cca aag cag aag aaa aag aag aga aag gga caa aag      576
Val Leu Gln Pro Pro Lys Gln Lys Lys Lys Lys Arg Lys Gly Gln Lys
            180                 185                 190

gcc tgt tcc att ttg tga                                              594
Ala Cys Ser Ile Leu
        195


<210> 22

<211> 197

<212> PRT

<213> Glycine max


<400> 22

Met Ser Ala Ser Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Arg Ala Ser Tyr Glu Asn Val
                85                  90                  95

Ala Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Gln Asp His Pro Gly Ala Val Pro Ile Thr Thr Ala Gln Gly Glu
    130                 135                 140

Glu Leu Arg Lys Leu Ile Gly Ala Pro Ile Tyr Ile Glu Cys Ser Ser
145                 150                 155                 160

Lys Thr Gln Gln Asn Val Lys Ala Val Phe Asp Ala Ala Ile Lys Val
                165                 170                 175

Val Leu Gln Pro Pro Lys Gln Lys Lys Lys Lys Arg Lys Gly Gln Lys
            180                 185                 190

Ala Cys Ser Ile Leu
        195

<210>  23

<211>  639

<212>  DNA

<213>  Glycine max


<220>

<221>  CDS

<222>  (1)..(639)

<223>

<400> 23
atg gct tcc gca act gcc cca aga ttc atc aag tgc gtg acc gtt ggt 48
Met Ala Ser Ala Thr Ala Pro Arg Phe Ile Lys Cys Val Thr Val Gly
1               5                   10                  15

gat gga gct gta ggg aag acc tgc atg ctc att tgc tat acc agc aac 96
Asp Gly Ala Val Gly Lys Thr Cys Met Leu Ile Cys Tyr Thr Ser Asn
            20                  25                  30

aaa ttc ccc acg gac tat atc ccc act gtg ttt gat aat ttc agt gca 144
Lys Phe Pro Thr Asp Tyr Ile Pro Thr Val Phe Asp Asn Phe Ser Ala
        35                  40                  45

aat gtg gtt gtt gaa ggc ata act gtc aac tta ggc ctt tgg gat aca 192
Asn Val Val Val Glu Gly Ile Thr Val Asn Leu Gly Leu Trp Asp Thr
    50                  55                  60

gct ggg caa gag gat tac aac agg ctg agg ccc ttg agc tac agg ggg 240
Ala Gly Gln Glu Asp Tyr Asn Arg Leu Arg Pro Leu Ser Tyr Arg Gly
65                  70                  75                  80

gca gat gtc ttt gtc ttg gct ttt tct tta gtt agt cgc gcg agc tat 288
Ala Asp Val Phe Val Leu Ala Phe Ser Leu Val Ser Arg Ala Ser Tyr
                85                  90                  95

gag aat gtg ctg aag aag tgg atc cct gaa ctc cag cat ttt gcc cct 336
Glu Asn Val Leu Lys Lys Trp Ile Pro Glu Leu Gln His Phe Ala Pro
            100                 105                 110

ggc atc ccg ttg gtg tta gtt ggc acc aaa ttg gat cta cga gaa gac 384
Gly Ile Pro Leu Val Leu Val Gly Thr Lys Leu Asp Leu Arg Glu Asp
        115                 120                 125

aag cac tac atg gct gat cat ccc agc ttg gtg cca gtg act act gat 432
Lys His Tyr Met Ala Asp His Pro Ser Leu Val Pro Val Thr Thr Asp
    130                 135                 140

caa ggt gag gaa ctc cgt aaa cac att gga gct acc tac tat att gag 480
Gln Gly Glu Glu Leu Arg Lys His Ile Gly Ala Thr Tyr Tyr Ile Glu
145                 150                 155                 160

tgc agc tca aaa act cag cag aat gtg aag gca gtt ttt gat gct gct 528
Cys Ser Ser Lys Thr Gln Gln Asn Val Lys Ala Val Phe Asp Ala Ala
                165                 170                 175

att aga atg gtc atc aag cct cca caa aag caa aac gag aaa aga aag 576
Ile Arg Met Val Ile Lys Pro Pro Gln Lys Gln Asn Glu Lys Arg Lys
            180                 185                 190

aaa aaa cca cgt ggc tgt ttc cta aac gtc ctc tgt cga agg aac att 624
Lys Lys Pro Arg Gly Cys Phe Leu Asn Val Leu Cys Arg Arg Asn Ile
        195                 200                 205

gtt cgc ctt aaa tga 639
Val Arg Leu Lys
    210

<210> 24

<211> 212

<212> PRT

<213>  Glycine max

<400>  24

Met Ala Ser Ala Thr Ala Pro Arg Phe Ile Lys Cys Val Thr Val Gly
1               5                   10                  15

Asp Gly Ala Val Gly Lys Thr Cys Met Leu Ile Cys Tyr Thr Ser Asn
            20                  25                  30

Lys Phe Pro Thr Asp Tyr Ile Pro Thr Val Phe Asp Asn Phe Ser Ala
        35                  40                  45

Asn Val Val Val Glu Gly Ile Thr Val Asn Leu Gly Leu Trp Asp Thr
    50                  55                  60

Ala Gly Gln Glu Asp Tyr Asn Arg Leu Arg Pro Leu Ser Tyr Arg Gly
65                  70                  75                  80

Ala Asp Val Phe Val Leu Ala Phe Ser Leu Val Ser Arg Ala Ser Tyr
                85                  90                  95

Glu Asn Val Leu Lys Lys Trp Ile Pro Glu Leu Gln His Phe Ala Pro
            100                 105                 110

Gly Ile Pro Leu Val Leu Val Gly Thr Lys Leu Asp Leu Arg Glu Asp
        115                 120                 125

Lys His Tyr Met Ala Asp His Pro Ser Leu Val Pro Val Thr Thr Asp
    130                 135                 140

Gln Gly Glu Glu Leu Arg Lys His Ile Gly Ala Thr Tyr Tyr Ile Glu
145                 150                 155                 160

Cys Ser Ser Lys Thr Gln Gln Asn Val Lys Ala Val Phe Asp Ala Ala
                165                 170                 175

Ile Arg Met Val Ile Lys Pro Pro Gln Lys Gln Asn Glu Lys Arg Lys
            180                 185                 190

Lys Lys Pro Arg Gly Cys Phe Leu Asn Val Leu Cys Arg Arg Asn Ile
        195                 200                 205

Val Arg Leu Lys
    210

<210>  25

<211>  762

<212>  DNA

<213>  Brassica napus

<220>

<221>  CDS

<222>  (1)..(762)

<223>

<400>  25
atg gtt gga cca gga aga cct caa atc gtc ctc ttc ggt tca tca atc      48
Met Val Gly Pro Gly Arg Pro Gln Ile Val Leu Phe Gly Ser Ser Ile
1               5                   10                  15

gtt caa tac agc ttc agc gat ggt gga tgg gga gcc act ctc gct aac      96
Val Gln Tyr Ser Phe Ser Asp Gly Gly Trp Gly Ala Thr Leu Ala Asn
            20                  25                  30

atc tac tct cgc acg gct gac gta atc ctt cgt ggg tat gct ggt tgg      144
Ile Tyr Ser Arg Thr Ala Asp Val Ile Leu Arg Gly Tyr Ala Gly Trp
        35                  40                  45

aac tcc aga tct gcc ttg aag gtg tta aac caa gtg ttc cca aag gat      192
Asn Ser Arg Ser Ala Leu Lys Val Leu Asn Gln Val Phe Pro Lys Asp
    50                  55                  60

gct gtt ata caa cct tct ttg gtg ata gtc tat ttc gga ggg aat gat      240
Ala Val Ile Gln Pro Ser Leu Val Ile Val Tyr Phe Gly Gly Asn Asp
65                  70                  75                  80

tca atg cct cct cat cca tca ggg caa gga cct cat gtt cct ctc tct      288
Ser Met Pro Pro His Pro Ser Gly Gln Gly Pro His Val Pro Leu Ser
                85                  90                  95

gaa ttc act gag aac atg agg aag atc gga gag cat ctt ttg agc ctc      336
Glu Phe Thr Glu Asn Met Arg Lys Ile Gly Glu His Leu Leu Ser Leu
            100                 105                 110

tcg gac aag acc cgt gtc att ttt ctc act ccc cca cca atg aac gag      384
Ser Asp Lys Thr Arg Val Ile Phe Leu Thr Pro Pro Pro Met Asn Glu
        115                 120                 125

aga caa atc caa cta gtg ttt gga gat gca atg aga ggc cgg agt aac      432
Arg Gln Ile Gln Leu Val Phe Gly Asp Ala Met Arg Gly Arg Ser Asn
    130                 135                 140

gag ctg tgt cgt cca tac gca gaa gcg ttg ttg aat cta tgc aga gag      480
Glu Leu Cys Arg Pro Tyr Ala Glu Ala Leu Leu Asn Leu Cys Arg Glu
145                 150                 155                 160

atc aat gtg aaa ggt atc gat ctt tgg aac gca ata cag caa caa gat      528
Ile Asn Val Lys Gly Ile Asp Leu Trp Asn Ala Ile Gln Gln Gln Asp
                165                 170                 175

gat tgg tta cac act tgc ttc act gac ggt atc cat ttc acg gcc aag      576
Asp Trp Leu His Thr Cys Phe Thr Asp Gly Ile His Phe Thr Ala Lys
            180                 185                 190

gcg agc gag att gtg gtg aag gag ata ttg aaa gta gtg aga gaa gct      624
Ala Ser Glu Ile Val Val Lys Glu Ile Leu Lys Val Val Arg Glu Ala
        195                 200                 205

gat tgg aaa ccg agt ctt gac agg aag tca tta ccg gtt gag ttt cca      672

Asp Trp Lys Pro Ser Leu Asp Arg Lys Ser Leu Pro Val Glu Phe Pro
    210                 215                 220

ttt gat tct ggt cta cca aac tcc cca aga cat agt gat cta gaa tta       720
Phe Asp Ser Gly Leu Pro Asn Ser Pro Arg His Ser Asp Leu Glu Leu
225                 230                 235                 240

act aga aac aag aag ttg gag cct cgt atg gcc cga ttg taa               762
Thr Arg Asn Lys Lys Leu Glu Pro Arg Met Ala Arg Leu
                245                 250

<210>  26

<211>  253

<212>  PRT

<213>  Brassica napus

<400>  26

Met Val Gly Pro Gly Arg Pro Gln Ile Val Leu Phe Gly Ser Ser Ile
1               5                   10                  15

Val Gln Tyr Ser Phe Ser Asp Gly Gly Trp Gly Ala Thr Leu Ala Asn
            20                  25                  30

Ile Tyr Ser Arg Thr Ala Asp Val Ile Leu Arg Gly Tyr Ala Gly Trp
        35                  40                  45

Asn Ser Arg Ser Ala Leu Lys Val Leu Asn Gln Val Phe Pro Lys Asp
    50                  55                  60

Ala Val Ile Gln Pro Ser Leu Val Ile Val Tyr Phe Gly Gly Asn Asp
65                  70                  75                  80

Ser Met Pro Pro His Pro Ser Gly Gln Gly Pro His Val Pro Leu Ser
                85                  90                  95

Glu Phe Thr Glu Asn Met Arg Lys Ile Gly Glu His Leu Leu Ser Leu
            100                 105                 110

Ser Asp Lys Thr Arg Val Ile Phe Leu Thr Pro Pro Pro Met Asn Glu
        115                 120                 125

Arg Gln Ile Gln Leu Val Phe Gly Asp Ala Met Arg Gly Arg Ser Asn
    130                 135                 140

Glu Leu Cys Arg Pro Tyr Ala Glu Ala Leu Leu Asn Leu Cys Arg Glu
145                 150                 155                 160

Ile Asn Val Lys Gly Ile Asp Leu Trp Asn Ala Ile Gln Gln Gln Asp
                165                 170                 175

Asp Trp Leu His Thr Cys Phe Thr Asp Gly Ile His Phe Thr Ala Lys
            180                 185                 190

Ala Ser Glu Ile Val Val Lys Glu Ile Leu Lys Val Val Arg Glu Ala
        195                 200                 205

Asp Trp Lys Pro Ser Leu Asp Arg Lys Ser Leu Pro Val Glu Phe Pro
    210                 215                 220

Phe Asp Ser Gly Leu Pro Asn Ser Pro Arg His Ser Asp Leu Glu Leu
225                 230                 235                 240

Thr Arg Asn Lys Lys Leu Glu Pro Arg Met Ala Arg Leu
                245                 250

<210> 27

<211> 651

<212> DNA

<213> Brassica napus

<220>

<221> CDS

<222> (1)..(261)

<223>

<400> 27
atg gct tca act gct tca aag ttc ata aaa tgt gtg act gtt ggt gat 48
Met Ala Ser Thr Ala Ser Lys Phe Ile Lys Cys Val Thr Val Gly Asp
1               5                   10                  15

ggc gcc gta ggt aaa acc tgt atg ctc atc tgc tac acc agc aac aaa 96
Gly Ala Val Gly Lys Thr Cys Met Leu Ile Cys Tyr Thr Ser Asn Lys
            20                  25                  30

ttc cct act gac tac ata cca aca gtt ttt gac aac ttt agt gca aac 144
Phe Pro Thr Asp Tyr Ile Pro Thr Val Phe Asp Asn Phe Ser Ala Asn
        35                  40                  45

gtt gta gtt gaa ggc acc act gtg aac cta ggc cta tgg gac act gct 192
Val Val Val Glu Gly Thr Thr Val Asn Leu Gly Leu Trp Asp Thr Ala
    50                  55                  60

ggg caa gaa gac tac aac aga tta agg cct tta agt tac aga gga gca 240
Gly Gln Glu Asp Tyr Asn Arg Leu Arg Pro Leu Ser Tyr Arg Gly Ala
65                  70                  75                  80

gat gtt ttc gtc ctg tct ttc tccttggtca gccgagctag ctacgagaat 291
Asp Val Phe Val Leu Ser Phe
                85

gtttataaaa agtggatccc tgaactccaa cactttgccc caggagttcc attagtcctt 351

gttggtacca aactagatct ccgtgaagat aataagcatt atttggctga ccatcctgga 411

ctatcccctg taactactgc acagggagag gaattgcgta agctaatcgg tgcgacatat 471

tacattgaat gtagctcgaa aactcaacag aatgtgaaag cagtttttga ttcagcgatc 531

aaggaagtga tcaaaccggt ggttaaacaa aaggagaaga cgcagaaaac gaagaagcaa 591

aagtctaatc atggctgttt atcaaacgtt ctgtgtggga ggatagtgac tcggcattga 651

<210> 28

<211> 87

<212> PRT

<213> Brassica napus

<400> 28

Met Ala Ser Thr Ala Ser Lys Phe Ile Lys Cys Val Thr Val Gly Asp
1               5                   10                  15

Gly Ala Val Gly Lys Thr Cys Met Leu Ile Cys Tyr Thr Ser Asn Lys
            20                  25                  30

Phe Pro Thr Asp Tyr Ile Pro Thr Val Phe Asp Asn Phe Ser Ala Asn
        35                  40                  45

Val Val Val Glu Gly Thr Thr Val Asn Leu Gly Leu Trp Asp Thr Ala
    50                  55                  60

Gly Gln Glu Asp Tyr Asn Arg Leu Arg Pro Leu Ser Tyr Arg Gly Ala
65                  70                  75                  80

Asp Val Phe Val Leu Ser Phe
                85

<210> 29

<211> 651

<212> DNA

<213> Brassica napus

<220>

<221> CDS

<222> (1)..(651)

<223>

<400> 29
atg gct tca agt gct tca aag ttc atc aaa tgt gta act gtt ggt gat 48

Met Ala Ser Ser Ala Ser Lys Phe Ile Lys Cys Val Thr Val Gly Asp
1               5                   10                  15

ggt gcc gtt ggt aaa acc tgt atg ctc atc tgc tat acc agc aac aag     96
Gly Ala Val Gly Lys Thr Cys Met Leu Ile Cys Tyr Thr Ser Asn Lys
            20                  25                  30

ttc cct act gac tat gta cca acg gtt ttt gac aac ttt agt gca aac     144
Phe Pro Thr Asp Tyr Val Pro Thr Val Phe Asp Asn Phe Ser Ala Asn
        35                  40                  45

gtt gta gtt gaa gga act act gtg aac tta ggg cta tgg gat act gct     192
Val Val Val Glu Gly Thr Thr Val Asn Leu Gly Leu Trp Asp Thr Ala
    50                  55                  60

gga caa gaa gac tat aac aga tta agg cct tta agc tac aga gga gca     240
Gly Gln Glu Asp Tyr Asn Arg Leu Arg Pro Leu Ser Tyr Arg Gly Ala
65                  70                  75                  80

gat gtc ttc gtc ttg tct ttc tca ttg gtt agc cga gct agc tac gag     288
Asp Val Phe Val Leu Ser Phe Ser Leu Val Ser Arg Ala Ser Tyr Glu
                85                  90                  95

aat gtt ttt aaa aag tgg atc cct gaa ctc caa cac ttt gct cca gga     336
Asn Val Phe Lys Lys Trp Ile Pro Glu Leu Gln His Phe Ala Pro Gly
            100                 105                 110

gtt cca tta gtt ctt gtc ggt acc aaa tta gat ctc cgt gag gat aag     384
Val Pro Leu Val Leu Val Gly Thr Lys Leu Asp Leu Arg Glu Asp Lys
        115                 120                 125

cat tat ctg gct gac cat cct gga cta tcc cct gta act act gca cag     432
His Tyr Leu Ala Asp His Pro Gly Leu Ser Pro Val Thr Thr Ala Gln
    130                 135                 140

gga gag gag ttg cgt aag ctc att ggt gca aca tat tac att gaa tgt     480
Gly Glu Glu Leu Arg Lys Leu Ile Gly Ala Thr Tyr Tyr Ile Glu Cys
145                 150                 155                 160

agc tca aaa act caa cag aat gtc aaa gca gtt ttt gat tcg gca atc     528
Ser Ser Lys Thr Gln Gln Asn Val Lys Ala Val Phe Asp Ser Ala Ile
                165                 170                 175

aag gaa gtg atc aaa ccg gtg ctt aaa cag aag ggc aag acc aag aaa     576
Lys Glu Val Ile Lys Pro Val Leu Lys Gln Lys Gly Lys Thr Lys Lys
            180                 185                 190

aag aag aag caa cag tcg aat cac cac ggg tgt tta tca aac gtt ttg     624
Lys Lys Lys Gln Gln Ser Asn His His Gly Cys Leu Ser Asn Val Leu
        195                 200                 205

tgt ggg agg ata gtg acc cgg cat tga                                 651
Cys Gly Arg Ile Val Thr Arg His
    210                 215

<210>  30

<211>  216

<212>  PRT

<213>  Brassica napus

<400>  30

Met Ala Ser Ser Ala Ser Lys Phe Ile Lys Cys Val Thr Val Gly Asp
1               5                   10                  15

Gly Ala Val Gly Lys Thr Cys Met Leu Ile Cys Tyr Thr Ser Asn Lys
            20                  25                  30

Phe Pro Thr Asp Tyr Val Pro Thr Val Phe Asp Asn Phe Ser Ala Asn
        35                  40                  45

Val Val Val Glu Gly Thr Thr Val Asn Leu Gly Leu Trp Asp Thr Ala
    50                  55                  60

Gly Gln Glu Asp Tyr Asn Arg Leu Arg Pro Leu Ser Tyr Arg Gly Ala
65                  70                  75                  80

Asp Val Phe Val Leu Ser Phe Ser Leu Val Ser Arg Ala Ser Tyr Glu
                85                  90                  95

Asn Val Phe Lys Lys Trp Ile Pro Glu Leu Gln His Phe Ala Pro Gly
            100                 105                 110

Val Pro Leu Val Leu Val Gly Thr Lys Leu Asp Leu Arg Glu Asp Lys
        115                 120                 125

His Tyr Leu Ala Asp His Pro Gly Leu Ser Pro Val Thr Thr Ala Gln
    130                 135                 140

Gly Glu Glu Leu Arg Lys Leu Ile Gly Ala Thr Tyr Tyr Ile Glu Cys
145                 150                 155                 160

Ser Ser Lys Thr Gln Gln Asn Val Lys Ala Val Phe Asp Ser Ala Ile
                165                 170                 175

Lys Glu Val Ile Lys Pro Val Leu Lys Gln Lys Gly Lys Thr Lys Lys
            180                 185                 190

Lys Lys Lys Gln Gln Ser Asn His His Gly Cys Leu Ser Asn Val Leu
        195                 200                 205

Cys Gly Arg Ile Val Thr Arg His
    210                 215

<210>  31

<211>  606

<212>  DNA

<213>  Brassica napus


<220>

<221>  CDS

<222>  (1)..(606)

<223>

<400>  31
atg agc aca gcg aga ttc atc aag tgt gtg acg gtc gga gat gga gct      48
Met Ser Thr Ala Arg Phe Ile Lys Cys Val Thr Val Gly Asp Gly Ala
1               5                   10                  15

gtg ggg aag act tgt atg ctg att tca tat acc agc aat act ttt cct      96
Val Gly Lys Thr Cys Met Leu Ile Ser Tyr Thr Ser Asn Thr Phe Pro
            20                  25                  30

acg gat tac gtt ccg aca gtt ttt gac aat ttc agt gcg aat gtg gtg      144
Thr Asp Tyr Val Pro Thr Val Phe Asp Asn Phe Ser Ala Asn Val Val
        35                  40                  45

gtc gat gga agt act gtc aac ctc ggc ctg tgg gat act gct ggg cag      192
Val Asp Gly Ser Thr Val Asn Leu Gly Leu Trp Asp Thr Ala Gly Gln
    50                  55                  60

gaa gat tat aac agg ctt cgg cct ttg agt tac aga gga gca gat gtg      240
Glu Asp Tyr Asn Arg Leu Arg Pro Leu Ser Tyr Arg Gly Ala Asp Val
65                  70                  75                  80

ttt tta ttg gca ttt tcc cta att agc aag gcc agt tac gag aac att      288
Phe Leu Leu Ala Phe Ser Leu Ile Ser Lys Ala Ser Tyr Glu Asn Ile
                85                  90                  95

tac aaa aag tgg ctt ccg gag ctg aaa cat tat gcg cct agc atc ccc      336
Tyr Lys Lys Trp Leu Pro Glu Leu Lys His Tyr Ala Pro Ser Ile Pro
            100                 105                 110

att gta ctc gtc gga acc aag tta gat ttg agg gat gac aaa cag ttc      384
Ile Val Leu Val Gly Thr Lys Leu Asp Leu Arg Asp Asp Lys Gln Phe
        115                 120                 125

ttg aaa gat cat cca gga gca gct tca ata aca act gcc cag gga gag      432
Leu Lys Asp His Pro Gly Ala Ala Ser Ile Thr Thr Ala Gln Gly Glu
    130                 135                 140

gaa tta aga aag atg att gga gcc atc aag tac tta gaa tgc agc tcc      480
Glu Leu Arg Lys Met Ile Gly Ala Ile Lys Tyr Leu Glu Cys Ser Ser
145                 150                 155                 160

aaa acc cag cag aat gtg aag gca gtg ttt gat aca gcg atc cgg gta      528
Lys Thr Gln Gln Asn Val Lys Ala Val Phe Asp Thr Ala Ile Arg Val
                165                 170                 175

gcg ttg agg cct cca aag gca aag aag aag ata aag cca ttg agg acc      576
Ala Leu Arg Pro Pro Lys Ala Lys Lys Lys Ile Lys Pro Leu Arg Thr
            180                 185                 190

aaa aga tca aga aca tgc ttt ttc ttc taa                              606
Lys Arg Ser Arg Thr Cys Phe Phe Phe
        195                 200

<210>  32

<211>  201

<212>  PRT

<213>  Brassica napus

<400>  32

Met Ser Thr Ala Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Lys Ala Ser Tyr Glu Asn Ile
                85                  90                  95

Tyr Lys Lys Trp Leu Pro Glu Leu Lys His Tyr Ala Pro Ser Ile Pro
            100                 105                 110

Ile Val Leu Val Gly Thr Lys Leu Asp Leu Arg Asp Asp Lys Gln Phe
        115                 120                 125

Leu Lys Asp His Pro Gly Ala Ala Ser Ile Thr Thr Ala Gln Gly Glu
    130                 135                 140

Glu Leu Arg Lys Met Ile Gly Ala Ile Lys Tyr Leu Glu Cys Ser Ser
145                 150                 155                 160

Lys Thr Gln Gln Asn Val Lys Ala Val Phe Asp Thr Ala Ile Arg Val
                165                 170                 175

Ala Leu Arg Pro Pro Lys Ala Lys Lys Lys Ile Lys Pro Leu Arg Thr
            180                 185                 190

Lys Arg Ser Arg Thr Cys Phe Phe Phe
        195                 200

<210>  33

<211>  636

<212>  DNA

<213>  Brassica napus

<220>

<221>  CDS

<222>  (1)..(636)

<223>

<400>  33
atg tca gct tca gtg gct gct gca tca gta tca aca aca aca aca gca      48
Met Ser Ala Ser Val Ala Ala Ala Ser Val Ser Thr Thr Thr Thr Ala
1               5                   10                  15

gct aca acg ttt atc aag tgc gtc act gtt ggc gat gga gct gtg ggc      96
Ala Thr Thr Phe Ile Lys Cys Val Thr Val Gly Asp Gly Ala Val Gly
            20                  25                  30

aaa act tgt ctt ctt atc tcc tac acc agc aac acc ttt cct act gat      144
Lys Thr Cys Leu Leu Ile Ser Tyr Thr Ser Asn Thr Phe Pro Thr Asp
        35                  40                  45

tat gtt cct aca gtg ttc gac aac ttc agt gca aat gtt cta gtc gat      192
Tyr Val Pro Thr Val Phe Asp Asn Phe Ser Ala Asn Val Leu Val Asp
    50                  55                  60

ggc aaa acc gtc aat ctt ggt ctt tgg gat act gct ggt caa gaa gat      240
Gly Lys Thr Val Asn Leu Gly Leu Trp Asp Thr Ala Gly Gln Glu Asp
65                  70                  75                  80

tac aat agg ctt aga cca ttg agt tac aga gga gca gat gtt ttc att      288
Tyr Asn Arg Leu Arg Pro Leu Ser Tyr Arg Gly Ala Asp Val Phe Ile
                85                  90                  95

ctt gcc ttt tct ctt atc agc agg cct agc ttt gag aac att gct aaa      336
Leu Ala Phe Ser Leu Ile Ser Arg Pro Ser Phe Glu Asn Ile Ala Lys
            100                 105                 110

aag tgg gtc cct gag ctg cga cat tat gcc cct aac gtg cct att gtt      384
Lys Trp Val Pro Glu Leu Arg His Tyr Ala Pro Asn Val Pro Ile Val
        115                 120                 125

cta gtg gga act aaa tta gat cta aga gag gat aag aag ttc cca atg      432
Leu Val Gly Thr Lys Leu Asp Leu Arg Glu Asp Lys Lys Phe Pro Met
    130                 135                 140

aac tat cca ggt gct tgc aca atc tca aca gaa caa ggt caa gag cta      480
Asn Tyr Pro Gly Ala Cys Thr Ile Ser Thr Glu Gln Gly Gln Glu Leu
145                 150                 155                 160

aga aaa gag ata gga gca tta gca tat ata gag tgc agc tca aaa aca      528
Arg Lys Glu Ile Gly Ala Leu Ala Tyr Ile Glu Cys Ser Ser Lys Thr
                165                 170                 175

caa cag aac gtg aaa gcg gtg ttt gat gca gcg ata aaa gta gtt cta      576
Gln Gln Asn Val Lys Ala Val Phe Asp Ala Ala Ile Lys Val Val Leu
            180                 185                 190

cag cct cct aca aaa att aag aaa caa aag aga aga ttt cgt ttc tgc      624
Gln Pro Pro Thr Lys Ile Lys Lys Gln Lys Arg Arg Phe Arg Phe Cys
        195                 200                 205

cat gct ctc tga                                                      636
His Ala Leu
    210

<210>  34

<211>  211

<212>  PRT

<213>  Brassica napus

<400>  34

Met Ser Ala Ser Val Ala Ala Ala Ser Val Ser Thr Thr Thr Thr Ala
1               5                   10                  15

Ala Thr Thr Phe Ile Lys Cys Val Thr Val Gly Asp Gly Ala Val Gly
            20                  25                  30

Lys Thr Cys Leu Leu Ile Ser Tyr Thr Ser Asn Thr Phe Pro Thr Asp
        35                  40                  45

Tyr Val Pro Thr Val Phe Asp Asn Phe Ser Ala Asn Val Leu Val Asp
    50                  55                  60

Gly Lys Thr Val Asn Leu Gly Leu Trp Asp Thr Ala Gly Gln Glu Asp
65                  70                  75                  80

Tyr Asn Arg Leu Arg Pro Leu Ser Tyr Arg Gly Ala Asp Val Phe Ile
                85                  90                  95

Leu Ala Phe Ser Leu Ile Ser Arg Pro Ser Phe Glu Asn Ile Ala Lys
            100                 105                 110

Lys Trp Val Pro Glu Leu Arg His Tyr Ala Pro Asn Val Pro Ile Val
        115                 120                 125

Leu Val Gly Thr Lys Leu Asp Leu Arg Glu Asp Lys Lys Phe Pro Met
    130                 135                 140

Asn Tyr Pro Gly Ala Cys Thr Ile Ser Thr Glu Gln Gly Gln Glu Leu
145                 150                 155                 160

Arg Lys Glu Ile Gly Ala Leu Ala Tyr Ile Glu Cys Ser Ser Lys Thr
                165                 170                 175

Gln Gln Asn Val Lys Ala Val Phe Asp Ala Ala Ile Lys Val Val Leu
            180                 185                 190

Gln Pro Pro Thr Lys Ile Lys Lys Gln Lys Arg Arg Phe Arg Phe Cys
        195                 200                 205

His Ala Leu
    210

<210>  35

<211>  588

<212>  DNA

<213>  Brassica napus

<220>

<221>  CDS

<222>  (1)..(588)

<223>

<400>  35
atg agc gct tcg agg ttc ata aag tgt gtc acc gtc ggc gat ggt gcc      48
Met Ser Ala Ser Arg Phe Ile Lys Cys Val Thr Val Gly Asp Gly Ala
1               5                   10                  15

gtc gga aaa acc tgt atg ctg atc tct tac acg agc aac acc ttc cct      96
Val Gly Lys Thr Cys Met Leu Ile Ser Tyr Thr Ser Asn Thr Phe Pro
            20                  25                  30

acg gac tat gta cca act gtt ttc gat aac ttc agt gct aat gtg gtt      144
Thr Asp Tyr Val Pro Thr Val Phe Asp Asn Phe Ser Ala Asn Val Val
        35                  40                  45

gtt gat ggg aac act gtg aat ctt ggc ttg tgg gat aca gct ggt caa      192
Val Asp Gly Asn Thr Val Asn Leu Gly Leu Trp Asp Thr Ala Gly Gln
    50                  55                  60

gaa gac tat aac agg tta aga cca ttg agt tac cgt ggt gcg gat gtc      240
Glu Asp Tyr Asn Arg Leu Arg Pro Leu Ser Tyr Arg Gly Ala Asp Val
65                  70                  75                  80

ttc att ctt gct ttc tct ctt atc agc aaa gct agc tac gag aat ata      288
Phe Ile Leu Ala Phe Ser Leu Ile Ser Lys Ala Ser Tyr Glu Asn Ile
                85                  90                  95

gct aag aag tgg att cct gag ctc agg cac tat gcc cct ggt gtt cct      336
Ala Lys Lys Trp Ile Pro Glu Leu Arg His Tyr Ala Pro Gly Val Pro
            100                 105                 110

att atc ctc gtt gga aca aaa ctc gat ctt cga gat gac aag cag ttc      384
Ile Ile Leu Val Gly Thr Lys Leu Asp Leu Arg Asp Asp Lys Gln Phe
        115                 120                 125

ttc ata gac cac cct ggt gca gtg ccg att agt act aac cag gga gag      432
Phe Ile Asp His Pro Gly Ala Val Pro Ile Ser Thr Asn Gln Gly Glu
    130                 135                 140

gaa cta aag aaa ctg ata ggg tct ccg gct tac att gaa tgc agt tca      480
Glu Leu Lys Lys Leu Ile Gly Ser Pro Ala Tyr Ile Glu Cys Ser Ser
145                 150                 155                 160

aag acg cag cag aac gtg aag gca gtc ttt gac gca gcc ata aaa gta      528
Lys Thr Gln Gln Asn Val Lys Ala Val Phe Asp Ala Ala Ile Lys Val
                165                 170                 175

gtg ctt cag cca cca aag caa aag aag aag aaa aag aag aat ggt tgt      576
Val Leu Gln Pro Pro Lys Gln Lys Lys Lys Lys Lys Lys Asn Gly Cys
            180                 185                 190

gtt ttc ttg tga                                                    588
Val Phe Leu
        195

<210>  36

<211>  195

<212>  PRT

<213>  Brassica napus

<400>  36

Met Ser Ala Ser Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Asn Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Ile
                85                  90                  95

Ala Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Ile Asp His Pro Gly Ala Val Pro Ile Ser Thr Asn Gln Gly Glu
    130                 135                 140

Glu Leu Lys Lys Leu Ile Gly Ser Pro Ala Tyr Ile Glu Cys Ser Ser
145                 150                 155                 160

Lys Thr Gln Gln Asn Val Lys Ala Val Phe Asp Ala Ala Ile Lys Val
                165                 170                 175

Val Leu Gln Pro Pro Lys Gln Lys Lys Lys Lys Lys Lys Asn Gly Cys
            180                 185                 190

Val Phe Leu
        195

<210>  37

<211>  594

<212>  DNA

<213>  Brassica napus

.

<220>

<221>  CDS

<222>  (1)..(594)

<223>

<400>  37
atg agc gca tcg agg ttc ata aag tgt gtg acc gtt ggt gac gga gct        48
Met Ser Ala Ser Arg Phe Ile Lys Cys Val Thr Val Gly Asp Gly Ala
1               5                   10                  15

gtg ggt aaa aca tgt ctc ctc att tct tac acc agc aac act ttc cct        96
Val Gly Lys Thr Cys Leu Leu Ile Ser Tyr Thr Ser Asn Thr Phe Pro
            20                  25                  30

acg gat tat gtt ccg act gtt ttt gat aac ttc agc gct aat gtg gtt       144
Thr Asp Tyr Val Pro Thr Val Phe Asp Asn Phe Ser Ala Asn Val Val
        35                  40                  45

gtt aac gga gcc act gtc aac ctt ggc ttg tgg gat acc gct ggg cag       192
Val Asn Gly Ala Thr Val Asn Leu Gly Leu Trp Asp Thr Ala Gly Gln
    50                  55                  60

gag gat tat aac agg tta aga cca ttg agt tac cgc ggt gct gat gtt       240
Glu Asp Tyr Asn Arg Leu Arg Pro Leu Ser Tyr Arg Gly Ala Asp Val
65                  70                  75                  80

ttc atc tta gcc ttc tcc ctc atc agt aag gct agt tat gag aat gtc       288
Phe Ile Leu Ala Phe Ser Leu Ile Ser Lys Ala Ser Tyr Glu Asn Val
                85                  90                  95

tcc aag aag tgg atc cct gag ctg act cac tat gcc cct ggt gtc cca       336
Ser Lys Lys Trp Ile Pro Glu Leu Thr His Tyr Ala Pro Gly Val Pro
            100                 105                 110

att gtt ctt gtt ggt acc aaa cta gat ctt agg gat gac aaa cag ttc       384
Ile Val Leu Val Gly Thr Lys Leu Asp Leu Arg Asp Asp Lys Gln Phe
        115                 120                 125

ttc gtt gac cac cct ggt gct gta cct att acc act gct cag gga gag       432
Phe Val Asp His Pro Gly Ala Val Pro Ile Thr Thr Ala Gln Gly Glu
    130                 135                 140

gaa ctg atg aag cta att gga gct cct tcg tac atc gag tgc agt tca       480
Glu Leu Met Lys Leu Ile Gly Ala Pro Ser Tyr Ile Glu Cys Ser Ser
145                 150                 155                 160

aaa tca cag gag aac gtg aag ggg gtg ttt gat gca gcg att aga gtg       528
Lys Ser Gln Glu Asn Val Lys Gly Val Phe Asp Ala Ala Ile Arg Val
                165                 170                 175

gta ctt caa cct cca aag cag aag aaa aag aag ggc aaa gta caa aag       576

Val Leu Gln Pro Pro Lys Gln Lys Lys Lys Lys Gly Lys Val Gln Lys
        180                 185                 190

gcc tgc tcc att ttg taa                                           594
Ala Cys Ser Ile Leu
        195

<210>  38

<211>  197

<212>  PRT

<213>  Brassica napus

<400>  38

Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asn Gly Ala Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Val
                85                  90                  95

Ser Lys Lys Trp Ile Pro Glu Leu Thr His Tyr Ala Pro Gly Val Pro
            100                 105                 110

Ile Val Leu Val Gly Thr Lys Leu Asp Leu Arg Asp Asp Lys Gln Phe
        115                 120                 125

Phe Val Asp His Pro Gly Ala Val Pro Ile Thr Thr Ala Gln Gly Glu
    130                 135                 140

Glu Leu Met Lys Leu Ile Gly Ala Pro Ser Tyr Ile Glu Cys Ser Ser
145                 150                 155                 160

Lys Ser Gln Glu Asn Val Lys Gly Val Phe Asp Ala Ala Ile Arg Val
                165                 170                 175

Val Leu Gln Pro Pro Lys Gln Lys Lys Lys Lys Gly Lys Val Gln Lys
            180                 185                 190

Ala Cys Ser Ile Leu
        195

<210>  39

<211>  645

<212>  DNA

<213>  Oryza sativa

<220>

<221>  CDS

<222>  (1)..(645)

<223>

<400>  39
atg agc tcg gcg gcg gcg gcg acg agg ttc atc aag tgc gtc acc gtg      48
Met Ser Ser Ala Ala Ala Ala Thr Arg Phe Ile Lys Cys Val Thr Val
1               5                   10                  15

ggg gac ggc gcg gtg ggg aag acg tgc atg ctc atc tgc tac acc tgc      96
Gly Asp Gly Ala Val Gly Lys Thr Cys Met Leu Ile Cys Tyr Thr Cys
            20                  25                  30

aac aag ttc ccc acc gat tac atc ccc acc gtg ttc gac aac ttc agc      144
Asn Lys Phe Pro Thr Asp Tyr Ile Pro Thr Val Phe Asp Asn Phe Ser
        35                  40                  45

gcc aat gtc tcc gtg gac ggg agc gtc gtc aac ctc ggc ctc tgg gac      192
Ala Asn Val Ser Val Asp Gly Ser Val Val Asn Leu Gly Leu Trp Asp
    50                  55                  60

act gca ggt cag gag gat tac agc agg ttg agg cct ctg agc tac agg      240
Thr Ala Gly Gln Glu Asp Tyr Ser Arg Leu Arg Pro Leu Ser Tyr Arg
65                  70                  75                  80

gga gcc gat gtg ttc atc ctg tcc ttc tcc ctg ata agc agg gcg agc      288
Gly Ala Asp Val Phe Ile Leu Ser Phe Ser Leu Ile Ser Arg Ala Ser
                85                  90                  95

tat gag aat gtt cag aag aag tgg atg cca gag ctt cgc cgg ttt gcg      336
Tyr Glu Asn Val Gln Lys Lys Trp Met Pro Glu Leu Arg Arg Phe Ala
            100                 105                 110

cct ggt gtt cct gta gtt ctt gtt gga acc aag ttg gat ctc cgt gaa      384
Pro Gly Val Pro Val Val Leu Val Gly Thr Lys Leu Asp Leu Arg Glu
        115                 120                 125

gat agg gcc tat ctt gct gat cat cca gct tct tcc ata ata aca acg      432
Asp Arg Ala Tyr Leu Ala Asp His Pro Ala Ser Ser Ile Ile Thr Thr
    130                 135                 140

gag cag gga gaa gaa ctg agg aag cta ata gga gcg gtc gcc tac atc      480
Glu Gln Gly Glu Glu Leu Arg Lys Leu Ile Gly Ala Val Ala Tyr Ile
145                 150                 155                 160

gaa tgc agc tcc aag aca cag aga aac att aaa gct gtt ttc gac act      528
Glu Cys Ser Ser Lys Thr Gln Arg Asn Ile Lys Ala Val Phe Asp Thr
                165                 170                 175

gcc atc aaa gtg gtg ctt caa cct cca aga cat aag gat gta acc aga     576
Ala Ile Lys Val Val Leu Gln Pro Pro Arg His Lys Asp Val Thr Arg
            180                 185                 190

aag aaa ctc caa tca agc tcc aat cgg cca gta agg cgg tac ttt tgc     624
Lys Lys Leu Gln Ser Ser Ser Asn Arg Pro Val Arg Arg Tyr Phe Cys
        195                 200                 205

gga agc gct tgt ttc gcg tag                                         645
Gly Ser Ala Cys Phe Ala
    210

<210>  40

<211>  214

<212>  PRT

<213>  Oryza sativa

<400>  40

Met Ser Ser Ala Ala Ala Ala Thr Arg Phe Ile Lys Cys Val Thr Val
1               5                   10                  15

Gly Asp Gly Ala Val Gly Lys Thr Cys Met Leu Ile Cys Tyr Thr Cys
            20                  25                  30

Asn Lys Phe Pro Thr Asp Tyr Ile Pro Thr Val Phe Asp Asn Phe Ser
        35                  40                  45

Ala Asn Val Ser Val Asp Gly Ser Val Val Asn Leu Gly Leu Trp Asp
    50                  55                  60

Thr Ala Gly Gln Glu Asp Tyr Ser Arg Leu Arg Pro Leu Ser Tyr Arg
65                  70                  75                  80

Gly Ala Asp Val Phe Ile Leu Ser Phe Ser Leu Ile Ser Arg Ala Ser
                85                  90                  95

Tyr Glu Asn Val Gln Lys Lys Trp Met Pro Glu Leu Arg Arg Phe Ala
            100                 105                 110

Pro Gly Val Pro Val Val Leu Val Gly Thr Lys Leu Asp Leu Arg Glu
        115                 120                 125

Asp Arg Ala Tyr Leu Ala Asp His Pro Ala Ser Ser Ile Ile Thr Thr
    130                 135                 140

Glu Gln Gly Glu Glu Leu Arg Lys Leu Ile Gly Ala Val Ala Tyr Ile
145                 150                 155                 160

Glu Cys Ser Ser Lys Thr Gln Arg Asn Ile Lys Ala Val Phe Asp Thr
                165                 170                 175

Ala Ile Lys Val Val Leu Gln Pro Pro Arg His Lys Asp Val Thr Arg
            180                 185                 190

Lys Lys Leu Gln Ser Ser Ser Asn Arg Pro Val Arg Arg Tyr Phe Cys
        195                 200                 205

Gly Ser Ala Cys Phe Ala
    210

<210>  41

<211>  645

<212>  DNA

<213>  Oryza sativa

<220>

<221>  CDS

<222>  (1)..(645)

<223>

<400>  41
atg agc ggc gcc acc aag ttc atc aag tgc gtc acc gtc ggc gac ggc       48
Met Ser Gly Ala Thr Lys Phe Ile Lys Cys Val Thr Val Gly Asp Gly
1               5                   10                  15

gcc gtc ggc aag acc tgc atg ctc atc tgc tac atc agc aac aag ttc       96
Ala Val Gly Lys Thr Cys Met Leu Ile Cys Tyr Ile Ser Asn Lys Phe
            20                  25                  30

ccc acc gat tac atc ccc acc gtg ttc gac aac ttc agt gct aat gtt      144
Pro Thr Asp Tyr Ile Pro Thr Val Phe Asp Asn Phe Ser Ala Asn Val
        35                  40                  45

tca gtg gat ggg aac atc gtc aac ttg gga tta tgg gac act gct gga      192
Ser Val Asp Gly Asn Ile Val Asn Leu Gly Leu Trp Asp Thr Ala Gly
    50                  55                  60

caa gag gat tac agc agg ctg agg cca ctg agc tac agg gga gcg gat      240
Gln Glu Asp Tyr Ser Arg Leu Arg Pro Leu Ser Tyr Arg Gly Ala Asp
65                  70                  75                  80

ata ttt gtg ctg gca ttc tca ctg atc agc aga gca agc tat gag aat      288
Ile Phe Val Leu Ala Phe Ser Leu Ile Ser Arg Ala Ser Tyr Glu Asn
                85                  90                  95

gtt ctc aag aag tgg atg ccg gag ctt cgc cgg ttc gca ccg aat gtt      336
Val Leu Lys Lys Trp Met Pro Glu Leu Arg Arg Phe Ala Pro Asn Val
            100                 105                 110

cca att gtt ctt gtt ggg acc aag tta gat cta cgt gac cac aga tct      384
Pro Ile Val Leu Val Gly Thr Lys Leu Asp Leu Arg Asp His Arg Ser
        115                 120                 125

tac ctt gcg gac cat cct gct gct tcc gca att acg act gca cag ggt      432

Tyr Leu Ala Asp His Pro Ala Ala Ser Ala Ile Thr Thr Ala Gln Gly
    130                 135                 140

gaa gaa ctt aga aag cag ata ggc gcc gcg gct tac atc gaa tgc agt 480
Glu Glu Leu Arg Lys Gln Ile Gly Ala Ala Ala Tyr Ile Glu Cys Ser
145                 150                 155                 160

tcg aaa aca caa cag aac atc aag gcc gtg ttt gat act gcc atc aag 528
Ser Lys Thr Gln Gln Asn Ile Lys Ala Val Phe Asp Thr Ala Ile Lys
                165                 170                 175

gtg gtc ctt cag cct cct cgg aga agg ggg gag acg acg atg gca agg 576
Val Val Leu Gln Pro Pro Arg Arg Arg Gly Glu Thr Thr Met Ala Arg
            180                 185                 190

aag aag aca agg cga agc acc ggc tgc tcg tta aag aac ttg atg tgt 624
Lys Lys Thr Arg Arg Ser Thr Gly Cys Ser Leu Lys Asn Leu Met Cys
        195                 200                 205

ggc agt gca tgt gtt gtt tag 645
Gly Ser Ala Cys Val Val
    210

<210> 42

<211> 214

<212> PRT

<213> Oryza sativa

<400> 42

Met Ser Gly Ala Thr Lys Phe Ile Lys Cys Val Thr Val Gly Asp Gly
1               5                   10                  15

Ala Val Gly Lys Thr Cys Met Leu Ile Cys Tyr Ile Ser Asn Lys Phe
            20                  25                  30

Pro Thr Asp Tyr Ile Pro Thr Val Phe Asp Asn Phe Ser Ala Asn Val
        35                  40                  45

Ser Val Asp Gly Asn Ile Val Asn Leu Gly Leu Trp Asp Thr Ala Gly
    50                  55                  60

Gln Glu Asp Tyr Ser Arg Leu Arg Pro Leu Ser Tyr Arg Gly Ala Asp
65                  70                  75                  80

Ile Phe Val Leu Ala Phe Ser Leu Ile Ser Arg Ala Ser Tyr Glu Asn
                85                  90                  95

Val Leu Lys Lys Trp Met Pro Glu Leu Arg Arg Phe Ala Pro Asn Val
            100                 105                 110

Pro Ile Val Leu Val Gly Thr Lys Leu Asp Leu Arg Asp His Arg Ser
        115                 120                 125

Tyr Leu Ala Asp His Pro Ala Ala Ser Ala Ile Thr Thr Ala Gln Gly
    130                 135                 140

Glu Glu Leu Arg Lys Gln Ile Gly Ala Ala Ala Tyr Ile Glu Cys Ser
145                 150                 155                 160

Ser Lys Thr Gln Gln Asn Ile Lys Ala Val Phe Asp Thr Ala Ile Lys
                165                 170                 175

Val Val Leu Gln Pro Pro Arg Arg Arg Gly Glu Thr Thr Met Ala Arg
            180                 185                 190

Lys Lys Thr Arg Arg Ser Thr Gly Cys Ser Leu Lys Asn Leu Met Cys
        195                 200                 205

Gly Ser Ala Cys Val Val
    210

<210> 43

<211> 639

<212> DNA

<213> Oryza sativa

<220>

<221> CDS

<222> (1)..(639)

<223>

<400> 43
atg ggc tcg aag ccg ccg ccg ccg ccg cag ccc agc gtc tcc ttc aag 48
Met Gly Ser Lys Pro Pro Pro Pro Pro Gln Pro Ser Val Ser Phe Lys
1               5                   10                  15

ctc gtc ctc ctc ggc gac ggg aga gta ggc aaa aca tct ctt gtg ctg 96
Leu Val Leu Leu Gly Asp Gly Arg Val Gly Lys Thr Ser Leu Val Leu
            20                  25                  30

cgg tat gtg aat gat gtc ttc tca gac aaa cag gaa gca act gtt caa 144
Arg Tyr Val Asn Asp Val Phe Ser Asp Lys Gln Glu Ala Thr Val Gln
        35                  40                  45

gct tca tat ttg aca aag cgc ctt gtt gtt gaa ggt gtg cct att acg 192
Ala Ser Tyr Leu Thr Lys Arg Leu Val Val Glu Gly Val Pro Ile Thr
    50                  55                  60

ctc tct atc tgg gat aca gct gga caa gag aag ttc cat gca cta ggc 240
Leu Ser Ile Trp Asp Thr Ala Gly Gln Glu Lys Phe His Ala Leu Gly
65                  70                  75                  80

cct ata tac tat cgt gat gca gac gct gct ctt tta gta tat gac atc 288
Pro Ile Tyr Tyr Arg Asp Ala Asp Ala Ala Leu Leu Val Tyr Asp Ile
                85                  90                  95

aca gac aat gat act ttt ctt cgt gtc aca aag tgg gtg aaa gag ctt 336
Thr Asp Asn Asp Thr Phe Leu Arg Val Thr Lys Trp Val Lys Glu Leu
            100                 105                 110

aag caa atg gca aat aaa gat att gtt atg gcc att gca gca aat aaa 384
Lys Gln Met Ala Asn Lys Asp Ile Val Met Ala Ile Ala Ala Asn Lys
        115                 120                 125

agt gac ctg gtt aga tca aaa cac ata gac act aat gaa gca gca agc 432
Ser Asp Leu Val Arg Ser Lys His Ile Asp Thr Asn Glu Ala Ala Ser
    130                 135                 140

tat gca gaa agt att ggg gca act ctc ttt gtt act tct gcc aaa gct 480
Tyr Ala Glu Ser Ile Gly Ala Thr Leu Phe Val Thr Ser Ala Lys Ala
145                 150                 155                 160

ggt act ggg att gat gat atc ttc agt gac ata gcc aag aga tta tta 528
Gly Thr Gly Ile Asp Asp Ile Phe Ser Asp Ile Ala Lys Arg Leu Leu
                165                 170                 175

gag aga aga aaa aac agc tcc gat ggc ttg tca ctg gct cat cca aag 576
Glu Arg Arg Lys Asn Ser Ser Asp Gly Leu Ser Leu Ala His Pro Lys
            180                 185                 190

aaa ggc att tta atc gtc gac gat gaa cct gag aaa gaa cct cca cca 624
Lys Gly Ile Leu Ile Val Asp Asp Glu Pro Glu Lys Glu Pro Pro Pro
        195                 200                 205

aag tgc tgc tca tag 639
Lys Cys Cys Ser
    210

<210> 44

<211> 212

<212> PRT

<213> Oryza sativa

<400> 44

Met Gly Ser Lys Pro Pro Pro Pro Pro Gln Pro Ser Val Ser Phe Lys
1               5                   10                  15

Leu Val Leu Leu Gly Asp Gly Arg Val Gly Lys Thr Ser Leu Val Leu
            20                  25                  30

Arg Tyr Val Asn Asp Val Phe Ser Asp Lys Gln Glu Ala Thr Val Gln
        35                  40                  45

Ala Ser Tyr Leu Thr Lys Arg Leu Val Val Glu Gly Val Pro Ile Thr
    50                  55                  60

Leu Ser Ile Trp Asp Thr Ala Gly Gln Glu Lys Phe His Ala Leu Gly
65                  70                  75                  80

Pro Ile Tyr Tyr Arg Asp Ala Asp Ala Ala Leu Leu Val Tyr Asp Ile
                85                  90                  95

Thr Asp Asn Asp Thr Phe Leu Arg Val Thr Lys Trp Val Lys Glu Leu
            100                 105                 110

Lys Gln Met Ala Asn Lys Asp Ile Val Met Ala Ile Ala Ala Asn Lys
        115                 120                 125

Ser Asp Leu Val Arg Ser Lys His Ile Asp Thr Asn Glu Ala Ala Ser
    130                 135                 140

Tyr Ala Glu Ser Ile Gly Ala Thr Leu Phe Val Thr Ser Ala Lys Ala
145                 150                 155                 160

Gly Thr Gly Ile Asp Asp Ile Phe Ser Asp Ile Ala Lys Arg Leu Leu
                165                 170                 175

Glu Arg Arg Lys Asn Ser Ser Asp Gly Leu Ser Leu Ala His Pro Lys
            180                 185                 190

Lys Gly Ile Leu Ile Val Asp Asp Glu Pro Glu Lys Glu Pro Pro Pro
        195                 200                 205

Lys Cys Cys Ser
    210

<210>  45

<211>  806

<212>  DNA

<213>  Brassica napus

<220>

<221>  CDS

<222>  (6)..(596)

<223>

<400>  45
ggaaa atg agc gca tct cgg ttc ata aag tgc gtg acg gtc ggt gac gga      50
      Met Ser Ala Ser Arg Phe Ile Lys Cys Val Thr Val Gly Asp Gly
      1               5                   10                  15

gca gtg ggc aaa aca tgt ctc ctc atc tct tac acc agc aac act ttc      98
Ala Val Gly Lys Thr Cys Leu Leu Ile Ser Tyr Thr Ser Asn Thr Phe
                20                  25                  30

cct acg gat tat gtg ccc act gtt ttc gat aac ttt agc gct aac gtt      146
Pro Thr Asp Tyr Val Pro Thr Val Phe Asp Asn Phe Ser Ala Asn Val
            35                  40                  45

gtt gtt aac gga gcc act gtc aac tta gga ctc tgg gat aca gca ggg      194

Val Val Asn Gly Ala Thr Val Asn Leu Gly Leu Trp Asp Thr Ala Gly
        50                  55                  60

cag gag gat tat aac aga ttg aga ccc ttg agt tac cgc ggt gct gac        242
Gln Glu Asp Tyr Asn Arg Leu Arg Pro Leu Ser Tyr Arg Gly Ala Asp
    65                  70                  75

gtt ttc atc tta gcc ttc tct ctt atc agt aag gct agt tat gag aat        290
Val Phe Ile Leu Ala Phe Ser Leu Ile Ser Lys Ala Ser Tyr Glu Asn
80                  85                  90                  95

gtc tcc aag aag tgg atc cct gag ctg acc cac tat gcc cct ggt gtc        338
Val Ser Lys Lys Trp Ile Pro Glu Leu Thr His Tyr Ala Pro Gly Val
                100                 105                 110

cct att gtt ctt gtt gga acc aaa cta gat ctt agg gat gac aaa cag        386
Pro Ile Val Leu Val Gly Thr Lys Leu Asp Leu Arg Asp Asp Lys Gln
            115                 120                 125

ttc ttc gtt gac cac cct ggt gct gta cct att acc act gct cag ggc        434
Phe Phe Val Asp His Pro Gly Ala Val Pro Ile Thr Thr Ala Gln Gly
        130                 135                 140

gag gaa cta atg aag cta att gga gct cct tcg tac atc gag tgc agc        482
Glu Glu Leu Met Lys Leu Ile Gly Ala Pro Ser Tyr Ile Glu Cys Ser
    145                 150                 155

tca aaa tca cag gag aac gtg aag ggg gtg ttt gat gca gcg atc aga        530
Ser Lys Ser Gln Glu Asn Val Lys Gly Val Phe Asp Ala Ala Ile Arg
160                 165                 170                 175

gtg gta ctt caa cct cca aag cag aag aaa aag aag agc aaa gct caa        578
Val Val Leu Gln Pro Pro Lys Gln Lys Lys Lys Lys Ser Lys Ala Gln
                180                 185                 190

aag gcc tgc tcc att ttg tgatttctct acgctcatct ctcttccact        626
Lys Ala Cys Ser Ile Leu
            195

ctctagtgaa ggcttaagaa gaagaaacac tttagccttt aagatttggt tcagagttcg        686

ttgtgataag cctcgcttaa tccttagaaa cgattacttc tggttttact gataaagagc        746

ttccagcttt atagctgaga gctaatattg cactaccact ataaaaaaaa aaaaaaaaaa        806

<210>  46

<211>  197

<212>  PRT

<213>  Brassica napus

<400>  46

Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asn Gly Ala Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Val
                85                  90                  95

Ser Lys Lys Trp Ile Pro Glu Leu Thr His Tyr Ala Pro Gly Val Pro
            100                 105                 110

Ile Val Leu Val Gly Thr Lys Leu Asp Leu Arg Asp Asp Lys Gln Phe
        115                 120                 125

Phe Val Asp His Pro Gly Ala Val Pro Ile Thr Thr Ala Gln Gly Glu
    130                 135                 140

Glu Leu Met Lys Leu Ile Gly Ala Pro Ser Tyr Ile Glu Cys Ser Ser
145                 150                 155                 160

Lys Ser Gln Glu Asn Val Lys Gly Val Phe Asp Ala Ala Ile Arg Val
                165                 170                 175

Val Leu Gln Pro Pro Lys Gln Lys Lys Lys Lys Ser Lys Ala Gln Lys
            180                 185                 190

Ala Cys Ser Ile Leu
        195

<210>  47

<211>  32

<212>  PRT

<213>  consensus

<220>

<221>  MISC_FEATURE

<222>  (1)..(32)

<223>  Xaa in position 15 is Leu, Ile or Met; Xaa in position 16 is Leu
       or Val; Xaa in position 17 is Ile, Leu or Tyr; Xaa in position 18
        is Ser, Cys, Val or Arg; Xaa in position 20 is Thr, Ile or Val;
       Xaa in position 23 is Thr, Lys, Gln or Val

<400>  47

Lys Cys Val Thr Val Gly Asp Gly Ala Val Gly Lys Thr Cys Xaa Xaa

1                   5                        10                       15

Xaa Xaa Tyr Xaa Ser Asn Xaa Phe Pro Thr Asp Tyr Val Pro Thr Val
            20                       25                       30

<210>  48

<211>  32

<212>  PRT

<213>  consensus

<220>

<221>  MISC_FEATURE

<222>  (1)..(32)

<223>  Xaa any natural amino acid

<400>  48

Lys Xaa Val Xaa Xaa Gly Asp Xaa Xaa Xaa Gly Lys Xaa Xaa Xaa Xaa
1                   5                        10                       15

Xaa Xaa Xaa Xaa Xaa Xaa Xaa Phe Xaa Xaa Xaa Xaa Xaa Xaa Thr Val
            20                       25                       30

<210>  49

<211>  16

<212>  PRT

<213>  consensus

<220>

<221>  MISC_FEATURE

<222>  (1)..(16)

<223>

<400>  49

Leu Trp Asp Thr Ala Gly Gln Glu Asp Tyr Asn Arg Leu Arg Pro Leu
1                   5                        10                       15

<210>  50

<211>  16

<212>  PRT

<213>  consensus

<220>

<221>  MISC_FEATURE

<222>  (1)..(16)

<223>  Xaa in position 1 is Leu or Ile; Xaa in position 9 is Asp, Lys or
       Glu; Xaa in position 10 is Tyr or Phe; Xaa in position 11 is Asn
      , Ser, Ala, His or Glu; Xaa in position 12 is Arg or Ala; Xaa in
      position 14 is Arg, Ala or Gly; Xaa in position 16 is Leu, Ile or
       Phe

<400>  50

Xaa Trp Asp Thr Ala Gly Gln Glu Xaa Xaa Xaa Xaa Leu Xaa Pro Xaa
1               5                   10                  15

<210>  51

<211>  7

<212>  PRT

<213>  consensus

<400>  51

Gly Thr Lys Leu Asp Leu Arg
1               5

<210>  52

<211>  7

<212>  PRT

<213>  consensus

<220>

<221>  MISC_FEATURE

<222>  (1)..(7)

<223>  Xaa in postion 1 is Gly or Ala, Xaa in postion 2 is Thr, Asn or L
      eu, Xaa in postion 4 is Leu, Ala, Ser or Lys, Xaa in postion 7 is
       Arg, His or Val

<400>  52

Xaa Xaa Lys Xaa Asp Leu Xaa
1               5

<210> 53

<211> 24

<212> DNA

<213> primer

<400> 53
atgtctgaaa aggccgttag aagg                                              24

<210> 54

<211> 26

<212> DNA

<213> primer

<400> 54
ttataaaatt atgcaacagt tagccc                                            26

<210> 55
<211> 579
<212> DNA
<213> Saccharomyces cerevisiae

<220>
<221> CDS
<222> (1)..(579)

<400> 55
atg tct gaa aag gcc gtt aga agg aaa ctt gtt att att ggt gat ggt      48
Met Ser Glu Lys Ala Val Arg Arg Lys Leu Val Ile Ile Gly Asp Gly
1               5                   10                  15
gct tgt ggc aag acc tct tta cta tat gta ttt aca tta gga aaa ttc      96
Ala Cys Gly Lys Thr Ser Leu Leu Tyr Val Phe Thr Leu Gly Lys Phe
            20                  25                  30
cct gaa caa tat cat ccg aca gtg ttc gag aat tat gtc act gat tgc      144
Pro Glu Gln Tyr His Pro Thr Val Phe Glu Asn Tyr Val Thr Asp Cys
        35                  40                  45
aga gtt gac gga ata aaa gtg tcc tta acg ctc tgg gat aca gcg gga      192
Arg Val Asp Gly Ile Lys Val Ser Leu Thr Leu Trp Asp Thr Ala Gly
    50                  55                  60
caa gag gaa tat gaa cgt tta cgt cca ttc tca tat tca aaa gca gat      240
Gln Glu Glu Tyr Glu Arg Leu Arg Pro Phe Ser Tyr Ser Lys Ala Asp
65                  70                  75                  80
ata ata tta att ggg ttt gct gta gac aat ttt gaa tca cta att aac      288
Ile Ile Leu Ile Gly Phe Ala Val Asp Asn Phe Glu Ser Leu Ile Asn
                85                  90                  95
gca agg acg aaa tgg gcg gat gag gca tta cga tat tgt cct gac gca      336
Ala Arg Thr Lys Trp Ala Asp Glu Ala Leu Arg Tyr Cys Pro Asp Ala
            100                 105                 110
cca atc gtt ctt gta ggc ttg aaa aaa gat ttg agg caa gaa gcc cat      384
Pro Ile Val Leu Val Gly Leu Lys Lys Asp Leu Arg Gln Glu Ala His
        115                 120                 125
ttt aaa gag aat gct acc gat gaa atg gtt ccc att gaa gat gca aaa      432
Phe Lys Glu Asn Ala Thr Asp Glu Met Val Pro Ile Glu Asp Ala Lys
    130                 135                 140
caa gtt gca agg gcc att ggg gcc aag aaa tac atg gaa tgt agt gca      480

Gln Val Ala Arg Ala Ile Gly Ala Lys Lys Tyr Met Glu Cys Ser Ala
145                 150                 155                 160
ctg act ggt gag ggt gtg gat gat gtc ttt gaa gta gct aca aga acc     528
Leu Thr Gly Glu Gly Val Asp Asp Val Phe Glu Val Ala Thr Arg Thr
                165                 170                 175
agt ttg ctt atg aag aag gaa cca ggg gct aac tgt tgc ata att tta     576
Ser Leu Leu Met Lys Lys Glu Pro Gly Ala Asn Cys Cys Ile Ile Leu
            180                 185                 190
taa                                                                 579

<210> 56
<211> 192
<212> PRT
<213> Saccharomyces cerevisiae

<400> 56
Met Ser Glu Lys Ala Val Arg Arg Lys Leu Val Ile Ile Gly Asp Gly
1               5                   10                  15
Ala Cys Gly Lys Thr Ser Leu Leu Tyr Val Phe Thr Leu Gly Lys Phe
            20                  25                  30
Pro Glu Gln Tyr His Pro Thr Val Phe Glu Asn Tyr Val Thr Asp Cys
        35                  40                  45
Arg Val Asp Gly Ile Lys Val Ser Leu Thr Leu Trp Asp Thr Ala Gly
    50                  55                  60
Gln Glu Glu Tyr Glu Arg Leu Arg Pro Phe Ser Tyr Ser Lys Ala Asp
65                  70                  75                  80
Ile Ile Leu Ile Gly Phe Ala Val Asp Asn Phe Glu Ser Leu Ile Asn
                85                  90                  95
Ala Arg Thr Lys Trp Ala Asp Glu Ala Leu Arg Tyr Cys Pro Asp Ala
            100                 105                 110
Pro Ile Val Leu Val Gly Leu Lys Lys Asp Leu Arg Gln Glu Ala His
        115                 120                 125
Phe Lys Glu Asn Ala Thr Asp Glu Met Val Pro Ile Glu Asp Ala Lys
    130                 135                 140
Gln Val Ala Arg Ala Ile Gly Ala Lys Lys Tyr Met Glu Cys Ser Ala
145                 150                 155                 160
Leu Thr Gly Glu Gly Val Asp Asp Val Phe Glu Val Ala Thr Arg Thr
                165                 170                 175
Ser Leu Leu Met Lys Lys Glu Pro Gly Ala Asn Cys Cys Ile Ile Leu
            180                 185                 190

<210> 57
<211> 803
<212> DNA
<213> Nicotiana tabacum

<220>
<221> CDS
<222> (49)..(642)

<400> 57
aaaaaaaaa aaaaaaaaa aaaaaaaaa aaaagaaaa agaaaaa atg agt gct     57
                                                Met Ser Ala
                                                1
tca agg ttt atc aag tgt gtt acg gtt ggt gat ggt gct gtt ggg aaa     105
Ser Arg Phe Ile Lys Cys Val Thr Val Gly Asp Gly Ala Val Gly Lys
    5                   10                  15
act tgt ctt ttg att tca tac acc agc aat act ttc cct acg gat tat     153
Thr Cys Leu Leu Ile Ser Tyr Thr Ser Asn Thr Phe Pro Thr Asp Tyr
20                  25                  30                  35
gtg ccc act gtg ttt gac aat ttc agt gca aat gtt gtt gtc aat ggg     201
Val Pro Thr Val Phe Asp Asn Phe Ser Ala Asn Val Val Val Asn Gly
                40                  45                  50
agc act gtc aac cta ggg ttg tgg gat act gct gga cag gag gat tac     249
Ser Thr Val Asn Leu Gly Leu Trp Asp Thr Ala Gly Gln Glu Asp Tyr
            55                  60                  65

aat agg tta aga cct ctg agt tac cgt gga gcc gat gtt ttc att ttg 297
Asn Arg Leu Arg Pro Leu Ser Tyr Arg Gly Ala Asp Val Phe Ile Leu
        70                  75                  80
gca ttc tct ctc att agt aaa gcc agc tat gag aat gtt tcc aag aag 345
Ala Phe Ser Leu Ile Ser Lys Ala Ser Tyr Glu Asn Val Ser Lys Lys
    85                  90                  95
tgg att cct gag ttg aag cac tat gct cct ggt gtc cca ata gtt ctt 393
Trp Ile Pro Glu Leu Lys His Tyr Ala Pro Gly Val Pro Ile Val Leu
100                 105                 110                 115
gtt gga aca aag ctt gat ctt cgg gat gat aag caa ttc ttc ata gac 441
Val Gly Thr Lys Leu Asp Leu Arg Asp Asp Lys Gln Phe Phe Ile Asp
                120                 125                 130
cat ccc ggt gct gtg cca att act act gct cag ggc gag gag cta agg 489
His Pro Gly Ala Val Pro Ile Thr Thr Ala Gln Gly Glu Glu Leu Arg
            135                 140                 145
aaa act att ggt gca cct gct tac att gaa tgt agt tca aaa aca caa 537
Lys Thr Ile Gly Ala Pro Ala Tyr Ile Glu Cys Ser Ser Lys Thr Gln
        150                 155                 160
cag aat gtg aaa gca gtc ttt gat gct gcc att aag gtc gtc ctc cag 585
Gln Asn Val Lys Ala Val Phe Asp Ala Ala Ile Lys Val Val Leu Gln
    165                 170                 175
ccg cct aag acg aag aaa aag aag ggg aaa tct caa aaa tcc tgc tcg 633
Pro Pro Lys Thr Lys Lys Lys Lys Gly Lys Ser Gln Lys Ser Cys Ser
180                 185                 190                 195
ata ttg tgatcaaact gtaggtccaa cgatagtgct ggtcacaatt ctcgttgtaa 689
Ile Leu

tccctcactc actcgtatcc ctttatcttc cttcgtctca gaggaagtac gaggtcatcg 749

tttgtcttat atttgaaact actaaccatc tcttggaaat atcaggtggc ttgg 803

<210> 58
<211> 197
<212> PRT
<213> Nicotiana tabacum

<400> 58
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Ile Val Leu Val Gly Thr Lys Leu Asp Leu Arg Asp Asp Lys Gln Phe
        115                 120                 125
Phe Ile Asp His Pro Gly Ala Val Pro Ile Thr Thr Ala Gln Gly Glu
    130                 135                 140
Glu Leu Arg Lys Thr Ile Gly Ala Pro Ala Tyr Ile Glu Cys Ser Ser
145                 150                 155                 160
Lys Thr Gln Gln Asn Val Lys Ala Val Phe Asp Ala Ala Ile Lys Val
                165                 170                 175
Val Leu Gln Pro Pro Lys Thr Lys Lys Lys Lys Gly Lys Ser Gln Lys
            180                 185                 190
Ser Cys Ser Ile Leu
        195

<210> 59
<211> 993
<212> DNA
<213> Nicotiana tabacum

<220>
<221> CDS
<222> (29)..(625)

<400> 59
ggcacgaggg agatttgggt tttgtaga atg agt gcg tct agg ttc ata aag 52
Met Ser Ala Ser Arg Phe Ile Lys
1               5
tgt gtc acc gtt ggc gac gga gct gtg ggt aaa act tgt ctt ctc att 100
Cys Val Thr Val Gly Asp Gly Ala Val Gly Lys Thr Cys Leu Leu Ile
    10                  15                  20
tcc tat acc agc aac aca ttt ccc act gat tac gtc cca act gta ttc 148
Ser Tyr Thr Ser Asn Thr Phe Pro Thr Asp Tyr Val Pro Thr Val Phe
25                  30                  35                  40
gac aat ttt agt gca aat gtg gtt gtc gat ggg agc act gtc aat ctg 196
Asp Asn Phe Ser Ala Asn Val Val Val Asp Gly Ser Thr Val Asn Leu
                45                  50                  55
ggg ctg tgg gat act gca ggt cag gag gat tac aat aga tta aga ccg 244
Gly Leu Trp Asp Thr Ala Gly Gln Glu Asp Tyr Asn Arg Leu Arg Pro
            60                  65                  70
ttg agc tac cgg ggg gca gat gta ttt ata ctg gca ttt tct ctc att 292
Leu Ser Tyr Arg Gly Ala Asp Val Phe Ile Leu Ala Phe Ser Leu Ile
        75                  80                  85
agc aaa gcg agc tat gaa aat gtc tcc aaa aag tgg att cct gaa ttg 340
Ser Lys Ala Ser Tyr Glu Asn Val Ser Lys Lys Trp Ile Pro Glu Leu
    90                  95                  100
agg cat tat gct cct gga gtt cca att att ctt gtt gga aca aag cta 388
Arg His Tyr Ala Pro Gly Val Pro Ile Ile Leu Val Gly Thr Lys Leu
105                 110                 115                 120
gat ctc cga gag gat aag caa ttc ttc ctg gac cat cca ggt gct gtt 436
Asp Leu Arg Glu Asp Lys Gln Phe Phe Leu Asp His Pro Gly Ala Val
                125                 130                 135
cca ctt acc aca gct cag ggt gaa gag ctg aga aag tcg att ggt gct 484
Pro Leu Thr Thr Ala Gln Gly Glu Glu Leu Arg Lys Ser Ile Gly Ala
            140                 145                 150
tcc gct tac att gaa tgt agt gca aaa aca caa cag aat gtg aag gct 532
Ser Ala Tyr Ile Glu Cys Ser Ala Lys Thr Gln Gln Asn Val Lys Ala
        155                 160                 165
gtt ttt gat gct gcc att aag gtg gtt cta caa cca ccc aaa caa aag 580
Val Phe Asp Ala Ala Ile Lys Val Val Leu Gln Pro Pro Lys Gln Lys
    170                 175                 180
aag aaa aag aag aga aag ggt caa aaa gcc tgc tct atc ttg tgattgctga 632
Lys Lys Lys Lys Arg Lys Gly Gln Lys Ala Cys Ser Ile Leu
185                 190                 195
aataatagac aagtgatgga gatgtagatt gttatcaatg tcttccaagt tcaaagaatg 692

cagtgtaagg ttcaacgttg gtagtcctga ctgactatga taggaaagca tgaatctgcc 752

ttgtccgtaa cattggaggc caagatgtat atttgtgatc cgcatatggt tggggataca 812

gatgtgcaaa attctctgtt tcgcgttgat tctgtgtaat atattatgta acacttgtgt 872

gatgattcct tgaatttgca tctactatgt gttgttaaaa tgtaaccgaa catactgatt 932

tagattgtcc aagaagttga atttgctatg tcataaaaaa aaaaaaaaaa aaaaaaaaaa 992

a 993

<210> 60
<211> 198
<212> PRT
<213> Nicotiana tabacum

<400> 60
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Arg His Tyr Ala Pro Gly Val Pro
            100                 105                 110
Ile Ile Leu Val Gly Thr Lys Leu Asp Leu Arg Glu Asp Lys Gln Phe
        115                 120                 125
Phe Leu Asp His Pro Gly Ala Val Pro Leu Thr Thr Ala Gln Gly Glu
    130                 135                 140
Glu Leu Arg Lys Ser Ile Gly Ala Ser Ala Tyr Ile Glu Cys Ser Ala
145                 150                 155                 160
Lys Thr Gln Gln Asn Val Lys Ala Val Phe Asp Ala Ala Ile Lys Val
                165                 170                 175
Val Leu Gln Pro Pro Lys Gln Lys Lys Lys Lys Arg Lys Gly Gln
            180                 185                 190
Lys Ala Cys Ser Ile Leu
        195

<210> 61
<211> 640
<212> DNA
<213> Gossypium hirsutum

<220>
<221> CDS
<222> (17)..(613)

<400> 61
cacgcgtggc gtaagt atg agt gct tcc agg ttc ata aaa tgc gtc acg gtc      52
                  Met Ser Ala Ser Arg Phe Ile Lys Cys Val Thr Val
                  1               5                   10
ggt gac ggt gcc gtc ggc aag act tgt ctg ctc att tcc tac act agc      100
Gly Asp Gly Ala Val Gly Lys Thr Cys Leu Leu Ile Ser Tyr Thr Ser
        15                  20                  25
aat act ttc cct acc gat tat gtg cca act gtc ttt gac aac ttt agt      148
Asn Thr Phe Pro Thr Asp Tyr Val Pro Thr Val Phe Asp Asn Phe Ser
    30                  35                  40
gct aat gtg gtt gtg gat ggg aac act gtt aat ctg gga ttg tgg gat      196
Ala Asn Val Val Val Asp Gly Asn Thr Val Asn Leu Gly Leu Trp Asp
45                  50                  55                  60
act gct gga cag gaa gat tac aat aga tta aga cca ttg agc tat cgt      244
Thr Ala Gly Gln Glu Asp Tyr Asn Arg Leu Arg Pro Leu Ser Tyr Arg
                65                  70                  75
gga gca gat gtc ttc ttg ctg gca ttt tct ctc att agc aaa gct agc      292
Gly Ala Asp Val Phe Leu Leu Ala Phe Ser Leu Ile Ser Lys Ala Ser
            80                  85                  90
tat gaa aat gtt gct aag aaa tgg att cca gaa ttg aga cat tat gca      340
Tyr Glu Asn Val Ala Lys Lys Trp Ile Pro Glu Leu Arg His Tyr Ala
        95                  100                 105

ccc ggt gtt cca att att ctt gtt ggg act aag ctt gat ctt cga gaa 388
Pro Gly Val Pro Ile Ile Leu Val Gly Thr Lys Leu Asp Leu Arg Glu
    110                 115                 120
gat aag cag ttc ttc ata gat cac cct ggt gcg gtg ccc att acc aca 436
Asp Lys Gln Phe Phe Ile Asp His Pro Gly Ala Val Pro Ile Thr Thr
125                 130                 135                 140
gca cag ggt gag gaa ttg aga aag cta att gga gcg cat ttt tac att 484
Ala Gln Gly Glu Glu Leu Arg Lys Leu Ile Gly Ala His Phe Tyr Ile
                145                 150                 155
gag tgt agt tca aaa aca caa cag aat gtg aaa gcg gtc ttt gat gcg 532
Glu Cys Ser Ser Lys Thr Gln Gln Asn Val Lys Ala Val Phe Asp Ala
            160                 165                 170
gcc atc aaa gta gtt ctc cag cct cca aag aag aag aag aaa aag aag 580
Ala Ile Lys Val Val Leu Gln Pro Pro Lys Lys Lys Lys Lys Lys Lys
        175                 180                 185
aga aag gca caa aaa gct tgc tca ata ttg tgatcatgca aagaagtgat 630
Arg Lys Ala Gln Lys Ala Cys Ser Ile Leu
    190                 195
attggtgcag 640

<210> 62
<211> 198
<212> PRT
<213> Gossypium hirsutum

<400> 62
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Asn Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Val
                85                  90                  95
Ala Lys Lys Trp Ile Pro Glu Leu Arg His Tyr Ala Pro Gly Val Pro
            100                 105                 110
Ile Ile Leu Val Gly Thr Lys Leu Asp Leu Arg Glu Asp Lys Gln Phe
        115                 120                 125
Phe Ile Asp His Pro Gly Ala Val Pro Ile Thr Thr Ala Gln Gly Glu
    130                 135                 140
Glu Leu Arg Lys Leu Ile Gly Ala His Phe Tyr Ile Glu Cys Ser Ser
145                 150                 155                 160
Lys Thr Gln Gln Asn Val Lys Ala Val Phe Asp Ala Ala Ile Lys Val
                165                 170                 175
Val Leu Gln Pro Pro Lys Lys Lys Lys Lys Lys Lys Arg Lys Ala Gln
            180                 185                 190
Lys Ala Cys Ser Ile Leu
        195

<210> 63
<211> 1025
<212> DNA
<213> Nicotiana tabacum

<220>
<221> CDS
<222> (103)..(696)

<400> 63
ttcttgtatc cgcccagctc ccccataccc atttcaatct ttttttttgt ttataatttt 60

tttctgggaa tcaggtcttc tcaagaagat ctgagaagaa aa atg agt gct tcg 114
Met Ser Ala Ser
1
agg ttt ata aaa tgt gtg acg gtg ggt gat ggt gcc gtt gga aaa acg 162
Arg Phe Ile Lys Cys Val Thr Val Gly Asp Gly Ala Val Gly Lys Thr
5                   10                  15                  20
tgt ctc ttg att tct tac acc agc aac acc ttt cct acg gat tat gtg 210
Cys Leu Leu Ile Ser Tyr Thr Ser Asn Thr Phe Pro Thr Asp Tyr Val
                25                  30                  35
cct act gtg ttc gat aat ttc agt gca aat gtg gtc gtc aat ggg agt 258
Pro Thr Val Phe Asp Asn Phe Ser Ala Asn Val Val Val Asn Gly Ser
            40                  45                  50
act gtc aac cta ggg ttg tgg gat aca gct gga cag gag gat tac aat 306
Thr Val Asn Leu Gly Leu Trp Asp Thr Ala Gly Gln Glu Asp Tyr Asn
        55                  60                  65
agg tta aga cct ctg agt tac cgt gga gct gat gtt ttc atc ttg gca 354
Arg Leu Arg Pro Leu Ser Tyr Arg Gly Ala Asp Val Phe Ile Leu Ala
    70                  75                  80
ttc tct ctc att agt aaa gcc agc tat gaa aat gtt tcc aag aag tgg 402
Phe Ser Leu Ile Ser Lys Ala Ser Tyr Glu Asn Val Ser Lys Lys Trp
85                  90                  95                  100
att cct gag ttg aag cat tat gct ccc ggt gtt cca ata gtg ctt gtt 450
Ile Pro Glu Leu Lys His Tyr Ala Pro Gly Val Pro Ile Val Leu Val
                105                 110                 115
gga aca aaa ctt gat ctt cgg gat gat aag cag ttc ttc ata gac cat 498
Gly Thr Lys Leu Asp Leu Arg Asp Asp Lys Gln Phe Phe Ile Asp His
            120                 125                 130
cct ggt gct gtg cca att act act gct cag ggc gag gag ctg agg aaa 546
Pro Gly Ala Val Pro Ile Thr Thr Ala Gln Gly Glu Glu Leu Arg Lys
        135                 140                 145
acg att ggg gcc cct gct tac att gaa tgt agt tca aaa aca caa cag 594
Thr Ile Gly Ala Pro Ala Tyr Ile Glu Cys Ser Ser Lys Thr Gln Gln
    150                 155                 160
aat gtg aaa gca gtc ttt gat gct gct att aag gtc gtg ctc cag cca 642
Asn Val Lys Ala Val Phe Asp Ala Ala Ile Lys Val Val Leu Gln Pro
165                 170                 175                 180
ccc aag cag aaa aaa aag aag ggg aag gcc caa aag gca tgt tcg att 690
Pro Lys Gln Lys Lys Lys Lys Gly Lys Ala Gln Lys Ala Cys Ser Ile
                185                 190                 195
ttg tgatcaacag taggtccaac aatattactg gtcacagttc tccttgtaat 743
Leu

ctctccctca tattcctttg tttttccttt gtctcggaga aagtaggagg tccatgtatg 803

tctctttaaa attacttgtg atctcttgaa ttgcaggtgg ctcggttttt atactgctgt 863

catctctagt tgttgagtca cagcaccttg ttgtaggcca ctgattgggc cctctctggt 923

ttcatttgct ttatgattaa tgactgaaag atccagttgg aaaaaaaaaa aaaaaaaaaa 983

aaaaaactcg gggggggccg ggcccaatcc cctatagggg tg 1025

<210> 64
<211> 197
<212> PRT
<213> Nicotiana tabacum

<400> 64
Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Ile Val Leu Val Gly Thr Lys Leu Asp Leu Arg Asp Asp Lys Gln Phe
        115                 120                 125
Phe Ile Asp His Pro Gly Ala Val Pro Ile Thr Thr Ala Gln Gly Glu
    130                 135                 140
Glu Leu Arg Lys Thr Ile Gly Ala Pro Ala Tyr Ile Glu Cys Ser Ser
145                 150                 155                 160
Lys Thr Gln Gln Asn Val Lys Ala Val Phe Asp Ala Ala Ile Lys Val
                165                 170                 175
Val Leu Gln Pro Pro Lys Gln Lys Lys Lys Lys Gly Lys Ala Gln Lys
            180                 185                 190
Ala Cys Ser Ile Leu
        195

<210> 65
<211> 1178
<212> DNA
<213> Medicago truncatula

<220>
<221> CDS
<222> (136)..(729)

<400> 65
cctatttcat cttcatcacc gattattctt gaacagttac caatttcaaa ctcaaacaca      60

caacaacatt gacacatgaa taattagggt ttatacctca aagaaacaca acaaacttga     120

ataattaggt tcgaa atg agt ggt tcc agg ttc atc aag tgt gtc aca gtt      171
                 Met Ser Gly Ser Arg Phe Ile Lys Cys Val Thr Val
                 1               5                   10
ggt gat ggt gcc gtt gga aag act tgt ttg ctt atc tcc tac acc agc      219
Gly Asp Gly Ala Val Gly Lys Thr Cys Leu Leu Ile Ser Tyr Thr Ser
        15                  20                  25
aac act ttc cct acg gac tat gtg ccg act gtc ttt gac aat ttc agt      267
Asn Thr Phe Pro Thr Asp Tyr Val Pro Thr Val Phe Asp Asn Phe Ser
    30                  35                  40
gca aat gta gtt gtg gat ggg agc act ata aat ctc ggg ttg tgg gat      315
Ala Asn Val Val Val Asp Gly Ser Thr Ile Asn Leu Gly Leu Trp Asp
45                  50                  55                  60
act gct ggc caa gaa gat tac aat aga tta aga ccc tta agc tat cgt      363
Thr Ala Gly Gln Glu Asp Tyr Asn Arg Leu Arg Pro Leu Ser Tyr Arg
                65                  70                  75
gga gca gat gtt ttc ctg ctt gct ttt tct ctc ata agc aag gct agc      411
Gly Ala Asp Val Phe Leu Leu Ala Phe Ser Leu Ile Ser Lys Ala Ser
            80                  85                  90
tat gaa aat att gcc aaa aaa tgg att cct gag ttg agg cat tat gct      459
Tyr Glu Asn Ile Ala Lys Lys Trp Ile Pro Glu Leu Arg His Tyr Ala
        95                  100                 105
cct ggt gtt cca att att ctc gtt gga aca aaa ctt gat ctt cgg gat      507
Pro Gly Val Pro Ile Ile Leu Val Gly Thr Lys Leu Asp Leu Arg Asp
    110                 115                 120
gat agc cag ttc ttt caa gac cat cct ggt gcg gcg cca atc acc aca      555
Asp Ser Gln Phe Phe Gln Asp His Pro Gly Ala Ala Pro Ile Thr Thr
125                 130                 135                 140

gca cag ggt gag gaa ctg aga aaa ctt atc ggt gct cca gtt tac att 603
Ala Gln Gly Glu Glu Leu Arg Lys Leu Ile Gly Ala Pro Val Tyr Ile
                145                 150                 155
gaa tgt agt tcc aaa aca cag aag aat gtg aag gct gtt ttt gat tcg 651
Glu Cys Ser Ser Lys Thr Gln Lys Asn Val Lys Ala Val Phe Asp Ser
            160                 165                 170
gcc atc aaa gta gtt cta cag cca cca aag caa aag aaa aca aag aga 699
Ala Ile Lys Val Val Leu Gln Pro Pro Lys Gln Lys Lys Thr Lys Arg
        175                 180                 185
aag ggg caa aaa gcc tgt tcc att ttg tgatcttcag ttctttcgta 746
Lys Gly Gln Lys Ala Cys Ser Ile Leu
    190                 195
ttgtagtttg tggaggatga catgacagta gccattttcc tgggtaatcc tttgccattc 806

tgattctata tatatcctta tactattttc gacctagatg gaggaaccgc catgtccaac 866

tgaccgcgtt aaagcttgct tataaaaaat caagctattt gtctctggct gaaggataat 926

atgtgaagtc ataattccat catgtttggt gtgtacaatt gttgtgtttc tccggaatgt 986

ttaccttttg tattgattat caaatggtca tctccatttg tggacatggg ttttactcac 1046

attgtagtct aatcttagga agtgcttgca acatatgtgc tttggatgtg aactaagtat 1106

atgtaattaa ttatgttagc tacttaacat ccttaatata gctcggtttg ttcaaaaaaa 1166

aaaaaaaaaa aa 1178

<210> 66
<211> 197
<212> PRT
<213> Medicago truncatula

<400> 66
Met Ser Gly Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Ser Thr Ile Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Ile
                85                  90                  95
Ala Lys Lys Trp Ile Pro Glu Leu Arg His Tyr Ala Pro Gly Val Pro
            100                 105                 110
Ile Ile Leu Val Gly Thr Lys Leu Asp Leu Arg Asp Asp Ser Gln Phe
        115                 120                 125
Phe Gln Asp His Pro Gly Ala Ala Pro Ile Thr Thr Ala Gln Gly Glu
    130                 135                 140
Glu Leu Arg Lys Leu Ile Gly Ala Pro Val Tyr Ile Glu Cys Ser Ser
145                 150                 155                 160
Lys Thr Gln Lys Asn Val Lys Ala Val Phe Asp Ser Ala Ile Lys Val
                165                 170                 175
Val Leu Gln Pro Pro Lys Gln Lys Lys Thr Lys Arg Lys Gly Gln Lys
            180                 185                 190

Ala Cys Ser Ile Leu
        195

<210> 67
<211> 1139
<212> DNA
<213> Medicago truncatula

<220>
<221> CDS
<222> (174)..(767)

<400> 67
acgaacgaac gcttcaacgg caaaatttcc cgggttcctt gtctctcttt catgcgcgag      60

aattctcatt ttctcacgct cctaatggat gcgtaattga agcgaataat tggaggagtg     120

tgtgagtgat ttggagtaat aaggtgaaag atctggatag ggtttgaaag aag atg        176
                                                           Met
                                                           1
agc gct tct agg ttc atc aag tgt gtt act gtt ggg gat gga gct gtt       224
Ser Ala Ser Arg Phe Ile Lys Cys Val Thr Val Gly Asp Gly Ala Val
            5                   10                  15
ggt aaa act tgt ttg tta att tca tac acc agc aat acc ttc ccc act       272
Gly Lys Thr Cys Leu Leu Ile Ser Tyr Thr Ser Asn Thr Phe Pro Thr
        20                  25                  30
gac tat gtg cca act gtc ttc gac aat ttc agt gca aat gtg gtt gtg       320
Asp Tyr Val Pro Thr Val Phe Asp Asn Phe Ser Ala Asn Val Val Val
    35                  40                  45
aat gga agc act gtg aat ctg ggt ttg tgg gac act gca gga caa gag       368
Asn Gly Ser Thr Val Asn Leu Gly Leu Trp Asp Thr Ala Gly Gln Glu
50                  55                  60                  65
gat tat aac aga tta aga cct ttg agt tat cgt ggt gcc gat gtt ttc       416
Asp Tyr Asn Arg Leu Arg Pro Leu Ser Tyr Arg Gly Ala Asp Val Phe
                70                  75                  80
att ctc gct ttc tcc ctc ata agc aag gcc agt tat gaa aat gtt tcc       464
Ile Leu Ala Phe Ser Leu Ile Ser Lys Ala Ser Tyr Glu Asn Val Ser
            85                  90                  95
aaa aag tgg att cca gag ttg aag cat tat gca cct ggt gtt ccc att       512
Lys Lys Trp Ile Pro Glu Leu Lys His Tyr Ala Pro Gly Val Pro Ile
        100                 105                 110
att ctg gtt ggc aca aag ctt gac ctt cgg gat gac aag cag ttc ttc       560
Ile Leu Val Gly Thr Lys Leu Asp Leu Arg Asp Asp Lys Gln Phe Phe
    115                 120                 125
gtc gac cat cct ggt gct gtt cct att acc act gct cag gga gaa gag       608
Val Asp His Pro Gly Ala Val Pro Ile Thr Thr Ala Gln Gly Glu Glu
130                 135                 140                 145
ctt agg aag ctg atc aat gca cct gct tat atc gaa tgc agt tcg aaa       656
Leu Arg Lys Leu Ile Asn Ala Pro Ala Tyr Ile Glu Cys Ser Ser Lys
                150                 155                 160
tca cag cag aat gtg aaa gca gtc ttt gat gca gcc ata aga gtt gtc       704
Ser Gln Gln Asn Val Lys Ala Val Phe Asp Ala Ala Ile Arg Val Val
            165                 170                 175
ctt caa cca cca aag caa aag aaa aag aag agt aaa gca cca aag gct       752
Leu Gln Pro Pro Lys Gln Lys Lys Lys Lys Ser Lys Ala Pro Lys Ala
        180                 185                 190
tgt tcg ata ttg tgatttgcta ggtcggtcta aagaaatttg acaaccattc           804
Cys Ser Ile Leu
    195
tccaacatgt atttctctcc tcttcttcct attcttcatt cctattatgc tgaagaagtt     864

ggagtctatg tgggtgttct ttcgtctaac agactctaga tctcgttgaa gcataaggtg     924

gttggtgctt tgctttcttg gaattattgt gacttctaat tgtcaaattt gtatttcatg     984

gttcccacct ttttcttaac ataagtttta tgtattaata gactggaata tcccagttag 1044

gattactacc ttgtttgctt gaagatatat ataatatcta cattattttg catttagtta 1104

taatgcttgc tctattcaaa aaaaaaaaaa aaaaa 1139

<210> 68
<211> 197
<212> PRT
<213> Medicago truncatula

<400> 68
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Ile Ile Leu Val Gly Thr Lys Leu Asp Leu Arg Asp Asp Lys Gln Phe
        115                 120                 125
Phe Val Asp His Pro Gly Ala Val Pro Ile Thr Thr Ala Gln Gly Glu
    130                 135                 140
Glu Leu Arg Lys Leu Ile Asn Ala Pro Ala Tyr Ile Glu Cys Ser Ser
145                 150                 155                 160
Lys Ser Gln Gln Asn Val Lys Ala Val Phe Asp Ala Ala Ile Arg Val
                165                 170                 175
Val Leu Gln Pro Pro Lys Gln Lys Lys Lys Lys Ser Lys Ala Pro Lys
            180                 185                 190
Ala Cys Ser Ile Leu
        195

<210> 69
<211> 985
<212> DNA
<213> Medicago truncatula

<220>
<221> CDS
<222> (143)..(736)

<400> 69
gttcaacgct aaaatcacac tctcatcttc caaatagatg tgtgatagta attgaaagag 60

aagaagaaga agaaagtact ggaggagggg gactgtcctc aggtgaagat ctgaggctat 120

tttgggtatt tcatttttca ag atg agt gct tct agg ttt att aaa tgt gtt 172
Met Ser Ala Ser Arg Phe Ile Lys Cys Val
1               5                   10
act gtt ggt gat gga gct gtt ggc aaa act tgt ttg ttg att tct tac 220
Thr Val Gly Asp Gly Ala Val Gly Lys Thr Cys Leu Leu Ile Ser Tyr
                15                  20                  25

acc agc aat act ttc ccc acg gat tat gtg ccg aca gtt ttt gac aat 268
Thr Ser Asn Thr Phe Pro Thr Asp Tyr Val Pro Thr Val Phe Asp Asn
            30                  35                  40
ttc agt gcg aat gtg gtt gtt aat gga agt att gtg aat ctg ggt ttg 316
Phe Ser Ala Asn Val Val Val Asn Gly Ser Ile Val Asn Leu Gly Leu
        45                  50                  55
tgg gat act gct gga caa gag gat tat aac aga tta aga cct ttg agt 364
Trp Asp Thr Ala Gly Gln Glu Asp Tyr Asn Arg Leu Arg Pro Leu Ser
    60                  65                  70
tac cgt ggt gcc gat gtt ttc ata ttg gct ttc tct ctc ata agc aaa 412
Tyr Arg Gly Ala Asp Val Phe Ile Leu Ala Phe Ser Leu Ile Ser Lys
75                  80                  85                  90
gcc agt tat gaa aat gtc tcc aaa aag tgg att cca gag ttg aag cat 460
Ala Ser Tyr Glu Asn Val Ser Lys Lys Trp Ile Pro Glu Leu Lys His
                95                  100                 105
tat gca cct ggt gtc ccc ata att ctg gtt gga aca aag ctt gat ctt 508
Tyr Ala Pro Gly Val Pro Ile Ile Leu Val Gly Thr Lys Leu Asp Leu
            110                 115                 120
cgg gat gat aag cag ttc tgc ata gac cat cct ggt gcc gtt ccc att 556
Arg Asp Asp Lys Gln Phe Cys Ile Asp His Pro Gly Ala Val Pro Ile
        125                 130                 135
acc aca gct cag gga gaa gag ctg agg aag ctg att aat gca cca gct 604
Thr Thr Ala Gln Gly Glu Glu Leu Arg Lys Leu Ile Asn Ala Pro Ala
    140                 145                 150
tac att gaa tgc agt tca aaa tca cag gag aac gtg aag gcg gtg ttt 652
Tyr Ile Glu Cys Ser Ser Lys Ser Gln Glu Asn Val Lys Ala Val Phe
155                 160                 165                 170
gat gca gcc ata aga gtt gtc ctt caa cca cca aag cag aag aaa aag 700
Asp Ala Ala Ile Arg Val Val Leu Gln Pro Pro Lys Gln Lys Lys Lys
                175                 180                 185
aag aat aaa gca caa aag gcc tgt tca ata ttg taattcacta ggtgtaaaca 753
Lys Asn Lys Ala Gln Lys Ala Cys Ser Ile Leu
            190                 195
tttgattgtc ctttgttcta cctgtattcc cctccccttc ttttcatcat tttctttgtt 813

attgaagaag ttggagagtc tttgtgggtg ttctttcaga caaatactct agatctcctt 873

gaagcataag gtggcttaat gtttttgctt tctatgaatt attattgtca actctaatag 933

ttaaatttgc atttcatgct tcccatcttt ttaaaaaaaa aaaaaaaaaa aa 985

<210> 70
<211> 197
<212> PRT
<213> Medicago truncatula

<400> 70
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ser Ile Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Ile Ile Leu Val Gly Thr Lys Leu Asp Leu Arg Asp Asp Lys Gln Phe

                115                 120                 125
Cys Ile Asp His Pro Gly Ala Val Pro Ile Thr Thr Ala Gln Gly Glu
    130                 135                 140
Glu Leu Arg Lys Leu Ile Asn Ala Pro Ala Tyr Ile Glu Cys Ser Ser
145                 150                 155                 160
Lys Ser Gln Glu Asn Val Lys Ala Val Phe Asp Ala Ala Ile Arg Val
                165                 170                 175
Val Leu Gln Pro Pro Lys Gln Lys Lys Lys Lys Asn Lys Ala Gln Lys
            180                 185                 190
Ala Cys Ser Ile Leu
        195

<210> 71
<211> 779
<212> DNA
<213> Brassica napus

<220>
<221> CDS
<222> (183)..(776)

<400> 71
ggctcatagg gtttctagtt ttcgggagaa agttctttca tttttcaaga atgcccagat      60

gctcgattga ggttgagcat ccagacaacg aagaagaaaa ttaacaaaat ctttgagctt     120

ttcttttat ttttggtttt tccccctga aagtattaac aaataatctt cgagtagaga      180

aa atg agc gca tct cgg ttc ata aag tgt gta acg gtt ggt gat gga       227
   Met Ser Ala Ser Arg Phe Ile Lys Cys Val Thr Val Gly Asp Gly
   1               5                   10                  15
gct gtc gga aaa aca tgt ttg ttg att tct tac aca agc aac act ttc      275
Ala Val Gly Lys Thr Cys Leu Leu Ile Ser Tyr Thr Ser Asn Thr Phe
                20                  25                  30
cct acg gac tat gtg ccc acc gtt ttc gat aat ttc agt gct aat gtg      323
Pro Thr Asp Tyr Val Pro Thr Val Phe Asp Asn Phe Ser Ala Asn Val
            35                  40                  45
gtg gtt aac gga gcc acc gtt aat ctt gga ttg tgg gat act gca ggg      371
Val Val Asn Gly Ala Thr Val Asn Leu Gly Leu Trp Asp Thr Ala Gly
        50                  55                  60
caa gag gat tac aac aga tta aga cca cta agc tac cgt gga gct gat      419
Gln Glu Asp Tyr Asn Arg Leu Arg Pro Leu Ser Tyr Arg Gly Ala Asp
    65                  70                  75
gtt ttc ata ttg gcc ttc tct ctt atc agt aaa gcc agt tat gaa aac      467
Val Phe Ile Leu Ala Phe Ser Leu Ile Ser Lys Ala Ser Tyr Glu Asn
80                  85                  90                  95
gtc tcc aaa aag tgg atc ccg gag ttg aag cat tac gcg cct ggt gtc      515
Val Ser Lys Lys Trp Ile Pro Glu Leu Lys His Tyr Ala Pro Gly Val
                100                 105                 110
ccc gtc atc ctt gtt gga tca aag ctt gat ctt cga gat gat aag caa      563
Pro Val Ile Leu Val Gly Ser Lys Leu Asp Leu Arg Asp Asp Lys Gln
            115                 120                 125
ttc ttc gtc gac cat cct ggc gct gtc ccg att aca act gct cag gga      611
Phe Phe Val Asp His Pro Gly Ala Val Pro Ile Thr Thr Ala Gln Gly
        130                 135                 140
gag gag ctg agg aag cta ata gat gca cct act tac atc gaa tgc agt      659
Glu Glu Leu Arg Lys Leu Ile Asp Ala Pro Thr Tyr Ile Glu Cys Ser
    145                 150                 155
tcc aaa tct caa gag aat gtg aaa gct gtc ttt gac gca gcc ata cga      707
Ser Lys Ser Gln Glu Asn Val Lys Ala Val Phe Asp Ala Ala Ile Arg
160                 165                 170                 175
gtg gtg ttg caa ccg cct aag cag aag aag aaa aag agc aaa gcg cag      755
Val Val Leu Gln Pro Pro Lys Gln Lys Lys Lys Lys Ser Lys Ala Gln
                180                 185                 190
aag gca tgc tcc atc cag tgatat                                       779

Lys Ala Cys Ser Ile Gln
            195

<210> 72
<211> 197
<212> PRT
<213> Brassica napus

<400> 72
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ala Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Val Ile Leu Val Gly Ser Lys Leu Asp Leu Arg Asp Asp Lys Gln Phe
        115                 120                 125
Phe Val Asp His Pro Gly Ala Val Pro Ile Thr Thr Ala Gln Gly Glu
    130                 135                 140
Glu Leu Arg Lys Leu Ile Asp Ala Pro Thr Tyr Ile Glu Cys Ser Ser
145                 150                 155                 160
Lys Ser Gln Glu Asn Val Lys Ala Val Phe Asp Ala Ala Ile Arg Val
                165                 170                 175
Val Leu Gln Pro Pro Lys Gln Lys Lys Lys Lys Ser Lys Ala Gln Lys
            180                 185                 190
Ala Cys Ser Ile Gln
        195

<210> 73
<211> 757
<212> DNA
<213> Brassica napus

<220>
<221> CDS
<222> (164)..(757)

<400> 73
cgatacaccg attcatttca ttcttcattg atcgcttcct ctcacatacc cgatttcgtt      60

tagggttaac attttctagg gttttagaga taggcgaaac tgaaattgag aagaagacgg     120

gtgttgtttc ctcatagatc tgaggggttg aatttttcga ttt atg agc gca tct       175
                                                Met Ser Ala Ser
                                                1
cgg ttc ata aag tgc gtg acg gtt ggg gac gga gca gtg ggc aaa aca       223
Arg Phe Ile Lys Cys Val Thr Val Gly Asp Gly Ala Val Gly Lys Thr
5                   10                  15                  20
tgt ctc ctc atc tct tac acc agc aac act ttc cct acg gat tat gtt       271
Cys Leu Leu Ile Ser Tyr Thr Ser Asn Thr Phe Pro Thr Asp Tyr Val
                25                  30                  35
cca act gtt ttc gat aac ttc agc gct aat gtt gtt gtt aac gga gcc       319
Pro Thr Val Phe Asp Asn Phe Ser Ala Asn Val Val Val Asn Gly Ala
            40                  45                  50
act gtc aac tta gga ctc tgg gat acc gca ggg cag gag gat tat aac       367
Thr Val Asn Leu Gly Leu Trp Asp Thr Ala Gly Gln Glu Asp Tyr Asn
        55                  60                  65

aga ttg aga ccc ttg agt tac cgc ggt gct gac gtt ttc atc ttg gcc 415
Arg Leu Arg Pro Leu Ser Tyr Arg Gly Ala Asp Val Phe Ile Leu Ala
    70                  75                  80
ttc tct ctc atc agt aag gct agt tat gag aat gtc tcc aag aag tgg 463
Phe Ser Leu Ile Ser Lys Ala Ser Tyr Glu Asn Val Ser Lys Lys Trp
85                  90                  95                  100
atc cct gag ctg acc cac tat gcc cct ggt gtc cct atc gtt ctt gtt 511
Ile Pro Glu Leu Thr His Tyr Ala Pro Gly Val Pro Ile Val Leu Val
                105                 110                 115
ggt acc aaa cta gat ctt agg gat gac aaa cag ttc ttc gtt gac cac 559
Gly Thr Lys Leu Asp Leu Arg Asp Asp Lys Gln Phe Phe Val Asp His
            120                 125                 130
cct ggt gct gta cct att acc act tct cag gga gag gaa cta atg aag 607
Pro Gly Ala Val Pro Ile Thr Thr Ser Gln Gly Glu Glu Leu Met Lys
        135                 140                 145
cta att gga gct cct tcg tac atc gag tgc agt tca aaa tct caa gag 655
Leu Ile Gly Ala Pro Ser Tyr Ile Glu Cys Ser Ser Lys Ser Gln Glu
    150                 155                 160
aac gtg aaa ggg gtg ttt gat gca gcg atc aga gtg gta ctt caa cct 703
Asn Val Lys Gly Val Phe Asp Ala Ala Ile Arg Val Val Leu Gln Pro
165                 170                 175                 180
cca aag cag aag aaa aag aag agc aag gca caa aag gcc tgc tcc att 751
Pro Lys Gln Lys Lys Lys Lys Ser Lys Ala Gln Lys Ala Cys Ser Ile
                185                 190                 195
ttg taa 757
Leu

<210> 74
<211> 197
<212> PRT
<213> Brassica napus

<400> 74
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ala Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Thr His Tyr Ala Pro Gly Val Pro
            100                 105                 110
Ile Val Leu Val Gly Thr Lys Leu Asp Leu Arg Asp Asp Lys Gln Phe
        115                 120                 125
Phe Val Asp His Pro Gly Ala Val Pro Ile Thr Thr Ser Gln Gly Glu
    130                 135                 140
Glu Leu Met Lys Leu Ile Gly Ala Pro Ser Tyr Ile Glu Cys Ser Ser
145                 150                 155                 160
Lys Ser Gln Glu Asn Val Lys Gly Val Phe Asp Ala Ala Ile Arg Val
                165                 170                 175
Val Leu Gln Pro Pro Lys Gln Lys Lys Lys Lys Ser Lys Ala Gln Lys
            180                 185                 190
Ala Cys Ser Ile Leu
        195

<210> 75
<211> 645
<212> DNA
<213> Oryza sativa

<220>

<221> CDS
<222> (1)..(645)

<400> 75
atg agc tcg gcg gcg gcg gcg acg agg ttc atc aag tgc gtc acc gtg 48
Met Ser Ser Ala Ala Ala Ala Thr Arg Phe Ile Lys Cys Val Thr Val
1               5                   10                  15
ggg gac ggc gcg gtg ggg aag acg tgc atg ctc atc tgc tac acc tgc 96
Gly Asp Gly Ala Val Gly Lys Thr Cys Met Leu Ile Cys Tyr Thr Cys
            20                  25                  30
aac aag ttc ccc acc gat tac atc ccc acc gtg ttc gac aac ttc agc 144
Asn Lys Phe Pro Thr Asp Tyr Ile Pro Thr Val Phe Asp Asn Phe Ser
        35                  40                  45
gcc aat gtc tcc gtg gac ggg agc gtc gtc aac ctc ggc ctc tgg gac 192
Ala Asn Val Ser Val Asp Gly Ser Val Val Asn Leu Gly Leu Trp Asp
    50                  55                  60
act gca ggt cag gag gat tac agc agg ttg agg cct ctg agc tac agg 240
Thr Ala Gly Gln Glu Asp Tyr Ser Arg Leu Arg Pro Leu Ser Tyr Arg
65                  70                  75                  80
gga gcc gat gtg ttc atc ctg tcc ttc tcc ctg ata agc agg gcg agc 288
Gly Ala Asp Val Phe Ile Leu Ser Phe Ser Leu Ile Ser Arg Ala Ser
                85                  90                  95
tat gag aat gtt cag aag aag tgg atg cca gag ctt cgc cgg ttt gcg 336
Tyr Glu Asn Val Gln Lys Lys Trp Met Pro Glu Leu Arg Arg Phe Ala
            100                 105                 110
cct ggt gtt cct gta gtt ctt gtt gga acc aag ttg gat ctc cgt gaa 384
Pro Gly Val Pro Val Val Leu Val Gly Thr Lys Leu Asp Leu Arg Glu
        115                 120                 125
gat agg gcc tat ctt gct gat cat cca gct tct tcc ata ata aca acg 432
Asp Arg Ala Tyr Leu Ala Asp His Pro Ala Ser Ser Ile Ile Thr Thr
    130                 135                 140
gag cag gga gaa gaa ctg agg aag cta ata gga gcg gtc gcc tac atc 480
Glu Gln Gly Glu Glu Leu Arg Lys Leu Ile Gly Ala Val Ala Tyr Ile
145                 150                 155                 160
gaa tgc agc tcc aag aca cag aga aac att aaa gct gtt ttc gac act 528
Glu Cys Ser Ser Lys Thr Gln Arg Asn Ile Lys Ala Val Phe Asp Thr
                165                 170                 175
gcc atc aaa gtg gtg ctt caa cct cca aga cat aag gat gta acc aga 576
Ala Ile Lys Val Val Leu Gln Pro Pro Arg His Lys Asp Val Thr Arg
            180                 185                 190
aag aaa ctc caa tca agc tcc aat cgg cca gta agg cgg tac ttt tgc 624
Lys Lys Leu Gln Ser Ser Ser Asn Arg Pro Val Arg Arg Tyr Phe Cys
        195                 200                 205
gga agc gct tgt ttc gcg tag 645
Gly Ser Ala Cys Phe Ala
    210

<210> 76
<211> 214
<212> PRT
<213> Oryza sativa

<400> 76
Met Ser Ser Ala Ala Ala Ala Thr Arg Phe Ile Lys Cys Val Thr Val
1               5                   10                  15
Gly Asp Gly Ala Val Gly Lys Thr Cys Met Leu Ile Cys Tyr Thr Cys
            20                  25                  30
Asn Lys Phe Pro Thr Asp Tyr Ile Pro Thr Val Phe Asp Asn Phe Ser
        35                  40                  45
Ala Asn Val Ser Val Asp Gly Ser Val Val Asn Leu Gly Leu Trp Asp
    50                  55                  60
Thr Ala Gly Gln Glu Asp Tyr Ser Arg Leu Arg Pro Leu Ser Tyr Arg
65                  70                  75                  80
Gly Ala Asp Val Phe Ile Leu Ser Phe Ser Leu Ile Ser Arg Ala Ser
                85                  90                  95
Tyr Glu Asn Val Gln Lys Lys Trp Met Pro Glu Leu Arg Arg Phe Ala
            100                 105                 110

Pro Gly Val Pro Val Val Leu Val Gly Thr Lys Leu Asp Leu Arg Glu
        115                 120                 125
Asp Arg Ala Tyr Leu Ala Asp His Pro Ala Ser Ser Ile Ile Thr Thr
    130                 135                 140
Glu Gln Gly Glu Glu Leu Arg Lys Leu Ile Gly Ala Val Ala Tyr Ile
145                 150                 155                 160
Glu Cys Ser Ser Lys Thr Gln Arg Asn Ile Lys Ala Val Phe Asp Thr
                165                 170                 175
Ala Ile Lys Val Val Leu Gln Pro Pro Arg His Lys Asp Val Thr Arg
            180                 185                 190
Lys Lys Leu Gln Ser Ser Ser Asn Arg Pro Val Arg Arg Tyr Phe Cys
        195                 200                 205
Gly Ser Ala Cys Phe Ala
    210

<210> 77
<211> 1135
<212> DNA
<213> Homo sapiens (man)

<220>
<221> CDS
<222> (105)..(680)

<400> 77
ggcagccgag gagaccccgc gcagtgctgc caacgccccg gtggagaagc tgaggtcatc      60

atcagatttg aaatatttaa agtggataca aaactatttc agca atg cag aca att      116
                                                 Met Gln Thr Ile
                                                 1
aag tgt gtt gtt gtg ggc gat ggt gct gtt ggt aaa aca tgt ctc ctg      164
Lys Cys Val Val Val Gly Asp Gly Ala Val Gly Lys Thr Cys Leu Leu
5                   10                  15                  20
ata tcc tac aca aca aac aaa ttt cca tcg gaa tat gta ccg act gtt      212
Ile Ser Tyr Thr Thr Asn Lys Phe Pro Ser Glu Tyr Val Pro Thr Val
                25                  30                  35
ttt gac aac tat gca gtc aca gtt atg att ggt gga gaa cca tat act      260
Phe Asp Asn Tyr Ala Val Thr Val Met Ile Gly Gly Glu Pro Tyr Thr
            40                  45                  50
ctt gga ctt ttt gat act gca ggg caa gag gat tat gac aga tta cga      308
Leu Gly Leu Phe Asp Thr Ala Gly Gln Glu Asp Tyr Asp Arg Leu Arg
        55                  60                  65
ccg ctg agt tat cca caa aca gat gta ttt cta gtc tgt ttt tca gtg      356
Pro Leu Ser Tyr Pro Gln Thr Asp Val Phe Leu Val Cys Phe Ser Val
    70                  75                  80
gtc tct cca tct tca ttt gaa aac gtg aaa gaa aag tgg gtg cct gag      404
Val Ser Pro Ser Ser Phe Glu Asn Val Lys Glu Lys Trp Val Pro Glu
85                  90                  95                  100
ata act cac cac tgt cca aag act cct ttc ttg ctt gtt ggg act caa      452
Ile Thr His His Cys Pro Lys Thr Pro Phe Leu Leu Val Gly Thr Gln
                105                 110                 115
att gat ctc aga gat gac ccc tct act att gag aaa ctt gcc aag aac      500
Ile Asp Leu Arg Asp Asp Pro Ser Thr Ile Glu Lys Leu Ala Lys Asn
            120                 125                 130
aaa cag aag cct atc act cca gag act gct gaa aag ctg gcc cgt gac      548
Lys Gln Lys Pro Ile Thr Pro Glu Thr Ala Glu Lys Leu Ala Arg Asp
        135                 140                 145
ctg aag gct gtc aag tat gtg gag tgt tct gca ctt aca cag aga ggt      596
Leu Lys Ala Val Lys Tyr Val Glu Cys Ser Ala Leu Thr Gln Arg Gly
    150                 155                 160
ctg aag aat gtg ttt gat gag gct atc cta gct gcc ctc gag cct ccg      644
Leu Lys Asn Val Phe Asp Glu Ala Ile Leu Ala Ala Leu Glu Pro Pro
165                 170                 175                 180
gaa act caa ccc aaa agg aag tgc tgt ata ttc taaactgttt tctccttccc      697
Glu Thr Gln Pro Lys Arg Lys Cys Cys Ile Phe
                185                 190
ttctttgctg ctgcttcctg tcccactact gtagaaagat cgtttaaaaa caaaggaata      757

aaaccatcct gtttgaaagc ctctgcgtct ttttactcac caccttagag caacctctgt 817

attagttttt gatcaagaat tgcaatatca tataaatttt ttgtgatcag tagtcaagtt 877

ggacttgttt taacgttctg ctgcttgagt tgcctgatgc tcagagcttt ttggtttgga 937

ttactattgc aaagggaact tggtctggct tagatgtcct cttggagaaa ataacaagag 997

ttttaacact tctagatctt agttcagatg gagaaagtaa cacaaacatc attttactct 1057

tatgatcaat tgttaattgt aattgcatga caaaccttat ggaaaagggg tgacctagta 1117

gagtgtaatg gggaaggg 1135

<210> 78
<211> 191
<212> PRT
<213> Homo sapiens (man)

<400> 78
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Glu Tyr
            20                  25                  30
Val Pro Thr Val Phe Asp Asn Tyr Ala Val Thr Val Met Ile Gly Gly
        35                  40                  45
Glu Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Val Ser Pro Ser Ser Phe Glu Asn Val Lys Glu Lys
                85                  90                  95
Trp Val Pro Glu Ile Thr His His Cys Pro Lys Thr Pro Phe Leu Leu
            100                 105                 110
Val Gly Thr Gln Ile Asp Leu Arg Asp Asp Pro Ser Thr Ile Glu Lys
        115                 120                 125
Leu Ala Lys Asn Lys Gln Lys Pro Ile Thr Pro Glu Thr Ala Glu Lys
    130                 135                 140
Leu Ala Arg Asp Leu Lys Ala Val Lys Tyr Val Glu Cys Ser Ala Leu
145                 150                 155                 160
Thr Gln Arg Gly Leu Lys Asn Val Phe Asp Glu Ala Ile Leu Ala Ala
                165                 170                 175
Leu Glu Pro Pro Glu Thr Gln Pro Lys Arg Lys Cys Cys Ile Phe
            180                 185                 190

<210> 79
<211> 576
<212> DNA
<213> Caenorhabditis elegans

<220>
<221> CDS
<222> (1)..(576)

<400> 79
atg caa gcg atc aaa tgt gtc gtc gtt ggt gac gga gcc gtc ggt aaa 48
Met Gln Ala Ile Lys Cys Val Val Val Gly Asp Gly Ala Val Gly Lys

1                   5                   10                  15
acg tgt ctc ctg ata tcc tac acc aca aac gca ttt ccc gga gaa tat        96
Thr Cys Leu Leu Ile Ser Tyr Thr Thr Asn Ala Phe Pro Gly Glu Tyr
                20                  25                  30
att ccg acg gta ttc gac aac tac tca gca aat gtg atg gtc gac ggt        144
Ile Pro Thr Val Phe Asp Asn Tyr Ser Ala Asn Val Met Val Asp Gly
            35                  40                  45
cgg ccg ata aat ctc ggg ctc tgg gat aca gct gga cag gaa gat tac        192
Arg Pro Ile Asn Leu Gly Leu Trp Asp Thr Ala Gly Gln Glu Asp Tyr
    50                  55                  60
gat cga ctc cga cca ctg tca tat cca caa aca gac gtg ttt ctc gta        240
Asp Arg Leu Arg Pro Leu Ser Tyr Pro Gln Thr Asp Val Phe Leu Val
65                  70                  75                  80
tgc ttt gcc ctg aac aat ccg gcg agt ttt gag aat gtt cgt gcg aaa        288
Cys Phe Ala Leu Asn Asn Pro Ala Ser Phe Glu Asn Val Arg Ala Lys
                85                  90                  95
tgg tat cca gaa gtg tca cat cat tgc ccg aat acg ccg att att ttg        336
Trp Tyr Pro Glu Val Ser His His Cys Pro Asn Thr Pro Ile Ile Leu
            100                 105                 110
gtt gga acg aaa gct gat ctg cgt gag gat cga gat act gtt gaa cgg        384
Val Gly Thr Lys Ala Asp Leu Arg Glu Asp Arg Asp Thr Val Glu Arg
        115                 120                 125
ctc cgc gaa cgc cgg ctc caa cca gtg agc caa acc cag ggc tac gtg        432
Leu Arg Glu Arg Arg Leu Gln Pro Val Ser Gln Thr Gln Gly Tyr Val
    130                 135                 140
atg gca aag gaa atc aag gct gtc aag tat ctg gag tgc tcg gcg ctc        480
Met Ala Lys Glu Ile Lys Ala Val Lys Tyr Leu Glu Cys Ser Ala Leu
145                 150                 155                 160
acg caa cgt ggt ctg aaa caa gtt ttc gat gag gcg atc cga gcc gtc        528
Thr Gln Arg Gly Leu Lys Gln Val Phe Asp Glu Ala Ile Arg Ala Val
                165                 170                 175
gtc acg ccg cca caa aga gcc aaa aag agc aag tgt acg gtg ctc            573
Val Thr Pro Pro Gln Arg Ala Lys Lys Ser Lys Cys Thr Val Leu
            180                 185                 190
taa                                                                    576

<210> 80
<211> 191
<212> PRT
<213> Caenorhabditis elegans

<400> 80
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly
        35                  40                  45
Arg Pro Ile Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ala Leu Asn Asn Pro Ala Ser Phe Glu Asn Val Arg Ala Lys
                85                  90                  95
Trp Tyr Pro Glu Val Ser His His Cys Pro Asn Thr Pro Ile Ile Leu
            100                 105                 110
Val Gly Thr Lys Ala Asp Leu Arg Glu Asp Arg Asp Thr Val Glu Arg
        115                 120                 125
Leu Arg Glu Arg Arg Leu Gln Pro Val Ser Gln Thr Gln Gly Tyr Val
    130                 135                 140
Met Ala Lys Glu Ile Lys Ala Val Lys Tyr Leu Glu Cys Ser Ala Leu
145                 150                 155                 160
Thr Gln Arg Gly Leu Lys Gln Val Phe Asp Glu Ala Ile Arg Ala Val
                165                 170                 175
Val Thr Pro Pro Gln Arg Ala Lys Lys Ser Lys Cys Thr Val Leu

180                                    185                                    190

<210> 81
<211> 579
<212> DNA
<213> Caenorhabditis elegans

<220>
<221> CDS
<222> (1)..(579)

<400> 81
atg gct gcg att aga aag aag ttg gtg atc gtc ggc gac gga gcc tgc          48
Met Ala Ala Ile Arg Lys Lys Leu Val Ile Val Gly Asp Gly Ala Cys
1               5                   10                  15
ggt aaa act tgc ctg ctc atc gtt ttc tca aag gat cag ttc cca gac          96
Gly Lys Thr Cys Leu Leu Ile Val Phe Ser Lys Asp Gln Phe Pro Asp
            20                  25                  30
gtg tat gtg ccg act gtt ttc gag aat tat gtt gcc gac att gaa gtt          144
Val Tyr Val Pro Thr Val Phe Glu Asn Tyr Val Ala Asp Ile Glu Val
        35                  40                  45
gac gga aag cag gtc gaa ctt gct cta tgg gat aca gct gga caa gag          192
Asp Gly Lys Gln Val Glu Leu Ala Leu Trp Asp Thr Ala Gly Gln Glu
    50                  55                  60
gac tat gat cgt ctg cgt cca ctc tct tat ccg gat act gac gtc att          240
Asp Tyr Asp Arg Leu Arg Pro Leu Ser Tyr Pro Asp Thr Asp Val Ile
65                  70                  75                  80
ctg atg tgc ttc tca att gat tca ccc gat tca ctg gag aat att cca          288
Leu Met Cys Phe Ser Ile Asp Ser Pro Asp Ser Leu Glu Asn Ile Pro
                85                  90                  95
gaa aaa tgg aca cca gaa gtc agg cat ttc tgt cca aat gtt ccg att          336
Glu Lys Trp Thr Pro Glu Val Arg His Phe Cys Pro Asn Val Pro Ile
            100                 105                 110
att ttg gtc gga aat aaa cga gat ctt cgt agt gat cca cag act gtt          384
Ile Leu Val Gly Asn Lys Arg Asp Leu Arg Ser Asp Pro Gln Thr Val
        115                 120                 125
cga gaa ttg gca aaa atg aaa cag gag ccc gtg aag cct gag cag gga          432
Arg Glu Leu Ala Lys Met Lys Gln Glu Pro Val Lys Pro Glu Gln Gly
    130                 135                 140
cga gca att gct gag caa att gga gca ttt gca tat ttg gag tgc tct          480
Arg Ala Ile Ala Glu Gln Ile Gly Ala Phe Ala Tyr Leu Glu Cys Ser
145                 150                 155                 160
gcg aag act aag gac gga att cgt gag gta ttc gag aag gcg aca cag          528
Ala Lys Thr Lys Asp Gly Ile Arg Glu Val Phe Glu Lys Ala Thr Gln
                165                 170                 175
gcg gca ttg caa cag aag aag aaa aag aag agc aag tgc atg att ttg          576
Ala Ala Leu Gln Gln Lys Lys Lys Lys Lys Ser Lys Cys Met Ile Leu
            180                 185                 190
taa                                                                      579

<210> 82
<211> 192
<212> PRT
<213> Caenorhabditis elegans

<400> 82
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Asp
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile

65                  70                  75                  80
Leu Met Cys Phe Ser Ile Asp Ser Pro Asp Ser Leu Glu Asn Ile Pro
                85                  90                  95
Glu Lys Trp Thr Pro Glu Val Arg His Phe Cys Pro Asn Val Pro Ile
            100                 105                 110
Ile Leu Val Gly Asn Lys Arg Asp Leu Arg Ser Asp Pro Gln Thr Val
        115                 120                 125
Arg Glu Leu Ala Lys Met Lys Gln Glu Pro Val Lys Pro Glu Gln Gly
    130                 135                 140
Arg Ala Ile Ala Glu Gln Ile Gly Ala Phe Ala Tyr Leu Glu Cys Ser
145                 150                 155                 160
Ala Lys Thr Lys Asp Gly Ile Arg Glu Val Phe Glu Lys Ala Thr Gln
                165                 170                 175
Ala Ala Leu Gln Gln Lys Lys Lys Lys Lys Ser Lys Cys Met Ile Leu
            180                 185                 190

<210> 83
<211> 928
<212> DNA
<213> Schizosaccharomyces pombe

<220>
<221> CDS
<222> (55)..(633)

<400> 83
gaattcaata aagtgaagca aagctttacg attaattatt ttttgtgaaa tagt atg      57
                                                            Met
                                                            1
ccc acc att aag tgt gtc gta gta gga gac ggt gct gta gga aag acc      105
Pro Thr Ile Lys Cys Val Val Val Gly Asp Gly Ala Val Gly Lys Thr
            5                   10                  15
tgt ctg ctt att tcc tat act aca aac aag ttt cct agt gac tat gtg      153
Cys Leu Leu Ile Ser Tyr Thr Thr Asn Lys Phe Pro Ser Asp Tyr Val
        20                  25                  30
cca act gta ttc gat aat tat gct gtc act gtc atg atc ggt gat gaa      201
Pro Thr Val Phe Asp Asn Tyr Ala Val Thr Val Met Ile Gly Asp Glu
    35                  40                  45
cca tac act ctt ggt tta ttc gat acc gct ggt cag gag gat tat gat      249
Pro Tyr Thr Leu Gly Leu Phe Asp Thr Ala Gly Gln Glu Asp Tyr Asp
50                  55                  60                  65
cgc ttg cgt cct tta tcc tat cct caa aca gac gtc ttt ttg gtt tgt      297
Arg Leu Arg Pro Leu Ser Tyr Pro Gln Thr Asp Val Phe Leu Val Cys
                70                  75                  80
ttt agt gta act tct cct gcc agt ttt gaa aat gtg aaa gaa aag tgg      345
Phe Ser Val Thr Ser Pro Ala Ser Phe Glu Asn Val Lys Glu Lys Trp
            85                  90                  95
ttt ccc gaa gtt cat cat cat tgt ccg ggc gtc ccg tgc tta att gtt      393
Phe Pro Glu Val His His His Cys Pro Gly Val Pro Cys Leu Ile Val
        100                 105                 110
ggt acc caa att gat tta cgt gat gac cct tct gtg caa cag aaa cta      441
Gly Thr Gln Ile Asp Leu Arg Asp Asp Pro Ser Val Gln Gln Lys Leu
    115                 120                 125
gct cgc cag cat cag cat ccc ctt aca cat gag caa ggt gaa cga tta      489
Ala Arg Gln His Gln His Pro Leu Thr His Glu Gln Gly Glu Arg Leu
130                 135                 140                 145
gct cgt gag ttg ggt gct gtc aag tat gtt gag tgt tcc gct ttg acc      537
Ala Arg Glu Leu Gly Ala Val Lys Tyr Val Glu Cys Ser Ala Leu Thr
                150                 155                 160
caa aaa ggg tta aag aat gtt ttt gat gaa gct att gta gcc gct ctt      585
Gln Lys Gly Leu Lys Asn Val Phe Asp Glu Ala Ile Val Ala Ala Leu
            165                 170                 175
gat cct cct gtt cct cac aag aaa aag tca aag tgt ttg gta ctg          630
Asp Pro Pro Val Pro His Lys Lys Lys Ser Lys Cys Leu Val Leu
        180                 185                 190
taatttaacc ctggttttct ttccttttcc gaaatttcct taatacagct agtttacaac      690

agtaaacaat gtcttagctt tctttaacga tccctcatat ttgcaaagat atttgctatt 750

tgttattttc atcctagctt ttgtgcttct tcattcattc tgtcaatcac acacttcttg 810

ggctttacat atctgtgttt tctggctagg catatttcct ttttccgtta aataaacgac 870

gattttgcta tacttttgct acattattgt attcttctgc cattactcgc aggaattc 928

<210> 84
<211> 192
<212> PRT
<213> Schizosaccharomyces pombe

<400> 84
Met Pro Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Asp Tyr
            20                  25                  30
Val Pro Thr Val Phe Asp Asn Tyr Ala Val Thr Val Met Ile Gly Asp
        35                  40                  45
Glu Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Thr Ser Pro Ala Ser Phe Glu Asn Val Lys Glu Lys
                85                  90                  95
Trp Phe Pro Glu Val His His His Cys Pro Gly Val Pro Cys Leu Ile
            100                 105                 110
Val Gly Thr Gln Ile Asp Leu Arg Asp Asp Pro Ser Val Gln Gln Lys
        115                 120                 125
Leu Ala Arg Gln His Gln His Pro Leu Thr His Glu Gln Gly Glu Arg
    130                 135                 140
Leu Ala Arg Glu Leu Gly Ala Val Lys Tyr Val Glu Cys Ser Ala Leu
145                 150                 155                 160
Thr Gln Lys Gly Leu Lys Asn Val Phe Asp Glu Ala Ile Val Ala Ala
                165                 170                 175
Leu Asp Pro Pro Val Pro His Lys Lys Lys Ser Lys Cys Leu Val Leu
            180                 185                 190

<210> 85
<211> 579
<212> DNA
<213> Mus musculus (house mouse)

<220>
<221> CDS
<222> (1)..(579)

<400> 85
atg cag gcc atc aag tgt gtg gtg gtg ggt gat gga gcc gtg ggc aag 48
Met Gln Ala Ile Lys Cys Val Val Val Gly Asp Gly Ala Val Gly Lys
1               5                   10                  15
acg tgt ctt ctc atc agc tac acc acc aac gcc ttc cct gga gaa tac 96
Thr Cys Leu Leu Ile Ser Tyr Thr Thr Asn Ala Phe Pro Gly Glu Tyr
            20                  25                  30
atc ccc act gta ttt gac aac tac tca gcc aat gtg atg gtg gac agt 144
Ile Pro Thr Val Phe Asp Asn Tyr Ser Ala Asn Val Met Val Asp Ser
        35                  40                  45
aag ccg gtg aac ctg ggg ctg tgg gat acc gca ggt cag gag gac tat 192
Lys Pro Val Asn Leu Gly Leu Trp Asp Thr Ala Gly Gln Glu Asp Tyr
    50                  55                  60
gac cgc ctc cgg cca ctc tcc tac cca cag aca gat gta ttt ctc atc 240

Asp Arg Leu Arg Pro Leu Ser Tyr Pro Gln Thr Asp Val Phe Leu Ile
65                  70                  75                  80
tgc ttc tcg cta gtc agc cca gcc tcc tat gag aat gtc cgt gcc aag      288
Cys Phe Ser Leu Val Ser Pro Ala Ser Tyr Glu Asn Val Arg Ala Lys
                85                  90                  95
tgg ttc cct gag gta cgg cac cac tgc ccc agc acc ccc atc atc ctg      336
Trp Phe Pro Glu Val Arg His His Cys Pro Ser Thr Pro Ile Ile Leu
            100                 105                 110
gtg ggt acc aag ctg gac ctt cgc gat gac aag gac acc atc gag aag      384
Val Gly Thr Lys Leu Asp Leu Arg Asp Asp Lys Asp Thr Ile Glu Lys
        115                 120                 125
ctg aag gag aag aag ctg gct ccc atc acc tac ccg cag ggc ctg gca      432
Leu Lys Glu Lys Lys Leu Ala Pro Ile Thr Tyr Pro Gln Gly Leu Ala
    130                 135                 140
ctg gcc aag gat att gat tca gtc aag tac ttg gaa tgt tct gca ctc      480
Leu Ala Lys Asp Ile Asp Ser Val Lys Tyr Leu Glu Cys Ser Ala Leu
145                 150                 155                 160
acc cag cga ggc ctg aag acc gtc ttc gat gag gca atc cgc gca gtc      528
Thr Gln Arg Gly Leu Lys Thr Val Phe Asp Glu Ala Ile Arg Ala Val
                165                 170                 175
ctc tgc cca cag ccc aca cga cag cag aag cgc ccc tgc agc ctg ctc      576
Leu Cys Pro Gln Pro Thr Arg Gln Gln Lys Arg Pro Cys Ser Leu Leu
            180                 185                 190
tag                                                                  579

<210> 86
<211> 192
<212> PRT
<213> Mus musculus (house mouse)

<400> 86
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Ser
        35                  40                  45
Lys Pro Val Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Ser Pro Ala Ser Tyr Glu Asn Val Arg Ala Lys
                85                  90                  95
Trp Phe Pro Glu Val Arg His His Cys Pro Ser Thr Pro Ile Ile Leu
            100                 105                 110
Val Gly Thr Lys Leu Asp Leu Arg Asp Asp Lys Asp Thr Ile Glu Lys
        115                 120                 125
Leu Lys Glu Lys Lys Leu Ala Pro Ile Thr Tyr Pro Gln Gly Leu Ala
    130                 135                 140
Leu Ala Lys Asp Ile Asp Ser Val Lys Tyr Leu Glu Cys Ser Ala Leu
145                 150                 155                 160
Thr Gln Arg Gly Leu Lys Thr Val Phe Asp Glu Ala Ile Arg Ala Val
                165                 170                 175
Leu Cys Pro Gln Pro Thr Arg Gln Gln Lys Arg Pro Cys Ser Leu Leu
            180                 185                 190

<210> 87
<211> 840
<212> DNA
<213> Discopyge ommata

<220>
<221> CDS
<222> (97)..(675)

<400> 87
gaccggcgcg ccattggacg gtcgctaggc ggaggcggaa gggattggtt gccgtgcgac 60

gtcagtcagg tgcttcagga gcgacggggc acagcg atg gca gcg atc cgg aag 114
Met Ala Ala Ile Arg Lys
1               5
aag ctc gtc atc gtt ggc gat gga gct tgt ggc aag acc tgc ctc ctg 162
Lys Leu Val Ile Val Gly Asp Gly Ala Cys Gly Lys Thr Cys Leu Leu
            10                  15                  20
atc gtc ttc agc aag gac cag ttc ccg gag gtc tac gtc ccc act gtc 210
Ile Val Phe Ser Lys Asp Gln Phe Pro Glu Val Tyr Val Pro Thr Val
        25                  30                  35
ttc gag aac tac gtg gca gac atc gag gtg gac ggg aag cag gtg gag 258
Phe Glu Asn Tyr Val Ala Asp Ile Glu Val Asp Gly Lys Gln Val Glu
    40                  45                  50
ctc gct ctc tgg gac acg gct gga cag gag gac tat gac cgc ctg cga 306
Leu Ala Leu Trp Asp Thr Ala Gly Gln Glu Asp Tyr Asp Arg Leu Arg
55                  60                  65                  70
ccc ctc tcc tac cct gac acc gac gtc att ctg atg tgc ttt tct atc 354
Pro Leu Ser Tyr Pro Asp Thr Asp Val Ile Leu Met Cys Phe Ser Ile
                75                  80                  85
gac agc ccc gac agc ttg gag aac att ccg gag aag tgg acc ccg gag 402
Asp Ser Pro Asp Ser Leu Glu Asn Ile Pro Glu Lys Trp Thr Pro Glu
            90                  95                  100
gtg aag cac ttc tgc ccc aac atc ccc atc atc ctg gtg ggc aac aag 450
Val Lys His Phe Cys Pro Asn Ile Pro Ile Ile Leu Val Gly Asn Lys
        105                 110                 115
aag act gcg ggg gac gag cac aca cgg agg gag cta gcc aag atg aag 498
Lys Thr Ala Gly Asp Glu His Thr Arg Arg Glu Leu Ala Lys Met Lys
    120                 125                 130
cag gag cct gtc aaa cca gat gat gcc aag gag atg ggg agc cgc atc 546
Gln Glu Pro Val Lys Pro Asp Asp Ala Lys Glu Met Gly Ser Arg Ile
135                 140                 145                 150
aaa gcc ttt ggc tac ctg gaa tgc tca gcg aag acg aag gaa ggc gtg 594
Lys Ala Phe Gly Tyr Leu Glu Cys Ser Ala Lys Thr Lys Glu Gly Val
                155                 160                 165
cgc gag gtc ttt gag ctg gcc tcg cgc gct gct ctg cag gcc aag aaa 642
Arg Glu Val Phe Glu Leu Ala Ser Arg Ala Ala Leu Gln Ala Lys Lys
            170                 175                 180
acc aag agc aag agc ccc tgt ctg ctt ctg tgagcaggag ctcccagcta 692
Thr Lys Ser Lys Ser Pro Cys Leu Leu Leu
        185                 190
tccccacttc tctctgcttc tgctcactct ccgtcctccc cctctcccat tccttctctt 752

tcctccctcg ttcttccctc tcttgtttct ccccatctct ctcctccctc tcgttctttc 812

ctctcctctt gttttcctcc ttgagcgc 840

<210> 88
<211> 192
<212> PRT
<213> Discopyge ommata

<400> 88
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu
    50                  55                  60

Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Lys His Phe Cys Pro Asn Ile Pro Ile
            100                 105                 110
Ile Leu Val Gly Asn Lys Lys Thr Ala Gly Asp Glu His Thr Arg Arg
        115                 120                 125
Glu Leu Ala Lys Met Lys Gln Glu Pro Val Lys Pro Asp Asp Ala Lys
    130                 135                 140
Glu Met Gly Ser Arg Ile Lys Ala Phe Gly Tyr Leu Glu Cys Ser Ala
145                 150                 155                 160
Lys Thr Lys Glu Gly Val Arg Glu Val Phe Glu Leu Ala Ser Arg Ala
                165                 170                 175
Ala Leu Gln Ala Lys Lys Thr Lys Ser Lys Ser Pro Cys Leu Leu Leu
            180                 185                 190

<210> 89
<211> 697
<212> DNA
<213> Canis lupus familiaris (dog)

<220>
<221> CDS
<222> (1)..(582)

<400> 89
atg gct gcc atc cgg aag aaa ctg gtg att gtt ggt gat gga gcc tgt      48
Met Ala Ala Ile Arg Lys Lys Leu Val Ile Val Gly Asp Gly Ala Cys
1               5                   10                  15
ggt aag act tgt ttg ctc atc gtc ttt agc aag gac cag ttc cca gag      96
Gly Lys Thr Cys Leu Leu Ile Val Phe Ser Lys Asp Gln Phe Pro Glu
            20                  25                  30
gtg tat gta ccc aca gtg ttt gag aac tat gtg gca gat att gaa gtt      144
Val Tyr Val Pro Thr Val Phe Glu Asn Tyr Val Ala Asp Ile Glu Val
        35                  40                  45
gat gga aag cag gta gag ttg gct ttg tgg gat aca gct ggg cag gaa      192
Asp Gly Lys Gln Val Glu Leu Ala Leu Trp Asp Thr Ala Gly Gln Glu
    50                  55                  60
gat tat gat cgc ttg agg cct ctc tcc tat cca gac act gat gtt ata      240
Asp Tyr Asp Arg Leu Arg Pro Leu Ser Tyr Pro Asp Thr Asp Val Ile
65                  70                  75                  80
ctg atg tgt ttc tct att gac agc cct gat agc tta gaa aac atc cca      288
Leu Met Cys Phe Ser Ile Asp Ser Pro Asp Ser Leu Glu Asn Ile Pro
                85                  90                  95
gaa aaa tgg acc cca gaa gtc aag cac ttc tgt ccc aac gtg ccc atc      336
Glu Lys Trp Thr Pro Glu Val Lys His Phe Cys Pro Asn Val Pro Ile
            100                 105                 110
atc ctg gtt ggg aac aag aag gat ctt cgg aat gat gag cac aca agg      384
Ile Leu Val Gly Asn Lys Lys Asp Leu Arg Asn Asp Glu His Thr Arg
        115                 120                 125
cgg gag cta gcc aag atg aag cag gag ccg gtg aaa ccg aca gaa ggc      432
Arg Glu Leu Ala Lys Met Lys Gln Glu Pro Val Lys Pro Thr Glu Gly
    130                 135                 140
aga gat atg gca aac agg att ggt gct ttt ggg tac atg gag tgt tca      480
Arg Asp Met Ala Asn Arg Ile Gly Ala Phe Gly Tyr Met Glu Cys Ser
145                 150                 155                 160
gca aag acc aaa gat gga gtg agg gag gtt ttt gaa atg gcc acg aga      528
Ala Lys Thr Lys Asp Gly Val Arg Glu Val Phe Glu Met Ala Thr Arg
                165                 170                 175
gct gct ctg caa gcc aga cgt ggg aag aaa aaa tct ggg tgc ctt gtc      576
Ala Ala Leu Gln Ala Arg Arg Gly Lys Lys Lys Ser Gly Cys Leu Val
            180                 185                 190
ttg tgaaaccctg ctgcaagcac agccctcatg cggttaattt tgaagtgctg      629
Leu

tttattaatc ttagtgtatg attactggcc tttttcattt atctataatt tacctaagat      689

tacaaatc                                                          697

<210> 90
<211> 193
<212> PRT
<213> Canis lupus familiaris (dog)

<400> 90
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Lys His Phe Cys Pro Asn Val Pro Ile
            100                 105                 110
Ile Leu Val Gly Asn Lys Lys Asp Leu Arg Asn Asp Glu His Thr Arg
        115                 120                 125
Arg Glu Leu Ala Lys Met Lys Gln Glu Pro Val Lys Pro Thr Glu Gly
    130                 135                 140
Arg Asp Met Ala Asn Arg Ile Gly Ala Phe Gly Tyr Met Glu Cys Ser
145                 150                 155                 160
Ala Lys Thr Lys Asp Gly Val Arg Glu Val Phe Glu Met Ala Thr Arg
                165                 170                 175
Ala Ala Leu Gln Ala Arg Arg Gly Lys Lys Lys Ser Gly Cys Leu Val
            180                 185                 190
Leu


<210> 91
<211> 705
<212> DNA
<213> Drosophila melanogaster

<220>
<221> CDS
<222> (25)..(603)

<400> 91
tagcactcag tcagaaatcg aacc atg cag gcg atc aag tgc gtc gtc gtg      51
                           Met Gln Ala Ile Lys Cys Val Val Val
                           1               5

ggc gac gga gcc gtg gga aag acc tgc ctg ctg atc agc tac acg acc      99
Gly Asp Gly Ala Val Gly Lys Thr Cys Leu Leu Ile Ser Tyr Thr Thr
10                  15                  20                  25

aat gcc ttt ccc ggc gag tac ata ccc acc gtg ttc gac aac tac tcg     147
Asn Ala Phe Pro Gly Glu Tyr Ile Pro Thr Val Phe Asp Asn Tyr Ser
                30                  35                  40

gcc aac gtg atg gtg gac gcc aag ccc atc aac ctg ggc ctg tgg gat     195
Ala Asn Val Met Val Asp Ala Lys Pro Ile Asn Leu Gly Leu Trp Asp
            45                  50                  55

acg gcc ggg cag gag gac tac gac cga ctg agg cca ctg tct tat ccc     243
Thr Ala Gly Gln Glu Asp Tyr Asp Arg Leu Arg Pro Leu Ser Tyr Pro
        60                  65                  70

cag acc gat gtc ttc ctc atc tgc ttc tcg ctg gtg aat ccg gca tcg     291
Gln Thr Asp Val Phe Leu Ile Cys Phe Ser Leu Val Asn Pro Ala Ser
    75                  80                  85

ttc gag aac gtg cgg gcc aag tgg tat ccg gag gtg cgc cac cac tgc     339

Phe Glu Asn Val Arg Ala Lys Trp Tyr Pro Glu Val Arg His His Cys
90                  95                  100                 105
ccc agc acg ccc atc atc ctg gtg ggc acc aag ctg gat ttg cgc gac     387
Pro Ser Thr Pro Ile Ile Leu Val Gly Thr Lys Leu Asp Leu Arg Asp
                110                 115                 120
gac aag aac aca atc gaa aag ctg agg gac aag aaa ctg gtg ccc atc     435
Asp Lys Asn Thr Ile Glu Lys Leu Arg Asp Lys Lys Leu Val Pro Ile
            125                 130                 135
acc tat ccg cag ggc ctg gcc atg gcc aag gaa atc gga gcg gtc aag     483
Thr Tyr Pro Gln Gly Leu Ala Met Ala Lys Glu Ile Gly Ala Val Lys
        140                 145                 150
tat ctg gag tgc tcg gcc ctg acg cag aag ggt ctg aaa acc gtt ttc     531
Tyr Leu Glu Cys Ser Ala Leu Thr Gln Lys Gly Leu Lys Thr Val Phe
    155                 160                 165
gac gag gcc atc cgg tcg gtt ttg tgc ccc gtg ctg cag ccc aag tcc     579
Asp Glu Ala Ile Arg Ser Val Leu Cys Pro Val Leu Gln Pro Lys Ser
170                 175                 180                 185
aag cgc aag tgc gcc ctg ctc taaaagagtt agacagttgg tcggtggtgt     630
Lys Arg Lys Cys Ala Leu Leu
                190
tccaccgcag cgactcctgc ttaaacgcta cactactcta ctctaaccta taataaccat     690

tatgtaacct aagcc                                                    705

<210> 92
<211> 192
<212> PRT
<213> Drosophila melanogaster

<400> 92
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Ala
        35                  40                  45
Lys Pro Ile Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Asn Pro Ala Ser Phe Glu Asn Val Arg Ala Lys
                85                  90                  95
Trp Tyr Pro Glu Val Arg His His Cys Pro Ser Thr Pro Ile Ile Leu
            100                 105                 110
Val Gly Thr Lys Leu Asp Leu Arg Asp Asp Lys Asn Thr Ile Glu Lys
        115                 120                 125
Leu Arg Asp Lys Lys Leu Val Pro Ile Thr Tyr Pro Gln Gly Leu Ala
    130                 135                 140
Met Ala Lys Glu Ile Gly Ala Val Lys Tyr Leu Glu Cys Ser Ala Leu
145                 150                 155                 160
Thr Gln Lys Gly Leu Lys Thr Val Phe Asp Glu Ala Ile Arg Ser Val
                165                 170                 175
Leu Cys Pro Val Leu Gln Pro Lys Ser Lys Arg Lys Cys Ala Leu Leu
            180                 185                 190

<210> 93
<211> 801
<212> DNA
<213> Drosophila melanogaster

<220>
<221> CDS
<222> (160)..(735)

<400> 93
ctcgcacgca aagagctcgc tggagagaga gaacgctaac acaaaaggaa atagtagttt      60

tccgtacgta tcgagcccag cccagaaaag cggaaaacta aacggattcg acagcggttg      120

caaattttaa ataactgagt catcacccct ctgcacaac atg caa acc atc aag      174
                                           Met Gln Thr Ile Lys
                                           1               5
tgc gtg gtc gtc ggc gac gga gcc gtg ggt aag aca tgc ctg ctc atc      222
Cys Val Val Val Gly Asp Gly Ala Val Gly Lys Thr Cys Leu Leu Ile
                10                  15                  20
tcg tat aca acc aac aag ttc ccg tcg gag tac gtg ccc acg gtg ttc      270
Ser Tyr Thr Thr Asn Lys Phe Pro Ser Glu Tyr Val Pro Thr Val Phe
            25                  30                  35
gac aac tat gcg gtc act gtg atg atc ggc ggt gag ccc tac aca ctg      318
Asp Asn Tyr Ala Val Thr Val Met Ile Gly Gly Glu Pro Tyr Thr Leu
        40                  45                  50
ggc ctg ttc gat acg gcc gga cag gag gat tac gat cgg ctg cgt ccg      366
Gly Leu Phe Asp Thr Ala Gly Gln Glu Asp Tyr Asp Arg Leu Arg Pro
    55                  60                  65
ctc tcc tat ccg cag acg gat gtc ttc ctt gtc tgc ttt tcg gtg gtc      414
Leu Ser Tyr Pro Gln Thr Asp Val Phe Leu Val Cys Phe Ser Val Val
70                  75                  80                  85
agt ccc agt tcc ttc gag aac gtc aag gag aag tgg gtg ccc gag att      462
Ser Pro Ser Ser Phe Glu Asn Val Lys Glu Lys Trp Val Pro Glu Ile
                90                  95                  100
aca cac cat tgc caa aag acg ccg ttc ctg ctg gtg ggc aca cag att      510
Thr His His Cys Gln Lys Thr Pro Phe Leu Leu Val Gly Thr Gln Ile
            105                 110                 115
gat ttg cgc gac gag aac agc acg ctg gag aag ctg gcc aag aac aag      558
Asp Leu Arg Asp Glu Asn Ser Thr Leu Glu Lys Leu Ala Lys Asn Lys
        120                 125                 130
cag aag ccc atc acc atg gag cag ggc gag aag ctg gcc aag gag ctg      606
Gln Lys Pro Ile Thr Met Glu Gln Gly Glu Lys Leu Ala Lys Glu Leu
    135                 140                 145
aag gcc gtc aag tac gtg gag tgc tcg gcc ttg aca cag aag ggc ctg      654
Lys Ala Val Lys Tyr Val Glu Cys Ser Ala Leu Thr Gln Lys Gly Leu
150                 155                 160                 165
aaa aat gta ttc gac gag gcc atc ctg gcc gcc ctc gag cca cca gag      702
Lys Asn Val Phe Asp Glu Ala Ile Leu Ala Ala Leu Glu Pro Pro Glu
                170                 175                 180
ccc aca aag aaa agg aag tgc aaa ttc tta taattctgct ttcctcacac      752
Pro Thr Lys Lys Arg Lys Cys Lys Phe Leu
            185                 190
gcggtatccc tttcattctg gctttctact atcgcaatga ctccttccc      801



<210> 94
<211> 191
<212> PRT
<213> Drosophila melanogaster

<400> 94
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Glu Tyr
            20                  25                  30
Val Pro Thr Val Phe Asp Asn Tyr Ala Val Thr Val Met Ile Gly Gly
        35                  40                  45
Glu Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Val Ser Pro Ser Ser Phe Glu Asn Val Lys Glu Lys

85                  90                  95
Trp Val Pro Glu Ile Thr His His Cys Gln Lys Thr Pro Phe Leu Leu
            100                 105                 110
Val Gly Thr Gln Ile Asp Leu Arg Asp Glu Asn Ser Thr Leu Glu Lys
        115                 120                 125
Leu Ala Lys Asn Lys Gln Lys Pro Ile Thr Met Glu Gln Gly Glu Lys
    130                 135                 140
Leu Ala Lys Glu Leu Lys Ala Val Lys Tyr Val Glu Cys Ser Ala Leu
145                 150                 155                 160
Thr Gln Lys Gly Leu Lys Asn Val Phe Asp Glu Ala Ile Leu Ala Ala
                165                 170                 175
Leu Glu Pro Pro Glu Pro Thr Lys Lys Arg Lys Cys Lys Phe Leu
            180                 185                 190

<210> 95
<211> 609
<212> DNA
<213> Schizosaccharomyces pombe

<220>
<221> CDS
<222> (1)..(609)

<400> 95
atg gcg aca gaa ctt cgc aga aag ctc gtt att gtg gga gat ggt gca      48
Met Ala Thr Glu Leu Arg Arg Lys Leu Val Ile Val Gly Asp Gly Ala
1               5                   10                  15
tgt ggt aaa aca tgc tta tta att gta ttt tct aaa gga acc ttt ccc      96
Cys Gly Lys Thr Cys Leu Leu Ile Val Phe Ser Lys Gly Thr Phe Pro
            20                  25                  30
gag gtc tat gtt ccc act gtt ttt gaa aat tat gta gct gat gtt gag      144
Glu Val Tyr Val Pro Thr Val Phe Glu Asn Tyr Val Ala Asp Val Glu
        35                  40                  45
gtt gat gga cgc cac gtt gag ttg gct ctt tgg gat acg gct gga caa      192
Val Asp Gly Arg His Val Glu Leu Ala Leu Trp Asp Thr Ala Gly Gln
    50                  55                  60
gaa gat tac gac cgt cta cgt ccc ttg tca tat cct gac tca cat gtt      240
Glu Asp Tyr Asp Arg Leu Arg Pro Leu Ser Tyr Pro Asp Ser His Val
65                  70                  75                  80
atc ctt att tgc ttt gct gtt gat tct ccc gat tct ctt gac aat gtt      288
Ile Leu Ile Cys Phe Ala Val Asp Ser Pro Asp Ser Leu Asp Asn Val
                85                  90                  95
caa gaa aaa tgg att tcc gag gtt ctc cat ttc tgt tcc agt ctt cct      336
Gln Glu Lys Trp Ile Ser Glu Val Leu His Phe Cys Ser Ser Leu Pro
            100                 105                 110
att ttg ctt gtc gct tgc aag gct gat ctc cgt aac gac cca aaa att      384
Ile Leu Leu Val Ala Cys Lys Ala Asp Leu Arg Asn Asp Pro Lys Ile
        115                 120                 125
att gag gag tta tcc aag act aat cag cat ccc gtc acc aca gaa gaa      432
Ile Glu Glu Leu Ser Lys Thr Asn Gln His Pro Val Thr Thr Glu Glu
    130                 135                 140
ggt caa gca gta gct cag aag att ggt gct tac aaa tac ctt gag tgt      480
Gly Gln Ala Val Ala Gln Lys Ile Gly Ala Tyr Lys Tyr Leu Glu Cys
145                 150                 155                 160
tct gcc aag acg aat gaa ggt gtt cgt gag gtt ttt gaa tca gcc act      528
Ser Ala Lys Thr Asn Glu Gly Val Arg Glu Val Phe Glu Ser Ala Thr
                165                 170                 175
cgt gct gct atg ctc aaa cac aag ccc aaa gtg aag ccc tct agt gga      576
Arg Ala Ala Met Leu Lys His Lys Pro Lys Val Lys Pro Ser Ser Gly
            180                 185                 190
act aag aag aag aag cgt tgt atc ttg ttg taa                          609
Thr Lys Lys Lys Lys Arg Cys Ile Leu Leu
        195                 200

<210> 96
<211> 202
<212> PRT

<213> Schizosaccharomyces pombe

<400> 96
Met Ala Thr Glu Leu Arg Arg Lys Leu Val Ile Val Gly Asp Gly Ala
1               5                   10                  15
Cys Gly Lys Thr Cys Leu Leu Ile Val Phe Ser Lys Gly Thr Phe Pro
            20                  25                  30
Glu Val Tyr Val Pro Thr Val Phe Glu Asn Tyr Val Ala Asp Val Glu
        35                  40                  45
Val Asp Gly Arg His Val Glu Leu Ala Leu Trp Asp Thr Ala Gly Gln
    50                  55                  60
Glu Asp Tyr Asp Arg Leu Arg Pro Leu Ser Tyr Pro Asp Ser His Val
65                  70                  75                  80
Ile Leu Ile Cys Phe Ala Val Asp Ser Pro Asp Ser Leu Asp Asn Val
                85                  90                  95
Gln Glu Lys Trp Ile Ser Glu Val Leu His Phe Cys Ser Ser Leu Pro
            100                 105                 110
Ile Leu Leu Val Ala Cys Lys Ala Asp Leu Arg Asn Asp Pro Lys Ile
        115                 120                 125
Ile Glu Glu Leu Ser Lys Thr Asn Gln His Pro Val Thr Thr Glu Glu
    130                 135                 140
Gly Gln Ala Val Ala Gln Lys Ile Gly Ala Tyr Lys Tyr Leu Glu Cys
145                 150                 155                 160
Ser Ala Lys Thr Asn Glu Gly Val Arg Glu Val Phe Glu Ser Ala Thr
                165                 170                 175
Arg Ala Ala Met Leu Lys His Lys Pro Lys Val Lys Pro Ser Ser Gly
            180                 185                 190
Thr Lys Lys Lys Lys Arg Cys Ile Leu Leu
        195                 200

<210> 97
<211> 603
<212> DNA
<213> Schizosaccharomyces pombe

<220>
<221> CDS
<222> (1)..(603)

<400> 97
atg ttg caa tct caa ccg att aga agg aaa cta gta gtt gta ggc gat      48
Met Leu Gln Ser Gln Pro Ile Arg Arg Lys Leu Val Val Val Gly Asp
1               5                   10                  15
ggt gcc tgc ggg aag acg tct ttg tta tcc gtt ttc act ttg gga tat      96
Gly Ala Cys Gly Lys Thr Ser Leu Leu Ser Val Phe Thr Leu Gly Tyr
            20                  25                  30
ttt cct act gaa tat gtt ccg act gtg ttt gaa aac tat gtt tca gat      144
Phe Pro Thr Glu Tyr Val Pro Thr Val Phe Glu Asn Tyr Val Ser Asp
        35                  40                  45
tgt cga gtt gat gga aaa tca gtt caa ctg gca ttg tgg gat acc gca      192
Cys Arg Val Asp Gly Lys Ser Val Gln Leu Ala Leu Trp Asp Thr Ala
    50                  55                  60
ggt caa gag gaa tat gag aga tta aga cca atg tct tac gct aaa gca      240
Gly Gln Glu Glu Tyr Glu Arg Leu Arg Pro Met Ser Tyr Ala Lys Ala
65                  70                  75                  80
cac att att ttg gtt ggg ttt gcg ata gac tct cct gat tca ctt gaa      288
His Ile Ile Leu Val Gly Phe Ala Ile Asp Ser Pro Asp Ser Leu Glu
                85                  90                  95
aat gtt tct acg aag tgg att gag gaa att aat aca ctt tgc cca aat      336
Asn Val Ser Thr Lys Trp Ile Glu Glu Ile Asn Thr Leu Cys Pro Asn
            100                 105                 110
gtt ccg ttt att tta gtg ggc atg aaa gcc gac tta agg tca gat ccc      384
Val Pro Phe Ile Leu Val Gly Met Lys Ala Asp Leu Arg Ser Asp Pro
        115                 120                 125
gtt gct att gaa gaa atg cga cgt cga aat caa aat ttt gta aaa tca      432
Val Ala Ile Glu Glu Met Arg Arg Arg Asn Gln Asn Phe Val Lys Ser
    130                 135                 140
caa cag gct gaa tta gta gct cag cgg att ggt gcg aga aag tat atg      480

Gln Gln Ala Glu Leu Val Ala Gln Arg Ile Gly Ala Arg Lys Tyr Met
145                 150                 155                 160
gaa tgt tct tca ttg act ggt gac ggc gtg gac gat gta ttt gaa gct      528
Glu Cys Ser Ser Leu Thr Gly Asp Gly Val Asp Asp Val Phe Glu Ala
                165                 170                 175
gct act agg gca gca cta aca gtt cgg gat tcg gaa aat gac aag agt      576
Ala Thr Arg Ala Ala Leu Thr Val Arg Asp Ser Glu Asn Asp Lys Ser
            180                 185                 190
tct aca aaa tgc tgc atc att tca taa                                  603
Ser Thr Lys Cys Cys Ile Ile Ser
        195                 200

<210> 98
<211> 200
<212> PRT
<213> Schizosaccharomyces pombe

<400> 98
Met Leu Gln Ser Gln Pro Ile Arg Arg Lys Leu Val Val Val Gly Asp
1               5                   10                  15
Gly Ala Cys Gly Lys Thr Ser Leu Leu Ser Val Phe Thr Leu Gly Tyr
            20                  25                  30
Phe Pro Thr Glu Tyr Val Pro Thr Val Phe Glu Asn Tyr Val Ser Asp
        35                  40                  45
Cys Arg Val Asp Gly Lys Ser Val Gln Leu Ala Leu Trp Asp Thr Ala
    50                  55                  60
Gly Gln Glu Glu Tyr Glu Arg Leu Arg Pro Met Ser Tyr Ala Lys Ala
65                  70                  75                  80
His Ile Ile Leu Val Gly Phe Ala Ile Asp Ser Pro Asp Ser Leu Glu
                85                  90                  95
Asn Val Ser Thr Lys Trp Ile Glu Glu Ile Asn Thr Leu Cys Pro Asn
            100                 105                 110
Val Pro Phe Ile Leu Val Gly Met Lys Ala Asp Leu Arg Ser Asp Pro
        115                 120                 125
Val Ala Ile Glu Glu Met Arg Arg Arg Asn Gln Asn Phe Val Lys Ser
    130                 135                 140
Gln Gln Ala Glu Leu Val Ala Gln Arg Ile Gly Ala Arg Lys Tyr Met
145                 150                 155                 160
Glu Cys Ser Ser Leu Thr Gly Asp Gly Val Asp Asp Val Phe Glu Ala
                165                 170                 175
Ala Thr Arg Ala Ala Leu Thr Val Arg Asp Ser Glu Asn Asp Lys Ser
            180                 185                 190
Ser Thr Lys Cys Cys Ile Ile Ser
        195                 200

<210> 99
<211> 726
<212> DNA
<213> Entamoeba histolytica

<220>
<221> CDS
<222> (97)..(687)

<400> 99
aaatgtaaag aacattaaaa ataaaaaaac agtgaaaaga aatggattaa aaatagaatt      60

tcaaggaact gaaactcgta attcaaaaga aaacac atg caa gct gtc aaa tgt      114
                                        Met Gln Ala Val Lys Cys
                                        1               5
gtc att gtt ggg gat gga gct gta gga aaa act tgt ctt tta att tct      162
Val Ile Val Gly Asp Gly Ala Val Gly Lys Thr Cys Leu Leu Ile Ser
            10                  15                  20
tac act aca aat gca ttt cct aat gaa tat att cca aca gta ttt gat      210
Tyr Thr Thr Asn Ala Phe Pro Asn Glu Tyr Ile Pro Thr Val Phe Asp
        25                  30                  35

aat tat tct gct act gtt atg gtt gat tca aaa cca att aat ctt gga 258
Asn Tyr Ser Ala Thr Val Met Val Asp Ser Lys Pro Ile Asn Leu Gly
    40                  45                  50
ctt tgg gat act gct gga caa gaa gat tat gat aga tta aga cca ttg 306
Leu Trp Asp Thr Ala Gly Gln Glu Asp Tyr Asp Arg Leu Arg Pro Leu
55                  60                  65                  70
tca tat cca caa acc gat gta ttc tta att tgt ttc tca gtt gtt tct 354
Ser Tyr Pro Gln Thr Asp Val Phe Leu Ile Cys Phe Ser Val Val Ser
                75                  80                  85
cca cca tca ttt gat aat gtc tca tct aaa tgg caa cca gaa gtt tct 402
Pro Pro Ser Phe Asp Asn Val Ser Ser Lys Trp Gln Pro Glu Val Ser
            90                  95                  100
cat cat tgt cca aag act cct tgt tta tta gtt gga act aaa ctt gat 450
His His Cys Pro Lys Thr Pro Cys Leu Leu Val Gly Thr Lys Leu Asp
        105                 110                 115
atg aga gaa gat aaa gaa caa tta aag aga tta gaa gag aaa aaa att 498
Met Arg Glu Asp Lys Glu Gln Leu Lys Arg Leu Glu Glu Lys Lys Ile
    120                 125                 130
act cca atc act act gaa caa ggt gag gct aaa tgc aaa gat att gga 546
Thr Pro Ile Thr Thr Glu Gln Gly Glu Ala Lys Cys Lys Asp Ile Gly
135                 140                 145                 150
gct gtt aag tat atc gaa tgt tct gct tta act caa aag aat tta aga 594
Ala Val Lys Tyr Ile Glu Cys Ser Ala Leu Thr Gln Lys Asn Leu Arg
                155                 160                 165
ctt gtt ttt gat gaa gct gtt aga gca gtt att tct cct gca ggt gga 642
Leu Val Phe Asp Glu Ala Val Arg Ala Val Ile Ser Pro Ala Gly Gly
            170                 175                 180
gca aaa aag gat aaa aag aat aat aga gga tgt tta tta ttc taaattcgat 694
Ala Lys Lys Asp Lys Lys Asn Asn Arg Gly Cys Leu Leu Phe
        185                 190                 195
tcattccttt tttaaacaac ttacaaacaa ta 726

<210> 100
<211> 196
<212> PRT
<213> Entamoeba histolytica

<400> 100
Met Gln Ala Val Lys Cys Val Ile Val Gly Asp Gly Ala Val Gly Lys
1               5                   10                  15
Thr Cys Leu Leu Ile Ser Tyr Thr Thr Asn Ala Phe Pro Asn Glu Tyr
            20                  25                  30
Ile Pro Thr Val Phe Asp Asn Tyr Ser Ala Thr Val Met Val Asp Ser
        35                  40                  45
Lys Pro Ile Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Val Val Ser Pro Pro Ser Phe Asp Asn Val Ser Ser Lys
                85                  90                  95
Trp Gln Pro Glu Val Ser His His Cys Pro Lys Thr Pro Cys Leu Leu
            100                 105                 110
Val Gly Thr Lys Leu Asp Met Arg Glu Asp Lys Glu Gln Leu Lys Arg
        115                 120                 125
Leu Glu Glu Lys Lys Ile Thr Pro Ile Thr Thr Glu Gln Gly Glu Ala
    130                 135                 140
Lys Cys Lys Asp Ile Gly Ala Val Lys Tyr Ile Glu Cys Ser Ala Leu
145                 150                 155                 160
Thr Gln Lys Asn Leu Arg Leu Val Phe Asp Glu Ala Val Arg Ala Val
                165                 170                 175
Ile Ser Pro Ala Gly Gly Ala Lys Lys Asp Lys Lys Asn Asn Arg Gly
            180                 185                 190
Cys Leu Leu Phe
        195

<210> 101
<211> 744
<212> DNA
<213> Entamoeba histolytica

<220>
<221> CDS
<222> (145)..(729)

<400> 101
aaatataatc ttaaaataga aagaactgca aaaactaaaa aacataaaaa aagaaactct 60

agaaaagcta ttaaagtaac gaaaaaaaca tgaaacaaag aaataataga caactcagaa 120

ctaaaaaaag ttgaaagaaa atca atg agt gaa aaa ccc aca tca atc aaa 171
Met Ser Glu Lys Pro Thr Ser Ile Lys
1               5
tta gtc gtt gtt ggg gat ggt gct gtt gga aaa aca tgt tta tta att 219
Leu Val Val Val Gly Asp Gly Ala Val Gly Lys Thr Cys Leu Leu Ile
10                  15                  20                  25
tgc tat aca act aat gaa ttt cca aaa gat tat att cct aca gta ttc 267
Cys Tyr Thr Thr Asn Glu Phe Pro Lys Asp Tyr Ile Pro Thr Val Phe
                30                  35                  40
gat aat tat gtt gtg tca tta act gca ggc aca aga caa att caa ttg 315
Asp Asn Tyr Val Val Ser Leu Thr Ala Gly Thr Arg Gln Ile Gln Leu
            45                  50                  55
gcg ttg tgg gat act gct gga caa gaa gag tat gat caa ttg aga cca 363
Ala Leu Trp Asp Thr Ala Gly Gln Glu Glu Tyr Asp Gln Leu Arg Pro
        60                  65                  70
ttg agt tat tca tct gct tca ata ttt tta ata tgt ttc tct gtc aca 411
Leu Ser Tyr Ser Ser Ala Ser Ile Phe Leu Ile Cys Phe Ser Val Thr
    75                  80                  85
agt tct gta tca tat gat aat gta att aca aaa tgg cac cct gaa gtt 459
Ser Ser Val Ser Tyr Asp Asn Val Ile Thr Lys Trp His Pro Glu Val
90                  95                  100                 105
att cac ttt gca cca aaa gtt cct att ata tta gtt gga aca aaa ctt 507
Ile His Phe Ala Pro Lys Val Pro Ile Ile Leu Val Gly Thr Lys Leu
                110                 115                 120
gat act aga aat gat cct gct att gtt aaa aga tta aca gaa cag ggt 555
Asp Thr Arg Asn Asp Pro Ala Ile Val Lys Arg Leu Thr Glu Gln Gly
            125                 130                 135
atg act gtt att aat act gca aaa gga gaa gaa ctt aaa aat cgt ata 603
Met Thr Val Ile Asn Thr Ala Lys Gly Glu Glu Leu Lys Asn Arg Ile
        140                 145                 150
aaa gct gtc aaa tat att gaa tgt tct gca aaa aca agc gaa aat tta 651
Lys Ala Val Lys Tyr Ile Glu Cys Ser Ala Lys Thr Ser Glu Asn Leu
    155                 160                 165
aag aca gtt ttc gat gaa gca gtt aaa act gtg tta atg aat aaa cca 699
Lys Thr Val Phe Asp Glu Ala Val Lys Thr Val Leu Met Asn Lys Pro
170                 175                 180                 185
cag caa agg tca aag tgt gct ctt tta taattcactt ttaaatta 744
Gln Gln Arg Ser Lys Cys Ala Leu Leu
                190

<210> 102
<211> 194
<212> PRT
<213> Entamoeba histolytica

<400> 102
Met Ser Glu Lys Pro Thr Ser Ile Lys Leu Val Val Val Gly Asp Gly
1               5                   10                  15
Ala Val Gly Lys Thr Cys Leu Leu Ile Cys Tyr Thr Thr Asn Glu Phe
            20                  25                  30
Pro Lys Asp Tyr Ile Pro Thr Val Phe Asp Asn Tyr Val Val Ser Leu

35                  40                  45
Thr Ala Gly Thr Arg Gln Ile Gln Leu Ala Leu Trp Asp Thr Ala Gly
    50                  55                  60
Gln Glu Glu Tyr Asp Gln Leu Arg Pro Leu Ser Tyr Ser Ser Ala Ser
65                  70                  75                  80
Ile Phe Leu Ile Cys Phe Ser Val Thr Ser Ser Val Ser Tyr Asp Asn
                85                  90                  95
Val Ile Thr Lys Trp His Pro Glu Val Ile His Phe Ala Pro Lys Val
            100                 105                 110
Pro Ile Ile Leu Val Gly Thr Lys Leu Asp Thr Arg Asn Asp Pro Ala
        115                 120                 125
Ile Val Lys Arg Leu Thr Glu Gln Gly Met Thr Val Ile Asn Thr Ala
    130                 135                 140
Lys Gly Glu Glu Leu Lys Asn Arg Ile Lys Ala Val Lys Tyr Ile Glu
145                 150                 155                 160
Cys Ser Ala Lys Thr Ser Glu Asn Leu Lys Thr Val Phe Asp Glu Ala
                165                 170                 175
Val Lys Thr Val Leu Met Asn Lys Pro Gln Gln Arg Ser Lys Cys Ala
            180                 185                 190
Leu Leu

<210> 103
<211> 588
<212> DNA
<213> Caenorhabditis elegans

<220>
<221> CDS
<222> (1)..(588)

<400> 103
atg caa gca atc aaa tgt gtc gtc gtt ggt gac gga gcc gtc ggt aag      48
Met Gln Ala Ile Lys Cys Val Val Val Gly Asp Gly Ala Val Gly Lys
1               5                   10                  15
acg tgt ctc ctg cta tcg tac act aca aac gct ttt ccc gga gaa tat      96
Thr Cys Leu Leu Leu Ser Tyr Thr Thr Asn Ala Phe Pro Gly Glu Tyr
            20                  25                  30
att cta acg gta ttc gac acc tac tca aca aat gtg atg gtc gac gga      144
Ile Leu Thr Val Phe Asp Thr Tyr Ser Thr Asn Val Met Val Asp Gly
        35                  40                  45
agg cca ata aat ctc agc cta tgg gac aca gct gga cag gac gat tac      192
Arg Pro Ile Asn Leu Ser Leu Trp Asp Thr Ala Gly Gln Asp Asp Tyr
    50                  55                  60
gat caa ttc cgc cac ctg tca ttt cca caa aca gac gta ttc ctc gta      240
Asp Gln Phe Arg His Leu Ser Phe Pro Gln Thr Asp Val Phe Leu Val
65                  70                  75                  80
tgc ttt gca ctg aat aat cca gca agt ttt gag aat gtt cgt gca aaa      288
Cys Phe Ala Leu Asn Asn Pro Ala Ser Phe Glu Asn Val Arg Ala Lys
                85                  90                  95
tgg tat cca gaa gta tca cat cat tgc cca aat aca ccg att att ttg      336
Trp Tyr Pro Glu Val Ser His His Cys Pro Asn Thr Pro Ile Ile Leu
            100                 105                 110
gtt gga act aaa gct gat tta cgc gag gat cga gat act att gaa cgg      384
Val Gly Thr Lys Ala Asp Leu Arg Glu Asp Arg Asp Thr Ile Glu Arg
        115                 120                 125
ctc cgt gaa cgt cgg ctc caa cca gtg agc cac acc cag ggt tac gtg      432
Leu Arg Glu Arg Arg Leu Gln Pro Val Ser His Thr Gln Gly Tyr Val
    130                 135                 140
atg gca aag gaa atc aag gcg gtc aag tac ctg gaa tgc tcg gcg ctt      480
Met Ala Lys Glu Ile Lys Ala Val Lys Tyr Leu Glu Cys Ser Ala Leu
145                 150                 155                 160
acc caa att gga ttg aaa caa gtt ttc gat gag gca att cgt act ggg      528
Thr Gln Ile Gly Leu Lys Gln Val Phe Asp Glu Ala Ile Arg Thr Gly
                165                 170                 175
ctc acc ccg cca caa aca cca caa acg aga gcc aaa aag agc aat tgc      576
Leu Thr Pro Pro Gln Thr Pro Gln Thr Arg Ala Lys Lys Ser Asn Cys
            180                 185                 190

acg gtg ctt taa                                                    588
Thr Val Leu
        195

<210> 104
<211> 195
<212> PRT
<213> Caenorhabditis elegans

<400> 104
Met Gln Ala Ile Lys Cys Val Val Val Gly Asp Gly Ala Val Gly Lys
1               5                   10                  15
Thr Cys Leu Leu Leu Ser Tyr Thr Thr Asn Ala Phe Pro Gly Glu Tyr
            20                  25                  30
Ile Leu Thr Val Phe Asp Thr Tyr Ser Thr Asn Val Met Val Asp Gly
        35                  40                  45
Arg Pro Ile Asn Leu Ser Leu Trp Asp Thr Ala Gly Gln Asp Asp Tyr
    50                  55                  60
Asp Gln Phe Arg His Leu Ser Phe Pro Gln Thr Asp Val Phe Leu Val
65                  70                  75                  80
Cys Phe Ala Leu Asn Asn Pro Ala Ser Phe Glu Asn Val Arg Ala Lys
                85                  90                  95
Trp Tyr Pro Glu Val Ser His His Cys Pro Asn Thr Pro Ile Ile Leu
            100                 105                 110
Val Gly Thr Lys Ala Asp Leu Arg Glu Asp Arg Asp Thr Ile Glu Arg
        115                 120                 125
Leu Arg Glu Arg Arg Leu Gln Pro Val Ser His Thr Gln Gly Tyr Val
    130                 135                 140
Met Ala Lys Glu Ile Lys Ala Val Lys Tyr Leu Glu Cys Ser Ala Leu
145                 150                 155                 160
Thr Gln Ile Gly Leu Lys Gln Val Phe Asp Glu Ala Ile Arg Thr Gly
                165                 170                 175
Leu Thr Pro Pro Gln Thr Pro Gln Thr Arg Ala Lys Lys Ser Asn Cys
            180                 185                 190
Thr Val Leu
        195

<210> 105
<211> 594
<212> DNA
<213> Zea mays (maize)

<220>
<221> CDS
<222> (1)..(594)

<400> 105
atg agc gcg tcc agg ttc ata aag tgc gtc acg gtc ggg gac ggc gcc      48
Met Ser Ala Ser Arg Phe Ile Lys Cys Val Thr Val Gly Asp Gly Ala
1               5                   10                  15
gtc ggc aag acc tgc atg ctc atc tcc tac acc tcc aac acc ttc ccc      96
Val Gly Lys Thr Cys Met Leu Ile Ser Tyr Thr Ser Asn Thr Phe Pro
            20                  25                  30
acc gac tat gtt ccg aca gtg ttt gat aac ttc agt gcc aac gtt gtg      144
Thr Asp Tyr Val Pro Thr Val Phe Asp Asn Phe Ser Ala Asn Val Val
        35                  40                  45
gtt gat ggt aat act gtc aac ctc ggc ctc tgg gac act gca ggt caa      192
Val Asp Gly Asn Thr Val Asn Leu Gly Leu Trp Asp Thr Ala Gly Gln
    50                  55                  60
gag gat tac aac aga ctg aga cca ctg agc tat cgt gga gct gat gtt      240
Glu Asp Tyr Asn Arg Leu Arg Pro Leu Ser Tyr Arg Gly Ala Asp Val
65                  70                  75                  80
ttt ctt ctg gct ttc tca ctg atc agt aag gcc agc tat gag aat gtt      288
Phe Leu Leu Ala Phe Ser Leu Ile Ser Lys Ala Ser Tyr Glu Asn Val
                85                  90                  95
tcg aag aag tgg ata cct gaa ctg aag cat tat gca cct ggt gtg cca      336
Ser Lys Lys Trp Ile Pro Glu Leu Lys His Tyr Ala Pro Gly Val Pro

100                 105                 110
att att ctc gta ggg aca aag ctt gat ctt cga gac gac aag cag ttc      384
Ile Ile Leu Val Gly Thr Lys Leu Asp Leu Arg Asp Asp Lys Gln Phe
        115                 120                 125
ttt gtg gac cat cct ggt gct gtc cct atc act act gct cag gga gag      432
Phe Val Asp His Pro Gly Ala Val Pro Ile Thr Thr Ala Gln Gly Glu
    130                 135                 140
gag cta aga aag caa ata ggc gct cca tac tac atc gaa tgc agc tcg      480
Glu Leu Arg Lys Gln Ile Gly Ala Pro Tyr Tyr Ile Glu Cys Ser Ser
145                 150                 155                 160
aag acc caa cta aac gtg aag ggc gtc ttt gat gcg gcg ata aag gtt      528
Lys Thr Gln Leu Asn Val Lys Gly Val Phe Asp Ala Ala Ile Lys Val
                165                 170                 175
gtg ctg cag ccg cct aag gcg aag aag aag aaa aag gtg cag agg ggg      576
Val Leu Gln Pro Pro Lys Ala Lys Lys Lys Lys Lys Val Gln Arg Gly
            180                 185                 190
gcg tgc tcc att ttg tga                                              594
Ala Cys Ser Ile Leu
        195


<210> 106
<211> 197
<212> PRT
<213> Zea mays (maize)

<400> 106
Met Ser Ala Ser Arg Phe Ile Lys Cys Val Thr Val Gly Asp Gly Ala
1               5                   10                  15
Val Gly Lys Thr Cys Met Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Asn Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Ile Ile Leu Val Gly Thr Lys Leu Asp Leu Arg Asp Asp Lys Gln Phe
        115                 120                 125
Phe Val Asp His Pro Gly Ala Val Pro Ile Thr Thr Ala Gln Gly Glu
    130                 135                 140
Glu Leu Arg Lys Gln Ile Gly Ala Pro Tyr Tyr Ile Glu Cys Ser Ser
145                 150                 155                 160
Lys Thr Gln Leu Asn Val Lys Gly Val Phe Asp Ala Ala Ile Lys Val
                165                 170                 175
Val Leu Gln Pro Pro Lys Ala Lys Lys Lys Lys Lys Val Gln Arg Gly
            180                 185                 190
Ala Cys Ser Ile Leu
        195

<210> 107
<211> 1058
<212> DNA
<213> Zea mays (maize)

<220>
<221> CDS
<222> (176)..(769)

<400> 107
gaattcggca cgagagctct caagacggcc gacggccggc ttgcctacct gctcccatcc      60


ttcccgaggg accgagaaag ataagaaagg cggtggtcaa cttgtgtcct gaggtgcccg      120

tagaagccca aggacaagaa acaaggagaa gagtagatct acatctactc caccg atg     178
                                                              Met
                                                              1
agc gcg tct cgg ttc atc aag tgc gtc acc gtg ggg gac ggt gcc gtc     226
Ser Ala Ser Arg Phe Ile Lys Cys Val Thr Val Gly Asp Gly Ala Val
        5                   10                  15
gga aag acc tgc atg ctc atc tcc tac aca tcc aac act ttc ccc act     274
Gly Lys Thr Cys Met Leu Ile Ser Tyr Thr Ser Asn Thr Phe Pro Thr
    20                  25                  30
gac tat gtt cca act gtg ttc gac aac ttc agt gcc aat gtt gtg gtt     322
Asp Tyr Val Pro Thr Val Phe Asp Asn Phe Ser Ala Asn Val Val Val
    35                  40                  45
gac ggg agc act gtc aac ttg ggt ctg tgg gat aca gca gga caa gaa     370
Asp Gly Ser Thr Val Asn Leu Gly Leu Trp Asp Thr Ala Gly Gln Glu
50                  55                  60                  65
gat tac aat aga ctg cgt ccg ttg agc tat cgt ggt gct gat gtt ttt     418
Asp Tyr Asn Arg Leu Arg Pro Leu Ser Tyr Arg Gly Ala Asp Val Phe
                70                  75                  80
ctg ctc gcc ttt tct ctt atc agc aaa gca agc tat gag aat gtc tct     466
Leu Leu Ala Phe Ser Leu Ile Ser Lys Ala Ser Tyr Glu Asn Val Ser
            85                  90                  95
aag aag tgg gtt cct gaa tta agg cac tat gct cct ggc gtg ccc ata     514
Lys Lys Trp Val Pro Glu Leu Arg His Tyr Ala Pro Gly Val Pro Ile
        100                 105                 110
atc ctt gtt ggg aca aaa ctt gat ctg cgt gat gat aag cag ttt ttt     562
Ile Leu Val Gly Thr Lys Leu Asp Leu Arg Asp Asp Lys Gln Phe Phe
    115                 120                 125
gtt gat cac cct ggt gct gtt cca att tcc act gcc cag ggc gaa gag     610
Val Asp His Pro Gly Ala Val Pro Ile Ser Thr Ala Gln Gly Glu Glu
130                 135                 140                 145
ctg agg aag cta att ggt gct gcc gcc tac atc gaa tgc agt tca aaa     658
Leu Arg Lys Leu Ile Gly Ala Ala Ala Tyr Ile Glu Cys Ser Ser Lys
                150                 155                 160
atc cag cag aac ata aaa gca gtg ttt gac gca gca att aag gtg gtt     706
Ile Gln Gln Asn Ile Lys Ala Val Phe Asp Ala Ala Ile Lys Val Val
            165                 170                 175
ctc cag cca cca aag caa aag aag agg aag aag aag gtg cag aag gga     754
Leu Gln Pro Pro Lys Gln Lys Lys Arg Lys Lys Lys Val Gln Lys Gly
        180                 185                 190
tgc acc att ttg taactacaaa cggtagaggg caacagtctg gctgcggcgc         806
Cys Thr Ile Leu
    195
tgctgccaat gataaccatc gcctccttgc tgtataatat atcgcctgat catgccacca   866

gcatgcacaa gggagatggt ggttttagga tccttgtcct actgtgttgt gtagaccacc   926

gggtgtagtt gactgtatct ggttgtttgt atgtatggac aagacaaaac tagcactgca   986

gatggtatgg taaggcgtaa gcaaatacaa tatgacattg gtccagttcc aggaaaaaaa   1046

aaaaaaaaaa aa                                                       1058

<210> 108
<211> 197
<212> PRT
<213> Zea mays (maize)

<400> 108
Met Ser Ala Ser Arg Phe Ile Lys Cys Val Thr Val Gly Asp Gly Ala

1                   5                   10                  15
Val Gly Lys Thr Cys Met Leu Ile Ser Tyr Thr Ser Asn Thr Phe Pro
                20                  25                  30
Thr Asp Tyr Val Pro Thr Val Phe Asp Asn Phe Ser Ala Asn Val Val
            35                  40                  45
Val Asp Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Val
                85                  90                  95
Ser Lys Lys Trp Val Pro Glu Leu Arg His Tyr Ala Pro Gly Val Pro
            100                 105                 110
Ile Ile Leu Val Gly Thr Lys Leu Asp Leu Arg Asp Asp Lys Gln Phe
        115                 120                 125
Phe Val Asp His Pro Gly Ala Val Pro Ile Ser Thr Ala Gln Gly Glu
    130                 135                 140
Glu Leu Arg Lys Leu Ile Gly Ala Ala Ala Tyr Ile Glu Cys Ser Ser
145                 150                 155                 160
Lys Ile Gln Gln Asn Ile Lys Ala Val Phe Asp Ala Ala Ile Lys Val
                165                 170                 175
Val Leu Gln Pro Pro Lys Gln Lys Lys Arg Lys Lys Lys Val Gln Lys
            180                 185                 190
Gly Cys Thr Ile Leu
        195

<210> 109
<211> 1045
<212> DNA
<213> Zea mays (maize)

<220>
<221> CDS
<222> (45)..(707)

<400> 109
gaattcggca cgagctggct cgtgcagcgg cggcagtgag agcg atg agc gcg gcg      56
                                                 Met Ser Ala Ala
                                                 1

gca gcg gcg gcg gcg agc tcg gtc acc aag ttc atc aag tgc gtc acg      104
Ala Ala Ala Ala Ala Ser Ser Val Thr Lys Phe Ile Lys Cys Val Thr
5                   10                  15                  20

gtc ggc gat ggg gcc gtc ggg aag acc tgc atg ctc atc tgc tac acc      152
Val Gly Asp Gly Ala Val Gly Lys Thr Cys Met Leu Ile Cys Tyr Thr
                25                  30                  35

tgc aac aag ttc ccc acg gat tac atc ccc acc gta ttt gac aac ttc      200
Cys Asn Lys Phe Pro Thr Asp Tyr Ile Pro Thr Val Phe Asp Asn Phe
            40                  45                  50

agc gcc aat gtc tcc gtg ggt ggg agc atc gtc aac ttg ggc ctc tgg      248
Ser Ala Asn Val Ser Val Gly Gly Ser Ile Val Asn Leu Gly Leu Trp
        55                  60                  65

gac acg gca ggc cag gag gat tac agc agg ttg agg cct ctc agc tac      296
Asp Thr Ala Gly Gln Glu Asp Tyr Ser Arg Leu Arg Pro Leu Ser Tyr
    70                  75                  80

agg ggt gct gat gtg ttc atc ctc tcc ttc tcc ctg gtc agc agg gcg      344
Arg Gly Ala Asp Val Phe Ile Leu Ser Phe Ser Leu Val Ser Arg Ala
85                  90                  95                  100

agc tat gag aac gtc ctg aag aag tgg atg cca gag ctt cgc cga ttt      392
Ser Tyr Glu Asn Val Leu Lys Lys Trp Met Pro Glu Leu Arg Arg Phe
                105                 110                 115

tca cct act gtt cct gta gtt ctt gtt gga acc aaa cta gat ctc cgt      440
Ser Pro Thr Val Pro Val Val Leu Val Gly Thr Lys Leu Asp Leu Arg
            120                 125                 130

gaa gac aga tct tac ctt gct gac cat tct gct gct tcc atc atc tct      488
Glu Asp Arg Ser Tyr Leu Ala Asp His Ser Ala Ala Ser Ile Ile Ser
        135                 140                 145

act gaa cag gga gaa gag ctc agg aag cag ata ggt gct gtg gcg tac      536
Thr Glu Gln Gly Glu Glu Leu Arg Lys Gln Ile Gly Ala Val Ala Tyr

150                 155                 160
ata gaa tgc agc tca aag aca cag agg aac gta aag gct gtg ttc gac        584
Ile Glu Cys Ser Ser Lys Thr Gln Arg Asn Val Lys Ala Val Phe Asp
165                 170                 175                 180
act gca att aaa gta gtg ctg caa cca ccg agg aga aga gaa gtt acc        632
Thr Ala Ile Lys Val Val Leu Gln Pro Pro Arg Arg Arg Glu Val Thr
                185                 190                 195
agg aag aaa atg aag aca agt tcg aat cag tct ctg aga aga tac ctc        680
Arg Lys Lys Met Lys Thr Ser Ser Asn Gln Ser Leu Arg Arg Tyr Leu
            200                 205                 210
tgt gga agc gga tgt ttc aca tcg taaagcacag actcttctgc gactgttgta      734
Cys Gly Ser Gly Cys Phe Thr Ser
        215                 220
ctggacttgc tagatggttg cagctctatg aatgagtagt cccctccgca gccactggga      794

acttctggtt ctctgctacc ttccgataga gtgctctttt gcgttcacca gctgagaaaa      854

atgaagcgag gttctagttt ataaattccc tacgaggtgt accttcttta gtatgaatgg      914

tgggctattt agcagttcag caaagtgtga agtgaccctt ctatgcatgt tttgtttcca      974

aaaactgatg ttgctaaatg gctaatgaat ggttatggtc gcaccggaag aaaaaaaaaa      1034

aaaaaaaaaa a                                                           1045

<210> 110
<211> 220
<212> PRT
<213> Zea mays (maize)

<400> 110
Met Ser Ala Ala Ala Ala Ala Ala Ala Ser Ser Val Thr Lys Phe Ile
1               5                   10                  15
Lys Cys Val Thr Val Gly Asp Gly Ala Val Gly Lys Thr Cys Met Leu
            20                  25                  30
Ile Cys Tyr Thr Cys Asn Lys Phe Pro Thr Asp Tyr Ile Pro Thr Val
        35                  40                  45
Phe Asp Asn Phe Ser Ala Asn Val Ser Val Gly Gly Ser Ile Val Asn
    50                  55                  60
Leu Gly Leu Trp Asp Thr Ala Gly Gln Glu Asp Tyr Ser Arg Leu Arg
65                  70                  75                  80
Pro Leu Ser Tyr Arg Gly Ala Asp Val Phe Ile Leu Ser Phe Ser Leu
                85                  90                  95
Val Ser Arg Ala Ser Tyr Glu Asn Val Leu Lys Lys Trp Met Pro Glu
            100                 105                 110
Leu Arg Arg Phe Ser Pro Thr Val Pro Val Val Leu Val Gly Thr Lys
        115                 120                 125
Leu Asp Leu Arg Glu Asp Arg Ser Tyr Leu Ala Asp His Ser Ala Ala
    130                 135                 140
Ser Ile Ile Ser Thr Glu Gln Gly Glu Glu Leu Arg Lys Gln Ile Gly
145                 150                 155                 160
Ala Val Ala Tyr Ile Glu Cys Ser Ser Lys Thr Gln Arg Asn Val Lys
                165                 170                 175
Ala Val Phe Asp Thr Ala Ile Lys Val Val Leu Gln Pro Pro Arg Arg
            180                 185                 190
Arg Glu Val Thr Arg Lys Lys Met Lys Thr Ser Ser Asn Gln Ser Leu
        195                 200                 205
Arg Arg Tyr Leu Cys Gly Ser Gly Cys Phe Thr Ser
    210                 215                 220

<210> 111
<211> 876
<212> DNA
<213> Saccharomyces cerevisiae

<220>
<221> CDS
<222> (1)..(876)

<400> 111
atg aat aca cta tta ttt aag cga aaa ggt ggc aat tgt ggg aac gaa 48
Met Asn Thr Leu Leu Phe Lys Arg Lys Gly Gly Asn Cys Gly Asn Glu
1               5                   10                  15
agt aac ata gtt tcg cag gga tcg ccc tca agt agc aat ctt cct gaa 96
Ser Asn Ile Val Ser Gln Gly Ser Pro Ser Ser Ser Asn Leu Pro Glu
            20                  25                  30
tca cct ggc act tta gat gaa aag aat ctt ccc aga ttg cct act cca 144
Ser Pro Gly Thr Leu Asp Glu Lys Asn Leu Pro Arg Leu Pro Thr Pro
        35                  40                  45
ttc gct aga agc ctt tct acc att cct agt tat gag cag atg aaa cgt 192
Phe Ala Arg Ser Leu Ser Thr Ile Pro Ser Tyr Glu Gln Met Lys Arg
    50                  55                  60
aca aac aaa ctg cca gat tat cac cta aag att gtt gtt gtg gga gat 240
Thr Asn Lys Leu Pro Asp Tyr His Leu Lys Ile Val Val Val Gly Asp
65                  70                  75                  80
ggc gct gta ggg aag acg tgc ctg ctg ata tct tat gtc caa gga aca 288
Gly Ala Val Gly Lys Thr Cys Leu Leu Ile Ser Tyr Val Gln Gly Thr
                85                  90                  95
ttt ccg act gat tat att cct act att ttc gaa aat tat gtc aca aac 336
Phe Pro Thr Asp Tyr Ile Pro Thr Ile Phe Glu Asn Tyr Val Thr Asn
            100                 105                 110
ata gaa gga ccc aac ggt caa att ata gaa ttg gca tta tgg gac act 384
Ile Glu Gly Pro Asn Gly Gln Ile Ile Glu Leu Ala Leu Trp Asp Thr
        115                 120                 125
gcc ggc caa gaa gag tat agt aga ctt aga ccg ctt tca tat acg aat 432
Ala Gly Gln Glu Glu Tyr Ser Arg Leu Arg Pro Leu Ser Tyr Thr Asn
    130                 135                 140
gca gat gtg ctg atg gtg tgc tat tct gtt ggt agt aag aca tcg ctt 480
Ala Asp Val Leu Met Val Cys Tyr Ser Val Gly Ser Lys Thr Ser Leu
145                 150                 155                 160
aaa aat gtg gaa gat ctc tgg ttc cca gag gtt aag cat ttt tgt cct 528
Lys Asn Val Glu Asp Leu Trp Phe Pro Glu Val Lys His Phe Cys Pro
                165                 170                 175
tcc act cca atc atg cta gtc ggc ctt aaa tca gat cta tat gaa gct 576
Ser Thr Pro Ile Met Leu Val Gly Leu Lys Ser Asp Leu Tyr Glu Ala
            180                 185                 190
gat aac ctt tca gat ctg gtg gaa cca agt tca gca gaa tcc ttg gcc 624
Asp Asn Leu Ser Asp Leu Val Glu Pro Ser Ser Ala Glu Ser Leu Ala
        195                 200                 205
aag cgt ctg ggg gca ttt gca cat att caa tgc tca gca cga ttg aaa 672
Lys Arg Leu Gly Ala Phe Ala His Ile Gln Cys Ser Ala Arg Leu Lys
    210                 215                 220
gaa aat atc gat gaa gta ttt gaa act gcc ata cac acg tta ctt tcc 720
Glu Asn Ile Asp Glu Val Phe Glu Thr Ala Ile His Thr Leu Leu Ser
225                 230                 235                 240
gat tca tta tat gct ccc aga gag cct aca cat aca atc aaa aat ccc 768
Asp Ser Leu Tyr Ala Pro Arg Glu Pro Thr His Thr Ile Lys Asn Pro
                245                 250                 255
ttt aaa aga aat acc acc aga tca gat atc gat tct tct act gga gat 816
Phe Lys Arg Asn Thr Thr Arg Ser Asp Ile Asp Ser Ser Thr Gly Asp
            260                 265                 270
acc agc gtc tct att tcc gga acg aaa aga tta aga aaa aac aag tgt 864
Thr Ser Val Ser Ile Ser Gly Thr Lys Arg Leu Arg Lys Asn Lys Cys
        275                 280                 285
att ata atg taa 876
Ile Ile Met
    290

<210> 112
<211> 291
<212> PRT
<213> Saccharomyces cerevisiae

<400> 112
Met Asn Thr Leu Leu Phe Lys Arg Lys Gly Gly Asn Cys Gly Asn Glu
1               5                   10                  15
Ser Asn Ile Val Ser Gln Gly Ser Pro Ser Ser Ser Asn Leu Pro Glu
            20                  25                  30
Ser Pro Gly Thr Leu Asp Glu Lys Asn Leu Pro Arg Leu Pro Thr Pro
        35                  40                  45
Phe Ala Arg Ser Leu Ser Thr Ile Pro Ser Tyr Glu Gln Met Lys Arg
    50                  55                  60
Thr Asn Lys Leu Pro Asp Tyr His Leu Lys Ile Val Val Val Gly Asp
65                  70                  75                  80
Gly Ala Val Gly Lys Thr Cys Leu Leu Ile Ser Tyr Val Gln Gly Thr
                85                  90                  95
Phe Pro Thr Asp Tyr Ile Pro Thr Ile Phe Glu Asn Tyr Val Thr Asn
            100                 105                 110
Ile Glu Gly Pro Asn Gly Gln Ile Ile Glu Leu Ala Leu Trp Asp Thr
        115                 120                 125
Ala Gly Gln Glu Glu Tyr Ser Arg Leu Arg Pro Leu Ser Tyr Thr Asn
    130                 135                 140
Ala Asp Val Leu Met Val Cys Tyr Ser Val Gly Ser Lys Thr Ser Leu
145                 150                 155                 160
Lys Asn Val Glu Asp Leu Trp Phe Pro Glu Val Lys His Phe Cys Pro
                165                 170                 175
Ser Thr Pro Ile Met Leu Val Gly Leu Lys Ser Asp Leu Tyr Glu Ala
            180                 185                 190
Asp Asn Leu Ser Asp Leu Val Glu Pro Ser Ser Ala Glu Ser Leu Ala
        195                 200                 205
Lys Arg Leu Gly Ala Phe Ala His Ile Gln Cys Ser Ala Arg Leu Lys
    210                 215                 220
Glu Asn Ile Asp Glu Val Phe Glu Thr Ala Ile His Thr Leu Leu Ser
225                 230                 235                 240
Asp Ser Leu Tyr Ala Pro Arg Glu Pro Thr His Thr Ile Lys Asn Pro
                245                 250                 255
Phe Lys Arg Asn Thr Thr Arg Ser Asp Ile Asp Ser Ser Thr Gly Asp
            260                 265                 270
Thr Ser Val Ser Ile Ser Gly Thr Lys Arg Leu Arg Lys Asn Lys Cys
        275                 280                 285
Ile Ile Met
    290

<210> 113
<211> 696
<212> DNA
<213> Saccharomyces cerevisiae

<220>
<221> CDS
<222> (1)..(696)

<400> 113
atg tca ttt cta tgt ggg tca gct tca acg tca aat aaa ccg atc gaa       48
Met Ser Phe Leu Cys Gly Ser Ala Ser Thr Ser Asn Lys Pro Ile Glu
1               5                   10                  15
aga aag atc gtt att ttg ggc gac ggt gcc tgt ggt aaa act tcg ttg       96
Arg Lys Ile Val Ile Leu Gly Asp Gly Ala Cys Gly Lys Thr Ser Leu
            20                  25                  30
ctg aat gtt ttc acc aga ggt tat ttt ccc gaa gtt tat gag cct act      144
Leu Asn Val Phe Thr Arg Gly Tyr Phe Pro Glu Val Tyr Glu Pro Thr
        35                  40                  45
gtt ttt gaa aac tat atc cat gat att ttc gtt gac agt aaa cat atc      192
Val Phe Glu Asn Tyr Ile His Asp Ile Phe Val Asp Ser Lys His Ile
    50                  55                  60

acg cta tcg ttg tgg gat act gcg ggc caa gag gaa ttt gac agg tta 240
Thr Leu Ser Leu Trp Asp Thr Ala Gly Gln Glu Glu Phe Asp Arg Leu
65                  70                  75                  80
cga tcc ttg tct tat tca gat acg caa tgt ata atg tta tgt ttc agt 288
Arg Ser Leu Ser Tyr Ser Asp Thr Gln Cys Ile Met Leu Cys Phe Ser
                85                  90                  95
att gat tca cgc gat tct tta gaa aat gtc caa aat aaa tgg gtg ggt 336
Ile Asp Ser Arg Asp Ser Leu Glu Asn Val Gln Asn Lys Trp Val Gly
            100                 105                 110
gaa atc act gat cat tgt gaa ggc gtc aag tta gtc tta gtt gca cta 384
Glu Ile Thr Asp His Cys Glu Gly Val Lys Leu Val Leu Val Ala Leu
        115                 120                 125
aag tgt gac tta aga aac aat gaa aat gaa tct aac gca atc aca ccg 432
Lys Cys Asp Leu Arg Asn Asn Glu Asn Glu Ser Asn Ala Ile Thr Pro
    130                 135                 140
aac aat atc caa cag gat aac agc gtt tct aac gac aac gga aat aac 480
Asn Asn Ile Gln Gln Asp Asn Ser Val Ser Asn Asp Asn Gly Asn Asn
145                 150                 155                 160
ata aat agc acc tca aac ggc aaa aac ctg ata agt tat gaa gaa ggt 528
Ile Asn Ser Thr Ser Asn Gly Lys Asn Leu Ile Ser Tyr Glu Glu Gly
                165                 170                 175
cta gct atg gct aaa aag atc ggt gcg cta cgt tat ttg gaa tgt agc 576
Leu Ala Met Ala Lys Lys Ile Gly Ala Leu Arg Tyr Leu Glu Cys Ser
            180                 185                 190
gct aag ctg aat aaa ggt gtc aac gaa gct ttc aca gaa gcc gca aga 624
Ala Lys Leu Asn Lys Gly Val Asn Glu Ala Phe Thr Glu Ala Ala Arg
        195                 200                 205
gtt gct tta acc gcg ggc cca gta gca acc gaa gtg aaa agt gac agt 672
Val Ala Leu Thr Ala Gly Pro Val Ala Thr Glu Val Lys Ser Asp Ser
    210                 215                 220
gga tcc agc tgt acc att atg taa 696
Gly Ser Ser Cys Thr Ile Met
225                 230

<210> 114
<211> 231
<212> PRT
<213> Saccharomyces cerevisiae

<400> 114
Met Ser Phe Leu Cys Gly Ser Ala Ser Thr Ser Asn Lys Pro Ile Glu
1               5                   10                  15
Arg Lys Ile Val Ile Leu Gly Asp Gly Ala Cys Gly Lys Thr Ser Leu
            20                  25                  30
Leu Asn Val Phe Thr Arg Gly Tyr Phe Pro Glu Val Tyr Glu Pro Thr
        35                  40                  45
Val Phe Glu Asn Tyr Ile His Asp Ile Phe Val Asp Ser Lys His Ile
    50                  55                  60
Thr Leu Ser Leu Trp Asp Thr Ala Gly Gln Glu Glu Phe Asp Arg Leu
65                  70                  75                  80
Arg Ser Leu Ser Tyr Ser Asp Thr Gln Cys Ile Met Leu Cys Phe Ser
                85                  90                  95
Ile Asp Ser Arg Asp Ser Leu Glu Asn Val Gln Asn Lys Trp Val Gly
            100                 105                 110
Glu Ile Thr Asp His Cys Glu Gly Val Lys Leu Val Leu Val Ala Leu
        115                 120                 125
Lys Cys Asp Leu Arg Asn Asn Glu Asn Glu Ser Asn Ala Ile Thr Pro
    130                 135                 140
Asn Asn Ile Gln Gln Asp Asn Ser Val Ser Asn Asp Asn Gly Asn Asn
145                 150                 155                 160
Ile Asn Ser Thr Ser Asn Gly Lys Asn Leu Ile Ser Tyr Glu Glu Gly
                165                 170                 175
Leu Ala Met Ala Lys Lys Ile Gly Ala Leu Arg Tyr Leu Glu Cys Ser
            180                 185                 190
Ala Lys Leu Asn Lys Gly Val Asn Glu Ala Phe Thr Glu Ala Ala Arg
        195                 200                 205
Val Ala Leu Thr Ala Gly Pro Val Ala Thr Glu Val Lys Ser Asp Ser

210                 215                 220
Gly Ser Ser Cys Thr Ile Met
225                 230

<210> 115
<211> 579
<212> DNA
<213> Drosophila melanogaster

<220>
<221> CDS
<222> (1)..(579)

<400> 115
atg acg acg att cgc aag aaa ttg gta att gtc ggc gac ggt gcc tgc      48
Met Thr Thr Ile Arg Lys Lys Leu Val Ile Val Gly Asp Gly Ala Cys
1               5                   10                  15
ggt aaa act tgc ctt ctg att gtc ttc agc aaa gat cag ttc ccc gag      96
Gly Lys Thr Cys Leu Leu Ile Val Phe Ser Lys Asp Gln Phe Pro Glu
            20                  25                  30
gtc tat gtg ccc acc gta ttc gag aat tat gtg gcc gac atc gag gtg      144
Val Tyr Val Pro Thr Val Phe Glu Asn Tyr Val Ala Asp Ile Glu Val
        35                  40                  45
gat ggc aaa cag gtg gag ctg gcc ttg tgg gat acg gcc ggg cag gag      192
Asp Gly Lys Gln Val Glu Leu Ala Leu Trp Asp Thr Ala Gly Gln Glu
    50                  55                  60
gac tac gac aga cta cga ccg ctg agc tat ccc gac act gac gtc ata      240
Asp Tyr Asp Arg Leu Arg Pro Leu Ser Tyr Pro Asp Thr Asp Val Ile
65                  70                  75                  80
ctg atg tgt ttc tca gtg gat tca ccc gat tcg cta gaa aat att cct      288
Leu Met Cys Phe Ser Val Asp Ser Pro Asp Ser Leu Glu Asn Ile Pro
                85                  90                  95
gaa aaa tgg acc cca gag gtc aaa cac ttt tgt cca aat gtt cca atc      336
Glu Lys Trp Thr Pro Glu Val Lys His Phe Cys Pro Asn Val Pro Ile
            100                 105                 110
att ttg gta gga aat aag aaa gat ttg cga aat gat ccc aac aca att      384
Ile Leu Val Gly Asn Lys Lys Asp Leu Arg Asn Asp Pro Asn Thr Ile
        115                 120                 125
cgg gat cta gca aaa atg aag cag gag ccg gtg aag ccg cag gag ggt      432
Arg Asp Leu Ala Lys Met Lys Gln Glu Pro Val Lys Pro Gln Glu Gly
    130                 135                 140
cgc gcc atg gcc gag aag att aat gcc ttt gcc tat ttg gag tgt tcg      480
Arg Ala Met Ala Glu Lys Ile Asn Ala Phe Ala Tyr Leu Glu Cys Ser
145                 150                 155                 160
gct aag tcc aag gag ggt gtg cga gat gtt ttc gag acg gca act agg      528
Ala Lys Ser Lys Glu Gly Val Arg Asp Val Phe Glu Thr Ala Thr Arg
                165                 170                 175
gcc gcg ctg caa gtc aaa aag agg aag aag acc aga tgc ctt ttg ctc      576
Ala Ala Leu Gln Val Lys Lys Arg Lys Lys Thr Arg Cys Leu Leu Leu
            180                 185                 190
taa                                                                  579

<210> 116
<211> 192
<212> PRT
<213> Drosophila melanogaster

<400> 116
Met Thr Thr Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu

50                  55                  60
Asp Tyr Asp Arg Leu Arg Pro Leu Ser Tyr Pro Asp Thr Asp Val Ile
65                  70                  75                  80
Leu Met Cys Phe Ser Val Asp Ser Pro Asp Ser Leu Glu Asn Ile Pro
                85                  90                  95
Glu Lys Trp Thr Pro Glu Val Lys His Phe Cys Pro Asn Val Pro Ile
            100                 105                 110
Ile Leu Val Gly Asn Lys Lys Asp Leu Arg Asn Asp Pro Asn Thr Ile
        115                 120                 125
Arg Asp Leu Ala Lys Met Lys Gln Glu Pro Val Lys Pro Gln Glu Gly
    130                 135                 140
Arg Ala Met Ala Glu Lys Ile Asn Ala Phe Ala Tyr Leu Glu Cys Ser
145                 150                 155                 160
Ala Lys Ser Lys Glu Gly Val Arg Asp Val Phe Glu Thr Ala Thr Arg
                165                 170                 175
Ala Ala Leu Gln Val Lys Lys Arg Lys Lys Thr Arg Cys Leu Leu Leu
            180                 185                 190

<210> 117
<211> 579
<212> DNA
<213> Drosophila melanogaster

<220>
<221> CDS
<222> (1)..(579)

<400> 117
atg cag gcg atc aag tgc gtc gtc gtg ggc gac gga gcc gtg gga aag      48
Met Gln Ala Ile Lys Cys Val Val Val Gly Asp Gly Ala Val Gly Lys
1               5                   10                  15
acc tgc ctg ctg atc agc tac acg acc aat gcc ttt ccc ggc gag tac      96
Thr Cys Leu Leu Ile Ser Tyr Thr Thr Asn Ala Phe Pro Gly Glu Tyr
            20                  25                  30
ata ccc acc gtg ttc gac aac tac tcg gcc aac gtg atg gtg gac gcc      144
Ile Pro Thr Val Phe Asp Asn Tyr Ser Ala Asn Val Met Val Asp Ala
        35                  40                  45
aag ccc atc aac ctg ggc ctg tgg gat acg gcc ggg cag gag gac tac      192
Lys Pro Ile Asn Leu Gly Leu Trp Asp Thr Ala Gly Gln Glu Asp Tyr
    50                  55                  60
gac cga ctg agg cca ctg tcc tat ccc cag acc gat gtc ttc ctc atc      240
Asp Arg Leu Arg Pro Leu Ser Tyr Pro Gln Thr Asp Val Phe Leu Ile
65                  70                  75                  80
tgc ttc tcg ctg gtg aat ccg gca tcg ttc gag aac gtg cgg gca aag      288
Cys Phe Ser Leu Val Asn Pro Ala Ser Phe Glu Asn Val Arg Ala Lys
                85                  90                  95
tgg tat ccg gag gtg cgc cac cac tgc ccc agc acg ccc atc atc ctg      336
Trp Tyr Pro Glu Val Arg His His Cys Pro Ser Thr Pro Ile Ile Leu
            100                 105                 110
gtg ggc acc aag ctg gat ttg cgc gac gac aag aac aca atc gaa aag      384
Val Gly Thr Lys Leu Asp Leu Arg Asp Asp Lys Asn Thr Ile Glu Lys
        115                 120                 125
ctg agg gac aag aaa ctg gcg ccc atc acc tat ccg cag ggc tct ggc      432
Leu Arg Asp Lys Lys Leu Ala Pro Ile Thr Tyr Pro Gln Gly Ser Gly
    130                 135                 140
cat ggc aag gaa atc gga gcg gtc aag tat ctg gag tgc tcg gcc ctg      480
His Gly Lys Glu Ile Gly Ala Val Lys Tyr Leu Glu Cys Ser Ala Leu
145                 150                 155                 160
acg cag aag ggt ctg aaa acc gtt ttc gac gag gcc atc cgg tcg gtt      528
Thr Gln Lys Gly Leu Lys Thr Val Phe Asp Glu Ala Ile Arg Ser Val
                165                 170                 175
ttg tgc ccc gtg ctg cag ccc aag tcc aag cgc aag tgc gcc ctg ctc      576
Leu Cys Pro Val Leu Gln Pro Lys Ser Lys Arg Lys Cys Ala Leu Leu
            180                 185                 190
taa                                                                  579

<210> 118
<211> 192
<212> PRT
<213> Drosophila melanogaster

<400> 118
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Ala
        35                  40                  45
Lys Pro Ile Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Asn Pro Ala Ser Phe Glu Asn Val Arg Ala Lys
                85                  90                  95
Trp Tyr Pro Glu Val Arg His His Cys Pro Ser Thr Pro Ile Ile Leu
            100                 105                 110
Val Gly Thr Lys Leu Asp Leu Arg Asp Asp Lys Asn Thr Ile Glu Lys
        115                 120                 125
Leu Arg Asp Lys Lys Leu Ala Pro Ile Thr Tyr Pro Gln Gly Ser Gly
    130                 135                 140
His Gly Lys Glu Ile Gly Ala Val Lys Tyr Leu Glu Cys Ser Ala Leu
145                 150                 155                 160
Thr Gln Lys Gly Leu Lys Thr Val Phe Asp Glu Ala Ile Arg Ser Val
                165                 170                 175
Leu Cys Pro Val Leu Gln Pro Lys Ser Lys Arg Lys Cys Ala Leu Leu
            180                 185                 190

<210> 119
<211> 579
<212> DNA
<213> Drosophila melanogaster

<220>
<221> CDS
<222> (1)..(579)

<400> 119
atg cag gcc atc aag tgt gtg gtt gtg ggc gac gga gcg gtg gga aag      48
Met Gln Ala Ile Lys Cys Val Val Val Gly Asp Gly Ala Val Gly Lys
1               5                   10                  15
acc tgt ctg ctg atc agc tat acg acc aac gcc ttc ccc ggc gag tac      96
Thr Cys Leu Leu Ile Ser Tyr Thr Thr Asn Ala Phe Pro Gly Glu Tyr
            20                  25                  30
ata ccc acg gtg ttc gac aac tat tcg gcg aat gtg atg gtg gat gcc      144
Ile Pro Thr Val Phe Asp Asn Tyr Ser Ala Asn Val Met Val Asp Ala
        35                  40                  45
aag ccc atc aat ctg ggc ctc tgg gat acg gct gga cag gag gac tac      192
Lys Pro Ile Asn Leu Gly Leu Trp Asp Thr Ala Gly Gln Glu Asp Tyr
    50                  55                  60
gat cgc ctg agg ccg cta tcc tat ccg caa acg gat gtc ttt ctc atc      240
Asp Arg Leu Arg Pro Leu Ser Tyr Pro Gln Thr Asp Val Phe Leu Ile
65                  70                  75                  80
tgt ttc tca ctg gtg aat ccg gca tcg ttt gag aat gtg cga gcc aaa      288
Cys Phe Ser Leu Val Asn Pro Ala Ser Phe Glu Asn Val Arg Ala Lys
                85                  90                  95
tgg ttt ccc gag gtg cgt cat cat tgc ccg agt gtg ccg ata atc ctg      336
Trp Phe Pro Glu Val Arg His His Cys Pro Ser Val Pro Ile Ile Leu
            100                 105                 110
gtc ggc acc aaa ctg gat ctg cgc gac gat aag cag acg atc gag aag      384
Val Gly Thr Lys Leu Asp Leu Arg Asp Asp Lys Gln Thr Ile Glu Lys
        115                 120                 125
ctg aag gac aag aag cta aca ccg atc acc tat ccc caa gga ctg gcg      432
Leu Lys Asp Lys Lys Leu Thr Pro Ile Thr Tyr Pro Gln Gly Leu Ala

130                 135                 140
atg gcc aag gaa ata gct gcg gtc aag tat ctg gag tgc tcg gcc ctg      480
Met Ala Lys Glu Ile Ala Ala Val Lys Tyr Leu Glu Cys Ser Ala Leu
145                 150                 155                 160
acc caa aag ggt ctg aag acg gtc ttc gac gag gcc ata cga tcc gtg      528
Thr Gln Lys Gly Leu Lys Thr Val Phe Asp Glu Ala Ile Arg Ser Val
                165                 170                 175
cta tgt cct gtc gtt cga gga ccc aag cgg cac aag tgc gcc ctg ctc      576
Leu Cys Pro Val Val Arg Gly Pro Lys Arg His Lys Cys Ala Leu Leu
            180                 185                 190
taa                                                                  579

<210> 120
<211> 192
<212> PRT
<213> Drosophila melanogaster

<400> 120
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Ala
        35                  40                  45
Lys Pro Ile Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Asn Pro Ala Ser Phe Glu Asn Val Arg Ala Lys
                85                  90                  95
Trp Phe Pro Glu Val Arg His His Cys Pro Ser Val Pro Ile Ile Leu
            100                 105                 110
Val Gly Thr Lys Leu Asp Leu Arg Asp Asp Lys Gln Thr Ile Glu Lys
        115                 120                 125
Leu Lys Asp Lys Lys Leu Thr Pro Ile Thr Tyr Pro Gln Gly Leu Ala
    130                 135                 140
Met Ala Lys Glu Ile Ala Ala Val Lys Tyr Leu Glu Cys Ser Ala Leu
145                 150                 155                 160
Thr Gln Lys Gly Leu Lys Thr Val Phe Asp Glu Ala Ile Arg Ser Val
                165                 170                 175
Leu Cys Pro Val Val Arg Gly Pro Lys Arg His Lys Cys Ala Leu Leu
            180                 185                 190

<210> 121
<211> 913
<212> DNA
<213> Gossypium hirsutum (upland cotton)

<220>
<221> CDS
<222> (12)..(602)

<400> 121
gagaaaaaac a atg agc act gca aga ttt atc aag tgt gtc acg gtc ggt      50
             Met Ser Thr Ala Arg Phe Ile Lys Cys Val Thr Val Gly
             1               5                   10
gat gga gct gtg ggg aaa act tgt atg ctc att tca tat acc agc aat      98
Asp Gly Ala Val Gly Lys Thr Cys Met Leu Ile Ser Tyr Thr Ser Asn
    15                  20                  25
act ttc cca acg gat tat gtt cca aca gta ttt gat aac ttt agt gcc      146
Thr Phe Pro Thr Asp Tyr Val Pro Thr Val Phe Asp Asn Phe Ser Ala
30                  35                  40                  45
aat gtg gtg gtg gat ggc agc aca gtg aac ctt ggc cta tgg gac act      194
Asn Val Val Val Asp Gly Ser Thr Val Asn Leu Gly Leu Trp Asp Thr
                50                  55                  60

gcc ggg caa gaa gat tat aat agg cta agg cca ctg agt tat aga gga 242
Ala Gly Gln Glu Asp Tyr Asn Arg Leu Arg Pro Leu Ser Tyr Arg Gly
            65                  70                  75
gct gat gtg ttt ttg ttg gcc ttt tct ctt ata agc aag gcc agt tat 290
Ala Asp Val Phe Leu Leu Ala Phe Ser Leu Ile Ser Lys Ala Ser Tyr
        80                  85                  90
gaa aac atc tac aaa aag tgg atc cca gag cta aga cat tat gct cat 338
Glu Asn Ile Tyr Lys Lys Trp Ile Pro Glu Leu Arg His Tyr Ala His
    95                  100                 105
aat gta cca gtt gtg ctt gtt gga acc aaa cta gat ttg cga gat gac 386
Asn Val Pro Val Val Leu Val Gly Thr Lys Leu Asp Leu Arg Asp Asp
110                 115                 120                 125
aag cag ttc ctc att gat cac cct gga gca aca cca ata tca aca tct 434
Lys Gln Phe Leu Ile Asp His Pro Gly Ala Thr Pro Ile Ser Thr Ser
                130                 135                 140
cag gga gaa gaa cta aag aag atg ata gga gca gtt act tat ata gaa 482
Gln Gly Glu Glu Leu Lys Lys Met Ile Gly Ala Val Thr Tyr Ile Glu
            145                 150                 155
tgc agc tcc aaa acc caa cag aat gtg aag gct gtt ttc gat gct gca 530
Cys Ser Ser Lys Thr Gln Gln Asn Val Lys Ala Val Phe Asp Ala Ala
        160                 165                 170
ata aaa gta gct ttg agg cca cca aaa cca aag aga aag cct tgc aaa 578
Ile Lys Val Ala Leu Arg Pro Pro Lys Pro Lys Arg Lys Pro Cys Lys
    175                 180                 185
agg aga aca tgt gct ttc ctt tgaatattgg atcattatta cagtcaaaaa 629
Arg Arg Thr Cys Ala Phe Leu
190                 195
cagttaacaa aagctgttgc agataaacac tgaatctgct atagtttgtt tttggtttac 689

atatgttcca cgtgaaacta tgaagcatct ctaagaaaac ccaaactatc atatcaaccc 749

atcgatcaat gaatcgattt caattttcgc agtataagtt ccttttaatc ctttcttttt 809

acttcatttt ataacgaatt ctatggataa tgttccctac aaacatgtca ttacaatgtt 869

taattataaa ttccattctt ctattttact aaaaaaaaaa aaaa 913

<210> 122
<211> 196
<212> PRT
<213> Gossypium hirsutum (upland cotton)

<400> 122
Met Ser Thr Ala Arg Phe Ile Lys Cys Val Thr Val Gly Asp Gly Ala
1               5                   10                  15
Val Gly Lys Thr Cys Met Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Ile
                85                  90                  95
Tyr Lys Lys Trp Ile Pro Glu Leu Arg His Tyr Ala His Asn Val Pro
            100                 105                 110
Val Val Leu Val Gly Thr Lys Leu Asp Leu Arg Asp Asp Lys Gln Phe
        115                 120                 125
Leu Ile Asp His Pro Gly Ala Thr Pro Ile Ser Thr Ser Gln Gly Glu
    130                 135                 140

Glu Leu Lys Lys Met Ile Gly Ala Val Thr Tyr Ile Glu Cys Ser Ser
145                 150                 155                 160
Lys Thr Gln Gln Asn Val Lys Ala Val Phe Asp Ala Ala Ile Lys Val
                165                 170                 175
Ala Leu Arg Pro Pro Lys Pro Lys Arg Lys Pro Cys Lys Arg Arg Thr
            180                 185                 190
Cys Ala Phe Leu
        195

<210> 123
<211> 1067
<212> DNA
<213> Arabidopsis thaliana (mouse-ear cress)

<220>
<221> CDS
<222> (314)..(907)

<400> 123
cttttctctc tctagttgtt gttctctctc tctctcgcat cctccaattc atcgtcctca      60

cgttcccttt tgtttattca tcttccttcc ttcttcacat ttctgatttt ctctatttgg     120

ggggtttgtt tccacattat acatatctag ggttttgaga tggttaattg aaagataatg     180

gtcgaagttt cggaggaatt ggcttcacat tgtgggtgtt tctggcttcc tcaggtttaa     240

atctgaggtt gatctctttt gttttttggt aaattgtgac atattttggc tcgaagaaga     300

agaagaagag gca atg agc gca tca agg ttc ata aag tgc gtc acc gtt        349
               Met Ser Ala Ser Arg Phe Ile Lys Cys Val Thr Val
               1               5                   10
ggt gat gga gct gtt ggt aaa acc tgt ttg ctg att tct tat acc agc       397
Gly Asp Gly Ala Val Gly Lys Thr Cys Leu Leu Ile Ser Tyr Thr Ser
        15                  20                  25
aac acc ttt ccc acg gat tat gtt ccg act gtt ttc gat aac ttt agt       445
Asn Thr Phe Pro Thr Asp Tyr Val Pro Thr Val Phe Asp Asn Phe Ser
    30                  35                  40
gca aat gtg gtt gtc aat ggg gcc acg gtg aat ctt gga ttg tgg gat       493
Ala Asn Val Val Val Asn Gly Ala Thr Val Asn Leu Gly Leu Trp Asp
45                  50                  55                  60
act gca ggg caa gag gac tat aac agg tta aga cct ttg agt tac cgt       541
Thr Ala Gly Gln Glu Asp Tyr Asn Arg Leu Arg Pro Leu Ser Tyr Arg
                65                  70                  75
ggt gct gat gtt ttc att ctt gcc ttc tct ctc att agt aag gct agt       589
Gly Ala Asp Val Phe Ile Leu Ala Phe Ser Leu Ile Ser Lys Ala Ser
            80                  85                  90
tat gag aat gtt tcc aag aag tgg att cca gag ttg aag cac tat gct       637
Tyr Glu Asn Val Ser Lys Lys Trp Ile Pro Glu Leu Lys His Tyr Ala
        95                  100                 105
cct ggt gtc cca att gtc ctt gtt gga acc aaa cta gat ctt cga gat       685
Pro Gly Val Pro Ile Val Leu Val Gly Thr Lys Leu Asp Leu Arg Asp
    110                 115                 120
gac aaa cag ttt ttc atc gac cat cct ggt gct gtc cct att acc act       733
Asp Lys Gln Phe Phe Ile Asp His Pro Gly Ala Val Pro Ile Thr Thr
125                 130                 135                 140
gtt cag gga gag gag ctg aag aag cta att gga gcg cca gct tac atc       781
Val Gln Gly Glu Glu Leu Lys Lys Leu Ile Gly Ala Pro Ala Tyr Ile
                145                 150                 155
gag tgc agt tca aaa tca caa gag aac gtg aag ggc gtg ttt gat gca       829
Glu Cys Ser Ser Lys Ser Gln Glu Asn Val Lys Gly Val Phe Asp Ala
            160                 165                 170
gcg atc aga gtg gtc ctt caa cct cca aag cag aag aaa aag aag aac       877

Ala Ile Arg Val Val Leu Gln Pro Pro Lys Gln Lys Lys Lys Lys Asn
        175                 180                 185
aaa gca caa aag gcc tgc tcc atc ttg taatagcact catggaagtc 924
Lys Ala Gln Lys Ala Cys Ser Ile Leu
    190                 195
aagaagctct ttgagatgag gatgacaggg tggtttaaaa aagtatcgct ttttcatttt 984

tgttgttgtt ttccgttttc atttgtgatc tcttagttcc gtacaaaacg aattgtgagg 1044

ttaattcaac ttctatttta act 1067

<210> 124
<211> 197
<212> PRT
<213> Arabidopsis thaliana (mouse-ear cress)

<400> 124
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ala Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Ile Val Leu Val Gly Thr Lys Leu Asp Leu Arg Asp Asp Lys Gln Phe
        115                 120                 125
Phe Ile Asp His Pro Gly Ala Val Pro Ile Thr Thr Val Gln Gly Glu
    130                 135                 140
Glu Leu Lys Lys Leu Ile Gly Ala Pro Ala Tyr Ile Glu Cys Ser Ser
145                 150                 155                 160
Lys Ser Gln Glu Asn Val Lys Gly Val Phe Asp Ala Ala Ile Arg Val
                165                 170                 175
Val Leu Gln Pro Pro Lys Gln Lys Lys Lys Lys Asn Lys Ala Gln Lys
            180                 185                 190
Ala Cys Ser Ile Leu
        195

<210> 125
<211> 695
<212> DNA
<213> Arabidopsis thaliana (mouse-ear cress)

<220>
<221> CDS
<222> (1)..(594)

<400> 125
atg agc gct tcg agg ttc ata aag tgt gtc acc gtt ggc gac gga gct 48
Met Ser Ala Ser Arg Phe Ile Lys Cys Val Thr Val Gly Asp Gly Ala
1               5                   10                  15
gtt ggt aaa acc tgt ttg ctg att tct tac acc agc aac act ttt cct 96
Val Gly Lys Thr Cys Leu Leu Ile Ser Tyr Thr Ser Asn Thr Phe Pro
            20                  25                  30
acg gat tat gta ccg act gtt ttc gat aac ttt agc gca aat gtg gtt 144
Thr Asp Tyr Val Pro Thr Val Phe Asp Asn Phe Ser Ala Asn Val Val
        35                  40                  45

gtt aat gga gcc act gtg aat ctt ggg cta tgg gat acc gca ggg cag 192
Val Asn Gly Ala Thr Val Asn Leu Gly Leu Trp Asp Thr Ala Gly Gln
    50                  55                  60
gag gat tat aac aga tta aga cct ttg agt tac cgc ggt gct gat gtt 240
Glu Asp Tyr Asn Arg Leu Arg Pro Leu Ser Tyr Arg Gly Ala Asp Val
65                  70                  75                  80
ttc atc tta gca ttc tct ctt atc agt aag gct agt tat gag aat gtc 288
Phe Ile Leu Ala Phe Ser Leu Ile Ser Lys Ala Ser Tyr Glu Asn Val
                85                  90                  95
tcc aag aag tgg atc cca gag ctg aag cat tat gcc cct ggt gtc cct 336
Ser Lys Lys Trp Ile Pro Glu Leu Lys His Tyr Ala Pro Gly Val Pro
            100                 105                 110
ata gtt ctt gtt gga acc aaa cta gat ctt cgg gat gac aaa cag ttc 384
Ile Val Leu Val Gly Thr Lys Leu Asp Leu Arg Asp Asp Lys Gln Phe
        115                 120                 125
ttc att gac cac cct ggc gct gta cca att act act gct cag gga gag 432
Phe Ile Asp His Pro Gly Ala Val Pro Ile Thr Thr Ala Gln Gly Glu
    130                 135                 140
gaa ctg aag aaa cta att gga gct ccc gca tac atc gag tgc agt tca 480
Glu Leu Lys Lys Leu Ile Gly Ala Pro Ala Tyr Ile Glu Cys Ser Ser
145                 150                 155                 160
aaa aca caa gag aac gtg aaa gga gta ttt gat gca gcg atc cga gtg 528
Lys Thr Gln Glu Asn Val Lys Gly Val Phe Asp Ala Ala Ile Arg Val
                165                 170                 175
gtt ctt caa cct cca aag cag aag aaa aag aaa agc aaa gca caa aaa 576
Val Leu Gln Pro Pro Lys Gln Lys Lys Lys Lys Ser Lys Ala Gln Lys
            180                 185                 190
gcc tgc tcc att ttg taatttctct acgcttctct tacgtatctc tctctgtctc 631
Ala Cys Ser Ile Leu
        195
tctgtctctt ccactcttct agtgaaggct taagaagaaa acacattggg cttaaaaatt 691

gttc 695

<210> 126
<211> 197
<212> PRT
<213> Arabidopsis thaliana (mouse-ear cress)

<400> 126
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ala Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Ile Val Leu Val Gly Thr Lys Leu Asp Leu Arg Asp Asp Lys Gln Phe
        115                 120                 125
Phe Ile Asp His Pro Gly Ala Val Pro Ile Thr Thr Ala Gln Gly Glu
    130                 135                 140
Glu Leu Lys Lys Leu Ile Gly Ala Pro Ala Tyr Ile Glu Cys Ser Ser
145                 150                 155                 160
Lys Thr Gln Glu Asn Val Lys Gly Val Phe Asp Ala Ala Ile Arg Val
                165                 170                 175
Val Leu Gln Pro Pro Lys Gln Lys Lys Lys Lys Ser Lys Ala Gln Lys
            180                 185                 190

Ala Cys Ser Ile Leu
        195

<210> 127
<211> 630
<212> DNA
<213> Arabidopsis thaliana (mouse-ear cress)

<220>
<221> CDS
<222> (1)..(630)

<400> 127
atg agt gct tcg aag ttc ata aaa tgt gtt act gtt gga gat ggg gct        48
Met Ser Ala Ser Lys Phe Ile Lys Cys Val Thr Val Gly Asp Gly Ala
1               5                   10                  15
gtt ggg aag aca tgt atg ctt atc tgt tac act agc aac aag ttt cct        96
Val Gly Lys Thr Cys Met Leu Ile Cys Tyr Thr Ser Asn Lys Phe Pro
            20                  25                  30
act gat tat ata ccg act gtg ttc gac aat ttc agt gcc aat gta gct        144
Thr Asp Tyr Ile Pro Thr Val Phe Asp Asn Phe Ser Ala Asn Val Ala
        35                  40                  45
gtg gat gga caa atc gtt aat tta ggg cta tgg gac act gcc ggt caa        192
Val Asp Gly Gln Ile Val Asn Leu Gly Leu Trp Asp Thr Ala Gly Gln
    50                  55                  60
gaa gat tac agt agg tta aga cca ttg agt tat aga gga gct gat atc        240
Glu Asp Tyr Ser Arg Leu Arg Pro Leu Ser Tyr Arg Gly Ala Asp Ile
65                  70                  75                  80
ttc gtc tta gcc ttt tcg ctt att agc aag gcg agt tac gaa aat gta        288
Phe Val Leu Ala Phe Ser Leu Ile Ser Lys Ala Ser Tyr Glu Asn Val
                85                  90                  95
ctc aag aag tgg atg cct gaa ctt cgt cgg ttt gcg cca aat gtt ccc        336
Leu Lys Lys Trp Met Pro Glu Leu Arg Arg Phe Ala Pro Asn Val Pro
            100                 105                 110
ata gtt ctt gtt ggt aca aag cta gat ctc cgg gat gac aag gga tac        384
Ile Val Leu Val Gly Thr Lys Leu Asp Leu Arg Asp Asp Lys Gly Tyr
        115                 120                 125
ctc gcg gat cac acc aat gtc att acc tct act cag gga gag gaa ttg        432
Leu Ala Asp His Thr Asn Val Ile Thr Ser Thr Gln Gly Glu Glu Leu
    130                 135                 140
agg aag caa att ggt gca gct gct tat att gag tgt agt tcc aag act        480
Arg Lys Gln Ile Gly Ala Ala Ala Tyr Ile Glu Cys Ser Ser Lys Thr
145                 150                 155                 160
caa caa aat gtg aaa gca gtg ttt gat aca gcg atc aag gtg gtt ctt        528
Gln Gln Asn Val Lys Ala Val Phe Asp Thr Ala Ile Lys Val Val Leu
                165                 170                 175
cag cct cca agg agg aaa gag gtc ccg agg agg agg aag aat cat aga        576
Gln Pro Pro Arg Arg Lys Glu Val Pro Arg Arg Arg Lys Asn His Arg
            180                 185                 190
aga tcc ggt tgc tcc att gcg agt att gtc tgt gga ggt tgc acc gct        624
Arg Ser Gly Cys Ser Ile Ala Ser Ile Val Cys Gly Gly Cys Thr Ala
        195                 200                 205
gct taa                                                                630
Ala

<210> 128
<211> 209
<212> PRT
<213> Arabidopsis thaliana (mouse-ear cress)

<400> 128
Met Ser Ala Ser Lys Phe Ile Lys Cys Val Thr Val Gly Asp Gly Ala
1               5                   10                  15
Val Gly Lys Thr Cys Met Leu Ile Cys Tyr Thr Ser Asn Lys Phe Pro
            20                  25                  30
Thr Asp Tyr Ile Pro Thr Val Phe Asp Asn Phe Ser Ala Asn Val Ala

35                  40                  45
Val Asp Gly Gln Ile Val Asn Leu Gly Leu Trp Asp Thr Ala Gly Gln
    50                  55                  60
Glu Asp Tyr Ser Arg Leu Arg Pro Leu Ser Tyr Arg Gly Ala Asp Ile
65                  70                  75                  80
Phe Val Leu Ala Phe Ser Leu Ile Ser Lys Ala Ser Tyr Glu Asn Val
                85                  90                  95
Leu Lys Lys Trp Met Pro Glu Leu Arg Arg Phe Ala Pro Asn Val Pro
            100                 105                 110
Ile Val Leu Val Gly Thr Lys Leu Asp Leu Arg Asp Asp Lys Gly Tyr
        115                 120                 125
Leu Ala Asp His Thr Asn Val Ile Thr Ser Thr Gln Gly Glu Glu Leu
    130                 135                 140
Arg Lys Gln Ile Gly Ala Ala Ala Tyr Ile Glu Cys Ser Ser Lys Thr
145                 150                 155                 160
Gln Gln Asn Val Lys Ala Val Phe Asp Thr Ala Ile Lys Val Val Leu
                165                 170                 175
Gln Pro Pro Arg Arg Lys Glu Val Pro Arg Arg Arg Lys Asn His Arg
            180                 185                 190
Arg Ser Gly Cys Ser Ile Ala Ser Ile Val Cys Gly Gly Cys Thr Ala
        195                 200                 205
Ala

<210> 129
<211> 588
<212> DNA
<213> Caenorhabditis elegans

<220>
<221> CDS
<222> (1)..(588)

<400> 129
atg tct tca ccg tcg agg cag atc aaa tgt gta gtt gtt gga gac gga      48
Met Ser Ser Pro Ser Arg Gln Ile Lys Cys Val Val Val Gly Asp Gly
1               5                   10                  15
aca gtt gga aaa aca tgc atg tta ata tct tac aca act gac tct ttt      96
Thr Val Gly Lys Thr Cys Met Leu Ile Ser Tyr Thr Thr Asp Ser Phe
            20                  25                  30
cca gtt cag tat gtg cct aca gta ttt gat aac tat tcg gca cag atg      144
Pro Val Gln Tyr Val Pro Thr Val Phe Asp Asn Tyr Ser Ala Gln Met
        35                  40                  45
agt ctt gat ggg aac gtt gtg aac tta gga ttg tgg gat act gct gga      192
Ser Leu Asp Gly Asn Val Val Asn Leu Gly Leu Trp Asp Thr Ala Gly
    50                  55                  60
cag gag gat tat gat cgt tta cga cca ctt tcc tac cca cag acg gat      240
Gln Glu Asp Tyr Asp Arg Leu Arg Pro Leu Ser Tyr Pro Gln Thr Asp
65                  70                  75                  80
gtt ttc att ctc tgc ttc tct gtc gtc tcg ccc gta tcg ttt gac aat      288
Val Phe Ile Leu Cys Phe Ser Val Val Ser Pro Val Ser Phe Asp Asn
                85                  90                  95
gtg gca agc aag tgg att ccg gaa ata cga cag cat tgt cca gat gcg      336
Val Ala Ser Lys Trp Ile Pro Glu Ile Arg Gln His Cys Pro Asp Ala
            100                 105                 110
cct gtc att cta gtt ggt acc aaa ctc gat ttg cgc gac gag gcc gaa      384
Pro Val Ile Leu Val Gly Thr Lys Leu Asp Leu Arg Asp Glu Ala Glu
        115                 120                 125
ccg atg cgt gct ctg cag gcc gaa gga aag tcc cca att tcc aaa acg      432
Pro Met Arg Ala Leu Gln Ala Glu Gly Lys Ser Pro Ile Ser Lys Thr
    130                 135                 140
caa ggc atg aaa atg gct caa aaa att aaa gct gtc aag tat ttg gaa      480
Gln Gly Met Lys Met Ala Gln Lys Ile Lys Ala Val Lys Tyr Leu Glu
145                 150                 155                 160
tgc tct gca ttg acg caa cag gga ctc aca cag gtg ttc gaa gac gcc      528
Cys Ser Ala Leu Thr Gln Gln Gly Leu Thr Gln Val Phe Glu Asp Ala
                165                 170                 175
gta cgg tcc att ctt cat ccg aaa cca cag aaa aag aag aag tct tgc      576

Val Arg Ser Ile Leu His Pro Lys Pro Gln Lys Lys Lys Lys Ser Cys
            180                 185                 190
aat att atg taa                                                   588
Asn Ile Met
        195

<210> 130
<211> 195
<212> PRT
<213> Caenorhabditis elegans

<400> 130
Met Ser Ser Pro Ser Arg Gln Ile Lys Cys Val Val Val Gly Asp Gly
1               5                   10                  15
Thr Val Gly Lys Thr Cys Met Leu Ile Ser Tyr Thr Thr Asp Ser Phe
            20                  25                  30
Pro Val Gln Tyr Val Pro Thr Val Phe Asp Asn Tyr Ser Ala Gln Met
        35                  40                  45
Ser Leu Asp Gly Asn Val Val Asn Leu Gly Leu Trp Asp Thr Ala Gly
    50                  55                  60
Gln Glu Asp Tyr Asp Arg Leu Arg Pro Leu Ser Tyr Pro Gln Thr Asp
65                  70                  75                  80
Val Phe Ile Leu Cys Phe Ser Val Val Ser Pro Val Ser Phe Asp Asn
                85                  90                  95
Val Ala Ser Lys Trp Ile Pro Glu Ile Arg Gln His Cys Pro Asp Ala
            100                 105                 110
Pro Val Ile Leu Val Gly Thr Lys Leu Asp Leu Arg Asp Glu Ala Glu
        115                 120                 125
Pro Met Arg Ala Leu Gln Ala Glu Gly Lys Ser Pro Ile Ser Lys Thr
    130                 135                 140
Gln Gly Met Lys Met Ala Gln Lys Ile Lys Ala Val Lys Tyr Leu Glu
145                 150                 155                 160
Cys Ser Ala Leu Thr Gln Gln Gly Leu Thr Gln Val Phe Glu Asp Ala
                165                 170                 175
Val Arg Ser Ile Leu His Pro Lys Pro Gln Lys Lys Lys Lys Ser Cys
            180                 185                 190
Asn Ile Met
        195

<210> 131
<211> 843
<212> DNA
<213> Arabidopsis thaliana (mouse-ear cress)

<220>
<221> CDS
<222> (90)..(683)

<400> 131
acgcgtcgag aaactccaat tccaaaggct aaatctttga gatctttttt tttataaatt      60

tctctgaaat taataaactt tgaggggaa atg agc gct tcg agg ttc gta aag      113
                                Met Ser Ala Ser Arg Phe Val Lys
                                1               5
tgc gtg acg gtt ggt gat gga gct gtc gga aaa act tgt ttg ttg att      161
Cys Val Thr Val Gly Asp Gly Ala Val Gly Lys Thr Cys Leu Leu Ile
    10                  15                  20
tct tac aca agc aac act ttc cct acg gat tat gtg cct acc gtt ttc      209
Ser Tyr Thr Ser Asn Thr Phe Pro Thr Asp Tyr Val Pro Thr Val Phe
25                  30                  35                  40
gat aat ttc agt gcc aat gtt gtg gtt aat gga agc act gtg aat ctt      257
Asp Asn Phe Ser Ala Asn Val Val Val Asn Gly Ser Thr Val Asn Leu
                45                  50                  55
gga ttg tgg gac act gca ggg caa gag gat tac aat aga tta aga cca      305
Gly Leu Trp Asp Thr Ala Gly Gln Glu Asp Tyr Asn Arg Leu Arg Pro
            60                  65                  70

ctg agt tac cgt gga gca gat gtt ttc att ttg gcc ttc tct ctt atc 353
Leu Ser Tyr Arg Gly Ala Asp Val Phe Ile Leu Ala Phe Ser Leu Ile
        75                  80                  85
agt aaa gcc agt tat gaa aac gtc tcc aaa aag tgg atc ccg gag ttg 401
Ser Lys Ala Ser Tyr Glu Asn Val Ser Lys Lys Trp Ile Pro Glu Leu
    90                  95                  100
aaa cat tac gcg cct ggt gtc ccc atc gtc ctt gtt gga aca aag ctt 449
Lys His Tyr Ala Pro Gly Val Pro Ile Val Leu Val Gly Thr Lys Leu
105                 110                 115                 120
gat ctt cga gat gat aaa cag ttc ttt atc gac cat cct ggt gct gtt 497
Asp Leu Arg Asp Asp Lys Gln Phe Phe Ile Asp His Pro Gly Ala Val
                125                 130                 135
ccg att act act gct cag gga gag gag ctg agg aag caa ata gga gca 545
Pro Ile Thr Thr Ala Gln Gly Glu Glu Leu Arg Lys Gln Ile Gly Ala
            140                 145                 150
cct act tac atc gaa tgc agt tcc aaa act caa gag aat gtg aag gcg 593
Pro Thr Tyr Ile Glu Cys Ser Ser Lys Thr Gln Glu Asn Val Lys Ala
        155                 160                 165
gtg ttt gac gca gcc atc cga gtg gtg ttg caa ccg cca aag cag aag 641
Val Phe Asp Ala Ala Ile Arg Val Val Leu Gln Pro Pro Lys Gln Lys
    170                 175                 180
aag aag aag agc aaa gcg cag aag gca tgc tcc att cta tgatgattgg 690
Lys Lys Lys Ser Lys Ala Gln Lys Ala Cys Ser Ile Leu
185                 190                 195
aaatctctgt ttttatgtat ttggttttgg tatattaatc ttatatcaat gaatgaatta 750

atgtgttaat ggacagacac ccaagtttga ctggtccttt ttgttcttaa tattaatgga 810

gtttgtcgga aaaaaaaaaa aaaaaaaaaa aaa 843

<210> 132
<211> 197
<212> PRT
<213> Arabidopsis thaliana (mouse-ear cress)

<400> 132
Met Ser Ala Ser Arg Phe Val Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Ile Val Leu Val Gly Thr Lys Leu Asp Leu Arg Asp Asp Lys Gln Phe
        115                 120                 125
Phe Ile Asp His Pro Gly Ala Val Pro Ile Thr Thr Ala Gln Gly Glu
    130                 135                 140
Glu Leu Arg Lys Gln Ile Gly Ala Pro Thr Tyr Ile Glu Cys Ser Ser
145                 150                 155                 160
Lys Thr Gln Glu Asn Val Lys Ala Val Phe Asp Ala Ala Ile Arg Val
                165                 170                 175
Val Leu Gln Pro Pro Lys Gln Lys Lys Lys Lys Ser Lys Ala Gln Lys
            180                 185                 190
Ala Cys Ser Ile Leu
        195

<210> 133
<211> 841
<212> DNA
<213> Arabidopsis thaliana (mouse-ear cress)

<220>
<221> CDS
<222> (28)..(633)

<400> 133
aaggaattga agacaaaagc aaagaaa atg agc aca gca aga ttc att aag tgt     54
                              Met Ser Thr Ala Arg Phe Ile Lys Cys
                              1               5
gtg act gtc gga gat gga gca gtg gga aag act tgt atg ctc att tca     102
Val Thr Val Gly Asp Gly Ala Val Gly Lys Thr Cys Met Leu Ile Ser
10                  15                  20                  25
tat acc agc aat acg ttt cct acg gat tat gtt cca aca gtt ttc gac     150
Tyr Thr Ser Asn Thr Phe Pro Thr Asp Tyr Val Pro Thr Val Phe Asp
                30                  35                  40
aac ttc agc gca aat gtg gtg gtc gac ggg agt acc gtg aac ctt ggc     198
Asn Phe Ser Ala Asn Val Val Val Asp Gly Ser Thr Val Asn Leu Gly
            45                  50                  55
ctg tgg gat act gcc ggt cag gaa gat tat aat agg ctt agg cct ttg     246
Leu Trp Asp Thr Ala Gly Gln Glu Asp Tyr Asn Arg Leu Arg Pro Leu
        60                  65                  70
agt tac aga gga gca gat gtc ttc tta tta gca ttt tcc ctt ata agc     294
Ser Tyr Arg Gly Ala Asp Val Phe Leu Leu Ala Phe Ser Leu Ile Ser
    75                  80                  85
aag gcc agt tac gag aat att cac aaa aag tgg ctt ccg gag ctg aaa     342
Lys Ala Ser Tyr Glu Asn Ile His Lys Lys Trp Leu Pro Glu Leu Lys
90                  95                  100                 105
cat tat gct cct ggc atc ccc att gtg ctc gtc gga aca aaa tta gat     390
His Tyr Ala Pro Gly Ile Pro Ile Val Leu Val Gly Thr Lys Leu Asp
                110                 115                 120
ttg agg gat gac aag cag ttc ttg aag gat cat cca gga gca gct tct     438
Leu Arg Asp Asp Lys Gln Phe Leu Lys Asp His Pro Gly Ala Ala Ser
            125                 130                 135
ata aca act gct cag gga gaa gaa tta agg aaa atg att gga gct gtt     486
Ile Thr Thr Ala Gln Gly Glu Glu Leu Arg Lys Met Ile Gly Ala Val
        140                 145                 150
agg tac tta gag tgc agc tcc aaa acc caa cag aat gtg aag gca gtg     534
Arg Tyr Leu Glu Cys Ser Ser Lys Thr Gln Gln Asn Val Lys Ala Val
    155                 160                 165
ttt gat aca gcg ata agg gta gct ttg agg cca cca aag gca aag aaa     582
Phe Asp Thr Ala Ile Arg Val Ala Leu Arg Pro Pro Lys Ala Lys Lys
170                 175                 180                 185
aag ata aaa cca ttg aag act aag aga tca aga ata tgc ttt ttc cta     630
Lys Ile Lys Pro Leu Lys Thr Lys Arg Ser Arg Ile Cys Phe Phe Leu
                190                 195                 200
taatatctga acagatttca ataaataaca aacatttata tatacttgta ttttgatatt     690

tttttgaata ttttctactt gaaatagtgg actttgcttc atcacgttat tggcgagagt     750

attgtcgaat catgttacca ttattcggga gaaatttgta aaatagtagt aatttggcaa     810

gaaaattgag gattgatatt ttttccccat a                                    841

<210> 134
<211> 201
<212> PRT
<213> Arabidopsis thaliana (mouse-ear cress)

<400> 134
Met Ser Thr Ala Arg Phe Ile Lys Cys Val Thr Val Gly Asp Gly Ala
1               5                   10                  15
Val Gly Lys Thr Cys Met Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Ile
                85                  90                  95
His Lys Lys Trp Leu Pro Glu Leu Lys His Tyr Ala Pro Gly Ile Pro
            100                 105                 110
Ile Val Leu Val Gly Thr Lys Leu Asp Leu Arg Asp Asp Lys Gln Phe
        115                 120                 125
Leu Lys Asp His Pro Gly Ala Ala Ser Ile Thr Thr Ala Gln Gly Glu
    130                 135                 140
Glu Leu Arg Lys Met Ile Gly Ala Val Arg Tyr Leu Glu Cys Ser Ser
145                 150                 155                 160
Lys Thr Gln Gln Asn Val Lys Ala Val Phe Asp Thr Ala Ile Arg Val
                165                 170                 175
Ala Leu Arg Pro Pro Lys Ala Lys Lys Lys Ile Lys Pro Leu Lys Thr
            180                 185                 190
Lys Arg Ser Arg Ile Cys Phe Phe Leu
        195                 200

<210> 135
<211> 784
<212> DNA
<213> Arabidopsis thaliana (mouse-ear cress)

<220>
<221> CDS
<222> (1)..(591)

<400> 135
atg agt gct tcg agg ttt ata aag tgt gtc acc gtc ggc gat ggt gcc      48
Met Ser Ala Ser Arg Phe Ile Lys Cys Val Thr Val Gly Asp Gly Ala
1               5                   10                  15
gtc gga aaa act tgt atg ctg att tct tac aca agc aac act ttc cct      96
Val Gly Lys Thr Cys Met Leu Ile Ser Tyr Thr Ser Asn Thr Phe Pro
            20                  25                  30
acg gac tat gtt cca act gtt ttc gac aac ttc agt gct aat gtg gtt      144
Thr Asp Tyr Val Pro Thr Val Phe Asp Asn Phe Ser Ala Asn Val Val
        35                  40                  45
gta gat ggg aac acg gtg aat ctt gga ttg tgg gat aca gct ggt caa      192
Val Asp Gly Asn Thr Val Asn Leu Gly Leu Trp Asp Thr Ala Gly Gln
    50                  55                  60
gaa gac tat aac agg tta aga ccg ttg agt tac cgt ggt gcc gat gtc      240
Glu Asp Tyr Asn Arg Leu Arg Pro Leu Ser Tyr Arg Gly Ala Asp Val
65                  70                  75                  80
ttc att ctt gca ttc tcg ctt att agc aaa gct agc tac gag aat gta      288
Phe Ile Leu Ala Phe Ser Leu Ile Ser Lys Ala Ser Tyr Glu Asn Val
                85                  90                  95
gcc aag aag tgg att cct gag ctt agg cat tat gcc cct ggt gtt cct      336
Ala Lys Lys Trp Ile Pro Glu Leu Arg His Tyr Ala Pro Gly Val Pro
            100                 105                 110
ata atc ctc gtt gga acg aaa ctc gat ctt cga gat gac aag caa ttc      384
Ile Ile Leu Val Gly Thr Lys Leu Asp Leu Arg Asp Asp Lys Gln Phe
        115                 120                 125
ttc ata gac cat cct ggt gca gtg cct att act aca aac cag gga gag      432
Phe Ile Asp His Pro Gly Ala Val Pro Ile Thr Thr Asn Gln Gly Glu
    130                 135                 140
gaa cta aag aaa ctg ata gga tca cca atc tac att gaa tgt agt tca      480
Glu Leu Lys Lys Leu Ile Gly Ser Pro Ile Tyr Ile Glu Cys Ser Ser
145                 150                 155                 160

aag act cag cag aat gtg aaa gca gtc ttt gac gca gcc ata aaa gtg 528
Lys Thr Gln Gln Asn Val Lys Ala Val Phe Asp Ala Ala Ile Lys Val
                165                 170                 175
gtg ctt cag cca ccg aaa cag aag aag aag aaa aag aac aag aac cgc 576
Val Leu Gln Pro Pro Lys Gln Lys Lys Lys Lys Lys Asn Lys Asn Arg
            180                 185                 190
tgc gtg ttc ttg tgatcgaaca tctcttaaaa cgaaaaaagg tttaggtaac 628
Cys Val Phe Leu
        195
aaaagaagct gaaggaaaac gaacacctgc aacattgtat agttgttgaa tccggcttgt 688

ttcggtcttt actcttcttt ttgctggtga atctaatgag tgaactgagg ccaagaggag 748

gattctgggt aagagaaggg gatgtgttct tttctt 784

<210> 136
<211> 196
<212> PRT
<213> Arabidopsis thaliana (mouse-ear cress)

<400> 136
Met Ser Ala Ser Arg Phe Ile Lys Cys Val Thr Val Gly Asp Gly Ala
1               5                   10                  15
Val Gly Lys Thr Cys Met Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Asn Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ala Lys Lys Trp Ile Pro Glu Leu Arg His Tyr Ala Pro Gly Val Pro
            100                 105                 110
Ile Ile Leu Val Gly Thr Lys Leu Asp Leu Arg Asp Asp Lys Gln Phe
        115                 120                 125
Phe Ile Asp His Pro Gly Ala Val Pro Ile Thr Thr Asn Gln Gly Glu
    130                 135                 140
Glu Leu Lys Lys Leu Ile Gly Ser Pro Ile Tyr Ile Glu Cys Ser Ser
145                 150                 155                 160
Lys Thr Gln Gln Asn Val Lys Ala Val Phe Asp Ala Ala Ile Lys Val
                165                 170                 175
Val Leu Gln Pro Pro Lys Gln Lys Lys Lys Lys Lys Asn Lys Asn Arg
            180                 185                 190
Cys Val Phe Leu
        195

<210> 137
<211> 679
<212> DNA
<213> Arabidopsis thaliana (mouse-ear cress)

<220>
<221> CDS
<222> (1)..(648)

<400> 137
atg gct tca agt gct tca aag ttc atc aag tgt gtg act gtt ggt gat 48
Met Ala Ser Ser Ala Ser Lys Phe Ile Lys Cys Val Thr Val Gly Asp
1               5                   10                  15
ggt gct gtt ggt aaa acc tgt atg ctc atc tgc tac acc agc aat aaa 96
Gly Ala Val Gly Lys Thr Cys Met Leu Ile Cys Tyr Thr Ser Asn Lys

                    20                  25                  30
ttc ccc act gac tac ata cca aca gtt ttt gac aac ttt agt gca aat        144
Phe Pro Thr Asp Tyr Ile Pro Thr Val Phe Asp Asn Phe Ser Ala Asn
        35                  40                  45
gtt gtt gtt gaa ggc acc act gtc aat ttg ggg ctt tgg gac act gct        192
Val Val Val Glu Gly Thr Thr Val Asn Leu Gly Leu Trp Asp Thr Ala
    50                  55                  60
ggg caa gaa gac tat aac aga tta agg cct tta agt tac agg gga gca        240
Gly Gln Glu Asp Tyr Asn Arg Leu Arg Pro Leu Ser Tyr Arg Gly Ala
65                  70                  75                  80
gat gtt ttc gtc ttg tct ttc tca tta gtc agc cga gct agc tac gag        288
Asp Val Phe Val Leu Ser Phe Ser Leu Val Ser Arg Ala Ser Tyr Glu
                85                  90                  95
aat gtt ttt aaa aag tgg atc cct gaa ctc caa cac ttt gct cca gga        336
Asn Val Phe Lys Lys Trp Ile Pro Glu Leu Gln His Phe Ala Pro Gly
            100                 105                 110
gtt ccc ctt gtc ctt gtt ggt acc aaa tta gat ctt cgt gaa gat aag        384
Val Pro Leu Val Leu Val Gly Thr Lys Leu Asp Leu Arg Glu Asp Lys
        115                 120                 125
cat tat ttg gct gat cat cct gga cta tcc cct gta act act gca cag        432
His Tyr Leu Ala Asp His Pro Gly Leu Ser Pro Val Thr Thr Ala Gln
    130                 135                 140
gga gag gag ttg cgt aag cta att ggt gcg acg tat tac att gag tgt        480
Gly Glu Glu Leu Arg Lys Leu Ile Gly Ala Thr Tyr Tyr Ile Glu Cys
145                 150                 155                 160
agt tca aaa act caa cag aat gtg aaa gca gtt ttt gat tct gcg ata        528
Ser Ser Lys Thr Gln Gln Asn Val Lys Ala Val Phe Asp Ser Ala Ile
                165                 170                 175
aag gaa gtg atc aaa cct ctg gtt aaa caa aag gag aag act aag aag        576
Lys Glu Val Ile Lys Pro Leu Val Lys Gln Lys Glu Lys Thr Lys Lys
            180                 185                 190
aag aag aag caa aag tcg aat cac ggc tgt tta tca aat gtt ctg tgt        624
Lys Lys Lys Gln Lys Ser Asn His Gly Cys Leu Ser Asn Val Leu Cys
        195                 200                 205
ggg agg ata gtg act cgg cat tgatgacgat gacccaactc agtctgatga           675
Gly Arg Ile Val Thr Arg His
    210                 215
tttt                                                                   679

<210> 138
<211> 215
<212> PRT
<213> Arabidopsis thaliana (mouse-ear cress)

<400> 138
Met Ala Ser Ser Ala Ser Lys Phe Ile Lys Cys Val Thr Val Gly Asp
1               5                   10                  15
Gly Ala Val Gly Lys Thr Cys Met Leu Ile Cys Tyr Thr Ser Asn Lys
            20                  25                  30
Phe Pro Thr Asp Tyr Ile Pro Thr Val Phe Asp Asn Phe Ser Ala Asn
        35                  40                  45
Val Val Val Glu Gly Thr Thr Val Asn Leu Gly Leu Trp Asp Thr Ala
    50                  55                  60
Gly Gln Glu Asp Tyr Asn Arg Leu Arg Pro Leu Ser Tyr Arg Gly Ala
65                  70                  75                  80
Asp Val Phe Val Leu Ser Phe Ser Leu Val Ser Arg Ala Ser Tyr Glu
                85                  90                  95
Asn Val Phe Lys Lys Trp Ile Pro Glu Leu Gln His Phe Ala Pro Gly
            100                 105                 110
Val Pro Leu Val Leu Val Gly Thr Lys Leu Asp Leu Arg Glu Asp Lys
        115                 120                 125
His Tyr Leu Ala Asp His Pro Gly Leu Ser Pro Val Thr Thr Ala Gln
    130                 135                 140
Gly Glu Glu Leu Arg Lys Leu Ile Gly Ala Thr Tyr Tyr Ile Glu Cys
145                 150                 155                 160

Ser Ser Lys Thr Gln Gln Asn Val Lys Ala Val Phe Asp Ser Ala Ile
                165                 170                 175
Lys Glu Val Ile Lys Pro Leu Val Lys Gln Lys Glu Lys Thr Lys Lys
            180                 185                 190
Lys Lys Lys Gln Lys Ser Asn His Gly Cys Leu Ser Asn Val Leu Cys
        195                 200                 205
Gly Arg Ile Val Thr Arg His
    210                 215

<210> 139
<211> 630
<212> DNA
<213> Saccharomyces cerevisiae

<220>
<221> CDS
<222> (1)..(630)

<400> 139
   atg tca caa caa gtt ggt aac agt atc aga aga aag ctg gta atc gtt      48
   Met Ser Gln Gln Val Gly Asn Ser Ile Arg Arg Lys Leu Val Ile Val
   1               5                   10                  15
   ggt gat ggt gcc tgt ggt aag aca tgt tta tta atc gtc ttt tcc aag      96
   Gly Asp Gly Ala Cys Gly Lys Thr Cys Leu Leu Ile Val Phe Ser Lys
               20                  25                  30
   ggc caa ttt cca gaa gtc tac gta cca act gtc ttt gaa aac tat gta      144
   Gly Gln Phe Pro Glu Val Tyr Val Pro Thr Val Phe Glu Asn Tyr Val
           35                  40                  45
   gca gat gtt gaa gtt gat ggg cgt cgt gta gag cta gcg cta tgg gat      192
   Ala Asp Val Glu Val Asp Gly Arg Arg Val Glu Leu Ala Leu Trp Asp
       50                  55                  60
   acc gct ggt caa gaa gat tat gat aga cta aga cca ttg tca tac cca      240
   Thr Ala Gly Gln Glu Asp Tyr Asp Arg Leu Arg Pro Leu Ser Tyr Pro
   65                  70                  75                  80
   gac tcc aat gtc gta tta att tgt ttc tct atc gat ctt cca gat tct      288
   Asp Ser Asn Val Val Leu Ile Cys Phe Ser Ile Asp Leu Pro Asp Ser
                   85                  90                  95
   tta gag aat gta caa gaa aaa tgg att gcc gaa gta tta cat ttc tgt      336
   Leu Glu Asn Val Gln Glu Lys Trp Ile Ala Glu Val Leu His Phe Cys
               100                 105                 110
   caa ggt gtg cca att att ctt gtt ggt tgt aaa gtg gat ttg aga aac      384
   Gln Gly Val Pro Ile Ile Leu Val Gly Cys Lys Val Asp Leu Arg Asn
           115                 120                 125
   gac cca caa acc att gaa caa tta aga caa gaa ggt caa caa ccc gtt      432
   Asp Pro Gln Thr Ile Glu Gln Leu Arg Gln Glu Gly Gln Gln Pro Val
       130                 135                 140
   aca tca cag gag gga caa tct gta gca gac cag att ggc gca acc gga      480
   Thr Ser Gln Glu Gly Gln Ser Val Ala Asp Gln Ile Gly Ala Thr Gly
   145                 150                 155                 160
   tac tac gaa tgt tcg gcc aag act ggt tat ggt gtc aga gaa gtg ttt      528
   Tyr Tyr Glu Cys Ser Ala Lys Thr Gly Tyr Gly Val Arg Glu Val Phe
                   165                 170                 175
   gag gcc gcc act aga gct tca ttg atg ggt aaa tct aaa acg aat ggt      576
   Glu Ala Ala Thr Arg Ala Ser Leu Met Gly Lys Ser Lys Thr Asn Gly
               180                 185                 190
   aaa gct aag aag aac act act gaa aag aag aag aag aag tgt gtc ttg 624
   Lys Ala Lys Lys Asn Thr Thr Glu Lys Lys Lys Lys Lys Cys Val Leu
   195 200 205
   tta tag                                                              630
   Leu

<210> 140
<211> 209
<212> PRT
<213> Saccharomyces cerevisiae

<400> 140
   Met Ser Gln Gln Val Gly Asn Ser Ile Arg Arg Lys Leu Val Ile Val
   1               5                   10                  15
   Gly Asp Gly Ala Cys Gly Lys Thr Cys Leu Leu Ile Val Phe Ser Lys
               20                  25                  30
   Gly Gln Phe Pro Glu Val Tyr Val Pro Thr Val Phe Glu Asn Tyr Val
           35                  40                  45
   Ala Asp Val Glu Val Asp Gly Arg Arg Val Glu Leu Ala Leu Trp Asp
       50                  55                  60
   Thr Ala Gly Gln Glu Asp Tyr Asp Arg Leu Arg Pro Leu Ser Tyr Pro
   65                  70                  75                  80
   Asp Ser Asn Val Val Leu Ile Cys Phe Ser Ile Asp Leu Pro Asp Ser
                   85                  90                  95
   Leu Glu Asn Val Gln Glu Lys Trp Ile Ala Glu Val Leu His Phe Cys
               100                 105                 110
   Gln Gly Val Pro Ile Ile Leu Val Gly Cys Lys Val Asp Leu Arg Asn
           115                 120                 125
   Asp Pro Gln Thr Ile Glu Gln Leu Arg Gln Glu Gly Gln Gln Pro Val
       130                 135                 140
   Thr Ser Gln Glu Gly Gln Ser Val Ala Asp Gln Ile Gly Ala Thr Gly
   145                 150                 155                 160
   Tyr Tyr Glu Cys Ser Ala Lys Thr Gly Tyr Gly Val Arg Glu Val Phe
                   165                 170                 175
   Glu Ala Ala Thr Arg Ala Ser Leu Met Gly Lys Ser Lys Thr Asn Gly
               180                 185                 190
   Lys Ala Lys Lys Asn Thr Thr Glu Lys Lys Lys Lys Lys Cys Val Leu
           195                 200                 205
   Leu

<210> 141
<211> 987
<212> DNA
<213> Aplysia californica (California sea hare)

<220>
<221> CDS
<222> (14)..(592)

<400> 141
ccaagtgcca acc atg gca gcg ata cga aag aag ctt gtt ata gtc gga       49
               Met Ala Ala Ile Arg Lys Lys Leu Val Ile Val Gly
               1               5                   10
gat ggt gcg tgt ggt aaa aca tgt cta ctt att gtc ttc agc aaa gac       97
Asp Gly Ala Cys Gly Lys Thr Cys Leu Leu Ile Val Phe Ser Lys Asp
        15                  20                  25
cag ttc cct gaa gtt tac gtg cca aca gtt ttt gaa aat tat gta gca      145
Gln Phe Pro Glu Val Tyr Val Pro Thr Val Phe Glu Asn Tyr Val Ala
    30                  35                  40
gac att gaa gtt gat ggc aaa cag gtt gag cta gct ctg tgg gac aca      193
Asp Ile Glu Val Asp Gly Lys Gln Val Glu Leu Ala Leu Trp Asp Thr
45                  50                  55                  60
gcg gga caa gag gac tat gac aga ctg agg ccg ctg tct tac cct gac      241
Ala Gly Gln Glu Asp Tyr Asp Arg Leu Arg Pro Leu Ser Tyr Pro Asp
                65                  70                  75
aca gat gtc atc ctc atg tgt ttc tct ata gac agt cca gac agt ctg      289
Thr Asp Val Ile Leu Met Cys Phe Ser Ile Asp Ser Pro Asp Ser Leu
            80                  85                  90
gag aac ata ccg gag aag tgg acg cct gag gtt cgt cac ttt tgt cca      337
Glu Asn Ile Pro Glu Lys Trp Thr Pro Glu Val Arg His Phe Cys Pro
        95                  100                 105
aat gtt cct ata ata ctt gtg ggt aac aaa aag gat ctt cgc aac gat      385
Asn Val Pro Ile Ile Leu Val Gly Asn Lys Lys Asp Leu Arg Asn Asp
    110                 115                 120
gaa agt acc aaa cgt gag ctc atg aaa atg aaa cag gaa cca gtg aga      433
Glu Ser Thr Lys Arg Glu Leu Met Lys Met Lys Gln Glu Pro Val Arg
125                 130                 135                 140
cca gag gat ggg cgc gcc atg gct gag aaa atc aac gcc tac tct tat      481

Pro Glu Asp Gly Arg Ala Met Ala Glu Lys Ile Asn Ala Tyr Ser Tyr
                145                 150                 155
ctt gag tgc tct gct aaa acc aag gag ggc gtg agg gat gtg ttt gag      529
Leu Glu Cys Ser Ala Lys Thr Lys Glu Gly Val Arg Asp Val Phe Glu
        160                 165                 170
aca gct acc aga gct gcg ctg caa gtg aaa aag aag aag aag ggt gga      577
Thr Ala Thr Arg Ala Ala Leu Gln Val Lys Lys Lys Lys Lys Gly Gly
    175                 180                 185
tgt gtt gta ttg tgaataagtc gctgttttct tcaattcccc acaacagggc          629
Cys Val Val Leu
    190
tggtgaaagg aggactgcat gaaattttgt cttttgaaga tattcctttt aaaattattt    689

tttttaaaat accaattttc aacttaagat tgttcaatta taattagtgc aaaaagacgg      749

atgcttcccg actgaaacac aagaaaggcc atatcagagt ggcatatgat cacagtcata    809

ttttgtggct tgtgtgattg catatatatt taagaacaaa tataagcgtc aactatctat    869

ggctggagaa tgaggtaatg gaaatgtttt atttgagtaa attcctcatc tgcaaaaagt    929

ttgtactaca gttgagtaaa atttcaagca ttgtctcctt tacaaattgt nngccccc      987

<210> 142
<211> 192
<212> PRT
<213> Aplysia californica (California sea hare)

<400> 142
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Arg His Phe Cys Pro Asn Val Pro Ile
            100                 105                 110
Ile Leu Val Gly Asn Lys Lys Asp Leu Arg Asn Asp Glu Ser Thr Lys
        115                 120                 125
Arg Glu Leu Met Lys Met Lys Gln Glu Pro Val Arg Pro Glu Asp Gly
    130                 135                 140
Arg Ala Met Ala Glu Lys Ile Asn Ala Tyr Ser Tyr Leu Glu Cys Ser
145                 150                 155                 160
Ala Lys Thr Lys Glu Gly Val Arg Asp Val Phe Glu Thr Ala Thr Arg
                165                 170                 175
Ala Ala Leu Gln Val Lys Lys Lys Lys Lys Gly Gly Cys Val Val Leu
            180                 185                 190

<210> 143
<211> 579
<212> DNA
<213> Homo sapiens (man)

<220>

<221> CDS
<222> (1)..(579)

<400> 143
atg cag gcc atc aag tgt gtg gtg gtg gga gac gga gct gta ggt aaa      48
Met Gln Ala Ile Lys Cys Val Val Val Gly Asp Gly Ala Val Gly Lys
1               5                   10                  15
act tgc cta ctg atc agt tac aca acc aat gca ttt cct gga gaa tat      96
Thr Cys Leu Leu Ile Ser Tyr Thr Thr Asn Ala Phe Pro Gly Glu Tyr
            20                  25                  30
atc cct act gtc ttt gac aat tat tct gcc aat gtt atg gta gat gga      144
Ile Pro Thr Val Phe Asp Asn Tyr Ser Ala Asn Val Met Val Asp Gly
        35                  40                  45
aaa ccg gtg aat ctg ggc tta tgg gat aca gct gga caa gaa gat tat      192
Lys Pro Val Asn Leu Gly Leu Trp Asp Thr Ala Gly Gln Glu Asp Tyr
    50                  55                  60
gac aga tta cgc ccc cta tcc tat ccg caa aca gat gtg ttc tta att      240
Asp Arg Leu Arg Pro Leu Ser Tyr Pro Gln Thr Asp Val Phe Leu Ile
65                  70                  75                  80
tgc ttt tcc ctt gtg agt cct gca tca ttt gaa aat gtc cgt gca aag      288
Cys Phe Ser Leu Val Ser Pro Ala Ser Phe Glu Asn Val Arg Ala Lys
                85                  90                  95
tgg tat cct gag gtg cgg cac cac tgt ccc aac act ccc atc atc cta      336
Trp Tyr Pro Glu Val Arg His His Cys Pro Asn Thr Pro Ile Ile Leu
            100                 105                 110
gtg gga act aaa ctt gat ctt agg gat gat aaa gac acg atc gag aaa      384
Val Gly Thr Lys Leu Asp Leu Arg Asp Asp Lys Asp Thr Ile Glu Lys
        115                 120                 125
ctg aag gag aag aag ctg act ccc atc acc tat ccg cag ggt cta gcc      432
Leu Lys Glu Lys Lys Leu Thr Pro Ile Thr Tyr Pro Gln Gly Leu Ala
    130                 135                 140
atg gct aag gag att ggt gct gta aaa tac ctg gag tgc tcg gcg ctc      480
Met Ala Lys Glu Ile Gly Ala Val Lys Tyr Leu Glu Cys Ser Ala Leu
145                 150                 155                 160
aca cag cga ggc ctc aag aca gtg ttt gac gaa gcg atc cga gca gtc      528
Thr Gln Arg Gly Leu Lys Thr Val Phe Asp Glu Ala Ile Arg Ala Val
                165                 170                 175
ctc tgc ccg cct ccc gtg aag aag agg aag aga aaa tgc ctg ctg ttg      576
Leu Cys Pro Pro Pro Val Lys Lys Arg Lys Arg Lys Cys Leu Leu Leu
            180                 185                 190
taa                                                                  579


<210> 144
<211> 192
<212> PRT
<213> Homo sapiens (man)

<400> 144
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly
        35                  40                  45
Lys Pro Val Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Ser Pro Ala Ser Phe Glu Asn Val Arg Ala Lys
                85                  90                  95
Trp Tyr Pro Glu Val Arg His His Cys Pro Asn Thr Pro Ile Ile Leu
            100                 105                 110
Val Gly Thr Lys Leu Asp Leu Arg Asp Asp Lys Asp Thr Ile Glu Lys
        115                 120                 125
Leu Lys Glu Lys Lys Leu Thr Pro Ile Thr Tyr Pro Gln Gly Leu Ala

130                 135                 140
Met Ala Lys Glu Ile Gly Ala Val Lys Tyr Leu Glu Cys Ser Ala Leu
145                 150                 155                 160
Thr Gln Arg Gly Leu Lys Thr Val Phe Asp Glu Ala Ile Arg Ala Val
                165                 170                 175
Leu Cys Pro Pro Pro Val Lys Lys Arg Lys Arg Lys Cys Leu Leu Leu
            180                 185                 190

<210> 145
<211> 642
<212> DNA
<213> Homo sapiens (man)

<220>
<221> CDS
<222> (1)..(642)

<400> 145
atg ccc gga gcc ggc cgc agc agc atg gct cac ggg ccc ggc gcg ctg      48
Met Pro Gly Ala Gly Arg Ser Ser Met Ala His Gly Pro Gly Ala Leu
1               5                   10                  15
atg ctc aag tgc gtg gtg gtc ggc gac ggg gcg gtg ggc aag acg tgc      96
Met Leu Lys Cys Val Val Val Gly Asp Gly Ala Val Gly Lys Thr Cys
            20                  25                  30
cta ctc atg agc tat gcc aac gac gcc ttc ccg gag gag tac gtg ccc      144
Leu Leu Met Ser Tyr Ala Asn Asp Ala Phe Pro Glu Glu Tyr Val Pro
        35                  40                  45
acc gtc ttc gac cac tac gca gtc agc gtc acc gtg ggg ggc aag cag      192
Thr Val Phe Asp His Tyr Ala Val Ser Val Thr Val Gly Gly Lys Gln
    50                  55                  60
tac ctc cta gga ctc tat gac acg gcc gga cag gaa gac tat gac cgt      240
Tyr Leu Leu Gly Leu Tyr Asp Thr Ala Gly Gln Glu Asp Tyr Asp Arg
65                  70                  75                  80
ctg agg cct tta tct tac cca atg acc gat gtc ttc ctt ata tgc ttc      288
Leu Arg Pro Leu Ser Tyr Pro Met Thr Asp Val Phe Leu Ile Cys Phe
                85                  90                  95
tcg gtg gta aat cca gcc tca ttt caa aat gtg aaa gag gag tgg gta      336
Ser Val Val Asn Pro Ala Ser Phe Gln Asn Val Lys Glu Glu Trp Val
            100                 105                 110
ccg gaa ctt aag gaa tac gca cca aat gta ccc ttt tta tta ata gga      384
Pro Glu Leu Lys Glu Tyr Ala Pro Asn Val Pro Phe Leu Leu Ile Gly
        115                 120                 125
act cag att gat ctc cga gat gac ccc aaa act tta gca aga ctg aat      432
Thr Gln Ile Asp Leu Arg Asp Asp Pro Lys Thr Leu Ala Arg Leu Asn
    130                 135                 140
gat atg aaa gaa aaa cct ata tgt gtg gaa caa gga cag aaa cta gca      480
Asp Met Lys Glu Lys Pro Ile Cys Val Glu Gln Gly Gln Lys Leu Ala
145                 150                 155                 160
aaa gag ata gga gca tgc tgc tat gtg gaa tgt tca gct tta acc cag      528
Lys Glu Ile Gly Ala Cys Cys Tyr Val Glu Cys Ser Ala Leu Thr Gln
                165                 170                 175
aag gga ttg aag act gtt ttt gat gag gct atc ata gcc att tta act      576
Lys Gly Leu Lys Thr Val Phe Asp Glu Ala Ile Ile Ala Ile Leu Thr
            180                 185                 190
cca aag aaa cac act gta aaa aaa aga ata gga tca aga tgt ata aac      624
Pro Lys Lys His Thr Val Lys Lys Arg Ile Gly Ser Arg Cys Ile Asn
        195                 200                 205
tgt tgt tta att acg tga                                              642
Cys Cys Leu Ile Thr
    210

<210> 146
<211> 213
<212> PRT
<213> Homo sapiens (man)

<400> 146

Met Pro Gly Ala Gly Arg Ser Ser Met Ala His Gly Pro Gly Ala Leu
1               5                   10                  15
Met Leu Lys Cys Val Val Val Gly Asp Gly Ala Val Gly Lys Thr Cys
            20                  25                  30
Leu Leu Met Ser Tyr Ala Asn Asp Ala Phe Pro Glu Glu Tyr Val Pro
        35                  40                  45
Thr Val Phe Asp His Tyr Ala Val Ser Val Thr Val Gly Gly Lys Gln
    50                  55                  60
Tyr Leu Leu Gly Leu Tyr Asp Thr Ala Gly Gln Glu Asp Tyr Asp Arg
65                  70                  75                  80
Leu Arg Pro Leu Ser Tyr Pro Met Thr Asp Val Phe Leu Ile Cys Phe
                85                  90                  95
Ser Val Val Asn Pro Ala Ser Phe Gln Asn Val Lys Glu Glu Trp Val
            100                 105                 110
Pro Glu Leu Lys Glu Tyr Ala Pro Asn Val Pro Phe Leu Leu Ile Gly
        115                 120                 125
Thr Gln Ile Asp Leu Arg Asp Asp Pro Lys Thr Leu Ala Arg Leu Asn
    130                 135                 140
Asp Met Lys Glu Lys Pro Ile Cys Val Glu Gln Gly Gln Lys Leu Ala
145                 150                 155                 160
Lys Glu Ile Gly Ala Cys Cys Tyr Val Glu Cys Ser Ala Leu Thr Gln
                165                 170                 175
Lys Gly Leu Lys Thr Val Phe Asp Glu Ala Ile Ile Ala Ile Leu Thr
            180                 185                 190
Pro Lys Lys His Thr Val Lys Lys Arg Ile Gly Ser Arg Cys Ile Asn
        195                 200                 205
Cys Cys Leu Ile Thr
    210

<210> 147
<211> 582
<212> DNA
<213> Homo sapiens (man)

<220>
<221> CDS
<222> (1)..(582)

<400> 147
atg gct gca atc cga aag aag ctg gtg atc gtt ggg gat ggt gcc tgt      48
Met Ala Ala Ile Arg Lys Lys Leu Val Ile Val Gly Asp Gly Ala Cys
1               5                   10                  15
ggg aag acc tgc ctc ctc atc gtc ttc agc aag gat cag ttt ccg gag      96
Gly Lys Thr Cys Leu Leu Ile Val Phe Ser Lys Asp Gln Phe Pro Glu
            20                  25                  30
gtc tac gtc cct act gtc ttt gag aac tat att gcg gac att gag gtg      144
Val Tyr Val Pro Thr Val Phe Glu Asn Tyr Ile Ala Asp Ile Glu Val
        35                  40                  45
gac ggc aag cag gtg gag ctg gct ctg tgg gac aca gca ggg cag gaa      192
Asp Gly Lys Gln Val Glu Leu Ala Leu Trp Asp Thr Ala Gly Gln Glu
    50                  55                  60
gac tat gat cga ctg cgg cct ctc tcc tac ccg gac act gat gtc atc      240
Asp Tyr Asp Arg Leu Arg Pro Leu Ser Tyr Pro Asp Thr Asp Val Ile
65                  70                  75                  80
ctc atg tgc ttc tcc atc gac agc cct gac agc ctg gaa aac att cct      288
Leu Met Cys Phe Ser Ile Asp Ser Pro Asp Ser Leu Glu Asn Ile Pro
                85                  90                  95
gag aag tgg acc cca gag gtg aag cac ttc tgc ccc aac gtg ccc atc      336
Glu Lys Trp Thr Pro Glu Val Lys His Phe Cys Pro Asn Val Pro Ile
            100                 105                 110
atc ctg gtg ggg aat aag aag gac ctg agg caa gac gag cac acc agg      384
Ile Leu Val Gly Asn Lys Lys Asp Leu Arg Gln Asp Glu His Thr Arg
        115                 120                 125
aga gag ctg gcc aag atg aag cag gag ccc gtt cgg tct gag gaa ggc      432
Arg Glu Leu Ala Lys Met Lys Gln Glu Pro Val Arg Ser Glu Glu Gly
    130                 135                 140
cgg gac atg gcg aac cgg atc agt gcc ttt ggc tac ctt gag tgc tca      480
Arg Asp Met Ala Asn Arg Ile Ser Ala Phe Gly Tyr Leu Glu Cys Ser

145                 150                 155                 160
gcc aag acc aag gag gga gtg cgg gag gtg ttt gag atg gcc act cgg       528
Ala Lys Thr Lys Glu Gly Val Arg Glu Val Phe Glu Met Ala Thr Arg
                165                 170                 175
gct ggc ctc cag gtc cgc aag aac aag cgt cgg agg ggc tgt ccc att       576
Ala Gly Leu Gln Val Arg Lys Asn Lys Arg Arg Arg Gly Cys Pro Ile
            180                 185                 190
ctc tga                                                               582
Leu

<210> 148
<211> 193
<212> PRT
<213> Homo sapiens (man)

<400> 148
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Ile Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Lys His Phe Cys Pro Asn Val Pro Ile
            100                 105                 110
Ile Leu Val Gly Asn Lys Lys Asp Leu Arg Gln Asp Glu His Thr Arg
        115                 120                 125
Arg Glu Leu Ala Lys Met Lys Gln Glu Pro Val Arg Ser Glu Glu Gly
    130                 135                 140
Arg Asp Met Ala Asn Arg Ile Ser Ala Phe Gly Tyr Leu Glu Cys Ser
145                 150                 155                 160
Ala Lys Thr Lys Glu Gly Val Arg Glu Val Phe Glu Met Ala Thr Arg
                165                 170                 175
Ala Gly Leu Gln Val Arg Lys Asn Lys Arg Arg Arg Gly Cys Pro Ile
            180                 185                 190
Leu

<210> 149
<211> 576
<212> DNA
<213> Homo sapiens (man)

<220>
<221> CDS
<222> (1)..(576)

<400> 149
atg cag agc atc aag tgc gtg gtg gtg ggt gat ggg gct gtg ggc aag       48
Met Gln Ser Ile Lys Cys Val Val Val Gly Asp Gly Ala Val Gly Lys
1               5                   10                  15
acg tgc ctg ctc atc tgc tac aca act aac gct ttc ccc aaa gag tac       96
Thr Cys Leu Leu Ile Cys Tyr Thr Thr Asn Ala Phe Pro Lys Glu Tyr
            20                  25                  30
atc ccc acc gtg ttc gac aat tac agc gcg cag agc gca gtt gac ggg       144
Ile Pro Thr Val Phe Asp Asn Tyr Ser Ala Gln Ser Ala Val Asp Gly
        35                  40                  45
cgc aca gtg aac ctg aac ctg tgg gac act gcg ggc cag gag gag tat       192
Arg Thr Val Asn Leu Asn Leu Trp Asp Thr Ala Gly Gln Glu Glu Tyr
    50                  55                  60
gac cgc ctc cgt aca ctc tcc tac cct cag acc aac gtt ttc gtc atc       240

Asp Arg Leu Arg Thr Leu Ser Tyr Pro Gln Thr Asn Val Phe Val Ile
65                  70                  75                  80
tgt ttc tcc att gcc agt ccg ccg tcc tat gag aac gtg cgg cac aag      288
Cys Phe Ser Ile Ala Ser Pro Pro Ser Tyr Glu Asn Val Arg His Lys
                85                  90                  95
tgg cat cca gag gtg tgc cac cac tgc cct gat gtg ccc atc ctg ctg      336
Trp His Pro Glu Val Cys His His Cys Pro Asp Val Pro Ile Leu Leu
            100                 105                 110
gtg ggc acc aag aag gac ctg aga gcc cag cct gac acc cta cgg cgc      384
Val Gly Thr Lys Lys Asp Leu Arg Ala Gln Pro Asp Thr Leu Arg Arg
        115                 120                 125
ctc aag gag cag agc cag gcg ccc atc aca ccg cag cag ggc cag gca      432
Leu Lys Glu Gln Ser Gln Ala Pro Ile Thr Pro Gln Gln Gly Gln Ala
    130                 135                 140
ctc gcg aaa cag atc cac gct gtg cgc tac ctc gaa tgc tca gcc ctg      480
Leu Ala Lys Gln Ile His Ala Val Arg Tyr Leu Glu Cys Ser Ala Leu
145                 150                 155                 160
caa cag gat ggt gtc aag gaa gtg ttc gcc gag gct gtc cgg gct gtg      528
Gln Gln Asp Gly Val Lys Glu Val Phe Ala Glu Ala Val Arg Ala Val
                165                 170                 175
ctc aac ccc acg ccg atc aag cgt ggg cgg tcc tgc atc ctc ttg          573
Leu Asn Pro Thr Pro Ile Lys Arg Gly Arg Ser Cys Ile Leu Leu
            180                 185                 190
tga                                                                  576

<210> 150
<211> 191
<212> PRT
<213> Homo sapiens (man)

<400> 150
Met Gln Ser Ile Lys Cys Val Val Val Gly Asp Gly Ala Val Gly Lys
1               5                   10                  15
Thr Cys Leu Leu Ile Cys Tyr Thr Thr Asn Ala Phe Pro Lys Glu Tyr
            20                  25                  30
Ile Pro Thr Val Phe Asp Asn Tyr Ser Ala Gln Ser Ala Val Asp Gly
        35                  40                  45
Arg Thr Val Asn Leu Asn Leu Trp Asp Thr Ala Gly Gln Glu Glu Tyr
    50                  55                  60
Asp Arg Leu Arg Thr Leu Ser Tyr Pro Gln Thr Asn Val Phe Val Ile
65                  70                  75                  80
Cys Phe Ser Ile Ala Ser Pro Pro Ser Tyr Glu Asn Val Arg His Lys
                85                  90                  95
Trp His Pro Glu Val Cys His His Cys Pro Asp Val Pro Ile Leu Leu
            100                 105                 110
Val Gly Thr Lys Lys Asp Leu Arg Ala Gln Pro Asp Thr Leu Arg Arg
        115                 120                 125
Leu Lys Glu Gln Ser Gln Ala Pro Ile Thr Pro Gln Gln Gly Gln Ala
    130                 135                 140
Leu Ala Lys Gln Ile His Ala Val Arg Tyr Leu Glu Cys Ser Ala Leu
145                 150                 155                 160
Gln Gln Asp Gly Val Lys Glu Val Phe Ala Glu Ala Val Arg Ala Val
                165                 170                 175
Leu Asn Pro Thr Pro Ile Lys Arg Gly Arg Ser Cys Ile Leu Leu
            180                 185                 190

<210> 151
<211> 2292
<212> DNA
<213> Homo sapiens

<220>
<221> CDS
<222> (1)..(2292)

<400> 151
atg gtg agc aag ggc gag gag ctg ttc acc ggg gtg gtg ccc atc ctg 48
Met Val Ser Lys Gly Glu Glu Leu Phe Thr Gly Val Val Pro Ile Leu
1               5                   10                  15
gtc gag ctg gac ggc gac gta aac ggc cac aag ttc agc gtg tcc ggc 96
Val Glu Leu Asp Gly Asp Val Asn Gly His Lys Phe Ser Val Ser Gly
            20                  25                  30
gag ggc gag ggc gat gcc acc tac ggc aag ctg acc ctg aag ttc atc 144
Glu Gly Glu Gly Asp Ala Thr Tyr Gly Lys Leu Thr Leu Lys Phe Ile
        35                  40                  45
tgc acc acc ggc aag ctg ccc gtg ccc tgg ccc acc ctc gtg acc acc 192
Cys Thr Thr Gly Lys Leu Pro Val Pro Trp Pro Thr Leu Val Thr Thr
    50                  55                  60
ctg ggc tac ggc ctg cag tgc ttc gcc cgc tac ccc gac cac atg aag 240
Leu Gly Tyr Gly Leu Gln Cys Phe Ala Arg Tyr Pro Asp His Met Lys
65                  70                  75                  80
cag cac gac ttc ttc aag tcc gcc atg ccc gaa ggc tac gtc cag gag 288
Gln His Asp Phe Phe Lys Ser Ala Met Pro Glu Gly Tyr Val Gln Glu
                85                  90                  95
cgc acc atc ttc ttc aag gac gac ggc aac tac aag acc cgc gcc gag 336
Arg Thr Ile Phe Phe Lys Asp Asp Gly Asn Tyr Lys Thr Arg Ala Glu
            100                 105                 110
gtg aag ttc gag ggc gac acc ctg gtg aac cgc atc gag ctg aag ggc 384
Val Lys Phe Glu Gly Asp Thr Leu Val Asn Arg Ile Glu Leu Lys Gly
        115                 120                 125
atc gac ttc aag gag gac ggc aac atc ctg ggg cac aag ctg gag tac 432
Ile Asp Phe Lys Glu Asp Gly Asn Ile Leu Gly His Lys Leu Glu Tyr
    130                 135                 140
aac tac aac agc cac aac gtc tat atc acc gcc gac aag cag aag aac 480
Asn Tyr Asn Ser His Asn Val Tyr Ile Thr Ala Asp Lys Gln Lys Asn
145                 150                 155                 160
ggc atc aag gcc aac ttc aag atc cgc cac aac atc gag gac ggc ggc 528
Gly Ile Lys Ala Asn Phe Lys Ile Arg His Asn Ile Glu Asp Gly Gly
                165                 170                 175
gtg cag ctc gcc gac cac tac cag cag aac acc ccc atc ggc gac ggc 576
Val Gln Leu Ala Asp His Tyr Gln Gln Asn Thr Pro Ile Gly Asp Gly
            180                 185                 190
ccc gtg ctg ctg ccc gac aac cac tac ctg agc tac cag tcc gcc ctg 624
Pro Val Leu Leu Pro Asp Asn His Tyr Leu Ser Tyr Gln Ser Ala Leu
        195                 200                 205
agc aaa gac ccc aac gag aag cgc gat cac atg gtc ctg ctg gag ttc 672
Ser Lys Asp Pro Asn Glu Lys Arg Asp His Met Val Leu Leu Glu Phe
    210                 215                 220
gtg acc gcc gcc ctc gag aaa gag aaa gag cgg cca gag att tct ctc 720
Val Thr Ala Ala Leu Glu Lys Glu Lys Glu Arg Pro Glu Ile Ser Leu
225                 230                 235                 240
cct tca gat ttt gaa cac aca att cat gtc ggt ttt gat gct gtc aca 768
Pro Ser Asp Phe Glu His Thr Ile His Val Gly Phe Asp Ala Val Thr
                245                 250                 255
ggg gag ttt acg gga atg cca gag cag tgg gcc cgc ttg ctt cag aca 816
Gly Glu Phe Thr Gly Met Pro Glu Gln Trp Ala Arg Leu Leu Gln Thr
            260                 265                 270
tca aat atc act aag tcg gag cag aag aaa aac ccg cag gct gtt ctg 864
Ser Asn Ile Thr Lys Ser Glu Gln Lys Lys Asn Pro Gln Ala Val Leu
        275                 280                 285
gat gtg ttg gag ttt tac aac tcg aag aag aca tcc aac agc cag aaa 912
Asp Val Leu Glu Phe Tyr Asn Ser Lys Lys Thr Ser Asn Ser Gln Lys
    290                 295                 300
tac atg agc ttt aca gat aag tca gct tcc gga ggt gga acc ggt ggt 960
Tyr Met Ser Phe Thr Asp Lys Ser Ala Ser Gly Gly Gly Thr Gly Gly
305                 310                 315                 320
gga ggt acc atg cag gcc atc aag tgt gtg gtg gtg gga gac gga gct 1008
Gly Gly Thr Met Gln Ala Ile Lys Cys Val Val Val Gly Asp Gly Ala
                325                 330                 335
gta ggt aaa act tgc cta ctg atc agt tac aca acc aat gca ttt cct 1056
Val Gly Lys Thr Cys Leu Leu Ile Ser Tyr Thr Thr Asn Ala Phe Pro
            340                 345                 350
gga gaa tat atc cct act gtc ttt gac aat tat tct gcc aat gtt atg 1104

Gly Glu Tyr Ile Pro Thr Val Phe Asp Asn Tyr Ser Ala Asn Val Met
        355                 360                 365
gta gat gga aaa ccg gtg aat ctg ggc tta tgg gat aca gct gga caa      1152
Val Asp Gly Lys Pro Val Asn Leu Gly Leu Trp Asp Thr Ala Gly Gln
    370                 375                 380
gaa gat tat gac aga tta cgc ccc cta tcc tat ccg caa aca gat gtg      1200
Glu Asp Tyr Asp Arg Leu Arg Pro Leu Ser Tyr Pro Gln Thr Asp Val
385                 390                 395                 400
ttc tta att tgc ttt tcc ctt gtg agt cct gca tca ttt gaa aat gtc      1248
Phe Leu Ile Cys Phe Ser Leu Val Ser Pro Ala Ser Phe Glu Asn Val
                405                 410                 415
cgt gca aag tgg tat cct gag gtg cgg cac cac tgt ccc aac act ccc      1296
Arg Ala Lys Trp Tyr Pro Glu Val Arg His His Cys Pro Asn Thr Pro
            420                 425                 430
atc atc cta gtg gga act aaa ctt gat ctt agg gat gat aaa gac acg      1344
Ile Ile Leu Val Gly Thr Lys Leu Asp Leu Arg Asp Asp Lys Asp Thr
        435                 440                 445
atc gag aaa ctg aag gag aag aag ctg act ccc atc acc tat ccg cag      1392
Ile Glu Lys Leu Lys Glu Lys Lys Leu Thr Pro Ile Thr Tyr Pro Gln
    450                 455                 460
ggt cta gcc atg gct aag gag att ggt gct gta aaa tac ctg gag tgc      1440
Gly Leu Ala Met Ala Lys Glu Ile Gly Ala Val Lys Tyr Leu Glu Cys
465                 470                 475                 480
tcg gcg ctc aca cag cga ggc ctc aag aca gtg ttt gac gaa gcg atc      1488
Ser Ala Leu Thr Gln Arg Gly Leu Lys Thr Val Phe Asp Glu Ala Ile
                485                 490                 495
cga gca gtc cgc ggc cgc atg gtg agc aag ggc gag gag ctg ttc acc      1536
Arg Ala Val Arg Gly Arg Met Val Ser Lys Gly Glu Glu Leu Phe Thr
            500                 505                 510
ggg gtg gtg ccc atc ctg gtc gag ctg gac ggc gac gta aac ggc cac      1584
Gly Val Val Pro Ile Leu Val Glu Leu Asp Gly Asp Val Asn Gly His
        515                 520                 525
aag ttc agc gtg tcc ggc gag ggc gag ggc gat gcc acc tac ggc aag      1632
Lys Phe Ser Val Ser Gly Glu Gly Glu Gly Asp Ala Thr Tyr Gly Lys
    530                 535                 540
ctg acc ctg aag ttc atc tgc acc acc ggc aag ctg ccc gtg ccc tgg      1680
Leu Thr Leu Lys Phe Ile Cys Thr Thr Gly Lys Leu Pro Val Pro Trp
545                 550                 555                 560
ccc acc ctc gtg acc acc ctg acc tgg ggc gtg cag tgc ttc agc cgc      1728
Pro Thr Leu Val Thr Thr Leu Thr Trp Gly Val Gln Cys Phe Ser Arg
                565                 570                 575
tac ccc gac cac atg aag cag cac gac ttc ttc aag tcc gcc atg ccc      1776
Tyr Pro Asp His Met Lys Gln His Asp Phe Phe Lys Ser Ala Met Pro
            580                 585                 590
gaa ggc tac gtc cag gag cgc acc atc ttc ttc aag gac gac ggc aac      1824
Glu Gly Tyr Val Gln Glu Arg Thr Ile Phe Phe Lys Asp Asp Gly Asn
        595                 600                 605
tac aag acc cgc gcc gag gtg aag ttc gag ggc gac acc ctg gtg aac      1872
Tyr Lys Thr Arg Ala Glu Val Lys Phe Glu Gly Asp Thr Leu Val Asn
    610                 615                 620
cgc atc gag ctg aag ggc atc gac ttc aag gag gac ggc aac atc ctg      1920
Arg Ile Glu Leu Lys Gly Ile Asp Phe Lys Glu Asp Gly Asn Ile Leu
625                 630                 635                 640
ggg cac aag ctg gag tac aac tac atc agc cac aac gtc tat atc acc      1968
Gly His Lys Leu Glu Tyr Asn Tyr Ile Ser His Asn Val Tyr Ile Thr
                645                 650                 655
gcc gac aag cag aag aac ggc atc aag gcc aac ttc aag atc cgc cac      2016
Ala Asp Lys Gln Lys Asn Gly Ile Lys Ala Asn Phe Lys Ile Arg His
            660                 665                 670
aac atc gag gac ggc agc gtg cag ctc gcc gac cac tac cag cag aac      2064
Asn Ile Glu Asp Gly Ser Val Gln Leu Ala Asp His Tyr Gln Gln Asn
        675                 680                 685
acc ccc atc ggc gac ggc ccc gtg ctg ctg ccc gac aac cac tac ttg      2112
Thr Pro Ile Gly Asp Gly Pro Val Leu Leu Pro Asp Asn His Tyr Leu
    690                 695                 700
agc acc cag tcc gcc ctg agc aaa gac ccc aac gag aag cgc gat cac      2160
Ser Thr Gln Ser Ala Leu Ser Lys Asp Pro Asn Glu Lys Arg Asp His
705                 710                 715                 720

atg gtc ctg ctg gag ttc gtg acc gcc gcc ggg atc act ctc ggc atg 2208
Met Val Leu Leu Glu Phe Val Thr Ala Ala Gly Ile Thr Leu Gly Met
                725                 730                 735
gac gag ctg ggc cgc tct aga aag atg agc aaa gat ggt aaa aag aag 2256
Asp Glu Leu Gly Arg Ser Arg Lys Met Ser Lys Asp Gly Lys Lys Lys
            740                 745                 750
aaa aag aag tca aag aca aag tgt gta att atg taa 2292
Lys Lys Lys Ser Lys Thr Lys Cys Val Ile Met
        755                 760

<210> 152
<211> 763
<212> PRT
<213> Homo sapiens

<400> 152
Met Val Ser Lys Gly Glu Glu Leu Phe Thr Gly Val Val Pro Ile Leu
1               5                   10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Gly Tyr Gly Leu Gln Cys Phe Ala Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Asn Ser His Asn Val Tyr Ile Thr Ala Asp Lys Gln Lys Asn
145                 150                 155                 160
Gly Ile Lys Ala Asn Phe Lys Ile Arg His Asn Ile Glu Asp Gly Gly
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Tyr Gln Ser Ala Leu
        195                 200                 205
Ser Lys Asp Pro Asn Glu Lys Arg Asp His Met Val Leu Leu Glu Phe
    210                 215                 220
Val Thr Ala Ala Leu Glu Lys Glu Lys Glu Arg Pro Glu Ile Ser Leu
225                 230                 235                 240
Pro Ser Asp Phe Glu His Thr Ile His Val Gly Phe Asp Ala Val Thr
                245                 250                 255
Gly Glu Phe Thr Gly Met Pro Glu Gln Trp Ala Arg Leu Leu Gln Thr
            260                 265                 270
Ser Asn Ile Thr Lys Ser Glu Gln Lys Lys Asn Pro Gln Ala Val Leu
        275                 280                 285
Asp Val Leu Glu Phe Tyr Asn Ser Lys Lys Thr Ser Asn Ser Gln Lys
    290                 295                 300
Tyr Met Ser Phe Thr Asp Lys Ser Ala Ser Gly Gly Gly Thr Gly Gly
305                 310                 315                 320
Gly Gly Thr Met Gln Ala Ile Lys Cys Val Val Val Gly Asp Gly Ala
                325                 330                 335
Val Gly Lys Thr Cys Leu Leu Ile Ser Tyr Thr Thr Asn Ala Phe Pro
            340                 345                 350
Gly Glu Tyr Ile Pro Thr Val Phe Asp Asn Tyr Ser Ala Asn Val Met
        355                 360                 365
Val Asp Gly Lys Pro Val Asn Leu Gly Leu Trp Asp Thr Ala Gly Gln
    370                 375                 380
Glu Asp Tyr Asp Arg Leu Arg Pro Leu Ser Tyr Pro Gln Thr Asp Val
385                 390                 395                 400
Phe Leu Ile Cys Phe Ser Leu Val Ser Pro Ala Ser Phe Glu Asn Val

                405                 410                 415
Arg Ala Lys Trp Tyr Pro Glu Val Arg His His Cys Pro Asn Thr Pro
            420                 425                 430
Ile Ile Leu Val Gly Thr Lys Leu Asp Leu Arg Asp Asp Lys Asp Thr
        435                 440                 445
Ile Glu Lys Leu Lys Glu Lys Lys Leu Thr Pro Ile Thr Tyr Pro Gln
    450                 455                 460
Gly Leu Ala Met Ala Lys Glu Ile Gly Ala Val Lys Tyr Leu Glu Cys
465                 470                 475                 480
Ser Ala Leu Thr Gln Arg Gly Leu Lys Thr Val Phe Asp Glu Ala Ile
                485                 490                 495
Arg Ala Val Arg Gly Arg Met Val Ser Lys Gly Glu Glu Leu Phe Thr
            500                 505                 510
Gly Val Val Pro Ile Leu Val Glu Leu Asp Gly Asp Val Asn Gly His
        515                 520                 525
Lys Phe Ser Val Ser Gly Glu Gly Glu Gly Asp Ala Thr Tyr Gly Lys
    530                 535                 540
Leu Thr Leu Lys Phe Ile Cys Thr Thr Gly Lys Leu Pro Val Pro Trp
545                 550                 555                 560
Pro Thr Leu Val Thr Thr Leu Thr Trp Gly Val Gln Cys Phe Ser Arg
                565                 570                 575
Tyr Pro Asp His Met Lys Gln His Asp Phe Phe Lys Ser Ala Met Pro
            580                 585                 590
Glu Gly Tyr Val Gln Glu Arg Thr Ile Phe Phe Lys Asp Asp Gly Asn
        595                 600                 605
Tyr Lys Thr Arg Ala Glu Val Lys Phe Glu Gly Asp Thr Leu Val Asn
    610                 615                 620
Arg Ile Glu Leu Lys Gly Ile Asp Phe Lys Glu Asp Gly Asn Ile Leu
625                 630                 635                 640
Gly His Lys Leu Glu Tyr Asn Tyr Ile Ser His Asn Val Tyr Ile Thr
                645                 650                 655
Ala Asp Lys Gln Lys Asn Gly Ile Lys Ala Asn Phe Lys Ile Arg His
            660                 665                 670
Asn Ile Glu Asp Gly Ser Val Gln Leu Ala Asp His Tyr Gln Gln Asn
        675                 680                 685
Thr Pro Ile Gly Asp Gly Pro Val Leu Leu Pro Asp Asn His Tyr Leu
    690                 695                 700
Ser Thr Gln Ser Ala Leu Ser Lys Asp Pro Asn Glu Lys Arg Asp His
705                 710                 715                 720
Met Val Leu Leu Glu Phe Val Thr Ala Ala Gly Ile Thr Leu Gly Met
                725                 730                 735
Asp Glu Leu Gly Arg Ser Arg Lys Met Ser Lys Asp Gly Lys Lys Lys
            740                 745                 750
Lys Lys Lys Ser Lys Thr Lys Cys Val Ile Met
        755                 760

<210> 153
<211> 2289
<212> DNA
<213> Homo sapiens

<220>
<221> CDS
<222> (1)..(2289)

<400> 153
atg gtg agc aag ggc gag gag ctg ttc acc ggg gtg gtg ccc atc ctg 48
Met Val Ser Lys Gly Glu Glu Leu Phe Thr Gly Val Val Pro Ile Leu
1               5                   10                  15
gtc gag ctg gac ggc gac gta aac ggc cac aag ttc agc gtg tcc ggc 96
Val Glu Leu Asp Gly Asp Val Asn Gly His Lys Phe Ser Val Ser Gly
            20                  25                  30
gag ggc gag ggc gat gcc acc tac ggc aag ctg acc ctg aag ttc atc 144
Glu Gly Glu Gly Asp Ala Thr Tyr Gly Lys Leu Thr Leu Lys Phe Ile
        35                  40                  45
tgc acc acc ggc aag ctg ccc gtg ccc tgg ccc acc ctc gtg acc acc 192
Cys Thr Thr Gly Lys Leu Pro Val Pro Trp Pro Thr Leu Val Thr Thr
    50                  55                  60

ctg ggc tac ggc ctg cag tgc ttc gcc cgc tac ccc gac cac atg aag 240
Leu Gly Tyr Gly Leu Gln Cys Phe Ala Arg Tyr Pro Asp His Met Lys
65                  70                  75                  80
cag cac gac ttc ttc aag tcc gcc atg ccc gaa ggc tac gtc cag gag 288
Gln His Asp Phe Phe Lys Ser Ala Met Pro Glu Gly Tyr Val Gln Glu
                85                  90                  95
cgc acc atc ttc ttc aag gac gac ggc aac tac aag acc cgc gcc gag 336
Arg Thr Ile Phe Phe Lys Asp Asp Gly Asn Tyr Lys Thr Arg Ala Glu
            100                 105                 110
gtg aag ttc gag ggc gac acc ctg gtg aac cgc atc gag ctg aag ggc 384
Val Lys Phe Glu Gly Asp Thr Leu Val Asn Arg Ile Glu Leu Lys Gly
        115                 120                 125
atc gac ttc aag gag gac ggc aac atc ctg ggg cac aag ctg gag tac 432
Ile Asp Phe Lys Glu Asp Gly Asn Ile Leu Gly His Lys Leu Glu Tyr
    130                 135                 140
aac tac aac agc cac aac gtc tat atc acc gcc gac aag cag aag aac 480
Asn Tyr Asn Ser His Asn Val Tyr Ile Thr Ala Asp Lys Gln Lys Asn
145                 150                 155                 160
ggc atc aag gcc aac ttc aag atc cgc cac aac atc gag gac ggc ggc 528
Gly Ile Lys Ala Asn Phe Lys Ile Arg His Asn Ile Glu Asp Gly Gly
                165                 170                 175
gtg cag ctc gcc gac cac tac cag cag aac acc ccc atc ggc gac ggc 576
Val Gln Leu Ala Asp His Tyr Gln Gln Asn Thr Pro Ile Gly Asp Gly
            180                 185                 190
ccc gtg ctg ctg ccc gac aac cac tac ctg agc tac cag tcc gcc ctg 624
Pro Val Leu Leu Pro Asp Asn His Tyr Leu Ser Tyr Gln Ser Ala Leu
        195                 200                 205
agc aaa gac ccc aac gag aag cgc gat cac atg gtc ctg ctg gag ttc 672
Ser Lys Asp Pro Asn Glu Lys Arg Asp His Met Val Leu Leu Glu Phe
    210                 215                 220
gtg acc gcc gcc ctc gag aaa gag aaa gag cgg cca gag att tct ctc 720
Val Thr Ala Ala Leu Glu Lys Glu Lys Glu Arg Pro Glu Ile Ser Leu
225                 230                 235                 240
cct tca gat ttt gaa cac aca att cat gtc ggt ttt gat gct gtc aca 768
Pro Ser Asp Phe Glu His Thr Ile His Val Gly Phe Asp Ala Val Thr
                245                 250                 255
ggg gag ttt acg gga atg cca gag cag tgg gcc cgc ttg ctt cag aca 816
Gly Glu Phe Thr Gly Met Pro Glu Gln Trp Ala Arg Leu Leu Gln Thr
            260                 265                 270
tca aat atc act aag tcg gag cag aag aaa aac ccg cag gct gtt ctg 864
Ser Asn Ile Thr Lys Ser Glu Gln Lys Lys Asn Pro Gln Ala Val Leu
        275                 280                 285
gat gtg ttg gag ttt tac aac tcg aag aag aca tcc aac agc cag aaa 912
Asp Val Leu Glu Phe Tyr Asn Ser Lys Lys Thr Ser Asn Ser Gln Lys
    290                 295                 300
tac atg agc ttt aca gat aag tca gct tcc gga ggt gga acc ggt ggt 960
Tyr Met Ser Phe Thr Asp Lys Ser Ala Ser Gly Gly Gly Thr Gly Gly
305                 310                 315                 320
gga ggt acc cag aca att aag tgt gtt gtt gtg ggc gat ggt gct gtt 1008
Gly Gly Thr Gln Thr Ile Lys Cys Val Val Val Gly Asp Gly Ala Val
                325                 330                 335
ggt aaa aca tgt ctc ctg ata tcc tac aca aca aac aaa ttt cca tcg 1056
Gly Lys Thr Cys Leu Leu Ile Ser Tyr Thr Thr Asn Lys Phe Pro Ser
            340                 345                 350
gaa tat gta ccg act gtt ttt gac aac tat gca gtc aca gtt atg att 1104
Glu Tyr Val Pro Thr Val Phe Asp Asn Tyr Ala Val Thr Val Met Ile
        355                 360                 365
ggt gga gaa cca tat act ctt gga ctt ttt gat act gca ggg caa gag 1152
Gly Gly Glu Pro Tyr Thr Leu Gly Leu Phe Asp Thr Ala Gly Gln Glu
    370                 375                 380
gat tat gac aga tta cga ccg ctg agt tat cca caa aca gat gta ttt 1200
Asp Tyr Asp Arg Leu Arg Pro Leu Ser Tyr Pro Gln Thr Asp Val Phe
385                 390                 395                 400
cta gtc tgt ttt tca gtg gtc tct cca tct tca ttt gaa aac gtg aaa 1248
Leu Val Cys Phe Ser Val Val Ser Pro Ser Ser Phe Glu Asn Val Lys
                405                 410                 415
gaa aag tgg gtg cct gag ata act cac cac tgt cca aag act cct ttc 1296
Glu Lys Trp Val Pro Glu Ile Thr His His Cys Pro Lys Thr Pro Phe

            420                 425                 430
ttg ctt gtt ggg act caa att gat ctc aga gat gac ccc tct act att 1344
Leu Leu Val Gly Thr Gln Ile Asp Leu Arg Asp Asp Pro Ser Thr Ile
        435                 440                 445
gag aaa ctt gcc aag aac aaa cag aag cct atc act cca gag act gct 1392
Glu Lys Leu Ala Lys Asn Lys Gln Lys Pro Ile Thr Pro Glu Thr Ala
    450                 455                 460
gaa aag ctg gcc cgt gac ctg aag gct gtc aag tat gtg gag tgt tct 1440
Glu Lys Leu Ala Arg Asp Leu Lys Ala Val Lys Tyr Val Glu Cys Ser
465                 470                 475                 480
gca ctt aca cag aga ggt ctg aag aat gtg ttt gat gag gct atc cta 1488
Ala Leu Thr Gln Arg Gly Leu Lys Asn Val Phe Asp Glu Ala Ile Leu
                485                 490                 495
gct gcc ggc ggc cgc atg gtg agc aag ggc gag gag ctg ttc acc ggg 1536
Ala Ala Gly Gly Arg Met Val Ser Lys Gly Glu Glu Leu Phe Thr Gly
            500                 505                 510
gtg gtg ccc atc ctg gtc gag ctg gac ggc gac gta aac ggc cac aag 1584
Val Val Pro Ile Leu Val Glu Leu Asp Gly Asp Val Asn Gly His Lys
        515                 520                 525
ttc agc gtg tcc ggc gag ggc gag ggc gat gcc acc tac ggc aag ctg 1632
Phe Ser Val Ser Gly Glu Gly Glu Gly Asp Ala Thr Tyr Gly Lys Leu
    530                 535                 540
acc ctg aag ttc atc tgc acc acc ggc aag ctg ccc gtg ccc tgg ccc 1680
Thr Leu Lys Phe Ile Cys Thr Thr Gly Lys Leu Pro Val Pro Trp Pro
545                 550                 555                 560
acc ctc gtg acc acc ctg acc tgg ggc gtg cag tgc ttc agc cgc tac 1728
Thr Leu Val Thr Thr Leu Thr Trp Gly Val Gln Cys Phe Ser Arg Tyr
                565                 570                 575
ccc gac cac atg aag cag cac gac ttc ttc aag tcc gcc atg ccc gaa 1776
Pro Asp His Met Lys Gln His Asp Phe Phe Lys Ser Ala Met Pro Glu
            580                 585                 590
ggc tac gtc cag gag cgc acc atc ttc ttc aag gac gac ggc aac tac 1824
Gly Tyr Val Gln Glu Arg Thr Ile Phe Phe Lys Asp Asp Gly Asn Tyr
        595                 600                 605
aag acc cgc gcc gag gtg aag ttc gag ggc gac acc ctg gtg aac cgc 1872
Lys Thr Arg Ala Glu Val Lys Phe Glu Gly Asp Thr Leu Val Asn Arg
    610                 615                 620
atc gag ctg aag ggc atc gac ttc aag gag gac ggc aac atc ctg ggg 1920
Ile Glu Leu Lys Gly Ile Asp Phe Lys Glu Asp Gly Asn Ile Leu Gly
625                 630                 635                 640
cac aag ctg gag tac aac tac atc agc cac aac gtc tat atc acc gcc 1968
His Lys Leu Glu Tyr Asn Tyr Ile Ser His Asn Val Tyr Ile Thr Ala
                645                 650                 655
gac aag cag aag aac ggc atc aag gcc aac ttc aag atc cgc cac aac 2016
Asp Lys Gln Lys Asn Gly Ile Lys Ala Asn Phe Lys Ile Arg His Asn
            660                 665                 670
atc gag gac ggc agc gtg cag ctc gcc gac cac tac cag cag aac acc 2064
Ile Glu Asp Gly Ser Val Gln Leu Ala Asp His Tyr Gln Gln Asn Thr
        675                 680                 685
ccc atc ggc gac ggc ccc gtg ctg ctg ccc gac aac cac tac ttg agc 2112
Pro Ile Gly Asp Gly Pro Val Leu Leu Pro Asp Asn His Tyr Leu Ser
    690                 695                 700
acc cag tcc gcc ctg agc aaa gac ccc aac gag aag cgc gat cac atg 2160
Thr Gln Ser Ala Leu Ser Lys Asp Pro Asn Glu Lys Arg Asp His Met
705                 710                 715                 720
gtc ctg ctg gag ttc gtg acc gcc gcc ggg atc act ctc ggc atg gac 2208
Val Leu Leu Glu Phe Val Thr Ala Ala Gly Ile Thr Leu Gly Met Asp
                725                 730                 735
gag ctg ggc cgc tct aga aag atg agc aaa gat ggt aaa aag aag aaa 2256
Glu Leu Gly Arg Ser Arg Lys Met Ser Lys Asp Gly Lys Lys Lys Lys
            740                 745                 750
aag aag tca aag aca aag tgt gta att atg taa 2289
Lys Lys Ser Lys Thr Lys Cys Val Ile Met
        755                 760

<210> 154
<211> 762

<212> PRT
<213> Homo sapiens

<400> 154
Met Val Ser Lys Gly Glu Glu Leu Phe Thr Gly Val Val Pro Ile Leu
1               5                   10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Gly Tyr Gly Leu Gln Cys Phe Ala Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Asn Ser His Asn Val Tyr Ile Thr Ala Asp Lys Gln Lys Asn
145                 150                 155                 160
Gly Ile Lys Ala Asn Phe Lys Ile Arg His Asn Ile Glu Asp Gly Gly
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Tyr Gln Ser Ala Leu
        195                 200                 205
Ser Lys Asp Pro Asn Glu Lys Arg Asp His Met Val Leu Leu Glu Phe
    210                 215                 220
Val Thr Ala Ala Leu Glu Lys Glu Lys Glu Arg Pro Glu Ile Ser Leu
225                 230                 235                 240
Pro Ser Asp Phe Glu His Thr Ile His Val Gly Phe Asp Ala Val Thr
                245                 250                 255
Gly Glu Phe Thr Gly Met Pro Glu Gln Trp Ala Arg Leu Leu Gln Thr
            260                 265                 270
Ser Asn Ile Thr Lys Ser Glu Gln Lys Lys Asn Pro Gln Ala Val Leu
        275                 280                 285
Asp Val Leu Glu Phe Tyr Asn Ser Lys Lys Thr Ser Asn Ser Gln Lys
    290                 295                 300
Tyr Met Ser Phe Thr Asp Lys Ser Ala Ser Gly Gly Gly Thr Gly Gly
305                 310                 315                 320
Gly Gly Thr Gln Thr Ile Lys Cys Val Val Val Gly Asp Gly Ala Val
                325                 330                 335
Gly Lys Thr Cys Leu Leu Ile Ser Tyr Thr Thr Asn Lys Phe Pro Ser
            340                 345                 350
Glu Tyr Val Pro Thr Val Phe Asp Asn Tyr Ala Val Thr Val Met Ile
        355                 360                 365
Gly Gly Glu Pro Tyr Thr Leu Gly Leu Phe Asp Thr Ala Gly Gln Glu
    370                 375                 380
Asp Tyr Asp Arg Leu Arg Pro Leu Ser Tyr Pro Gln Thr Asp Val Phe
385                 390                 395                 400
Leu Val Cys Phe Ser Val Val Ser Pro Ser Ser Phe Glu Asn Val Lys
                405                 410                 415
Glu Lys Trp Val Pro Glu Ile Thr His His Cys Pro Lys Thr Pro Phe
            420                 425                 430
Leu Leu Val Gly Thr Gln Ile Asp Leu Arg Asp Asp Pro Ser Thr Ile
        435                 440                 445
Glu Lys Leu Ala Lys Asn Lys Gln Lys Pro Ile Thr Pro Glu Thr Ala
    450                 455                 460
Glu Lys Leu Ala Arg Asp Leu Lys Ala Val Lys Tyr Val Glu Cys Ser
465                 470                 475                 480
Ala Leu Thr Gln Arg Gly Leu Lys Asn Val Phe Asp Glu Ala Ile Leu
                485                 490                 495
Ala Ala Gly Gly Arg Met Val Ser Lys Gly Glu Glu Leu Phe Thr Gly
            500                 505                 510

Val Val Pro Ile Leu Val Glu Leu Asp Gly Asp Val Asn Gly His Lys
        515                 520                 525
Phe Ser Val Ser Gly Glu Gly Glu Gly Asp Ala Thr Tyr Gly Lys Leu
    530                 535                 540
Thr Leu Lys Phe Ile Cys Thr Thr Gly Lys Leu Pro Val Pro Trp Pro
545                 550                 555                 560
Thr Leu Val Thr Thr Leu Thr Trp Gly Val Gln Cys Phe Ser Arg Tyr
                565                 570                 575
Pro Asp His Met Lys Gln His Asp Phe Phe Lys Ser Ala Met Pro Glu
            580                 585                 590
Gly Tyr Val Gln Glu Arg Thr Ile Phe Phe Lys Asp Asp Gly Asn Tyr
        595                 600                 605
Lys Thr Arg Ala Glu Val Lys Phe Glu Gly Asp Thr Leu Val Asn Arg
    610                 615                 620
Ile Glu Leu Lys Gly Ile Asp Phe Lys Glu Asp Gly Asn Ile Leu Gly
625                 630                 635                 640
His Lys Leu Glu Tyr Asn Tyr Ile Ser His Asn Val Tyr Ile Thr Ala
                645                 650                 655
Asp Lys Gln Lys Asn Gly Ile Lys Ala Asn Phe Lys Ile Arg His Asn
            660                 665                 670
Ile Glu Asp Gly Ser Val Gln Leu Ala Asp His Tyr Gln Gln Asn Thr
        675                 680                 685
Pro Ile Gly Asp Gly Pro Val Leu Leu Pro Asp Asn His Tyr Leu Ser
    690                 695                 700
Thr Gln Ser Ala Leu Ser Lys Asp Pro Asn Glu Lys Arg Asp His Met
705                 710                 715                 720
Val Leu Leu Glu Phe Val Thr Ala Ala Gly Ile Thr Leu Gly Met Asp
                725                 730                 735
Glu Leu Gly Arg Ser Arg Lys Met Ser Lys Asp Gly Lys Lys Lys Lys
            740                 745                 750
Lys Lys Ser Lys Thr Lys Cys Val Ile Met
        755                 760

<210> 155
<211> 1139
<212> DNA
<213> Arabidopsis thaliana

<220>
<221> CDS
<222> (66)..(875)

<400> 155
tcgacccacg cgtccggtct agtcttttat attgtagata atatttctat ctggacataa      60

caaca atg gca gat gca aac gaa gta gtg gaa tgg gcc aac aat tta aag     110
      Met Ala Asp Ala Asn Glu Val Val Glu Trp Ala Asn Asn Leu Lys
      1               5                   10                  15
tgg gat aaa tat gct gga aat aaa gaa att gtt gat gga ttg gcc gct       158
Trp Asp Lys Tyr Ala Gly Asn Lys Glu Ile Val Asp Gly Leu Ala Ala
                20                  25                  30
ttg aga caa aaa atc aca gct ttg gac ggc gct gaa gga ggc tct ggt       206
Leu Arg Gln Lys Ile Thr Ala Leu Asp Gly Ala Glu Gly Gly Ser Gly
            35                  40                  45
ggc aat caa aga gtt aaa tta gta gtt gtc ggt gat ggt gcc gtc ggc       254
Gly Asn Gln Arg Val Lys Leu Val Val Val Gly Asp Gly Ala Val Gly
        50                  55                  60
aaa aca tct ctg ttg att tca ttc gct gaa aac aaa ttc cct gag gat       302
Lys Thr Ser Leu Leu Ile Ser Phe Ala Glu Asn Lys Phe Pro Glu Asp
    65                  70                  75
tac gtc cct aca gtc ttc gaa aac tac act tct aaa atc act cgc gac       350
Tyr Val Pro Thr Val Phe Glu Asn Tyr Thr Ser Lys Ile Thr Arg Asp
80                  85                  90                  95
gat ggt aca ctt gtt ctg ttg cat ttg tgg gat aca gcc ggt caa gag       398
Asp Gly Thr Leu Val Leu Leu His Leu Trp Asp Thr Ala Gly Gln Glu
                100                 105                 110
gac tac gac cga ttg aga cct ttg agt tat cct ggc gct gat gtc atc       446

Asp Tyr Asp Arg Leu Arg Pro Leu Ser Tyr Pro Gly Ala Asp Val Ile
            115                 120                 125
ctg ctc tgt ttc tcc acc gtc act gcc tcg tca ttc gcc tcc att aaa 494
Leu Leu Cys Phe Ser Thr Val Thr Ala Ser Ser Phe Ala Ser Ile Lys
        130                 135                 140
gaa aag tgg tac ccc gaa gtc aac cac tac gtg ccc gat gct cct tat 542
Glu Lys Trp Tyr Pro Glu Val Asn His Tyr Val Pro Asp Ala Pro Tyr
    145                 150                 155
att ttg gtc ggc acc aag ctc gac ttg cgt gag gct ggt ctg ccc gat 590
Ile Leu Val Gly Thr Lys Leu Asp Leu Arg Glu Ala Gly Leu Pro Asp
160                 165                 170                 175
ccc tcc act ggt gag tca gat ccc gtc acc ccc gag aag gct gag gag 638
Pro Ser Thr Gly Glu Ser Asp Pro Val Thr Pro Glu Lys Ala Glu Glu
                180                 185                 190
atg cga aag caa atc aaa gcg ttg aag tat att gaa gtc tct gcc aag 686
Met Arg Lys Gln Ile Lys Ala Leu Lys Tyr Ile Glu Val Ser Ala Lys
            195                 200                 205
aca cga aag aac ttg cag act ctg ttt tca gaa gcc gtg gac att gtc 734
Thr Arg Lys Asn Leu Gln Thr Leu Phe Ser Glu Ala Val Asp Ile Val
        210                 215                 220
ttg gct gaa cga cgc gca cac gag cct ccc aag ccc acc gct gct tca 782
Leu Ala Glu Arg Arg Ala His Glu Pro Pro Lys Pro Thr Ala Ala Ser
    225                 230                 235
tcg acg aac ggc act aac ggc act aag act gag cac act cct cgc gaa 830
Ser Thr Asn Gly Thr Asn Gly Thr Lys Thr Glu His Thr Pro Arg Glu
240                 245                 250                 255
gag ccc atc cga aag aag aag gag cgt cgc tgc ttg ttg atg taagagccct 882
Glu Pro Ile Arg Lys Lys Lys Glu Arg Arg Cys Leu Leu Met
                260                 265
gtgattggtc actctaacat cgactcgaga tctgcactat tactatttta tttgtcgttt 942

ttaactttat ttaatcaaaa aaccctttca accaataagc cacgataaga gtcgtgcagt 1002

caaatttatt ttggtagccc ttggatctgc tatcaagatt ttcttgtatt atcgacttgt 1062

aattaatagg actgtagcga tatcgtgctt ctttatttat ttcaataaaa acaaaaaatt 1122

aaaaaaaaaa aaaaaaa 1139

<210> 156
<211> 269
<212> PRT
<213> Arabidopsis thaliana

<400> 156
Met Ala Asp Ala Asn Glu Val Val Glu Trp Ala Asn Asn Leu Lys Trp
1               5                   10                  15
Asp Lys Tyr Ala Gly Asn Lys Glu Ile Val Asp Gly Leu Ala Ala Leu
            20                  25                  30
Arg Gln Lys Ile Thr Ala Leu Asp Gly Ala Glu Gly Gly Ser Gly Gly
        35                  40                  45
Asn Gln Arg Val Lys Leu Val Val Val Gly Asp Gly Ala Val Gly Lys
    50                  55                  60
Thr Ser Leu Leu Ile Ser Phe Ala Glu Asn Lys Phe Pro Glu Asp Tyr
65                  70                  75                  80
Val Pro Thr Val Phe Glu Asn Tyr Thr Ser Lys Ile Thr Arg Asp Asp
                85                  90                  95
Gly Thr Leu Val Leu Leu His Leu Trp Asp Thr Ala Gly Gln Glu Asp
            100                 105                 110
Tyr Asp Arg Leu Arg Pro Leu Ser Tyr Pro Gly Ala Asp Val Ile Leu
        115                 120                 125

Leu Cys Phe Ser Thr Val Thr Ala Ser Ser Phe Ala Ser Ile Lys Glu
    130                 135                 140
Lys Trp Tyr Pro Glu Val Asn His Tyr Val Pro Asp Ala Pro Tyr Ile
145                 150                 155                 160
Leu Val Gly Thr Lys Leu Asp Leu Arg Glu Ala Gly Leu Pro Asp Pro
                165                 170                 175
Ser Thr Gly Glu Ser Asp Pro Val Thr Pro Glu Lys Ala Glu Glu Met
            180                 185                 190
Arg Lys Gln Ile Lys Ala Leu Lys Tyr Ile Glu Val Ser Ala Lys Thr
        195                 200                 205
Arg Lys Asn Leu Gln Thr Leu Phe Ser Glu Ala Val Asp Ile Val Leu
    210                 215                 220
Ala Glu Arg Arg Ala His Glu Pro Pro Lys Pro Thr Ala Ala Ser Ser
225                 230                 235                 240
Thr Asn Gly Thr Asn Gly Thr Lys Thr Glu His Thr Pro Arg Glu Glu
                245                 250                 255
Pro Ile Arg Lys Lys Lys Glu Arg Arg Cys Leu Leu Met
            260                 265

<210> 157
<211> 582
<212> DNA
<213> Caenorhabditis elegans

<220>
<221> CDS
<222> (1)..(582)

<400> 157
atg caa gca atc aaa tgt gtc gtc gtt ggt gac gga gcc gtc ggt aag      48
Met Gln Ala Ile Lys Cys Val Val Val Gly Asp Gly Ala Val Gly Lys
1               5                   10                  15
acg tgt ctc ctg cta tcg tac act aca aac gct ttt ccc gga gaa tat      96
Thr Cys Leu Leu Leu Ser Tyr Thr Thr Asn Ala Phe Pro Gly Glu Tyr
            20                  25                  30
att cta acg gtg ttc gac acc tac tca aca aat gtg atg gtc gac gga      144
Ile Leu Thr Val Phe Asp Thr Tyr Ser Thr Asn Val Met Val Asp Gly
        35                  40                  45
cgg cca ata aat ctc agc cta tgg gac aca gct gga cag gaa gat tac      192
Arg Pro Ile Asn Leu Ser Leu Trp Asp Thr Ala Gly Gln Glu Asp Tyr
    50                  55                  60
gat caa ttc cgc caa cat ttc cac aaa aaa ttt cca caa aca gac gta      240
Asp Gln Phe Arg Gln His Phe His Lys Lys Phe Pro Gln Thr Asp Val
65                  70                  75                  80
ttt ctc gta tgc ttt gca ttg aat aat aat gtt cgt gca aaa tgg tat      288
Phe Leu Val Cys Phe Ala Leu Asn Asn Asn Val Arg Ala Lys Trp Tyr
                85                  90                  95
cca gaa gta tca cat cat tgc cca aat aca ccg att att ttg gtt gga      336
Pro Glu Val Ser His His Cys Pro Asn Thr Pro Ile Ile Leu Val Gly
            100                 105                 110
acg aaa gct gat ttg cgc gag gat cga gat act gtt gaa cgg ctc cgc      384
Thr Lys Ala Asp Leu Arg Glu Asp Arg Asp Thr Val Glu Arg Leu Arg
        115                 120                 125
gaa agc cgg ctc caa cca gtg agc cac acc cag ggt tac gtg atg gca      432
Glu Ser Arg Leu Gln Pro Val Ser His Thr Gln Gly Tyr Val Met Ala
    130                 135                 140
aag gaa atc aag gcg gtc aag tac ctg gaa tgc tcg gcg ctt acc caa      480
Lys Glu Ile Lys Ala Val Lys Tyr Leu Glu Cys Ser Ala Leu Thr Gln
145                 150                 155                 160
att gga ttg aaa caa gtt ttc gat gag gca att cgt act ggg ctc acc      528
Ile Gly Leu Lys Gln Val Phe Asp Glu Ala Ile Arg Thr Gly Leu Thr
                165                 170                 175
ccg cca caa aca cca caa acg aga gcc aaa aag agc aat tgc acg gtg      576
Pro Pro Gln Thr Pro Gln Thr Arg Ala Lys Lys Ser Asn Cys Thr Val
            180                 185                 190
ctt taa                                                              582
Leu

<210> 158
<211> 193
<212> PRT
<213> Caenorhabditis elegans

<400> 158
Met Gln Ala Ile Lys Cys Val Val Val Gly Asp Gly Ala Val Gly Lys
1               5                   10                  15
Thr Cys Leu Leu Leu Ser Tyr Thr Thr Asn Ala Phe Pro Gly Glu Tyr
            20                  25                  30
Ile Leu Thr Val Phe Asp Thr Tyr Ser Thr Asn Val Met Val Asp Gly
        35                  40                  45
Arg Pro Ile Asn Leu Ser Leu Trp Asp Thr Ala Gly Gln Glu Asp Tyr
    50                  55                  60
Asp Gln Phe Arg Gln His Phe His Lys Lys Phe Pro Gln Thr Asp Val
65                  70                  75                  80
Phe Leu Val Cys Phe Ala Leu Asn Asn Asn Val Arg Ala Lys Trp Tyr
                85                  90                  95
Pro Glu Val Ser His His Cys Pro Asn Thr Pro Ile Ile Leu Val Gly
            100                 105                 110
Thr Lys Ala Asp Leu Arg Glu Asp Arg Asp Thr Val Glu Arg Leu Arg
        115                 120                 125
Glu Ser Arg Leu Gln Pro Val Ser His Thr Gln Gly Tyr Val Met Ala
    130                 135                 140
Lys Glu Ile Lys Ala Val Lys Tyr Leu Glu Cys Ser Ala Leu Thr Gln
145                 150                 155                 160
Ile Gly Leu Lys Gln Val Phe Asp Glu Ala Ile Arg Thr Gly Leu Thr
                165                 170                 175
Pro Pro Gln Thr Pro Gln Thr Arg Ala Lys Lys Ser Asn Cys Thr Val
            180                 185                 190
Leu


<210> 159
<211> 576
<212> DNA
<213> Ciona savignyi

<220>
<221> CDS
<222> (1)..(576)

<400> 159
atg caa act ata aag tgt gta gtt gtt gga gat ggt gct gtg ggt aaa      48
Met Gln Thr Ile Lys Cys Val Val Val Gly Asp Gly Ala Val Gly Lys
1               5                   10                  15
acc tgt ttg cta ata tcc tat acc aca aac aag ttt cca caa gaa tat      96
Thr Cys Leu Leu Ile Ser Tyr Thr Thr Asn Lys Phe Pro Gln Glu Tyr
            20                  25                  30
gtt cca acg gtc ttc gac aac tat gcc gtc act gtt atg atc gga ggg      144
Val Pro Thr Val Phe Asp Asn Tyr Ala Val Thr Val Met Ile Gly Gly
        35                  40                  45
gaa cca tac aca ttg ggt tta ttt gat act gca ggc cag gaa gat tat      192
Glu Pro Tyr Thr Leu Gly Leu Phe Asp Thr Ala Gly Gln Glu Asp Tyr
    50                  55                  60
gac agg tta agg cct ctc agt tat cca caa act gat gtg ttt ttg gtt      240
Asp Arg Leu Arg Pro Leu Ser Tyr Pro Gln Thr Asp Val Phe Leu Val
65                  70                  75                  80
tgt ttt tct gtt gta tca ccg tca tca tat gaa aat ata aaa gaa aag      288
Cys Phe Ser Val Val Ser Pro Ser Ser Tyr Glu Asn Ile Lys Glu Lys
                85                  90                  95
tgg gtc ccg gaa atc acc cat cat tgt cca aag acg cca ttt tta ttg      336
Trp Val Pro Glu Ile Thr His His Cys Pro Lys Thr Pro Phe Leu Leu
            100                 105                 110
gtt gga acc cag gtt gat ttg cga gat gat gct gca aca att gaa aaa      384
Val Gly Thr Gln Val Asp Leu Arg Asp Asp Ala Ala Thr Ile Glu Lys

115                 120                 125
ctt tca aag aac aaa cag aaa gct ata act cca gat ttg ggt gat aaa      432
Leu Ser Lys Asn Lys Gln Lys Ala Ile Thr Pro Asp Leu Gly Asp Lys
    130                 135                 140
ctg gca aga gag tta aaa gca gta aaa tat gtc gag tgc tct gcc ctt      480
Leu Ala Arg Glu Leu Lys Ala Val Lys Tyr Val Glu Cys Ser Ala Leu
145                 150                 155                 160
act caa aaa gga tta aag aat gta ttt gat gaa gct att tta gct gct      528
Thr Gln Lys Gly Leu Lys Asn Val Phe Asp Glu Ala Ile Leu Ala Ala
                165                 170                 175
tta gag cca ccg gag cct aaa aga cgt cgc cga tgt caa att ttg          573
Leu Glu Pro Pro Glu Pro Lys Arg Arg Arg Arg Cys Gln Ile Leu
            180                 185                 190
tga                                                                  576

<210> 160
<211> 191
<212> PRT
<213> Ciona savignyi

<400> 160
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Gln Glu Tyr
            20                  25                  30
Val Pro Thr Val Phe Asp Asn Tyr Ala Val Thr Val Met Ile Gly Gly
        35                  40                  45
Glu Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Val Ser Pro Ser Ser Tyr Glu Asn Ile Lys Glu Lys
                85                  90                  95
Trp Val Pro Glu Ile Thr His His Cys Pro Lys Thr Pro Phe Leu Leu
            100                 105                 110
Val Gly Thr Gln Val Asp Leu Arg Asp Asp Ala Ala Thr Ile Glu Lys
        115                 120                 125
Leu Ser Lys Asn Lys Gln Lys Ala Ile Thr Pro Asp Leu Gly Asp Lys
    130                 135                 140
Leu Ala Arg Glu Leu Lys Ala Val Lys Tyr Val Glu Cys Ser Ala Leu
145                 150                 155                 160
Thr Gln Lys Gly Leu Lys Asn Val Phe Asp Glu Ala Ile Leu Ala Ala
                165                 170                 175
Leu Glu Pro Pro Glu Pro Lys Arg Arg Arg Arg Cys Gln Ile Leu
            180                 185                 190

<210> 161
<211> 757
<212> DNA
<213> Cryptococcus neoformans

<220>
<221> CDS
<222> (9)..(602)

<400> 161
cacacacc atg tcc gga gaa atc agg aga aag ctc gtt att gtc ggc gac      50
         Met Ser Gly Glu Ile Arg Arg Lys Leu Val Ile Val Gly Asp
         1               5                   10
ggt gct tgt ggt aag aca tgt ctt ctt atc gtg ttc agc aag ggc atg      98
Gly Ala Cys Gly Lys Thr Cys Leu Leu Ile Val Phe Ser Lys Gly Met
15                  20                  25                  30
ttc ccc gag gtg tac gtg ccc acc gtc ttt gaa aac tat gtc gcc gat      146
Phe Pro Glu Val Tyr Val Pro Thr Val Phe Glu Asn Tyr Val Ala Asp
                35                  40                  45

gta gag gtg gac ggg aag aag gtc gag ctg gcg ttg tgg gat act gct 194
Val Glu Val Asp Gly Lys Lys Val Glu Leu Ala Leu Trp Asp Thr Ala
            50                  55                  60
gga cag gag gac tat gac cga ctc cga cct ctt tct tac ccc gac tcc 242
Gly Gln Glu Asp Tyr Asp Arg Leu Arg Pro Leu Ser Tyr Pro Asp Ser
        65                  70                  75
cat gtc att ctc atc tgt ttc gct att gac tcc ccc gac tcg ctt gac 290
His Val Ile Leu Ile Cys Phe Ala Ile Asp Ser Pro Asp Ser Leu Asp
    80                  85                  90
aat gtt caa gaa aag tgg att tcc gag gtc ctt cac ttc tgt cag ggt 338
Asn Val Gln Glu Lys Trp Ile Ser Glu Val Leu His Phe Cys Gln Gly
95                  100                 105                 110
ctc ccc atc gtc ctc gtg gcg tgc aag aag gat ctc cgc gac gat ggc 386
Leu Pro Ile Val Leu Val Ala Cys Lys Lys Asp Leu Arg Asp Asp Gly
                115                 120                 125
aag act atc cag gac ctt gcc agg atg aac cag agg cct gtt tcc cgg 434
Lys Thr Ile Gln Asp Leu Ala Arg Met Asn Gln Arg Pro Val Ser Arg
            130                 135                 140
gcc gag ggt atg gcc gtt gcc caa aag att ggg gcg cag ggc tac gtc 482
Ala Glu Gly Met Ala Val Ala Gln Lys Ile Gly Ala Gln Gly Tyr Val
        145                 150                 155
gag tgc agt gcc aag acg ggc gag ggt gtc agg gaa gtg ttc cag act 530
Glu Cys Ser Ala Lys Thr Gly Glu Gly Val Arg Glu Val Phe Gln Thr
    160                 165                 170
gct acc agg cat gct ctc cag agc aaa aag tcc aag tct ggc aga ggg 578
Ala Thr Arg His Ala Leu Gln Ser Lys Lys Ser Lys Ser Gly Arg Gly
175                 180                 185                 190
aag aag ggt tgt gtg gtg ctt tagggctcta taaatcgagt tgtactttat 629
Lys Lys Gly Cys Val Val Leu
                195
cttgttcgtc gctggcgtct ttatacctct ttctcttcac gccattctca cgccttggcc 689

caagtgtttg gggcttattc tctcttttac ttgctgtgat ttgatgaatc gatgagtagt 749

tatgcccg 757

<210> 162
<211> 197
<212> PRT
<213> Cryptococcus neoformans

<400> 162
Met Ser Gly Glu Ile Arg Arg Lys Leu Val Ile Val Gly Asp Gly Ala
1               5                   10                  15
Cys Gly Lys Thr Cys Leu Leu Ile Val Phe Ser Lys Gly Met Phe Pro
            20                  25                  30
Glu Val Tyr Val Pro Thr Val Phe Glu Asn Tyr Val Ala Asp Val Glu
        35                  40                  45
Val Asp Gly Lys Lys Val Glu Leu Ala Leu Trp Asp Thr Ala Gly Gln
    50                  55                  60
Glu Asp Tyr Asp Arg Leu Arg Pro Leu Ser Tyr Pro Asp Ser His Val
65                  70                  75                  80
Ile Leu Ile Cys Phe Ala Ile Asp Ser Pro Asp Ser Leu Asp Asn Val
                85                  90                  95
Gln Glu Lys Trp Ile Ser Glu Val Leu His Phe Cys Gln Gly Leu Pro
            100                 105                 110
Ile Val Leu Val Ala Cys Lys Lys Asp Leu Arg Asp Asp Gly Lys Thr
        115                 120                 125
Ile Gln Asp Leu Ala Arg Met Asn Gln Arg Pro Val Ser Arg Ala Glu
    130                 135                 140
Gly Met Ala Val Ala Gln Lys Ile Gly Ala Gln Gly Tyr Val Glu Cys
145                 150                 155                 160
Ser Ala Lys Thr Gly Glu Gly Val Arg Glu Val Phe Gln Thr Ala Thr

                165                 170                 175
Arg His Ala Leu Gln Ser Lys Lys Ser Lys Ser Gly Arg Gly Lys Lys
            180                 185                 190
Gly Cys Val Val Leu
        195

<210> 163
<211> 626
<212> DNA
<213> Gallus gallus

<220>
<221> CDS
<222> (27)..(605)

<400> 163
tctcggcggc gcgccatcgc gggcag atg cag gcg atc aag tgt gtg gtg gtg 53
Met Gln Ala Ile Lys Cys Val Val Val
1               5
ggc gac gga gct gta ggg aag acc tgc ttg ctg atc agt tac acc acg 101
Gly Asp Gly Ala Val Gly Lys Thr Cys Leu Leu Ile Ser Tyr Thr Thr
10                  15                  20                  25
aat gcc ttt cct gga gag tac atc ccc act gta ttt gat aac tat tct 149
Asn Ala Phe Pro Gly Glu Tyr Ile Pro Thr Val Phe Asp Asn Tyr Ser
                30                  35                  40
gcc aat gtc atg gta gat ggg aag cca gtg aat cta ggc ctc tgg gat 197
Ala Asn Val Met Val Asp Gly Lys Pro Val Asn Leu Gly Leu Trp Asp
            45                  50                  55
aca gca gga caa gag gat tat gac cga ctg cgg cct ctt tcc tac cca 245
Thr Ala Gly Gln Glu Asp Tyr Asp Arg Leu Arg Pro Leu Ser Tyr Pro
        60                  65                  70
cag aca gat gtt ttc ttg att tgc ttc tcc ctc gtg agt cca gcc tcc 293
Gln Thr Asp Val Phe Leu Ile Cys Phe Ser Leu Val Ser Pro Ala Ser
    75                  80                  85
ttt gag aat gtc aga gcc aag tgg tac cct gag gtc cga cac cac tgc 341
Phe Glu Asn Val Arg Ala Lys Trp Tyr Pro Glu Val Arg His His Cys
90                  95                  100                 105
cca aat aca cct atc atc ttg gtg ggc acc aag ctg gac tta agg gat 389
Pro Asn Thr Pro Ile Ile Leu Val Gly Thr Lys Leu Asp Leu Arg Asp
                110                 115                 120
gat aag gac acc att gaa agg tta cgt gat aag aaa ctg gct ccc atc 437
Asp Lys Asp Thr Ile Glu Arg Leu Arg Asp Lys Lys Leu Ala Pro Ile
            125                 130                 135
acc tac ccc caa ggt ttg gcc atg gct cgg gag att ggc tcg gta aag 485
Thr Tyr Pro Gln Gly Leu Ala Met Ala Arg Glu Ile Gly Ser Val Lys
        140                 145                 150
tac ctt gag tgc tct gcc ctg aca cag cgg ggc ttg aag acg gtg ttt 533
Tyr Leu Glu Cys Ser Ala Leu Thr Gln Arg Gly Leu Lys Thr Val Phe
    155                 160                 165
gat gaa gcc atc cgg gct gtg ctc tgc cca ccg ccc gtg aag aag cct 581
Asp Glu Ala Ile Arg Ala Val Leu Cys Pro Pro Pro Val Lys Lys Pro
170                 175                 180                 185
ggc aaa aag tgc acc gtg ttc tgagggctgt ggcctaggtg ctgg 626
Gly Lys Lys Cys Thr Val Phe
                190

<210> 164
<211> 192
<212> PRT
<213> Gallus gallus

<400> 164
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly

35                  40                  45
Lys Pro Val Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Ser Pro Ala Ser Phe Glu Asn Val Arg Ala Lys
                85                  90                  95
Trp Tyr Pro Glu Val Arg His His Cys Pro Asn Thr Pro Ile Ile Leu
            100                 105                 110
Val Gly Thr Lys Leu Asp Leu Arg Asp Asp Lys Asp Thr Ile Glu Arg
        115                 120                 125
Leu Arg Asp Lys Lys Leu Ala Pro Ile Thr Tyr Pro Gln Gly Leu Ala
    130                 135                 140
Met Ala Arg Glu Ile Gly Ser Val Lys Tyr Leu Glu Cys Ser Ala Leu
145                 150                 155                 160
Thr Gln Arg Gly Leu Lys Thr Val Phe Asp Glu Ala Ile Arg Ala Val
                165                 170                 175
Leu Cys Pro Pro Pro Val Lys Lys Pro Gly Lys Lys Cys Thr Val Phe
            180                 185                 190

<210> 165
<211> 731
<212> DNA
<213> Gallus gallus

<220>
<221> CDS
<222> (48)..(638)

<400> 165
cggccatcgt cctgctgcac ggcaaggcca gcttggcgag cctggcc atg gcc gcc      56
                                                    Met Ala Ala
                                                    1

atc cgc aag aag ctg gtg gtg gtg gga gac ggc gcc tgt ggc aag acc      104
Ile Arg Lys Lys Leu Val Val Val Gly Asp Gly Ala Cys Gly Lys Thr
    5                   10                  15

tgc ctc ctc atc gtc ttc agc aag gac gag ttc ccc gag gtt tac gtg      152
Cys Leu Leu Ile Val Phe Ser Lys Asp Glu Phe Pro Glu Val Tyr Val
20                  25                  30                  35

ccc acc gtc ttt gag aac tac gtg gcc gac atc gag gtg gac ggc aag      200
Pro Thr Val Phe Glu Asn Tyr Val Ala Asp Ile Glu Val Asp Gly Lys
                40                  45                  50

cag gtg gag ctg gcg ctg tgg gac acg gcc ggc cag gag gac tac gac      248
Gln Val Glu Leu Ala Leu Trp Asp Thr Ala Gly Gln Glu Asp Tyr Asp
            55                  60                  65

cgc ctg cgc cct ctc tcc tac cca gac acg gac gtg atc ctc atg tgc      296
Arg Leu Arg Pro Leu Ser Tyr Pro Asp Thr Asp Val Ile Leu Met Cys
        70                  75                  80

ttc tca gtg gac agc ccg gac tcg ctg gag aac atc ccg gag aag tgg      344
Phe Ser Val Asp Ser Pro Asp Ser Leu Glu Asn Ile Pro Glu Lys Trp
    85                  90                  95

gtg ccc gaa gtc aag cac ttc tgc ccc aac gtc ccc atc atc ctg gtg      392
Val Pro Glu Val Lys His Phe Cys Pro Asn Val Pro Ile Ile Leu Val
100                 105                 110                 115

gcc aac aag aaa gac ctg cgc aac gac gag cac gtg cgt aac gag ctg      440
Ala Asn Lys Lys Asp Leu Arg Asn Asp Glu His Val Arg Asn Glu Leu
                120                 125                 130

gcc cgc atg aag cag gag ccg gtg cgc act gag gat ggc cgc gcc atg      488
Ala Arg Met Lys Gln Glu Pro Val Arg Thr Glu Asp Gly Arg Ala Met
            135                 140                 145

gcc atc cgc atc cag gcc tac gac tac ctg gag tgc tcg gcc aag acc      536
Ala Ile Arg Ile Gln Ala Tyr Asp Tyr Leu Glu Cys Ser Ala Lys Thr
        150                 155                 160

aag gag ggt gtg cgg gag gtc ttt gag acg gcc acc cgg gcg gcc ttg      584
Lys Glu Gly Val Arg Glu Val Phe Glu Thr Ala Thr Arg Ala Ala Leu
    165                 170                 175

cag aag cgc tac ggc act cag aac ggc tgc atc aat tgc tgc aag gtc      632
Gln Lys Arg Tyr Gly Thr Gln Asn Gly Cys Ile Asn Cys Cys Lys Val

180                 185                 190                 195
cta tagggcccgg ctggagccgg cgctgggcac ggctctgggt cacctgttgg          685
Leu

caggcggaga ggagctgggg cacgcatgca cacagcatct gcctgt                  731

<210> 166
<211> 196
<212> PRT
<213> Gallus gallus

<400> 166
Met Ala Ala Ile Arg Lys Lys Leu Val Val Val Gly Asp Gly Ala Cys
1               5                   10                  15
Gly Lys Thr Cys Leu Leu Ile Val Phe Ser Lys Asp Glu Phe Pro Glu
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Val Asp Ser Pro Asp Ser Leu Glu Asn Ile Pro
                85                  90                  95
Glu Lys Trp Val Pro Glu Val Lys His Phe Cys Pro Asn Val Pro Ile
            100                 105                 110
Ile Leu Val Ala Asn Lys Lys Asp Leu Arg Asn Asp Glu His Val Arg
        115                 120                 125
Asn Glu Leu Ala Arg Met Lys Gln Glu Pro Val Arg Thr Glu Asp Gly
    130                 135                 140
Arg Ala Met Ala Ile Arg Ile Gln Ala Tyr Asp Tyr Leu Glu Cys Ser
145                 150                 155                 160
Ala Lys Thr Lys Glu Gly Val Arg Glu Val Phe Glu Thr Ala Thr Arg
                165                 170                 175
Ala Ala Leu Gln Lys Arg Tyr Gly Thr Gln Asn Gly Cys Ile Asn Cys
            180                 185                 190
Cys Lys Val Leu
        195

<210> 167
<211> 883
<212> DNA
<213> Hemicentrotus pulcherrimus

<220>
<221> CDS
<222> (61)..(639)

<400> 167
cgggtgtaat ctgcctcttc ctggtactat tttatgaaat ttgtgtgaaa acctaagaca      60

atg gct gct ata agg aaa aag ttg gtt atc gta gga gat ggt gct tgt      108
Met Ala Ala Ile Arg Lys Lys Leu Val Ile Val Gly Asp Gly Ala Cys
1               5                   10                  15
ggg aag acg tgt ctg ctc ata gta ttt agc aaa gac cag ttc cct gaa      156
Gly Lys Thr Cys Leu Leu Ile Val Phe Ser Lys Asp Gln Phe Pro Glu
            20                  25                  30
gtc tat gtc cca aca gtt ttt gag aac tat gta gct gat ata gaa gta      204
Val Tyr Val Pro Thr Val Phe Glu Asn Tyr Val Ala Asp Ile Glu Val
        35                  40                  45
gac ggt aaa cag gtt gag ttg gca tta tgg gat aca gca ggt cag gaa      252
Asp Gly Lys Gln Val Glu Leu Ala Leu Trp Asp Thr Ala Gly Gln Glu
    50                  55                  60
gac tac gac aga ctg aga ccg ctc tca tat cca gac aca gac gtt ata      300

Asp Tyr Asp Arg Leu Arg Pro Leu Ser Tyr Pro Asp Thr Asp Val Ile
65                  70                  75                  80
ctc atg tgc ttt tca atc gac aac cca gac agt tta gaa acc atc cca      348
Leu Met Cys Phe Ser Ile Asp Asn Pro Asp Ser Leu Glu Thr Ile Pro
                85                  90                  95
gaa aaa tgg aca cca gag gtg aag cac ttt tgc cct aat gta cct gtc      396
Glu Lys Trp Thr Pro Glu Val Lys His Phe Cys Pro Asn Val Pro Val
            100                 105                 110
atc ttg gtc ggc aac aag aaa gat ctt cga aat gac gat gcc aca aaa      444
Ile Leu Val Gly Asn Lys Lys Asp Leu Arg Asn Asp Asp Ala Thr Lys
        115                 120                 125
cgg gaa ctg agt aag atg aag cag gaa ccg gtg aaa tat aat gat gcc      492
Arg Glu Leu Ser Lys Met Lys Gln Glu Pro Val Lys Tyr Asn Asp Ala
    130                 135                 140
cag acc atg tca gat aag atc aac gcc tac aaa tac cta gaa tgc tca      540
Gln Thr Met Ser Asp Lys Ile Asn Ala Tyr Lys Tyr Leu Glu Cys Ser
145                 150                 155                 160
gcc aag tct aac gat ggt gtc agg gaa gtg ttt gag aca gca acc aga      588
Ala Lys Ser Asn Asp Gly Val Arg Glu Val Phe Glu Thr Ala Thr Arg
                165                 170                 175
gca gca cta caa gtt aaa aag aag aag tca tca aaa tgc agc ctc ttg      636
Ala Ala Leu Gln Val Lys Lys Lys Lys Ser Ser Lys Cys Ser Leu Leu
            180                 185                 190
taagagaagt cttcatgcaa gctggcaagg catggtagtg gtcaacaaga ggtcctcaaa      696

gaataatgca tttaatatac atgtatgtgt ttggggaaaa gagcgattat gtaatatttc      756

tggatgtgct ttgaaggtca aagacagcgg tagtgccagc atactttgtt taattgctgt      816

tgtgataata agaggcaatc ggcttaattt tgtagttgca gatttacgaa accacttttc      876

tttcttt                                                                883

<210> 168
<211> 192
<212> PRT
<213> Hemicentrotus pulcherrimus

<400> 168
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Asn Pro Asp Ser Leu Glu Thr Ile Pro
                85                  90                  95
Glu Lys Trp Thr Pro Glu Val Lys His Phe Cys Pro Asn Val Pro Val
            100                 105                 110
Ile Leu Val Gly Asn Lys Lys Asp Leu Arg Asn Asp Asp Ala Thr Lys
        115                 120                 125
Arg Glu Leu Ser Lys Met Lys Gln Glu Pro Val Lys Tyr Asn Asp Ala
    130                 135                 140
Gln Thr Met Ser Asp Lys Ile Asn Ala Tyr Lys Tyr Leu Glu Cys Ser
145                 150                 155                 160
Ala Lys Ser Asn Asp Gly Val Arg Glu Val Phe Glu Thr Ala Thr Arg
                165                 170                 175

Ala Ala Leu Gln Val Lys Lys Lys Lys Ser Ser Lys Cys Ser Leu Leu
            180                 185                 190

<210> 169
<211> 958
<212> DNA
<213> Arabidopsis thaliana

<220>
<221> CDS
<222> (146)..(742)

<400> 169
gtcgacccac gcgtccggag agactttcag cagctatttc tttgtcttct ttctctataa 60

agaacctcac tctctctctc tttagtcaaa aatccccaat tcgtgaacaa attagggttt 120

ttgaagtttg aagaaggaga gaaag atg agt gct tca agg ttt atc aag tgt 172
Met Ser Ala Ser Arg Phe Ile Lys Cys
1               5
gtc act gtc ggc gac ggt gct gtt gga aag act tgt ctt ctc atc tcc 220
Val Thr Val Gly Asp Gly Ala Val Gly Lys Thr Cys Leu Leu Ile Ser
10                  15                  20                  25
tac act agc aac act ttc ccc acg gat tat gtg cca act gtg ttc gat 268
Tyr Thr Ser Asn Thr Phe Pro Thr Asp Tyr Val Pro Thr Val Phe Asp
                30                  35                  40
aat ttc agt gcc aat gtg att gtt gat ggc aac act atc aac ttg gga 316
Asn Phe Ser Ala Asn Val Ile Val Asp Gly Asn Thr Ile Asn Leu Gly
            45                  50                  55
ttg tgg gat act gca ggg caa gag gac tac aat aga cta aga cct ttg 364
Leu Trp Asp Thr Ala Gly Gln Glu Asp Tyr Asn Arg Leu Arg Pro Leu
        60                  65                  70
agc tat cgc ggt gca gat gtc ttc tta ctt gca ttc tca ctt gtc agc 412
Ser Tyr Arg Gly Ala Asp Val Phe Leu Leu Ala Phe Ser Leu Val Ser
    75                  80                  85
aaa gct agc tat gaa aat gtt tct aaa aag tgg gtt cct gaa ctg aga 460
Lys Ala Ser Tyr Glu Asn Val Ser Lys Lys Trp Val Pro Glu Leu Arg
90                  95                  100                 105
cat tat gct cct ggt gtt ccc atc atc ctc gtt gga aca aag ctt gat 508
His Tyr Ala Pro Gly Val Pro Ile Ile Leu Val Gly Thr Lys Leu Asp
                110                 115                 120
ctt cga cat gat aag caa ttc ttt gcc gag cac cct ggt gct gtg cct 556
Leu Arg His Asp Lys Gln Phe Phe Ala Glu His Pro Gly Ala Val Pro
            125                 130                 135
atc tct acc gct cag ggt gaa gaa cta aag aag ctg att ggg gcg cct 604
Ile Ser Thr Ala Gln Gly Glu Glu Leu Lys Lys Leu Ile Gly Ala Pro
        140                 145                 150
gct tat atc gaa tgc agt gca aaa act caa cag aat gtg aaa gca gtg 652
Ala Tyr Ile Glu Cys Ser Ala Lys Thr Gln Gln Asn Val Lys Ala Val
    155                 160                 165
ttt gat gcg gct atc aag gtc gtt ctc cag cca cca aaa aac aag aag 700
Phe Asp Ala Ala Ile Lys Val Val Leu Gln Pro Pro Lys Asn Lys Lys
170                 175                 180                 185
aag aag aag aga aaa tct cag aaa ggt tgt tct ata ctc tgattcaaca 749
Lys Lys Lys Arg Lys Ser Gln Lys Gly Cys Ser Ile Leu
                190                 195
agaagaaagg aatcaacagg agaattagag ctataagtat ttgttttctc ctttttcata 809

acttgtttac ccaaggaatc aaaaatctgt tgtttgtagt ctgaaaatga caaaagttga 869

agaactagta acaagtattt gtattcttgt ttgcattttg atctgtaaat ataccacgaa 929

tccttgattt ctctgtttcc taaaaaaaa 958

<210> 170
<211> 198
<212> PRT
<213> Arabidopsis thaliana

<400> 170
   Met Ser Ala Ser Arg Phe Ile Lys Cys Val Thr Val Gly Asp Gly Ala
   1               5                   10                  15
   Val Gly Lys Thr Cys Leu Leu Ile Ser Tyr Thr Ser Asn Thr Phe Pro
               20                  25                  30
   Thr Asp Tyr Val Pro Thr Val Phe Asp Asn Phe Ser Ala Asn Val Ile
           35                  40                  45
   Val Asp Gly Asn Thr Ile Asn Leu Gly Leu Trp Asp Thr Ala Gly Gln
       50                  55                  60
   Glu Asp Tyr Asn Arg Leu Arg Pro Leu Ser Tyr Arg Gly Ala Asp Val
   65                  70                  75                  80
   Phe Leu Leu Ala Phe Ser Leu Val Ser Lys Ala Ser Tyr Glu Asn Val
                   85                  90                  95
   Ser Lys Lys Trp Val Pro Glu Leu Arg His Tyr Ala Pro Gly Val Pro
               100                 105                 110
   Ile Ile Leu Val Gly Thr Lys Leu Asp Leu Arg His Asp Lys Gln Phe
           115                 120                 125
   Phe Ala Glu His Pro Gly Ala Val Pro Ile Ser Thr Ala Gln Gly Glu
       130                 135                 140
   Glu Leu Lys Lys Leu Ile Gly Ala Pro Ala Tyr Ile Glu Cys Ser Ala
   145                 150                 155                 160
   Lys Thr Gln Gln Asn Val Lys Ala Val Phe Asp Ala Ala Ile Lys Val
                   165                 170                 175
   Val Leu Gln Pro Pro Lys Asn Lys Lys Lys Lys Lys Arg Lys Ser Gln
               180                 185                 190
   Lys Gly Cys Ser Ile Leu
           195

<210> 171
<211> 780
<212> DNA
<213> Anopheles gambiae str

<220>
<221> CDS
<222> (208)..(780)

<400> 171
gcggccgcaa aagcaaatga aaagttgaac tgaaacttcc aagcgcacaa agtcgagccg      60

tgaggtgttt tccttgtttt tccgtgttcg agtgtgtgtg tggtggttct gtgtttcgta     120

cgtgtgcggt gcgcgtttgt gttggaggtg tttcggtttg tattttcttc gtgtttccag     180

tttgtttttc ctaggtttcg ctaaaag atg tca tcc gga aga cct atc aaa tgt     234
                              Met Ser Ser Gly Arg Pro Ile Lys Cys
                              1               5
gtg gtg gtc ggc gac ggc acg gtg ggg aag acg tgc atg ttg atc agc       282
Val Val Val Gly Asp Gly Thr Val Gly Lys Thr Cys Met Leu Ile Ser
10                  15                  20                  25
tac acg acc gac agc ttt ccc ggc gaa tac gta ccc acg gtc ttc gac       330
Tyr Thr Thr Asp Ser Phe Pro Gly Glu Tyr Val Pro Thr Val Phe Asp
                30                  35                  40
aac tac tcc gcc ccg atg gtg gtg gac ggt gtg caa gtg tcg ctc ggg       378
Asn Tyr Ser Ala Pro Met Val Val Asp Gly Val Gln Val Ser Leu Gly
            45                  50                  55

ctg tgg gat acg gcc ggg cag gaa gac tac gat cgg cta agg ccc ctg 426
Leu Trp Asp Thr Ala Gly Gln Glu Asp Tyr Asp Arg Leu Arg Pro Leu
        60                  65                  70
tcc tac cca cag acg gac gtg ttc ctc ata tgc tac agt gtg gcc agc 474
Ser Tyr Pro Gln Thr Asp Val Phe Leu Ile Cys Tyr Ser Val Ala Ser
    75                  80                  85
ccg tcg tcg ttc gaa aac gtt acc tcc aaa tgg tat ccc gag atc aag 522
Pro Ser Ser Phe Glu Asn Val Thr Ser Lys Trp Tyr Pro Glu Ile Lys
90                  95                  100                 105
cac cac tgc ccg gat gcg ccc atc att tta gtc gga acc aaa atc gat 570
His His Cys Pro Asp Ala Pro Ile Ile Leu Val Gly Thr Lys Ile Asp
                110                 115                 120
ctg cgc gag gat cgg gaa acg ata agc ttg ctg gcg gac cag ggc ctt 618
Leu Arg Glu Asp Arg Glu Thr Ile Ser Leu Leu Ala Asp Gln Gly Leu
            125                 130                 135
tcc gcg ctg aag cgc gaa cag ggc caa aag cta gcg aac aag ata cgg 666
Ser Ala Leu Lys Arg Glu Gln Gly Gln Lys Leu Ala Asn Lys Ile Arg
        140                 145                 150
gcg gta aag tat atg gaa tgt tcg gca cta acc cag cgg ggc cta aag 714
Ala Val Lys Tyr Met Glu Cys Ser Ala Leu Thr Gln Arg Gly Leu Lys
    155                 160                 165
cag gtg ttt gac gaa gcg ctt tgc gcc acg gaa gag aaa gag cga atg 762
Gln Val Phe Asp Glu Ala Leu Cys Ala Thr Glu Glu Lys Glu Arg Met
170                 175                 180                 185
ccg gta gaa gag gag tag 780
Pro Val Glu Glu Glu
                190

<210> 172
<211> 190
<212> PRT
<213> Anopheles gambiae str

<400> 172
Met Ser Ser Gly Arg Pro Ile Lys Cys Val Val Val Gly Asp Gly Thr
1               5                   10                  15
Val Gly Lys Thr Cys Met Leu Ile Ser Tyr Thr Thr Asp Ser Phe Pro
            20                  25                  30
Gly Glu Tyr Val Pro Thr Val Phe Asp Asn Tyr Ser Ala Pro Met Val
        35                  40                  45
Val Asp Gly Val Gln Val Ser Leu Gly Leu Trp Asp Thr Ala Gly Gln
    50                  55                  60
Glu Asp Tyr Asp Arg Leu Arg Pro Leu Ser Tyr Pro Gln Thr Asp Val
65                  70                  75                  80
Phe Leu Ile Cys Tyr Ser Val Ala Ser Pro Ser Ser Phe Glu Asn Val
                85                  90                  95
Thr Ser Lys Trp Tyr Pro Glu Ile Lys His His Cys Pro Asp Ala Pro
            100                 105                 110
Ile Ile Leu Val Gly Thr Lys Ile Asp Leu Arg Glu Asp Arg Glu Thr
        115                 120                 125
Ile Ser Leu Leu Ala Asp Gln Gly Leu Ser Ala Leu Lys Arg Glu Gln
    130                 135                 140
Gly Gln Lys Leu Ala Asn Lys Ile Arg Ala Val Lys Tyr Met Glu Cys
145                 150                 155                 160
Ser Ala Leu Thr Gln Arg Gly Leu Lys Gln Val Phe Asp Glu Ala Leu
                165                 170                 175
Cys Ala Thr Glu Glu Lys Glu Arg Met Pro Val Glu Glu Glu
            180                 185                 190

<210> 173
<211> 657
<212> DNA
<213> Giardia lamblia ATCC 50803

<220>
<221> CDS
<222> (1)..(657)

<400> 173
atg act agt aca gga aat gag gat aca gcg ggt gca agg atg ata cat      48
Met Thr Ser Thr Gly Asn Glu Asp Thr Ala Gly Ala Arg Met Ile His
1               5                   10                  15
atc aaa gcc gta gtg gtt gga gac ggc tca gtg ggc aag acc tgc ctt      96
Ile Lys Ala Val Val Val Gly Asp Gly Ser Val Gly Lys Thr Cys Leu
            20                  25                  30
ctt ttt gtc tat gcg aat aac tca ttt cca gag gac tac ttg cca aca      144
Leu Phe Val Tyr Ala Asn Asn Ser Phe Pro Glu Asp Tyr Leu Pro Thr
        35                  40                  45
gtc ttt gac aac tat tct gcc aac gtc gtt gtt gac aac ttg acg ata      192
Val Phe Asp Asn Tyr Ser Ala Asn Val Val Val Asp Asn Leu Thr Ile
    50                  55                  60
aat att ggt ctc tgg gat act gcg ggc cag gag gat tac gac aaa ttg      240
Asn Ile Gly Leu Trp Asp Thr Ala Gly Gln Glu Asp Tyr Asp Lys Leu
65                  70                  75                  80
cga ccc ttg agc tat cca ggg gcg cac gta ttt ttg ctc tgc ttt agc      288
Arg Pro Leu Ser Tyr Pro Gly Ala His Val Phe Leu Leu Cys Phe Ser
                85                  90                  95
gtc gtt tcc agc act tcg ttt gca aac atc aga tcc aaa tgg tac aca      336
Val Val Ser Ser Thr Ser Phe Ala Asn Ile Arg Ser Lys Trp Tyr Thr
            100                 105                 110
gag gtc aag gag tac tgt cca aac gtg ccg atg atc ctt gtc gga aca      384
Glu Val Lys Glu Tyr Cys Pro Asn Val Pro Met Ile Leu Val Gly Thr
        115                 120                 125
aag tat gac ctg ctg tcc gac gag gct tac ctg gcc aag atg aaa gag      432
Lys Tyr Asp Leu Leu Ser Asp Glu Ala Tyr Leu Ala Lys Met Lys Glu
    130                 135                 140
aag aac caa tcc ccg gtg tct gat gaa cgt gca gag gaa gtt gca aaa      480
Lys Asn Gln Ser Pro Val Ser Asp Glu Arg Ala Glu Glu Val Ala Lys
145                 150                 155                 160
gag ata aag gcc att aag tac att agc tgc agt gcc cga tgc caa ctt      528
Glu Ile Lys Ala Ile Lys Tyr Ile Ser Cys Ser Ala Arg Cys Gln Leu
                165                 170                 175
cgc gta aag gac gtg ttt gat agt gca atc cgc gcg gct ctc aag aat      576
Arg Val Lys Asp Val Phe Asp Ser Ala Ile Arg Ala Ala Leu Lys Asn
            180                 185                 190
atg ggg atg atg ggt agt ggg act tcc aag gca gga aag aaa aag gat      624
Met Gly Met Met Gly Ser Gly Thr Ser Lys Ala Gly Lys Lys Lys Asp
        195                 200                 205
gga tct gga aag gga aag tgt gtt ata ttc tag                          657
Gly Ser Gly Lys Gly Lys Cys Val Ile Phe
    210                 215

<210> 174
<211> 218
<212> PRT
<213> Giardia lamblia ATCC 50803

<400> 174
Met Thr Ser Thr Gly Asn Glu Asp Thr Ala Gly Ala Arg Met Ile His
1               5                   10                  15
Ile Lys Ala Val Val Val Gly Asp Gly Ser Val Gly Lys Thr Cys Leu
            20                  25                  30
Leu Phe Val Tyr Ala Asn Asn Ser Phe Pro Glu Asp Tyr Leu Pro Thr
        35                  40                  45
Val Phe Asp Asn Tyr Ser Ala Asn Val Val Val Asp Asn Leu Thr Ile
    50                  55                  60
Asn Ile Gly Leu Trp Asp Thr Ala Gly Gln Glu Asp Tyr Asp Lys Leu
65                  70                  75                  80
Arg Pro Leu Ser Tyr Pro Gly Ala His Val Phe Leu Leu Cys Phe Ser
                85                  90                  95
Val Val Ser Ser Thr Ser Phe Ala Asn Ile Arg Ser Lys Trp Tyr Thr
            100                 105                 110
Glu Val Lys Glu Tyr Cys Pro Asn Val Pro Met Ile Leu Val Gly Thr
        115                 120                 125

Lys Tyr Asp Leu Leu Ser Asp Glu Ala Tyr Leu Ala Lys Met Lys Glu
    130                 135                 140
Lys Asn Gln Ser Pro Val Ser Asp Glu Arg Ala Glu Glu Val Ala Lys
145                 150                 155                 160
Glu Ile Lys Ala Ile Lys Tyr Ile Ser Cys Ser Ala Arg Cys Gln Leu
                165                 170                 175
Arg Val Lys Asp Val Phe Asp Ser Ala Ile Arg Ala Ala Leu Lys Asn
            180                 185                 190
Met Gly Met Met Gly Ser Gly Thr Ser Lys Ala Gly Lys Lys Lys Asp
        195                 200                 205
Gly Ser Gly Lys Gly Lys Cys Val Ile Phe
    210                 215

<210> 175
<211> 624
<212> DNA
<213> Neurospora crassa

<220>
<221> CDS
<222> (1)..(624)

<400> 175
atg gtg acg gga act atc aag tat gcc aaa aca aac cac ccc tta tcg       48
Met Val Thr Gly Thr Ile Lys Tyr Ala Lys Thr Asn His Pro Leu Ser
1               5                   10                  15
aga gag tgc gta gtc gtc ggt gac ggt gcc gtt gga aag aca tgt ctc       96
Arg Glu Cys Val Val Val Gly Asp Gly Ala Val Gly Lys Thr Cys Leu
            20                  25                  30
ctc atc agc tat aca acg aat aag ttc ccc tcg gaa tat gtg ccg aca      144
Leu Ile Ser Tyr Thr Thr Asn Lys Phe Pro Ser Glu Tyr Val Pro Thr
        35                  40                  45
gtt ttc gac aac tat gcc gtc acc gtc atg atc ggt gat gag ccc tat      192
Val Phe Asp Asn Tyr Ala Val Thr Val Met Ile Gly Asp Glu Pro Tyr
    50                  55                  60
acg ctc ggc ctg ttc gat aca gca gga caa gaa gat tac gac cgt tta      240
Thr Leu Gly Leu Phe Asp Thr Ala Gly Gln Glu Asp Tyr Asp Arg Leu
65                  70                  75                  80
cgt ccg cta tca tac ccc cag acc gac gtc ttc ctc atc tgc ttc agc      288
Arg Pro Leu Ser Tyr Pro Gln Thr Asp Val Phe Leu Ile Cys Phe Ser
                85                  90                  95
gtt gca tcg ccc gcc tcg ttc gaa aac gtg tcc cag aaa tgg gcc ccc      336
Val Ala Ser Pro Ala Ser Phe Glu Asn Val Ser Gln Lys Trp Ala Pro
            100                 105                 110
gaa gtc aac cat cac tgc ccc ggc gtg ccc ttc ctc atc gtc gga acg      384
Glu Val Asn His His Cys Pro Gly Val Pro Phe Leu Ile Val Gly Thr
        115                 120                 125
cag aag gat ttg cgc tcc gac aag gaa ctg agg gac aag ctt gcc cag      432
Gln Lys Asp Leu Arg Ser Asp Lys Glu Leu Arg Asp Lys Leu Ala Gln
    130                 135                 140
cgc aag cag tcg atg ata gag ttc aag cag gga gag aag ctt gct cag      480
Arg Lys Gln Ser Met Ile Glu Phe Lys Gln Gly Glu Lys Leu Ala Gln
145                 150                 155                 160
gat ctt gac gcc gtc aaa tac gtc gag tgc agt gcg ctg aca cag gaa      528
Asp Leu Asp Ala Val Lys Tyr Val Glu Cys Ser Ala Leu Thr Gln Glu
                165                 170                 175
ggt ctc aag aac gtg ttt gac gag gcc atc gtt gcg gca ctg gag ccg      576
Gly Leu Lys Asn Val Phe Asp Glu Ala Ile Val Ala Ala Leu Glu Pro
            180                 185                 190
cct cag aaa aag aca agt aaa agg gac aag aag tgc ttg att ctg          621
Pro Gln Lys Lys Thr Ser Lys Arg Asp Lys Lys Cys Leu Ile Leu
        195                 200                 205
tga                                                                  624

<210> 176

<211> 207
<212> PRT
<213> Neurospora crassa

<400> 176
Met Val Thr Gly Thr Ile Lys Tyr Ala Lys Thr Asn His Pro Leu Ser
1               5                   10                  15
Arg Glu Cys Val Val Val Gly Asp Gly Ala Val Gly Lys Thr Cys Leu
            20                  25                  30
Leu Ile Ser Tyr Thr Thr Asn Lys Phe Pro Ser Glu Tyr Val Pro Thr
        35                  40                  45
Val Phe Asp Asn Tyr Ala Val Thr Val Met Ile Gly Asp Glu Pro Tyr
    50                  55                  60
Thr Leu Gly Leu Phe Asp Thr Ala Gly Gln Glu Asp Tyr Asp Arg Leu
65                  70                  75                  80
Arg Pro Leu Ser Tyr Pro Gln Thr Asp Val Phe Leu Ile Cys Phe Ser
                85                  90                  95
Val Ala Ser Pro Ala Ser Phe Glu Asn Val Ser Gln Lys Trp Ala Pro
            100                 105                 110
Glu Val Asn His His Cys Pro Gly Val Pro Phe Leu Ile Val Gly Thr
        115                 120                 125
Gln Lys Asp Leu Arg Ser Asp Lys Glu Leu Arg Asp Lys Leu Ala Gln
    130                 135                 140
Arg Lys Gln Ser Met Ile Glu Phe Lys Gln Gly Glu Lys Leu Ala Gln
145                 150                 155                 160
Asp Leu Asp Ala Val Lys Tyr Val Glu Cys Ser Ala Leu Thr Gln Glu
                165                 170                 175
Gly Leu Lys Asn Val Phe Asp Glu Ala Ile Val Ala Ala Leu Glu Pro
            180                 185                 190
Pro Gln Lys Lys Thr Ser Lys Arg Asp Lys Lys Cys Leu Ile Leu
        195                 200                 205

<210> 177
<211> 603
<212> DNA
<213> Neurospora crassa

<220>
<221> CDS
<222> (1)..(603)

<400> 177
atg gca tca ggc agc cct cag aat gtc atc cgc agg aaa ctc gtc att      48
Met Ala Ser Gly Ser Pro Gln Asn Val Ile Arg Arg Lys Leu Val Ile
1               5                   10                  15
atc ggc gac ggt gct tgc gga aaa acc agt cta ctg agc gtc ttc act      96
Ile Gly Asp Gly Ala Cys Gly Lys Thr Ser Leu Leu Ser Val Phe Thr
            20                  25                  30
ttg ggt ttt ttc cca gcc aca tat atc ccc acc gtg ttc gag aat tac      144
Leu Gly Phe Phe Pro Ala Thr Tyr Ile Pro Thr Val Phe Glu Asn Tyr
        35                  40                  45
gtt acc gac tgc cga gta gat ggc aag tcg gtt caa cta gcg cta tgg      192
Val Thr Asp Cys Arg Val Asp Gly Lys Ser Val Gln Leu Ala Leu Trp
    50                  55                  60
gac aca gcc gga caa gaa gac tat gag cga tta cga cca cta gca tac      240
Asp Thr Ala Gly Gln Glu Asp Tyr Glu Arg Leu Arg Pro Leu Ala Tyr
65                  70                  75                  80
tca aag gcg cat gtc att cta ata ggg ttt tct gtc gac acg cca gat      288
Ser Lys Ala His Val Ile Leu Ile Gly Phe Ser Val Asp Thr Pro Asp
                85                  90                  95
tct ttg gac aac gtt aaa cac aag tgg gtt act gag gcc aat gaa aga      336
Ser Leu Asp Asn Val Lys His Lys Trp Val Thr Glu Ala Asn Glu Arg
            100                 105                 110
tgt ccc aat gtt ccc atc att ttg gtg ggt ctc aag aag gat ctc agg      384
Cys Pro Asn Val Pro Ile Ile Leu Val Gly Leu Lys Lys Asp Leu Arg
        115                 120                 125
gga gat cct gtt gcc atc gaa gaa atg cga aag cga tcc caa cga ttc      432
Gly Asp Pro Val Ala Ile Glu Glu Met Arg Lys Arg Ser Gln Arg Phe

130                 135                 140
gtg atg gag gac gaa ggc cag agg ata gca aag gag att ggc gct cga        480
Val Met Glu Asp Glu Gly Gln Arg Ile Ala Lys Glu Ile Gly Ala Arg
145                 150                 155                 160
aag tat ctc gaa tgt tcc agt ctt acc gga gaa ggt gtt gac gac gta        528
Lys Tyr Leu Glu Cys Ser Ser Leu Thr Gly Glu Gly Val Asp Asp Val
                165                 170                 175
ttc gag gca gcc acg cga gcg gcg ctg cta acg ttt gag aag aag gaa        576
Phe Glu Ala Ala Thr Arg Ala Ala Leu Leu Thr Phe Glu Lys Lys Glu
            180                 185                 190
gga agc ggg tgc tgt gtg att cta tga                                    603
Gly Ser Gly Cys Cys Val Ile Leu
        195                 200


<210> 178
<211> 200
<212> PRT
<213> Neurospora crassa

<400> 178
Met Ala Ser Gly Ser Pro Gln Asn Val Ile Arg Arg Lys Leu Val Ile
1               5                   10                  15
Ile Gly Asp Gly Ala Cys Gly Lys Thr Ser Leu Leu Ser Val Phe Thr
            20                  25                  30
Leu Gly Phe Phe Pro Ala Thr Tyr Ile Pro Thr Val Phe Glu Asn Tyr
        35                  40                  45
Val Thr Asp Cys Arg Val Asp Gly Lys Ser Val Gln Leu Ala Leu Trp
    50                  55                  60
Asp Thr Ala Gly Gln Glu Asp Tyr Glu Arg Leu Arg Pro Leu Ala Tyr
65                  70                  75                  80
Ser Lys Ala His Val Ile Leu Ile Gly Phe Ser Val Asp Thr Pro Asp
                85                  90                  95
Ser Leu Asp Asn Val Lys His Lys Trp Val Thr Glu Ala Asn Glu Arg
            100                 105                 110
Cys Pro Asn Val Pro Ile Ile Leu Val Gly Leu Lys Lys Asp Leu Arg
        115                 120                 125
Gly Asp Pro Val Ala Ile Glu Glu Met Arg Lys Arg Ser Gln Arg Phe
    130                 135                 140
Val Met Glu Asp Glu Gly Gln Arg Ile Ala Lys Glu Ile Gly Ala Arg
145                 150                 155                 160
Lys Tyr Leu Glu Cys Ser Ser Leu Thr Gly Glu Gly Val Asp Asp Val
                165                 170                 175
Phe Glu Ala Ala Thr Arg Ala Ala Leu Leu Thr Phe Glu Lys Lys Glu
            180                 185                 190
Gly Ser Gly Cys Cys Val Ile Leu
        195                 200

<210> 179
<211> 624
<212> DNA
<213> Neurospora crassa

<220>
<221> CDS
<222> (1)..(624)

<400> 179
atg cct tgc gga ctc gga ggg tcg aaa aca gtg cag cgc aag ctc gtc        48
Met Pro Cys Gly Leu Gly Gly Ser Lys Thr Val Gln Arg Lys Leu Val
1               5                   10                  15
ttg ctc ggc gat ggt gcc tgc gga aaa acc tca ctg ctc aac gtt ttc        96
Leu Leu Gly Asp Gly Ala Cys Gly Lys Thr Ser Leu Leu Asn Val Phe
            20                  25                  30
aca aga ggc tat ttc ccc act gta tac gag ccg aca gtc ttc gag aac        144
Thr Arg Gly Tyr Phe Pro Thr Val Tyr Glu Pro Thr Val Phe Glu Asn
        35                  40                  45
tat gtg cac gac att ttc gtc gac aat gtc cat att gaa ctg tca ttg        192

Tyr Val His Asp Ile Phe Val Asp Asn Val His Ile Glu Leu Ser Leu
    50                  55                  60
tgg gat acc gcc ggc cag gaa gaa ttt gac agg cta cga agt cta tca        240
Trp Asp Thr Ala Gly Gln Glu Glu Phe Asp Arg Leu Arg Ser Leu Ser
65                  70                  75                  80
tac gat gac acc gac tta atc atg ctt tgc tac tct gtc gac agc aag        288
Tyr Asp Asp Thr Asp Leu Ile Met Leu Cys Tyr Ser Val Asp Ser Lys
                85                  90                  95
gac tcg ctc gaa aac gtc gaa agc aaa tgg caa ggc gag att gct gaa        336
Asp Ser Leu Glu Asn Val Glu Ser Lys Trp Gln Gly Glu Ile Ala Glu
            100                 105                 110
aac tgc ccg ggc gtc aag att gtc ttg gtc gcg ttg aag tgc gac ctg        384
Asn Cys Pro Gly Val Lys Ile Val Leu Val Ala Leu Lys Cys Asp Leu
        115                 120                 125
cgc gag gcg acc gag gac gaa gag gat ggc gga gcc aac gag gac ggt        432
Arg Glu Ala Thr Glu Asp Glu Glu Asp Gly Gly Ala Asn Glu Asp Gly
    130                 135                 140
gct caa cgt gag aag aag cca atg atc agc tac gac cag ggc ctg gaa        480
Ala Gln Arg Glu Lys Lys Pro Met Ile Ser Tyr Asp Gln Gly Leu Glu
145                 150                 155                 160
gtg gct cgc cgg atc aag gcc ctt cga tat ctc gaa tgc tcc gcc atg        528
Val Ala Arg Arg Ile Lys Ala Leu Arg Tyr Leu Glu Cys Ser Ala Met
                165                 170                 175
cga aac cgg gga gtc aac gaa gcc ttc acc gaa gca gcg cgt gtc gct        576
Arg Asn Arg Gly Val Asn Glu Ala Phe Thr Glu Ala Ala Arg Val Ala
            180                 185                 190
ttg tca gtg aag aag gac cgg gag gag tcg aag tgc gtg gtc atg            621
Leu Ser Val Lys Lys Asp Arg Glu Glu Ser Lys Cys Val Val Met
        195                 200                 205
taa                                                                    624

<210> 180
<211> 207
<212> PRT
<213> Neurospora crassa

<400> 180
Met Pro Cys Gly Leu Gly Gly Ser Lys Thr Val Gln Arg Lys Leu Val
1               5                   10                  15
Leu Leu Gly Asp Gly Ala Cys Gly Lys Thr Ser Leu Leu Asn Val Phe
            20                  25                  30
Thr Arg Gly Tyr Phe Pro Thr Val Tyr Glu Pro Thr Val Phe Glu Asn
        35                  40                  45
Tyr Val His Asp Ile Phe Val Asp Asn Val His Ile Glu Leu Ser Leu
    50                  55                  60
Trp Asp Thr Ala Gly Gln Glu Glu Phe Asp Arg Leu Arg Ser Leu Ser
65                  70                  75                  80
Tyr Asp Asp Thr Asp Leu Ile Met Leu Cys Tyr Ser Val Asp Ser Lys
                85                  90                  95
Asp Ser Leu Glu Asn Val Glu Ser Lys Trp Gln Gly Glu Ile Ala Glu
            100                 105                 110
Asn Cys Pro Gly Val Lys Ile Val Leu Val Ala Leu Lys Cys Asp Leu
        115                 120                 125
Arg Glu Ala Thr Glu Asp Glu Glu Asp Gly Gly Ala Asn Glu Asp Gly
    130                 135                 140
Ala Gln Arg Glu Lys Lys Pro Met Ile Ser Tyr Asp Gln Gly Leu Glu
145                 150                 155                 160
Val Ala Arg Arg Ile Lys Ala Leu Arg Tyr Leu Glu Cys Ser Ala Met
                165                 170                 175
Arg Asn Arg Gly Val Asn Glu Ala Phe Thr Glu Ala Ala Arg Val Ala
            180                 185                 190
Leu Ser Val Lys Lys Asp Arg Glu Glu Ser Lys Cys Val Val Met
        195                 200                 205

<210> 181

<211> 645
<212> DNA
<213> Gallus gallus

<220>
<221> CDS
<222> (1)..(645)

<400> 181
atg ccc ggg gga ggg cgg gct ccg gcc atg gcc cat ggc agc gcc gcg 48
Met Pro Gly Gly Gly Arg Ala Pro Ala Met Ala His Gly Ser Ala Ala
1               5                   10                  15
gtc atg ctg aag tgc gtg gtg gtg ggg gac ggc gcc gtg ggc aag acg 96
Val Met Leu Lys Cys Val Val Val Gly Asp Gly Ala Val Gly Lys Thr
            20                  25                  30
tgc ctg ctg atg agc tac gcc aac gac gcc ttc cct gag gag tac gtg 144
Cys Leu Leu Met Ser Tyr Ala Asn Asp Ala Phe Pro Glu Glu Tyr Val
        35                  40                  45
ccc acc gtc ttc gac cac tac gca gtc agc gtt acc gtg gag ggc aag 192
Pro Thr Val Phe Asp His Tyr Ala Val Ser Val Thr Val Glu Gly Lys
    50                  55                  60
cag tac ctg ctg ggg ctc tac gac acc gcc ggg cag gaa gac tat gac 240
Gln Tyr Leu Leu Gly Leu Tyr Asp Thr Ala Gly Gln Glu Asp Tyr Asp
65                  70                  75                  80
cgt ctg aga cct tta tct tat cct atg acc gat gtc ttc ctt atc tgc 288
Arg Leu Arg Pro Leu Ser Tyr Pro Met Thr Asp Val Phe Leu Ile Cys
                85                  90                  95
ttc tca gtg gta aac cct gct tca ttt caa aac gtg aag gaa gaa tgg 336
Phe Ser Val Val Asn Pro Ala Ser Phe Gln Asn Val Lys Glu Glu Trp
            100                 105                 110
gta ccg gag ttg aag gaa tat gca cct aat gtt cct ttt tta cta gta 384
Val Pro Glu Leu Lys Glu Tyr Ala Pro Asn Val Pro Phe Leu Leu Val
        115                 120                 125
gga aca cag att gat ctt cgt gat gac ccc aaa act ctg gca aga ttg 432
Gly Thr Gln Ile Asp Leu Arg Asp Asp Pro Lys Thr Leu Ala Arg Leu
    130                 135                 140
aat gat atg aaa gag aag cct tta tct gtg gaa caa gga cag aaa tta 480
Asn Asp Met Lys Glu Lys Pro Leu Ser Val Glu Gln Gly Gln Lys Leu
145                 150                 155                 160
gca aaa gag ata gga gcc tac tgc tat gtg gag tgt tca gct tta aca 528
Ala Lys Glu Ile Gly Ala Tyr Cys Tyr Val Glu Cys Ser Ala Leu Thr
                165                 170                 175
cag aaa gga ctg aag act gtt ttt gat gaa gct att ata gcc att cta 576
Gln Lys Gly Leu Lys Thr Val Phe Asp Glu Ala Ile Ile Ala Ile Leu
            180                 185                 190
act cca aag aaa cac acg gtg aag aag aga ata ggt tcg aga tgc ata 624
Thr Pro Lys Lys His Thr Val Lys Lys Arg Ile Gly Ser Arg Cys Ile
        195                 200                 205
aac tgc tgt ttg atc acg tga 645
Asn Cys Cys Leu Ile Thr
    210


<210> 182
<211> 214
<212> PRT
<213> Gallus gallus

<400> 182
Met Pro Gly Gly Gly Arg Ala Pro Ala Met Ala His Gly Ser Ala Ala
1               5                   10                  15
Val Met Leu Lys Cys Val Val Val Gly Asp Gly Ala Val Gly Lys Thr
            20                  25                  30
Cys Leu Leu Met Ser Tyr Ala Asn Asp Ala Phe Pro Glu Glu Tyr Val
        35                  40                  45
Pro Thr Val Phe Asp His Tyr Ala Val Ser Val Thr Val Glu Gly Lys
    50                  55                  60
Gln Tyr Leu Leu Gly Leu Tyr Asp Thr Ala Gly Gln Glu Asp Tyr Asp

65                  70                  75                  80
Arg Leu Arg Pro Leu Ser Tyr Pro Met Thr Asp Val Phe Leu Ile Cys
                85                  90                  95
Phe Ser Val Val Asn Pro Ala Ser Phe Gln Asn Val Lys Glu Glu Trp
            100                 105                 110
Val Pro Glu Leu Lys Glu Tyr Ala Pro Asn Val Pro Phe Leu Leu Val
        115                 120                 125
Gly Thr Gln Ile Asp Leu Arg Asp Asp Pro Lys Thr Leu Ala Arg Leu
    130                 135                 140
Asn Asp Met Lys Glu Lys Pro Leu Ser Val Glu Gln Gly Gln Lys Leu
145                 150                 155                 160
Ala Lys Glu Ile Gly Ala Tyr Cys Tyr Val Glu Cys Ser Ala Leu Thr
                165                 170                 175
Gln Lys Gly Leu Lys Thr Val Phe Asp Glu Ala Ile Ile Ala Ile Leu
            180                 185                 190
Thr Pro Lys Lys His Thr Val Lys Lys Arg Ile Gly Ser Arg Cys Ile
        195                 200                 205
Asn Cys Cys Leu Ile Thr
    210

<210> 183
<211> 582
<212> DNA
<213> Brachydanio rerio

<220>
<221> CDS
<222> (1)..(582)

<400> 183
atg gct gca att cga aag aaa ctt gtc ata gtc ggt gat gga gcc tgt      48
Met Ala Ala Ile Arg Lys Lys Leu Val Ile Val Gly Asp Gly Ala Cys
1               5                   10                  15
gga aag acc tgt ttg ctc ata gtt ttc agt aaa gat cag ttt ccc gaa      96
Gly Lys Thr Cys Leu Leu Ile Val Phe Ser Lys Asp Gln Phe Pro Glu
            20                  25                  30
gtc tac gtg ccg aca gtc ttc gag aac tac gtt gca gat atc gag gtc      144
Val Tyr Val Pro Thr Val Phe Glu Asn Tyr Val Ala Asp Ile Glu Val
        35                  40                  45
gat tca aaa cag gtt gag ctt gcc tta tgg gat act gct gga cag gag      192
Asp Ser Lys Gln Val Glu Leu Ala Leu Trp Asp Thr Ala Gly Gln Glu
    50                  55                  60
gac tat gat cgg tta aga ccc ctc tcc tat cct gac aca gat gtt att      240
Asp Tyr Asp Arg Leu Arg Pro Leu Ser Tyr Pro Asp Thr Asp Val Ile
65                  70                  75                  80
ctc atg tgc ttc tcc atc gac agt cct gat agc ttg gaa aat atc cca      288
Leu Met Cys Phe Ser Ile Asp Ser Pro Asp Ser Leu Glu Asn Ile Pro
                85                  90                  95
gaa aaa tgg aca cca gag gtg aag cat ttc tgt ccc aat gtt ccc atc      336
Glu Lys Trp Thr Pro Glu Val Lys His Phe Cys Pro Asn Val Pro Ile
            100                 105                 110
atc ctc gtg ggt aac aaa aag gat ctc cgg aat gat gag cac aca cga      384
Ile Leu Val Gly Asn Lys Lys Asp Leu Arg Asn Asp Glu His Thr Arg
        115                 120                 125
agg gaa ctt gcc aaa atg aaa cag gag ccg gta aag cca gaa gaa ggg      432
Arg Glu Leu Ala Lys Met Lys Gln Glu Pro Val Lys Pro Glu Glu Gly
    130                 135                 140
cga gac atg gcc aac cga atc aat gcc ttt ggt tat cta gag tgt tcg      480
Arg Asp Met Ala Asn Arg Ile Asn Ala Phe Gly Tyr Leu Glu Cys Ser
145                 150                 155                 160
gcc aaa aca aaa gat ggc gtg aga gaa gtc ttt gaa atg gcc acc agg      528
Ala Lys Thr Lys Asp Gly Val Arg Glu Val Phe Glu Met Ala Thr Arg
                165                 170                 175
gcg gcg ctg caa gcc aga aaa cgt ggt aaa aag agc ggc tgc ctg ctg      576
Ala Ala Leu Gln Ala Arg Lys Arg Gly Lys Lys Ser Gly Cys Leu Leu
            180                 185                 190
tta taa                                                              582
Leu

<210> 184
<211> 193
<212> PRT
<213> Brachydanio rerio

<400> 184
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Ser Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Lys His Phe Cys Pro Asn Val Pro Ile
            100                 105                 110
Ile Leu Val Gly Asn Lys Lys Asp Leu Arg Asn Asp Glu His Thr Arg
        115                 120                 125
Arg Glu Leu Ala Lys Met Lys Gln Glu Pro Val Lys Pro Glu Glu Gly
    130                 135                 140
Arg Asp Met Ala Asn Arg Ile Asn Ala Phe Gly Tyr Leu Glu Cys Ser
145                 150                 155                 160
Ala Lys Thr Lys Asp Gly Val Arg Glu Val Phe Glu Met Ala Thr Arg
                165                 170                 175
Ala Ala Leu Gln Ala Arg Lys Arg Gly Lys Lys Ser Gly Cys Leu Leu
            180                 185                 190
Leu


<210> 185
<211> 582
<212> DNA
<213> Brachydanio rerio

<220>
<221> CDS
<222> (1)..(582)

<400> 185
atg gcg gct atc agg aaa aag ctg gtg atc gtg gga gat gga gca tgt       48
Met Ala Ala Ile Arg Lys Lys Leu Val Ile Val Gly Asp Gly Ala Cys
1               5                   10                  15
ggg aag acc tgt cta ctc ata gtg ttc agc aaa gac cag ttt cca gaa       96
Gly Lys Thr Cys Leu Leu Ile Val Phe Ser Lys Asp Gln Phe Pro Glu
            20                  25                  30
gtc tac gtg cct act gtg ttc gag aac tac att gct gac att gaa gtc       144
Val Tyr Val Pro Thr Val Phe Glu Asn Tyr Ile Ala Asp Ile Glu Val
        35                  40                  45
gac agc aaa cag gtg gag ctg gca ttg tgg gac aca gca gga cag gag       192
Asp Ser Lys Gln Val Glu Leu Ala Leu Trp Asp Thr Ala Gly Gln Glu
    50                  55                  60
gac tat gac cgt ctc aga cct ctg tct tac cca gac aca gat gtc atc       240
Asp Tyr Asp Arg Leu Arg Pro Leu Ser Tyr Pro Asp Thr Asp Val Ile
65                  70                  75                  80
ctc atg tgc ttc tcc ata gac agt ccc gac agt tta gag aat atc cca       288
Leu Met Cys Phe Ser Ile Asp Ser Pro Asp Ser Leu Glu Asn Ile Pro
                85                  90                  95
gaa aag tgg acg ccg gag gta aag cac ttc tgc ccc aac gtt ccc ata       336
Glu Lys Trp Thr Pro Glu Val Lys His Phe Cys Pro Asn Val Pro Ile
            100                 105                 110
atc ctg gtg ggc aat aag aga gat ctg cgt act gat gag aac aca cgg       384

Ile Leu Val Gly Asn Lys Arg Asp Leu Arg Thr Asp Glu Asn Thr Arg
        115                 120                 125
agg gag ctg act aag atg aag cag gag cca gtt aaa ata gaa gag ggc        432
Arg Glu Leu Thr Lys Met Lys Gln Glu Pro Val Lys Ile Glu Glu Gly
    130                 135                 140
agg gac atg gct aac cgc att agc gcc ttc ggc tac ctg gaa tgc tca        480
Arg Asp Met Ala Asn Arg Ile Ser Ala Phe Gly Tyr Leu Glu Cys Ser
145                 150                 155                 160
gct aag act aag gat ggc gtg agg gaa gtt ttt gaa atg gcc acc aga        528
Ala Lys Thr Lys Asp Gly Val Arg Glu Val Phe Glu Met Ala Thr Arg
                165                 170                 175
gcg gcg ctg cag gtt cgc aag agg aag aag agg agc ggg tgc tca ctg        576
Ala Ala Leu Gln Val Arg Lys Arg Lys Lys Arg Ser Gly Cys Ser Leu
            180                 185                 190
ttg tga                                                                582
Leu

<210> 186
<211> 193
<212> PRT
<213> Brachydanio rerio

<400> 186
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Ile Ala Asp Ile Glu Val
        35                  40                  45
Asp Ser Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Lys His Phe Cys Pro Asn Val Pro Ile
            100                 105                 110
Ile Leu Val Gly Asn Lys Arg Asp Leu Arg Thr Asp Glu Asn Thr Arg
        115                 120                 125
Arg Glu Leu Thr Lys Met Lys Gln Glu Pro Val Lys Ile Glu Glu Gly
    130                 135                 140
Arg Asp Met Ala Asn Arg Ile Ser Ala Phe Gly Tyr Leu Glu Cys Ser
145                 150                 155                 160
Ala Lys Thr Lys Asp Gly Val Arg Glu Val Phe Glu Met Ala Thr Arg
                165                 170                 175
Ala Ala Leu Gln Val Arg Lys Arg Lys Lys Arg Ser Gly Cys Ser Leu
            180                 185                 190
Leu

<210> 187
<211> 806
<212> DNA
<213> Ciona intestinalis

<220>
<221> CDS
<222> (1)..(750)

<400> 187
atg gaa agg ata ccc aat gac gtc aaa ggt gct ttc ccc gag cag caa        48
Met Glu Arg Ile Pro Asn Asp Val Lys Gly Ala Phe Pro Glu Gln Gln
1               5                   10                  15
ccc aaa att aaa tgt gtt ata gtt ggg gat ggt gga tgc ggt aaa act        96
Pro Lys Ile Lys Cys Val Ile Val Gly Asp Gly Gly Cys Gly Lys Thr
            20                  25                  30

tca atg ctg gtt gta tat act gaa ggc gaa ttc cct gaa gtg tac acg 144
Ser Met Leu Val Val Tyr Thr Glu Gly Glu Phe Pro Glu Val Tyr Thr
        35                  40                  45
ccc acg gtg ttt gaa aat tac agc aga gag gtg gaa tta aat cga aag 192
Pro Thr Val Phe Glu Asn Tyr Ser Arg Glu Val Glu Leu Asn Arg Lys
    50                  55                  60
aag gcg gtg cta aca ttg tgg gat acg gcg ggt cag gaa gat tat gac 240
Lys Ala Val Leu Thr Leu Trp Asp Thr Ala Gly Gln Glu Asp Tyr Asp
65                  70                  75                  80
cgg ctt cga cct ctc tct tac aac aac gca gac atc ata atc atc tgc 288
Arg Leu Arg Pro Leu Ser Tyr Asn Asn Ala Asp Ile Ile Ile Ile Cys
                85                  90                  95
tat gac gtc aca aac ctt tca agc ttc cag aac gta gaa atc cga tgg 336
Tyr Asp Val Thr Asn Leu Ser Ser Phe Gln Asn Val Glu Ile Arg Trp
            100                 105                 110
tcg cca gaa atc cgc cat ttt tgc ccc cac acg cct acg atg ctg gtt 384
Ser Pro Glu Ile Arg His Phe Cys Pro His Thr Pro Thr Met Leu Val
        115                 120                 125
gcg tgt aaa act gac ctg agg tct cag gtg aac tcg gat tca att tca 432
Ala Cys Lys Thr Asp Leu Arg Ser Gln Val Asn Ser Asp Ser Ile Ser
    130                 135                 140
aac tct atg act cga tct gct tcc atg gca acc aca tta aca aca act 480
Asn Ser Met Thr Arg Ser Ala Ser Met Ala Thr Thr Leu Thr Thr Thr
145                 150                 155                 160
gga gta gat tta aca gaa atg acg tca tct acg tta agt gta aat tcg 528
Gly Val Asp Leu Thr Glu Met Thr Ser Ser Thr Leu Ser Val Asn Ser
                165                 170                 175
gta cgc gca gaa gat gga aca gac aga cac gat gat caa gca ata gtt 576
Val Arg Ala Glu Asp Gly Thr Asp Arg His Asp Asp Gln Ala Ile Val
            180                 185                 190
aca aca gaa atg ggc gag aag atg tcg aaa cac atc aac gtc aat cat 624
Thr Thr Glu Met Gly Glu Lys Met Ser Lys His Ile Asn Val Asn His
        195                 200                 205
ttt gtt gaa tgt tct gcc aag gat ggc aca aac att gaa caa gta ttt 672
Phe Val Glu Cys Ser Ala Lys Asp Gly Thr Asn Ile Glu Gln Val Phe
    210                 215                 220
aaa ctt gca atc gaa tgc gtc att aaa cgt gaa gca aaa gtc ggt cca 720
Lys Leu Ala Ile Glu Cys Val Ile Lys Arg Glu Ala Lys Val Gly Pro
225                 230                 235                 240
aaa aac tgc tgc tgt tgt ttg cta ttg tgatacgctg ttacgtaaat 767
Lys Asn Cys Cys Cys Cys Leu Leu Leu
                245
aaattgactg tgtaggaaat gcctaacaaa tcaatcgcg 806

<210> 188
<211> 249
<212> PRT
<213> Ciona intestinalis

<400> 188
Met Glu Arg Ile Pro Asn Asp Val Lys Gly Ala Phe Pro Glu Gln Gln
1               5                   10                  15
Pro Lys Ile Lys Cys Val Ile Val Gly Asp Gly Gly Cys Gly Lys Thr
            20                  25                  30
Ser Met Leu Val Val Tyr Thr Glu Gly Glu Phe Pro Glu Val Tyr Thr
        35                  40                  45
Pro Thr Val Phe Glu Asn Tyr Ser Arg Glu Val Glu Leu Asn Arg Lys
    50                  55                  60
Lys Ala Val Leu Thr Leu Trp Asp Thr Ala Gly Gln Glu Asp Tyr Asp
65                  70                  75                  80
Arg Leu Arg Pro Leu Ser Tyr Asn Asn Ala Asp Ile Ile Ile Ile Cys
                85                  90                  95
Tyr Asp Val Thr Asn Leu Ser Ser Phe Gln Asn Val Glu Ile Arg Trp
            100                 105                 110
Ser Pro Glu Ile Arg His Phe Cys Pro His Thr Pro Thr Met Leu Val

115                 120                 125
Ala Cys Lys Thr Asp Leu Arg Ser Gln Val Asn Ser Asp Ser Ile Ser
    130                 135                 140
Asn Ser Met Thr Arg Ser Ala Ser Met Ala Thr Thr Leu Thr Thr Thr
145                 150                 155                 160
Gly Val Asp Leu Thr Glu Met Thr Ser Ser Thr Leu Ser Val Asn Ser
                165                 170                 175
Val Arg Ala Glu Asp Gly Thr Asp Arg His Asp Asp Gln Ala Ile Val
            180                 185                 190
Thr Thr Glu Met Gly Glu Lys Met Ser Lys His Ile Asn Val Asn His
        195                 200                 205
Phe Val Glu Cys Ser Ala Lys Asp Gly Thr Asn Ile Glu Gln Val Phe
    210                 215                 220
Lys Leu Ala Ile Glu Cys Val Ile Lys Arg Glu Ala Lys Val Gly Pro
225                 230                 235                 240
Lys Asn Cys Cys Cys Cys Leu Leu Leu
                245

<210> 189
<211> 726
<212> DNA
<213> Ciona intestinalis

<220>
<221> CDS
<222> (14)..(598)

<400> 189
ttgagttcat gaa atg cag tct ata aaa ttg gtc gtg gtg ggc gat ggt      49
               Met Gln Ser Ile Lys Leu Val Val Val Gly Asp Gly
               1               5                   10
gcc gtg gga aaa acg tgt cta ctt atc agc tac acg gcg aat gcc ttt      97
Ala Val Gly Lys Thr Cys Leu Leu Ile Ser Tyr Thr Ala Asn Ala Phe
        15                  20                  25
cca agg gag tat gtt ccg acc gtg ttt gaa aac tac atg gca aat atc      145
Pro Arg Glu Tyr Val Pro Thr Val Phe Glu Asn Tyr Met Ala Asn Ile
    30                  35                  40
acg gtc aac aat cag caa att tgc tta agt ctt tgg gat acc gct ggt      193
Thr Val Asn Asn Gln Gln Ile Cys Leu Ser Leu Trp Asp Thr Ala Gly
45                  50                  55                  60
caa gag gat ttt gac agg ttg aga ccg ctt tca tat cca gac acc gat      241
Gln Glu Asp Phe Asp Arg Leu Arg Pro Leu Ser Tyr Pro Asp Thr Asp
                65                  70                  75
gtc ttt gtt ctc tgc ttc tcg ata att tct cca acc tcc ttt gaa aat      289
Val Phe Val Leu Cys Phe Ser Ile Ile Ser Pro Thr Ser Phe Glu Asn
            80                  85                  90
ctc cag cac aaa tgg ttg ccc gag tta cga gaa cat tgt cct aat gtg      337
Leu Gln His Lys Trp Leu Pro Glu Leu Arg Glu His Cys Pro Asn Val
        95                  100                 105
ccc atc ctg cta gtg ggt acg aag ctt gac ttg aga gaa gac acc gaa      385
Pro Ile Leu Leu Val Gly Thr Lys Leu Asp Leu Arg Glu Asp Thr Glu
    110                 115                 120
att cta caa cag ctt tcc tcg aaa aat cta aaa cca ata aca cca gaa      433
Ile Leu Gln Gln Leu Ser Ser Lys Asn Leu Lys Pro Ile Thr Pro Glu
125                 130                 135                 140
gaa ggg gcg aaa atg gcg aaa gat att aaa gca gtt aaa tat tta gag      481
Glu Gly Ala Lys Met Ala Lys Asp Ile Lys Ala Val Lys Tyr Leu Glu
                145                 150                 155
tgt tct gct cta act cag gag tgt ctc agc caa gta ttt gat gac gct      529
Cys Ser Ala Leu Thr Gln Glu Cys Leu Ser Gln Val Phe Asp Asp Ala
            160                 165                 170
gtt att gca gtg cta aac cca tca cac ttt tca agt aac aat gac aac      577
Val Ile Ala Val Leu Asn Pro Ser His Phe Ser Ser Asn Asn Asp Asn
        175                 180                 185
agc tgt tgt aag atc gtt taaatttaca tctcgttgtt tacttacagt      625
Ser Cys Cys Lys Ile Val
    190
gttatgtgta ttaacattac cgtgatacac acacgagttc atagggttaa aaacgagagt      685

tatattttct ttaaaattag ataaacactc attcccgtca a                    726

<210> 190
<211> 194
<212> PRT
<213> Ciona intestinalis

<400> 190
Met Gln Ser Ile Lys Leu Val Val Val Gly Asp Gly Ala Val Gly Lys
1               5                   10                  15
Thr Cys Leu Leu Ile Ser Tyr Thr Ala Asn Ala Phe Pro Arg Glu Tyr
            20                  25                  30
Val Pro Thr Val Phe Glu Asn Tyr Met Ala Asn Ile Thr Val Asn Asn
        35                  40                  45
Gln Gln Ile Cys Leu Ser Leu Trp Asp Thr Ala Gly Gln Glu Asp Phe
    50                  55                  60
Asp Arg Leu Arg Pro Leu Ser Tyr Pro Asp Thr Asp Val Phe Val Leu
65                  70                  75                  80
Cys Phe Ser Ile Ile Ser Pro Thr Ser Phe Glu Asn Leu Gln His Lys
                85                  90                  95
Trp Leu Pro Glu Leu Arg Glu His Cys Pro Asn Val Pro Ile Leu Leu
            100                 105                 110
Val Gly Thr Lys Leu Asp Leu Arg Glu Asp Thr Glu Ile Leu Gln Gln
        115                 120                 125
Leu Ser Ser Lys Asn Leu Lys Pro Ile Thr Pro Glu Glu Gly Ala Lys
    130                 135                 140
Met Ala Lys Asp Ile Lys Ala Val Lys Tyr Leu Glu Cys Ser Ala Leu
145                 150                 155                 160
Thr Gln Glu Cys Leu Ser Gln Val Phe Asp Asp Ala Val Ile Ala Val
                165                 170                 175
Leu Asn Pro Ser His Phe Ser Ser Asn Asn Asp Asn Ser Cys Cys Lys
            180                 185                 190
Ile Val

<210> 191
<211> 827
<212> DNA
<213> Ciona intestinalis

<220>
<221> CDS
<222> (34)..(615)

<400> 191
atttgtaagg acgagaacca acacaacaaa ata atg cag tcc gtg aaa tgt gtt      54
                                     Met Gln Ser Val Lys Cys Val
                                     1               5
gtg gtt gga gat gga gct gtt ggt aaa acc tgt ttg ctg atc agc tac      102
Val Val Gly Asp Gly Ala Val Gly Lys Thr Cys Leu Leu Ile Ser Tyr
        10                  15                  20
aca acc aat gct ttc cct gga gaa tat att ccc act gtg ttt gaa agc      150
Thr Thr Asn Ala Phe Pro Gly Glu Tyr Ile Pro Thr Val Phe Glu Ser
    25                  30                  35
tat gct gca aat gtt gtg gtg gat ggg aac cca gtg aac atc ggt tta      198
Tyr Ala Ala Asn Val Val Val Asp Gly Asn Pro Val Asn Ile Gly Leu
40                  45                  50                  55
tgg gac aca gct ggc cag gaa gat tac gat aaa ctt cga ccg cta tca      246
Trp Asp Thr Ala Gly Gln Glu Asp Tyr Asp Lys Leu Arg Pro Leu Ser
                60                  65                  70
tac cca cag tca gat gtc ttt gtc atg tgc ttc tcg ttg gta aac cca      294
Tyr Pro Gln Ser Asp Val Phe Val Met Cys Phe Ser Leu Val Asn Pro
            75                  80                  85

aca tct tat gaa aac att gca gaa aaa tgg tat ccg gaa gtt cat gaa 342
Thr Ser Tyr Glu Asn Ile Ala Glu Lys Trp Tyr Pro Glu Val His Glu
        90                  95                  100
cac tgt ccc gac atc ccc att gta ctc gtt ggg aca aaa ctg gat tta 390
His Cys Pro Asp Ile Pro Ile Val Leu Val Gly Thr Lys Leu Asp Leu
    105                 110                 115
cgt gaa gac cca gaa aca ctc aaa acg ttg ggt gag aga aat ctt gtt 438
Arg Glu Asp Pro Glu Thr Leu Lys Thr Leu Gly Glu Arg Asn Leu Val
120                 125                 130                 135
cca gtt acc aaa acc cag ggc ctc cag ttg gcg aaa aaa gtt ggg gca 486
Pro Val Thr Lys Thr Gln Gly Leu Gln Leu Ala Lys Lys Val Gly Ala
                140                 145                 150
aag aaa tat ttt gag tgc tcg gcg ctt act cgt gaa aac tta gac gaa 534
Lys Lys Tyr Phe Glu Cys Ser Ala Leu Thr Arg Glu Asn Leu Asp Glu
            155                 160                 165
ctc ttt ctt gaa gca atg cgc aat gcc ctg aaa gga ccg gac aaa att 582
Leu Phe Leu Glu Ala Met Arg Asn Ala Leu Lys Gly Pro Asp Lys Ile
        170                 175                 180
gta aaa gag aaa cca aat tgt caa att atc taatcagcat ttaagaattt 632
Val Lys Glu Lys Pro Asn Cys Gln Ile Ile
    185                 190
tgagttaacg ctcgaataat tgttcagtat acaactgtat accaaagtcc ataaaaataa 692

aaattaaaac tatagacttt gctgtacaca ttatacatgt cgttaccgca tgtggtgtag 752

cttaaacgat tgtgcaatac ctttatatta aaggttaatg ttttacagtc gtgagtaact 812

ggaactctct gcgta 827

<210> 192
<211> 193
<212> PRT
<213> Ciona intestinalis

<400> 192
Met Gln Ser Val Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Glu Ser Tyr Ala Ala Asn Val Val Val Asp Gly
        35                  40                  45
Asn Pro Val Asn Ile Gly Leu Trp Asp Thr Ala Gly Gln Glu Asp Tyr
    50                  55                  60
Asp Lys Leu Arg Pro Leu Ser Tyr Pro Gln Ser Asp Val Phe Val Met
65                  70                  75                  80
Cys Phe Ser Leu Val Asn Pro Thr Ser Tyr Glu Asn Ile Ala Glu Lys
                85                  90                  95
Trp Tyr Pro Glu Val His Glu His Cys Pro Asp Ile Pro Ile Val Leu
            100                 105                 110
Val Gly Thr Lys Leu Asp Leu Arg Glu Asp Pro Glu Thr Leu Lys Thr
        115                 120                 125
Leu Gly Glu Arg Asn Leu Val Pro Val Thr Lys Thr Gln Gly Leu Gln
    130                 135                 140
Leu Ala Lys Lys Val Gly Ala Lys Lys Tyr Phe Glu Cys Ser Ala Leu
145                 150                 155                 160
Thr Arg Glu Asn Leu Asp Glu Leu Phe Leu Glu Ala Met Arg Asn Ala
                165                 170                 175
Leu Lys Gly Pro Asp Lys Ile Val Lys Glu Lys Pro Asn Cys Gln Ile
            180                 185                 190
Ile

<210> 193
<211> 718
<212> DNA
<213> Ciona intestinalis

<220>
<221> CDS
<222> (6)..(584)

<400> 193
aagat atg caa gcg ata aag tgc gtt gtg gtt gga gat gga gct gtt ggt      50
      Met Gln Ala Ile Lys Cys Val Val Val Gly Asp Gly Ala Val Gly
      1               5                   10                  15
aaa acc tgt ttg ctg atc agc tac aca act aac gct ttc cct gga gaa      98
Lys Thr Cys Leu Leu Ile Ser Tyr Thr Thr Asn Ala Phe Pro Gly Glu
                20                  25                  30
tat att ccc act gtg ttt gat aac tac tct gcc aat gtc atg gta gat      146
Tyr Ile Pro Thr Val Phe Asp Asn Tyr Ser Ala Asn Val Met Val Asp
            35                  40                  45
ggc cgc cct gtc aac ttg gga tta tgg gat aca gca gga cag gag gat      194
Gly Arg Pro Val Asn Leu Gly Leu Trp Asp Thr Ala Gly Gln Glu Asp
        50                  55                  60
tat gac aga ctc cga cct ctc tcc tac cca caa acc gat gtt ttt ctc      242
Tyr Asp Arg Leu Arg Pro Leu Ser Tyr Pro Gln Thr Asp Val Phe Leu
    65                  70                  75
att tgt ttc tct gtg gct tct ccc gct tcc tac gaa aac gtg cgc gca      290
Ile Cys Phe Ser Val Ala Ser Pro Ala Ser Tyr Glu Asn Val Arg Ala
80                  85                  90                  95
aag tgg cac ccg gag gtc gca cac cac tgc ccg gaa acg ccc gta ctt      338
Lys Trp His Pro Glu Val Ala His His Cys Pro Glu Thr Pro Val Leu
                100                 105                 110
ctc gtg gga aca aaa ctt gat tta cgt gac gat gcg gac act gtg aac      386
Leu Val Gly Thr Lys Leu Asp Leu Arg Asp Asp Ala Asp Thr Val Asn
            115                 120                 125
aag cta gct gag aaa aag ctc tcc acc att act act acc caa ggt tta      434
Lys Leu Ala Glu Lys Lys Leu Ser Thr Ile Thr Thr Thr Gln Gly Leu
        130                 135                 140
caa atg gcg aag gaa ctg ggg gcg gtt aaa tac caa gag tgc tct gct      482
Gln Met Ala Lys Glu Leu Gly Ala Val Lys Tyr Gln Glu Cys Ser Ala
    145                 150                 155
ctg acg caa aag ggg ctg aaa aat gtt ttc gac gaa gcg att cgg gcg      530
Leu Thr Gln Lys Gly Leu Lys Asn Val Phe Asp Glu Ala Ile Arg Ala
160                 165                 170                 175
gtt ctt aac cca aca aga aga gtc gtc cga acg aag aac tgc gaa att      578
Val Leu Asn Pro Thr Arg Arg Val Val Arg Thr Lys Asn Cys Glu Ile
                180                 185                 190
cta tgattacatt gacatgtaga gcggcctcga acgtttacaa taatttggaa      631
Leu

ttatgttcct atttaataaa tggagttcgg tggctttata tattctctct tatcgtttta      691


agcattttaa aatcgtcgct accgtgt      718




<210> 194
<211> 192
<212> PRT
<213> Ciona intestinalis

<400> 194
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly

                35                  40                  45
Arg Pro Val Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Val Ala Ser Pro Ala Ser Tyr Glu Asn Val Arg Ala Lys
                85                  90                  95
Trp His Pro Glu Val Ala His His Cys Pro Glu Thr Pro Val Leu Leu
            100                 105                 110
Val Gly Thr Lys Leu Asp Leu Arg Asp Asp Ala Asp Thr Val Asn Lys
        115                 120                 125
Leu Ala Glu Lys Lys Leu Ser Thr Ile Thr Thr Thr Gln Gly Leu Gln
    130                 135                 140
Met Ala Lys Glu Leu Gly Ala Val Lys Tyr Gln Glu Cys Ser Ala Leu
145                 150                 155                 160
Thr Gln Lys Gly Leu Lys Asn Val Phe Asp Glu Ala Ile Arg Ala Val
                165                 170                 175
Leu Asn Pro Thr Arg Arg Val Val Arg Thr Lys Asn Cys Glu Ile Leu
            180                 185                 190

<210> 195
<211> 579
<212> DNA
<213> Ciona intestinalis

<220>
<221> CDS
<222> (1)..(579)

<400> 195
atg cag gcg atc aag tgt gtt gtg gtt gga gat gga gct gtt ggt aaa      48
Met Gln Ala Ile Lys Cys Val Val Val Gly Asp Gly Ala Val Gly Lys
1               5                   10                  15
acc tgt ttg ctg atc agc tac aca acc aat gct ttc cct gga gaa tat      96
Thr Cys Leu Leu Ile Ser Tyr Thr Thr Asn Ala Phe Pro Gly Glu Tyr
            20                  25                  30
att ccc act gtg ttt gat aac tac tct gcc aat gtc atg gta gat ggc      144
Ile Pro Thr Val Phe Asp Asn Tyr Ser Ala Asn Val Met Val Asp Gly
        35                  40                  45
cgc cct gtc aac ttg gga tta tgg gat aca gca ggg cag gag gat tac      192
Arg Pro Val Asn Leu Gly Leu Trp Asp Thr Ala Gly Gln Glu Asp Tyr
    50                  55                  60
gac aga ctt cgg cct ctc tcc tac cca caa acg gac gtt ttt ctg atc      240
Asp Arg Leu Arg Pro Leu Ser Tyr Pro Gln Thr Asp Val Phe Leu Ile
65                  70                  75                  80
tgt ttc tca ctc gtg tca cca gcc tca ttc gaa aac gtg cgc gca aag      288
Cys Phe Ser Leu Val Ser Pro Ala Ser Phe Glu Asn Val Arg Ala Lys
                85                  90                  95
tgg tat cca gaa gtc gca cac cac tgt cca gat aca ccg gtc att ctc      336
Trp Tyr Pro Glu Val Ala His His Cys Pro Asp Thr Pro Val Ile Leu
            100                 105                 110
gtg gga aca aaa ctt gat tta cgt gac gac cag gaa acc atc caa aag      384
Val Gly Thr Lys Leu Asp Leu Arg Asp Asp Gln Glu Thr Ile Gln Lys
        115                 120                 125
ctg aaa gaa aag aaa ctt gcc cca atc ctc tac cca caa ggg ttg cag      432
Leu Lys Glu Lys Lys Leu Ala Pro Ile Leu Tyr Pro Gln Gly Leu Gln
    130                 135                 140
atg gcg aaa gaa gtg aac gcc gta aag tac ctg gag tgc tcg gct ctc      480
Met Ala Lys Glu Val Asn Ala Val Lys Tyr Leu Glu Cys Ser Ala Leu
145                 150                 155                 160
acc caa aaa ggc ctg aaa acc gtt ttc gac gag gcg atc cgc gcc gtc      528
Thr Gln Lys Gly Leu Lys Thr Val Phe Asp Glu Ala Ile Arg Ala Val
                165                 170                 175
ctg tgc cct gag caa aag ccg aag aaa aag aag ccc tgc gag ctt ttg      576
Leu Cys Pro Glu Gln Lys Pro Lys Lys Lys Lys Pro Cys Glu Leu Leu
            180                 185                 190
tga                                                                  579

152

<210> 196
<211> 192
<212> PRT
<213> Ciona intestinalis

<400> 196
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly
        35                  40                  45
Arg Pro Val Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Ser Pro Ala Ser Phe Glu Asn Val Arg Ala Lys
                85                  90                  95
Trp Tyr Pro Glu Val Ala His His Cys Pro Asp Thr Pro Val Ile Leu
            100                 105                 110
Val Gly Thr Lys Leu Asp Leu Arg Asp Asp Gln Glu Thr Ile Gln Lys
        115                 120                 125
Leu Lys Glu Lys Lys Leu Ala Pro Ile Leu Tyr Pro Gln Gly Leu Gln
    130                 135                 140
Met Ala Lys Glu Val Asn Ala Val Lys Tyr Leu Glu Cys Ser Ala Leu
145                 150                 155                 160
Thr Gln Lys Gly Leu Lys Thr Val Phe Asp Glu Ala Ile Arg Ala Val
                165                 170                 175
Leu Cys Pro Glu Gln Lys Pro Lys Lys Lys Lys Pro Cys Glu Leu Leu
            180                 185                 190

<210> 197
<211> 727
<212> DNA
<213> Ciona intestinalis

<220>
<221> CDS
<222> (1)..(582)

<400> 197
atg gct tca att cgt aag aag ctg gta ata gtt ggt gat ggt gct tgt      48
Met Ala Ser Ile Arg Lys Lys Leu Val Ile Val Gly Asp Gly Ala Cys
1               5                   10                  15
ggt aaa act tgc tta ctc atc gtg ttt agc aaa gat caa ttc cca gaa      96
Gly Lys Thr Cys Leu Leu Ile Val Phe Ser Lys Asp Gln Phe Pro Glu
            20                  25                  30
gtt tac gtc cca act gtt ttt gaa aac tat gtg gct gat att gaa gta      144
Val Tyr Val Pro Thr Val Phe Glu Asn Tyr Val Ala Asp Ile Glu Val
        35                  40                  45
gac tct aaa cag gtt gag ctt gct ttg tgg gat aca gct ggt caa gaa      192
Asp Ser Lys Gln Val Glu Leu Ala Leu Trp Asp Thr Ala Gly Gln Glu
    50                  55                  60
gat tac gac agg ctt cgt cca ctt tcc tac ccc gat act gat gtt att      240
Asp Tyr Asp Arg Leu Arg Pro Leu Ser Tyr Pro Asp Thr Asp Val Ile
65                  70                  75                  80
ctt atg tgt ttc tca atc gac agc cca gat tca ctt gag aac att ccc      288
Leu Met Cys Phe Ser Ile Asp Ser Pro Asp Ser Leu Glu Asn Ile Pro
                85                  90                  95
gaa aaa tgg acc cct gag gta agg cat ttt tgc cca agt gtt cca atc      336
Glu Lys Trp Thr Pro Glu Val Arg His Phe Cys Pro Ser Val Pro Ile
            100                 105                 110
att ttg gtt gga aac aaa aaa gat ctt cgt aac gac agt tca aca ata      384
Ile Leu Val Gly Asn Lys Lys Asp Leu Arg Asn Asp Ser Ser Thr Ile
        115                 120                 125

aag gaa ctt gct aaa atg aaa caa tct gcc gtt agt aac gaa gat gga 432
Lys Glu Leu Ala Lys Met Lys Gln Ser Ala Val Ser Asn Glu Asp Gly
    130                 135                 140
atg gcg atg gca gag aag att ggc gct tac gga tat atg gaa tgt tca 480
Met Ala Met Ala Glu Lys Ile Gly Ala Tyr Gly Tyr Met Glu Cys Ser
145                 150                 155                 160
gcg cgc acc aaa gaa ggt gtt cgg gaa gtg ttt gag ctt gca act aaa 528
Ala Arg Thr Lys Glu Gly Val Arg Glu Val Phe Glu Leu Ala Thr Lys
                165                 170                 175
gca gct tta caa acc aag aaa aga aag aaa aag agt gga tgt gaa gtc 576
Ala Ala Leu Gln Thr Lys Lys Arg Lys Lys Lys Ser Gly Cys Glu Val
            180                 185                 190
ttg taaagaaatt ttaactggag tctagcaatt gacttaacat tggacgcatg 629
Leu

ctttcatact ccccaacaaa ttaatattta caatgttgga aacgtattct gcatgacttg 689

aactcaatta ctcaaataga tggcaggatt tatacaca 727

<210> 198
<211> 193
<212> PRT
<213> Ciona intestinalis

<400> 198
Met Ala Ser Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Ser Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Arg His Phe Cys Pro Ser Val Pro Ile
            100                 105                 110
Ile Leu Val Gly Asn Lys Lys Asp Leu Arg Asn Asp Ser Ser Thr Ile
        115                 120                 125
Lys Glu Leu Ala Lys Met Lys Gln Ser Ala Val Ser Asn Glu Asp Gly
    130                 135                 140
Met Ala Met Ala Glu Lys Ile Gly Ala Tyr Gly Tyr Met Glu Cys Ser
145                 150                 155                 160
Ala Arg Thr Lys Glu Gly Val Arg Glu Val Phe Glu Leu Ala Thr Lys
                165                 170                 175
Ala Ala Leu Gln Thr Lys Lys Arg Lys Lys Lys Ser Gly Cys Glu Val
            180                 185                 190
Leu

<210> 199
<211> 714
<212> DNA
<213> Trichomonas vaginalis

<220>
<221> CDS
<222> (11)..(613)

<400> 199
atttcgctag atg aag cat atc aaa tgc gtt gtt gtc ggt gat ggt gca 49
Met Lys His Ile Lys Cys Val Val Val Gly Asp Gly Ala

1                   5                   10
gtc ggt aag act tgc ctt ctt atc tca ttc act aca aat gct ttc cca          97
Val Gly Lys Thr Cys Leu Leu Ile Ser Phe Thr Thr Asn Ala Phe Pro
    15                  20                  25
ggt gaa tat atc cca aca gtt ttc gat aac tac tct gca aac gtt atg         145
Gly Glu Tyr Ile Pro Thr Val Phe Asp Asn Tyr Ser Ala Asn Val Met
30                  35                  40                  45
gct gaa gga cat cct cca gtc aac ctc cag ctt tgg gat aca gct ggc         193
Ala Glu Gly His Pro Pro Val Asn Leu Gln Leu Trp Asp Thr Ala Gly
                50                  55                  60
cag gaa gac tac aag aag ctt cgc cca ctt tct tac cct caa aca gat         241
Gln Glu Asp Tyr Lys Lys Leu Arg Pro Leu Ser Tyr Pro Gln Thr Asp
            65                  70                  75
gtt ttc ctc ctc tgt ttc tct tta gtt tat cca gct tct ctc gag aac         289
Val Phe Leu Leu Cys Phe Ser Leu Val Tyr Pro Ala Ser Leu Glu Asn
        80                  85                  90
atc gag acc atg tgg atc aaa gaa atc aag gaa tac tgc cca gac aag         337
Ile Glu Thr Met Trp Ile Lys Glu Ile Lys Glu Tyr Cys Pro Asp Lys
    95                  100                 105
cca tac atc ctt gtc ggt ctt aag tcc gat ctt cgt gat gaa ttc gat         385
Pro Tyr Ile Leu Val Gly Leu Lys Ser Asp Leu Arg Asp Glu Phe Asp
110                 115                 120                 125
cag cgc gca gat gaa ctc cgc gct aag ggt tac gag cca atc cca cgc         433
Gln Arg Ala Asp Glu Leu Arg Ala Lys Gly Tyr Glu Pro Ile Pro Arg
                130                 135                 140
gct aag ggt gag gaa atg gca aag aag atc aac gcc tgc tcc tac ata         481
Ala Lys Gly Glu Glu Met Ala Lys Lys Ile Asn Ala Cys Ser Tyr Ile
            145                 150                 155
gaa tgc tct gcc ctt aaa tca tac aac ctc act gaa gtt ttc gat gaa         529
Glu Cys Ser Ala Leu Lys Ser Tyr Asn Leu Thr Glu Val Phe Asp Glu
        160                 165                 170
gcc gtc aag tac gct ctc gaa cca cca gct cag cag acc cag act aaa         577
Ala Val Lys Tyr Ala Leu Glu Pro Pro Ala Gln Gln Thr Gln Thr Lys
    175                 180                 185
gaa aag aca ggt ggt ggc tgc tgc gaa ctt att taaatttttt gcatatatgg       630
Glu Lys Thr Gly Gly Gly Cys Cys Glu Leu Ile
190                 195                 200
taccagagtt aaaataaaac atgttgaatg ctgtgacaaa caactttata atccgactcg       690

gtgtaataat aaaagggaat tagg                                              714

<210> 200
<211> 200
<212> PRT
<213> Trichomonas vaginalis

<400> 200
Met Lys His Ile Lys Cys Val Val Val Gly Asp Gly Ala Val Gly Lys
1               5                   10                  15
Thr Cys Leu Leu Ile Ser Phe Thr Thr Asn Ala Phe Pro Gly Glu Tyr
            20                  25                  30
Ile Pro Thr Val Phe Asp Asn Tyr Ser Ala Asn Val Met Ala Glu Gly
        35                  40                  45
His Pro Pro Val Asn Leu Gln Leu Trp Asp Thr Ala Gly Gln Glu Asp
    50                  55                  60
Tyr Lys Lys Leu Arg Pro Leu Ser Tyr Pro Gln Thr Asp Val Phe Leu
65                  70                  75                  80
Leu Cys Phe Ser Leu Val Tyr Pro Ala Ser Leu Glu Asn Ile Glu Thr
                85                  90                  95
Met Trp Ile Lys Glu Ile Lys Glu Tyr Cys Pro Asp Lys Pro Tyr Ile
            100                 105                 110
Leu Val Gly Leu Lys Ser Asp Leu Arg Asp Glu Phe Asp Gln Arg Ala
        115                 120                 125
Asp Glu Leu Arg Ala Lys Gly Tyr Glu Pro Ile Pro Arg Ala Lys Gly

    130                 135                 140
Glu Glu Met Ala Lys Lys Ile Asn Ala Cys Ser Tyr Ile Glu Cys Ser
145                 150                 155                 160
Ala Leu Lys Ser Tyr Asn Leu Thr Glu Val Phe Asp Glu Ala Val Lys
                165                 170                 175
Tyr Ala Leu Glu Pro Pro Ala Gln Gln Thr Gln Thr Lys Glu Lys Thr
            180                 185                 190
Gly Gly Gly Cys Cys Glu Leu Ile
        195                 200

<210> 201
<211> 600
<212> DNA
<213> Colletotrichum trifolii

<220>
<221> CDS
<222> (1)..(600)

<400> 201
atg gct caa cca gga gta cag tcg ctg aag tgt gtg gtg acg ggt gac 48
Met Ala Gln Pro Gly Val Gln Ser Leu Lys Cys Val Val Thr Gly Asp
1               5                   10                  15
ggt gct gtc gga aag aca tgt ttg ctg att tcc tac aca acg aac gcc 96
Gly Ala Val Gly Lys Thr Cys Leu Leu Ile Ser Tyr Thr Thr Asn Ala
            20                  25                  30
ttt cct ggc gag tac att cct act gtc ttc gac aac tac tcg gcg agt 144
Phe Pro Gly Glu Tyr Ile Pro Thr Val Phe Asp Asn Tyr Ser Ala Ser
        35                  40                  45
gta atg gtg gac gga aag cct att agc ttg gga ctg tgg gat act gcc 192
Val Met Val Asp Gly Lys Pro Ile Ser Leu Gly Leu Trp Asp Thr Ala
    50                  55                  60
ggc cag gaa gat tac gac aga ctg cga ccg ctt tcc tac ccc cag acc 240
Gly Gln Glu Asp Tyr Asp Arg Leu Arg Pro Leu Ser Tyr Pro Gln Thr
65                  70                  75                  80
gac gtc ttc ctg att tgc ttc tcc atc gtc agc ccc cca tcg ttt gac 288
Asp Val Phe Leu Ile Cys Phe Ser Ile Val Ser Pro Pro Ser Phe Asp
                85                  90                  95
aac gtc aag gcc aag tgg tac ccc gaa atc gat cat cac gcc ccc aac 336
Asn Val Lys Ala Lys Trp Tyr Pro Glu Ile Asp His His Ala Pro Asn
            100                 105                 110
atc ccc att atc ctc gtc ggc acc aag ctg gat ttg agg gag gat ccc 384
Ile Pro Ile Ile Leu Val Gly Thr Lys Leu Asp Leu Arg Glu Asp Pro
        115                 120                 125
aac acc ctc gag tcc ctc cgc caa aag cgg atg gag ccc gtg tcg tac 432
Asn Thr Leu Glu Ser Leu Arg Gln Lys Arg Met Glu Pro Val Ser Tyr
    130                 135                 140
gat caa gcc ctg atc tgc gcc aag gaa att aag gca cac aaa tac ctg 480
Asp Gln Ala Leu Ile Cys Ala Lys Glu Ile Lys Ala His Lys Tyr Leu
145                 150                 155                 160
gag tgt tct gcc ctg aca cag agg aat ctg aag agc gtt ttt gac gag 528
Glu Cys Ser Ala Leu Thr Gln Arg Asn Leu Lys Ser Val Phe Asp Glu
                165                 170                 175
gcc att cgt gct gtc ctg aac ccc agg ccc gtc gcg cag cag aag aag 576
Ala Ile Arg Ala Val Leu Asn Pro Arg Pro Val Ala Gln Gln Lys Lys
            180                 185                 190
aag tcg aag tgt acg att ttg tga 600
Lys Ser Lys Cys Thr Ile Leu
        195

<210> 202
<211> 199
<212> PRT
<213> Colletotrichum trifolii

<400> 202
Met Ala Gln Pro Gly Val Gln Ser Leu Lys Cys Val Val Thr Gly Asp

1               5                   10                  15
Gly Ala Val Gly Lys Thr Cys Leu Leu Ile Ser Tyr Thr Thr Asn Ala
            20                  25                  30
Phe Pro Gly Glu Tyr Ile Pro Thr Val Phe Asp Asn Tyr Ser Ala Ser
        35                  40                  45
Val Met Val Asp Gly Lys Pro Ile Ser Leu Gly Leu Trp Asp Thr Ala
    50                  55                  60
Gly Gln Glu Asp Tyr Asp Arg Leu Arg Pro Leu Ser Tyr Pro Gln Thr
65                  70                  75                  80
Asp Val Phe Leu Ile Cys Phe Ser Ile Val Ser Pro Pro Ser Phe Asp
                85                  90                  95
Asn Val Lys Ala Lys Trp Tyr Pro Glu Ile Asp His His Ala Pro Asn
            100                 105                 110
Ile Pro Ile Ile Leu Val Gly Thr Lys Leu Asp Leu Arg Glu Asp Pro
        115                 120                 125
Asn Thr Leu Glu Ser Leu Arg Gln Lys Arg Met Glu Pro Val Ser Tyr
    130                 135                 140
Asp Gln Ala Leu Ile Cys Ala Lys Glu Ile Lys Ala His Lys Tyr Leu
145                 150                 155                 160
Glu Cys Ser Ala Leu Thr Gln Arg Asn Leu Lys Ser Val Phe Asp Glu
                165                 170                 175
Ala Ile Arg Ala Val Leu Asn Pro Arg Pro Val Ala Gln Gln Lys Lys
            180                 185                 190
Lys Ser Lys Cys Thr Ile Leu
        195

<210> 203
<211> 618
<212> DNA
<213> Brachydanio rerio

<220>
<221> CDS
<222> (1)..(618)

<400> 203
atg gca aac gga acc ggc agc atc atg ttg aaa tgc gtg gtc gtc ggt      48
Met Ala Asn Gly Thr Gly Ser Ile Met Leu Lys Cys Val Val Val Gly
1               5                   10                  15
gac ggc gcc gtt gga aaa act tgt ctg ttg atg agc tat gcc aac gac      96
Asp Gly Ala Val Gly Lys Thr Cys Leu Leu Met Ser Tyr Ala Asn Asp
            20                  25                  30
gcc ttt cca gag gaa tat gtg ccc acc gtc ttt gat cac tat gca gtg      144
Ala Phe Pro Glu Glu Tyr Val Pro Thr Val Phe Asp His Tyr Ala Val
        35                  40                  45
agt gtg acg gtc ggg ggg aaa cag tac ctg ctg ggg ctc tat gac act      192
Ser Val Thr Val Gly Gly Lys Gln Tyr Leu Leu Gly Leu Tyr Asp Thr
    50                  55                  60
gcg ggt cag gag gac tat gat cgc ctg cga ccc ctg tcc tat cct atg      240
Ala Gly Gln Glu Asp Tyr Asp Arg Leu Arg Pro Leu Ser Tyr Pro Met
65                  70                  75                  80
acg gat gtc ttc ctc att tgc ttc tcc gtg gtc aat ccg gcc agc ttc      288
Thr Asp Val Phe Leu Ile Cys Phe Ser Val Val Asn Pro Ala Ser Phe
                85                  90                  95
cag aat gta aga gag gaa tgg gtc cct gag ctg cag gaa tat gca cca      336
Gln Asn Val Arg Glu Glu Trp Val Pro Glu Leu Gln Glu Tyr Ala Pro
            100                 105                 110
aac atc cct tac ctg ctc att ggc act cag att gac ctg cgg gat gac      384
Asn Ile Pro Tyr Leu Leu Ile Gly Thr Gln Ile Asp Leu Arg Asp Asp
        115                 120                 125
ccc aag act atc gcc aag ctc aac gac gtg aag gag aag ccc att gtg      432
Pro Lys Thr Ile Ala Lys Leu Asn Asp Val Lys Glu Lys Pro Ile Val
    130                 135                 140
acg gaa caa ggc caa aag cta gca aag gag att ggt gcc tgc tgc tat      480
Thr Glu Gln Gly Gln Lys Leu Ala Lys Glu Ile Gly Ala Cys Cys Tyr
145                 150                 155                 160
gtt gaa tgc tca gca cta aca cag aag ggc ctg aag act gtg ttt gat      528
Val Glu Cys Ser Ala Leu Thr Gln Lys Gly Leu Lys Thr Val Phe Asp

                    165                 170                 175
gag gcc att ata gcc att ctg gct cct aag aaa ggc gca ctg aag cga       576
Glu Ala Ile Ile Ala Ile Leu Ala Pro Lys Lys Gly Ala Leu Lys Arg
            180                 185                 190
aga cta ggc ccg cgc tgc att aac tgc tgc ctc atc aca tga               618
Arg Leu Gly Pro Arg Cys Ile Asn Cys Cys Leu Ile Thr
        195                 200                 205

<210> 204
<211> 205
<212> PRT
<213> Brachydanio rerio

<400> 204
Met Ala Asn Gly Thr Gly Ser Ile Met Leu Lys Cys Val Val Val Gly
1               5                   10                  15
Asp Gly Ala Val Gly Lys Thr Cys Leu Leu Met Ser Tyr Ala Asn Asp
            20                  25                  30
Ala Phe Pro Glu Glu Tyr Val Pro Thr Val Phe Asp His Tyr Ala Val
        35                  40                  45
Ser Val Thr Val Gly Gly Lys Gln Tyr Leu Leu Gly Leu Tyr Asp Thr
    50                  55                  60
Ala Gly Gln Glu Asp Tyr Asp Arg Leu Arg Pro Leu Ser Tyr Pro Met
65                  70                  75                  80
Thr Asp Val Phe Leu Ile Cys Phe Ser Val Val Asn Pro Ala Ser Phe
                85                  90                  95
Gln Asn Val Arg Glu Glu Trp Val Pro Glu Leu Gln Glu Tyr Ala Pro
            100                 105                 110
Asn Ile Pro Tyr Leu Leu Ile Gly Thr Gln Ile Asp Leu Arg Asp Asp
        115                 120                 125
Pro Lys Thr Ile Ala Lys Leu Asn Asp Val Lys Glu Lys Pro Ile Val
    130                 135                 140
Thr Glu Gln Gly Gln Lys Leu Ala Lys Glu Ile Gly Ala Cys Cys Tyr
145                 150                 155                 160
Val Glu Cys Ser Ala Leu Thr Gln Lys Gly Leu Lys Thr Val Phe Asp
                165                 170                 175
Glu Ala Ile Ile Ala Ile Leu Ala Pro Lys Lys Gly Ala Leu Lys Arg
            180                 185                 190
Arg Leu Gly Pro Arg Cys Ile Asn Cys Cys Leu Ile Thr
        195                 200                 205

<210> 205
<211> 582
<212> DNA
<213> Xenopus laevis

<220>
<221> CDS
<222> (1)..(582)

<400> 205
atg gca gcg att cgg aaa aag ctg gtg att gtt gga gat ggt gcc tgt       48
Met Ala Ala Ile Arg Lys Lys Leu Val Ile Val Gly Asp Gly Ala Cys
1               5                   10                  15
ggg aag acc tgc ctg ctc att gta ttc agc aag gat cag ttc cct gag       96
Gly Lys Thr Cys Leu Leu Ile Val Phe Ser Lys Asp Gln Phe Pro Glu
            20                  25                  30
gtt tat gtc cca act gtc ttt gag aac tac att gcc gat ata gaa gtc       144
Val Tyr Val Pro Thr Val Phe Glu Asn Tyr Ile Ala Asp Ile Glu Val
        35                  40                  45
gat gga aaa cag gtg gaa ctg gct ctg tgg gac aca gca gga cag gag       192
Asp Gly Lys Gln Val Glu Leu Ala Leu Trp Asp Thr Ala Gly Gln Glu
    50                  55                  60
gat tat gac agg cta cgc cca ctt tct tac cct gat act gat gtg att       240
Asp Tyr Asp Arg Leu Arg Pro Leu Ser Tyr Pro Asp Thr Asp Val Ile
65                  70                  75                  80
ttg atg tgt ttc tca att gac agc cct gac agt tta gag aat att cct       288

Leu Met Cys Phe Ser Ile Asp Ser Pro Asp Ser Leu Glu Asn Ile Pro
                85                  90                  95
gag aaa tgg act cca gaa gtg aag cat ttc tgt ccc aat gta ccc att      336
Glu Lys Trp Thr Pro Glu Val Lys His Phe Cys Pro Asn Val Pro Ile
            100                 105                 110
att ctt gtc ggt aac aaa aag gat cta aga aat gat gaa cac act aga      384
Ile Leu Val Gly Asn Lys Lys Asp Leu Arg Asn Asp Glu His Thr Arg
        115                 120                 125
aga gag ctg aca aag atg aag cag gag cca gtt aaa cca gaa gat ggc      432
Arg Glu Leu Thr Lys Met Lys Gln Glu Pro Val Lys Pro Glu Asp Gly
    130                 135                 140
aga gaa atg gcc aac aga atc aat gca ttt ggc tac ctg gaa tgt tct      480
Arg Glu Met Ala Asn Arg Ile Asn Ala Phe Gly Tyr Leu Glu Cys Ser
145                 150                 155                 160
gct aag aca aag gat ggc gtg aga gaa gtt ttt gaa atg gct aca cgg      528
Ala Lys Thr Lys Asp Gly Val Arg Glu Val Phe Glu Met Ala Thr Arg
                165                 170                 175
gct gcc ttg caa gtt agg aaa cac aga aag aag caa cgt tgt tct att      576
Ala Ala Leu Gln Val Arg Lys His Arg Lys Lys Gln Arg Cys Ser Ile
            180                 185                 190
cta taa                                                              582
Leu

<210> 206
<211> 193
<212> PRT
<213> Xenopus laevis

<400> 206
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Ile Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Lys His Phe Cys Pro Asn Val Pro Ile
            100                 105                 110
Ile Leu Val Gly Asn Lys Lys Asp Leu Arg Asn Asp Glu His Thr Arg
        115                 120                 125
Arg Glu Leu Thr Lys Met Lys Gln Glu Pro Val Lys Pro Glu Asp Gly
    130                 135                 140
Arg Glu Met Ala Asn Arg Ile Asn Ala Phe Gly Tyr Leu Glu Cys Ser
145                 150                 155                 160
Ala Lys Thr Lys Asp Gly Val Arg Glu Val Phe Glu Met Ala Thr Arg
                165                 170                 175
Ala Ala Leu Gln Val Arg Lys His Arg Lys Lys Gln Arg Cys Ser Ile
            180                 185                 190
Leu

<210> 207
<211> 1092
<212> DNA
<213> Xenopus laevis

<220>
<221> CDS
<222> (25)..(606)

<400> 207

ggagggagct gcgagtagga aggc atg gca gcc ata agg aag aag ctg gtg 51
Met Ala Ala Ile Arg Lys Lys Leu Val
1               5
att gtg ggc gac ggg gcg tgt ggc aag act tgt ctc ctc atc gtc ttt 99
Ile Val Gly Asp Gly Ala Cys Gly Lys Thr Cys Leu Leu Ile Val Phe
10                  15                  20                  25
agc aaa gac cag ttc cca gag gtc tat gtt ccc acc gtc ttt gag aac 147
Ser Lys Asp Gln Phe Pro Glu Val Tyr Val Pro Thr Val Phe Glu Asn
                30                  35                  40
tac gtg gcc gac atc gag gtg gac tcc aag cag gta gaa ctg gct ctg 195
Tyr Val Ala Asp Ile Glu Val Asp Ser Lys Gln Val Glu Leu Ala Leu
            45                  50                  55
tgg gac acg gct ggg caa gag gat tac gac cga ctg aga ccc ctc tcc 243
Trp Asp Thr Ala Gly Gln Glu Asp Tyr Asp Arg Leu Arg Pro Leu Ser
        60                  65                  70
tac cct gac aca gac gtc atc ctc atg tgt ttc tcc atc gac agc ccc 291
Tyr Pro Asp Thr Asp Val Ile Leu Met Cys Phe Ser Ile Asp Ser Pro
    75                  80                  85
gac agt ttg gaa aat atc cct gag aaa tgg acc cca gaa gtg aaa cat 339
Asp Ser Leu Glu Asn Ile Pro Glu Lys Trp Thr Pro Glu Val Lys His
90                  95                  100                 105
ttc tgc ccc aat gtg cca ata atc ctg gtg gga aac aag aag gat ctg 387
Phe Cys Pro Asn Val Pro Ile Ile Leu Val Gly Asn Lys Lys Asp Leu
                110                 115                 120
cgg aat gac gag cac acg cgc cgg gag ttg gcc aaa atg aag cag gag 435
Arg Asn Asp Glu His Thr Arg Arg Glu Leu Ala Lys Met Lys Gln Glu
            125                 130                 135
cca gta aga gca gag gag gga agg gac atg gcc aat cga atc agc gcc 483
Pro Val Arg Ala Glu Glu Gly Arg Asp Met Ala Asn Arg Ile Ser Ala
        140                 145                 150
ttt ggc tac ttg gag tgc tct gcc aag acc aag gat ggc gta aga gaa 531
Phe Gly Tyr Leu Glu Cys Ser Ala Lys Thr Lys Asp Gly Val Arg Glu
    155                 160                 165
gtg ttt gag atg gcg act cgg gcg gct ctg cag gcc aaa cgt ggg cgc 579
Val Phe Glu Met Ala Thr Arg Ala Ala Leu Gln Ala Lys Arg Gly Arg
170                 175                 180                 185
aag agg agc acc tgc cag tta ctg tgaggagaga agccccatgc cctcattctc 633
Lys Arg Ser Thr Cys Gln Leu Leu
                190
ccccttccct gtgccctctc tctccgctgt gggctagggg agctgcggac tgaggagacg 693
caccaataac caatcccttt cctgttctca tgggcagcgc caggccccga gtccagcaaa 753
tcacatcagt gccaggtctg tagcaacagg gggtttctta caacagtgcc attgtctagt 813
gcagggcccc ttcctctaaa aggcgggggg gggattattt ggctttagaa ggccccgctg 873
tcctgggggt cggattgtac acacgagagc gggacccctc gtgatattgg ttaattccac 933
cccgaccctg tgctggcgag ggttaatcct gcagctcatg agaggggtgg ggaggtttta 993
taataatgtt gtccctgctt gaatgacatt ttattgtcca ttgtactcga gtatcaacca 1053
ataaactttg tagccatgtt tagtaaaaaa aaaaaaaaa 1092

<210> 208
<211> 193
<212> PRT

<213> Xenopus laevis

<400> 208
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Ser Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Lys His Phe Cys Pro Asn Val Pro Ile
            100                 105                 110
Ile Leu Val Gly Asn Lys Lys Asp Leu Arg Asn Asp Glu His Thr Arg
        115                 120                 125
Arg Glu Leu Ala Lys Met Lys Gln Glu Pro Val Arg Ala Glu Glu Gly
    130                 135                 140
Arg Asp Met Ala Asn Arg Ile Ser Ala Phe Gly Tyr Leu Glu Cys Ser
145                 150                 155                 160
Ala Lys Thr Lys Asp Gly Val Arg Glu Val Phe Glu Met Ala Thr Arg
                165                 170                 175
Ala Ala Leu Gln Ala Lys Arg Gly Arg Lys Arg Ser Thr Cys Gln Leu
            180                 185                 190
Leu


<210> 209
<211> 582
<212> DNA
<213> Brachydanio rerio

<220>
<221> CDS
<222> (1)..(582)

<400> 209
atg gca gca att cgc aag aag ctg gtg att gta gga gat gga gca tgc      48
Met Ala Ala Ile Arg Lys Lys Leu Val Ile Val Gly Asp Gly Ala Cys
1               5                   10                  15
ggg aaa aca tgt ttg ctt att gtg ttc agt aaa gac cag ttt cct gaa      96
Gly Lys Thr Cys Leu Leu Ile Val Phe Ser Lys Asp Gln Phe Pro Glu
            20                  25                  30
gtc tac gta cca aca gtg ttt gaa aac tat gtt gcc gac att gaa gtt      144
Val Tyr Val Pro Thr Val Phe Glu Asn Tyr Val Ala Asp Ile Glu Val
        35                  40                  45
gac agc aaa cag gtg gaa ctc gct ctg tgg gat act gca ggg cag gag      192
Asp Ser Lys Gln Val Glu Leu Ala Leu Trp Asp Thr Ala Gly Gln Glu
    50                  55                  60
gac tac gac agg ctt cgt cct ctt tca tac cca gac acc gat gtc att      240
Asp Tyr Asp Arg Leu Arg Pro Leu Ser Tyr Pro Asp Thr Asp Val Ile
65                  70                  75                  80
ctt atg tgt ttc tct att gac agt cct gac agt tta gag aat att ccg      288
Leu Met Cys Phe Ser Ile Asp Ser Pro Asp Ser Leu Glu Asn Ile Pro
                85                  90                  95
gag aag tgg acg cct gag gtc aaa cac ttt tgt cca aac gct cca atc      336
Glu Lys Trp Thr Pro Glu Val Lys His Phe Cys Pro Asn Ala Pro Ile
            100                 105                 110
atc ctt gtg gga aac aaa aaa gac ctg cgc aat gat gaa cac aca cgc      384
Ile Leu Val Gly Asn Lys Lys Asp Leu Arg Asn Asp Glu His Thr Arg
        115                 120                 125
agg gaa ctg acc aag atg aag cag gag cca gtt aaa gca gag gaa ggg      432
Arg Glu Leu Thr Lys Met Lys Gln Glu Pro Val Lys Ala Glu Glu Gly
    130                 135                 140
aga gac atg gcc aat cga att gga gca ttt ggt tat atg gaa tgt tct      480

Arg Asp Met Ala Asn Arg Ile Gly Ala Phe Gly Tyr Met Glu Cys Ser
145                 150                 155                 160
gcc aaa aca aaa gac ggt gtt cgg gag gtg ttt gaa atg gcc act aga       528
Ala Lys Thr Lys Asp Gly Val Arg Glu Val Phe Glu Met Ala Thr Arg
                165                 170                 175
gcg gcg ctg cag gca cgc agg gga aag aag agc aac aaa tgc tgt ctg       576
Ala Ala Leu Gln Ala Arg Arg Gly Lys Lys Ser Asn Lys Cys Cys Leu
            180                 185                 190
ctg tga                                                               582
Leu

<210> 210
<211> 193
<212> PRT
<213> Brachydanio rerio

<400> 210
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Ser Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Lys His Phe Cys Pro Asn Ala Pro Ile
            100                 105                 110
Ile Leu Val Gly Asn Lys Lys Asp Leu Arg Asn Asp Glu His Thr Arg
        115                 120                 125
Arg Glu Leu Thr Lys Met Lys Gln Glu Pro Val Lys Ala Glu Glu Gly
    130                 135                 140
Arg Asp Met Ala Asn Arg Ile Gly Ala Phe Gly Tyr Met Glu Cys Ser
145                 150                 155                 160
Ala Lys Thr Lys Asp Gly Val Arg Glu Val Phe Glu Met Ala Thr Arg
                165                 170                 175
Ala Ala Leu Gln Ala Arg Arg Gly Lys Lys Ser Asn Lys Cys Cys Leu
            180                 185                 190
Leu

<210> 211
<211> 576
<212> DNA
<213> Brachydanio rerio

<220>
<221> CDS
<222> (1)..(576)

<400> 211
atg cag agc atc aag tgt gtg gtg gtg gga gac gga gcg gtg gga aaa       48
Met Gln Ser Ile Lys Cys Val Val Val Gly Asp Gly Ala Val Gly Lys
1               5                   10                  15
acc tgc ctg ctc atc tcc tac acg acc ggc gcc ttc ccc aaa gag tac       96
Thr Cys Leu Leu Ile Ser Tyr Thr Thr Gly Ala Phe Pro Lys Glu Tyr
            20                  25                  30
att ccc acc gtc ttc gac aac tac agc tcc cag gtc agc gtg gac aac       144
Ile Pro Thr Val Phe Asp Asn Tyr Ser Ser Gln Val Ser Val Asp Asn
        35                  40                  45
cgt acc gtc agc ctc aat ctg tgg gac acc gcg ggt cag gag gag tac       192
Arg Thr Val Ser Leu Asn Leu Trp Asp Thr Ala Gly Gln Glu Glu Tyr
    50                  55                  60

gac cgc ctt cgc acg ctg tcc tat cct caa acc aac gtc ttc atc atc 240
Asp Arg Leu Arg Thr Leu Ser Tyr Pro Gln Thr Asn Val Phe Ile Ile
65                  70                  75                  80
tgc ttc tcc atc tcc agc cct ccg tcc tac gag aac atc aag cat aag 288
Cys Phe Ser Ile Ser Ser Pro Pro Ser Tyr Glu Asn Ile Lys His Lys
                85                  90                  95
tgg cac ccg gag gtg aca cac cac tgt cct agc gtg ccc atc ctg ctt 336
Trp His Pro Glu Val Thr His His Cys Pro Ser Val Pro Ile Leu Leu
            100                 105                 110
gtg gga acc aag agt gat ctc cgc aat gac gcc gac gtg ctg aag aag 384
Val Gly Thr Lys Ser Asp Leu Arg Asn Asp Ala Asp Val Leu Lys Lys
        115                 120                 125
ctg aag gag cag aac cag gcg ccc atc acg acc cag cag ggt caa gcg 432
Leu Lys Glu Gln Asn Gln Ala Pro Ile Thr Thr Gln Gln Gly Gln Ala
    130                 135                 140
ctg gct cgc caa atc cac gcc gtc aag tac cgt gag tgt tcg gca ctc 480
Leu Ala Arg Gln Ile His Ala Val Lys Tyr Arg Glu Cys Ser Ala Leu
145                 150                 155                 160
agt cag gac ggc atc aag gac gtg ttt gca gac gca gtg cgt gcc tac 528
Ser Gln Asp Gly Ile Lys Asp Val Phe Ala Asp Ala Val Arg Ala Tyr
                165                 170                 175
ctt agt cct caa ccc gtg gct aac aag aag ccc tgt ata ctc ctc 573
Leu Ser Pro Gln Pro Val Ala Asn Lys Lys Pro Cys Ile Leu Leu
            180                 185                 190
tga 576

<210> 212
<211> 191
<212> PRT
<213> Brachydanio rerio

<400> 212
Met Gln Ser Ile Lys Cys Val Val Val Gly Asp Gly Ala Val Gly Lys
1               5                   10                  15
Thr Cys Leu Leu Ile Ser Tyr Thr Thr Gly Ala Phe Pro Lys Glu Tyr
            20                  25                  30
Ile Pro Thr Val Phe Asp Asn Tyr Ser Ser Gln Val Ser Val Asp Asn
        35                  40                  45
Arg Thr Val Ser Leu Asn Leu Trp Asp Thr Ala Gly Gln Glu Glu Tyr
    50                  55                  60
Asp Arg Leu Arg Thr Leu Ser Tyr Pro Gln Thr Asn Val Phe Ile Ile
65                  70                  75                  80
Cys Phe Ser Ile Ser Ser Pro Pro Ser Tyr Glu Asn Ile Lys His Lys
                85                  90                  95
Trp His Pro Glu Val Thr His His Cys Pro Ser Val Pro Ile Leu Leu
            100                 105                 110
Val Gly Thr Lys Ser Asp Leu Arg Asn Asp Ala Asp Val Leu Lys Lys
        115                 120                 125
Leu Lys Glu Gln Asn Gln Ala Pro Ile Thr Thr Gln Gln Gly Gln Ala
    130                 135                 140
Leu Ala Arg Gln Ile His Ala Val Lys Tyr Arg Glu Cys Ser Ala Leu
145                 150                 155                 160
Ser Gln Asp Gly Ile Lys Asp Val Phe Ala Asp Ala Val Arg Ala Tyr
                165                 170                 175
Leu Ser Pro Gln Pro Val Ala Asn Lys Lys Pro Cys Ile Leu Leu
            180                 185                 190

<210> 213
<211> 576
<212> DNA
<213> Brachydanio rerio

<220>
<221> CDS
<222> (1)..(576)

<400> 213
atg cag acc ata aag tgt gtt gtt gtt ggt gat ggt gca gtg gga aag      48
Met Gln Thr Ile Lys Cys Val Val Val Gly Asp Gly Ala Val Gly Lys
1               5                   10                  15
acc tgt ctg ttg atc tcc tac acc acc aat gcc ttc cca gac gag tac      96
Thr Cys Leu Leu Ile Ser Tyr Thr Thr Asn Ala Phe Pro Asp Glu Tyr
            20                  25                  30
att cct aca gtg ttc gac aac tac agc act caa acc tgt gtg gac ggc      144
Ile Pro Thr Val Phe Asp Asn Tyr Ser Thr Gln Thr Cys Val Asp Gly
        35                  40                  45
cgt gct gtc agc ctc aac ctg tgg gac acc gcc ggt cag gag gag tac      192
Arg Ala Val Ser Leu Asn Leu Trp Asp Thr Ala Gly Gln Glu Glu Tyr
    50                  55                  60
gac cgc ctg cga acc ctc tcc tac ccg cag aca cac gtc ttc atc atc      240
Asp Arg Leu Arg Thr Leu Ser Tyr Pro Gln Thr His Val Phe Ile Ile
65                  70                  75                  80
tgt ttc tct gtg gct agt cct tcc tct cac gcc aac gtc agg cat aaa      288
Cys Phe Ser Val Ala Ser Pro Ser Ser His Ala Asn Val Arg His Lys
                85                  90                  95
tgg cac ccg gag gtc tgc cac cac tgt cct ggc gta cct gtg ctg ctg      336
Trp His Pro Glu Val Cys His His Cys Pro Gly Val Pro Val Leu Leu
            100                 105                 110
gtg ggc acc aag aga gac ctg cga ggg gac aag gag acc ctg gag aag      384
Val Gly Thr Lys Arg Asp Leu Arg Gly Asp Lys Glu Thr Leu Glu Lys
        115                 120                 125
ctg aag gag cag ggg atg agt ccg acc act cca cag caa ggc agt gca      432
Leu Lys Glu Gln Gly Met Ser Pro Thr Thr Pro Gln Gln Gly Ser Ala
    130                 135                 140
ctg gcc cgc agc atc ggg gcg gtg cga tat ctg gag tgc tcg gcc ctt      480
Leu Ala Arg Ser Ile Gly Ala Val Arg Tyr Leu Glu Cys Ser Ala Leu
145                 150                 155                 160
ctc cag gag ggc gtc cga gag gtc ttc aac gaa gca gtt agg gct gtt      528
Leu Gln Glu Gly Val Arg Glu Val Phe Asn Glu Ala Val Arg Ala Val
                165                 170                 175
ctc tac ccc aac gcc aag aag cac acc aaa aaa tgt gtg ctg ctt      573
Leu Tyr Pro Asn Ala Lys Lys His Thr Lys Lys Cys Val Leu Leu
            180                 185                 190
taa                                                              576

<210> 214
<211> 191
<212> PRT
<213> Brachydanio rerio

<400> 214
Met Gln Thr Ile Lys Cys Val Val Val Gly Asp Gly Ala Val Gly Lys
1               5                   10                  15
Thr Cys Leu Leu Ile Ser Tyr Thr Thr Asn Ala Phe Pro Asp Glu Tyr
            20                  25                  30
Ile Pro Thr Val Phe Asp Asn Tyr Ser Thr Gln Thr Cys Val Asp Gly
        35                  40                  45
Arg Ala Val Ser Leu Asn Leu Trp Asp Thr Ala Gly Gln Glu Glu Tyr
    50                  55                  60
Asp Arg Leu Arg Thr Leu Ser Tyr Pro Gln Thr His Val Phe Ile Ile
65                  70                  75                  80
Cys Phe Ser Val Ala Ser Pro Ser Ser His Ala Asn Val Arg His Lys
                85                  90                  95
Trp His Pro Glu Val Cys His His Cys Pro Gly Val Pro Val Leu Leu
            100                 105                 110
Val Gly Thr Lys Arg Asp Leu Arg Gly Asp Lys Glu Thr Leu Glu Lys
        115                 120                 125
Leu Lys Glu Gln Gly Met Ser Pro Thr Thr Pro Gln Gln Gly Ser Ala
    130                 135                 140
Leu Ala Arg Ser Ile Gly Ala Val Arg Tyr Leu Glu Cys Ser Ala Leu

145                 150                 155                 160
Leu Gln Glu Gly Val Arg Glu Val Phe Asn Glu Ala Val Arg Ala Val
                165                 170                 175
Leu Tyr Pro Asn Ala Lys Lys His Thr Lys Lys Cys Val Leu Leu
            180                 185                 190

<210> 215
<211> 1124
<212> DNA
<213> Rattus norvegicus

<220>
<221> CDS
<222> (100)..(684)

<400> 215
aggtggtggc tcaggatagg tgtcctctgc actaataggc ctacctgggt gtagtctgac      60

agcctgtggt ctgagaagtc ccaggtgctt tgtacagca atg gct gcc atc cgg      114
                                           Met Ala Ala Ile Arg
                                           1               5
aag aaa ctg gtg att gtg gga gat gga gct tgt gga aaa aca tgc ttg      162
Lys Lys Leu Val Ile Val Gly Asp Gly Ala Cys Gly Lys Thr Cys Leu
                10                  15                  20
ctc atc gtc ttc agc aag gac cag ttt cct gat gtt tat gtg ccc aca      210
Leu Ile Val Phe Ser Lys Asp Gln Phe Pro Asp Val Tyr Val Pro Thr
            25                  30                  35
gtg ttt gag aac tat gtg gct gat atc gaa gtg gat gga aaa cag gtg      258
Val Phe Glu Asn Tyr Val Ala Asp Ile Glu Val Asp Gly Lys Gln Val
        40                  45                  50
gag ttg gcc ctc tgg gac aca gct ggt caa gaa gat tat gat cgc ttg      306
Glu Leu Ala Leu Trp Asp Thr Ala Gly Gln Glu Asp Tyr Asp Arg Leu
    55                  60                  65
agg cca ctc tcc tat ccg gac act gat gtc ctc ttg ata tgt ttc tcc      354
Arg Pro Leu Ser Tyr Pro Asp Thr Asp Val Leu Leu Ile Cys Phe Ser
70                  75                  80                  85
att ggc aac cct gat agc ttt ggg aac atc cca cac aaa tgg ata cca      402
Ile Gly Asn Pro Asp Ser Phe Gly Asn Ile Pro His Lys Trp Ile Pro
                90                  95                  100
gaa gta aag cat ttc tgt ccc aac gtg ccc atc atc ctg gtc ggg agc      450
Glu Val Lys His Phe Cys Pro Asn Val Pro Ile Ile Leu Val Gly Ser
            105                 110                 115
aag aag gat ctt cgg aat gac ttg aac aca ata caa gag tta gcc aag      498
Lys Lys Asp Leu Arg Asn Asp Leu Asn Thr Ile Gln Glu Leu Ala Lys
        120                 125                 130
agg aaa caa gag ccg gtg aaa cct gaa caa ggc cga gat ttg gcg aac      546
Arg Lys Gln Glu Pro Val Lys Pro Glu Gln Gly Arg Asp Leu Ala Asn
    135                 140                 145
agc att ggc gct ttc gag tat gtg gag tgt tct gca aag acc aaa gat      594
Ser Ile Gly Ala Phe Glu Tyr Val Glu Cys Ser Ala Lys Thr Lys Asp
150                 155                 160                 165
gga gtg aga aag gtc ttt gaa aaa agc cac caa ggc ttg ctc ttg caa      642
Gly Val Arg Lys Val Phe Glu Lys Ser His Gln Gly Leu Leu Leu Gln
                170                 175                 180
acg aac cgt gtg aag aaa aag act ggc tgc ttt gtc ttt tgaagtcttt      691
Thr Asn Arg Val Lys Lys Lys Thr Gly Cys Phe Val Phe
            185                 190
gtgcaaacac agccctagta cagttaattt tcaagtgcta tttacgaatg ttattgtgta      751

attgctagcc tttttgttta tttatatatc caagattaca ggtcagaagt catcttgcta      811

ctaatgtttg gaaatcaact gtgattatta atgacattca ttctgtctga ccttccaggg      871

tcctgactct tctgtaacag accattctgc atttcacctg aaacccaagg cactaattga      931

agaacttatt atctgtagaa agagagatgg taacttcatc atttagactg taactacttt 991

ctgaccaccc taccatccat atatgagctg agaagaaagc tgttgagtca ccacttgagt 1051

gtttgctccc taacaacttt aatcaccatc cttattctct tcgaaatgtc tctccaggca 1111

gctgtggtag agt 1124

<210> 216
<211> 194
<212> PRT
<213> Rattus norvegicus

<400> 216
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Asp
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Leu
65                  70                  75                  80
Leu Ile Cys Phe Ser Ile Gly Asn Pro Asp Ser Phe Gly Asn Ile Pro
                85                  90                  95
His Lys Trp Ile Pro Glu Val Lys His Phe Cys Pro Asn Val Pro Ile
            100                 105                 110
Ile Leu Val Gly Ser Lys Lys Asp Leu Arg Asn Asp Leu Asn Thr Ile
        115                 120                 125
Gln Glu Leu Ala Lys Arg Lys Gln Glu Pro Val Lys Pro Glu Gln Gly
    130                 135                 140
Arg Asp Leu Ala Asn Ser Ile Gly Ala Phe Glu Tyr Val Glu Cys Ser
145                 150                 155                 160
Ala Lys Thr Lys Asp Gly Val Arg Lys Val Phe Glu Lys Ser His Gln
                165                 170                 175
Gly Leu Leu Leu Gln Thr Asn Arg Val Lys Lys Lys Thr Gly Cys Phe
            180                 185                 190
Val Phe

<210> 217
<211> 576
<212> DNA
<213> Aplysia californica

<220>
<221> CDS
<222> (1)..(576)

<400> 217
atg caa acc ata aag tgt gtg gtt gtg ggg gat ggg gca gtc gga aaa 48
Met Gln Thr Ile Lys Cys Val Val Val Gly Asp Gly Ala Val Gly Lys
1               5                   10                  15
acc tgc ctt ctc att tca tac acc aca aat aaa ttt cct tca gaa tat 96
Thr Cys Leu Leu Ile Ser Tyr Thr Thr Asn Lys Phe Pro Ser Glu Tyr
            20                  25                  30
gtg cca aca gtg ttt gac aac tat gcg gtg acc gtg atg atc ggg gga 144
Val Pro Thr Val Phe Asp Asn Tyr Ala Val Thr Val Met Ile Gly Gly
        35                  40                  45

gag ccc tac acc ctg ggc ctg ttt gat acc gcg ggt cag gag gac tac 192
Glu Pro Tyr Thr Leu Gly Leu Phe Asp Thr Ala Gly Gln Glu Asp Tyr
    50                  55                  60
gac agg cta cgt cca ctc agc tac cct cag acg gac gtc ttc ctc gtc 240
Asp Arg Leu Arg Pro Leu Ser Tyr Pro Gln Thr Asp Val Phe Leu Val
65                  70                  75                  80
tgc ttc tcc gtg gtc acc ccg tcc tcc ttc gag aat gtc agg gaa aag 288
Cys Phe Ser Val Val Thr Pro Ser Ser Phe Glu Asn Val Arg Glu Lys
                85                  90                  95
tgg gtt ccg gag atc aca cat cac tgt cag cgc acg ccg ttc ctg ctg 336
Trp Val Pro Glu Ile Thr His His Cys Gln Arg Thr Pro Phe Leu Leu
            100                 105                 110
gtg ggg aca cag att gat ctg cgc gac gac acg ccc acg gtg gag aag 384
Val Gly Thr Gln Ile Asp Leu Arg Asp Asp Thr Pro Thr Val Glu Lys
        115                 120                 125
ctg gcc aag aac aaa cag aag ccc atc acc gcc gac cac ggg gag cgt 432
Leu Ala Lys Asn Lys Gln Lys Pro Ile Thr Ala Asp His Gly Glu Arg
    130                 135                 140
ctg gcc cgc gag ctc agg gcg gtc aag tac gtc gag tgc tcc gcc ctc 480
Leu Ala Arg Glu Leu Arg Ala Val Lys Tyr Val Glu Cys Ser Ala Leu
145                 150                 155                 160
aac cag aga ggt ctg aag aac gtg ttt gat gag gct atc ctg gca gcg 528
Asn Gln Arg Gly Leu Lys Asn Val Phe Asp Glu Ala Ile Leu Ala Ala
                165                 170                 175
cta gaa ccc cca gag cca ccc aag aaa aag aaa tgt gtg ctc ttg 573
Leu Glu Pro Pro Glu Pro Pro Lys Lys Lys Lys Cys Val Leu Leu
            180                 185                 190
taa 576

<210> 218
<211> 191
<212> PRT
<213> Aplysia californica

<400> 218
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Glu Tyr
            20                  25                  30
Val Pro Thr Val Phe Asp Asn Tyr Ala Val Thr Val Met Ile Gly Gly
        35                  40                  45
Glu Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Val Thr Pro Ser Ser Phe Glu Asn Val Arg Glu Lys
                85                  90                  95
Trp Val Pro Glu Ile Thr His His Cys Gln Arg Thr Pro Phe Leu Leu
            100                 105                 110
Val Gly Thr Gln Ile Asp Leu Arg Asp Asp Thr Pro Thr Val Glu Lys
        115                 120                 125
Leu Ala Lys Asn Lys Gln Lys Pro Ile Thr Ala Asp His Gly Glu Arg
    130                 135                 140
Leu Ala Arg Glu Leu Arg Ala Val Lys Tyr Val Glu Cys Ser Ala Leu
145                 150                 155                 160
Asn Gln Arg Gly Leu Lys Asn Val Phe Asp Glu Ala Ile Leu Ala Ala
                165                 170                 175
Leu Glu Pro Pro Glu Pro Pro Lys Lys Lys Lys Cys Val Leu Leu
            180                 185                 190

<210> 219
<211> 579
<212> DNA
<213> Aplysia californica

<220>
<221> CDS
<222> (1)..(579)

<400> 219
atg cag gct atc aag tgt gtg gta gtc ggg gac ggt gct gtg ggt aag 48
Met Gln Ala Ile Lys Cys Val Val Val Gly Asp Gly Ala Val Gly Lys
1               5                   10                  15
aca tgt ctt ctt atc agc tac act acc aat gcc ttc ccc gga gaa tac 96
Thr Cys Leu Leu Ile Ser Tyr Thr Thr Asn Ala Phe Pro Gly Glu Tyr
            20                  25                  30
att cca act gtc ttt gac aac tac tct gca aat gtt atg gta gat ggc 144
Ile Pro Thr Val Phe Asp Asn Tyr Ser Ala Asn Val Met Val Asp Gly
        35                  40                  45
aag cca atc aat ctt gga cta tgg gat act gct gga caa gag gac tat 192
Lys Pro Ile Asn Leu Gly Leu Trp Asp Thr Ala Gly Gln Glu Asp Tyr
    50                  55                  60
gat cgt ctc aga ccc ttg tca tat ccg caa act gat gtc ttt ctt ata 240
Asp Arg Leu Arg Pro Leu Ser Tyr Pro Gln Thr Asp Val Phe Leu Ile
65                  70                  75                  80
tgt ttt tcc ttg atc agt cct aca agt ttc gag aat gtt cga gca aag 288
Cys Phe Ser Leu Ile Ser Pro Thr Ser Phe Glu Asn Val Arg Ala Lys
                85                  90                  95
tgg ttt cct gaa gtg agc cat cat tgc cct cat acc cct atc atc tta 336
Trp Phe Pro Glu Val Ser His His Cys Pro His Thr Pro Ile Ile Leu
            100                 105                 110
gtg ggt acc aag ctt gat ctg cgt gaa gac aag gag aca att gag aaa 384
Val Gly Thr Lys Leu Asp Leu Arg Glu Asp Lys Glu Thr Ile Glu Lys
        115                 120                 125
ctg aga gac aag aag ctg tcc cct atc acc tac cct caa ggg ctg gcc 432
Leu Arg Asp Lys Lys Leu Ser Pro Ile Thr Tyr Pro Gln Gly Leu Ala
    130                 135                 140
atg gca agg gag ata agt gct gtg aag tat ctt gag tgc tca gcc tta 480
Met Ala Arg Glu Ile Ser Ala Val Lys Tyr Leu Glu Cys Ser Ala Leu
145                 150                 155                 160
acc caa aaa ggg ctg aag aat gtc ttt gat gag gct att cgg gct gtt 528
Thr Gln Lys Gly Leu Lys Asn Val Phe Asp Glu Ala Ile Arg Ala Val
                165                 170                 175
ttg tgc cct aag cct aag cca aag aag aag aaa gga tgc gaa att ttg 576
Leu Cys Pro Lys Pro Lys Pro Lys Lys Lys Lys Gly Cys Glu Ile Leu
            180                 185                 190
tga 579

<210> 220
<211> 192
<212> PRT
<213> Aplysia californica

<400> 220
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly
        35                  40                  45
Lys Pro Ile Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Ile Ser Pro Thr Ser Phe Glu Asn Val Arg Ala Lys
                85                  90                  95
Trp Phe Pro Glu Val Ser His His Cys Pro His Thr Pro Ile Ile Leu
            100                 105                 110
Val Gly Thr Lys Leu Asp Leu Arg Glu Asp Lys Glu Thr Ile Glu Lys
        115                 120                 125

Leu Arg Asp Lys Lys Leu Ser Pro Ile Thr Tyr Pro Gln Gly Leu Ala
    130                 135                 140
Met Ala Arg Glu Ile Ser Ala Val Lys Tyr Leu Glu Cys Ser Ala Leu
145                 150                 155                 160
Thr Gln Lys Gly Leu Lys Asn Val Phe Asp Glu Ala Ile Arg Ala Val
                165                 170                 175
Leu Cys Pro Lys Pro Lys Pro Lys Lys Lys Lys Gly Cys Glu Ile Leu
            180                 185                 190

<210> 221
<211> 558
<212> DNA
<213> Schistosoma japonicum

<220>
<221> CDS
<222> (1)..(558)

<400> 221
atg caa gcc atc aaa tgt gta gtt atc ggt gat ggt gct gta gga aaa      48
Met Gln Ala Ile Lys Cys Val Val Ile Gly Asp Gly Ala Val Gly Lys
1               5                   10                  15
aca tgt ttg ctc att agc tac act aca aat gct ttt cct ggt gag tat      96
Thr Cys Leu Leu Ile Ser Tyr Thr Thr Asn Ala Phe Pro Gly Glu Tyr
            20                  25                  30
gtc cca act gtc ttc gat aac tac tct gcc aat gta atg gtt ggt gaa      144
Val Pro Thr Val Phe Asp Asn Tyr Ser Ala Asn Val Met Val Gly Glu
        35                  40                  45
aaa cgt gtg aac ctt ggt ttg tgg gat aca gct ggt caa gaa gat tat      192
Lys Arg Val Asn Leu Gly Leu Trp Asp Thr Ala Gly Gln Glu Asp Tyr
    50                  55                  60
gat aga tta cgg cca cta tcg tat cca cag act gac gtt ttc ttg gtt      240
Asp Arg Leu Arg Pro Leu Ser Tyr Pro Gln Thr Asp Val Phe Leu Val
65                  70                  75                  80
tgc ttt tct ttg atc agt cct tca tca ttt gat aac gtt cgt gct aaa      288
Cys Phe Ser Leu Ile Ser Pro Ser Ser Phe Asp Asn Val Arg Ala Lys
                85                  90                  95
tgg tat cca gaa ata cgc cac ttt tct cca aac aca ccc att att ctt      336
Trp Tyr Pro Glu Ile Arg His Phe Ser Pro Asn Thr Pro Ile Ile Leu
            100                 105                 110
gtt gga aca aaa ctg gat ctc cgc aac agt tct acc agt cct aaa aac      384
Val Gly Thr Lys Leu Asp Leu Arg Asn Ser Ser Thr Ser Pro Lys Asn
        115                 120                 125
aac caa cca tca ata tct tat gaa caa ggt tta att atg gca aga gag      432
Asn Gln Pro Ser Ile Ser Tyr Glu Gln Gly Leu Ile Met Ala Arg Glu
    130                 135                 140
att gga gct cat aag tat cta gaa tgt tca gcg tta act cag gat gga      480
Ile Gly Ala His Lys Tyr Leu Glu Cys Ser Ala Leu Thr Gln Asp Gly
145                 150                 155                 160
tta aca gga tgt ttt cga tgc agc tat ccg ggc agt act cat gcc tcc      528
Leu Thr Gly Cys Phe Arg Cys Ser Tyr Pro Gly Ser Thr His Ala Ser
                165                 170                 175
agc tcg gaa aaa aaa aca tac ctt atg tga                              558
Ser Ser Glu Lys Lys Thr Tyr Leu Met
            180                 185


<210> 222
<211> 185
<212> PRT
<213> Schistosoma japonicum

<400> 222
Met Gln Ala Ile Lys Cys Val Val Ile Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Val Pro Thr Val Phe Asp Asn Tyr Ser Ala Asn Val Met Val Gly Glu

                35                  40                  45
Lys Arg Val Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Leu Ile Ser Pro Ser Ser Phe Asp Asn Val Arg Ala Lys
                85                  90                  95
Trp Tyr Pro Glu Ile Arg His Phe Ser Pro Asn Thr Pro Ile Ile Leu
            100                 105                 110
Val Gly Thr Lys Leu Asp Leu Arg Asn Ser Ser Thr Ser Pro Lys Asn
        115                 120                 125
Asn Gln Pro Ser Ile Ser Tyr Glu Gln Gly Leu Ile Met Ala Arg Glu
    130                 135                 140
Ile Gly Ala His Lys Tyr Leu Glu Cys Ser Ala Leu Thr Gln Asp Gly
145                 150                 155                 160
Leu Thr Gly Cys Phe Arg Cys Ser Tyr Pro Gly Ser Thr His Ala Ser
                165                 170                 175
Ser Ser Glu Lys Lys Thr Tyr Leu Met
            180                 185

<210> 223
<211> 576
<212> DNA
<213> Blumeria graminis

<220>
<221> CDS
<222> (1)..(576)

<400> 223
atg tct ctt aga tgc gtt gtt gtt ggc gat ggt gcc gtt gga aaa act      48
Met Ser Leu Arg Cys Val Val Val Gly Asp Gly Ala Val Gly Lys Thr
1               5                   10                  15
tgt ctt ctc atc agc tat aca aca aat aaa ttc ccc tcc gaa tat gtc      96
Cys Leu Leu Ile Ser Tyr Thr Thr Asn Lys Phe Pro Ser Glu Tyr Val
            20                  25                  30
cct act gtc ttc gat aat tat gct gtt act gtt atg att ggc gat gag      144
Pro Thr Val Phe Asp Asn Tyr Ala Val Thr Val Met Ile Gly Asp Glu
        35                  40                  45
cca tat acc ctt ggc cta ttc gac act gct ggt caa gaa gat tac gat      192
Pro Tyr Thr Leu Gly Leu Phe Asp Thr Ala Gly Gln Glu Asp Tyr Asp
    50                  55                  60
cgc tta cgg ccc ctt tca tac cct caa act gat gta ttt ctc gtc tgt      240
Arg Leu Arg Pro Leu Ser Tyr Pro Gln Thr Asp Val Phe Leu Val Cys
65                  70                  75                  80
ttc tct gtt act tca ccc gca tca ttt gaa aat gtt cgc gag aaa tgg      288
Phe Ser Val Thr Ser Pro Ala Ser Phe Glu Asn Val Arg Glu Lys Trp
                85                  90                  95
ttt cct gaa gtc cat cat cac tgc cct gga gtc cct tgc tta att gtt      336
Phe Pro Glu Val His His His Cys Pro Gly Val Pro Cys Leu Ile Val
            100                 105                 110
ggc acc caa aca gat ctg aga gat gat ctt agc gtt cga gaa aag tta      384
Gly Thr Gln Thr Asp Leu Arg Asp Asp Leu Ser Val Arg Glu Lys Leu
        115                 120                 125
aac aaa cag aaa atg caa cca gtt aag aga gaa gat gga gag aga atg      432
Asn Lys Gln Lys Met Gln Pro Val Lys Arg Glu Asp Gly Glu Arg Met
    130                 135                 140
gct aag gat ttg ggt gca gtt aga tat gtg gaa tgc agc gcg ttg aca      480
Ala Lys Asp Leu Gly Ala Val Arg Tyr Val Glu Cys Ser Ala Leu Thr
145                 150                 155                 160
cag tat aag ttg aag gat gta ttc gat gag gct ata gtg gct gca cta      528
Gln Tyr Lys Leu Lys Asp Val Phe Asp Glu Ala Ile Val Ala Ala Leu
                165                 170                 175
gaa cct ccc gct cca aaa aag aag cat cga aac tgt cta atc cta      573
Glu Pro Pro Ala Pro Lys Lys Lys His Arg Asn Cys Leu Ile Leu
            180                 185                 190
tga                                                              576

<210> 224
<211> 191
<212> PRT
<213> Blumeria graminis

<400> 224
Met Ser Leu Arg Cys Val Val Val Gly Asp Gly Ala Val Gly Lys Thr
1               5                   10                  15
Cys Leu Leu Ile Ser Tyr Thr Thr Asn Lys Phe Pro Ser Glu Tyr Val
            20                  25                  30
Pro Thr Val Phe Asp Asn Tyr Ala Val Thr Val Met Ile Gly Asp Glu
        35                  40                  45
Pro Tyr Thr Leu Gly Leu Phe Asp Thr Ala Gly Gln Glu Asp Tyr Asp
    50                  55                  60
Arg Leu Arg Pro Leu Ser Tyr Pro Gln Thr Asp Val Phe Leu Val Cys
65                  70                  75                  80
Phe Ser Val Thr Ser Pro Ala Ser Phe Glu Asn Val Arg Glu Lys Trp
                85                  90                  95
Phe Pro Glu Val His His His Cys Pro Gly Val Pro Cys Leu Ile Val
            100                 105                 110
Gly Thr Gln Thr Asp Leu Arg Asp Asp Leu Ser Val Arg Glu Lys Leu
        115                 120                 125
Asn Lys Gln Lys Met Gln Pro Val Lys Arg Glu Asp Gly Glu Arg Met
    130                 135                 140
Ala Lys Asp Leu Gly Ala Val Arg Tyr Val Glu Cys Ser Ala Leu Thr
145                 150                 155                 160
Gln Tyr Lys Leu Lys Asp Val Phe Asp Glu Ala Ile Val Ala Ala Leu
                165                 170                 175
Glu Pro Pro Ala Pro Lys Lys Lys His Arg Asn Cys Leu Ile Leu
            180                 185                 190

<210> 225
<211> 757
<212> DNA
<213> Brassica napus

<220>
<221> CDS
<222> (164)..(757)

<400> 225
cgatacaccg attcatttca ttcttcattg atcgcttcct ctcacatacc cgatttcgtt      60

tagggttaac attttctagg gttttagaga taggcgaaac tgaaattgag aagaagacgg     120

gtgttgtttc ctcatagatc tgaggggttg aatttttcga ttt atg agc gca tct       175
                                                Met Ser Ala Ser
                                                1
cgg ttc ata aag tgc gtg acg gtt ggg gac gga gca gtg ggc aaa aca       223
Arg Phe Ile Lys Cys Val Thr Val Gly Asp Gly Ala Val Gly Lys Thr
5                   10                  15                  20
tgt ctc ctc atc tct tac acc agc aac act ttc cct acg gat tat gtt       271
Cys Leu Leu Ile Ser Tyr Thr Ser Asn Thr Phe Pro Thr Asp Tyr Val
                25                  30                  35
cca act gtt ttc gat aac ttc agc gct aat gtt gtt gtt aac gga gcc       319
Pro Thr Val Phe Asp Asn Phe Ser Ala Asn Val Val Val Asn Gly Ala
            40                  45                  50
act gtc aac tta gga ctc tgg gat acc gca ggg cag gag gat tat aac       367
Thr Val Asn Leu Gly Leu Trp Asp Thr Ala Gly Gln Glu Asp Tyr Asn
        55                  60                  65
aga ttg aga ccc ttg agt tac cgc ggt gct gac gtt ttc atc ttg gcc       415
Arg Leu Arg Pro Leu Ser Tyr Arg Gly Ala Asp Val Phe Ile Leu Ala
    70                  75                  80

ttc tct ctc atc agt aag gct agt tat gag aat gtc tcc aag aag tgg 463
Phe Ser Leu Ile Ser Lys Ala Ser Tyr Glu Asn Val Ser Lys Lys Trp
85                  90                  95                  100
atc cct gag ctg acc cac tat gcc cct ggt gtc cct atc gtt ctt gtt 511
Ile Pro Glu Leu Thr His Tyr Ala Pro Gly Val Pro Ile Val Leu Val
                105                 110                 115
ggt acc aaa cta gat ctt agg gat gac aaa cag ttc ttc gtt gac cac 559
Gly Thr Lys Leu Asp Leu Arg Asp Asp Lys Gln Phe Phe Val Asp His
            120                 125                 130
cct ggt gct gta cct att acc act tct cag gga gag gaa cta atg aag 607
Pro Gly Ala Val Pro Ile Thr Thr Ser Gln Gly Glu Glu Leu Met Lys
        135                 140                 145
cta att gga gct cct tcg tac atc gag tgc agt tca aaa tct caa gag 655
Leu Ile Gly Ala Pro Ser Tyr Ile Glu Cys Ser Ser Lys Ser Gln Glu
    150                 155                 160
aac gtg aaa ggg gtg ttt gat gca gcg atc aga gtg gta ctt caa cct 703
Asn Val Lys Gly Val Phe Asp Ala Ala Ile Arg Val Val Leu Gln Pro
165                 170                 175                 180
cca aag cag aag aaa aag aag agc aag gca caa aag gcc tgc tcc att 751
Pro Lys Gln Lys Lys Lys Lys Ser Lys Ala Gln Lys Ala Cys Ser Ile
                185                 190                 195
ttg taa 757
Leu

<210> 226
<211> 197
<212> PRT
<213> Brassica napus

<400> 226
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ala Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Thr His Tyr Ala Pro Gly Val Pro
            100                 105                 110
Ile Val Leu Val Gly Thr Lys Leu Asp Leu Arg Asp Asp Lys Gln Phe
        115                 120                 125
Phe Val Asp His Pro Gly Ala Val Pro Ile Thr Thr Ser Gln Gly Glu
    130                 135                 140
Glu Leu Met Lys Leu Ile Gly Ala Pro Ser Tyr Ile Glu Cys Ser Ser
145                 150                 155                 160
Lys Ser Gln Glu Asn Val Lys Gly Val Phe Asp Ala Ala Ile Arg Val
                165                 170                 175
Val Leu Gln Pro Pro Lys Gln Lys Lys Lys Lys Ser Lys Ala Gln Lys
            180                 185                 190
Ala Cys Ser Ile Leu
        195

<210> 227
<211> 1015
<212> DNA
<213> Zinnia elegans

<220>
<221> CDS
<222> (170)..(763)

<400> 227
ggtttgttct tggggtttgg ctggaaaaag attcaatttt tcggtgtgtt tttgggaaaa      60

agatttggtg aaatttgtgg gtgttgaaga aagattgttg gggattattg ggtgtgagtg     120

atttggtggt gatcaggtct tctgggtgtg gaagaagatc tgaagaaat atg agt gca     178
                                                      Met Ser Ala
                                                      1
tca aga ttt ata aag tgt gtt act gtt ggt gat ggg gct gtt gga aag       226
Ser Arg Phe Ile Lys Cys Val Thr Val Gly Asp Gly Ala Val Gly Lys
    5                   10                  15
act tgt ttg ttg att tct tac acc agt aat acc ttc cct acg gat tac       274
Thr Cys Leu Leu Ile Ser Tyr Thr Ser Asn Thr Phe Pro Thr Asp Tyr
20                  25                  30                  35
gtc cca acg gta ttt gat aat ttc agt gct aat gta gtt gtc aat gga       322
Val Pro Thr Val Phe Asp Asn Phe Ser Ala Asn Val Val Val Asn Gly
                40                  45                  50
agc aca gtt aat cta ggc ttg tgg gac act gct gga cag gag gac tat       370
Ser Thr Val Asn Leu Gly Leu Trp Asp Thr Ala Gly Gln Glu Asp Tyr
            55                  60                  65
aac aga tta aga ccg ttg agt tat cgt gga gct gat gtt ttc att ttg       418
Asn Arg Leu Arg Pro Leu Ser Tyr Arg Gly Ala Asp Val Phe Ile Leu
        70                  75                  80
gcg ttc tca ctc att agc aag gct agc tat gag aac gtt tcc aag aag       466
Ala Phe Ser Leu Ile Ser Lys Ala Ser Tyr Glu Asn Val Ser Lys Lys
    85                  90                  95
tgg att cca gaa ttg aag cat tat gct cct ggt gtt cca atc gtt ctt       514
Trp Ile Pro Glu Leu Lys His Tyr Ala Pro Gly Val Pro Ile Val Leu
100                 105                 110                 115
gtt ggg acg aaa cta gat ctc cga gat gat aag cag ttt ttt gta gat       562
Val Gly Thr Lys Leu Asp Leu Arg Asp Asp Lys Gln Phe Phe Val Asp
                120                 125                 130
cac cct ggt gca act cct att acc act gca cag gga gag gaa tta aag       610
His Pro Gly Ala Thr Pro Ile Thr Thr Ala Gln Gly Glu Glu Leu Lys
            135                 140                 145
aag aca atc ggt gct cct gag tac att gaa tgt agt tca aaa aca caa       658
Lys Thr Ile Gly Ala Pro Glu Tyr Ile Glu Cys Ser Ser Lys Thr Gln
        150                 155                 160
ttg aat gtg aag caa gtt ttc gat gct gcc att aaa gtc gtt ctg gcg       706
Leu Asn Val Lys Gln Val Phe Asp Ala Ala Ile Lys Val Val Leu Ala
    165                 170                 175
cct cca aag gcg aag aaa aag aag gga aaa gct caa aag gca tgc tca       754
Pro Pro Lys Ala Lys Lys Lys Lys Gly Lys Ala Gln Lys Ala Cys Ser
180                 185                 190                 195
ata ttg tgatgaagaa gatgaaaacc agttgttcaa cgcttatctc gtcttgaatt        810
Ile Leu

caattccata tgttggttga tttgtatttt gatctttgag tactcgaaaa tcttggtgta     870

ctaattctgt atcttggtca aaaatttgta tttccatgct cgttttctgt ttgcgactgt     930

tttactactt tgttcttcta agttgaaaaa tcggtatcta tcgttgaatc aatgaattcc     990

attctaatga aaaaaaaaaa aaaaa                                          1015


<210> 228
<211> 197
<212> PRT
<213> Zinnia elegans

<400> 228
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Ile Val Leu Val Gly Thr Lys Leu Asp Leu Arg Asp Asp Lys Gln Phe
        115                 120                 125
Phe Val Asp His Pro Gly Ala Thr Pro Ile Thr Thr Ala Gln Gly Glu
    130                 135                 140
Glu Leu Lys Lys Thr Ile Gly Ala Pro Glu Tyr Ile Glu Cys Ser Ser
145                 150                 155                 160
Lys Thr Gln Leu Asn Val Lys Gln Val Phe Asp Ala Ala Ile Lys Val
                165                 170                 175
Val Leu Ala Pro Pro Lys Ala Lys Lys Lys Lys Gly Lys Ala Gln Lys
            180                 185                 190
Ala Cys Ser Ile Leu
        195

<210> 229
<211> 843
<212> DNA
<213> Zinnia elegans

<220>
<221> CDS
<222> (114)..(710)

<400> 229
aaacacttgt cccattacta ctacacttat tccttttatc ttcctttccc ttgcttaaca      60

acaacaacca aaccacttgt cgatctcttt gtatcatatt ccgatcgacc gaa atg        116
                                                           Met
                                                           1
gca gca gca gcc acc aga ttc atc aag tgc gtc acc gtt ggc gac ggt       164
Ala Ala Ala Ala Thr Arg Phe Ile Lys Cys Val Thr Val Gly Asp Gly
            5                   10                  15
gcg gtc gga aaa acc tgc atg ctc atc tcc tac acc agc aat act ttc       212
Ala Val Gly Lys Thr Cys Met Leu Ile Ser Tyr Thr Ser Asn Thr Phe
        20                  25                  30
ccc acc gat tat gtg ccg aca gtg ttt gat aac ttc agt gcg aat gtt       260
Pro Thr Asp Tyr Val Pro Thr Val Phe Asp Asn Phe Ser Ala Asn Val
    35                  40                  45
gtg gtc gga gac agc act gtc aat ctt ggc ctt tgg gat act gcc ggt       308
Val Val Gly Asp Ser Thr Val Asn Leu Gly Leu Trp Asp Thr Ala Gly
50                  55                  60                  65
cag gag gat tat aac aga ctg agg cca ctg agt tat aga ggt gca gat       356
Gln Glu Asp Tyr Asn Arg Leu Arg Pro Leu Ser Tyr Arg Gly Ala Asp
                70                  75                  80
gtg ttt tta ttg gca ttt tct ctc att agc aga ccc agt tat gaa aac       404
Val Phe Leu Leu Ala Phe Ser Leu Ile Ser Arg Pro Ser Tyr Glu Asn
            85                  90                  95
atc tcc aag aag tgg atc tca gaa cta agg cat tat gcc cca gat gtc       452
Ile Ser Lys Lys Trp Ile Ser Glu Leu Arg His Tyr Ala Pro Asp Val
        100                 105                 110
ccc att gtg ctg gtg gga acc aaa tta gat tta cgt gaa gac aaa caa       500
Pro Ile Val Leu Val Gly Thr Lys Leu Asp Leu Arg Glu Asp Lys Gln
    115                 120                 125

tat ttg agt gat cat cca aat gtt aca gcc atc aca act tct cag ggt 548
Tyr Leu Ser Asp His Pro Asn Val Thr Ala Ile Thr Thr Ser Gln Gly
130                 135                 140                 145
gaa gaa ctg aag aaa agt att ggt gca gca gtg tac att gag tgt agc 596
Glu Glu Leu Lys Lys Ser Ile Gly Ala Ala Val Tyr Ile Glu Cys Ser
                150                 155                 160
tcc aaa act caa cag aat gtg aag gct gtt ttt gat gct gcg att aga 644
Ser Lys Thr Gln Gln Asn Val Lys Ala Val Phe Asp Ala Ala Ile Arg
            165                 170                 175
gtt gtt tta caa cca cca aag ctg aag aaa aag cga agc aag caa cga 692
Val Val Leu Gln Pro Pro Lys Leu Lys Lys Lys Arg Ser Lys Gln Arg
        180                 185                 190
cta tgt gtc tat cta tagatctgct tctctgtgca attgcatgta taatgttcta 747
Leu Cys Val Tyr Leu
    195
tgtataatgt ctttctcatt tagaacttta aatgatgtaa tgaaatccaa gtcatcaaat 807

ggtttccttg taaaaaaaaa aaaaaaaaaa aaaaaa 843

<210> 230
<211> 198
<212> PRT
<213> Zinnia elegans

<400> 230
Met Ala Ala Ala Ala Thr Arg Phe Ile Lys Cys Val Thr Val Gly Asp
1               5                   10                  15
Gly Ala Val Gly Lys Thr Cys Met Leu Ile Ser Tyr Thr Ser Asn Thr
            20                  25                  30
Phe Pro Thr Asp Tyr Val Pro Thr Val Phe Asp Asn Phe Ser Ala Asn
        35                  40                  45
Val Val Val Gly Asp Ser Thr Val Asn Leu Gly Leu Trp Asp Thr Ala
    50                  55                  60
Gly Gln Glu Asp Tyr Asn Arg Leu Arg Pro Leu Ser Tyr Arg Gly Ala
65                  70                  75                  80
Asp Val Phe Leu Leu Ala Phe Ser Leu Ile Ser Arg Pro Ser Tyr Glu
                85                  90                  95
Asn Ile Ser Lys Lys Trp Ile Ser Glu Leu Arg His Tyr Ala Pro Asp
            100                 105                 110
Val Pro Ile Val Leu Val Gly Thr Lys Leu Asp Leu Arg Glu Asp Lys
        115                 120                 125
Gln Tyr Leu Ser Asp His Pro Asn Val Thr Ala Ile Thr Thr Ser Gln
    130                 135                 140
Gly Glu Glu Leu Lys Lys Ser Ile Gly Ala Ala Val Tyr Ile Glu Cys
145                 150                 155                 160
Ser Ser Lys Thr Gln Gln Asn Val Lys Ala Val Phe Asp Ala Ala Ile
                165                 170                 175
Arg Val Val Leu Gln Pro Pro Lys Leu Lys Lys Lys Arg Ser Lys Gln
            180                 185                 190
Arg Leu Cys Val Tyr Leu
        195

<210> 231
<211> 659
<212> DNA
<213> Ciona intestinalis

<220>
<221> CDS
<222> (24)..(605)

<400> 231
ataaaattta ttgaaacacg aag atg nct tca att cgt aag aag ctg aaa ata 53
Met Xaa Ser Ile Arg Lys Lys Leu Lys Ile

                                    1               5                   10
gat ggc gaa ggg gct tgt ggt aaa act tgc tta ctc atc gng ttt agc        101
Asp Gly Glu Gly Ala Cys Gly Lys Thr Cys Leu Leu Ile Xaa Phe Ser
                15                  20                  25
aaa gat caa ttc cca gaa gtt tac gtc cca act gtt ttt gaa aac tat        149
Lys Asp Gln Phe Pro Glu Val Tyr Val Pro Thr Val Phe Glu Asn Tyr
                30                  35                  40
gtg gct gat att gaa gta gac tct aaa cag gtt gag ctt gct ttg tgg        197
Val Ala Asp Ile Glu Val Asp Ser Lys Gln Val Glu Leu Ala Leu Trp
        45                  50                  55
gat aca gct ggt caa gaa gat tac gac agg ctt cgt cca ctt tcc tac        245
Asp Thr Ala Gly Gln Glu Asp Tyr Asp Arg Leu Arg Pro Leu Ser Tyr
    60                  65                  70
ccc gat act gat gtt att ctt atg tgt ttc tca atc gac agt cca gat        293
Pro Asp Thr Asp Val Ile Leu Met Cys Phe Ser Ile Asp Ser Pro Asp
75                  80                  85                  90
tca ctt gag aac att ccc gaa aaa tgg acc cct gag gta agg cat ttt        341
Ser Leu Glu Asn Ile Pro Glu Lys Trp Thr Pro Glu Val Arg His Phe
                95                  100                 105
tgc cca agt gtt cca aty att ttg gtt gga aac aaa aaa gat ctt cgt        389
Cys Pro Ser Val Pro Ile Ile Leu Val Gly Asn Lys Lys Asp Leu Arg
            110                 115                 120
aac ggc agt tca aca ata aaa gaa ctt gct aaa atg aaa caa tct gcc        437
Asn Gly Ser Ser Thr Ile Lys Glu Leu Ala Lys Met Lys Gln Ser Ala
        125                 130                 135
gtt agt aac gaa gat gga atg gcg atg gca gac aag att ggc gct tac        485
Val Ser Asn Glu Asp Gly Met Ala Met Ala Asp Lys Ile Gly Ala Tyr
    140                 145                 150
gga tat atg gaa tgt tca gcg cgc acc aaa gaa ggt gtt cgg gaa gtg        533
Gly Tyr Met Glu Cys Ser Ala Arg Thr Lys Glu Gly Val Arg Glu Val
155                 160                 165                 170
ttt gag ctt gca act aaa gca gct tta caa acc aag aaa aga aag aaa        581
Phe Glu Leu Ala Thr Lys Ala Ala Leu Gln Thr Lys Lys Arg Lys Lys
                175                 180                 185
aag agt gga tct gaa gtc ttg taaagaaatt ttaactggag tctagcaatt           632
Lys Ser Gly Ser Glu Val Leu
            190
gacttaacat tggacgcatg ctttcat                                          659

<210> 232
<211> 193
<212> PRT
<213> Ciona intestinalis

<400> 232
Met Xaa Ser Ile Arg Lys Lys Leu Lys Ile Asp Gly Glu Gly Ala Cys
1               5                   10                  15
Gly Lys Thr Cys Leu Leu Ile Xaa Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Ser Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Arg His Phe Cys Pro Ser Val Pro Ile
            100                 105                 110
Ile Leu Val Gly Asn Lys Lys Asp Leu Arg Asn Gly Ser Ser Thr Ile
        115                 120                 125
Lys Glu Leu Ala Lys Met Lys Gln Ser Ala Val Ser Asn Glu Asp Gly
    130                 135                 140
Met Ala Met Ala Asp Lys Ile Gly Ala Tyr Gly Tyr Met Glu Cys Ser
145                 150                 155                 160

Ala Arg Thr Lys Glu Gly Val Arg Glu Val Phe Glu Leu Ala Thr Lys
                165                 170                 175
Ala Ala Leu Gln Thr Lys Lys Arg Lys Lys Lys Ser Gly Ser Glu Val
            180                 185                 190
Leu

<210> 233
<211> 567
<212> DNA
<213> Schistosoma mansoni

<220>
<221> CDS
<222> (1)..(567)

<400> 233
atg caa gcc atc aaa tgt gtg gtt gtc ggt gat ggc gct gtc gga aag      48
Met Gln Ala Ile Lys Cys Val Val Val Gly Asp Gly Ala Val Gly Lys
1               5                   10                  15
acc tgt tta ctc att agc tac acg acg aat gcc ttt cct ggt gag tac      96
Thr Cys Leu Leu Ile Ser Tyr Thr Thr Asn Ala Phe Pro Gly Glu Tyr
            20                  25                  30
gtc cca acc gtt ttc gat aac tat tct gcc aac gta atg gtt gac cga      144
Val Pro Thr Val Phe Asp Asn Tyr Ser Ala Asn Val Met Val Asp Arg
        35                  40                  45
aaa cct gtg aac ctt ggt ttg tgg gat aca gct ggt cag gag gat tat      192
Lys Pro Val Asn Leu Gly Leu Trp Asp Thr Ala Gly Gln Glu Asp Tyr
    50                  55                  60
gac aga cta aga cca ctc tca tat cca caa act gat gtg ttt ttg gtg      240
Asp Arg Leu Arg Pro Leu Ser Tyr Pro Gln Thr Asp Val Phe Leu Val
65                  70                  75                  80
tgc ttc tcc tta gtc agt cgc aca tcg ttc gag aat gtt cgt agt aaa      288
Cys Phe Ser Leu Val Ser Arg Thr Ser Phe Glu Asn Val Arg Ser Lys
                85                  90                  95
tgg cat ccg gag ata tcc gca tat gtt cca aga gct cct att att ctt      336
Trp His Pro Glu Ile Ser Ala Tyr Val Pro Arg Ala Pro Ile Ile Leu
            100                 105                 110
gtc gga act aaa cga gac ctt cgg gat agt cct aat ggc cta aaa tca      384
Val Gly Thr Lys Arg Asp Leu Arg Asp Ser Pro Asn Gly Leu Lys Ser
        115                 120                 125
aca acg ttt cca gtc acg tat gca gag ggt tgc agg atg gcc aga gaa      432
Thr Thr Phe Pro Val Thr Tyr Ala Glu Gly Cys Arg Met Ala Arg Glu
    130                 135                 140
att aaa gct gtg aaa tac ctg gaa tgc tct gct cta act cag ttt gga      480
Ile Lys Ala Val Lys Tyr Leu Glu Cys Ser Ala Leu Thr Gln Phe Gly
145                 150                 155                 160
tta aaa gat gtc ttc gac gaa gct att cga gca gta ctg atg cct gaa      528
Leu Lys Asp Val Phe Asp Glu Ala Ile Arg Ala Val Leu Met Pro Glu
                165                 170                 175
ggg aag aaa aag aaa cat agt tca tgt gaa tta att taa                  567
Gly Lys Lys Lys Lys His Ser Ser Cys Glu Leu Ile
            180                 185

<210> 234
<211> 188
<212> PRT
<213> Schistosoma mansoni

<400> 234
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Val Pro Thr Val Phe Asp Asn Tyr Ser Ala Asn Val Met Val Asp Arg
        35                  40                  45
Lys Pro Val Asn Leu Gly Leu Trp Asp Thr Ala Gly Gln Glu Asp Tyr

50                  55                  60
Asp Arg Leu Arg Pro Leu Ser Tyr Pro Gln Thr Asp Val Phe Leu Val
65                  70                  75                  80
Cys Phe Ser Leu Val Ser Arg Thr Ser Phe Glu Asn Val Arg Ser Lys
                85                  90                  95
Trp His Pro Glu Ile Ser Ala Tyr Val Pro Arg Ala Pro Ile Ile Leu
            100                 105                 110
Val Gly Thr Lys Arg Asp Leu Arg Asp Ser Pro Asn Gly Leu Lys Ser
        115                 120                 125
Thr Thr Phe Pro Val Thr Tyr Ala Glu Gly Cys Arg Met Ala Arg Glu
    130                 135                 140
Ile Lys Ala Val Lys Tyr Leu Glu Cys Ser Ala Leu Thr Gln Phe Gly
145                 150                 155                 160
Leu Lys Asp Val Phe Asp Glu Ala Ile Arg Ala Val Leu Met Pro Glu
                165                 170                 175
Gly Lys Lys Lys Lys His Ser Ser Cys Glu Leu Ile
            180                 185

<210> 235
<211> 600
<212> DNA
<213> Penicillium marneffei

<220>
<221> CDS
<222> (1)..(600)

<400> 235
atg gcg tct ggg cct gcg act caa tcg ttg aag tgt gtg gtg acc ggt      48
Met Ala Ser Gly Pro Ala Thr Gln Ser Leu Lys Cys Val Val Thr Gly
1               5                   10                  15
gat ggt gct gtc ggc aag aca tgt ctc ctg ata tca tac acc acc aat      96
Asp Gly Ala Val Gly Lys Thr Cys Leu Leu Ile Ser Tyr Thr Thr Asn
            20                  25                  30
gcc ttt ccc ggc gaa tac att ccc acc gta ttc gac aac tac tcc gct      144
Ala Phe Pro Gly Glu Tyr Ile Pro Thr Val Phe Asp Asn Tyr Ser Ala
        35                  40                  45
agt gtc atg gtc gac ggc agg ccc atc agc ctc gga ctc tgg gat aca      192
Ser Val Met Val Asp Gly Arg Pro Ile Ser Leu Gly Leu Trp Asp Thr
    50                  55                  60
gct ggt caa gag gat tat gac cgt ctc cgc ccc tta tcc tac cct caa      240
Ala Gly Gln Glu Asp Tyr Asp Arg Leu Arg Pro Leu Ser Tyr Pro Gln
65                  70                  75                  80
acc gac gtc ttc ttg atc tgc ttc tct atc gtc tct cca cca tcc ttt      288
Thr Asp Val Phe Leu Ile Cys Phe Ser Ile Val Ser Pro Pro Ser Phe
                85                  90                  95
gac aac gta aaa gcc aag tgg ttt ccc gaa atc gag cac cat gca ccc      336
Asp Asn Val Lys Ala Lys Trp Phe Pro Glu Ile Glu His His Ala Pro
            100                 105                 110
ggc gtg ccc atc att ctt gtc ggc aca aag ctt gat ttg aga gaa gat      384
Gly Val Pro Ile Ile Leu Val Gly Thr Lys Leu Asp Leu Arg Glu Asp
        115                 120                 125
aga gct acc gct gag gcg ctg cga gcc aaa aag atg gag ccg gtg tcg      432
Arg Ala Thr Ala Glu Ala Leu Arg Ala Lys Lys Met Glu Pro Val Ser
    130                 135                 140
tat gag cag gcg ctt gca gtt gca aag gag att agg gcg cat aag tat      480
Tyr Glu Gln Ala Leu Ala Val Ala Lys Glu Ile Arg Ala His Lys Tyr
145                 150                 155                 160
ctg gag tgt tcg gcc ttg acc cag agg aat ttg aag agc gtg ttt gat      528
Leu Glu Cys Ser Ala Leu Thr Gln Arg Asn Leu Lys Ser Val Phe Asp
                165                 170                 175
gaa gct att cgg gcc gtc ctc aat cct cgc cct caa ccc aag aac aag      576
Glu Ala Ile Arg Ala Val Leu Asn Pro Arg Pro Gln Pro Lys Asn Lys
            180                 185                 190
gca aaa cga tgc act att ctg taa                                      600
Ala Lys Arg Cys Thr Ile Leu
        195

<210> 236
<211> 199
<212> PRT
<213> Penicillium marneffei

<400> 236
Met Ala Ser Gly Pro Ala Thr Gln Ser Leu Lys Cys Val Val Thr Gly
1               5                   10                  15
Asp Gly Ala Val Gly Lys Thr Cys Leu Leu Ile Ser Tyr Thr Thr Asn
            20                  25                  30
Ala Phe Pro Gly Glu Tyr Ile Pro Thr Val Phe Asp Asn Tyr Ser Ala
        35                  40                  45
Ser Val Met Val Asp Gly Arg Pro Ile Ser Leu Gly Leu Trp Asp Thr
    50                  55                  60
Ala Gly Gln Glu Asp Tyr Asp Arg Leu Arg Pro Leu Ser Tyr Pro Gln
65                  70                  75                  80
Thr Asp Val Phe Leu Ile Cys Phe Ser Ile Val Ser Pro Pro Ser Phe
                85                  90                  95
Asp Asn Val Lys Ala Lys Trp Phe Pro Glu Ile Glu His His Ala Pro
            100                 105                 110
Gly Val Pro Ile Ile Leu Val Gly Thr Lys Leu Asp Leu Arg Glu Asp
        115                 120                 125
Arg Ala Thr Ala Glu Ala Leu Arg Ala Lys Lys Met Glu Pro Val Ser
    130                 135                 140
Tyr Glu Gln Ala Leu Ala Val Ala Lys Glu Ile Arg Ala His Lys Tyr
145                 150                 155                 160
Leu Glu Cys Ser Ala Leu Thr Gln Arg Asn Leu Lys Ser Val Phe Asp
                165                 170                 175
Glu Ala Ile Arg Ala Val Leu Asn Pro Arg Pro Gln Pro Lys Asn Lys
            180                 185                 190
Ala Lys Arg Cys Thr Ile Leu
        195

<210> 237
<211> 845
<212> DNA
<213> Schistosoma japonicum

<220>
<221> CDS
<222> (39)..(617)

<400> 237
ccgggccccc ccttggactt tactggtttg ctggagtg atg agt gca ata agg aaa      56
                                          Met Ser Ala Ile Arg Lys
                                          1               5
aag ctc gtt att gtt ggg gat gga gcc tgt ggg aaa act tgt cta cta      104
Lys Leu Val Ile Val Gly Asp Gly Ala Cys Gly Lys Thr Cys Leu Leu
            10                  15                  20
ata gta ttt agc aaa gac cag ttt cct gaa gtt tac gtt ccg act gta      152
Ile Val Phe Ser Lys Asp Gln Phe Pro Glu Val Tyr Val Pro Thr Val
        25                  30                  35
ttc gaa aac tat gtc gcg gat att gag gta gat aac aaa cag gtt gaa      200
Phe Glu Asn Tyr Val Ala Asp Ile Glu Val Asp Asn Lys Gln Val Glu
    40                  45                  50
ttg gca ctt tgg gat act gct ggt caa gaa gac tat gac aga tta aga      248
Leu Ala Leu Trp Asp Thr Ala Gly Gln Glu Asp Tyr Asp Arg Leu Arg
55                  60                  65                  70
cca ctt tct tac ccc gat acg gat gtt att tta atg tgc ttt agc atc      296
Pro Leu Ser Tyr Pro Asp Thr Asp Val Ile Leu Met Cys Phe Ser Ile
                75                  80                  85
gac act cca gat agt ttg gaa aac ata cct gag aaa tgg aca cca gaa      344
Asp Thr Pro Asp Ser Leu Glu Asn Ile Pro Glu Lys Trp Thr Pro Glu
            90                  95                  100
gtc cga cat ttc tgc cct gat gta cct att gtc tta gtt gga aac aaa      392
Val Arg His Phe Cys Pro Asp Val Pro Ile Val Leu Val Gly Asn Lys
        105                 110                 115

aag gac ttg cga agt gaa aac tca aca aga gat gac gga cat aga gga     440
Lys Asp Leu Arg Ser Glu Asn Ser Thr Arg Asp Asp Gly His Arg Gly
    120                 125                 130
aaa cag gaa ttt gtt aaa tcg gat gag ggt tat gca atg gcc gat cgt     488
Lys Gln Glu Phe Val Lys Ser Asp Glu Gly Tyr Ala Met Ala Asp Arg
135                 140                 145                 150
att cat gct tac tca tat atc gag tgt tca gct aag acg aag gaa gga     536
Ile His Ala Tyr Ser Tyr Ile Glu Cys Ser Ala Lys Thr Lys Glu Gly
                155                 160                 165
gtt cgt gaa gtt ttc gag acc gcg act aga gct gct tta cag tcg aag     584
Val Arg Glu Val Phe Glu Thr Ala Thr Arg Ala Ala Leu Gln Ser Lys
            170                 175                 180
aaa acg aaa aag aag aag tgt gat cta att tgattctcgg ttttctccaa       634
Lys Thr Lys Lys Lys Lys Cys Asp Leu Ile
        185                 190
atatggttat ccggttttaa agacttctta cattcccccc ttccgatttc gcttcaggat   694

attaaatttc gactttttat taactgcttg tttcttcaac aaacatattt tggtttttta   754

aaaagacaat ccattccatg ataaccttta ttatccgttt ttatcttgat actaaaatct   814

gtcaggcctg ttaaaaaaaa aaaaaaaaaa a                                  845

<210> 238
<211> 192
<212> PRT
<213> Schistosoma japonicum

<400> 238
Met Ser Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Asn Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Thr Pro Asp Ser Leu Glu Asn Ile Pro
                85                  90                  95
Glu Lys Trp Thr Pro Glu Val Arg His Phe Cys Pro Asp Val Pro Ile
            100                 105                 110
Val Leu Val Gly Asn Lys Lys Asp Leu Arg Ser Glu Asn Ser Thr Arg
        115                 120                 125
Asp Asp Gly His Arg Gly Lys Gln Glu Phe Val Lys Ser Asp Glu Gly
    130                 135                 140
Tyr Ala Met Ala Asp Arg Ile His Ala Tyr Ser Tyr Ile Glu Cys Ser
145                 150                 155                 160
Ala Lys Thr Lys Glu Gly Val Arg Glu Val Phe Glu Thr Ala Thr Arg
                165                 170                 175
Ala Ala Leu Gln Ser Lys Lys Thr Lys Lys Lys Lys Cys Asp Leu Ile
            180                 185                 190

<210> 239
<211> 582
<212> DNA
<213> Ustilago maydis

<220>
<221> CDS
<222> (1)..(582)

<400> 239
atg cag acc atc aag tgt gta gtc gtc gga gac ggt gcc gtc gga aag 48
Met Gln Thr Ile Lys Cys Val Val Val Gly Asp Gly Ala Val Gly Lys
1               5                   10                  15
acg tgt ctt ctt atc tcg tat aca acc aac gcc ttc ccg ggc gaa tac 96
Thr Cys Leu Leu Ile Ser Tyr Thr Thr Asn Ala Phe Pro Gly Glu Tyr
            20                  25                  30
atc ccc aca gtg ttt gac aac tat tcg gca aac gtc atg gtc gac ggt 144
Ile Pro Thr Val Phe Asp Asn Tyr Ser Ala Asn Val Met Val Asp Gly
        35                  40                  45
aaa ccc gtc tct ctc ggt ctc tgg gat act gcg ggt caa gaa gac tac 192
Lys Pro Val Ser Leu Gly Leu Trp Asp Thr Ala Gly Gln Glu Asp Tyr
    50                  55                  60
gac cgg tta aga ccg ctc tcg tac ccg caa acc gac gtg ttc ctc gtg 240
Asp Arg Leu Arg Pro Leu Ser Tyr Pro Gln Thr Asp Val Phe Leu Val
65                  70                  75                  80
tgc ttt tca ctg gtg agt cca cct tcg ttc gaa aac gtt cga acc aag 288
Cys Phe Ser Leu Val Ser Pro Pro Ser Phe Glu Asn Val Arg Thr Lys
                85                  90                  95
tgg tgg ccc gaa gtg tcg cat cac gct ccc aac att ccc acc atc ctc 336
Trp Trp Pro Glu Val Ser His His Ala Pro Asn Ile Pro Thr Ile Leu
            100                 105                 110
gtg gga acc aag ttg gat ctg cgc gag gat cca gaa acg att gcc aag 384
Val Gly Thr Lys Leu Asp Leu Arg Glu Asp Pro Glu Thr Ile Ala Lys
        115                 120                 125
ctg cgc gat cgc agg atg cag cct atc acg tat gcg cag ggc aat cag 432
Leu Arg Asp Arg Arg Met Gln Pro Ile Thr Tyr Ala Gln Gly Asn Gln
    130                 135                 140
atg gcg agg gac att cac gct acc aag tat ttg gag tgc tct gca ctc 480
Met Ala Arg Asp Ile His Ala Thr Lys Tyr Leu Glu Cys Ser Ala Leu
145                 150                 155                 160
acc cag aag gga ttg aag ggc gta ttt gat gaa gcg atc agg agc gtt 528
Thr Gln Lys Gly Leu Lys Gly Val Phe Asp Glu Ala Ile Arg Ser Val
                165                 170                 175
ttg gct cct gca cca gtc cag agt aag aag aaa aac aac tgt ttg att 576
Leu Ala Pro Ala Pro Val Gln Ser Lys Lys Lys Asn Asn Cys Leu Ile
            180                 185                 190
ctt taa 582
Leu

<210> 240
<211> 193
<212> PRT
<213> Ustilago maydis

<400> 240
Met Gln Thr Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly
        35                  40                  45
Lys Pro Val Ser Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Leu Val Ser Pro Pro Ser Phe Glu Asn Val Arg Thr Lys
                85                  90                  95
Trp Trp Pro Glu Val Ser His His Ala Pro Asn Ile Pro Thr Ile Leu
            100                 105                 110
Val Gly Thr Lys Leu Asp Leu Arg Glu Asp Pro Glu Thr Ile Ala Lys
        115                 120                 125
Leu Arg Asp Arg Arg Met Gln Pro Ile Thr Tyr Ala Gln Gly Asn Gln
    130                 135                 140
Met Ala Arg Asp Ile His Ala Thr Lys Tyr Leu Glu Cys Ser Ala Leu

145                 150                 155                 160
Thr Gln Lys Gly Leu Lys Gly Val Phe Asp Glu Ala Ile Arg Ser Val
                165                 170                 175
Leu Ala Pro Ala Pro Val Gln Ser Lys Lys Lys Asn Asn Cys Leu Ile
            180                 185                 190
Leu

<210> 241
<211> 1196
<212> DNA
<213> Ustilago maydis

<220>
<221> CDS
<222> (436)..(1080)

<400> 241
aattgcgatg ggatgcaacc gtccacaggg tcccctaagc ccgtgggttc atgtggtcaa      60

cctcgccacg gccttgatca acacccacca ccgcattcgt agattttcaa acaataccgg     120

ccccatttca tcgccttttg gttttggaag tcaggaacaa gcagcatcat agcaagtgga     180

cgcaccaacg agcccaagtt acagccaatg gacattcagc tttcctccgg ttcgctcgac     240

ggccgaatcc tcctcctctc tgtcgggaac gggcgcacaa ccattgtagg aattgacacc     300

gattgtttca attgtcccag ccagaagttt gcaccgctca gcacctttgg cattggtagg     360

aacagaaatc ccttttcgtt tccatcgcaa acaggaacac tgtcttgttg atcatctttg     420

ctcccgccgc tcagc atg gca ccg gca gca att tgt agg aag ctt gta att     471
                 Met Ala Pro Ala Ala Ile Cys Arg Lys Leu Val Ile
                 1               5                   10
gtc ggc gac ggt gct tgc ggc aag acg agt ttg ctt tgc gtt ttt gcc     519
Val Gly Asp Gly Ala Cys Gly Lys Thr Ser Leu Leu Cys Val Phe Ala
        15                  20                  25
att ggc gag ttc ccg caa gag tat gaa ccc acc att ttc gaa aac tac     567
Ile Gly Glu Phe Pro Gln Glu Tyr Glu Pro Thr Ile Phe Glu Asn Tyr
    30                  35                  40
gtc gcc gag atc cgc ctt gat ggc aag cct gtc cag ctg gcg cta tgg     615
Val Ala Glu Ile Arg Leu Asp Gly Lys Pro Val Gln Leu Ala Leu Trp
45                  50                  55                  60
gac acc gcg ggt cag gaa gaa tac gag cgt ctt cgt cca ctc tcc tac     663
Asp Thr Ala Gly Gln Glu Glu Tyr Glu Arg Leu Arg Pro Leu Ser Tyr
                65                  70                  75
tca caa gca cac gtc atc ttg atc gcc ttt gcc atc gat aca ccc gac     711
Ser Gln Ala His Val Ile Leu Ile Ala Phe Ala Ile Asp Thr Pro Asp
            80                  85                  90
tcg ctc gaa aac gtg caa gtc aag tgg atg gag gag gta cgt caa ata     759
Ser Leu Glu Asn Val Gln Val Lys Trp Met Glu Glu Val Arg Gln Ile
        95                  100                 105
tgc ggc ccc tca gtg cct gtg ctc ctg gta ggc tgc aag aag gat ctt     807
Cys Gly Pro Ser Val Pro Val Leu Leu Val Gly Cys Lys Lys Asp Leu
    110                 115                 120
cgc gaa aat gcc atc gct aag ggc aag ccg gtt cag ggt cac tat gta     855
Arg Glu Asn Ala Ile Ala Lys Gly Lys Pro Val Gln Gly His Tyr Val
125                 130                 135                 140
aag aga caa cag gct aaa ctg gta gca gca cag atc ggc gct cga tcg     903
Lys Arg Gln Gln Ala Lys Leu Val Ala Ala Gln Ile Gly Ala Arg Ser

                145                     150                     155
tat cac gaa tgc tca tca ctc aac aac caa ggc gtc gac gcc gtg ttc        951
Tyr His Glu Cys Ser Ser Leu Asn Asn Gln Gly Val Asp Ala Val Phe
            160                     165                     170
gaa gcc gcg acg cgc gcg gcc atg ctt gtg cgc aat tcc ggt gct tct        999
Glu Ala Ala Thr Arg Ala Ala Met Leu Val Arg Asn Ser Gly Ala Ser
        175                     180                     185
tca gga ggc gcc atc tcg cag agc aag acc aag gag gca tta cac aac        1047
Ser Gly Gly Ala Ile Ser Gln Ser Lys Thr Lys Glu Ala Leu His Asn
    190                     195                     200
gat gct ggc tct tgc aaa tgt atc gtc ctc tagaaatctc tcttgtgctt        1097
Asp Ala Gly Ser Cys Lys Cys Ile Val Leu
205                     210
cgtcttgtcg acccttgtcc taagcctcgc tctcgtctgc ctttccatcg tacagtcgct        1157

ctccctgcat ttttgtccct ccactcctcc catgttcta        1196

<210> 242
<211> 214
<212> PRT
<213> Ustilago maydis

<400> 242
Met Ala Pro Ala Ala Ile Cys Arg Lys Leu Val Ile Val Gly Asp Gly
1               5                   10                  15
Ala Cys Gly Lys Thr Ser Leu Leu Cys Val Phe Ala Ile Gly Glu Phe
            20                  25                  30
Pro Gln Glu Tyr Glu Pro Thr Ile Phe Glu Asn Tyr Val Ala Glu Ile
        35                  40                  45
Arg Leu Asp Gly Lys Pro Val Gln Leu Ala Leu Trp Asp Thr Ala Gly
    50                  55                  60
Gln Glu Glu Tyr Glu Arg Leu Arg Pro Leu Ser Tyr Ser Gln Ala His
65                  70                  75                  80
Val Ile Leu Ile Ala Phe Ala Ile Asp Thr Pro Asp Ser Leu Glu Asn
                85                  90                  95
Val Gln Val Lys Trp Met Glu Glu Val Arg Gln Ile Cys Gly Pro Ser
            100                 105                 110
Val Pro Val Leu Leu Val Gly Cys Lys Lys Asp Leu Arg Glu Asn Ala
        115                 120                 125
Ile Ala Lys Gly Lys Pro Val Gln Gly His Tyr Val Lys Arg Gln Gln
    130                 135                 140
Ala Lys Leu Val Ala Ala Gln Ile Gly Ala Arg Ser Tyr His Glu Cys
145                 150                 155                 160
Ser Ser Leu Asn Asn Gln Gly Val Asp Ala Val Phe Glu Ala Ala Thr
                165                 170                 175
Arg Ala Ala Met Leu Val Arg Asn Ser Gly Ala Ser Ser Gly Gly Ala
            180                 185                 190
Ile Ser Gln Ser Lys Thr Lys Glu Ala Leu His Asn Asp Ala Gly Ser
        195                 200                 205
Cys Lys Cys Ile Val Leu
    210

<210> 243
<211> 576
<212> DNA
<213> Ustilago maydis

<220>
<221> CDS
<222> (1)..(576)

<400> 243
atg cag acc atc aag tgt gtt gtt gtc gga gat ggt gcg gtc ggt aag        48
Met Gln Thr Ile Lys Cys Val Val Val Gly Asp Gly Ala Val Gly Lys

1                 5                   10                  15
acc tgc ctg ttg att tcg tac acc acc aac aag ttt ccc tcg gag tat          96
Thr Cys Leu Leu Ile Ser Tyr Thr Thr Asn Lys Phe Pro Ser Glu Tyr
            20                  25                  30
gtt ccg aca gtg ttt gac aac tac gcc gtg act gtc atg att ggc gag          144
Val Pro Thr Val Phe Asp Asn Tyr Ala Val Thr Val Met Ile Gly Glu
        35                  40                  45
gat ccg tac aca ctc gga ttg ttc gat acc gcc ggt cag gag gac tac          192
Asp Pro Tyr Thr Leu Gly Leu Phe Asp Thr Ala Gly Gln Glu Asp Tyr
    50                  55                  60
gac cga ctg cga ccg ctt tca tac ccg cag acg gat gtc ttc ctg gtc          240
Asp Arg Leu Arg Pro Leu Ser Tyr Pro Gln Thr Asp Val Phe Leu Val
65                  70                  75                  80
tgc ttc tcg gtc acc tca cca gcc tcg ttc gaa aat gtc aag gaa aag          288
Cys Phe Ser Val Thr Ser Pro Ala Ser Phe Glu Asn Val Lys Glu Lys
                85                  90                  95
tgg ttc ccg gag gtg cat cac cat tgc cct ggt gtg ccg tgc ctg att          336
Trp Phe Pro Glu Val His His His Cys Pro Gly Val Pro Cys Leu Ile
            100                 105                 110
gtg gga acc cag gtg gat ttg cgc gac gac cac gcc gtc atc gag aag          384
Val Gly Thr Gln Val Asp Leu Arg Asp Asp His Ala Val Ile Glu Lys
        115                 120                 125
ctt gca cgt tca aag cag cgt cct gtg ccc ttt gag gcg ggt gag cgt          432
Leu Ala Arg Ser Lys Gln Arg Pro Val Pro Phe Glu Ala Gly Glu Arg
    130                 135                 140
ttg gcg aga gag ttg ggt gcg gtc aag tac gtc gag tgc tcg gcg ctg          480
Leu Ala Arg Glu Leu Gly Ala Val Lys Tyr Val Glu Cys Ser Ala Leu
145                 150                 155                 160
acg caa aag gga ttg aag aac gtc ttc gac gag gcc atc gtg gct gcg          528
Thr Gln Lys Gly Leu Lys Asn Val Phe Asp Glu Ala Ile Val Ala Ala
                165                 170                 175
ctg gaa ccg cct gta atc cgc aag aag tcc aag tgc gcc att ctc              573
Leu Glu Pro Pro Val Ile Arg Lys Lys Ser Lys Cys Ala Ile Leu
            180                 185                 190
tga                                                                      576

<210> 244
<211> 191
<212> PRT
<213> Ustilago maydis

<400> 244
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Glu Tyr
            20                  25                  30
Val Pro Thr Val Phe Asp Asn Tyr Ala Val Thr Val Met Ile Gly Glu
        35                  40                  45
Asp Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Thr Ser Pro Ala Ser Phe Glu Asn Val Lys Glu Lys
                85                  90                  95
Trp Phe Pro Glu Val His His His Cys Pro Gly Val Pro Cys Leu Ile
            100                 105                 110
Val Gly Thr Gln Val Asp Leu Arg Asp Asp His Ala Val Ile Glu Lys
        115                 120                 125
Leu Ala Arg Ser Lys Gln Arg Pro Val Pro Phe Glu Ala Gly Glu Arg
    130                 135                 140
Leu Ala Arg Glu Leu Gly Ala Val Lys Tyr Val Glu Cys Ser Ala Leu
145                 150                 155                 160
Thr Gln Lys Gly Leu Lys Asn Val Phe Asp Glu Ala Ile Val Ala Ala
                165                 170                 175
Leu Glu Pro Pro Val Ile Arg Lys Lys Ser Lys Cys Ala Ile Leu

            180                 185                 190

<210> 245
<211> 633
<212> DNA
<213> Encephalitozoon cuniculi

<220>
<221> CDS
<222> (1)..(633)

<400> 245
atg aag gag aca aac tgg ata atg aac agt gaa aca cac cga tct gga 48
Met Lys Glu Thr Asn Trp Ile Met Asn Ser Glu Thr His Arg Ser Gly
1               5                   10                  15
aaa gtt gtc att gtg gga gat gga gcc tgc ggt aaa aca tgt ctg ctc 96
Lys Val Val Ile Val Gly Asp Gly Ala Cys Gly Lys Thr Cys Leu Leu
            20                  25                  30
gag gtg ttt aga aga aac cag ttt cct gaa aca tac atc cca acc gtt 144
Glu Val Phe Arg Arg Asn Gln Phe Pro Glu Thr Tyr Ile Pro Thr Val
        35                  40                  45
gtc gac aac ttc gtg aag gaa gtg aag gtc gct gac gac aaa tat gtt 192
Val Asp Asn Phe Val Lys Glu Val Lys Val Ala Asp Asp Lys Tyr Val
    50                  55                  60
tcc tta gcc tta tgg gat act gca ggg caa gag gat tat gat aca atc 240
Ser Leu Ala Leu Trp Asp Thr Ala Gly Gln Glu Asp Tyr Asp Thr Ile
65                  70                  75                  80
agg cct ctt tca tac aag gag aca gat ctt gta cta ctg tgc tat aca 288
Arg Pro Leu Ser Tyr Lys Glu Thr Asp Leu Val Leu Leu Cys Tyr Thr
                85                  90                  95
ata gaa aac aag aag aaa atc cca aac atc tca aga aaa tgg ctg atg 336
Ile Glu Asn Lys Lys Lys Ile Pro Asn Ile Ser Arg Lys Trp Leu Met
            100                 105                 110
gag ata aag aac tac tgc ccc tcg tcg caa ttc ttt ctt atc ggg ttg 384
Glu Ile Lys Asn Tyr Cys Pro Ser Ser Gln Phe Phe Leu Ile Gly Leu
        115                 120                 125
aag aag gat ata agg gat atg gat gat ccg aca atc gac aag tcg tca 432
Lys Lys Asp Ile Arg Asp Met Asp Asp Pro Thr Ile Asp Lys Ser Ser
    130                 135                 140
ata gtc acg gaa agc gag ggt cgg aag atc gca gat aat atc aat gct 480
Ile Val Thr Glu Ser Glu Gly Arg Lys Ile Ala Asp Asn Ile Asn Ala
145                 150                 155                 160
gcg agg ttt ttt gag tgc tct gcc cgt acc aga gaa aac gtg aac ctt 528
Ala Arg Phe Phe Glu Cys Ser Ala Arg Thr Arg Glu Asn Val Asn Leu
                165                 170                 175
gta ttc gtg gaa gcc gca aag tat ata tgg gac acg aag cag gct gct 576
Val Phe Val Glu Ala Ala Lys Tyr Ile Trp Asp Thr Lys Gln Ala Ala
            180                 185                 190
tct gat gcc aga tcg tgt ggc ttt ttt tcg tgc ata aga tgc tgc cgt 624
Ser Asp Ala Arg Ser Cys Gly Phe Phe Ser Cys Ile Arg Cys Cys Arg
        195                 200                 205
tat aga tag 633
Tyr Arg
    210

<210> 246
<211> 210
<212> PRT
<213> Encephalitozoon cuniculi

<400> 246
Met Lys Glu Thr Asn Trp Ile Met Asn Ser Glu Thr His Arg Ser Gly
1               5                   10                  15
Lys Val Val Ile Val Gly Asp Gly Ala Cys Gly Lys Thr Cys Leu Leu
            20                  25                  30
Glu Val Phe Arg Arg Asn Gln Phe Pro Glu Thr Tyr Ile Pro Thr Val
        35                  40                  45

Val Asp Asn Phe Val Lys Glu Val Lys Val Ala Asp Asp Lys Tyr Val
    50                  55                  60
Ser Leu Ala Leu Trp Asp Thr Ala Gly Gln Glu Asp Tyr Asp Thr Ile
65                  70                  75                  80
Arg Pro Leu Ser Tyr Lys Glu Thr Asp Leu Val Leu Leu Cys Tyr Thr
                85                  90                  95
Ile Glu Asn Lys Lys Lys Ile Pro Asn Ile Ser Arg Lys Trp Leu Met
            100                 105                 110
Glu Ile Lys Asn Tyr Cys Pro Ser Ser Gln Phe Phe Leu Ile Gly Leu
        115                 120                 125
Lys Lys Asp Ile Arg Asp Met Asp Asp Pro Thr Ile Asp Lys Ser Ser
    130                 135                 140
Ile Val Thr Glu Ser Glu Gly Arg Lys Ile Ala Asp Asn Ile Asn Ala
145                 150                 155                 160
Ala Arg Phe Phe Glu Cys Ser Ala Arg Thr Arg Glu Asn Val Asn Leu
                165                 170                 175
Val Phe Val Glu Ala Ala Lys Tyr Ile Trp Asp Thr Lys Gln Ala Ala
            180                 185                 190
Ser Asp Ala Arg Ser Cys Gly Phe Phe Ser Cys Ile Arg Cys Cys Arg
        195                 200                 205
Tyr Arg
    210

<210> 247
<211> 588
<212> DNA
<213> Mucor rouxii

<220>
<221> CDS
<222> (1)..(588)

<400> 247
atg gct gaa atc aga cga aaa ctt gtg att gtt gga gat ggt gct tgt      48
Met Ala Glu Ile Arg Arg Lys Leu Val Ile Val Gly Asp Gly Ala Cys
1               5                   10                  15
ggt aaa acc tgt ttg ttg att gtc ttt tca aag ggt act ttt cct gag      96
Gly Lys Thr Cys Leu Leu Ile Val Phe Ser Lys Gly Thr Phe Pro Glu
            20                  25                  30
ttc tat gtt ccc acc gtt ttt gaa aat tac gta gct gat gtc gaa gtc      144
Phe Tyr Val Pro Thr Val Phe Glu Asn Tyr Val Ala Asp Val Glu Val
        35                  40                  45
gat gga aaa cac gtg gaa tta gct tta tgg gat aca gca ggc caa gaa      192
Asp Gly Lys His Val Glu Leu Ala Leu Trp Asp Thr Ala Gly Gln Glu
    50                  55                  60
gat tat gat cgt ctc cgt ccc ttg tct tac cct gat tct cat gtt atc      240
Asp Tyr Asp Arg Leu Arg Pro Leu Ser Tyr Pro Asp Ser His Val Ile
65                  70                  75                  80
ttg att tgt ttt gct gtt gat tca ccc gat tca ttg gaa aac gtt caa      288
Leu Ile Cys Phe Ala Val Asp Ser Pro Asp Ser Leu Glu Asn Val Gln
                85                  90                  95
gag aag tgg atc tct gaa gta ctc cac ttc tgt caa ggt tta cct att      336
Glu Lys Trp Ile Ser Glu Val Leu His Phe Cys Gln Gly Leu Pro Ile
            100                 105                 110
gtt tta gtt ggt tgt aag aaa gat tta aga aat gat cct gga aca att      384
Val Leu Val Gly Cys Lys Lys Asp Leu Arg Asn Asp Pro Gly Thr Ile
        115                 120                 125
gaa gaa ctt aga aga aac tct caa aaa cct gtc agt tca gaa gag ggt      432
Glu Glu Leu Arg Arg Asn Ser Gln Lys Pro Val Ser Ser Glu Glu Gly
    130                 135                 140
gct tct att gct caa aga att agt gct tac aag tac ctt gaa tgt tct      480
Ala Ser Ile Ala Gln Arg Ile Ser Ala Tyr Lys Tyr Leu Glu Cys Ser
145                 150                 155                 160
gcc aag act ggt gaa ggt gta cgt gaa gta ttt gaa cac gca aca aga      528
Ala Lys Thr Gly Glu Gly Val Arg Glu Val Phe Glu His Ala Thr Arg
                165                 170                 175
gct gca tta atg gtt tct aag aag aaa aag tca aag agt ggt gtc tgc      576
Ala Ala Leu Met Val Ser Lys Lys Lys Lys Ser Lys Ser Gly Val Cys

180                 185                 190
aac ctt ttg taa                                                   588
Asn Leu Leu
        195

<210> 248
<211> 195
<212> PRT
<213> Mucor rouxii

<400> 248
Met Ala Glu Ile Arg Arg Lys Leu Val Ile Val Gly Asp Gly Ala Cys
1               5                   10                  15
Gly Lys Thr Cys Leu Leu Ile Val Phe Ser Lys Gly Thr Phe Pro Glu
            20                  25                  30
Phe Tyr Val Pro Thr Val Phe Glu Asn Tyr Val Ala Asp Val Glu Val
        35                  40                  45
Asp Gly Lys His Val Glu Leu Ala Leu Trp Asp Thr Ala Gly Gln Glu
    50                  55                  60
Asp Tyr Asp Arg Leu Arg Pro Leu Ser Tyr Pro Asp Ser His Val Ile
65                  70                  75                  80
Leu Ile Cys Phe Ala Val Asp Ser Pro Asp Ser Leu Glu Asn Val Gln
                85                  90                  95
Glu Lys Trp Ile Ser Glu Val Leu His Phe Cys Gln Gly Leu Pro Ile
            100                 105                 110
Val Leu Val Gly Cys Lys Lys Asp Leu Arg Asn Asp Pro Gly Thr Ile
        115                 120                 125
Glu Glu Leu Arg Arg Asn Ser Gln Lys Pro Val Ser Ser Glu Glu Gly
    130                 135                 140
Ala Ser Ile Ala Gln Arg Ile Ser Ala Tyr Lys Tyr Leu Glu Cys Ser
145                 150                 155                 160
Ala Lys Thr Gly Glu Gly Val Arg Glu Val Phe Glu His Ala Thr Arg
                165                 170                 175
Ala Ala Leu Met Val Ser Lys Lys Lys Lys Ser Lys Ser Gly Val Cys
            180                 185                 190
Asn Leu Leu
        195

<210> 249
<211> 1014
<212> DNA
<213> Neurospora crassa

<220>
<221> CDS
<222> (259)..(1014)

<400> 249
atgtcgtcgt caagcaagtt ccgcagctca caccactatc attcgcagtc ggtgtcgtca      60

atcctaggca ggcacgatag caccagcccc ggcgcagaac tccaacgacg accgaccacc     120

acctcgtcct acacatcctc tggtagcgcc agccgacgag taccacgcga caacgggacg     180

agatcgagcg atgggacagt gagtaccatg atgagcacca catcgtcgac cggaagagaa     240

tcagcagcaa ctacggcc atg acc gag ggc ccg gcc tac tcc aag aag gtg       291
                    Met Thr Glu Gly Pro Ala Tyr Ser Lys Lys Val
                    1               5                   10
gtg gtc gtg ggc gat ggc ggt tgc gga aag aca tgt ctc ctg atc agt       339
Val Val Val Gly Asp Gly Gly Cys Gly Lys Thr Cys Leu Leu Ile Ser
            15                  20                  25
tat agt cag gga tac ttc cca gag aaa tat gtc cca acc gtc ttt gag       387

Tyr Ser Gln Gly Tyr Phe Pro Glu Lys Tyr Val Pro Thr Val Phe Glu
        30                  35                  40
aac tac atc acc tac cca acg cat cca ccg acc ggt aag acc gtc gag 435
Asn Tyr Ile Thr Tyr Pro Thr His Pro Pro Thr Gly Lys Thr Val Glu
    45                  50                  55
ctc gcc ctg tgg gat acc gcc ggc caa gag gaa tac gac cgc ttg cga 483
Leu Ala Leu Trp Asp Thr Ala Gly Gln Glu Glu Tyr Asp Arg Leu Arg
60                  65                  70                  75
ccg ctt tca tac cca gaa acc gac ctt att ttt gtc tgc ttc gcc att 531
Pro Leu Ser Tyr Pro Glu Thr Asp Leu Ile Phe Val Cys Phe Ala Ile
                80                  85                  90
gac tgc ccc aac tcc ctc gag aat gtc atg gac aag tgg tac ccc gaa 579
Asp Cys Pro Asn Ser Leu Glu Asn Val Met Asp Lys Trp Tyr Pro Glu
            95                  100                 105
gtc ctc cac ttc tgt ccg tat aca ccc ctt atc ctc gtc ggc ctc aag 627
Val Leu His Phe Cys Pro Tyr Thr Pro Leu Ile Leu Val Gly Leu Lys
        110                 115                 120
tcc gac ctc cgc aat aag aag acg tgc atc gac atg ctc aag aca caa 675
Ser Asp Leu Arg Asn Lys Lys Thr Cys Ile Asp Met Leu Lys Thr Gln
    125                 130                 135
ggt ctc acc ccc gtc acc acc gaa caa gga ctc gcc gtc gct aag aag 723
Gly Leu Thr Pro Val Thr Thr Glu Gln Gly Leu Ala Val Ala Lys Lys
140                 145                 150                 155
atg ggc gct cag tac atg gag tgc tca tca aag gag atg aag ggt gta 771
Met Gly Ala Gln Tyr Met Glu Cys Ser Ser Lys Glu Met Lys Gly Val
                160                 165                 170
gag gag att ttt gag cag gcc atc ctc aca gta gtc gcc aac gac agg 819
Glu Glu Ile Phe Glu Gln Ala Ile Leu Thr Val Val Ala Asn Asp Arg
            175                 180                 185
aaa aca ctg gaa cag gaa gcc gcg aac ggc atg ctg ggt gtt ggc gcg 867
Lys Thr Leu Glu Gln Glu Ala Ala Asn Gly Met Leu Gly Val Gly Ala
        190                 195                 200
ggc tcg gga agc gga aag ggc agc gga atc tcg ttc agc agt ggt gac 915
Gly Ser Gly Ser Gly Lys Gly Ser Gly Ile Ser Phe Ser Ser Gly Asp
    205                 210                 215
aag gcc ggt tcc ggg ata ggg ccc gtc aag gcg gcc ggt gtg ggt ggc 963
Lys Ala Gly Ser Gly Ile Gly Pro Val Lys Ala Ala Gly Val Gly Gly
220                 225                 230                 235
acg att gtc ccg aaa acg agg aag aag aag aga aag tgt ggt atg atg 1011
Thr Ile Val Pro Lys Thr Arg Lys Lys Lys Arg Lys Cys Gly Met Met
                240                 245                 250
tga 1014

<210> 250
<211> 251
<212> PRT
<213> Neurospora crassa

<400> 250
Met Thr Glu Gly Pro Ala Tyr Ser Lys Lys Val Val Val Val Gly Asp
1               5                   10                  15
Gly Gly Cys Gly Lys Thr Cys Leu Leu Ile Ser Tyr Ser Gln Gly Tyr
            20                  25                  30
Phe Pro Glu Lys Tyr Val Pro Thr Val Phe Glu Asn Tyr Ile Thr Tyr
        35                  40                  45
Pro Thr His Pro Pro Thr Gly Lys Thr Val Glu Leu Ala Leu Trp Asp
    50                  55                  60
Thr Ala Gly Gln Glu Glu Tyr Asp Arg Leu Arg Pro Leu Ser Tyr Pro
65                  70                  75                  80
Glu Thr Asp Leu Ile Phe Val Cys Phe Ala Ile Asp Cys Pro Asn Ser
                85                  90                  95
Leu Glu Asn Val Met Asp Lys Trp Tyr Pro Glu Val Leu His Phe Cys
            100                 105                 110
Pro Tyr Thr Pro Leu Ile Leu Val Gly Leu Lys Ser Asp Leu Arg Asn
        115                 120                 125

Lys Lys Thr Cys Ile Asp Met Leu Lys Thr Gln Gly Leu Thr Pro Val
    130                 135                 140
Thr Thr Glu Gln Gly Leu Ala Val Ala Lys Lys Met Gly Ala Gln Tyr
145                 150                 155                 160
Met Glu Cys Ser Ser Lys Glu Met Lys Gly Val Glu Glu Ile Phe Glu
                165                 170                 175
Gln Ala Ile Leu Thr Val Val Ala Asn Asp Arg Lys Thr Leu Glu Gln
            180                 185                 190
Glu Ala Ala Asn Gly Met Leu Gly Val Gly Ala Gly Ser Gly Ser Gly
        195                 200                 205
Lys Gly Ser Gly Ile Ser Phe Ser Ser Gly Asp Lys Ala Gly Ser Gly
    210                 215                 220
Ile Gly Pro Val Lys Ala Ala Gly Val Gly Gly Thr Ile Val Pro Lys
225                 230                 235                 240
Thr Arg Lys Lys Lys Arg Lys Cys Gly Met Met
                245                 250

<210> 251
<211> 915
<212> DNA
<213> Oryza sativa

<220>
<221> CDS
<222> (85)..(678)

<400> 251
gggaccatca ctaccaccac ttcaccacca aacccaacca agaacaagtt ttcaggggca      60

aattaagaag agcttggtga cgat atg agc acg gcg agg ttc atc aag tgc        111
                           Met Ser Thr Ala Arg Phe Ile Lys Cys
                           1               5
gtc acc gtc ggc gac ggc gcc gtc ggc aag aca tgc atg ctc atc tcc       159
Val Thr Val Gly Asp Gly Ala Val Gly Lys Thr Cys Met Leu Ile Ser
10                  15                  20                  25
tac acc agc aac act ttc ccc acg gat tat gtg ccg acg gtt ttc gac       207
Tyr Thr Ser Asn Thr Phe Pro Thr Asp Tyr Val Pro Thr Val Phe Asp
                30                  35                  40
aac ttc agc gcc aac gtg gtc gtc gac ggc aac acg gtg aac ctt ggg       255
Asn Phe Ser Ala Asn Val Val Val Asp Gly Asn Thr Val Asn Leu Gly
            45                  50                  55
ctc tgg gat act gct gga caa gag gat tac aat agg ctg agg cct ctc       303
Leu Trp Asp Thr Ala Gly Gln Glu Asp Tyr Asn Arg Leu Arg Pro Leu
        60                  65                  70
agc tac aga gga gct gat gtc ttt tta cta gca ttt tcc ctc atc agc       351
Ser Tyr Arg Gly Ala Asp Val Phe Leu Leu Ala Phe Ser Leu Ile Ser
    75                  80                  85
aaa gcg agc tat gag aat att cac aag aag tgg ata cca gag ctg agg       399
Lys Ala Ser Tyr Glu Asn Ile His Lys Lys Trp Ile Pro Glu Leu Arg
90                  95                  100                 105
cat tat gct cct aat gtg cca atc gta tta gtt gga acc aag ctt gac       447
His Tyr Ala Pro Asn Val Pro Ile Val Leu Val Gly Thr Lys Leu Asp
                110                 115                 120
ttg cgt gag gac aag cag ttc ttc ctg gac cac cct ggt cta gca cct       495
Leu Arg Glu Asp Lys Gln Phe Phe Leu Asp His Pro Gly Leu Ala Pro
            125                 130                 135
att tcc act gca cag ggg gag gag ctg aag agg atg ata ggt gct gcg       543
Ile Ser Thr Ala Gln Gly Glu Glu Leu Lys Arg Met Ile Gly Ala Ala
        140                 145                 150
gcg tac atc gaa tgc agc tcc aag acg cag cag aat gtg aaa tca gta       591
Ala Tyr Ile Glu Cys Ser Ser Lys Thr Gln Gln Asn Val Lys Ser Val
    155                 160                 165
ttc gac tcg gcg atc aaa gtt gta ctt tgc ccg cca aag ccg aag aag       639
Phe Asp Ser Ala Ile Lys Val Val Leu Cys Pro Pro Lys Pro Lys Lys
170                 175                 180                 185
aag aac acc agg aag cag agg agt tgc tgg atc cta tgagtgataa            685
Lys Asn Thr Arg Lys Gln Arg Ser Cys Trp Ile Leu

                    190                    195
acagcaagat tggcgtattc cgcaggaaac ctgtattatt tttacagtgt gtgttgtgtt     745

atgtttgtgt gttgcagatt agatgcagtt tgggttacaa aatttgctgg gtctgaaata     805

ttcagctcag tgcttcttga gtactacctc tgatgattct tacaaaagtg ttctttggtt     865

ggttcagata tatatattat gattattata aaaaaaaaaa aaaaaaaaaa                  915

<210> 252
<211> 197
<212> PRT
<213> Oryza sativa

<400> 252
Met Ser Thr Ala Arg Phe Ile Lys Cys Val Thr Val Gly Asp Gly Ala
1               5                   10                  15
Val Gly Lys Thr Cys Met Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Asn Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Ile
                85                  90                  95
His Lys Lys Trp Ile Pro Glu Leu Arg His Tyr Ala Pro Asn Val Pro
            100                 105                 110
Ile Val Leu Val Gly Thr Lys Leu Asp Leu Arg Glu Asp Lys Gln Phe
        115                 120                 125
Phe Leu Asp His Pro Gly Leu Ala Pro Ile Ser Thr Ala Gln Gly Glu
    130                 135                 140
Glu Leu Lys Arg Met Ile Gly Ala Ala Ala Tyr Ile Glu Cys Ser Ser
145                 150                 155                 160
Lys Thr Gln Gln Asn Val Lys Ser Val Phe Asp Ser Ala Ile Lys Val
                165                 170                 175
Val Leu Cys Pro Pro Lys Pro Lys Lys Lys Asn Thr Arg Lys Gln Arg
            180                 185                 190
Ser Cys Trp Ile Leu
        195

<210> 253
<211> 1010
<212> DNA
<213> Zea mays

<220>
<221> CDS
<222> (74)..(718)

<400> 253
   cactccgtcc ctctcctcca ggaccgcccg ctcttatccc gtttctccct tctctgggct      60
   cggagaatcg gag atg gcg tcc agc gcc tct cgg ttc atc aag tgc gtc        109
                  Met Ala Ser Ser Ala Ser Arg Phe Ile Lys Cys Val
                  1               5                   10
   acg gtc ggc gac ggt gcc gtg ggc aag aca tgt atg ctc atc tgc tac        157
   Thr Val Gly Asp Gly Ala Val Gly Lys Thr Cys Met Leu Ile Cys Tyr
           15                  20                  25
   acc agc aac aag ttc ccc act gat tac ata ccc acg gtg ttc gac aat        205
   Thr Ser Asn Lys Phe Pro Thr Asp Tyr Ile Pro Thr Val Phe Asp Asn
   30 35 40
   ttc agt gca aat gtc gtt gtg gat ggc acc acg gtg aat ttg ggc ctg 253
   Phe Ser Ala Asn Val Val Val Asp Gly Thr Thr Val Asn Leu Gly Leu
   45 50 55 60
   tgg gat acc gca ggg cag gaa gat tac aac cga ctg agg cct cta agc 301
   Trp Asp Thr Ala Gly Gln Glu Asp Tyr Asn Arg Leu Arg Pro Leu Ser
   65 70 75
   tac cgt ggt gca gat gtt ttc gtg ctt gca ttc tca ctt gtg agt cga 349
   Tyr Arg Gly Ala Asp Val Phe Val Leu Ala Phe Ser Leu Val Ser Arg
   80 85 90
   gct agc tat gag aat atc atg aag aag tgg ata ccg gag ctt cag cat 397
   Ala Ser Tyr Glu Asn Ile Met Lys Lys Trp Ile Pro Glu Leu Gln His
   95 100 105
   tat gca cgt ggg gtg cct gtt gtg ttg gta ggc aca aaa ttt gat ctt 445
   Tyr Ala Arg Gly Val Pro Val Val Leu Val Gly Thr Lys Phe Asp Leu
   110 115 120
   cgt gaa gac aag cac tac ttg atg gac cat cct ggg ttg gtg cct gtt 493
   Arg Glu Asp Lys His Tyr Leu Met Asp His Pro Gly Leu Val Pro Val
   125 130 135 140
   acc aca gca caa ggg gag gaa ctt cgt aga caa att ggt gct atg tat 541
   Thr Thr Ala Gln Gly Glu Glu Leu Arg Arg Gln Ile Gly Ala Met Tyr
   145 150 155
   tac atc gaa tgc agt tcg aag aca cag cag aat gtc aaa gct gtg ttc 589
   Tyr Ile Glu Cys Ser Ser Lys Thr Gln Gln Asn Val Lys Ala Val Phe
   160 165 170
   gat gct gcc atc aag gtt gta atc cag cct cca act aaa cta aga gaa 637
   Asp Ala Ala Ile Lys Val Val Ile Gln Pro Pro Thr Lys Leu Arg Glu
   175 180 185
   aag aag aaa aag aaa tca cgc aag gga tgt tcg atg gtg aac atc tta 685
   Lys Lys Lys Lys Lys Ser Arg Lys Gly Cys Ser Met Val Asn Ile Leu
   190 195 200
   tct gga aga aaa atg cta tgc ttc aag tcc tgaatgatcg aagggggtct        735
   Ser Gly Arg Lys Met Leu Cys Phe Lys Ser
   205                 210
   tacctgaact aataccatga gtgtgacccc aagttcgcga agcttgaaat cttgatgcgc 795
   tcgttgcgca tgtatatttg cacctttggt tattaatcac tagaggtaga tgattgaaac 855
   taatctgctt aaccaatgtg cactgctggg cgctggcgtg gttagctata tcagttaggc 915
   agttcgacag aggtcaaaga gattgagatt ttgttctttc ttggcaatgt cacagctttt 975
   gtgaaaaaaa aaaaaaaaaa aaaaaaaaaa aaaaa 1010

<210> 254
<211> 214
<212> PRT
<213> Zea mays

<400> 254
Met Ala Ser Ser Ala Ser Arg Phe Ile Lys Cys Val Thr Val Gly Asp
1               5                   10                  15
Gly Ala Val Gly Lys Thr Cys Met Leu Ile Cys Tyr Thr Ser Asn Lys
            20                  25                  30
Phe Pro Thr Asp Tyr Ile Pro Thr Val Phe Asp Asn Phe Ser Ala Asn
        35                  40                  45
Val Val Val Asp Gly Thr Thr Val Asn Leu Gly Leu Trp Asp Thr Ala
    50                  55                  60
Gly Gln Glu Asp Tyr Asn Arg Leu Arg Pro Leu Ser Tyr Arg Gly Ala
65                  70                  75                  80

Asp Val Phe Val Leu Ala Phe Ser Leu Val Ser Arg Ala Ser Tyr Glu
                85                  90                  95
Asn Ile Met Lys Lys Trp Ile Pro Glu Leu Gln His Tyr Ala Arg Gly
            100                 105                 110
Val Pro Val Val Leu Val Gly Thr Lys Phe Asp Leu Arg Glu Asp Lys
        115                 120                 125
His Tyr Leu Met Asp His Pro Gly Leu Val Pro Val Thr Thr Ala Gln
    130                 135                 140
Gly Glu Glu Leu Arg Arg Gln Ile Gly Ala Met Tyr Tyr Ile Glu Cys
145                 150                 155                 160
Ser Ser Lys Thr Gln Gln Asn Val Lys Ala Val Phe Asp Ala Ala Ile
                165                 170                 175
Lys Val Val Ile Gln Pro Pro Thr Lys Leu Arg Glu Lys Lys Lys Lys
            180                 185                 190
Lys Ser Arg Lys Gly Cys Ser Met Val Asn Ile Leu Ser Gly Arg Lys
        195                 200                 205
Met Leu Cys Phe Lys Ser
    210

<210> 255
<211> 579
<212> DNA
<213> Schizophyllum commune

<220>
<221> CDS
<222> (1)..(579)

<400> 255
atg cag acc atc aag tgt gta gtt gtt gga gac ggt gcg gtt gga aag      48
Met Gln Thr Ile Lys Cys Val Val Val Gly Asp Gly Ala Val Gly Lys
1               5                   10                  15
acg tgc ctg ttg atc tcc tat acg acc aac aag ttc ccg agc gag tat      96
Thr Cys Leu Leu Ile Ser Tyr Thr Thr Asn Lys Phe Pro Ser Glu Tyr
            20                  25                  30
gtg ccc acc gtc ttc gac aat tat gcc gtg acc gtc atg atc ggc gag      144
Val Pro Thr Val Phe Asp Asn Tyr Ala Val Thr Val Met Ile Gly Glu
        35                  40                  45
gac ccc tat aca ctg ggt ctg ttt gat act gct ggc cag gag gat tac      192
Asp Pro Tyr Thr Leu Gly Leu Phe Asp Thr Ala Gly Gln Glu Asp Tyr
    50                  55                  60
gat cga ctc cgg ccg ttg tcg tac ccg cag acg gac gtg ttt ctt gtg      240
Asp Arg Leu Arg Pro Leu Ser Tyr Pro Gln Thr Asp Val Phe Leu Val
65                  70                  75                  80
tgc ttt agt gta aca tct cca gcc tcc ttc gag aac gta aag gag aaa      288
Cys Phe Ser Val Thr Ser Pro Ala Ser Phe Glu Asn Val Lys Glu Lys
                85                  90                  95
tgg ttc ccg gag gtt cgc cac cac tgt ccg ggc gtg ccc tgc ctc atc      336
Trp Phe Pro Glu Val Arg His His Cys Pro Gly Val Pro Cys Leu Ile
            100                 105                 110
gtc ggc acg caa atc gac ttg agg gac gac tcg cag gtg atc gag aag      384
Val Gly Thr Gln Ile Asp Leu Arg Asp Asp Ser Gln Val Ile Glu Lys
        115                 120                 125
ctg gcg cgg caa aag cag aga ccg gtc acg agc gac caa ggc gag cgg      432
Leu Ala Arg Gln Lys Gln Arg Pro Val Thr Ser Asp Gln Gly Glu Arg
    130                 135                 140
ctc gtt cgg gaa ctt ggc gcg gtc aag tac gtc gag tgc tca gca ctc      480
Leu Val Arg Glu Leu Gly Ala Val Lys Tyr Val Glu Cys Ser Ala Leu
145                 150                 155                 160
acg cag aag gga ttg aag aac gtg ttt gat gag gcc atc gtc gcc gcg      528
Thr Gln Lys Gly Leu Lys Asn Val Phe Asp Glu Ala Ile Val Ala Ala
                165                 170                 175
ctg gag cca ccc gtg gtg aag aag aag ggg ccg aaa tgt gtc atc ctt      576
Leu Glu Pro Pro Val Val Lys Lys Lys Gly Pro Lys Cys Val Ile Leu
            180                 185                 190
tga                                                                  579

<210> 256
<211> 192
<212> PRT
<213> Schizophyllum commune

<400> 256
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Glu Tyr
            20                  25                  30
Val Pro Thr Val Phe Asp Asn Tyr Ala Val Thr Val Met Ile Gly Glu
        35                  40                  45
Asp Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Thr Ser Pro Ala Ser Phe Glu Asn Val Lys Glu Lys
                85                  90                  95
Trp Phe Pro Glu Val Arg His His Cys Pro Gly Val Pro Cys Leu Ile
            100                 105                 110
Val Gly Thr Gln Ile Asp Leu Arg Asp Asp Ser Gln Val Ile Glu Lys
        115                 120                 125
Leu Ala Arg Gln Lys Gln Arg Pro Val Thr Ser Asp Gln Gly Glu Arg
    130                 135                 140
Leu Val Arg Glu Leu Gly Ala Val Lys Tyr Val Glu Cys Ser Ala Leu
145                 150                 155                 160
Thr Gln Lys Gly Leu Lys Asn Val Phe Asp Glu Ala Ile Val Ala Ala
                165                 170                 175
Leu Glu Pro Pro Val Val Lys Lys Lys Gly Pro Lys Cys Val Ile Leu
            180                 185                 190

<210> 257
<211> 1075
<212> DNA
<213> Aspergillus fumigatus

<220>
<221> CDS
<222> (197)..(793)

<400> 257
ttaacctcca aaagatatac ccttaaagat tattatcgcc ccatccgcct tcacttgacg      60

ctaaaccccc ccaagaattc acttcgacgc taattgatcg ctatcatttg ggttcatctt     120

tacaagctgt tcatcatcaa atcctctctg tgattttctt ctctgtgcat atattcttaa     180

ttccaacaac aaaaac atg gcg tcc ggc ccc gct act cag tca tta aag tgt     232
                  Met Ala Ser Gly Pro Ala Thr Gln Ser Leu Lys Cys
                  1               5                   10
gtg gtg aca ggt gat ggt gca gtt gga aag aca tgc ctc ctc atc tcg       280
Val Val Thr Gly Asp Gly Ala Val Gly Lys Thr Cys Leu Leu Ile Ser
        15                  20                  25
tat acc acc aat gca ttc ccc gga gaa tac atc cct acc gta ttt gac       328
Tyr Thr Thr Asn Ala Phe Pro Gly Glu Tyr Ile Pro Thr Val Phe Asp
    30                  35                  40
aac tat acg gct agt gtg atg gta gat ggt agg ccc atc agc ttg gga       376
Asn Tyr Thr Ala Ser Val Met Val Asp Gly Arg Pro Ile Ser Leu Gly
45                  50                  55                  60
ctg tgg gat act gct ggg caa gaa gat tat gac cga ctg aga ccg ctg       424
Leu Trp Asp Thr Ala Gly Gln Glu Asp Tyr Asp Arg Leu Arg Pro Leu
                65                  70                  75
tcc tac cct caa acc gac gtc ttc ctc atc tgc ttc tcc att gtc agc       472

Ser Tyr Pro Gln Thr Asp Val Phe Leu Ile Cys Phe Ser Ile Val Ser
            80                  85                  90
cct cct tcg ttt gac aac gtc aag gca aag tgg tac ccg gag att gag 520
Pro Pro Ser Phe Asp Asn Val Lys Ala Lys Trp Tyr Pro Glu Ile Glu
        95                  100                 105
cac cat gcc ccc aat gtt ccc atc atc ctt gtt ggt acc aaa ctc gat 568
His His Ala Pro Asn Val Pro Ile Ile Leu Val Gly Thr Lys Leu Asp
    110                 115                 120
ctg aga gac gat ccc gcg aca gca gaa tcc ctc cgg cag aaa aag atg 616
Leu Arg Asp Asp Pro Ala Thr Ala Glu Ser Leu Arg Gln Lys Lys Met
125                 130                 135                 140
gac ctg tct cgt acg aga cac tgg ccg tcg cca aaa gag atc cga gcg 664
Asp Leu Ser Arg Thr Arg His Trp Pro Ser Pro Lys Glu Ile Arg Ala
                145                 150                 155
cac aag tat ctc gaa tgt tct gcc ctc agg cag cgc aac ttc aaa agc 712
His Lys Tyr Leu Glu Cys Ser Ala Leu Arg Gln Arg Asn Phe Lys Ser
            160                 165                 170
gtc ttt gat gaa gcc att cgt gct gtc ctc aac cct gga cct gcc gca 760
Val Phe Asp Glu Ala Ile Arg Ala Val Leu Asn Pro Gly Pro Ala Ala
        175                 180                 185
aaa ccg aag agc aag aaa tgc acc ata ctg tagaccatta ctttcagctt 810
Lys Pro Lys Ser Lys Lys Cys Thr Ile Leu
    190                 195
ttcatcatct aatcataaac aactaattcg gcgtctggga tagttgaagg ttttgcaatg 870

atccccttga tgattgttcc aactgtgttc atatttcttt ctctctttct cggtcacatg 930

tcatgatcct gatgagcttc tttctgggtc aggacacccc tttcctcatc ttgtcgtctt 990

tttgtgcaca tgcagcataa acttgtaaca tgctacgcca taacaggaag ctgtcactta 1050

atcgatttaa cctccattgt cctaa 1075

<210> 258
<211> 198
<212> PRT
<213> Aspergillus fumigatus

<400> 258
Met Ala Ser Gly Pro Ala Thr Gln Ser Leu Lys Cys Val Val Thr Gly
1               5                   10                  15
Asp Gly Ala Val Gly Lys Thr Cys Leu Leu Ile Ser Tyr Thr Thr Asn
            20                  25                  30
Ala Phe Pro Gly Glu Tyr Ile Pro Thr Val Phe Asp Asn Tyr Thr Ala
        35                  40                  45
Ser Val Met Val Asp Gly Arg Pro Ile Ser Leu Gly Leu Trp Asp Thr
    50                  55                  60
Ala Gly Gln Glu Asp Tyr Asp Arg Leu Arg Pro Leu Ser Tyr Pro Gln
65                  70                  75                  80
Thr Asp Val Phe Leu Ile Cys Phe Ser Ile Val Ser Pro Pro Ser Phe
                85                  90                  95
Asp Asn Val Lys Ala Lys Trp Tyr Pro Glu Ile Glu His His Ala Pro
            100                 105                 110
Asn Val Pro Ile Ile Leu Val Gly Thr Lys Leu Asp Leu Arg Asp Asp
        115                 120                 125
Pro Ala Thr Ala Glu Ser Leu Arg Gln Lys Lys Met Asp Leu Ser Arg
    130                 135                 140
Thr Arg His Trp Pro Ser Pro Lys Glu Ile Arg Ala His Lys Tyr Leu
145                 150                 155                 160
Glu Cys Ser Ala Leu Arg Gln Arg Asn Phe Lys Ser Val Phe Asp Glu
                165                 170                 175

Ala Ile Arg Ala Val Leu Asn Pro Gly Pro Ala Ala Lys Pro Lys Ser
            180                 185                 190
Lys Lys Cys Thr Ile Leu
        195

<210> 259
<211> 594
<212> DNA
<213> Aspergillus fumigatus

<220>
<221> CDS
<222> (1)..(594)

<400> 259
atg gag ctg tgc ggg cgc cag aaa gtt gtc cag cgc aag atg gta ctc      48
Met Glu Leu Cys Gly Arg Gln Lys Val Val Gln Arg Lys Met Val Leu
1               5                   10                  15
tta gga gat ggt gct tgc ggc aag acg tca gct ttg aat gtg ttt aca      96
Leu Gly Asp Gly Ala Cys Gly Lys Thr Ser Ala Leu Asn Val Phe Thr
            20                  25                  30
aga ggg ttc ttc ccg aca gtc tat gag ccg act gtt ttt gaa aac tat      144
Arg Gly Phe Phe Pro Thr Val Tyr Glu Pro Thr Val Phe Glu Asn Tyr
        35                  40                  45
gtc cat gac att ttc gtc gat aac gta cac atg gag ttg tcg ctg tgg      192
Val His Asp Ile Phe Val Asp Asn Val His Met Glu Leu Ser Leu Trp
    50                  55                  60
gat aca gcc ggt caa gaa gaa ttc gat cga tta cga gca ctg tcc tac      240
Asp Thr Ala Gly Gln Glu Glu Phe Asp Arg Leu Arg Ala Leu Ser Tyr
65                  70                  75                  80
gag gat aca cat gtt att atg cta tgt ttc agc gtc gat agc cct gac      288
Glu Asp Thr His Val Ile Met Leu Cys Phe Ser Val Asp Ser Pro Asp
                85                  90                  95
tcg ttc gaa aat gtg gcg acg aaa tgg att gat gag att cgc gag aat      336
Ser Phe Glu Asn Val Ala Thr Lys Trp Ile Asp Glu Ile Arg Glu Asn
            100                 105                 110
tgc ccc ggc gtg aag tta gtc ctc acg gca ctc aaa tgc gat ctg cga      384
Cys Pro Gly Val Lys Leu Val Leu Thr Ala Leu Lys Cys Asp Leu Arg
        115                 120                 125
aaa gac gac gag ttg aac gac aac ccg aac gcc atc acg ttc gaa caa      432
Lys Asp Asp Glu Leu Asn Asp Asn Pro Asn Ala Ile Thr Phe Glu Gln
    130                 135                 140
gga tta gcg aaa gca aag gaa atc ggc gct gta aaa tac ctt gaa tgc      480
Gly Leu Ala Lys Ala Lys Glu Ile Gly Ala Val Lys Tyr Leu Glu Cys
145                 150                 155                 160
tct gct gtt cag aat cgc ggt atc agg gag acc ttt tat gaa gcc gcc      528
Ser Ala Val Gln Asn Arg Gly Ile Arg Glu Thr Phe Tyr Glu Ala Ala
                165                 170                 175
aag gtc gct ctt gat gtg aag cct gca gga tcc agc ggg tcc aag gga      576
Lys Val Ala Leu Asp Val Lys Pro Ala Gly Ser Ser Gly Ser Lys Gly
            180                 185                 190
cag tgc att atc ctc tga                                              594
Gln Cys Ile Ile Leu
        195


<210> 260
<211> 197
<212> PRT
<213> Aspergillus fumigatus

<400> 260
Met Glu Leu Cys Gly Arg Gln Lys Val Val Gln Arg Lys Met Val Leu
1               5                   10                  15
Leu Gly Asp Gly Ala Cys Gly Lys Thr Ser Ala Leu Asn Val Phe Thr
            20                  25                  30
Arg Gly Phe Phe Pro Thr Val Tyr Glu Pro Thr Val Phe Glu Asn Tyr
        35                  40                  45

Val His Asp Ile Phe Val Asp Asn Val His Met Glu Leu Ser Leu Trp
    50                  55                  60
Asp Thr Ala Gly Gln Glu Glu Phe Asp Arg Leu Arg Ala Leu Ser Tyr
65                  70                  75                  80
Glu Asp Thr His Val Ile Met Leu Cys Phe Ser Val Asp Ser Pro Asp
                85                  90                  95
Ser Phe Glu Asn Val Ala Thr Lys Trp Ile Asp Glu Ile Arg Glu Asn
            100                 105                 110
Cys Pro Gly Val Lys Leu Val Leu Thr Ala Leu Lys Cys Asp Leu Arg
        115                 120                 125
Lys Asp Asp Glu Leu Asn Asp Asn Pro Asn Ala Ile Thr Phe Glu Gln
    130                 135                 140
Gly Leu Ala Lys Ala Lys Glu Ile Gly Ala Val Lys Tyr Leu Glu Cys
145                 150                 155                 160
Ser Ala Val Gln Asn Arg Gly Ile Arg Glu Thr Phe Tyr Glu Ala Ala
                165                 170                 175
Lys Val Ala Leu Asp Val Lys Pro Ala Gly Ser Ser Gly Ser Lys Gly
            180                 185                 190
Gln Cys Ile Ile Leu
        195

<210> 261
<211> 582
<212> DNA
<213> Mus musculus

<220>
<221> CDS
<222> (1)..(582)

<400> 261
atg gct gcc atc cgg aag aaa ctg gtg atc gtg gga gat gga gct tgt      48
Met Ala Ala Ile Arg Lys Lys Leu Val Ile Val Gly Asp Gly Ala Cys
1               5                   10                  15
gga aaa aca tgt ttg ctc atc gtc ttc agc aag gac cag ttt cct gag      96
Gly Lys Thr Cys Leu Leu Ile Val Phe Ser Lys Asp Gln Phe Pro Glu
            20                  25                  30
gtt tac gtg ccc aca gta ttt gag aac tat gtg gct gat atc gaa gtg      144
Val Tyr Val Pro Thr Val Phe Glu Asn Tyr Val Ala Asp Ile Glu Val
        35                  40                  45
gat gga aaa cag gtg gag ttg gcc ctg tgg gat aca gct gga caa gaa      192
Asp Gly Lys Gln Val Glu Leu Ala Leu Trp Asp Thr Ala Gly Gln Glu
    50                  55                  60
gat tat gat cgc ctg agg cca ctc tcc tat ccc gac act gat gtt ctc      240
Asp Tyr Asp Arg Leu Arg Pro Leu Ser Tyr Pro Asp Thr Asp Val Leu
65                  70                  75                  80
ttg tta tgt ttc tcc att ggc aac cct gat agc ttt ggg aac atc cca      288
Leu Leu Cys Phe Ser Ile Gly Asn Pro Asp Ser Phe Gly Asn Ile Pro
                85                  90                  95
cat aaa tgg att cca gaa gtc aag cat ttc tgt ccc aac gtg ccc atc      336
His Lys Trp Ile Pro Glu Val Lys His Phe Cys Pro Asn Val Pro Ile
            100                 105                 110
atc ctg gtt ggg agt aag aag gat ctt cgg aat gac ttc tac acg ata      384
Ile Leu Val Gly Ser Lys Lys Asp Leu Arg Asn Asp Phe Tyr Thr Ile
        115                 120                 125
caa gag tta gcc aag agg aag caa gcg cct gtg aga cct gaa caa ggc      432
Gln Glu Leu Ala Lys Arg Lys Gln Ala Pro Val Arg Pro Glu Gln Gly
    130                 135                 140
caa ggg ttg gcg aac agc att ggc gct ttc gag tat gtg gag tgt tca      480
Gln Gly Leu Ala Asn Ser Ile Gly Ala Phe Glu Tyr Val Glu Cys Ser
145                 150                 155                 160
gcg aag acc aaa gat gga gtg agg agg gtc ttt gaa aag gcc aca agg      528
Ala Lys Thr Lys Asp Gly Val Arg Arg Val Phe Glu Lys Ala Thr Arg
                165                 170                 175
gct gct ctg caa acg aat cga gtg aag aaa aag act ggt tgc ttt gtc      576
Ala Ala Leu Gln Thr Asn Arg Val Lys Lys Lys Thr Gly Cys Phe Val
            180                 185                 190
ttt tga                                                              582

Phe

<210> 262
<211> 193
<212> PRT
<213> Mus musculus

<400> 262
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Leu
65                  70                  75                  80
Leu Leu Cys Phe Ser Ile Gly Asn Pro Asp Ser Phe Gly Asn Ile Pro
                85                  90                  95
His Lys Trp Ile Pro Glu Val Lys His Phe Cys Pro Asn Val Pro Ile
            100                 105                 110
Ile Leu Val Gly Ser Lys Lys Asp Leu Arg Asn Asp Phe Tyr Thr Ile
        115                 120                 125
Gln Glu Leu Ala Lys Arg Lys Gln Ala Pro Val Arg Pro Glu Gln Gly
    130                 135                 140
Gln Gly Leu Ala Asn Ser Ile Gly Ala Phe Glu Tyr Val Glu Cys Ser
145                 150                 155                 160
Ala Lys Thr Lys Asp Gly Val Arg Arg Val Phe Glu Lys Ala Thr Arg
                165                 170                 175
Ala Ala Leu Gln Thr Asn Arg Val Lys Lys Lys Thr Gly Cys Phe Val
            180                 185                 190
Phe

<210> 263
<211> 576
<212> DNA
<213> Mus musculus

<220>
<221> CDS
<222> (1)..(576)

<400> 263
atg ctg agc tca atc aag tgc gtg ctg gta ggg gac agt gct gtg ggg      48
Met Leu Ser Ser Ile Lys Cys Val Leu Val Gly Asp Ser Ala Val Gly
1               5                   10                  15
aaa acc tca ctg ttg gtg cgc ttc acc tct gag acc ttc ccg gag gcc      96
Lys Thr Ser Leu Leu Val Arg Phe Thr Ser Glu Thr Phe Pro Glu Ala
            20                  25                  30
tac aaa ccc acg gtg tac gag aat acg ggt gta gac gtc ttc atg gat      144
Tyr Lys Pro Thr Val Tyr Glu Asn Thr Gly Val Asp Val Phe Met Asp
        35                  40                  45
ggc atc cag atc agc ctg ggt ctc tgg gac act gcc ggc aac gac gcc      192
Gly Ile Gln Ile Ser Leu Gly Leu Trp Asp Thr Ala Gly Asn Asp Ala
    50                  55                  60
ttc aga agt atc cgg ccc ctg tcc tac cag cag gca gac gtg gta ctg      240
Phe Arg Ser Ile Arg Pro Leu Ser Tyr Gln Gln Ala Asp Val Val Leu
65                  70                  75                  80
atg tgc tac tct gtg gcc aac cat aac tcg ttc ctg aac ttg aag aac      288
Met Cys Tyr Ser Val Ala Asn His Asn Ser Phe Leu Asn Leu Lys Asn
                85                  90                  95
aaa tgg att agt gag atc agg agc aac cta ccc tgt acc ccg gtg ctg      336
Lys Trp Ile Ser Glu Ile Arg Ser Asn Leu Pro Cys Thr Pro Val Leu
            100                 105                 110

gtt gtg gcc aca cag acg gac cag aga gag gtg gga cct cac agg gct 384
Val Val Ala Thr Gln Thr Asp Gln Arg Glu Val Gly Pro His Arg Ala
        115                 120                 125
tcc tgc atc aat gcc ata gaa ggg aag aga ctt gcc cag gat gtg aga 432
Ser Cys Ile Asn Ala Ile Glu Gly Lys Arg Leu Ala Gln Asp Val Arg
    130                 135                 140
gcc aag ggc tac ctg gag tgc tca gcc ctc agc aac cgg gga gta cag 480
Ala Lys Gly Tyr Leu Glu Cys Ser Ala Leu Ser Asn Arg Gly Val Gln
145                 150                 155                 160
cag gta ttt gaa tgt gct gtc cga aca gct gtc aac cag gcc agg agg 528
Gln Val Phe Glu Cys Ala Val Arg Thr Ala Val Asn Gln Ala Arg Arg
                165                 170                 175
cga aac aga agg aag ctg ttc tcc atc aat gaa tgc aag atc ttc 573
Arg Asn Arg Arg Lys Leu Phe Ser Ile Asn Glu Cys Lys Ile Phe
            180                 185                 190
taa 576

<210> 264
<211> 191
<212> PRT
<213> Mus musculus

<400> 264
Met Leu Ser Ser Ile Lys Cys Val Leu Val Gly Asp Ser Ala Val Gly
1               5                   10                  15
Lys Thr Ser Leu Leu Val Arg Phe Thr Ser Glu Thr Phe Pro Glu Ala
            20                  25                  30
Tyr Lys Pro Thr Val Tyr Glu Asn Thr Gly Val Asp Val Phe Met Asp
        35                  40                  45
Gly Ile Gln Ile Ser Leu Gly Leu Trp Asp Thr Ala Gly Asn Asp Ala
    50                  55                  60
Phe Arg Ser Ile Arg Pro Leu Ser Tyr Gln Gln Ala Asp Val Val Leu
65                  70                  75                  80
Met Cys Tyr Ser Val Ala Asn His Asn Ser Phe Leu Asn Leu Lys Asn
                85                  90                  95
Lys Trp Ile Ser Glu Ile Arg Ser Asn Leu Pro Cys Thr Pro Val Leu
            100                 105                 110
Val Val Ala Thr Gln Thr Asp Gln Arg Glu Val Gly Pro His Arg Ala
        115                 120                 125
Ser Cys Ile Asn Ala Ile Glu Gly Lys Arg Leu Ala Gln Asp Val Arg
    130                 135                 140
Ala Lys Gly Tyr Leu Glu Cys Ser Ala Leu Ser Asn Arg Gly Val Gln
145                 150                 155                 160
Gln Val Phe Glu Cys Ala Val Arg Thr Ala Val Asn Gln Ala Arg Arg
                165                 170                 175
Arg Asn Arg Arg Lys Leu Phe Ser Ile Asn Glu Cys Lys Ile Phe
            180                 185                 190

<210> 265
<211> 786
<212> DNA
<213> Mus musculus

<220>
<221> CDS
<222> (1)..(786)

<400> 265
atg gcc ccg cag caa ggc cgg ccg gcg ctg ccc gcc cgc tgc gag ccg 48
Met Ala Pro Gln Gln Gly Arg Pro Ala Leu Pro Ala Arg Cys Glu Pro
1               5                   10                  15
ccg gcg gcg ccg ccg gta ccg cct cgc cga gag cgc ggg ggg cgc ggg 96
Pro Ala Ala Pro Pro Val Pro Pro Arg Arg Glu Arg Gly Gly Arg Gly
            20                  25                  30
gcg cgc ggg ccc ggg gtg tcc ggg ggt cgg ggg cgc gcg ggc ggc gcc 144

Ala Arg Gly Pro Gly Val Ser Gly Gly Arg Gly Arg Ala Gly Gly Ala
        35                  40                  45
gag gga cgc ggc gtc aag tgc gtg ctg gtc ggc gac ggc gcg gtg ggc      192
Glu Gly Arg Gly Val Lys Cys Val Leu Val Gly Asp Gly Ala Val Gly
    50                  55                  60
aag acc agc ctg gtg gtc agc tac acc act aac ggc tac ccc acc gag      240
Lys Thr Ser Leu Val Val Ser Tyr Thr Thr Asn Gly Tyr Pro Thr Glu
65                  70                  75                  80
tac atc cct acg gcc ttc gac aac ttc tcg gcc gtg gtg tct gta gat      288
Tyr Ile Pro Thr Ala Phe Asp Asn Phe Ser Ala Val Val Ser Val Asp
                85                  90                  95
ggg cgg cct gtg aga ctc cag ctc tgt gac act gca gga cag gat gag      336
Gly Arg Pro Val Arg Leu Gln Leu Cys Asp Thr Ala Gly Gln Asp Glu
            100                 105                 110
ttt gac aag ctg agg ccc ctc tgc tac acc aac aca gac atc ttc ctg      384
Phe Asp Lys Leu Arg Pro Leu Cys Tyr Thr Asn Thr Asp Ile Phe Leu
        115                 120                 125
ctg tgc ttc agc gtg gtg agc ccc aca tcc ttc cag aac gtg ggc gag      432
Leu Cys Phe Ser Val Val Ser Pro Thr Ser Phe Gln Asn Val Gly Glu
    130                 135                 140
aag tgg gtt cca gag att cga cgt cac tgc cca aag gcc ccc atc atc      480
Lys Trp Val Pro Glu Ile Arg Arg His Cys Pro Lys Ala Pro Ile Ile
145                 150                 155                 160
ctg gtc ggg aca cag tcg gac ctc agg gag gac gtc aaa gtg ctc ata      528
Leu Val Gly Thr Gln Ser Asp Leu Arg Glu Asp Val Lys Val Leu Ile
                165                 170                 175
gaa ctg gac aag tgc aaa gag aag ccg gtg cct gaa gag gcg gcg aag      576
Glu Leu Asp Lys Cys Lys Glu Lys Pro Val Pro Glu Glu Ala Ala Lys
            180                 185                 190
ctg tgc gcg gag gaa gtc aaa gct gtc tcc tac atc gag tgc tca gcg      624
Leu Cys Ala Glu Glu Val Lys Ala Val Ser Tyr Ile Glu Cys Ser Ala
        195                 200                 205
ttg act cag aaa aac ctc aaa gag gtt ttc gac gcc gcc att gtt gct      672
Leu Thr Gln Lys Asn Leu Lys Glu Val Phe Asp Ala Ala Ile Val Ala
    210                 215                 220
ggt atc cag cac tca gac tcc cag cta cag cca aag aag tct aaa agc      720
Gly Ile Gln His Ser Asp Ser Gln Leu Gln Pro Lys Lys Ser Lys Ser
225                 230                 235                 240
agg acc ccg gat aag gtg cgg gac ctg tcc aag tct tgg tgg agg aag      768
Arg Thr Pro Asp Lys Val Arg Asp Leu Ser Lys Ser Trp Trp Arg Lys
                245                 250                 255
tat tgc tgc ctg gcc tga                                              786
Tyr Cys Cys Leu Ala
            260

<210> 266
<211> 261
<212> PRT
<213> Mus musculus

<400> 266
Met Ala Pro Gln Gln Gly Arg Pro Ala Leu Pro Ala Arg Cys Glu Pro
1               5                   10                  15
Pro Ala Ala Pro Pro Val Pro Pro Arg Arg Glu Arg Gly Gly Arg Gly
            20                  25                  30
Ala Arg Gly Pro Gly Val Ser Gly Gly Arg Gly Arg Ala Gly Gly Ala
        35                  40                  45
Glu Gly Arg Gly Val Lys Cys Val Leu Val Gly Asp Gly Ala Val Gly
    50                  55                  60
Lys Thr Ser Leu Val Val Ser Tyr Thr Thr Asn Gly Tyr Pro Thr Glu
65                  70                  75                  80
Tyr Ile Pro Thr Ala Phe Asp Asn Phe Ser Ala Val Val Ser Val Asp
                85                  90                  95
Gly Arg Pro Val Arg Leu Gln Leu Cys Asp Thr Ala Gly Gln Asp Glu
            100                 105                 110
Phe Asp Lys Leu Arg Pro Leu Cys Tyr Thr Asn Thr Asp Ile Phe Leu
        115                 120                 125

Leu Cys Phe Ser Val Val Ser Pro Thr Ser Phe Gln Asn Val Gly Glu
    130                 135                 140
Lys Trp Val Pro Glu Ile Arg Arg His Cys Pro Lys Ala Pro Ile Ile
145                 150                 155                 160
Leu Val Gly Thr Gln Ser Asp Leu Arg Glu Asp Val Lys Val Leu Ile
                165                 170                 175
Glu Leu Asp Lys Cys Lys Glu Lys Pro Val Pro Glu Glu Ala Ala Lys
            180                 185                 190
Leu Cys Ala Glu Glu Val Lys Ala Val Ser Tyr Ile Glu Cys Ser Ala
        195                 200                 205
Leu Thr Gln Lys Asn Leu Lys Glu Val Phe Asp Ala Ala Ile Val Ala
    210                 215                 220
Gly Ile Gln His Ser Asp Ser Gln Leu Gln Pro Lys Lys Ser Lys Ser
225                 230                 235                 240
Arg Thr Pro Asp Lys Val Arg Asp Leu Ser Lys Ser Trp Trp Arg Lys
                245                 250                 255
Tyr Cys Cys Leu Ala
            260

<210> 267
<211> 624
<212> DNA
<213> Trichoderma reesei

<220>
<221> CDS
<222> (1)..(624)

<400> 267
atg cct ctc tgc ggc ggc tcc aag acg gtg cag cgc aag ctg gtt ctt      48
Met Pro Leu Cys Gly Gly Ser Lys Thr Val Gln Arg Lys Leu Val Leu
1               5                   10                  15
ctg ggc gat ggt gcc agc gga aag acg tcg ctg ctc aac gtc ttc aca      96
Leu Gly Asp Gly Ala Ser Gly Lys Thr Ser Leu Leu Asn Val Phe Thr
            20                  25                  30
aga ggt tac ttt ccc acc gtc tac gaa cct acc gtc ttt gaa aat tac      144
Arg Gly Tyr Phe Pro Thr Val Tyr Glu Pro Thr Val Phe Glu Asn Tyr
        35                  40                  45
gtc cac gac atc ttt gtc gac aac gtc cac atc gag ctc tcc ctc tgg      192
Val His Asp Ile Phe Val Asp Asn Val His Ile Glu Leu Ser Leu Trp
    50                  55                  60
gat acg gcg gga cag gag gaa ttc gat cgg ctg cga tcg ctc tcc tac      240
Asp Thr Ala Gly Gln Glu Glu Phe Asp Arg Leu Arg Ser Leu Ser Tyr
65                  70                  75                  80
gat gac acc gat ttg atc gtg ctc tgt tac tcg gtc gat agc aaa gac      288
Asp Asp Thr Asp Leu Ile Val Leu Cys Tyr Ser Val Asp Ser Lys Asp
                85                  90                  95
tcg cta gaa aac gtc gaa tcc aaa tgg gtc gga gag att gcc gac aac      336
Ser Leu Glu Asn Val Glu Ser Lys Trp Val Gly Glu Ile Ala Asp Asn
            100                 105                 110
tgc ccc ggc gtc aag ctg gtc ctc gtc gcc ctc aag tgc gac ctg cgc      384
Cys Pro Gly Val Lys Leu Val Leu Val Ala Leu Lys Cys Asp Leu Arg
        115                 120                 125
cag caa gag gac gac gag ccc gag gac cag gca gcg gcc gac ggc aac      432
Gln Gln Glu Asp Asp Glu Pro Glu Asp Gln Ala Ala Ala Asp Gly Asn
    130                 135                 140
gca cag cgc gag aaa ccg ccc acg att tcc tac gac gag ggc ctt gag      480
Ala Gln Arg Glu Lys Pro Pro Thr Ile Ser Tyr Asp Glu Gly Leu Glu
145                 150                 155                 160
gtc gcc aag cgg ata ggc gcc tcg cgc tac ctg gag tgc tcg gcg atg      528
Val Ala Lys Arg Ile Gly Ala Ser Arg Tyr Leu Glu Cys Ser Ala Met
                165                 170                 175
aag aac cgc ggc gtc aac gag gcc ttt acc gag gcg gcc cgc gta gcg      576
Lys Asn Arg Gly Val Asn Glu Ala Phe Thr Glu Ala Ala Arg Val Ala
            180                 185                 190
cta agc gtc aag aag gag agg gaa gac aac aag tgc aca atc atg          621
Leu Ser Val Lys Lys Glu Arg Glu Asp Asn Lys Cys Thr Ile Met
        195                 200                 205

taa                                                                 624

<210> 268
<211> 207
<212> PRT
<213> Trichoderma reesei

<400> 268
Met Pro Leu Cys Gly Gly Ser Lys Thr Val Gln Arg Lys Leu Val Leu
1               5                   10                  15
Leu Gly Asp Gly Ala Ser Gly Lys Thr Ser Leu Leu Asn Val Phe Thr
            20                  25                  30
Arg Gly Tyr Phe Pro Thr Val Tyr Glu Pro Thr Val Phe Glu Asn Tyr
        35                  40                  45
Val His Asp Ile Phe Val Asp Asn Val His Ile Glu Leu Ser Leu Trp
    50                  55                  60
Asp Thr Ala Gly Gln Glu Glu Phe Asp Arg Leu Arg Ser Leu Ser Tyr
65                  70                  75                  80
Asp Asp Thr Asp Leu Ile Val Leu Cys Tyr Ser Val Asp Ser Lys Asp
                85                  90                  95
Ser Leu Glu Asn Val Glu Ser Lys Trp Val Gly Glu Ile Ala Asp Asn
            100                 105                 110
Cys Pro Gly Val Lys Leu Val Leu Val Ala Leu Lys Cys Asp Leu Arg
        115                 120                 125
Gln Gln Glu Asp Asp Glu Pro Glu Asp Gln Ala Ala Ala Asp Gly Asn
    130                 135                 140
Ala Gln Arg Glu Lys Pro Pro Thr Ile Ser Tyr Asp Glu Gly Leu Glu
145                 150                 155                 160
Val Ala Lys Arg Ile Gly Ala Ser Arg Tyr Leu Glu Cys Ser Ala Met
                165                 170                 175
Lys Asn Arg Gly Val Asn Glu Ala Phe Thr Glu Ala Ala Arg Val Ala
            180                 185                 190
Leu Ser Val Lys Lys Glu Arg Glu Asp Asn Lys Cys Thr Ile Met
        195                 200                 205

<210> 269
<211> 675
<212> DNA
<213> Ashbya gossypii

<220>
<221> CDS
<222> (1)..(675)

<400> 269
atg cct ctg tgt ggg tcg agc tcg tcg tcg aag cat cct atc gag cgc      48
Met Pro Leu Cys Gly Ser Ser Ser Ser Ser Lys His Pro Ile Glu Arg
1               5                   10                  15
aag atc gtc atc ctc gga gac ggt gct tgc ggg aag acg tcg ctg ttg      96
Lys Ile Val Ile Leu Gly Asp Gly Ala Cys Gly Lys Thr Ser Leu Leu
            20                  25                  30
aac gtg ttc acg cga ggg tac ttt ccg aag gtg tac gag ccc acg gta      144
Asn Val Phe Thr Arg Gly Tyr Phe Pro Lys Val Tyr Glu Pro Thr Val
        35                  40                  45
ttc gaa aac tac atc cat gac atc ttc gtg gac aac cag cac atc acg      192
Phe Glu Asn Tyr Ile His Asp Ile Phe Val Asp Asn Gln His Ile Thr
    50                  55                  60
ctg agc ctg tgg gac act gct ggg cag gag gag ttt gac cgg ttg cga      240
Leu Ser Leu Trp Asp Thr Ala Gly Gln Glu Glu Phe Asp Arg Leu Arg
65                  70                  75                  80
tcg ctg tcg tac tcg gac aca cac acg att atg ctg tgt ttc tcg gtg      288
Ser Leu Ser Tyr Ser Asp Thr His Thr Ile Met Leu Cys Phe Ser Val
                85                  90                  95
gac tcg cgg gac tcg ctg gag aac gtc aag aac aag tgg gtg agc gaa      336
Asp Ser Arg Asp Ser Leu Glu Asn Val Lys Asn Lys Trp Val Ser Glu

                100                 105                 110
att gcg gac cac tgc gag ggc gtg aag ctg gtg cta gtg gcg ctg aag        384
Ile Ala Asp His Cys Glu Gly Val Lys Leu Val Leu Val Ala Leu Lys
        115                 120                 125
tgc gac ttg cgc agc agc gac gag tac ggc aac gag agc gcc atc acg        432
Cys Asp Leu Arg Ser Ser Asp Glu Tyr Gly Asn Glu Ser Ala Ile Thr
    130                 135                 140
ccg ggg tcc atc cag aac cag aag tac aac ggc ggc ggc ggc aac ggg        480
Pro Gly Ser Ile Gln Asn Gln Lys Tyr Asn Gly Gly Gly Gly Asn Gly
145                 150                 155                 160
ctg atc ccc tac gac gag ggg ctg gcg atg gcc aag cag att ggg gcg        528
Leu Ile Pro Tyr Asp Glu Gly Leu Ala Met Ala Lys Gln Ile Gly Ala
                165                 170                 175
ctg cgc tat ctg gag tgc agc gcc aag atg aac cgt ggc gtg aac gag        576
Leu Arg Tyr Leu Glu Cys Ser Ala Lys Met Asn Arg Gly Val Asn Glu
            180                 185                 190
gcg ttc acc gag gct gcg cgc tgc gcg ctg act gcg aca ccg aag ggg        624
Ala Phe Thr Glu Ala Ala Arg Cys Ala Leu Thr Ala Thr Pro Lys Gly
        195                 200                 205
gcc cgg gac tct gcg ccc gag gcc gaa agc agc agt tgt act atc atg        672
Ala Arg Asp Ser Ala Pro Glu Ala Glu Ser Ser Ser Cys Thr Ile Met
    210                 215                 220
tga                                                                    675

<210> 270
<211> 224
<212> PRT
<213> Ashbya gossypii

<400> 270
Met Pro Leu Cys Gly Ser Ser Ser Ser Ser Lys His Pro Ile Glu Arg
1               5                   10                  15
Lys Ile Val Ile Leu Gly Asp Gly Ala Cys Gly Lys Thr Ser Leu Leu
            20                  25                  30
Asn Val Phe Thr Arg Gly Tyr Phe Pro Lys Val Tyr Glu Pro Thr Val
        35                  40                  45
Phe Glu Asn Tyr Ile His Asp Ile Phe Val Asp Asn Gln His Ile Thr
    50                  55                  60
Leu Ser Leu Trp Asp Thr Ala Gly Gln Glu Glu Phe Asp Arg Leu Arg
65                  70                  75                  80
Ser Leu Ser Tyr Ser Asp Thr His Thr Ile Met Leu Cys Phe Ser Val
                85                  90                  95
Asp Ser Arg Asp Ser Leu Glu Asn Val Lys Asn Lys Trp Val Ser Glu
            100                 105                 110
Ile Ala Asp His Cys Glu Gly Val Lys Leu Val Leu Val Ala Leu Lys
        115                 120                 125
Cys Asp Leu Arg Ser Ser Asp Glu Tyr Gly Asn Glu Ser Ala Ile Thr
    130                 135                 140
Pro Gly Ser Ile Gln Asn Gln Lys Tyr Asn Gly Gly Gly Gly Asn Gly
145                 150                 155                 160
Leu Ile Pro Tyr Asp Glu Gly Leu Ala Met Ala Lys Gln Ile Gly Ala
                165                 170                 175
Leu Arg Tyr Leu Glu Cys Ser Ala Lys Met Asn Arg Gly Val Asn Glu
            180                 185                 190
Ala Phe Thr Glu Ala Ala Arg Cys Ala Leu Thr Ala Thr Pro Lys Gly
        195                 200                 205
Ala Arg Asp Ser Ala Pro Glu Ala Glu Ser Ser Ser Cys Thr Ile Met
    210                 215                 220

<210> 271
<211> 624
<212> DNA
<213> Ashbya gossypii

<220>

<221> CDS
<222> (1)..(624)

<400> 271
atg tct cag caa atg cat aac ccc agt atc agg aga aaa ttg gtg atc 48
Met Ser Gln Gln Met His Asn Pro Ser Ile Arg Arg Lys Leu Val Ile
1               5                   10                  15
gtc gga gat ggt gca tgc ggg aaa aca tgt ctt ttg att gtg ttt gcc 96
Val Gly Asp Gly Ala Cys Gly Lys Thr Cys Leu Leu Ile Val Phe Ala
            20                  25                  30
aag gga aag ttc cca cag gtg tat gtt cct acg gtt ttc gac aac tac 144
Lys Gly Lys Phe Pro Gln Val Tyr Val Pro Thr Val Phe Asp Asn Tyr
        35                  40                  45
gtt gca gat gtg gag gta gac ggc aga cgg gtg gag ctt gcg ctt tgg 192
Val Ala Asp Val Glu Val Asp Gly Arg Arg Val Glu Leu Ala Leu Trp
    50                  55                  60
gat acg gct ggg cag gag gat tac gac agg cta cgg ccg tta tcg tac 240
Asp Thr Ala Gly Gln Glu Asp Tyr Asp Arg Leu Arg Pro Leu Ser Tyr
65                  70                  75                  80
cca gac tcc aat gtt gtg ttg atc tgc tac tcg att gac cta cca gac 288
Pro Asp Ser Asn Val Val Leu Ile Cys Tyr Ser Ile Asp Leu Pro Asp
                85                  90                  95
tcg ttg gag aac gtg atg gag aag tgg atc agc gag gtg cta tac ttc 336
Ser Leu Glu Asn Val Met Glu Lys Trp Ile Ser Glu Val Leu Tyr Phe
            100                 105                 110
tgc cag ggt gtt ccg atc atc ttg gtg ggg tgc aag gct gac ttg cgg 384
Cys Gln Gly Val Pro Ile Ile Leu Val Gly Cys Lys Ala Asp Leu Arg
        115                 120                 125
aac gat ccg caa gtg atc gag cag ttg aga cag cag gga cag cag cct 432
Asn Asp Pro Gln Val Ile Glu Gln Leu Arg Gln Gln Gly Gln Gln Pro
    130                 135                 140
gtc tcg cag gct cag gcg cag gag gta gcg gac cag atc ggc gcg gta 480
Val Ser Gln Ala Gln Ala Gln Glu Val Ala Asp Gln Ile Gly Ala Val
145                 150                 155                 160
gag tac att gag tgc tct gca aag acc ggc ttt ggt gtg cgc gag gtg 528
Glu Tyr Ile Glu Cys Ser Ala Lys Thr Gly Phe Gly Val Arg Glu Val
                165                 170                 175
ttt gag gcg gcc acg cgt gct tcc ttg atg ggg aaa caa ggc aag tct 576
Phe Glu Ala Ala Thr Arg Ala Ser Leu Met Gly Lys Gln Gly Lys Ser
            180                 185                 190
aag gcg aag tct gac aag aag aag aag aaa aag tgt gtg gtc ttg 621
Lys Ala Lys Ser Asp Lys Lys Lys Lys Lys Lys Cys Val Val Leu
        195                 200                 205
tag 624

<210> 272
<211> 207
<212> PRT
<213> Ashbya gossypii

<400> 272
Met Ser Gln Gln Met His Asn Pro Ser Ile Arg Arg Lys Leu Val Ile
1               5                   10                  15
Val Gly Asp Gly Ala Cys Gly Lys Thr Cys Leu Leu Ile Val Phe Ala
            20                  25                  30
Lys Gly Lys Phe Pro Gln Val Tyr Val Pro Thr Val Phe Asp Asn Tyr
        35                  40                  45
Val Ala Asp Val Glu Val Asp Gly Arg Arg Val Glu Leu Ala Leu Trp
    50                  55                  60
Asp Thr Ala Gly Gln Glu Asp Tyr Asp Arg Leu Arg Pro Leu Ser Tyr
65                  70                  75                  80
Pro Asp Ser Asn Val Val Leu Ile Cys Tyr Ser Ile Asp Leu Pro Asp
                85                  90                  95
Ser Leu Glu Asn Val Met Glu Lys Trp Ile Ser Glu Val Leu Tyr Phe
            100                 105                 110

Cys Gln Gly Val Pro Ile Ile Leu Val Gly Cys Lys Ala Asp Leu Arg
        115                 120                 125
Asn Asp Pro Gln Val Ile Glu Gln Leu Arg Gln Gln Gly Gln Gln Pro
    130                 135                 140
Val Ser Gln Ala Gln Ala Gln Glu Val Ala Asp Gln Ile Gly Ala Val
145                 150                 155                 160
Glu Tyr Ile Glu Cys Ser Ala Lys Thr Gly Phe Gly Val Arg Glu Val
                165                 170                 175
Phe Glu Ala Ala Thr Arg Ala Ser Leu Met Gly Lys Gln Gly Lys Ser
            180                 185                 190
Lys Ala Lys Ser Asp Lys Lys Lys Lys Lys Lys Cys Val Val Leu
        195                 200                 205

<210> 273
<211> 615
<212> DNA
<213> Yarrowia lipolytica

<220>
<221> CDS
<222> (1)..(615)

<400> 273
atg ccc caa cca acg gat ctg cga aga aaa ctc gtc att gtc ggc gac 48
Met Pro Gln Pro Thr Asp Leu Arg Arg Lys Leu Val Ile Val Gly Asp
1               5                   10                  15
gga gcc tgc ggc aag acc tgt ctg ctg att gtc ttc gcg aag ggc acc 96
Gly Ala Cys Gly Lys Thr Cys Leu Leu Ile Val Phe Ala Lys Gly Thr
            20                  25                  30
ttc ccg gag gtg tac gtg ccc acc gtg ttt gag aac tac gtg gcc gac 144
Phe Pro Glu Val Tyr Val Pro Thr Val Phe Glu Asn Tyr Val Ala Asp
        35                  40                  45
gtg gag att gac ggc cga cga gtg gag ctg gcc ctg tgg gat acc gcc 192
Val Glu Ile Asp Gly Arg Arg Val Glu Leu Ala Leu Trp Asp Thr Ala
    50                  55                  60
ggc cag gag gat tac gac cga ctg cga ccc ctg tcc tac ccc gac gcc 240
Gly Gln Glu Asp Tyr Asp Arg Leu Arg Pro Leu Ser Tyr Pro Asp Ala
65                  70                  75                  80
aac gtc atc atc atc tgc ttc gcc atc gac agc ccc gac tcg ctc gac 288
Asn Val Ile Ile Ile Cys Phe Ala Ile Asp Ser Pro Asp Ser Leu Asp
                85                  90                  95
aac gtg cag gag aag tgg atc tcc gag gtg ctg cac ttt tgc cag ggc 336
Asn Val Gln Glu Lys Trp Ile Ser Glu Val Leu His Phe Cys Gln Gly
            100                 105                 110
gtg cct att ctg ctg gtc ggt tgc aag gtc gac ctg cga aac gac ccc 384
Val Pro Ile Leu Leu Val Gly Cys Lys Val Asp Leu Arg Asn Asp Pro
        115                 120                 125
aag acc atc gag gag ctg aga aga acg tcc cag cga ccc gtc acg acc 432
Lys Thr Ile Glu Glu Leu Arg Arg Thr Ser Gln Arg Pro Val Thr Thr
    130                 135                 140
gag gag gga aat gcc gtg gcc cag aag att ggc gcc ggc aag tac ctg 480
Glu Glu Gly Asn Ala Val Ala Gln Lys Ile Gly Ala Gly Lys Tyr Leu
145                 150                 155                 160
gag tgc tct gcc cga acc cac gac gga gtg aga gag gtc ttc gag cat 528
Glu Cys Ser Ala Arg Thr His Asp Gly Val Arg Glu Val Phe Glu His
                165                 170                 175
gcc acc cga gct gcc ctg act gcc cac ggc cag aag ggc tcc aag agc 576
Ala Thr Arg Ala Ala Leu Thr Ala His Gly Gln Lys Gly Ser Lys Ser
            180                 185                 190
tcc agc aga gag ggc aag aaa aag tgt ttg att ttg taa 615
Ser Ser Arg Glu Gly Lys Lys Lys Cys Leu Ile Leu
        195                 200

<210> 274
<211> 204
<212> PRT
<213> Yarrowia lipolytica

<400> 274
Met Pro Gln Pro Thr Asp Leu Arg Arg Lys Leu Val Ile Val Gly Asp
1               5                   10                  15
Gly Ala Cys Gly Lys Thr Cys Leu Leu Ile Val Phe Ala Lys Gly Thr
            20                  25                  30
Phe Pro Glu Val Tyr Val Pro Thr Val Phe Glu Asn Tyr Val Ala Asp
        35                  40                  45
Val Glu Ile Asp Gly Arg Arg Val Glu Leu Ala Leu Trp Asp Thr Ala
    50                  55                  60
Gly Gln Glu Asp Tyr Asp Arg Leu Arg Pro Leu Ser Tyr Pro Asp Ala
65                  70                  75                  80
Asn Val Ile Ile Ile Cys Phe Ala Ile Asp Ser Pro Asp Ser Leu Asp
                85                  90                  95
Asn Val Gln Glu Lys Trp Ile Ser Glu Val Leu His Phe Cys Gln Gly
            100                 105                 110
Val Pro Ile Leu Leu Val Gly Cys Lys Val Asp Leu Arg Asn Asp Pro
        115                 120                 125
Lys Thr Ile Glu Glu Leu Arg Arg Thr Ser Gln Arg Pro Val Thr Thr
    130                 135                 140
Glu Glu Gly Asn Ala Val Ala Gln Lys Ile Gly Ala Gly Lys Tyr Leu
145                 150                 155                 160
Glu Cys Ser Ala Arg Thr His Asp Gly Val Arg Glu Val Phe Glu His
                165                 170                 175
Ala Thr Arg Ala Ala Leu Thr Ala His Gly Gln Lys Gly Ser Lys Ser
            180                 185                 190
Ser Ser Arg Glu Gly Lys Lys Lys Cys Leu Ile Leu
        195                 200

<210> 275
<211> 915
<212> DNA
<213> Zea mays

<220>
<221> CDS
<222> (10)..(657)

<400> 275
gtcttggcc atg gcc tcc agc gcc tcc cgg ttc atc aag tgc gtc aca gtg      51
          Met Ala Ser Ser Ala Ser Arg Phe Ile Lys Cys Val Thr Val
          1               5                   10
ggg gac ggc gcc gtg gga aag acc tgc atg ctc atc tgc tac acc agc      99
Gly Asp Gly Ala Val Gly Lys Thr Cys Met Leu Ile Cys Tyr Thr Ser
15                  20                  25                  30
aac aag ttc ccc act gat tac ata ccc act gtc ttc gat aac ttc agt      147
Asn Lys Phe Pro Thr Asp Tyr Ile Pro Thr Val Phe Asp Asn Phe Ser
                35                  40                  45
gca aac gtg gta gtc gac ggt acc acg gtg aat ttg ggc ctt tgg gat      195
Ala Asn Val Val Val Asp Gly Thr Thr Val Asn Leu Gly Leu Trp Asp
            50                  55                  60
act gca ggg cag gaa gat tac aac aga ctg agg cca ctg agc tac cgc      243
Thr Ala Gly Gln Glu Asp Tyr Asn Arg Leu Arg Pro Leu Ser Tyr Arg
        65                  70                  75
gga gca gat gtg ttc gtg ctc gcc ttc tcg ctt gtc agc cga gct agc      291
Gly Ala Asp Val Phe Val Leu Ala Phe Ser Leu Val Ser Arg Ala Ser
    80                  85                  90
tac gag aat gtt atg aag aag tgg cta ccg gag ctt cag cat tat gca      339
Tyr Glu Asn Val Met Lys Lys Trp Leu Pro Glu Leu Gln His Tyr Ala
95                  100                 105                 110
ccg ggt gtc ccc ata gtg ttg gcc gga act aaa ttg gat ctt cgt gaa      387
Pro Gly Val Pro Ile Val Leu Ala Gly Thr Lys Leu Asp Leu Arg Glu
                115                 120                 125
gac agg cac tac tta gtt gac cat cct ggt gcg gta cct gtt act aca      435
Asp Arg His Tyr Leu Val Asp His Pro Gly Ala Val Pro Val Thr Thr
            130                 135                 140
gca cag ggg gag gaa ctg cgc aag cac att ggc gca act tgc tac atc      483
Ala Gln Gly Glu Glu Leu Arg Lys His Ile Gly Ala Thr Cys Tyr Ile

        145                 150                 155
gaa tgc agc tca aaa act cag cag aat gtg aaa gct gtg ttt gat gct      531
Glu Cys Ser Ser Lys Thr Gln Gln Asn Val Lys Ala Val Phe Asp Ala
    160                 165                 170
gcc atc aag gta gta atc agg cct ccg acg aag cag cga gaa agg aag      579
Ala Ile Lys Val Val Ile Arg Pro Pro Thr Lys Gln Arg Glu Arg Lys
175                 180                 185                 190
aaa aag aaa gaa cgg cga gga tgc tca ata ttc tgc agc cgt atc atg      627
Lys Lys Lys Glu Arg Arg Gly Cys Ser Ile Phe Cys Ser Arg Ile Met
                195                 200                 205
cac gca aga aga cta gga tgc ttc aag tgatagaagg ccttcttcga            674
His Ala Arg Arg Leu Gly Cys Phe Lys
            210                 215
cgataacgta ggcttctaag atgaatcgag atcgtgtata gtttgtctcc atgttccaaa    734

gtgctcgttg cgcttgtgca cttgggctgg taatgtgtat atttagtctt tgccaattga    794

tatttaggag aaatattaag cacttcgttt atgtaccatt ggctcataat gagctgggaa    854

gtccaataca tactaagaag taacaatatt ttcttccgat gattttatta tggtttctcg     914

t                                                                    915

<210> 276
<211> 215
<212> PRT
<213> Zea mays

<400> 276
Met Ala Ser Ser Ala Ser Arg Phe Ile Lys Cys Val Thr Val Gly Asp
1               5                   10                  15
Gly Ala Val Gly Lys Thr Cys Met Leu Ile Cys Tyr Thr Ser Asn Lys
            20                  25                  30
Phe Pro Thr Asp Tyr Ile Pro Thr Val Phe Asp Asn Phe Ser Ala Asn
        35                  40                  45
Val Val Val Asp Gly Thr Thr Val Asn Leu Gly Leu Trp Asp Thr Ala
    50                  55                  60
Gly Gln Glu Asp Tyr Asn Arg Leu Arg Pro Leu Ser Tyr Arg Gly Ala
65                  70                  75                  80
Asp Val Phe Val Leu Ala Phe Ser Leu Val Ser Arg Ala Ser Tyr Glu
                85                  90                  95
Asn Val Met Lys Lys Trp Leu Pro Glu Leu Gln His Tyr Ala Pro Gly
            100                 105                 110
Val Pro Ile Val Leu Ala Gly Thr Lys Leu Asp Leu Arg Glu Asp Arg
        115                 120                 125
His Tyr Leu Val Asp His Pro Gly Ala Val Pro Val Thr Thr Ala Gln
    130                 135                 140
Gly Glu Glu Leu Arg Lys His Ile Gly Ala Thr Cys Tyr Ile Glu Cys
145                 150                 155                 160
Ser Ser Lys Thr Gln Gln Asn Val Lys Ala Val Phe Asp Ala Ala Ile
                165                 170                 175
Lys Val Val Ile Arg Pro Pro Thr Lys Gln Arg Glu Arg Lys Lys Lys
            180                 185                 190
Lys Glu Arg Arg Gly Cys Ser Ile Phe Cys Ser Arg Ile Met His Ala
        195                 200                 205
Arg Arg Leu Gly Cys Phe Lys
    210                 215

<210> 277
<211> 1002
<212> DNA

<213> Zea mays

<220>
<221> CDS
<222> (149)..(787)

<400> 277
agaagataag caaagcaagg caaccgttgt cgttgtctct gttcctccgc ttcctgctct      60

cttggcttgc tgcttgccct cggagcagtg ccttccgccg ccgccgccgc cgcctgttgt     120

gagaaggaga ggccggggct gggaggag atg agc gtg acc aag ttc atc aag        172
                               Met Ser Val Thr Lys Phe Ile Lys
                               1               5
tgc gtc acg gtg ggg gac ggc gcg gtg ggc aag acc tgc atg ctc atc       220
Cys Val Thr Val Gly Asp Gly Ala Val Gly Lys Thr Cys Met Leu Ile
    10                  15                  20
tgc tac acc agc aac aag ttc ccc acg gat tac atc ccc acg gtg ttc       268
Cys Tyr Thr Ser Asn Lys Phe Pro Thr Asp Tyr Ile Pro Thr Val Phe
25                  30                  35                  40
gac aac ttc agc gcc aac gtc tcc gtg gac ggc agc atc gtc aac ctg       316
Asp Asn Phe Ser Ala Asn Val Ser Val Asp Gly Ser Ile Val Asn Leu
                45                  50                  55
ggc ctc tgg gac act gca gga caa gag gac tac agc aga ttg cgg cca       364
Gly Leu Trp Asp Thr Ala Gly Gln Glu Asp Tyr Ser Arg Leu Arg Pro
            60                  65                  70
ctg agc tac agg ggc gcg gac gtg ttc gtg ctg gcc ttc tcc ttg atc       412
Leu Ser Tyr Arg Gly Ala Asp Val Phe Val Leu Ala Phe Ser Leu Ile
        75                  80                  85
agc agg gcg agc tat gag aac gtc ctt aag aag tgg gtg cca gag ctt       460
Ser Arg Ala Ser Tyr Glu Asn Val Leu Lys Lys Trp Val Pro Glu Leu
    90                  95                  100
cgc aga ttc gcg ccc gac gtc ccg gtc gtt ctt gtc ggg acc aag tta       508
Arg Arg Phe Ala Pro Asp Val Pro Val Val Leu Val Gly Thr Lys Leu
105                 110                 115                 120
gat ctc cgt gac cac agg gcc tac ctt gct gac cat cct gga gcg tcg       556
Asp Leu Arg Asp His Arg Ala Tyr Leu Ala Asp His Pro Gly Ala Ser
                125                 130                 135
acg atc acg acg gca cag ggc gaa gaa ctg agg agg cag atc ggc gct       604
Thr Ile Thr Thr Ala Gln Gly Glu Glu Leu Arg Arg Gln Ile Gly Ala
            140                 145                 150
gcg gct tac atc gag tgc agt tcc aaa acg cag cag aat gtc aag tcg       652
Ala Ala Tyr Ile Glu Cys Ser Ser Lys Thr Gln Gln Asn Val Lys Ser
        155                 160                 165
gtc ttc gac aca gcc atc aaa gtg gtc ctt cag ccc ccg cgg agg agg       700
Val Phe Asp Thr Ala Ile Lys Val Val Leu Gln Pro Pro Arg Arg Arg
    170                 175                 180
gag gcg acg cct gcc agg agg aag aac agg cgt ggc tcc ggg tgc tct       748
Glu Ala Thr Pro Ala Arg Arg Lys Asn Arg Arg Gly Ser Gly Cys Ser
185                 190                 195                 200
atc atg aac ctc atg tgt ggc agc acg tgc gct gct taggagtcta            794
Ile Met Asn Leu Met Cys Gly Ser Thr Cys Ala Ala
                205                 210
gaacactgat ctggaaggag gtgaaggtga aggcatggtg tctatgtgct atggcgactg     854

gcaagttaat ggggccgcat ggatgactgc tgctcttgtt tttttaagct cgtctgccgt     914

atgctttgtt tttttaggct tcaaggactg acaattgcaa gaatgcagtg tttatgtaag     974

aggttgtttg ctggaatagg attgctgt                                       1002

<210> 278
<211> 212
<212> PRT
<213> Zea mays

<400> 278
Met Ser Val Thr Lys Phe Ile Lys Cys Val Thr Val Gly Asp Gly Ala
1               5                   10                  15
Val Gly Lys Thr Cys Met Leu Ile Cys Tyr Thr Ser Asn Lys Phe Pro
            20                  25                  30
Thr Asp Tyr Ile Pro Thr Val Phe Asp Asn Phe Ser Ala Asn Val Ser
        35                  40                  45
Val Asp Gly Ser Ile Val Asn Leu Gly Leu Trp Asp Thr Ala Gly Gln
    50                  55                  60
Glu Asp Tyr Ser Arg Leu Arg Pro Leu Ser Tyr Arg Gly Ala Asp Val
65                  70                  75                  80
Phe Val Leu Ala Phe Ser Leu Ile Ser Arg Ala Ser Tyr Glu Asn Val
                85                  90                  95
Leu Lys Lys Trp Val Pro Glu Leu Arg Arg Phe Ala Pro Asp Val Pro
            100                 105                 110
Val Val Leu Val Gly Thr Lys Leu Asp Leu Arg Asp His Arg Ala Tyr
        115                 120                 125
Leu Ala Asp His Pro Gly Ala Ser Thr Ile Thr Thr Ala Gln Gly Glu
    130                 135                 140
Glu Leu Arg Arg Gln Ile Gly Ala Ala Ala Tyr Ile Glu Cys Ser Ser
145                 150                 155                 160
Lys Thr Gln Gln Asn Val Lys Ser Val Phe Asp Thr Ala Ile Lys Val
                165                 170                 175
Val Leu Gln Pro Pro Arg Arg Arg Glu Ala Thr Pro Ala Arg Arg Lys
            180                 185                 190
Asn Arg Arg Gly Ser Gly Cys Ser Ile Met Asn Leu Met Cys Gly Ser
        195                 200                 205
Thr Cys Ala Ala
    210

<210> 279
<211> 850
<212> DNA
<213> Oryza sativa

<220>
<221> CDS
<222> (112)..(705)

<400> 279
agcaagcagc agctgaggtg aggtccgtgg cgttggagtg aggactgagg aggaagaaga      60

gggcgggatc tagggtaccg gatgcgctgg ctgtgctgag tgagagtaga g atg agc      117
                                                         Met Ser
                                                         1
gcg tct cgg ttc atc aag tgc gtc acc gtg ggg gac ggc gcc gtg ggc      165
Ala Ser Arg Phe Ile Lys Cys Val Thr Val Gly Asp Gly Ala Val Gly
        5                   10                  15
aag acc tgc atg ctc atc tcc tac acc tcc aac acc ttc ccc acg gac      213
Lys Thr Cys Met Leu Ile Ser Tyr Thr Ser Asn Thr Phe Pro Thr Asp
    20                  25                  30
tat gtt cca act gtt ttt gat aac ttc agt gca aat gtt gtg gtc gat      261
Tyr Val Pro Thr Val Phe Asp Asn Phe Ser Ala Asn Val Val Val Asp
35                  40                  45                  50
ggg agc act gtg aac ttg ggg ttg tgg gat aca gca gga caa gag gac      309
Gly Ser Thr Val Asn Leu Gly Leu Trp Asp Thr Ala Gly Gln Glu Asp
                55                  60                  65
tac aat agg cta cgc ccg ttg agc tat cgt ggc gct gat gtt ttc ctg      357
Tyr Asn Arg Leu Arg Pro Leu Ser Tyr Arg Gly Ala Asp Val Phe Leu
            70                  75                  80
ctg gcc ttt tct ctg atc agc aaa gca agc tat gag aat gtt tct aaa      405

Leu Ala Phe Ser Leu Ile Ser Lys Ala Ser Tyr Glu Asn Val Ser Lys
        85                  90                  95
aag tgg ata cct gaa tta agg cat tat gct cct ggt gtg cca ata att     453
Lys Trp Ile Pro Glu Leu Arg His Tyr Ala Pro Gly Val Pro Ile Ile
    100                 105                 110
ctc gtt gga aca aag ctt gat ctg cgg gat gat aag caa ttt ttc gta     501
Leu Val Gly Thr Lys Leu Asp Leu Arg Asp Asp Lys Gln Phe Phe Val
115                 120                 125                 130
gat cac cct ggt gct gta cct att tcc act gct cag ggc gaa gag ctg     549
Asp His Pro Gly Ala Val Pro Ile Ser Thr Ala Gln Gly Glu Glu Leu
                135                 140                 145
agg aaa ctc att ggt gca gcg gca tac att gaa tgc agt tca aaa aca     597
Arg Lys Leu Ile Gly Ala Ala Ala Tyr Ile Glu Cys Ser Ser Lys Thr
            150                 155                 160
cag caa aac atc aag gca gtt ttc gat gct gcg att aag gtg gtt ctc     645
Gln Gln Asn Ile Lys Ala Val Phe Asp Ala Ala Ile Lys Val Val Leu
        165                 170                 175
cag cct cca aag caa aag aag aag aag aaa aag gcg cag aaa gga tgt     693
Gln Pro Pro Lys Gln Lys Lys Lys Lys Lys Lys Ala Gln Lys Gly Cys
    180                 185                 190
gcc atc ttg taattaaatg gtagacagtg cagtgcagat cgatgtatcc cttcatttgt     752
Ala Ile Leu
195
agcctctggc ttcaatcgtc gcttgtttgt ataattacgc tagatgccac cggcagaaga     812

tataatatag tcctcctgcc tttgtggtgt tggtctct     850

<210> 280
<211> 197
<212> PRT
<213> Oryza sativa

<400> 280
Met Ser Ala Ser Arg Phe Ile Lys Cys Val Thr Val Gly Asp Gly Ala
1               5                   10                  15
Val Gly Lys Thr Cys Met Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Val
                85                  90                  95
Ser Lys Lys Trp Ile Pro Glu Leu Arg His Tyr Ala Pro Gly Val Pro
            100                 105                 110
Ile Ile Leu Val Gly Thr Lys Leu Asp Leu Arg Asp Asp Lys Gln Phe
        115                 120                 125
Phe Val Asp His Pro Gly Ala Val Pro Ile Ser Thr Ala Gln Gly Glu
    130                 135                 140
Glu Leu Arg Lys Leu Ile Gly Ala Ala Ala Tyr Ile Glu Cys Ser Ser
145                 150                 155                 160
Lys Thr Gln Gln Asn Ile Lys Ala Val Phe Asp Ala Ala Ile Lys Val
                165                 170                 175
Val Leu Gln Pro Pro Lys Gln Lys Lys Lys Lys Lys Lys Ala Gln Lys
            180                 185                 190
Gly Cys Ala Ile Leu
        195

<210> 281
<211> 800
<212> DNA
<213> Suillus bovinus

<220>
<221> CDS
<222> (123)..(707)

<400> 281
ccgtcacgtt gccttctacc atctttcaaa gttaaaggtt attcaagcac tcgctgattc     60

tgcgcacaca ttgctctcct tgagggcttc tctttcaggc aaccaagtag gattcaatca    120

ga atg cac aac atc aaa tgt gtt gta gtc ggc gat ggt gct gtt ggc      167
   Met His Asn Ile Lys Cys Val Val Val Gly Asp Gly Ala Val Gly
   1               5                   10                  15
aag acg tgt ctt ctc atc tct tat acc aca aat gcc ttt cca gga gaa      215
Lys Thr Cys Leu Leu Ile Ser Tyr Thr Thr Asn Ala Phe Pro Gly Glu
                20                  25                  30
tac gtg cca aca gta ttc gac aac tac tct gca aat gtg atg gtc gac      263
Tyr Val Pro Thr Val Phe Asp Asn Tyr Ser Ala Asn Val Met Val Asp
            35                  40                  45
ggg aaa act atc tct ctc ggt cta tgg gat acc gct gga caa gaa gat      311
Gly Lys Thr Ile Ser Leu Gly Leu Trp Asp Thr Ala Gly Gln Glu Asp
        50                  55                  60
tac gat cgt ctc cgg cca ctc tcc tac cct caa aca gat gtc ttt ttg      359
Tyr Asp Arg Leu Arg Pro Leu Ser Tyr Pro Gln Thr Asp Val Phe Leu
    65                  70                  75
atc tgt ttc tcg ctc gtc agt cca cct agc tac gag aac gtt aga acc      407
Ile Cys Phe Ser Leu Val Ser Pro Pro Ser Tyr Glu Asn Val Arg Thr
80                  85                  90                  95
aag tgg tgg cct gag att tca cat cat gcg cca tct act tcg gtg gtc      455
Lys Trp Trp Pro Glu Ile Ser His His Ala Pro Ser Thr Ser Val Val
                100                 105                 110
ttg gtt ggc act aaa ctg gat ttg cgt gaa gat cct gct acc atc gag      503
Leu Val Gly Thr Lys Leu Asp Leu Arg Glu Asp Pro Ala Thr Ile Glu
            115                 120                 125
aaa ctc cgt gac cgg cgt atg cag cct atc cag tac acg caa ggg gtc      551
Lys Leu Arg Asp Arg Arg Met Gln Pro Ile Gln Tyr Thr Gln Gly Val
        130                 135                 140
tcg atg gcg agg gat att ggc gcc gtc aag tat ctt gaa tgt tct gcg      599
Ser Met Ala Arg Asp Ile Gly Ala Val Lys Tyr Leu Glu Cys Ser Ala
    145                 150                 155
cta tcg caa aag ggc cta aag acc gtg ttt gat gag gtt atc cgt gct      647
Leu Ser Gln Lys Gly Leu Lys Thr Val Phe Asp Glu Val Ile Arg Ala
160                 165                 170                 175
gtt ttg aac cct ccc ccg aag gaa aag aag cgc agt ggt cgt ggt tgt      695
Val Leu Asn Pro Pro Pro Lys Glu Lys Lys Arg Ser Gly Arg Gly Cys
                180                 185                 190
gtt atc gta tgagtggatt tgccattcca catgctcata tagatggatt ttctttgttt   754
Val Ile Val

tatcatatca cgcccattta tgttgttgct tctgttaaaa aaaaaa                   800


<210> 282
<211> 194
<212> PRT
<213> Suillus bovinus

<400> 282
Met His Asn Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Val Pro Thr Val Phe Asp Asn Tyr Ser Ala Asn Val Met Val Asp Gly
        35                  40                  45

Lys Thr Ile Ser Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Ser Pro Pro Ser Tyr Glu Asn Val Arg Thr Lys
                85                  90                  95
Trp Trp Pro Glu Ile Ser His His Ala Pro Ser Thr Ser Val Val Leu
            100                 105                 110
Val Gly Thr Lys Leu Asp Leu Arg Glu Asp Pro Ala Thr Ile Glu Lys
        115                 120                 125
Leu Arg Asp Arg Arg Met Gln Pro Ile Gln Tyr Thr Gln Gly Val Ser
    130                 135                 140
Met Ala Arg Asp Ile Gly Ala Val Lys Tyr Leu Glu Cys Ser Ala Leu
145                 150                 155                 160
Ser Gln Lys Gly Leu Lys Thr Val Phe Asp Glu Val Ile Arg Ala Val
                165                 170                 175
Leu Asn Pro Pro Pro Lys Glu Lys Lys Arg Ser Gly Arg Gly Cys Val
            180                 185                 190
Ile Val

<210> 283
<211> 1065
<212> DNA
<213> Suillus bovinus

<220>
<221> CDS
<222> (170)..(745)

<400> 283
gccaacatcg acaccgtact ttctcatttc ttctaccgac gtcctgttcg taaattagag      60

tcaaccttct ctgtcgtgat tgggttaata gcatttcctc gcttcaactc tcctccttga     120

attttccgga agaatcgcca agtttatcac gtagaaccat ctcaacgac atg cag act     178
                                                      Met Gln Thr
                                                      1
atc aag gtt gta gtg gtg ggg gac ggt gct gta ggc aag act tgt ttg       226
Ile Lys Val Val Val Val Gly Asp Gly Ala Val Gly Lys Thr Cys Leu
    5                   10                  15
ttg atc tca tac acc acg aac aaa ttt cca agc gac tat gtt ccg act       274
Leu Ile Ser Tyr Thr Thr Asn Lys Phe Pro Ser Asp Tyr Val Pro Thr
20                  25                  30                  35
gtc ttc gat aac tac gct gtc act gta atg att ggt gaa gat cct tac       322
Val Phe Asp Asn Tyr Ala Val Thr Val Met Ile Gly Glu Asp Pro Tyr
                40                  45                  50
aca cta ggt ctt ttc gac acc gcc ggg caa gag gat tat gac cgt ctc       370
Thr Leu Gly Leu Phe Asp Thr Ala Gly Gln Glu Asp Tyr Asp Arg Leu
            55                  60                  65
cgg cca ctg tca tac ccc caa acc gac gtc ttc ctt gtt tgc ttt agt       418
Arg Pro Leu Ser Tyr Pro Gln Thr Asp Val Phe Leu Val Cys Phe Ser
        70                  75                  80
gta acg tcc cct cca tcc ttc gag aac gtg agg gag aaa tgg att ccg       466
Val Thr Ser Pro Pro Ser Phe Glu Asn Val Arg Glu Lys Trp Ile Pro
    85                  90                  95
gag gta cac cat cat tgt ccg ggt gtc cca tgt ctt atc gtt ggg acg       514
Glu Val His His His Cys Pro Gly Val Pro Cys Leu Ile Val Gly Thr
100                 105                 110                 115
caa atc gat ttg cgg gac gac gct caa gtg att gag aag ctc tcc agg       562
Gln Ile Asp Leu Arg Asp Asp Ala Gln Val Ile Glu Lys Leu Ser Arg
                120                 125                 130
cag aag caa cga ccg gtg ccg acc gag cac ggc gag agg ctt tcc aga       610
Gln Lys Gln Arg Pro Val Pro Thr Glu His Gly Glu Arg Leu Ser Arg
            135                 140                 145
gag ctt ggt gcc gtc aag tac gtg gag tgc tca gcc ctg acg cag aaa       658

Glu Leu Gly Ala Val Lys Tyr Val Glu Cys Ser Ala Leu Thr Gln Lys
        150                 155                 160
ggg ttg aag aac gtc ttc gac gag gct att gtc gct gcg cta gag cca 706
Gly Leu Lys Asn Val Phe Asp Glu Ala Ile Val Ala Ala Leu Glu Pro
    165                 170                 175
ccc gtg gtt aag aag aca cat aaa tgc gtc gtc gtt taatgaatga 752
Pro Val Val Lys Lys Thr His Lys Cys Val Val Val
180                 185                 190
tccatcacga cggtacagct tgttttatct tcttatgact cctttacgcc ttgttctgcc 812

tcatatatcc ttctcatctt tttttatgtc gtttctctcg ttgcgccaag acatcatgat 872

tttccctagt cctgttgcat ctgttctctt ccgcacgagc tgagtggact tgagctatct 932

cgtgctctta aagaagctga tctcatccgt ctttctacac cacaattgat gactagttcg 992

tattgttgga actgttgggt ttgataggaa tttaataccc atgacgggga cttgtcatgt 1052

caaaaaaaaa aaa 1065

<210> 284
<211> 191
<212> PRT
<213> Suillus bovinus

<400> 284
Met Gln Thr Ile Lys Val Val Val Val Gly Asp Gly Ala Val Gly Lys
1               5                   10                  15
Thr Cys Leu Leu Ile Ser Tyr Thr Thr Asn Lys Phe Pro Ser Asp Tyr
            20                  25                  30
Val Pro Thr Val Phe Asp Asn Tyr Ala Val Thr Val Met Ile Gly Glu
        35                  40                  45
Asp Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Thr Ser Pro Pro Ser Phe Glu Asn Val Arg Glu Lys
                85                  90                  95
Trp Ile Pro Glu Val His His His Cys Pro Gly Val Pro Cys Leu Ile
            100                 105                 110
Val Gly Thr Gln Ile Asp Leu Arg Asp Asp Ala Gln Val Ile Glu Lys
        115                 120                 125
Leu Ser Arg Gln Lys Gln Arg Pro Val Pro Thr Glu His Gly Glu Arg
    130                 135                 140
Leu Ser Arg Glu Leu Gly Ala Val Lys Tyr Val Glu Cys Ser Ala Leu
145                 150                 155                 160
Thr Gln Lys Gly Leu Lys Asn Val Phe Asp Glu Ala Ile Val Ala Ala
                165                 170                 175
Leu Glu Pro Pro Val Val Lys Lys Thr His Lys Cys Val Val Val
            180                 185                 190

<210> 285
<211> 576
<212> DNA
<213> Yarrowia lipolytica

<220>
<221> CDS
<222> (1)..(576)

<400> 285
atg cag acc ata aaa tgt gtt gtt gtc ggc gat ggt gcc gtc gga aag      48
Met Gln Thr Ile Lys Cys Val Val Val Gly Asp Gly Ala Val Gly Lys
1               5                   10                  15
act tgt ctc ctc atc tca tac aca aca aac aag ttc ccc tct gaa tac      96
Thr Cys Leu Leu Ile Ser Tyr Thr Thr Asn Lys Phe Pro Ser Glu Tyr
            20                  25                  30
gtt ccc acc gtt ttt gac aac tat gcc gtg act gtc atg att gga gac      144
Val Pro Thr Val Phe Asp Asn Tyr Ala Val Thr Val Met Ile Gly Asp
        35                  40                  45
gag ccc tac aca ctc gga ctg ttc gac acc gcc ggt cag gag gat tac      192
Glu Pro Tyr Thr Leu Gly Leu Phe Asp Thr Ala Gly Gln Glu Asp Tyr
    50                  55                  60
gac cga ctg cga cct ctt tgt tac cct cag acc gat gtt ttc ctc gtc      240
Asp Arg Leu Arg Pro Leu Cys Tyr Pro Gln Thr Asp Val Phe Leu Val
65                  70                  75                  80
tgc ttt tcc gtc acc tct ccc gcc tcc ttt gag aac gtc aag gag aag      288
Cys Phe Ser Val Thr Ser Pro Ala Ser Phe Glu Asn Val Lys Glu Lys
                85                  90                  95
tgg ttc cct gag gtc cac cac cac tgc ccc ggc gtg cct tgc ctc att      336
Trp Phe Pro Glu Val His His His Cys Pro Gly Val Pro Cys Leu Ile
            100                 105                 110
gtt ggt acc cag gtt gat ccg cga agt gac agg atg att ctt gac aag      384
Val Gly Thr Gln Val Asp Pro Arg Ser Asp Arg Met Ile Leu Asp Lys
        115                 120                 125
ctt tcc cga cac aag ctg cga ccc atg acc act gag caa ggc tac cag      432
Leu Ser Arg His Lys Leu Arg Pro Met Thr Thr Glu Gln Gly Tyr Gln
    130                 135                 140
ctc gcc cga gaa ctc ggt gcc gtc aag tac gtc gag tgt tct gcc ctt      480
Leu Ala Arg Glu Leu Gly Ala Val Lys Tyr Val Glu Cys Ser Ala Leu
145                 150                 155                 160
act cag aag ggt ctc aag gac gtt ttc gac gag gcc atc gtg gca gct      528
Thr Gln Lys Gly Leu Lys Asp Val Phe Asp Glu Ala Ile Val Ala Ala
                165                 170                 175
ctt gag cct cca gtg gtc aag aag aac aaa aag tgc att gtg ctc          573
Leu Glu Pro Pro Val Val Lys Lys Asn Lys Lys Cys Ile Val Leu
            180                 185                 190
tag                                                                  576

<210> 286
<211> 191
<212> PRT
<213> Yarrowia lipolytica

<400> 286
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Glu Tyr
            20                  25                  30
Val Pro Thr Val Phe Asp Asn Tyr Ala Val Thr Val Met Ile Gly Asp
        35                  40                  45
Glu Pro Tyr Thr Leu Gly Leu Phe Asp Thr Ala Gly Gln Glu Asp Tyr
    50                  55                  60
Asp Arg Leu Arg Pro Leu Cys Tyr Pro Gln Thr Asp Val Phe Leu Val
65                  70                  75                  80
Cys Phe Ser Val Thr Ser Pro Ala Ser Phe Glu Asn Val Lys Glu Lys
                85                  90                  95
Trp Phe Pro Glu Val His His His Cys Pro Gly Val Pro Cys Leu Ile
            100                 105                 110
Val Gly Thr Gln Val Asp Pro Arg Ser Asp Arg Met Ile Leu Asp Lys
        115                 120                 125
Leu Ser Arg His Lys Leu Arg Pro Met Thr Thr Glu Gln Gly Tyr Gln
    130                 135                 140
Leu Ala Arg Glu Leu Gly Ala Val Lys Tyr Val Glu Cys Ser Ala Leu
145                 150                 155                 160

Thr Gln Lys Gly Leu Lys Asp Val Phe Asp Glu Ala Ile Val Ala Ala
                165                 170                 175
Leu Glu Pro Pro Val Val Lys Lys Asn Lys Lys Cys Ile Val Leu
            180                 185                 190

<210> 287
<211> 1157
<212> DNA
<213> Ashbya gossypii

<220>
<221> CDS
<222> (192)..(971)

<400> 287
gcaagagcct taaatcacct gcatcccccg atttcaacac ccttgttaga aagaggtaaa 60

cagatcacca cggcgccagt aggtgaaaat cggcaggcag tgggtggcgt tcaggacgcg 120

gcaaggaagg gcagtggaag caatcgcggt cggtttagcg ggacagcgcg tcaagaggca 180

gacagaacga a atg agc gca ggg ccg ttg caa gct gcg cca aag aag aat 230
Met Ser Ala Gly Pro Leu Gln Ala Ala Pro Lys Lys Asn
1               5                   10
tac gga gca ctg ata ggc gcg ggg ccg gcg gtg ggc ggg gcg gca ttc 278
Tyr Gly Ala Leu Ile Gly Ala Gly Pro Ala Val Gly Gly Ala Ala Phe
    15                  20                  25
aac cgg acg ctg agc gag gtt gcg agc tac gag cgc agc cgg cgc gac 326
Asn Arg Thr Leu Ser Glu Val Ala Ser Tyr Glu Arg Ser Arg Arg Asp
30                  35                  40                  45
cac gcg acg ccg gac tac cgg atc aag atc gtg gtg gtg ggg gac ggc 374
His Ala Thr Pro Asp Tyr Arg Ile Lys Ile Val Val Val Gly Asp Gly
                50                  55                  60
gcg acg ggg aag acg tct ctg ctg atg tcg tac aca cag ggc cag ttc 422
Ala Thr Gly Lys Thr Ser Leu Leu Met Ser Tyr Thr Gln Gly Gln Phe
            65                  70                  75
cca gag gac tac gtg ccg acc atc ttc gag aac tac gtg acg aac att 470
Pro Glu Asp Tyr Val Pro Thr Ile Phe Glu Asn Tyr Val Thr Asn Ile
        80                  85                  90
gag ggc cca cgc ggg aag gtg atc gag ctg gcg ctg tgg gac act gct 518
Glu Gly Pro Arg Gly Lys Val Ile Glu Leu Ala Leu Trp Asp Thr Ala
    95                  100                 105
ggg cag gag gag tac agc cgg ctg cgg ccg ctg tct tac ggg gac gtg 566
Gly Gln Glu Glu Tyr Ser Arg Leu Arg Pro Leu Ser Tyr Gly Asp Val
110                 115                 120                 125
gac atc gtg atg gtg tgc tac gcg gca gac aac cgc acg tcg ctg acg 614
Asp Ile Val Met Val Cys Tyr Ala Ala Asp Asn Arg Thr Ser Leu Thr
                130                 135                 140
aac gcg gaa gag ctg tgg ttc ccg gag gtg cgc cac ttc tgc ccg cac 662
Asn Ala Glu Glu Leu Trp Phe Pro Glu Val Arg His Phe Cys Pro His
            145                 150                 155
gcg ccg atg atg ctg gtg ggg ctc aag agc gac ctg tac tcg ctt gat 710
Ala Pro Met Met Leu Val Gly Leu Lys Ser Asp Leu Tyr Ser Leu Asp
        160                 165                 170
gcg ctg gac cgg ctc gtg gac ccg acg gac gcg gaa ctg gtg gcg cgc 758
Ala Leu Asp Arg Leu Val Asp Pro Thr Asp Ala Glu Leu Val Ala Arg
    175                 180                 185
aag atg ggc gcc ttc gtg cac ctg cag tgc tcg gcc aag acg cga cag 806
Lys Met Gly Ala Phe Val His Leu Gln Cys Ser Ala Lys Thr Arg Gln
190                 195                 200                 205
tgc ctg gag gac gtg ttc aac acg gcg ata cac acg gcg ctg tac gac 854
Cys Leu Glu Asp Val Phe Asn Thr Ala Ile His Thr Ala Leu Tyr Asp
                210                 215                 220
gag ctg cgc gcg ccc ccg cag cgc ggg gtg aag ggc atg ttc aag aag 902
Glu Leu Arg Ala Pro Pro Gln Arg Gly Val Lys Gly Met Phe Lys Lys

            225                 230                 235
aag cag cag cgg gac ccg cag gcg cag tct tac aag cgg gtg cgc aag 950
Lys Gln Gln Arg Asp Pro Gln Ala Gln Ser Tyr Lys Arg Val Arg Lys
        240                 245                 250
cac cgc tgt gtg gtc cta tagcgcacgt gacagccatg tttcgccggc 998
His Arg Cys Val Val Leu
    255
cctggttcct ggcagcggag gcgcaagcgc cgcgccggcg cgcctagtcc gctgtcgagg 1058

cctgcgtgac cgtgtagtta ggcatctagt cgtagcgagc caagatgtta gtttaaatcg 1118

atcggtgcga cagcatcttg ttaggataga gtactggga 1157

<210> 288
<211> 259
<212> PRT
<213> Ashbya gossypii

<400> 288
Met Ser Ala Gly Pro Leu Gln Ala Ala Pro Lys Lys Asn Tyr Gly Ala
1               5                   10                  15
Leu Ile Gly Ala Gly Pro Ala Val Gly Gly Ala Ala Phe Asn Arg Thr
            20                  25                  30
Leu Ser Glu Val Ala Ser Tyr Glu Arg Ser Arg Arg Asp His Ala Thr
        35                  40                  45
Pro Asp Tyr Arg Ile Lys Ile Val Val Val Gly Asp Gly Ala Thr Gly
    50                  55                  60
Lys Thr Ser Leu Leu Met Ser Tyr Thr Gln Gly Gln Phe Pro Glu Asp
65                  70                  75                  80
Tyr Val Pro Thr Ile Phe Glu Asn Tyr Val Thr Asn Ile Glu Gly Pro
                85                  90                  95
Arg Gly Lys Val Ile Glu Leu Ala Leu Trp Asp Thr Ala Gly Gln Glu
            100                 105                 110
Glu Tyr Ser Arg Leu Arg Pro Leu Ser Tyr Gly Asp Val Asp Ile Val
        115                 120                 125
Met Val Cys Tyr Ala Ala Asp Asn Arg Thr Ser Leu Thr Asn Ala Glu
    130                 135                 140
Glu Leu Trp Phe Pro Glu Val Arg His Phe Cys Pro His Ala Pro Met
145                 150                 155                 160
Met Leu Val Gly Leu Lys Ser Asp Leu Tyr Ser Leu Asp Ala Leu Asp
                165                 170                 175
Arg Leu Val Asp Pro Thr Asp Ala Glu Leu Val Ala Arg Lys Met Gly
            180                 185                 190
Ala Phe Val His Leu Gln Cys Ser Ala Lys Thr Arg Gln Cys Leu Glu
        195                 200                 205
Asp Val Phe Asn Thr Ala Ile His Thr Ala Leu Tyr Asp Glu Leu Arg
    210                 215                 220
Ala Pro Pro Gln Arg Gly Val Lys Gly Met Phe Lys Lys Lys Gln Gln
225                 230                 235                 240
Arg Asp Pro Gln Ala Gln Ser Tyr Lys Arg Val Arg Lys His Arg Cys
                245                 250                 255
Val Val Leu

<210> 289
<211> 1067
<212> DNA
<213> Oryza sativa

<220>
<221> CDS
<222> (131)..(775)

<400> 289
aagaacgaac aactcttctc cccctctccc cctccttcct tgctcctcct cccatcttgt      60

ctcctccctc tctctcgcaa agtcgcaacg cgctcctcct cggtgaaacg ggagtgaggt     120

gcgtgcggcc atg gcg tcc agc gcc tcc cgg ttc atc aag tgc gtc acg        169
           Met Ala Ser Ser Ala Ser Arg Phe Ile Lys Cys Val Thr
           1               5                   10
gtg ggc gac ggc gcc gtg ggc aag acc tgc atg ctc atc tgc tac acc       217
Val Gly Asp Gly Ala Val Gly Lys Thr Cys Met Leu Ile Cys Tyr Thr
    15                  20                  25
agc aac aag ttc ccc act gac tac ata cca acg gtg ttc gac aat ttc       265
Ser Asn Lys Phe Pro Thr Asp Tyr Ile Pro Thr Val Phe Asp Asn Phe
30                  35                  40                  45
agc gca aac gtt gtt gtg gac agc acc acg gtg aat ctg ggc ctc tgg       313
Ser Ala Asn Val Val Val Asp Ser Thr Thr Val Asn Leu Gly Leu Trp
                50                  55                  60
gat act gct ggg caa gag gat tac aac cgg ctc agg cct ctg agc tat       361
Asp Thr Ala Gly Gln Glu Asp Tyr Asn Arg Leu Arg Pro Leu Ser Tyr
            65                  70                  75
cgt ggt gct gat gtt ttc gtg ctt gcc ttc tct ctt gtg agc cga gct       409
Arg Gly Ala Asp Val Phe Val Leu Ala Phe Ser Leu Val Ser Arg Ala
        80                  85                  90
agc tat gaa aat att atg aag aag tgg ata ccg gag cta cag cat tat       457
Ser Tyr Glu Asn Ile Met Lys Lys Trp Ile Pro Glu Leu Gln His Tyr
    95                  100                 105
gca ccc ggc gtg ccc att gtg ttg gtt ggc aca aaa ttg gat ctc cgt       505
Ala Pro Gly Val Pro Ile Val Leu Val Gly Thr Lys Leu Asp Leu Arg
110                 115                 120                 125
gaa gac aag cac tac ttg ttg gac cat cct ggc atg ata cct gtt acc       553
Glu Asp Lys His Tyr Leu Leu Asp His Pro Gly Met Ile Pro Val Thr
                130                 135                 140
aca gca cag ggt gaa gaa ctt cga aaa caa ata ggt gct gct tat tac       601
Thr Ala Gln Gly Glu Glu Leu Arg Lys Gln Ile Gly Ala Ala Tyr Tyr
            145                 150                 155
att gag tgc agc tca aag aca caa cag aat gtc aaa ggt gtg ttt gat       649
Ile Glu Cys Ser Ser Lys Thr Gln Gln Asn Val Lys Gly Val Phe Asp
        160                 165                 170
gct gct atc aag gta gta atc cag cct cca act aag cag agg gaa aag       697
Ala Ala Ile Lys Val Val Ile Gln Pro Pro Thr Lys Gln Arg Glu Lys
    175                 180                 185
aag aaa aag aaa tca cga caa gga tgc tct atg atg aac atg ttc cgt       745
Lys Lys Lys Lys Ser Arg Gln Gly Cys Ser Met Met Asn Met Phe Arg
190                 195                 200                 205
gga agg aaa atg tca tgc ttc aaa tcc tgattgatcg agatgtccct             792
Gly Arg Lys Met Ser Cys Phe Lys Ser
                210
tacatgatgc aatgttctgg gtgcaacctc aagctggcaa cctttggagt ctgaatgtgc     852

tggtagagtt agatgcaaag ttcttgtgta tatttgcatc ttttggtaat taattactag     912

agttagatgg ttaaactagt ctgcttatgt accgcacaat gcttnttatt attagctctt     972

gttggtctag aaagtctgat agatgccaaa ggaacaagga gacaccgttc tgtcaggaca    1032

aaatcacaga ttttgtaatc aaaaaaaaaa aaaaa                               1067

<210> 290
<211> 214

<212> PRT
<213> Oryza sativa

<400> 290
Met Ala Ser Ser Ala Ser Arg Phe Ile Lys Cys Val Thr Val Gly Asp
1               5                   10                  15
Gly Ala Val Gly Lys Thr Cys Met Leu Ile Cys Tyr Thr Ser Asn Lys
            20                  25                  30
Phe Pro Thr Asp Tyr Ile Pro Thr Val Phe Asp Asn Phe Ser Ala Asn
        35                  40                  45
Val Val Val Asp Ser Thr Thr Val Asn Leu Gly Leu Trp Asp Thr Ala
    50                  55                  60
Gly Gln Glu Asp Tyr Asn Arg Leu Arg Pro Leu Ser Tyr Arg Gly Ala
65                  70                  75                  80
Asp Val Phe Val Leu Ala Phe Ser Leu Val Ser Arg Ala Ser Tyr Glu
                85                  90                  95
Asn Ile Met Lys Lys Trp Ile Pro Glu Leu Gln His Tyr Ala Pro Gly
            100                 105                 110
Val Pro Ile Val Leu Val Gly Thr Lys Leu Asp Leu Arg Glu Asp Lys
        115                 120                 125
His Tyr Leu Leu Asp His Pro Gly Met Ile Pro Val Thr Thr Ala Gln
    130                 135                 140
Gly Glu Glu Leu Arg Lys Gln Ile Gly Ala Ala Tyr Tyr Ile Glu Cys
145                 150                 155                 160
Ser Ser Lys Thr Gln Gln Asn Val Lys Gly Val Phe Asp Ala Ala Ile
                165                 170                 175
Lys Val Val Ile Gln Pro Pro Thr Lys Gln Arg Glu Lys Lys Lys Lys
            180                 185                 190
Lys Ser Arg Gln Gly Cys Ser Met Met Asn Met Phe Arg Gly Arg Lys
        195                 200                 205
Met Ser Cys Phe Lys Ser
    210

<210> 291
<211> 1087
<212> DNA
<213> Oryza sativa

<220>
<221> CDS
<222> (141)..(785)

<400> 291
aacaaagcag cttgccaccg tccctgcctc tcctgctttg ctttgccttc tcggagaaga      60

caaatctact gtagctgctt gtctctgttc gccggtggcg accgtgagag gaggaggtag      120

tcctagtcct agtaggcgag atg agc ggc gcc acc aag ttc atc aag tgc gtc      173
                      Met Ser Gly Ala Thr Lys Phe Ile Lys Cys Val
                      1               5                   10
acc gtc ggc gac ggc gcc gtc ggc aag acc tgc atg ctc atc tgc tac      221
Thr Val Gly Asp Gly Ala Val Gly Lys Thr Cys Met Leu Ile Cys Tyr
            15                  20                  25
acc agc aac aag ttc ccc acc gat tac atc ccc acc gtg ttc gac aac      269
Thr Ser Asn Lys Phe Pro Thr Asp Tyr Ile Pro Thr Val Phe Asp Asn
        30                  35                  40
ttc agt gct aat gtt tca gtg gat ggg aac atc gtc aac ttg gga tta      317
Phe Ser Ala Asn Val Ser Val Asp Gly Asn Ile Val Asn Leu Gly Leu
    45                  50                  55
tgg gac act gct gga caa gag gat tac agc agg ctg agg cca ctg agc      365
Trp Asp Thr Ala Gly Gln Glu Asp Tyr Ser Arg Leu Arg Pro Leu Ser
60                  65                  70                  75
tac agg gga gcg gat ata ttt gtg ctg gca ttc tca ctg atc agc aga      413
Tyr Arg Gly Ala Asp Ile Phe Val Leu Ala Phe Ser Leu Ile Ser Arg
                80                  85                  90
gca agc tat gag aat gtt ctc aag aag tgg atg ccg gag ctt cgc cgg      461

Ala Ser Tyr Glu Asn Val Leu Lys Lys Trp Met Pro Glu Leu Arg Arg
            95                  100                 105
ttc gca ccg aat gtt cca att gtt ctt gtt ggg acc aag tta gat cta 509
Phe Ala Pro Asn Val Pro Ile Val Leu Val Gly Thr Lys Leu Asp Leu
        110                 115                 120
cgt gac cac aga tct tac ctt gcg gac cat cct gct gct tcc gca att 557
Arg Asp His Arg Ser Tyr Leu Ala Asp His Pro Ala Ala Ser Ala Ile
    125                 130                 135
acg act gca cag ggt gaa gaa ctt aga aag cag ata ggc gcc gcg gcc 605
Thr Thr Ala Gln Gly Glu Glu Leu Arg Lys Gln Ile Gly Ala Ala Ala
140                 145                 150                 155
tac atc gaa tgc agt tcg aaa aca caa cag aac atc aag gcc gtg ttt 653
Tyr Ile Glu Cys Ser Ser Lys Thr Gln Gln Asn Ile Lys Ala Val Phe
                160                 165                 170
gat act gcc atc aag gtg gtc ctt cag cct cct cgg aga agg ggg gag 701
Asp Thr Ala Ile Lys Val Val Leu Gln Pro Pro Arg Arg Arg Gly Glu
            175                 180                 185
acg acg atg gca agg aag aag aca agg cga agc acc ggc tgc tcg tta 749
Thr Thr Met Ala Arg Lys Lys Thr Arg Arg Ser Thr Gly Cys Ser Leu
        190                 195                 200
aag aac ttg atg tgt ggc agt gca tgt gtt gtt taggacctgt ctgaattctt 802
Lys Asn Leu Met Cys Gly Ser Ala Cys Val Val
    205                 210
gaaggtgtgt aagcgttgtg taatggcaag tgaatttgct gactgttctg ctgcttccat 862

ttactactac taagctcttt cgtgtgtttg aattactaca tctaaaacta gagcatcagg 922

ctcatagact gacttcatat caggtgtttg tatcattaat a[?]tattatgt aagaaggttg 982

tggtttgtgg aacaactaaa tacaacaatg caggctatgt tgtactatgc tctgctactg 1042

gtgtttgtta taaatatcac atcctgctaa aaaaaaaaaa aaaaa 1087

<210> 292
<211> 214
<212> PRT
<213> Oryza sativa

<400> 292
Met Ser Gly Ala Thr Lys Phe Ile Lys Cys Val Thr Val Gly Asp Gly
1               5                   10                  15
Ala Val Gly Lys Thr Cys Met Leu Ile Cys Tyr Thr Ser Asn Lys Phe
            20                  25                  30
Pro Thr Asp Tyr Ile Pro Thr Val Phe Asp Asn Phe Ser Ala Asn Val
        35                  40                  45
Ser Val Asp Gly Asn Ile Val Asn Leu Gly Leu Trp Asp Thr Ala Gly
    50                  55                  60
Gln Glu Asp Tyr Ser Arg Leu Arg Pro Leu Ser Tyr Arg Gly Ala Asp
65                  70                  75                  80
Ile Phe Val Leu Ala Phe Ser Leu Ile Ser Arg Ala Ser Tyr Glu Asn
                85                  90                  95
Val Leu Lys Lys Trp Met Pro Glu Leu Arg Arg Phe Ala Pro Asn Val
            100                 105                 110
Pro Ile Val Leu Val Gly Thr Lys Leu Asp Leu Arg Asp His Arg Ser
        115                 120                 125
Tyr Leu Ala Asp His Pro Ala Ala Ser Ala Ile Thr Thr Ala Gln Gly
    130                 135                 140
Glu Glu Leu Arg Lys Gln Ile Gly Ala Ala Ala Tyr Ile Glu Cys Ser
145                 150                 155                 160
Ser Lys Thr Gln Gln Asn Ile Lys Ala Val Phe Asp Thr Ala Ile Lys
                165                 170                 175

Val Val Leu Gln Pro Pro Arg Arg Arg Gly Glu Thr Thr Met Ala Arg
            180                 185                 190
Lys Lys Thr Arg Arg Ser Thr Gly Cys Ser Leu Lys Asn Leu Met Cys
        195                 200                 205
Gly Ser Ala Cys Val Val
    210

<210> 293
<211> 1059
<212> DNA
<213> Cicer arietinum

<220>
<221> CDS
<222> (118)..(711)

<400> 293
cttcttcatc ccttcttctt gatcattcat tcgttcattc accaagttcg aacttgaacg      60

cacaacagca ttaccacatg agtaattagg gtttatacag caacaagaaa cataaaa        117

atg agt ggg tcc agg ttc att aag tgt gtt acc gta ggt gac ggt gcc       165
Met Ser Gly Ser Arg Phe Ile Lys Cys Val Thr Val Gly Asp Gly Ala
1               5                   10                  15
gta ggc aag act tgt ttg ctt atc tcc tac acc agc aac act ttc cct       213
Val Gly Lys Thr Cys Leu Leu Ile Ser Tyr Thr Ser Asn Thr Phe Pro
            20                  25                  30
acg gac tat gtg cct act gtc ttt gat aat ttc agt gca aat gta gtt       261
Thr Asp Tyr Val Pro Thr Val Phe Asp Asn Phe Ser Ala Asn Val Val
        35                  40                  45
gtg gat ggg agc act ata aat ctg ggt ttg tgg gat act gct ggc caa       309
Val Asp Gly Ser Thr Ile Asn Leu Gly Leu Trp Asp Thr Ala Gly Gln
    50                  55                  60
gaa gat tac aat aga tta aga ccc tta agc tat cgt gga gcg gat gtt       357
Glu Asp Tyr Asn Arg Leu Arg Pro Leu Ser Tyr Arg Gly Ala Asp Val
65                  70                  75                  80
ttc ctg ctt gct ttc tca ctc ata agc agg gcc agc tat gaa aat atc       405
Phe Leu Leu Ala Phe Ser Leu Ile Ser Arg Ala Ser Tyr Glu Asn Ile
                85                  90                  95
gcc aag aaa tgg att cct gag ttg agg cat tat gct cct ggt gtt cca       453
Ala Lys Lys Trp Ile Pro Glu Leu Arg His Tyr Ala Pro Gly Val Pro
            100                 105                 110
att att ctc gtt gga aca aaa ctt gat ctt cgg gat gat aag caa ttc       501
Ile Ile Leu Val Gly Thr Lys Leu Asp Leu Arg Asp Asp Lys Gln Phe
        115                 120                 125
ttt caa gac cat cct ggt gcg gtg cca atc acc aca gca cag ggt gag       549
Phe Gln Asp His Pro Gly Ala Val Pro Ile Thr Thr Ala Gln Gly Glu
    130                 135                 140
gaa ctg aga aag ctt att ggt gct cca gtt tac att gaa tgt agt tcc       597
Glu Leu Arg Lys Leu Ile Gly Ala Pro Val Tyr Ile Glu Cys Ser Ser
145                 150                 155                 160
aaa aca caa aag aat gtg aag gct gtt ttt gat gcg gcc atc aaa gta       645
Lys Thr Gln Lys Asn Val Lys Ala Val Phe Asp Ala Ala Ile Lys Val
                165                 170                 175
gtt cta cag ccg cca aag caa aag aaa act aag aga aag ggg caa aaa       693
Val Leu Gln Pro Pro Lys Gln Lys Lys Thr Lys Arg Lys Gly Gln Lys
            180                 185                 190
gcc tgt tcc att ttg tgatcttcag ttcttcatat tgtagttggt ggagaggacg       748
Ala Cys Ser Ile Leu
        195
acatgatagt aaccatttcc tggtagtctt tgccattctg attctatatc ttaccctatt     808

ttcaacctac atggaggaat cgacgtgtcc aactgagtcc taactacata aggtttgctt     868

ataagcatca agctatttgt ctctggctga aggatatatg taaagtcata atttcaaaca 928

tgtttggtgt gtagaacaac tcttgtgttt ctctgggatg tttttacctg ttgtattgat 988

tattaaatga ccatctccaa ttgtggacat gggttttact cacattgtaa tctaatctag 1048

gaagtgcttg c 1059

<210> 294
<211> 197
<212> PRT
<213> Cicer arietinum

<400> 294
Met Ser Gly Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Ser Thr Ile Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Arg Ala Ser Tyr Glu Asn Ile
                85                  90                  95
Ala Lys Lys Trp Ile Pro Glu Leu Arg His Tyr Ala Pro Gly Val Pro
            100                 105                 110
Ile Ile Leu Val Gly Thr Lys Leu Asp Leu Arg Asp Asp Lys Gln Phe
        115                 120                 125
Phe Gln Asp His Pro Gly Ala Val Pro Ile Thr Thr Ala Gln Gly Glu
    130                 135                 140
Glu Leu Arg Lys Leu Ile Gly Ala Pro Val Tyr Ile Glu Cys Ser Ser
145                 150                 155                 160
Lys Thr Gln Lys Asn Val Lys Ala Val Phe Asp Ala Ala Ile Lys Val
                165                 170                 175
Val Leu Gln Pro Pro Lys Gln Lys Lys Thr Lys Arg Lys Gly Gln Lys
            180                 185                 190
Ala Cys Ser Ile Leu
        195

<210> 295
<211> 805
<212> DNA
<213> Wuchereria bancrofti

<220>
<221> CDS
<222> (26)..(601)

<400> 295
cggcacgagc aagtttgagg taaag atg cag act atc aaa tgt gta gtg gtt 52
Met Gln Thr Ile Lys Cys Val Val Val
1               5
gga gat ggt gct gtc ggc aaa aca tgc ctt ctt att tcg tat act acc 100
Gly Asp Gly Ala Val Gly Lys Thr Cys Leu Leu Ile Ser Tyr Thr Thr
10                  15                  20                  25
aac aag ttt ccc agt gaa tat gta ccc act gta ttt gac aat tat gct 148
Asn Lys Phe Pro Ser Glu Tyr Val Pro Thr Val Phe Asp Asn Tyr Ala
                30                  35                  40
gta aca gta atg att gga gga gaa cca tat acg ctt ggt ttg ttc gat 196
Val Thr Val Met Ile Gly Gly Glu Pro Tyr Thr Leu Gly Leu Phe Asp

            45                  50                  55
aca gcc ggt caa gag gat tat gat aga ttg cgt cct ctt tca tat ccg 244
Thr Ala Gly Gln Glu Asp Tyr Asp Arg Leu Arg Pro Leu Ser Tyr Pro
        60                  65                  70
caa aca gat gta ttt tta gtg tgc ttt tct gtc gtt gca cct tca tct 292
Gln Thr Asp Val Phe Leu Val Cys Phe Ser Val Val Ala Pro Ser Ser
    75                  80                  85
ttc gaa aat gtg aaa gag aag tgg gtg ccg gag ata gca cat cac tgc 340
Phe Glu Asn Val Lys Glu Lys Trp Val Pro Glu Ile Ala His His Cys
90                  95                  100                 105
atg aag aca ccg ttc ctg ctg gtc gga act cag att gat ctt cgt gac 388
Met Lys Thr Pro Phe Leu Leu Val Gly Thr Gln Ile Asp Leu Arg Asp
                110                 115                 120
gat cct tcc tac atc gaa aaa ttg gca aaa atc aag caa cga cca att 436
Asp Pro Ser Tyr Ile Glu Lys Leu Ala Lys Ile Lys Gln Arg Pro Ile
            125                 130                 135
aca ttc gaa gtt gga gag aag tta gcg aaa gaa tta aag gca gtg aaa 484
Thr Phe Glu Val Gly Glu Lys Leu Ala Lys Glu Leu Lys Ala Val Lys
        140                 145                 150
tac gtc gaa tgt tct gca ctc aca cag aaa ggt cta aaa aat gta ttt 532
Tyr Val Glu Cys Ser Ala Leu Thr Gln Lys Gly Leu Lys Asn Val Phe
    155                 160                 165
gat gaa gca ata ctg gca gca ttg gaa cca ccg gca cag gag aaa aag 580
Asp Glu Ala Ile Leu Ala Ala Leu Glu Pro Pro Ala Gln Glu Lys Lys
170                 175                 180                 185
aag aaa tgt act ata ctt tagcatcatt gtttctcgat cacgttatat 628
Lys Lys Cys Thr Ile Leu
                190
tgatttctga tttgttagtc atttatctgt aaatgattat gttaacgtct taaatgctgc 688

acataccatt actacgtccc ctcttctggt tggtccatct gcgcatatgt gtagtgtttc 748

catctatgcg atatacgtgt agaatgaatg tattcctgta caaaaaaaaa aaaaaaa 805

<210> 296
<211> 191
<212> PRT
<213> Wuchereria bancrofti

<400> 296
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Glu Tyr
            20                  25                  30
Val Pro Thr Val Phe Asp Asn Tyr Ala Val Thr Val Met Ile Gly Gly
        35                  40                  45
Glu Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Val Ala Pro Ser Ser Phe Glu Asn Val Lys Glu Lys
                85                  90                  95
Trp Val Pro Glu Ile Ala His His Cys Met Lys Thr Pro Phe Leu Leu
            100                 105                 110
Val Gly Thr Gln Ile Asp Leu Arg Asp Asp Pro Ser Tyr Ile Glu Lys
        115                 120                 125
Leu Ala Lys Ile Lys Gln Arg Pro Ile Thr Phe Glu Val Gly Glu Lys
    130                 135                 140
Leu Ala Lys Glu Leu Lys Ala Val Lys Tyr Val Glu Cys Ser Ala Leu
145                 150                 155                 160
Thr Gln Lys Gly Leu Lys Asn Val Phe Asp Glu Ala Ile Leu Ala Ala
                165                 170                 175
Leu Glu Pro Pro Ala Gln Glu Lys Lys Lys Lys Cys Thr Ile Leu

180                    185                    190

<210> 297
<211> 579
<212> DNA
<213> Emericella nidulans

<220>
<221> CDS
<222> (1)..(579)

<400> 297
atg gtc gta gca act atc aag tgt gtt gtg gtc ggt gat ggt gct gtc        48
Met Val Val Ala Thr Ile Lys Cys Val Val Val Gly Asp Gly Ala Val
1               5                   10                  15
ggg aag acc tgt ctc ctg atc tca tac aca acg aac aaa ttc cct tca        96
Gly Lys Thr Cys Leu Leu Ile Ser Tyr Thr Thr Asn Lys Phe Pro Ser
            20                  25                  30
gaa tat gtg ccc acc gtg ttc gat aac tat gct gtg act gtc atg atc        144
Glu Tyr Val Pro Thr Val Phe Asp Asn Tyr Ala Val Thr Val Met Ile
        35                  40                  45
ggc gat gag ccc tac act cta gga tta ttt gat act gct ggt caa gaa        192
Gly Asp Glu Pro Tyr Thr Leu Gly Leu Phe Asp Thr Ala Gly Gln Glu
    50                  55                  60
gat tat gac cga ctt cgt cct ctc tca tac ccg cag aca gat gta ttc        240
Asp Tyr Asp Arg Leu Arg Pro Leu Ser Tyr Pro Gln Thr Asp Val Phe
65                  70                  75                  80
ctt gtc tgc ttt tct gtg acc tcc ccc gca tct ttc gag aac gtg cgc        288
Leu Val Cys Phe Ser Val Thr Ser Pro Ala Ser Phe Glu Asn Val Arg
                85                  90                  95
gaa aag tgg ttc ccc gaa gtt cac cac cac tgc ccc ggc gtc cca tgc        336
Glu Lys Trp Phe Pro Glu Val His His His Cys Pro Gly Val Pro Cys
            100                 105                 110
ctg att gtt ggg aca caa gtc gat ctc cgg gat gac ccc gcc gtt cgc        384
Leu Ile Val Gly Thr Gln Val Asp Leu Arg Asp Asp Pro Ala Val Arg
        115                 120                 125
gac aag ctg gct cgg caa aag atg caa ccg att cgc aaa gaa gac ggt        432
Asp Lys Leu Ala Arg Gln Lys Met Gln Pro Ile Arg Lys Glu Asp Gly
    130                 135                 140
gac cgc atg gcc aag gat ctg ggc gcc gtg aaa tat gtc gaa tgc tcc        480
Asp Arg Met Ala Lys Asp Leu Gly Ala Val Lys Tyr Val Glu Cys Ser
145                 150                 155                 160
gca ttg acc caa tac aag ctc aag gat gtc ttt gac gag gct att gtt        528
Ala Leu Thr Gln Tyr Lys Leu Lys Asp Val Phe Asp Glu Ala Ile Val
                165                 170                 175
gcg gca ctc gaa ccg gcc cct aag aag agg tcg agg tgc gtt ctg cta        576
Ala Ala Leu Glu Pro Ala Pro Lys Lys Arg Ser Arg Cys Val Leu Leu
            180                 185                 190
tag                                                                    579

<210> 298
<211> 192
<212> PRT
<213> Emericella nidulans

<400> 298
Met Val Val Ala Thr Ile Lys Cys Val Val Val Gly Asp Gly Ala Val
1               5                   10                  15
Gly Lys Thr Cys Leu Leu Ile Ser Tyr Thr Thr Asn Lys Phe Pro Ser
            20                  25                  30
Glu Tyr Val Pro Thr Val Phe Asp Asn Tyr Ala Val Thr Val Met Ile
        35                  40                  45
Gly Asp Glu Pro Tyr Thr Leu Gly Leu Phe Asp Thr Ala Gly Gln Glu
    50                  55                  60
Asp Tyr Asp Arg Leu Arg Pro Leu Ser Tyr Pro Gln Thr Asp Val Phe

65                  70                  75                  80
Leu Val Cys Phe Ser Val Thr Ser Pro Ala Ser Phe Glu Asn Val Arg
                85                  90                  95
Glu Lys Trp Phe Pro Glu Val His His His Cys Pro Gly Val Pro Cys
            100                 105                 110
Leu Ile Val Gly Thr Gln Val Asp Leu Arg Asp Asp Pro Ala Val Arg
        115                 120                 125
Asp Lys Leu Ala Arg Gln Lys Met Gln Pro Ile Arg Lys Glu Asp Gly
    130                 135                 140
Asp Arg Met Ala Lys Asp Leu Gly Ala Val Lys Tyr Val Glu Cys Ser
145                 150                 155                 160
Ala Leu Thr Gln Tyr Lys Leu Lys Asp Val Phe Asp Glu Ala Ile Val
                165                 170                 175
Ala Ala Leu Glu Pro Ala Pro Lys Lys Arg Ser Arg Cys Val Leu Leu
            180                 185                 190

<210> 299
<211> 588
<212> DNA
<213> Drosophila melanogaster

<220>
<221> CDS
<222> (1)..(588)

<400> 299
atg tca acc gga agg ccc ata aag tgt gta gtc gtt ggt gac ggc acc      48
Met Ser Thr Gly Arg Pro Ile Lys Cys Val Val Val Gly Asp Gly Thr
1               5                   10                  15
gtc gga aag acc tgc atg cta atc tcc tac acg aca gac tgc ttt ccc      96
Val Gly Lys Thr Cys Met Leu Ile Ser Tyr Thr Thr Asp Cys Phe Pro
            20                  25                  30
ggc gaa tat gtg ccc aca gtc ttc gac aac tac tcg gcg ccc atg caa      144
Gly Glu Tyr Val Pro Thr Val Phe Asp Asn Tyr Ser Ala Pro Met Gln
        35                  40                  45
gtg gac aca ata cag gtc tcg ctg gga ctg tgg gat acg gcg ggt cag      192
Val Asp Thr Ile Gln Val Ser Leu Gly Leu Trp Asp Thr Ala Gly Gln
    50                  55                  60
gag gac tac gac cgc ctg aga ccg cta tcc tac ccg cag aca gac gtt      240
Glu Asp Tyr Asp Arg Leu Arg Pro Leu Ser Tyr Pro Gln Thr Asp Val
65                  70                  75                  80
ttc ctg ata tgc tac agc gtg gcg agt ccc tcg tcc ttt gag aac gtc      288
Phe Leu Ile Cys Tyr Ser Val Ala Ser Pro Ser Ser Phe Glu Asn Val
                85                  90                  95
acc tcg aaa tgg tat ccg gag ata aag cac cac tgt ccc gat gcg ccc      336
Thr Ser Lys Trp Tyr Pro Glu Ile Lys His His Cys Pro Asp Ala Pro
            100                 105                 110
atc att cta gtt ggc acc aaa atc gat ttg cgc gaa gat cga gag aca      384
Ile Ile Leu Val Gly Thr Lys Ile Asp Leu Arg Glu Asp Arg Glu Thr
        115                 120                 125
ctc agc ggc ctg gca gag cag gga ctg acg ccg ctg aag cgc gag cag      432
Leu Ser Gly Leu Ala Glu Gln Gly Leu Thr Pro Leu Lys Arg Glu Gln
    130                 135                 140
ggc cag aag ctg gca aac aag ata cgc gct gtg aaa tac atg gag tgc      480
Gly Gln Lys Leu Ala Asn Lys Ile Arg Ala Val Lys Tyr Met Glu Cys
145                 150                 155                 160
tcc gcc ttg acg cag cgc ggt ctc aag ccg gtg ttc gag gaa gcg gtg      528
Ser Ala Leu Thr Gln Arg Gly Leu Lys Pro Val Phe Glu Glu Ala Val
                165                 170                 175
cgc gcg gtg ctc aga cca gag ccg cta aag cga cgc cag cga aag tgt      576
Arg Ala Val Leu Arg Pro Glu Pro Leu Lys Arg Arg Gln Arg Lys Cys
            180                 185                 190
tta ata atg taa                                                      588
Leu Ile Met
        195

<210> 300

<211> 195
<212> PRT
<213> Drosophila melanogaster

<400> 300
Met Ser Thr Gly Arg Pro Ile Lys Cys Val Val Val Gly Asp Gly Thr
1               5                   10                  15
Val Gly Lys Thr Cys Met Leu Ile Ser Tyr Thr Thr Asp Cys Phe Pro
            20                  25                  30
Gly Glu Tyr Val Pro Thr Val Phe Asp Asn Tyr Ser Ala Pro Met Gln
        35                  40                  45
Val Asp Thr Ile Gln Val Ser Leu Gly Leu Trp Asp Thr Ala Gly Gln
    50                  55                  60
Glu Asp Tyr Asp Arg Leu Arg Pro Leu Ser Tyr Pro Gln Thr Asp Val
65                  70                  75                  80
Phe Leu Ile Cys Tyr Ser Val Ala Ser Pro Ser Ser Phe Glu Asn Val
                85                  90                  95
Thr Ser Lys Trp Tyr Pro Glu Ile Lys His His Cys Pro Asp Ala Pro
            100                 105                 110
Ile Ile Leu Val Gly Thr Lys Ile Asp Leu Arg Glu Asp Arg Glu Thr
        115                 120                 125
Leu Ser Gly Leu Ala Glu Gln Gly Leu Thr Pro Leu Lys Arg Glu Gln
    130                 135                 140
Gly Gln Lys Leu Ala Asn Lys Ile Arg Ala Val Lys Tyr Met Glu Cys
145                 150                 155                 160
Ser Ala Leu Thr Gln Arg Gly Leu Lys Pro Val Phe Glu Glu Ala Val
                165                 170                 175
Arg Ala Val Leu Arg Pro Glu Pro Leu Lys Arg Arg Gln Arg Lys Cys
            180                 185                 190
Leu Ile Met
        195

<210> 301
<211> 582
<212> DNA
<213> Xenopus laevis

<220>
<221> CDS
<222> (1)..(582)

<400> 301
atg gca gcc att cgt aag aag ctc gta att gtt gga gat ggt gca tgc      48
Met Ala Ala Ile Arg Lys Lys Leu Val Ile Val Gly Asp Gly Ala Cys
1               5                   10                  15
ggg aaa acc tgc ctt ctg att gtg ttc agt aaa gac cag ttt cca gaa      96
Gly Lys Thr Cys Leu Leu Ile Val Phe Ser Lys Asp Gln Phe Pro Glu
            20                  25                  30
gtg tat gtc cca aca gtt ttt gag aac tat gtg gca gac ata gaa gtg      144
Val Tyr Val Pro Thr Val Phe Glu Asn Tyr Val Ala Asp Ile Glu Val
        35                  40                  45
gat ggc aag cag gtt gag ttg gcc cta tgg gat aca gct ggt caa gaa      192
Asp Gly Lys Gln Val Glu Leu Ala Leu Trp Asp Thr Ala Gly Gln Glu
    50                  55                  60
gat tat gac aga ctg cga cct ctg tcc tat cca gac act gat gtt ata      240
Asp Tyr Asp Arg Leu Arg Pro Leu Ser Tyr Pro Asp Thr Asp Val Ile
65                  70                  75                  80
tta atg tgc ttt tcc att gat agc ccc gac agt tta gaa aac ata cct      288
Leu Met Cys Phe Ser Ile Asp Ser Pro Asp Ser Leu Glu Asn Ile Pro
                85                  90                  95
gag aaa tgg acc ccg gag gtg aaa cat ttc tgc ccc aat gtg ccc att      336
Glu Lys Trp Thr Pro Glu Val Lys His Phe Cys Pro Asn Val Pro Ile
            100                 105                 110
att tta gtt gga aac aag aag gat ctg cgt aat gat gaa cac act cgt      384
Ile Leu Val Gly Asn Lys Lys Asp Leu Arg Asn Asp Glu His Thr Arg
        115                 120                 125
agg gag ctc acc aaa atg aaa cag gag cct gtg aag ccc gaa gaa ggt      432
Arg Glu Leu Thr Lys Met Lys Gln Glu Pro Val Lys Pro Glu Glu Gly

130                 135                 140
cgt gac atg gca aac cgt atc tcc gcc tac gcg tac atg gaa tgc tct       480
Arg Asp Met Ala Asn Arg Ile Ser Ala Tyr Ala Tyr Met Glu Cys Ser
145                 150                 155                 160
gca aaa acg aaa gat ggc gtg agg gaa gtg ttt gag ctg gct aca cgg       528
Ala Lys Thr Lys Asp Gly Val Arg Glu Val Phe Glu Leu Ala Thr Arg
                165                 170                 175
gca gcc ctg caa gct agg cgt ggc aag aag aaa cca cgt tgc ctt ctc       576
Ala Ala Leu Gln Ala Arg Arg Gly Lys Lys Lys Pro Arg Cys Leu Leu
            180                 185                 190
atc taa                                                               582
Ile

<210> 302
<211> 193
<212> PRT
<213> Xenopus laevis

<400> 302
Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Lys His Phe Cys Pro Asn Val Pro Ile
            100                 105                 110
Ile Leu Val Gly Asn Lys Lys Asp Leu Arg Asn Asp Glu His Thr Arg
        115                 120                 125
Arg Glu Leu Thr Lys Met Lys Gln Glu Pro Val Lys Pro Glu Glu Gly
    130                 135                 140
Arg Asp Met Ala Asn Arg Ile Ser Ala Tyr Ala Tyr Met Glu Cys Ser
145                 150                 155                 160
Ala Lys Thr Lys Asp Gly Val Arg Glu Val Phe Glu Leu Ala Thr Arg
                165                 170                 175
Ala Ala Leu Gln Ala Arg Arg Gly Lys Lys Lys Pro Arg Cys Leu Leu
            180                 185                 190
Ile

<210> 303
<211> 591
<212> DNA
<213> Physcomitrella patens

<220>
<221> CDS
<222> (1)..(591)

<400> 303
atg agc act tca cgg ttt atc aag tgc gtg act gtt gga gat gga gct       48
Met Ser Thr Ser Arg Phe Ile Lys Cys Val Thr Val Gly Asp Gly Ala
1               5                   10                  15
gtc ggg aag acg tgc atg ctt att tca tac acc agc aac aca ttt cct       96
Val Gly Lys Thr Cys Met Leu Ile Ser Tyr Thr Ser Asn Thr Phe Pro
            20                  25                  30
act gat tac gtt cct acc gtg ttt gac aac ttc agc gca aat gta gtg       144
Thr Asp Tyr Val Pro Thr Val Phe Asp Asn Phe Ser Ala Asn Val Val
        35                  40                  45
gtc gat gga aat acc gtc aac ctc ggg tta tgg gat aca gca ggt caa       192

Val Asp Gly Asn Thr Val Asn Leu Gly Leu Trp Asp Thr Ala Gly Gln
    50                  55                  60
gaa gat tac aac agg ctt cgt cct ctg agt tac agg ggt gct gat gtt      240
Glu Asp Tyr Asn Arg Leu Arg Pro Leu Ser Tyr Arg Gly Ala Asp Val
65                  70                  75                  80
ttt ctc ctg gcg ttc tcc ctc atc agc aag gct agt tat gaa aac ata      288
Phe Leu Leu Ala Phe Ser Leu Ile Ser Lys Ala Ser Tyr Glu Asn Ile
                85                  90                  95
tca aag aag tgg atc ccg gaa ctg aga cat tac gcg cca tct gtg cca      336
Ser Lys Lys Trp Ile Pro Glu Leu Arg His Tyr Ala Pro Ser Val Pro
            100                 105                 110
atc att ctc gtc gga aca aaa ctt gat ctt cgc gat gac aaa caa ttc      384
Ile Ile Leu Val Gly Thr Lys Leu Asp Leu Arg Asp Asp Lys Gln Phe
        115                 120                 125
ttt gct gat cat cct gga gcg gct cca ata act act tct caa ggg gag      432
Phe Ala Asp His Pro Gly Ala Ala Pro Ile Thr Thr Ser Gln Gly Glu
    130                 135                 140
gag ctc agg aag tcg att ggg gcg gcc tcg tac ata gag tgc agc tca      480
Glu Leu Arg Lys Ser Ile Gly Ala Ala Ser Tyr Ile Glu Cys Ser Ser
145                 150                 155                 160
aag act cag cag aat gta aaa gca gtt ttt gac gca gca atc aag gtg      528
Lys Thr Gln Gln Asn Val Lys Ala Val Phe Asp Ala Ala Ile Lys Val
                165                 170                 175
gtt ctc caa cca cca aag cag aag aag aag aag aaa aaa caa aag aat      576
Val Leu Gln Pro Pro Lys Gln Lys Lys Lys Lys Lys Lys Gln Lys Asn
            180                 185                 190
tgc gtc att ctg tga                                                  591
Cys Val Ile Leu
        195


<210> 304
<211> 196
<212> PRT
<213> Physcomitrella patens

<400> 304
Met Ser Thr Ser Arg Phe Ile Lys Cys Val Thr Val Gly Asp Gly Ala
1               5                   10                  15
Val Gly Lys Thr Cys Met Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Asn Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Ile
                85                  90                  95
Ser Lys Lys Trp Ile Pro Glu Leu Arg His Tyr Ala Pro Ser Val Pro
            100                 105                 110
Ile Ile Leu Val Gly Thr Lys Leu Asp Leu Arg Asp Asp Lys Gln Phe
        115                 120                 125
Phe Ala Asp His Pro Gly Ala Ala Pro Ile Thr Thr Ser Gln Gly Glu
    130                 135                 140
Glu Leu Arg Lys Ser Ile Gly Ala Ala Ser Tyr Ile Glu Cys Ser Ser
145                 150                 155                 160
Lys Thr Gln Gln Asn Val Lys Ala Val Phe Asp Ala Ala Ile Lys Val
                165                 170                 175
Val Leu Gln Pro Pro Lys Gln Lys Lys Lys Lys Lys Lys Gln Lys Asn
            180                 185                 190
Cys Val Ile Leu
        195

<210> 305
<211> 841
<212> DNA
<213> Schistosoma mansoni

<220>
<221> CDS
<222> (7)..(588)

<400> 305
gcaaca atg gcg agt gcg gta cgt aag aaa ctt gtt att gtt gga gat 48
Met Ala Ser Ala Val Arg Lys Lys Leu Val Ile Val Gly Asp
1               5                   10
ggt gca tgc ggg aaa act tgt tta ctt att gta ttc agc aaa gac cag 96
Gly Ala Cys Gly Lys Thr Cys Leu Leu Ile Val Phe Ser Lys Asp Gln
15                  20                  25                  30
ttt ccc gaa gtt tac gtt cct act gta ttc gaa aac tat gtt gca gat 144
Phe Pro Glu Val Tyr Val Pro Thr Val Phe Glu Asn Tyr Val Ala Asp
                35                  40                  45
atc gaa gtt gat aac aga caa gtt gaa tta gct ctc tgg gac act gct 192
Ile Glu Val Asp Asn Arg Gln Val Glu Leu Ala Leu Trp Asp Thr Ala
            50                  55                  60
ggc caa gaa gat tat gat agg cta cgg cca ctt tct tat cct gat acg 240
Gly Gln Glu Asp Tyr Asp Arg Leu Arg Pro Leu Ser Tyr Pro Asp Thr
        65                  70                  75
gat gta gtt ctg tta tgt tat agt att gac tct ccc gat agt ttt gca 288
Asp Val Val Leu Leu Cys Tyr Ser Ile Asp Ser Pro Asp Ser Phe Ala
    80                  85                  90
aac att gag gaa aaa tgg tta cca gaa atc cgt cat ttt tgt cct gat 336
Asn Ile Glu Glu Lys Trp Leu Pro Glu Ile Arg His Phe Cys Pro Asp
95                  100                 105                 110
gtt ccc att gtc tta gtt gga aac aaa aaa gac tta cga cat gac gag 384
Val Pro Ile Val Leu Val Gly Asn Lys Lys Asp Leu Arg His Asp Glu
                115                 120                 125
gct aca aag aat gag cta cac aga acc aaa caa ctt cct gtc act ttt 432
Ala Thr Lys Asn Glu Leu His Arg Thr Lys Gln Leu Pro Val Thr Phe
            130                 135                 140
aac gag ggt aaa caa gta gct gaa aag att tct gct tat gcc ttc ttt 480
Asn Glu Gly Lys Gln Val Ala Glu Lys Ile Ser Ala Tyr Ala Phe Phe
        145                 150                 155
gag tgt tca gct aag acc aag gaa gga gtc agc gat gtt ttc gta gca 528
Glu Cys Ser Ala Lys Thr Lys Glu Gly Val Ser Asp Val Phe Val Ala
    160                 165                 170
gct act cga gcc gcc ttg aat tca gcg aag aag aag agg agg agg tgt 576
Ala Thr Arg Ala Ala Leu Asn Ser Ala Lys Lys Lys Arg Arg Arg Cys
175                 180                 185                 190
gat tta att tgatctccac tgttctctaa attgtctaga tttgattttc cggtctccga 635
Asp Leu Ile

aacatacatt cccctcctaa ttcttcctca ggaccttaaa ttgctacttc tatcggctgc 695

gtgtttcttt tacaatcata ttttttgttt gccaacaaga caatccattc cacggaaata 755

tttgccattt atttttgcct tggtactaaa atagggcatg cctcatgtaa cttatattta 815

ttgagaataa attactataa tcagtc 841

<210> 306
<211> 193
<212> PRT
<213> Schistosoma mansoni

<400> 306
Met Ala Ser Ala Val Arg Lys Lys Leu Val Ile Val Gly Asp Gly Ala
1               5                   10                  15
Cys Gly Lys Thr Cys Leu Leu Ile Val Phe Ser Lys Asp Gln Phe Pro

                    20                  25                  30
Glu Val Tyr Val Pro Thr Val Phe Glu Asn Tyr Val Ala Asp Ile Glu
        35                  40                  45
Val Asp Asn Arg Gln Val Glu Leu Ala Leu Trp Asp Thr Ala Gly Gln
    50                  55                  60
Glu Asp Tyr Asp Arg Leu Arg Pro Leu Ser Tyr Pro Asp Thr Asp Val
65                  70                  75                  80
Val Leu Leu Cys Tyr Ser Ile Asp Ser Pro Asp Ser Phe Ala Asn Ile
                85                  90                  95
Glu Glu Lys Trp Leu Pro Glu Ile Arg His Phe Cys Pro Asp Val Pro
            100                 105                 110
Ile Val Leu Val Gly Asn Lys Lys Asp Leu Arg His Asp Glu Ala Thr
        115                 120                 125
Lys Asn Glu Leu His Arg Thr Lys Gln Leu Pro Val Thr Phe Asn Glu
    130                 135                 140
Gly Lys Gln Val Ala Glu Lys Ile Ser Ala Tyr Ala Phe Phe Glu Cys
145                 150                 155                 160
Ser Ala Lys Thr Lys Glu Gly Val Ser Asp Val Phe Val Ala Ala Thr
                165                 170                 175
Arg Ala Ala Leu Asn Ser Ala Lys Lys Lys Arg Arg Arg Cys Asp Leu
            180                 185                 190
Ile

<210> 307
<211> 576
<212> DNA
<213> Gallus gallus

<220>
<221> CDS
<222> (1)..(576)

<400> 307
atg cag acg att aag tgt gta gtt gtg ggt gat ggt gct gtt ggt aaa      48
Met Gln Thr Ile Lys Cys Val Val Val Gly Asp Gly Ala Val Gly Lys
1               5                   10                  15
acc tgt ctc tta att tct tac aca aca aat aaa ttt cca tcg gaa tac      96
Thr Cys Leu Leu Ile Ser Tyr Thr Thr Asn Lys Phe Pro Ser Glu Tyr
            20                  25                  30
gta cca acg gtt ttt gat aac tat gct gta aca gtg atg att gga gga      144
Val Pro Thr Val Phe Asp Asn Tyr Ala Val Thr Val Met Ile Gly Gly
        35                  40                  45
gag cct tac act cta ggc ctc ttt gat act gca ggt cag gaa gat tat      192
Glu Pro Tyr Thr Leu Gly Leu Phe Asp Thr Ala Gly Gln Glu Asp Tyr
    50                  55                  60
gat aga tta cga ccc ctc agc tat cca cag aca gat gta ttt ctg gtc      240
Asp Arg Leu Arg Pro Leu Ser Tyr Pro Gln Thr Asp Val Phe Leu Val
65                  70                  75                  80
tgt ttt tca gtg gta tct cct tct tca ttt gaa aat gtg aaa gaa aag      288
Cys Phe Ser Val Val Ser Pro Ser Ser Phe Glu Asn Val Lys Glu Lys
                85                  90                  95
tgg gta cct gaa att act cac cat tgt cca aag act cct ttt ctg ctt      336
Trp Val Pro Glu Ile Thr His His Cys Pro Lys Thr Pro Phe Leu Leu
            100                 105                 110
gtt ggg acc caa att gat cta aga gat gat ccc tca aca att gaa aaa      384
Val Gly Thr Gln Ile Asp Leu Arg Asp Asp Pro Ser Thr Ile Glu Lys
        115                 120                 125
ctt gcc aag aac aag cag aag ccc ata act cca gag acg gct gaa aaa      432
Leu Ala Lys Asn Lys Gln Lys Pro Ile Thr Pro Glu Thr Ala Glu Lys
    130                 135                 140
ctg gcc cgg gac ctg aag gct gtt aaa tat gtg gaa tgc tct gcg ctt      480
Leu Ala Arg Asp Leu Lys Ala Val Lys Tyr Val Glu Cys Ser Ala Leu
145                 150                 155                 160
acg cag aaa ggc cta aag aat gta ttt gat gag gcg ata ttg gct gcc      528
Thr Gln Lys Gly Leu Lys Asn Val Phe Asp Glu Ala Ile Leu Ala Ala
                165                 170                 175
ctg gag cct ccg gag ccg aag aag act cgc agg tgt gtg ctg cta          573

Leu Glu Pro Pro Glu Pro Lys Lys Thr Arg Arg Cys Val Leu Leu
            180                 185                 190
tga                                                                576

<210> 308
<211> 191
<212> PRT
<213> Gallus gallus

<400> 308
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Glu Tyr
            20                  25                  30
Val Pro Thr Val Phe Asp Asn Tyr Ala Val Thr Val Met Ile Gly Gly
        35                  40                  45
Glu Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Val Ser Pro Ser Ser Phe Glu Asn Val Lys Glu Lys
                85                  90                  95
Trp Val Pro Glu Ile Thr His His Cys Pro Lys Thr Pro Phe Leu Leu
            100                 105                 110
Val Gly Thr Gln Ile Asp Leu Arg Asp Asp Pro Ser Thr Ile Glu Lys
        115                 120                 125
Leu Ala Lys Asn Lys Gln Lys Pro Ile Thr Pro Glu Thr Ala Glu Lys
    130                 135                 140
Leu Ala Arg Asp Leu Lys Ala Val Lys Tyr Val Glu Cys Ser Ala Leu
145                 150                 155                 160
Thr Gln Lys Gly Leu Lys Asn Val Phe Asp Glu Ala Ile Leu Ala Ala
                165                 170                 175
Leu Glu Pro Pro Glu Pro Lys Lys Thr Arg Arg Cys Val Leu Leu
            180                 185                 190

<210> 309
<211> 2011
<212> DNA
<213> Candida albicans

<220>
<221> CDS
<222> (540)..(1580)

<400> 309
tctagatttc cttactgtaa atcttaagga tcttcaatga tctatcccta acttcaatta      60

tagttcacaa tacgctatga cgagcatcta aactatgtca atatctgaaa aggctaattt     120

tttgttttgt tttaaagcac tttgtgattt taaggatatt ttccttaaat attattttta     180

gaaataaata cagggacgac tgattggaaa taataaaaca taaacactgc ccatttcagg     240

cacgcaaaaa aacaaaaaaa ccgtgccctt ttctttttca tgttttcctt tcccgatttt     300

ttctctctct ctttatttct ttctctctag atttttttta ctgatctcac actatcacca     360

catatacaca ctcacacatt atataatctt caattataac tcatctgggt tctaacagtt     420

tctttttttt tctaacttgc ttttttttta ataaagtttc tataatttcg atacaccaat 480

tttgttttca ctatttagtt attgtaacgt tctagtttga aaatccattt ctataagat 539

atg aca ccc aac ggc agt agg aga cat tcg gcg tac atg ggg tcg ccc 587
Met Thr Pro Asn Gly Ser Arg Arg His Ser Ala Tyr Met Gly Ser Pro
1               5                   10                  15
aga agc cag cat agt tcc aca atg gaa aca ggt tac aat cct tac gaa 635
Arg Ser Gln His Ser Ser Thr Met Glu Thr Gly Tyr Asn Pro Tyr Glu
            20                  25                  30
gca gta cag aag aaa cag gaa tta tac caa aat aac aac ggc aat tca 683
Ala Val Gln Lys Lys Gln Glu Leu Tyr Gln Asn Asn Asn Gly Asn Ser
        35                  40                  45
cca acc gtc atc att gaa gaa gat cca tac atc cca aat tat aaa gag 731
Pro Thr Val Ile Ile Glu Glu Asp Pro Tyr Ile Pro Asn Tyr Lys Glu
    50                  55                  60
ctg tct cta gca aat aag aaa aca aat tat aac atg aaa att gtc gtt 779
Leu Ser Leu Ala Asn Lys Lys Thr Asn Tyr Asn Met Lys Ile Val Val
65                  70                  75                  80
gtc ggt gac ggt ggg tgt ggt aag acg tgt tta tta tta gca tac aca 827
Val Gly Asp Gly Gly Cys Gly Lys Thr Cys Leu Leu Leu Ala Tyr Thr
                85                  90                  95
caa aac aaa ttt cct tca atc tat gtt ccc aca gtt ttt gag aat tat 875
Gln Asn Lys Phe Pro Ser Ile Tyr Val Pro Thr Val Phe Glu Asn Tyr
            100                 105                 110
gtg acg gca gta cag tcg cct aat ggt aaa acc gtg gaa ttg gct ctc 923
Val Thr Ala Val Gln Ser Pro Asn Gly Lys Thr Val Glu Leu Ala Leu
        115                 120                 125
tgg gat act gct ggc caa gaa gaa tac gat agg tta cga cca cta agc 971
Trp Asp Thr Ala Gly Gln Glu Glu Tyr Asp Arg Leu Arg Pro Leu Ser
    130                 135                 140
tat cct gac gtt gat att tta ttg gtg tgt ttt gcc gtg gac aat gaa 1019
Tyr Pro Asp Val Asp Ile Leu Leu Val Cys Phe Ala Val Asp Asn Glu
145                 150                 155                 160
gtt agt ttg gag aat gtc aag gat atg tgg ttc ccc gaa gtg aat cat 1067
Val Ser Leu Glu Asn Val Lys Asp Met Trp Phe Pro Glu Val Asn His
                165                 170                 175
tat tgt cct ggt att ccc att att ttg gtt ggt acc aag agt gat tta 1115
Tyr Cys Pro Gly Ile Pro Ile Ile Leu Val Gly Thr Lys Ser Asp Leu
            180                 185                 190
ctg tct gat atg aat cac gat gca tca ata cga gta gcc aaa gaa att 1163
Leu Ser Asp Met Asn His Asp Ala Ser Ile Arg Val Ala Lys Glu Ile
        195                 200                 205
ggc gcc att gga ttg att ttc aca tca gcc aag acc atg ttc aat gta 1211
Gly Ala Ile Gly Leu Ile Phe Thr Ser Ala Lys Thr Met Phe Asn Val
    210                 215                 220
cgg act gtt ttt aat ttt gca tta aac cat ttt caa aga aat atg gaa 1259
Arg Thr Val Phe Asn Phe Ala Leu Asn His Phe Gln Arg Asn Met Glu
225                 230                 235                 240
ttg cag gaa caa tat gaa aag aca ttg ggt tca aga aag aga ata agt 1307
Leu Gln Glu Gln Tyr Glu Lys Thr Leu Gly Ser Arg Lys Arg Ile Ser
                245                 250                 255
aga gta ttg ggt ggt agt aat gga ggc agt ggg aac cat tcc aga cat 1355
Arg Val Leu Gly Gly Ser Asn Gly Gly Ser Gly Asn His Ser Arg His
            260                 265                 270
cat tct aga aac tac tcc aac gtt tcg aac aac aga agg ggc cat ttg 1403
His Ser Arg Asn Tyr Ser Asn Val Ser Asn Asn Arg Arg Gly His Leu
        275                 280                 285
aag aac aca tca tac gat tcc acg gca ttg ttg gat caa ccg ttg aca 1451
Lys Asn Thr Ser Tyr Asp Ser Thr Ala Leu Leu Asp Gln Pro Leu Thr
    290                 295                 300
gaa gac acc tat gtg aaa aat cct tat ggg aac ttt ggc tat aaa gca 1499
Glu Asp Thr Tyr Val Lys Asn Pro Tyr Gly Asn Phe Gly Tyr Lys Ala
305                 310                 315                 320
aat gtt gaa agt ccg tat aat cag gat gag ttt gca ttt aca aga gaa 1547

Asn Val Glu Ser Pro Tyr Asn Gln Asp Glu Phe Ala Phe Thr Arg Glu
                325                 330                 335
aga aag aag aag aaa aag tgt gta ata ttg tagatacctc tttgattagg 1597
Arg Lys Lys Lys Lys Lys Cys Val Ile Leu
            340                 345
tccataataa tatattaaag ttctgcatat gacaagaaat cgttttgtag aaagcacatg 1657

aaatcatgtc acaattgcat ggctagttta caggtctctg gatttcgaat tggatgaaag 1717

tataattata aaaccaatta gtgtctggga atactacata actgtctact gagatttcct 1777

atagtgagat atctaactgg tcaaagtgta gtacttttga agtgatattg ggttacttgc 1837

tgtatatatc gtcaatgttc cgtttatcct tcttatccga gactagtata gcaattatta 1897

tcattaatcc ttaacaaatg aacaggctcc agcttgtcga aaaaacactg ggcacttcat 1957

cattttgtag tggtaaatct ttatattggt ttccaatatt atagatcctc taga 2011

<210> 310
<211> 346
<212> PRT
<213> Candida albicans

<400> 310
Met Thr Pro Asn Gly Ser Arg Arg His Ser Ala Tyr Met Gly Ser Pro
1               5                   10                  15
Arg Ser Gln His Ser Ser Thr Met Glu Thr Gly Tyr Asn Pro Tyr Glu
            20                  25                  30
Ala Val Gln Lys Lys Gln Glu Leu Tyr Gln Asn Asn Asn Gly Asn Ser
        35                  40                  45
Pro Thr Val Ile Ile Glu Glu Asp Pro Tyr Ile Pro Asn Tyr Lys Glu
    50                  55                  60
Leu Ser Leu Ala Asn Lys Lys Thr Asn Tyr Asn Met Lys Ile Val Val
65                  70                  75                  80
Val Gly Asp Gly Gly Cys Gly Lys Thr Cys Leu Leu Leu Ala Tyr Thr
                85                  90                  95
Gln Asn Lys Phe Pro Ser Ile Tyr Val Pro Thr Val Phe Glu Asn Tyr
            100                 105                 110
Val Thr Ala Val Gln Ser Pro Asn Gly Lys Thr Val Glu Leu Ala Leu
        115                 120                 125
Trp Asp Thr Ala Gly Gln Glu Glu Tyr Asp Arg Leu Arg Pro Leu Ser
    130                 135                 140
Tyr Pro Asp Val Asp Ile Leu Leu Val Cys Phe Ala Val Asp Asn Glu
145                 150                 155                 160
Val Ser Leu Glu Asn Val Lys Asp Met Trp Phe Pro Glu Val Asn His
                165                 170                 175
Tyr Cys Pro Gly Ile Pro Ile Ile Leu Val Gly Thr Lys Ser Asp Leu
            180                 185                 190
Leu Ser Asp Met Asn His Asp Ala Ser Ile Arg Val Ala Lys Glu Ile
        195                 200                 205
Gly Ala Ile Gly Leu Ile Phe Thr Ser Ala Lys Thr Met Phe Asn Val
    210                 215                 220
Arg Thr Val Phe Asn Phe Ala Leu Asn His Phe Gln Arg Asn Met Glu
225                 230                 235                 240
Leu Gln Glu Gln Tyr Glu Lys Thr Leu Gly Ser Arg Lys Arg Ile Ser
                245                 250                 255
Arg Val Leu Gly Gly Ser Asn Gly Gly Ser Gly Asn His Ser Arg His
            260                 265                 270

His Ser Arg Asn Tyr Ser Asn Val Ser Asn Asn Arg Arg Gly His Leu
        275                 280                 285
Lys Asn Thr Ser Tyr Asp Ser Thr Ala Leu Leu Asp Gln Pro Leu Thr
    290                 295                 300
Glu Asp Thr Tyr Val Lys Asn Pro Tyr Gly Asn Phe Gly Tyr Lys Ala
305                 310                 315                 320
Asn Val Glu Ser Pro Tyr Asn Gln Asp Glu Phe Ala Phe Thr Arg Glu
                325                 330                 335
Arg Lys Lys Lys Lys Lys Cys Val Ile Leu
            340                 345

<210> 311
<211> 710
<212> DNA
<213> Beta vulgaris

<220>
<221> CDS
<222> (91)..(684)

<400> 311
ttgattgatc aagaggagaa gagagagtga gaggttgttt gaatagaaga tcgttagata      60

tttttttttt taattttttt tgttaaaaaa atg agt gct tcg agg ttc ata aaa      114
                                 Met Ser Ala Ser Arg Phe Ile Lys
                                 1               5
tgt gtt aca gtt ggt gat ggt gcc gtt ggt aaa act tgc ttg ttg att      162
Cys Val Thr Val Gly Asp Gly Ala Val Gly Lys Thr Cys Leu Leu Ile
    10                  15                  20
tct tac acc agc aac acc ttt cct acg gac tac gtg ccc act gtt ttt      210
Ser Tyr Thr Ser Asn Thr Phe Pro Thr Asp Tyr Val Pro Thr Val Phe
25                  30                  35                  40
gac aat ttc agt gcc aat gtc gtc gtt aac ggg gcc aca gtt aat ctg      258
Asp Asn Phe Ser Ala Asn Val Val Val Asn Gly Ala Thr Val Asn Leu
                45                  50                  55
gga tta tgg gat act gca gga caa gag gat tat aac aga tta aga cct      306
Gly Leu Trp Asp Thr Ala Gly Gln Glu Asp Tyr Asn Arg Leu Arg Pro
            60                  65                  70
ttg agt tat cgt gga gca gat gtt ttt att ctc gct ttc tcc ctt att      354
Leu Ser Tyr Arg Gly Ala Asp Val Phe Ile Leu Ala Phe Ser Leu Ile
        75                  80                  85
agc aag gct agt tat gaa aat gtt tct aag aag tgg att cct gag ttg      402
Ser Lys Ala Ser Tyr Glu Asn Val Ser Lys Lys Trp Ile Pro Glu Leu
    90                  95                  100
aag cat tat gct cct ggt gtc ccc att gtt ctt gtt gga aca aag ctc      450
Lys His Tyr Ala Pro Gly Val Pro Ile Val Leu Val Gly Thr Lys Leu
105                 110                 115                 120
gat ctt cgg gat gac aag cag ttt ttt atc gac cac cct ggt gca gtt      498
Asp Leu Arg Asp Asp Lys Gln Phe Phe Ile Asp His Pro Gly Ala Val
                125                 130                 135
cca atc act aca gct cag gga gag gaa tta agg aaa ctg att ggg gct      546
Pro Ile Thr Thr Ala Gln Gly Glu Glu Leu Arg Lys Leu Ile Gly Ala
            140                 145                 150
cct gct tac atc gaa tgc agt tca aaa aca cag cag aat gtc aag gca      594
Pro Ala Tyr Ile Glu Cys Ser Ser Lys Thr Gln Gln Asn Val Lys Ala
        155                 160                 165
gtt ttt gat gca gcc att aag gtc gtg ctt caa cca cca aag aca aag      642
Val Phe Asp Ala Ala Ile Lys Val Val Leu Gln Pro Pro Lys Thr Lys
    170                 175                 180
aaa aag aag tca aag gca cag aag gct tgc tcc ata ttg taattgtgta      691
Lys Lys Lys Ser Lys Ala Gln Lys Ala Cys Ser Ile Leu
185                 190                 195
agttgtaaag agaaacaag                                                  710

<210> 312
<211> 197
<212> PRT
<213> Beta vulgaris

<400> 312
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asn Gly Ala Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Ile Leu Ala Phe Ser Leu Ile Ser Lys Ala Ser Tyr Glu Asn Val
                85                  90                  95
Ser Lys Lys Trp Ile Pro Glu Leu Lys His Tyr Ala Pro Gly Val Pro
            100                 105                 110
Ile Val Leu Val Gly Thr Lys Leu Asp Leu Arg Asp Asp Lys Gln Phe
        115                 120                 125
Phe Ile Asp His Pro Gly Ala Val Pro Ile Thr Thr Ala Gln Gly Glu
    130                 135                 140
Glu Leu Arg Lys Leu Ile Gly Ala Pro Ala Tyr Ile Glu Cys Ser Ser
145                 150                 155                 160
Lys Thr Gln Gln Asn Val Lys Ala Val Phe Asp Ala Ala Ile Lys Val
                165                 170                 175
Val Leu Gln Pro Pro Lys Thr Lys Lys Lys Lys Ser Lys Ala Gln Lys
            180                 185                 190
Ala Cys Ser Ile Leu
        195

<210> 313
<211> 581
<212> DNA
<213> Cavia porcellus

<400> 313
atg cag gcc atc aag tgt gtg gtg gtg gga gat gga gct gtg ggc aag      48
Met Gln Ala Ile Lys Cys Val Val Val Gly Asp Gly Ala Val Gly Lys
1               5                   10                  15
acc tgc ctc ctt atc agc tac acc acc aac gcc ttc ccc gga gag tac      96
Thr Cys Leu Leu Ile Ser Tyr Thr Thr Asn Ala Phe Pro Gly Glu Tyr
            20                  25                  30
atc ccc acc gtg ttt gac aac tat tca gcc aat gtg atg gtg gac agc      144
Ile Pro Thr Val Phe Asp Asn Tyr Ser Ala Asn Val Met Val Asp Ser
        35                  40                  45
aag ccc gtg aac ctg ggg ctg tgg gac aca gct ggc cag gag gac tat      192
Lys Pro Val Asn Leu Gly Leu Trp Asp Thr Ala Gly Gln Glu Asp Tyr
    50                  55                  60
gac cgc ctg cga ccg ctc tcc tac cca cag acg gac gtc ttc ctc atc      240
Asp Arg Leu Arg Pro Leu Ser Tyr Pro Gln Thr Asp Val Phe Leu Ile
65                  70                  75                  80
tgc ttc tcc ctc gtc agt ccg gcc tcc tat gag aat gtc cat gcc aat      288
Cys Phe Ser Leu Val Ser Pro Ala Ser Tyr Glu Asn Val His Ala Asn
                85                  90                  95
tgg tac ccc aaa gtg cgg cac cac tgc ccc agc acc ccc atc atc ctg      336
Trp Tyr Pro Lys Val Arg His His Cys Pro Ser Thr Pro Ile Ile Leu
            100                 105                 110
ttg ggc acc aag ctg gac ctg cgg gat gac aag gag acc atc gag aag      384
Leu Gly Thr Lys Leu Asp Leu Arg Asp Asp Lys Glu Thr Ile Glu Lys
        115                 120                 125
ctg aag gag aag aag ctg gcg ccc atc acc tac cca cag ggc ctg gca      432
Leu Lys Glu Lys Lys Leu Ala Pro Ile Thr Tyr Pro Gln Gly Leu Ala
    130                 135                 140
ctg gcc aag gag att gac tct gtg aag tac ctg gag tgc tca gcc ctc      480
Leu Ala Lys Glu Ile Asp Ser Val Lys Tyr Leu Glu Cys Ser Ala Leu

145                 150                 155                 160
acc cag aga ggc ctg aag acc gtc ttt gac gag gcc atc cgt gcc gtc       528
Thr Gln Arg Gly Leu Lys Thr Val Phe Asp Glu Ala Ile Arg Ala Val
                165                 170                 175
ctg tgc cct cag ccc acg cgg cca cag aag cgc gcc tgc agc ctc ctc       576
Leu Cys Pro Gln Pro Thr Arg Pro Gln Lys Arg Ala Cys Ser Leu Leu
            180                 185                 190
ta  tag                                                               581

<210> 314
<211> 192
<212> PRT
<213> Cavia porcellus

<400> 314
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Ser
        35                  40                  45
Lys Pro Val Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Ser Pro Ala Ser Tyr Glu Asn Val His Ala Asn
                85                  90                  95
Trp Tyr Pro Lys Val Arg His His Cys Pro Ser Thr Pro Ile Ile Leu
            100                 105                 110
Leu Gly Thr Lys Leu Asp Leu Arg Asp Asp Lys Glu Thr Ile Glu Lys
        115                 120                 125
Leu Lys Glu Lys Lys Leu Ala Pro Ile Thr Tyr Pro Gln Gly Leu Ala
    130                 135                 140
Leu Ala Lys Glu Ile Asp Ser Val Lys Tyr Leu Glu Cys Ser Ala Leu
145                 150                 155                 160
Thr Gln Arg Gly Leu Lys Thr Val Phe Asp Glu Ala Ile Arg Ala Val
                165                 170                 175
Leu Cys Pro Gln Pro Thr Arg Pro Gln Lys Arg Ala Cys Ser Leu Leu
            180                 185                 190

<210> 315
<211> 982
<212> DNA
<213> Lotus japonicus

<220>
<221> CDS
<222> (113)..(703)

<400> 315
attttgtgtg tctcactctt tttcgcttgt tctattttta gtttcaagtg aaagaaatat      60

ggaaagcttt gctgtaaggt aaggaaaatc agttcagaaa aggaggttga aa atg agc     118
                                                          Met Ser
                                                          1
aca gct aga ttc atc aag tgt gtt act gtt gga gat gga gca gtg gga       166
Thr Ala Arg Phe Ile Lys Cys Val Thr Val Gly Asp Gly Ala Val Gly
        5                   10                  15
aag acc tgt atg ctt atc tct tac acc agc aac aca ttc cca acg gat       214
Lys Thr Cys Met Leu Ile Ser Tyr Thr Ser Asn Thr Phe Pro Thr Asp
    20                  25                  30
tat gtg cct act gtt ttt gat aac ttc agt gca aat gtg gtg gtt gat       262
Tyr Val Pro Thr Val Phe Asp Asn Phe Ser Ala Asn Val Val Val Asp
35                  40                  45                  50

ggc agc aca gtt aac ctg gga tta tgg gac act gct gga cag gag gat 310
Gly Ser Thr Val Asn Leu Gly Leu Trp Asp Thr Ala Gly Gln Glu Asp
                55                  60                  65
tac aat agg ctt agg cct ttg agc tac aga gga gca gat gtg ttc ttg 358
Tyr Asn Arg Leu Arg Pro Leu Ser Tyr Arg Gly Ala Asp Val Phe Leu
            70                  75                  80
ctg gct ttt tcc ctc ctt agc aga gcc agc tat gaa aat atc tcc aaa 406
Leu Ala Phe Ser Leu Leu Ser Arg Ala Ser Tyr Glu Asn Ile Ser Lys
        85                  90                  95
aag tgg att cct gaa ctg aga cac tat gcc cca act gtg cca att gtt 454
Lys Trp Ile Pro Glu Leu Arg His Tyr Ala Pro Thr Val Pro Ile Val
    100                 105                 110
ctt gtg gga acc aaa ctt gat ttg agg gaa gac agg cag tat ttg att 502
Leu Val Gly Thr Lys Leu Asp Leu Arg Glu Asp Arg Gln Tyr Leu Ile
115                 120                 125                 130
gat cat cct gga gcc aca cct att act act gcc cag gga gaa gag ctg 550
Asp His Pro Gly Ala Thr Pro Ile Thr Thr Ala Gln Gly Glu Glu Leu
                135                 140                 145
aag aag gca att ggt gct gct gtg tac cta gaa tgc agc tca aag act 598
Lys Lys Ala Ile Gly Ala Ala Val Tyr Leu Glu Cys Ser Ser Lys Thr
            150                 155                 160
caa cag aat gtg aag gct gtg ttt gat gct gct atc aag gtt gtt ttg 646
Gln Gln Asn Val Lys Ala Val Phe Asp Ala Ala Ile Lys Val Val Leu
        165                 170                 175
cag cca cct aaa cca aag aaa aaa cga aag aag acc aga cca tgc gtt 694
Gln Pro Pro Lys Pro Lys Lys Lys Arg Lys Lys Thr Arg Pro Cys Val
    180                 185                 190
ttc ctt taattgatgt tcatgtttga ttcgcaatct gtagcattcg ggaccttctt 750
Phe Leu
195
cctatgtttt ttacccttta tttgattgca caaatatgct ttttgaacat atgtattaca 810

cactcttatc ttctttccgt tttcttgtat tttttgttgg tgggtctaag tgatatttca 870

atgtatctac tggaacaaac aaggagactg tactcgatca aagataaatt ttgtgataag 930

aaataatagc attcatagtg actttttta ttaaaaaaa aaaaaaaaa aa 982

<210> 316
<211> 196
<212> PRT
<213> Lotus japonicus

<400> 316
Met Ser Thr Ala Arg Phe Ile Lys Cys Val Thr Val Gly Asp Gly Ala
1               5                   10                  15
Val Gly Lys Thr Cys Met Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45
Val Asp Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Leu Ser Arg Ala Ser Tyr Glu Asn Ile
                85                  90                  95
Ser Lys Lys Trp Ile Pro Glu Leu Arg His Tyr Ala Pro Thr Val Pro
            100                 105                 110
Ile Val Leu Val Gly Thr Lys Leu Asp Leu Arg Glu Asp Arg Gln Tyr
        115                 120                 125
Leu Ile Asp His Pro Gly Ala Thr Pro Ile Thr Thr Ala Gln Gly Glu
    130                 135                 140

Glu Leu Lys Lys Ala Ile Gly Ala Ala Val Tyr Leu Glu Cys Ser Ser
145                 150                 155                 160
Lys Thr Gln Gln Asn Val Lys Ala Val Phe Asp Ala Ala Ile Lys Val
                165                 170                 175
Val Leu Gln Pro Pro Lys Pro Lys Lys Lys Arg Lys Lys Thr Arg Pro
            180                 185                 190
Cys Val Phe Leu
        195

<210> 317
<211> 659
<212> DNA
<213> Dictyostelium discoideum

<220>
<221> CDS
<222> (22)..(609)

<400> 317
ggttaaaaag tttacataat c atg caa tca att aaa ttg gtg gta gta ggt       51
                        Met Gln Ser Ile Lys Leu Val Val Val Gly
                        1               5                   10
gat ggt gct gtt ggt aaa act tgt tta tta att tct tat aca tca aat       99
Asp Gly Ala Val Gly Lys Thr Cys Leu Leu Ile Ser Tyr Thr Ser Asn
                15                  20                  25
tct ttc cca aca gaa tac gtt cca act gta ttt gat aac tat tca gca      147
Ser Phe Pro Thr Glu Tyr Val Pro Thr Val Phe Asp Asn Tyr Ser Ala
            30                  35                  40
aat gtt atg gta gat aat aaa act gtt tca tta ggt ctt tgg gat act      195
Asn Val Met Val Asp Asn Lys Thr Val Ser Leu Gly Leu Trp Asp Thr
        45                  50                  55
gct ggt caa gag gat tat gat cgt tta aga cca ctt tca tac cca caa      243
Ala Gly Gln Glu Asp Tyr Asp Arg Leu Arg Pro Leu Ser Tyr Pro Gln
    60                  65                  70
acc gat gtt ttt ctt att tgt ttc gct att ata agt caa act tca tat      291
Thr Asp Val Phe Leu Ile Cys Phe Ala Ile Ile Ser Gln Thr Ser Tyr
75                  80                  85                  90
aca aat gta aaa tct aaa tgg tgg cct gaa gtc aca cat cat tgt cca      339
Thr Asn Val Lys Ser Lys Trp Trp Pro Glu Val Thr His His Cys Pro
                95                  100                 105
aac tgc aca att att tta gtt ggt aca aaa tgt gat tta aga gaa gac      387
Asn Cys Thr Ile Ile Leu Val Gly Thr Lys Cys Asp Leu Arg Glu Asp
            110                 115                 120
aaa gaa agt tta gaa aaa ctc aga gaa aaa cat caa caa cca ctc acc      435
Lys Glu Ser Leu Glu Lys Leu Arg Glu Lys His Gln Gln Pro Leu Thr
        125                 130                 135
ttc caa caa ggt gaa caa atg gca aaa gaa att aaa gcc ttt tgt tat      483
Phe Gln Gln Gly Glu Gln Met Ala Lys Glu Ile Lys Ala Phe Cys Tyr
    140                 145                 150
atg gaa tgt tcc gct tta act caa aaa ggt ctc aaa caa gtt ttc gac      531
Met Glu Cys Ser Ala Leu Thr Gln Lys Gly Leu Lys Gln Val Phe Asp
155                 160                 165                 170
gaa gct att aaa gct gtt att ttc cca gat aga gat aag gcc aca aac      579
Glu Ala Ile Lys Ala Val Ile Phe Pro Asp Arg Asp Lys Ala Thr Asn
                175                 180                 185
aaa aag aat tca aaa tgt tca att tta taaaaacata tcaaaatatc            626
Lys Lys Asn Ser Lys Cys Ser Ile Leu
            190                 195
tctattcttt ataataatca caaccaaaaa aaa                                 659

<210> 318
<211> 195
<212> PRT
<213> Dictyostelium discoideum

<400> 318
Met Gln Ser Ile Lys Leu Val Val Val Gly Asp Gly Ala Val Gly Lys
1               5                   10                  15
Thr Cys Leu Leu Ile Ser Tyr Thr Ser Asn Ser Phe Pro Thr Glu Tyr
            20                  25                  30
Val Pro Thr Val Phe Asp Asn Tyr Ser Ala Asn Val Met Val Asp Asn
        35                  40                  45
Lys Thr Val Ser Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ala Ile Ile Ser Gln Thr Ser Tyr Thr Asn Val Lys Ser Lys
                85                  90                  95
Trp Trp Pro Glu Val Thr His His Cys Pro Asn Cys Thr Ile Ile Leu
            100                 105                 110
Val Gly Thr Lys Cys Asp Leu Arg Glu Asp Lys Glu Ser Leu Glu Lys
        115                 120                 125
Leu Arg Glu Lys His Gln Gln Pro Leu Thr Phe Gln Gln Gly Glu Gln
    130                 135                 140
Met Ala Lys Glu Ile Lys Ala Phe Cys Tyr Met Glu Cys Ser Ala Leu
145                 150                 155                 160
Thr Gln Lys Gly Leu Lys Gln Val Phe Asp Glu Ala Ile Lys Ala Val
                165                 170                 175
Ile Phe Pro Asp Arg Asp Lys Ala Thr Asn Lys Lys Asn Ser Lys Cys
            180                 185                 190
Ser Ile Leu
        195

<210> 319
<211> 634
<212> DNA
<213> Dictyostelium discoideum

<220>
<221> CDS
<222> (46)..(624)

<400> 319
gatcatattt aaattataca aaaaacaatt acatatatat aaaga atg tca gca gca      57
                                                  Met Ser Ala Ala
                                                  1
gaa gtt att aaa tta gtc gtt att ggt gat ggt gct gta ggt aaa act      105
Glu Val Ile Lys Leu Val Val Ile Gly Asp Gly Ala Val Gly Lys Thr
5                   10                  15                  20
tgt tta ttg att agt tat gca aac aat cgt ttc cca gaa gat tat att      153
Cys Leu Leu Ile Ser Tyr Ala Asn Asn Arg Phe Pro Glu Asp Tyr Ile
                25                  30                  35
cca act gta ttc gat aat tat gtt gta aat ctt aca gca ggt gat aga      201
Pro Thr Val Phe Asp Asn Tyr Val Val Asn Leu Thr Ala Gly Asp Arg
            40                  45                  50
aac ata gaa ctc gga ctt tgg gat act gca ggt caa gaa gag tac gat      249
Asn Ile Glu Leu Gly Leu Trp Asp Thr Ala Gly Gln Glu Glu Tyr Asp
        55                  60                  65
aaa tta aga cca tta agt tat gca aat gca aat gta ttt tta att tgc      297
Lys Leu Arg Pro Leu Ser Tyr Ala Asn Ala Asn Val Phe Leu Ile Cys
    70                  75                  80
ttc tca att acc aat cca gtt tca ttt gaa aat gtt tac aca aaa tgg      345
Phe Ser Ile Thr Asn Pro Val Ser Phe Glu Asn Val Tyr Thr Lys Trp
85                  90                  95                  100
tac cca gaa gtt atg cat ttt tgc cca gaa gtt cca caa att tta gtt      393
Tyr Pro Glu Val Met His Phe Cys Pro Glu Val Pro Gln Ile Leu Val
                105                 110                 115
ggt act aaa tta gat aca cgt gac gat aga ggt gtt tta gat aaa ctt      441
Gly Thr Lys Leu Asp Thr Arg Asp Asp Arg Gly Val Leu Asp Lys Leu
            120                 125                 130
caa caa act ggt cat aaa cca att aca acc gaa caa ggt aac gat tta      489
Gln Gln Thr Gly His Lys Pro Ile Thr Thr Glu Gln Gly Asn Asp Leu
        135                 140                 145

gcc aga aga att aaa gcc att aaa tat atg gaa tgt tct gcc aaa acc 537
Ala Arg Arg Ile Lys Ala Ile Lys Tyr Met Glu Cys Ser Ala Lys Thr
    150                 155                 160
tca caa aat ctc aaa caa gtc ttt gat gaa gcc att aaa tct gtt ttg 585
Ser Gln Asn Leu Lys Gln Val Phe Asp Glu Ala Ile Lys Ser Val Leu
165                 170                 175                 180
ttt atc aaa aaa aag aaa tcc aag tgt att gtt atg taacccgctc 631
Phe Ile Lys Lys Lys Lys Ser Lys Cys Ile Val Met
                185                 190
tcc 634

<210> 320
<211> 192
<212> PRT
<213> Dictyostelium discoideum

<400> 320
Met Ser Ala Ala Glu Val Ile Lys Leu Val Val Ile Gly Asp Gly Ala
1               5                   10                  15
Val Gly Lys Thr Cys Leu Leu Ile Ser Tyr Ala Asn Asn Arg Phe Pro
            20                  25                  30
Glu Asp Tyr Ile Pro Thr Val Phe Asp Asn Tyr Val Val Asn Leu Thr
        35                  40                  45
Ala Gly Asp Arg Asn Ile Glu Leu Gly Leu Trp Asp Thr Ala Gly Gln
    50                  55                  60
Glu Glu Tyr Asp Lys Leu Arg Pro Leu Ser Tyr Ala Asn Ala Asn Val
65                  70                  75                  80
Phe Leu Ile Cys Phe Ser Ile Thr Asn Pro Val Ser Phe Glu Asn Val
                85                  90                  95
Tyr Thr Lys Trp Tyr Pro Glu Val Met His Phe Cys Pro Glu Val Pro
            100                 105                 110
Gln Ile Leu Val Gly Thr Lys Leu Asp Thr Arg Asp Asp Arg Gly Val
        115                 120                 125
Leu Asp Lys Leu Gln Gln Thr Gly His Lys Pro Ile Thr Thr Glu Gln
    130                 135                 140
Gly Asn Asp Leu Ala Arg Arg Ile Lys Ala Ile Lys Tyr Met Glu Cys
145                 150                 155                 160
Ser Ala Lys Thr Ser Gln Asn Leu Lys Gln Val Phe Asp Glu Ala Ile
                165                 170                 175
Lys Ser Val Leu Phe Ile Lys Lys Lys Lys Ser Lys Cys Ile Val Met
            180                 185                 190

<210> 321
<211> 765
<212> DNA
<213> Dictyostelium discoideum

<220>
<221> CDS
<222> (1)..(765)

<400> 321
atg gct aca ggt att aag aaa act gtt aaa gta gta gtt gta ggt gat 48
Met Ala Thr Gly Ile Lys Lys Thr Val Lys Val Val Val Val Gly Asp
1               5                   10                  15
ggt gct gtt ggt aaa act tca ctt tta att tta tat aca act aaa gca 96
Gly Ala Val Gly Lys Thr Ser Leu Leu Ile Leu Tyr Thr Thr Lys Ala
            20                  25                  30
ttc cca aaa gat tat gta cca aca gta ttt gat aac ttt aat tgt tta 144
Phe Pro Lys Asp Tyr Val Pro Thr Val Phe Asp Asn Phe Asn Cys Leu
        35                  40                  45
gag atg tac gat aat aaa cca gtt aat tta gta ttg tgg gat acc gct 192
Glu Met Tyr Asp Asn Lys Pro Val Asn Leu Val Leu Trp Asp Thr Ala
    50                  55                  60
ggt caa gaa gat tat gat aat tta cgt cca ttg tca tat cca cag act 240

Gly Gln Glu Asp Tyr Asp Asn Leu Arg Pro Leu Ser Tyr Pro Gln Thr
65                  70                  75                  80
gat gta ttt ata ata tgt tat tca gta gtt aaa aga gat tcg tta gat        288
Asp Val Phe Ile Ile Cys Tyr Ser Val Val Lys Arg Asp Ser Leu Asp
                85                  90                  95
aat ata aag tat aaa tgg tta cct gaa att aat caa act aat cag ggt        336
Asn Ile Lys Tyr Lys Trp Leu Pro Glu Ile Asn Gln Thr Asn Gln Gly
            100                 105                 110
aca cca att att tta gtt ggt aca aag act gat tta agg gag gac aaa        384
Thr Pro Ile Ile Leu Val Gly Thr Lys Thr Asp Leu Arg Glu Asp Lys
        115                 120                 125
aaa aca tta tca caa tta caa gaa tca aaa caa gaa cca gtt tca aga        432
Lys Thr Leu Ser Gln Leu Gln Glu Ser Lys Gln Glu Pro Val Ser Arg
    130                 135                 140
gat gaa ggt gta gca tta gca aaa gag ata ggt gca gta caa ttt ttc        480
Asp Glu Gly Val Ala Leu Ala Lys Glu Ile Gly Ala Val Gln Phe Phe
145                 150                 155                 160
gaa tgt tct gca ttg aca ggt aat ggt gta aat gat att ttc gct gct        528
Glu Cys Ser Ala Leu Thr Gly Asn Gly Val Asn Asp Ile Phe Ala Ala
                165                 170                 175
gca att aaa gca gct ttt aat aaa cct gct gta act tca cca act tcg        576
Ala Ile Lys Ala Ala Phe Asn Lys Pro Ala Val Thr Ser Pro Thr Ser
            180                 185                 190
aaa tct agt ggt aaa tct tca cct tca tct acc tct tca aaa cct tct        624
Lys Ser Ser Gly Lys Ser Ser Pro Ser Ser Thr Ser Ser Lys Pro Ser
        195                 200                 205
aaa act act act aca act aca acc tct tct tct tct tct tca cca cct        672
Lys Thr Thr Thr Thr Thr Thr Thr Ser Ser Ser Ser Ser Ser Pro Pro
    210                 215                 220
gct gct tca act gca aaa cca gca ggt gaa aag aaa tta agt tgg ggt        720
Ala Ala Ser Thr Ala Lys Pro Ala Gly Glu Lys Lys Leu Ser Trp Gly
225                 230                 235                 240
ttg ttc cgt aaa aaa gat aaa gat gaa aag aaa cca gca aaa taa            765
Leu Phe Arg Lys Lys Asp Lys Asp Glu Lys Lys Pro Ala Lys
                245                 250


<210> 322
<211> 254
<212> PRT
<213> Dictyostelium discoideum

<400> 322
Met Ala Thr Gly Ile Lys Lys Thr Val Lys Val Val Val Val Gly Asp
1               5                   10                  15
Gly Ala Val Gly Lys Thr Ser Leu Leu Ile Leu Tyr Thr Thr Lys Ala
            20                  25                  30
Phe Pro Lys Asp Tyr Val Pro Thr Val Phe Asp Asn Phe Asn Cys Leu
        35                  40                  45
Glu Met Tyr Asp Asn Lys Pro Val Asn Leu Val Leu Trp Asp Thr Ala
    50                  55                  60
Gly Gln Glu Asp Tyr Asp Asn Leu Arg Pro Leu Ser Tyr Pro Gln Thr
65                  70                  75                  80
Asp Val Phe Ile Ile Cys Tyr Ser Val Val Lys Arg Asp Ser Leu Asp
                85                  90                  95
Asn Ile Lys Tyr Lys Trp Leu Pro Glu Ile Asn Gln Thr Asn Gln Gly
            100                 105                 110
Thr Pro Ile Ile Leu Val Gly Thr Lys Thr Asp Leu Arg Glu Asp Lys
        115                 120                 125
Lys Thr Leu Ser Gln Leu Gln Glu Ser Lys Gln Glu Pro Val Ser Arg
    130                 135                 140
Asp Glu Gly Val Ala Leu Ala Lys Glu Ile Gly Ala Val Gln Phe Phe
145                 150                 155                 160
Glu Cys Ser Ala Leu Thr Gly Asn Gly Val Asn Asp Ile Phe Ala Ala
                165                 170                 175
Ala Ile Lys Ala Ala Phe Asn Lys Pro Ala Val Thr Ser Pro Thr Ser
            180                 185                 190
Lys Ser Ser Gly Lys Ser Ser Pro Ser Ser Thr Ser Ser Lys Pro Ser

195                 200                 205
Lys Thr Thr Thr Thr Thr Thr Thr Ser Ser Ser Ser Ser Ser Pro Pro
    210                 215                 220
Ala Ala Ser Thr Ala Lys Pro Ala Gly Glu Lys Lys Leu Ser Trp Gly
225                 230                 235                 240
Leu Phe Arg Lys Lys Asp Lys Asp Glu Lys Lys Pro Ala Lys
                245                 250

<210> 323
<211> 672
<212> DNA
<213> Dictyostelium discoideum

<220>
<221> CDS
<222> (1)..(672)

<400> 323
atg tca gaa gat caa ggt tca gga gca aca aga gtt aaa tta gta gtt      48
Met Ser Glu Asp Gln Gly Ser Gly Ala Thr Arg Val Lys Leu Val Val
1               5                   10                  15
gtc ggt gat ggt gct gtt ggt aaa aca tgt ctt tta att tgt tat gca      96
Val Gly Asp Gly Ala Val Gly Lys Thr Cys Leu Leu Ile Cys Tyr Ala
            20                  25                  30
caa aat gat ttt cca gta gat tat gta cca act gtt ttt gaa aat tat      144
Gln Asn Asp Phe Pro Val Asp Tyr Val Pro Thr Val Phe Glu Asn Tyr
        35                  40                  45
aca gca acc aga aag aga gga aat gaa gat att aaa gta cat tta tgg      192
Thr Ala Thr Arg Lys Arg Gly Asn Glu Asp Ile Lys Val His Leu Trp
    50                  55                  60
gat act gca ggc caa gaa gaa tat gat cgt tta cgt cca tta tca tac      240
Asp Thr Ala Gly Gln Glu Glu Tyr Asp Arg Leu Arg Pro Leu Ser Tyr
65                  70                  75                  80
cca ggc gct gat gtt gtt ctc ctt tgt ttc agc aca atc agt caa tca      288
Pro Gly Ala Asp Val Val Leu Leu Cys Phe Ser Thr Ile Ser Gln Ser
                85                  90                  95
tca tat gaa gcc att aga gat aaa tgg gca cca gaa gtt aat cac tat      336
Ser Tyr Glu Ala Ile Arg Asp Lys Trp Ala Pro Glu Val Asn His Tyr
            100                 105                 110
atc cca gat gta cca tca att tta gtt ggt act aaa atc gat tta cgt      384
Ile Pro Asp Val Pro Ser Ile Leu Val Gly Thr Lys Ile Asp Leu Arg
        115                 120                 125
gaa caa caa cac cca gat cca aac tct ggt aaa ttc gaa cca atc act      432
Glu Gln Gln His Pro Asp Pro Asn Ser Gly Lys Phe Glu Pro Ile Thr
    130                 135                 140
gcc gat atg ggt att tca atg caa aaa caa att aaa gcc aag aaa tat      480
Ala Asp Met Gly Ile Ser Met Gln Lys Gln Ile Lys Ala Lys Lys Tyr
145                 150                 155                 160
tta gaa gtc tct gca aag act cgt caa ggt tta gaa gaa gtt ttc agt      528
Leu Glu Val Ser Ala Lys Thr Arg Gln Gly Leu Glu Glu Val Phe Ser
                165                 170                 175
gct gcc att gaa atc gtt ctt gaa tca aga ggt atg gat aaa aag agt      576
Ala Ala Ile Glu Ile Val Leu Glu Ser Arg Gly Met Asp Lys Lys Ser
            180                 185                 190
caa gat ggt tct tca agt gca tct ggt gtt cca tca ggt gat aaa cca      624
Gln Asp Gly Ser Ser Ser Ala Ser Gly Val Pro Ser Gly Asp Lys Pro
        195                 200                 205
aca aaa gga aaa gca ggt aaa aag aaa tct ggt tgt att ata ctt          669
Thr Lys Gly Lys Ala Gly Lys Lys Lys Ser Gly Cys Ile Ile Leu
    210                 215                 220
taa                                                                  672

<210> 324
<211> 223
<212> PRT

<213> Dictyostelium discoideum

<400> 324
Met Ser Glu Asp Gln Gly Ser Gly Ala Thr Arg Val Lys Leu Val Val
1               5                   10                  15
Val Gly Asp Gly Ala Val Gly Lys Thr Cys Leu Leu Ile Cys Tyr Ala
            20                  25                  30
Gln Asn Asp Phe Pro Val Asp Tyr Val Pro Thr Val Phe Glu Asn Tyr
        35                  40                  45
Thr Ala Thr Arg Lys Arg Gly Asn Glu Asp Ile Lys Val His Leu Trp
    50                  55                  60
Asp Thr Ala Gly Gln Glu Glu Tyr Asp Arg Leu Arg Pro Leu Ser Tyr
65                  70                  75                  80
Pro Gly Ala Asp Val Val Leu Leu Cys Phe Ser Thr Ile Ser Gln Ser
                85                  90                  95
Ser Tyr Glu Ala Ile Arg Asp Lys Trp Ala Pro Glu Val Asn His Tyr
            100                 105                 110
Ile Pro Asp Val Pro Ser Ile Leu Val Gly Thr Lys Ile Asp Leu Arg
        115                 120                 125
Glu Gln Gln His Pro Asp Pro Asn Ser Gly Lys Phe Glu Pro Ile Thr
    130                 135                 140
Ala Asp Met Gly Ile Ser Met Gln Lys Gln Ile Lys Ala Lys Lys Tyr
145                 150                 155                 160
Leu Glu Val Ser Ala Lys Thr Arg Gln Gly Leu Glu Glu Val Phe Ser
                165                 170                 175
Ala Ala Ile Glu Ile Val Leu Glu Ser Arg Gly Met Asp Lys Lys Ser
            180                 185                 190
Gln Asp Gly Ser Ser Ser Ala Ser Gly Val Pro Ser Gly Asp Lys Pro
        195                 200                 205
Thr Lys Gly Lys Ala Gly Lys Lys Lys Ser Gly Cys Ile Ile Leu
    210                 215                 220

<210> 325
<211> 579
<212> DNA
<213> Homo sapiens

<220>
<221> CDS
<222> (1)..(579)

<400> 325
atg cag gcc atc aag ggt gtg gtg gtg gga gac gga gct gta ggt aaa      48
Met Gln Ala Ile Lys Gly Val Val Val Gly Asp Gly Ala Val Gly Lys
1               5                   10                  15
act tgc cta ctg atc agt tac aca acc aac gca ttt cct gga gaa gat      96
Thr Cys Leu Leu Ile Ser Tyr Thr Thr Asn Ala Phe Pro Gly Glu Asp
            20                  25                  30
atc cct act gcc ttt gac aat tat tct gcc aat gtt atg gta gat gga      144
Ile Pro Thr Ala Phe Asp Asn Tyr Ser Ala Asn Val Met Val Asp Gly
        35                  40                  45
aaa ctg gtg aat ctg ggc tta tgg aat aca gct gga caa gaa gat tat      192
Lys Leu Val Asn Leu Gly Leu Trp Asn Thr Ala Gly Gln Glu Asp Tyr
    50                  55                  60
gac aga tta cgc ccc cta tcc tat ccg caa gca gat gtg ttc tta att      240
Asp Arg Leu Arg Pro Leu Ser Tyr Pro Gln Ala Asp Val Phe Leu Ile
65                  70                  75                  80
tgc ttt tcg ctt gtg agt cct gca tca ttt gaa aat gtc ctt gca aag      288
Cys Phe Ser Leu Val Ser Pro Ala Ser Phe Glu Asn Val Leu Ala Lys
                85                  90                  95
tgg tat cct gag gtg cag cac cac tgt ccc aac act ccc att atc cta      336
Trp Tyr Pro Glu Val Gln His His Cys Pro Asn Thr Pro Ile Ile Leu
            100                 105                 110
gtg gga act aaa ctt gat ctt agg gat gat aaa gac agg atc cag aaa      384
Val Gly Thr Lys Leu Asp Leu Arg Asp Asp Lys Asp Arg Ile Gln Lys
        115                 120                 125
ctg aag gag aag aag cta act ccc atc acc tat ccg cag ggt cta gcc      432
Leu Lys Glu Lys Lys Leu Thr Pro Ile Thr Tyr Pro Gln Gly Leu Ala

130                 135                 140
atg gct aag gag atg ggt gct gta aaa tac ctg gag tgc ttg gcc ctc       480
Met Ala Lys Glu Met Gly Ala Val Lys Tyr Leu Glu Cys Leu Ala Leu
145                 150                 155                 160
aca agg cga ggc ctc aag aca gtg ttt gac gaa gcg atc cga gct gtc       528
Thr Arg Arg Gly Leu Lys Thr Val Phe Asp Glu Ala Ile Arg Ala Val
                165                 170                 175
ctc tgc cca cct ccc gtg aag aag agg aag aga aaa tgc ctg cag ttg       576
Leu Cys Pro Pro Pro Val Lys Lys Arg Lys Arg Lys Cys Leu Gln Leu
            180                 185                 190
tag                                                                   579

<210> 326
<211> 192
<212> PRT
<213> Homo sapiens

<400> 326
Met Gln Ala Ile Lys Gly Val Val Val Gly Asp Gly Ala Val Gly Lys
1               5                   10                  15
Thr Cys Leu Leu Ile Ser Tyr Thr Thr Asn Ala Phe Pro Gly Glu Asp
            20                  25                  30
Ile Pro Thr Ala Phe Asp Asn Tyr Ser Ala Asn Val Met Val Asp Gly
        35                  40                  45
Lys Leu Val Asn Leu Gly Leu Trp Asn Thr Ala Gly Gln Glu Asp Tyr
    50                  55                  60
Asp Arg Leu Arg Pro Leu Ser Tyr Pro Gln Ala Asp Val Phe Leu Ile
65                  70                  75                  80
Cys Phe Ser Leu Val Ser Pro Ala Ser Phe Glu Asn Val Leu Ala Lys
                85                  90                  95
Trp Tyr Pro Glu Val Gln His His Cys Pro Asn Thr Pro Ile Ile Leu
            100                 105                 110
Val Gly Thr Lys Leu Asp Leu Arg Asp Asp Lys Asp Arg Ile Gln Lys
        115                 120                 125
Leu Lys Glu Lys Lys Leu Thr Pro Ile Thr Tyr Pro Gln Gly Leu Ala
    130                 135                 140
Met Ala Lys Glu Met Gly Ala Val Lys Tyr Leu Glu Cys Leu Ala Leu
145                 150                 155                 160
Thr Arg Arg Gly Leu Lys Thr Val Phe Asp Glu Ala Ile Arg Ala Val
                165                 170                 175
Leu Cys Pro Pro Pro Val Lys Lys Arg Lys Arg Lys Cys Leu Gln Leu
            180                 185                 190

<210> 327
<211> 594
<212> DNA
<213> Dictyostelium discoideum

<220>
<221> CDS
<222> (5)..(589)

<400> 327
cata atg caa gca att aaa tgt gta gtt gtt ggt gat ggt gca gtt ggt       49
     Met Gln Ala Ile Lys Cys Val Val Val Gly Asp Gly Ala Val Gly
     1               5                   10                  15
aaa aca tgt ctt tta att tca tat aca acc aat gct ttt cca gga gag       97
Lys Thr Cys Leu Leu Ile Ser Tyr Thr Thr Asn Ala Phe Pro Gly Glu
                20                  25                  30
tat atc cca aca gtt ttt gat aat tac agc gca aat gta atg gtt gat       145
Tyr Ile Pro Thr Val Phe Asp Asn Tyr Ser Ala Asn Val Met Val Asp
            35                  40                  45
ggt aaa cca att aat ctc gga tta tgg gat aca gca ggt caa gaa gat       193
Gly Lys Pro Ile Asn Leu Gly Leu Trp Asp Thr Ala Gly Gln Glu Asp
        50                  55                  60

tat gat cgt tta cgt cct tta tcc tat cct caa act gat gtt ttc tta 241
Tyr Asp Arg Leu Arg Pro Leu Ser Tyr Pro Gln Thr Asp Val Phe Leu
    65                  70                  75
att tgt ttt tca atc gtt tca cca gca tct ttt gag aat gta aat ggt 289
Ile Cys Phe Ser Ile Val Ser Pro Ala Ser Phe Glu Asn Val Asn Gly
80                  85                  90                  95
aaa tgg cat cca gaa ata tgt cac cat gca cca aat gtt cga atc att 337
Lys Trp His Pro Glu Ile Cys His His Ala Pro Asn Val Arg Ile Ile
                100                 105                 110
tta gtt ggt act aaa tta gat atg aga gaa gat aga gat act caa gat 385
Leu Val Gly Thr Lys Leu Asp Met Arg Glu Asp Arg Asp Thr Gln Asp
            115                 120                 125
aga tta aaa gag aaa aaa ctt tat cca gtt tcc tat gaa caa ggt ctt 433
Arg Leu Lys Glu Lys Lys Leu Tyr Pro Val Ser Tyr Glu Gln Gly Leu
        130                 135                 140
tca aaa atg aaa gaa att aat gct gtc aaa tat ctt gaa tgt tca gct 481
Ser Lys Met Lys Glu Ile Asn Ala Val Lys Tyr Leu Glu Cys Ser Ala
    145                 150                 155
ctc aca caa aaa ggt ctt aaa act gtt ttt gat gaa gca att aga tct 529
Leu Thr Gln Lys Gly Leu Lys Thr Val Phe Asp Glu Ala Ile Arg Ser
160                 165                 170                 175
gta att aat cca act ctt aag aaa aaa cca aaa tct tca aaa ggt tgt 577
Val Ile Asn Pro Thr Leu Lys Lys Lys Pro Lys Ser Ser Lys Gly Cys
                180                 185                 190
att ata atg taaaaaa 594
Ile Ile Met

<210> 328
<211> 194
<212> PRT
<213> Dictyostelium discoideum

<400> 328
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly
        35                  40                  45
Lys Pro Ile Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Ile Val Ser Pro Ala Ser Phe Glu Asn Val Asn Gly Lys
                85                  90                  95
Trp His Pro Glu Ile Cys His His Ala Pro Asn Val Arg Ile Ile Leu
            100                 105                 110
Val Gly Thr Lys Leu Asp Met Arg Glu Asp Arg Asp Thr Gln Asp Arg
        115                 120                 125
Leu Lys Glu Lys Lys Leu Tyr Pro Val Ser Tyr Glu Gln Gly Leu Ser
    130                 135                 140
Lys Met Lys Glu Ile Asn Ala Val Lys Tyr Leu Glu Cys Ser Ala Leu
145                 150                 155                 160
Thr Gln Lys Gly Leu Lys Thr Val Phe Asp Glu Ala Ile Arg Ser Val
                165                 170                 175
Ile Asn Pro Thr Leu Lys Lys Lys Pro Lys Ser Ser Lys Gly Cys Ile
            180                 185                 190
Ile Met

<210> 329
<211> 591
<212> DNA
<213> Dictyostelium discoideum

<220>

<221> CDS
<222> (8)..(589)

<400> 329
aaaaaaa atg caa gca att aaa tgt gta gtt gta ggt gat ggt gcg gtt 49
Met Gln Ala Ile Lys Cys Val Val Val Gly Asp Gly Ala Val
1               5                   10
ggt aaa aca tgt ctt tta att tct tat aca act aac gct ttt cct gga 97
Gly Lys Thr Cys Leu Leu Ile Ser Tyr Thr Thr Asn Ala Phe Pro Gly
15                  20                  25                  30
gag tat atc cca aca gtt ttc gat aat tac tca gct aat gtt atg gtt 145
Glu Tyr Ile Pro Thr Val Phe Asp Asn Tyr Ser Ala Asn Val Met Val
                35                  40                  45
gat ggt aaa cca att aat ctt ggc ttg tgg gat act gct ggt caa gaa 193
Asp Gly Lys Pro Ile Asn Leu Gly Leu Trp Asp Thr Ala Gly Gln Glu
            50                  55                  60
gat tat gat cgt tta cgt cca ctt tca tat cct caa act gat gtt ttc 241
Asp Tyr Asp Arg Leu Arg Pro Leu Ser Tyr Pro Gln Thr Asp Val Phe
        65                  70                  75
tta att tgc ttt tca att att tct cca tca tca tat gaa aat gtt tca 289
Leu Ile Cys Phe Ser Ile Ile Ser Pro Ser Ser Tyr Glu Asn Val Ser
    80                  85                  90
ggt aaa tgg gga cca gaa gta ttt cat cat gct cca aat gtt cca atc 337
Gly Lys Trp Gly Pro Glu Val Phe His His Ala Pro Asn Val Pro Ile
95                  100                 105                 110
att ttg gtt ggt aca aaa atg gat atg aga gaa gat aag gaa act caa 385
Ile Leu Val Gly Thr Lys Met Asp Met Arg Glu Asp Lys Glu Thr Gln
                115                 120                 125
gat aga tta aaa gaa aag aaa ctt tat cca gtt tcc tat gaa caa ggt 433
Asp Arg Leu Lys Glu Lys Lys Leu Tyr Pro Val Ser Tyr Glu Gln Gly
            130                 135                 140
ctt tta aaa atg aaa gaa att aat gct ttc aaa tat ctt gaa tgc tct 481
Leu Leu Lys Met Lys Glu Ile Asn Ala Phe Lys Tyr Leu Glu Cys Ser
        145                 150                 155
gct ctc act caa aaa ggt ctt aaa act gtt ttc gac gaa gct att aga 529
Ala Leu Thr Gln Lys Gly Leu Lys Thr Val Phe Asp Glu Ala Ile Arg
    160                 165                 170
tct gta att aat cca cca gtt aaa aaa tca aaa agt aaa agt gga tgt 577
Ser Val Ile Asn Pro Pro Val Lys Lys Ser Lys Ser Lys Ser Gly Cys
175                 180                 185                 190
aat atc ttg taaaa 591
Asn Ile Leu

<210> 330
<211> 193
<212> PRT
<213> Dictyostelium discoideum

<400> 330
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly
        35                  40                  45
Lys Pro Ile Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Ile Ile Ser Pro Ser Ser Tyr Glu Asn Val Ser Gly Lys
                85                  90                  95
Trp Gly Pro Glu Val Phe His His Ala Pro Asn Val Pro Ile Ile Leu
            100                 105                 110
Val Gly Thr Lys Met Asp Met Arg Glu Asp Lys Glu Thr Gln Asp Arg
        115                 120                 125
Leu Lys Glu Lys Lys Leu Tyr Pro Val Ser Tyr Glu Gln Gly Leu Leu

    130                 135                 140
Lys Met Lys Glu Ile Asn Ala Phe Lys Tyr Leu Glu Cys Ser Ala Leu
145                 150                 155                 160
Thr Gln Lys Gly Leu Lys Thr Val Phe Asp Glu Ala Ile Arg Ser Val
                165                 170                 175
Ile Asn Pro Pro Val Lys Lys Ser Lys Ser Lys Ser Gly Cys Asn Ile
            180                 185                 190
Leu

<210> 331
<211> 723
<212> DNA
<213> Entamoeba histolytica

<220>
<221> CDS
<222> (22)..(621)

<400> 331
aaataaaaaa aaacatgacc t atg aga cca gtg aaa ctt gtc atc gtc ggt 51
Met Arg Pro Val Lys Leu Val Ile Val Gly
1               5                   10
gat ggt gcc gtc ggt aaa act tgt atg tta att tca tat aca aca aat 99
Asp Gly Ala Val Gly Lys Thr Cys Met Leu Ile Ser Tyr Thr Thr Asn
                15                  20                  25
gct ttc cca aat gaa tat att cca aca gtc ttt gaa aat tat aat tct 147
Ala Phe Pro Asn Glu Tyr Ile Pro Thr Val Phe Glu Asn Tyr Asn Ser
            30                  35                  40
tca ttg gtt gtt gat gat gtt aaa att aat ctt gga tta tgg gat act 195
Ser Leu Val Val Asp Asp Val Lys Ile Asn Leu Gly Leu Trp Asp Thr
        45                  50                  55
gct gga caa gaa gat tat gat aga tta aga cca tta tca tat cca tca 243
Ala Gly Gln Glu Asp Tyr Asp Arg Leu Arg Pro Leu Ser Tyr Pro Ser
    60                  65                  70
act gat gta ttc ctt gtt tgt ttc tcc gtt att gct cca gct tca tat 291
Thr Asp Val Phe Leu Val Cys Phe Ser Val Ile Ala Pro Ala Ser Tyr
75                  80                  85                  90
gaa aat gtt gaa ggt aaa tgg aaa cca gaa att gat caa cac tgt cca 339
Glu Asn Val Glu Gly Lys Trp Lys Pro Glu Ile Asp Gln His Cys Pro
                95                  100                 105
aat gta cca att att ctt gtt gga act aaa att gat att aga gat gat 387
Asn Val Pro Ile Ile Leu Val Gly Thr Lys Ile Asp Ile Arg Asp Asp
            110                 115                 120
cca gaa caa gtt aaa cgt tta gct gaa aag aat att gtc cca att caa 435
Pro Glu Gln Val Lys Arg Leu Ala Glu Lys Asn Ile Val Pro Ile Gln
        125                 130                 135
cct cct caa gga gat gaa tta gca aag aaa att ggt gct gtt aaa tat 483
Pro Pro Gln Gly Asp Glu Leu Ala Lys Lys Ile Gly Ala Val Lys Tyr
    140                 145                 150
att gaa tgt tct gct tta aca caa gcc aat ctt aaa ctt gtt ttt gaa 531
Ile Glu Cys Ser Ala Leu Thr Gln Ala Asn Leu Lys Leu Val Phe Glu
155                 160                 165                 170
gaa gct gtt aga gct gtt ctt gct aaa gct gct aaa gag cca act gga 579
Glu Ala Val Arg Ala Val Leu Ala Lys Ala Ala Lys Glu Pro Thr Gly
                175                 180                 185
aag aaa gaa aaa gga ggt aag aaa gga tgc tca tta ttc taatttcact 628
Lys Lys Glu Lys Gly Gly Lys Lys Gly Cys Ser Leu Phe
            190                 195
ttttagttga taaattgaag aagttgttat aaaagaaaat atatttgttt tttgtaaaaa 688

aatagaaaat aaaagaagaa aaagattgat ttaaa 723

<210> 332

<211> 199
<212> PRT
<213> Entamoeba histolytica

<400> 332
Met Arg Pro Val Lys Leu Val Ile Val Gly Asp Gly Ala Val Gly Lys
1               5                   10                  15
Thr Cys Met Leu Ile Ser Tyr Thr Thr Asn Ala Phe Pro Asn Glu Tyr
            20                  25                  30
Ile Pro Thr Val Phe Glu Asn Tyr Asn Ser Ser Leu Val Val Asp Asp
        35                  40                  45
Val Lys Ile Asn Leu Gly Leu Trp Asp Thr Ala Gly Gln Glu Asp Tyr
    50                  55                  60
Asp Arg Leu Arg Pro Leu Ser Tyr Pro Ser Thr Asp Val Phe Leu Val
65                  70                  75                  80
Cys Phe Ser Val Ile Ala Pro Ala Ser Tyr Glu Asn Val Glu Gly Lys
                85                  90                  95
Trp Lys Pro Glu Ile Asp Gln His Cys Pro Asn Val Pro Ile Ile Leu
            100                 105                 110
Val Gly Thr Lys Ile Asp Ile Arg Asp Asp Pro Glu Gln Val Lys Arg
        115                 120                 125
Leu Ala Glu Lys Asn Ile Val Pro Ile Gln Pro Pro Gln Gly Asp Glu
    130                 135                 140
Leu Ala Lys Lys Ile Gly Ala Val Lys Tyr Ile Glu Cys Ser Ala Leu
145                 150                 155                 160
Thr Gln Ala Asn Leu Lys Leu Val Phe Glu Glu Ala Val Arg Ala Val
                165                 170                 175
Leu Ala Lys Ala Ala Lys Glu Pro Thr Gly Lys Lys Glu Lys Gly Gly
            180                 185                 190
Lys Lys Gly Cys Ser Leu Phe
        195

<210> 333
<211> 597
<212> DNA
<213> Candida albicans

<220>
<221> CDS
<222> (1)..(597)

<400> 333
atg gtt aac ggt cca gct gaa ctt cgt aga aaa tta gtc att gtc ggt      48
Met Val Asn Gly Pro Ala Glu Leu Arg Arg Lys Leu Val Ile Val Gly
1               5                   10                  15
gat ggt gct tgt ggt aag act tgt tta tta att gtt ttt tca aaa ggt      96
Asp Gly Ala Cys Gly Lys Thr Cys Leu Leu Ile Val Phe Ser Lys Gly
            20                  25                  30
act ttc cca gaa gtt tat gtc cca aca gtt ttt gaa aat tac gtt gct      144
Thr Phe Pro Glu Val Tyr Val Pro Thr Val Phe Glu Asn Tyr Val Ala
        35                  40                  45
gat gtt gaa gtt gat ggt aga aaa gtt gaa ttg gca tta tgg gat act      192
Asp Val Glu Val Asp Gly Arg Lys Val Glu Leu Ala Leu Trp Asp Thr
    50                  55                  60
gct ggt caa gaa gat tat gat aga tta aga cca tta tct tat cca gat      240
Ala Gly Gln Glu Asp Tyr Asp Arg Leu Arg Pro Leu Ser Tyr Pro Asp
65                  70                  75                  80
tct aat gtt att ttg att tgt ttt tca gtt gat tca cca gat tct tta      288
Ser Asn Val Ile Leu Ile Cys Phe Ser Val Asp Ser Pro Asp Ser Leu
                85                  90                  95
gat aac gtt tta gaa aaa tgg att tct gaa gtt tta cat ttc tgt caa      336
Asp Asn Val Leu Glu Lys Trp Ile Ser Glu Val Leu His Phe Cys Gln
            100                 105                 110
ggt gtt cca atc att tta gtt ggt tgt aaa tct gat tta aga gat gat      384
Gly Val Pro Ile Ile Leu Val Gly Cys Lys Ser Asp Leu Arg Asp Asp
        115                 120                 125
cct cat act att gaa gcc ttg aga caa caa caa caa cca gtc tca      432
Pro His Thr Ile Glu Ala Leu Arg Gln Gln Gln Gln Pro Val Ser

130                 135                 140
act tct gaa ggc caa caa gtt gct caa aga att ggt gct gct gat tac       480
Thr Ser Glu Gly Gln Gln Val Ala Gln Arg Ile Gly Ala Ala Asp Tyr
145                 150                 155                 160
ttg gaa tgt tct gct aaa acc ggt aga ggt gtt aga gaa gtg ttt gaa       528
Leu Glu Cys Ser Ala Lys Thr Gly Arg Gly Val Arg Glu Val Phe Glu
                165                 170                 175
gct gct act aga gct tct tta aga gtt aaa gaa aag aag gaa aag aag       576
Ala Ala Thr Arg Ala Ser Leu Arg Val Lys Glu Lys Lys Glu Lys Lys
            180                 185                 190
aag aaa tgt gtt gtc ttg taa                                           597
Lys Lys Cys Val Val Leu
        195

<210> 334
<211> 198
<212> PRT
<213> Candida albicans

<400> 334
   Met Val Asn Gly Pro Ala Glu Leu Arg Arg Lys Leu Val Ile Val Gly
   1               5                   10                  15
   Asp Gly Ala Cys Gly Lys Thr Cys Leu Leu Ile Val Phe Ser Lys Gly
               20                  25                  30
   Thr Phe Pro Glu Val Tyr Val Pro Thr Val Phe Glu Asn Tyr Val Ala
           35                  40                  45
   Asp Val Glu Val Asp Gly Arg Lys Val Glu Leu Ala Leu Trp Asp Thr
       50                  55                  60
   Ala Gly Gln Glu Asp Tyr Asp Arg Leu Arg Pro Leu Ser Tyr Pro Asp
   65                  70                  75                  80
   Ser Asn Val Ile Leu Ile Cys Phe Ser Val Asp Ser Pro Asp Ser Leu
                   85                  90                  95
   Asp Asn Val Leu Glu Lys Trp Ile Ser Glu Val Leu His Phe Cys Gln
               100                 105                 110
   Gly Val Pro Ile Ile Leu Val Gly Cys Lys Ser Asp Leu Arg Asp Asp
           115                 120                 125
   Pro His Thr Ile Glu Ala Leu Arg Gln Gln Gln Gln Gln Pro Val Ser
       130                 135                 140
   Thr Ser Glu Gly Gln Gln Val Ala Gln Arg Ile Gly Ala Ala Asp Tyr
   145                 150                 155                 160
   Leu Glu Cys Ser Ala Lys Thr Gly Arg Gly Val Arg Glu Val Phe Glu
                   165                 170                 175
   Ala Ala Thr Arg Ala Ser Leu Arg Val Lys Glu Lys Lys Glu Lys Lys
               180                 185                 190
   Lys Lys Cys Val Val Leu
           195

<210> 335
<211> 1165
<212> DNA
<213> Kluyveromyces lactis

<220>
<221> CDS
<222> (102)..(728)

<400> 335
aagtgtcata aaacagaatt actcaagagt tttcgaacag ctttgatttt ataatacaat      60

aaaaaaaagt agtgatttga tttgttttaa ccctttccat a atg tct caa gct gtt     116
                                              Met Ser Gln Ala Val
                                              1               5
ggt aat gtt gcc agc att aga aga aag cta gtt att gtc ggt gat ggt       164
Gly Asn Val Ala Ser Ile Arg Arg Lys Leu Val Ile Val Gly Asp Gly
                10                  15                  20
gcg tgt ggt aag act tgt tta ttg att gtt ttc gcc aag ggt aag ttc       212

Ala Cys Gly Lys Thr Cys Leu Leu Ile Val Phe Ala Lys Gly Lys Phe
            25                  30                  35
cct cag gtt tac gtg ccc act gtt ttc gat aat tac gtt gct gat gtc      260
Pro Gln Val Tyr Val Pro Thr Val Phe Asp Asn Tyr Val Ala Asp Val
        40                  45                  50
gaa gtt gac ggt cgt cgc gta gaa ttg gcg ttg tgg gat act gct ggc      308
Glu Val Asp Gly Arg Arg Val Glu Leu Ala Leu Trp Asp Thr Ala Gly
    55                  60                  65
caa gaa gat tat gat aga ttg aga cca ctt tcg tac cca gat tcc aac      356
Gln Glu Asp Tyr Asp Arg Leu Arg Pro Leu Ser Tyr Pro Asp Ser Asn
70                  75                  80                  85
gtt gtg tta atc tgt tac tca att gat ttg cca gat tcc tta gaa aac      404
Val Val Leu Ile Cys Tyr Ser Ile Asp Leu Pro Asp Ser Leu Glu Asn
                90                  95                  100
gtg atg gaa aaa tgg atc tct gaa gtt tta cac ttt tgt caa ggt gtt      452
Val Met Glu Lys Trp Ile Ser Glu Val Leu His Phe Cys Gln Gly Val
            105                 110                 115
cca atc atc ttg gtt ggt tgt aaa gca gat ttg aga aat gat cct caa      500
Pro Ile Ile Leu Val Gly Cys Lys Ala Asp Leu Arg Asn Asp Pro Gln
        120                 125                 130
gtt gtt gaa gaa cta aga gct caa gga tta caa cct gtg tct cag gct      548
Val Val Glu Glu Leu Arg Ala Gln Gly Leu Gln Pro Val Ser Gln Ala
    135                 140                 145
caa gca caa gaa gtc gct gac caa atc ggt gct gtt gac tac atc gaa      596
Gln Ala Gln Glu Val Ala Asp Gln Ile Gly Ala Val Asp Tyr Ile Glu
150                 155                 160                 165
tgt tct gcc aag acc ggt tac ggt gtc aga gaa gtc ttc gag gct gct      644
Cys Ser Ala Lys Thr Gly Tyr Gly Val Arg Glu Val Phe Glu Ala Ala
                170                 175                 180
aca aga gct tct ttg gtc ggt aaa caa ggt aag agc aaa cct aag act      692
Thr Arg Ala Ser Leu Val Gly Lys Gln Gly Lys Ser Lys Pro Lys Thr
            185                 190                 195
aag tct agc aaa aaa aag aag tgt gtc gtc ttg taaataaaga cacgcacgcc      745
Lys Ser Ser Lys Lys Lys Lys Cys Val Val Leu
        200                 205
ccaaacccac tcttcacccc cttatctcca acttcatttg aatttacctt gaaacagagg      805

actgagcttt gaggatatta ttattaggcg atgggaattg tttacaaccg ttaattggca      865

gaagtaaaga tccaaaaaaa aagaagaggg aagacgaaca aaccatattc atttgtttct      925

aattgatcaa tttttaatat acaaatgtca gcaattaatt tccgttgaat ttcctaattt      985

tttctcctcg ttcttttctt tgtttccatc tggtttcaag tgaccatccc tccctgacat      1045

tttctcttct acctctcttc tgttttgtgt gtttctacct ataaattatt tttatatacc      1105

tacactcttt tattttgtaa gaaacttaaa tattatcacc actatactgc actttctcat      1165

<210> 336
<211> 208
<212> PRT
<213> Kluyveromyces lactis

<400> 336
Met Ser Gln Ala Val Gly Asn Val Ala Ser Ile Arg Arg Lys Leu Val
1               5                   10                  15
Ile Val Gly Asp Gly Ala Cys Gly Lys Thr Cys Leu Leu Ile Val Phe
            20                  25                  30

Ala Lys Gly Lys Phe Pro Gln Val Tyr Val Pro Thr Val Phe Asp Asn
        35                  40                  45
Tyr Val Ala Asp Val Glu Val Asp Gly Arg Arg Val Glu Leu Ala Leu
    50                  55                  60
Trp Asp Thr Ala Gly Gln Glu Asp Tyr Asp Arg Leu Arg Pro Leu Ser
65                  70                  75                  80
Tyr Pro Asp Ser Asn Val Val Leu Ile Cys Tyr Ser Ile Asp Leu Pro
                85                  90                  95
Asp Ser Leu Glu Asn Val Met Glu Lys Trp Ile Ser Glu Val Leu His
            100                 105                 110
Phe Cys Gln Gly Val Pro Ile Ile Leu Val Gly Cys Lys Ala Asp Leu
        115                 120                 125
Arg Asn Asp Pro Gln Val Val Glu Glu Leu Arg Ala Gln Gly Leu Gln
    130                 135                 140
Pro Val Ser Gln Ala Gln Ala Gln Glu Val Ala Asp Gln Ile Gly Ala
145                 150                 155                 160
Val Asp Tyr Ile Glu Cys Ser Ala Lys Thr Gly Tyr Gly Val Arg Glu
                165                 170                 175
Val Phe Glu Ala Ala Thr Arg Ala Ser Leu Val Gly Lys Gln Gly Lys
            180                 185                 190
Ser Lys Pro Lys Thr Lys Ser Ser Lys Lys Lys Lys Cys Val Val Leu
        195                 200                 205

<210> 337
<211> 1181
<212> DNA
<213> Schizophyllum commune

<220>
<221> CDS
<222> (139)..(759)

<400> 337
ctggtctctc gtcctttctt ccaccacgcg cacgcgcacc cacgtttctt ctcccactcc      60

cggcgctgtc ggccccgtcg ccctcgcgtc cttcctcttc ctcttccgcc agctcagtga     120

caggtacaca ctcccctt atg gcg ctc tgc ggt tcg tcg aaa ggg cgc ggc       171
                    Met Ala Leu Cys Gly Ser Ser Lys Gly Arg Gly
                    1               5                   10
agg ggc cga ccg atc cag cgc aag gtc gtc gta tgc ggc gac ggc gcg       219
Arg Gly Arg Pro Ile Gln Arg Lys Val Val Val Cys Gly Asp Gly Ala
            15                  20                  25
tgc ggg aag acg agt ctg ctg aac gtc ttc acg agg ggg ttc ttc acg       267
Cys Gly Lys Thr Ser Leu Leu Asn Val Phe Thr Arg Gly Phe Phe Thr
        30                  35                  40
cag gtt tat gaa ccg acg gtg ttc gag aac tac gtg cac gat ctg tat       315
Gln Val Tyr Glu Pro Thr Val Phe Glu Asn Tyr Val His Asp Leu Tyr
    45                  50                  55
atc gac gac cag ctg gtg gag ctg agt ctc tgg gat acg gcg ggg cag       363
Ile Asp Asp Gln Leu Val Glu Leu Ser Leu Trp Asp Thr Ala Gly Gln
60                  65                  70                  75
gag gag ttc gac cgg cta cgg agc ctg tcg tat gca gaa acg cat gtg       411
Glu Glu Phe Asp Arg Leu Arg Ser Leu Ser Tyr Ala Glu Thr His Val
                80                  85                  90
atc atg ata tgc ttc agc gtc gac aat cca acg tcg ctc gag aat gtg       459
Ile Met Ile Cys Phe Ser Val Asp Asn Pro Thr Ser Leu Glu Asn Val
            95                  100                 105
gag agc aag tgg ctc gac gag att ttg gag tac tgt ccg ggc gtg aag       507
Glu Ser Lys Trp Leu Asp Glu Ile Leu Glu Tyr Cys Pro Gly Val Lys
        110                 115                 120
ttg gta ttg gtc gac tca aaa tgt gat cta cgc gac gac cct gca gta       555
Leu Val Leu Val Asp Ser Lys Cys Asp Leu Arg Asp Asp Pro Ala Val
    125                 130                 135
ctc gat cgg cta caa cga tac ggt aca cat acg gat caa tat gaa gag       603
Leu Asp Arg Leu Gln Arg Tyr Gly Thr His Thr Asp Gln Tyr Glu Glu

140                 145                 150                 155
ggc ctc ggg gtc gcg cga aga ata cga gct tca cga tac tta gag tgc      651
Gly Leu Gly Val Ala Arg Arg Ile Arg Ala Ser Arg Tyr Leu Glu Cys
                160                 165                 170
tcc tcc aaa cac aac cgg ggc gtt aac gaa gtc ttc tta cga ggc cgc      699
Ser Ser Lys His Asn Arg Gly Val Asn Glu Val Phe Leu Arg Gly Arg
            175                 180                 185
gcg cgt gtc act gtc cac tcg atc agg cag ggg agc gcc ggg tcg tgt      747
Ala Arg Val Thr Val His Ser Ile Arg Gln Gly Ser Ala Gly Ser Cys
        190                 195                 200
tgt gtc atg tagcgcacct cactctcgtc agtctagtcc cccaagccga tcgacgctgc   806
Cys Val Met
    205
gctggcgctc cccgtcgacc tctcaaactg ctctgcccca ctatgtcaca ccaagcggcc    866

catcgacccg tcctagacca cactacacag gtgttaggac gcaagagaac tccagcgcgg    926

cccgccctcg gtccccatgc tcctgggcgt gtagtgcttc gtgcactcct gttcgtaggt    986

gcgggggtac tcctccccac tcgtgcggtg ctcaaggacg tgtagggggc agctcggtgc     1046

gctgtccgaa taagatagat atataccccc ccaccccgaa ggtcacatac gcatggatat    1106

cttcacgctc aaatatgtta cgctccctta atctaactgc gtcttatatg ggaattgaat    1166

gcgtttgata tgggc                                                     1181

<210> 338
<211> 206
<212> PRT
<213> Schizophyllum commune

<400> 338
Met Ala Leu Cys Gly Ser Ser Lys Gly Arg Gly Arg Gly Arg Pro Ile
1               5                   10                  15
Gln Arg Lys Val Val Val Cys Gly Asp Gly Ala Cys Gly Lys Thr Ser
            20                  25                  30
Leu Leu Asn Val Phe Thr Arg Gly Phe Phe Thr Gln Val Tyr Glu Pro
        35                  40                  45
Thr Val Phe Glu Asn Tyr Val His Asp Leu Tyr Ile Asp Asp Gln Leu
    50                  55                  60
Val Glu Leu Ser Leu Trp Asp Thr Ala Gly Gln Glu Glu Phe Asp Arg
65                  70                  75                  80
Leu Arg Ser Leu Ser Tyr Ala Glu Thr His Val Ile Met Ile Cys Phe
                85                  90                  95
Ser Val Asp Asn Pro Thr Ser Leu Glu Asn Val Glu Ser Lys Trp Leu
            100                 105                 110
Asp Glu Ile Leu Glu Tyr Cys Pro Gly Val Lys Leu Val Leu Val Asp
        115                 120                 125
Ser Lys Cys Asp Leu Arg Asp Asp Pro Ala Val Leu Asp Arg Leu Gln
    130                 135                 140
Arg Tyr Gly Thr His Thr Asp Gln Tyr Glu Glu Gly Leu Gly Val Ala
145                 150                 155                 160
Arg Arg Ile Arg Ala Ser Arg Tyr Leu Glu Cys Ser Ser Lys His Asn
                165                 170                 175
Arg Gly Val Asn Glu Val Phe Leu Arg Gly Arg Ala Arg Val Thr Val
            180                 185                 190
His Ser Ile Arg Gln Gly Ser Ala Gly Ser Cys Cys Val Met
        195                 200                 205

<210> 339
<211> 618
<212> DNA
<213> Schizosaccharomyces pombe

<220>
<221> CDS
<222> (1)..(618)

<400> 339
atg tca agc tgt ttc gga agt aaa aag aag cct att tat cgg aaa atc 48
Met Ser Ser Cys Phe Gly Ser Lys Lys Lys Pro Ile Tyr Arg Lys Ile
1               5                   10                  15
gta att ctt ggt gat ggt gct gct ggt aaa acc agt ttg tta aat gta 96
Val Ile Leu Gly Asp Gly Ala Ala Gly Lys Thr Ser Leu Leu Asn Val
            20                  25                  30
ttt act aag ggt tat ttc cct cag gta tac gag cct act ata ttt gaa 144
Phe Thr Lys Gly Tyr Phe Pro Gln Val Tyr Glu Pro Thr Ile Phe Glu
        35                  40                  45
aac tac att cat gat atc ttt gtc gat gga aac agt ata gaa ctg tct 192
Asn Tyr Ile His Asp Ile Phe Val Asp Gly Asn Ser Ile Glu Leu Ser
    50                  55                  60
cta tgg gat aca gct ggt caa gaa gag tat gat caa ctg cgt tcg tta 240
Leu Trp Asp Thr Ala Gly Gln Glu Glu Tyr Asp Gln Leu Arg Ser Leu
65                  70                  75                  80
tca tat tca gat aca cat gtt att atg atc tgc ttt gcc gtg gat tca 288
Ser Tyr Ser Asp Thr His Val Ile Met Ile Cys Phe Ala Val Asp Ser
                85                  90                  95
cga gac tca tta gaa aat gta atc aca aaa tgg ctt ccg gaa gtc tct 336
Arg Asp Ser Leu Glu Asn Val Ile Thr Lys Trp Leu Pro Glu Val Ser
            100                 105                 110
agt aat tgc cct ggt gtt aaa ttg gtt ctt gtt gct cta aaa tgt gat 384
Ser Asn Cys Pro Gly Val Lys Leu Val Leu Val Ala Leu Lys Cys Asp
        115                 120                 125
tta cgt gga gct gat gag gag caa gtt gat cac agt aaa att att gat 432
Leu Arg Gly Ala Asp Glu Glu Gln Val Asp His Ser Lys Ile Ile Asp
    130                 135                 140
tac gag gaa gga ctg gca gcg gca aaa aaa atc aac gct gta cga tat 480
Tyr Glu Glu Gly Leu Ala Ala Ala Lys Lys Ile Asn Ala Val Arg Tyr
145                 150                 155                 160
tta gaa tgc agc gct aaa tta aat cgt ggc gta aat gaa gct ttc acg 528
Leu Glu Cys Ser Ala Lys Leu Asn Arg Gly Val Asn Glu Ala Phe Thr
                165                 170                 175
gaa gct gca cgc gtt gcc ctt gcc gcg caa cca aga ggt aca aag gat 576
Glu Ala Ala Arg Val Ala Leu Ala Ala Gln Pro Arg Gly Thr Lys Asp
            180                 185                 190
ggt gct gat gaa tcc cat ggt acc gga tgt atc att gct tga 618
Gly Ala Asp Glu Ser His Gly Thr Gly Cys Ile Ile Ala
        195                 200                 205


<210> 340
<211> 205
<212> PRT
<213> Schizosaccharomyces pombe

<400> 340
Met Ser Ser Cys Phe Gly Ser Lys Lys Lys Pro Ile Tyr Arg Lys Ile
1               5                   10                  15
Val Ile Leu Gly Asp Gly Ala Ala Gly Lys Thr Ser Leu Leu Asn Val
            20                  25                  30
Phe Thr Lys Gly Tyr Phe Pro Gln Val Tyr Glu Pro Thr Ile Phe Glu
        35                  40                  45
Asn Tyr Ile His Asp Ile Phe Val Asp Gly Asn Ser Ile Glu Leu Ser
    50                  55                  60
Leu Trp Asp Thr Ala Gly Gln Glu Glu Tyr Asp Gln Leu Arg Ser Leu
65                  70                  75                  80

Ser Tyr Ser Asp Thr His Val Ile Met Ile Cys Phe Ala Val Asp Ser
                85                  90                  95
Arg Asp Ser Leu Glu Asn Val Ile Thr Lys Trp Leu Pro Glu Val Ser
            100                 105                 110
Ser Asn Cys Pro Gly Val Lys Leu Val Leu Val Ala Leu Lys Cys Asp
        115                 120                 125
Leu Arg Gly Ala Asp Glu Glu Gln Val Asp His Ser Lys Ile Ile Asp
    130                 135                 140
Tyr Glu Glu Gly Leu Ala Ala Ala Lys Lys Ile Asn Ala Val Arg Tyr
145                 150                 155                 160
Leu Glu Cys Ser Ala Lys Leu Asn Arg Gly Val Asn Glu Ala Phe Thr
                165                 170                 175
Glu Ala Ala Arg Val Ala Leu Ala Ala Gln Pro Arg Gly Thr Lys Asp
            180                 185                 190
Gly Ala Asp Glu Ser His Gly Thr Gly Cys Ile Ile Ala
        195                 200                 205

<210> 341
<211> 582
<212> DNA
<213> Emericella nidulans

<220>
<221> CDS
<222> (1)..(582)

<400> 341
atg gct gag atc cgc cgc aag ctt gtt atc gtt ggt gat ggt gcc tgc      48
Met Ala Glu Ile Arg Arg Lys Leu Val Ile Val Gly Asp Gly Ala Cys
1               5                   10                  15
ggt aag acc tgt ctg ttg atc gtc ttc tca aag ggc act ttc cct gag      96
Gly Lys Thr Cys Leu Leu Ile Val Phe Ser Lys Gly Thr Phe Pro Glu
            20                  25                  30
gtc tac gtc ccc acc gtc ttt gag aac tac gtt gcc gat gtt gag gtt      144
Val Tyr Val Pro Thr Val Phe Glu Asn Tyr Val Ala Asp Val Glu Val
        35                  40                  45
gat ggc aag cac gtc gag ctc gct ctc tgg gat acg gct ggt caa gaa      192
Asp Gly Lys His Val Glu Leu Ala Leu Trp Asp Thr Ala Gly Gln Glu
    50                  55                  60
gat tac gac cgt ctc cgc cct ctc tcc tac cct gac tcg cat gtc atc      240
Asp Tyr Asp Arg Leu Arg Pro Leu Ser Tyr Pro Asp Ser His Val Ile
65                  70                  75                  80
ctg att tgc ttc gct gtc gac tca ccg gat tcc ctt gac aac gtt caa      288
Leu Ile Cys Phe Ala Val Asp Ser Pro Asp Ser Leu Asp Asn Val Gln
                85                  90                  95
gag aag tgg atc tct gaa gtc cta cac ttc tgc cag ggt ctc ccc atc      336
Glu Lys Trp Ile Ser Glu Val Leu His Phe Cys Gln Gly Leu Pro Ile
            100                 105                 110
atc ctc gtc gga tgc aag aag gat ctt cgc cat gac ccc aag acg atc      384
Ile Leu Val Gly Cys Lys Lys Asp Leu Arg His Asp Pro Lys Thr Ile
        115                 120                 125
gag gag ctg aac aag acc tct cag aag cct gtc acc ccc gaa cag ggt      432
Glu Glu Leu Asn Lys Thr Ser Gln Lys Pro Val Thr Pro Glu Gln Gly
    130                 135                 140
gag gaa gtc cgc aag aag att ggc gcc tac aag tac ctc gag tgc tct      480
Glu Glu Val Arg Lys Lys Ile Gly Ala Tyr Lys Tyr Leu Glu Cys Ser
145                 150                 155                 160
gct cga acc aac gag ggt gtc cgt gag gtc ttt gag gct gcc acg cgt      528
Ala Arg Thr Asn Glu Gly Val Arg Glu Val Phe Glu Ala Ala Thr Arg
                165                 170                 175
gct gcc ctc ttg acc aag acc cac aag agc aag aag aag tgc agc atc      576
Ala Ala Leu Leu Thr Lys Thr His Lys Ser Lys Lys Lys Cys Ser Ile
            180                 185                 190
ctg taa                                                              582
Leu

<210> 342
<211> 193
<212> PRT
<213> Emericella nidulans

<400> 342
Met Ala Glu Ile Arg Arg Lys Leu Val Ile Val Gly Asp Gly Ala Cys
1               5                   10                  15
Gly Lys Thr Cys Leu Leu Ile Val Phe Ser Lys Gly Thr Phe Pro Glu
            20                  25                  30
Val Tyr Val Pro Thr Val Phe Glu Asn Tyr Val Ala Asp Val Glu Val
        35                  40                  45
Asp Gly Lys His Val Glu Leu Ala Leu Trp Asp Thr Ala Gly Gln Glu
    50                  55                  60
Asp Tyr Asp Arg Leu Arg Pro Leu Ser Tyr Pro Asp Ser His Val Ile
65                  70                  75                  80
Leu Ile Cys Phe Ala Val Asp Ser Pro Asp Ser Leu Asp Asn Val Gln
                85                  90                  95
Glu Lys Trp Ile Ser Glu Val Leu His Phe Cys Gln Gly Leu Pro Ile
            100                 105                 110
Ile Leu Val Gly Cys Lys Lys Asp Leu Arg His Asp Pro Lys Thr Ile
        115                 120                 125
Glu Glu Leu Asn Lys Thr Ser Gln Lys Pro Val Thr Pro Glu Gln Gly
    130                 135                 140
Glu Glu Val Arg Lys Lys Ile Gly Ala Tyr Lys Tyr Leu Glu Cys Ser
145                 150                 155                 160
Ala Arg Thr Asn Glu Gly Val Arg Glu Val Phe Glu Ala Ala Thr Arg
                165                 170                 175
Ala Ala Leu Leu Thr Lys Thr His Lys Ser Lys Lys Lys Cys Ser Ile
            180                 185                 190
Leu

<210> 343
<211> 1086
<212> DNA
<213> Homo sapiens

<220>
<221> CDS
<222> (40)..(672)

<400> 343
cgcagccgcc cgcccgcccg ctcagcgccc ggccccggg atg acg gcg gcc cag      54
                                           Met Thr Ala Ala Gln
                                           1               5
gcc gcg ggt gag gag gcg cca cca ggc gtg cgg tcc gtc aag gtg gtc      102
Ala Ala Gly Glu Glu Ala Pro Pro Gly Val Arg Ser Val Lys Val Val
                10                  15                  20
ctg gtg ggc gac ggc ggc tgc ggg aag acg tcg ctg ctg atg gtc ttc      150
Leu Val Gly Asp Gly Gly Cys Gly Lys Thr Ser Leu Leu Met Val Phe
            25                  30                  35
gcc gat ggg gcc ttc ccc gag agc tac acc ccc acg gtg ttt gag cgg      198
Ala Asp Gly Ala Phe Pro Glu Ser Tyr Thr Pro Thr Val Phe Glu Arg
        40                  45                  50
tac atg gtc aac ctg caa gtg aaa ggc aaa cct gtg cac ctc cac atc      246
Tyr Met Val Asn Leu Gln Val Lys Gly Lys Pro Val His Leu His Ile
    55                  60                  65
tgg gac aca gca ggg caa gat gac tat gac cgc ctg cgg ccc ctg ttc      294
Trp Asp Thr Ala Gly Gln Asp Asp Tyr Asp Arg Leu Arg Pro Leu Phe
70                  75                  80                  85
tac cct gac gcc agc gtc ctg ctg ctt tgc ttc gat gtc acc agc ccg      342
Tyr Pro Asp Ala Ser Val Leu Leu Leu Cys Phe Asp Val Thr Ser Pro
                90                  95                  100
aac agc ttt gac aac atc ttt aac cgg tgg tac cca gaa gtg aat cat      390
Asn Ser Phe Asp Asn Ile Phe Asn Arg Trp Tyr Pro Glu Val Asn His
            105                 110                 115
ttc tgc aag aag gta ccc atc atc gtc gtg ggc tgc aag act gac ctg      438

Phe Cys Lys Lys Val Pro Ile Ile Val Val Gly Cys Lys Thr Asp Leu
        120                 125                 130
cgc aag gac aaa tca ctg gtg aac aag ctc cga aga aac gga ttg gag        486
Arg Lys Asp Lys Ser Leu Val Asn Lys Leu Arg Arg Asn Gly Leu Glu
    135                 140                 145
cct gtg acc tac cac agg ggc cag gag atg gcg agg tcc gtg ggc gcg        534
Pro Val Thr Tyr His Arg Gly Gln Glu Met Ala Arg Ser Val Gly Ala
150                 155                 160                 165
gtg gcc tac ctc gag tgc tcg gct cgg ctc cat gac aac gtc cac gcc        582
Val Ala Tyr Leu Glu Cys Ser Ala Arg Leu His Asp Asn Val His Ala
                170                 175                 180
gtc ttc cag gag gcc gcc gag gtg gcc ctc agc agc cgc ggt cgc aac        630
Val Phe Gln Glu Ala Ala Glu Val Ala Leu Ser Ser Arg Gly Arg Asn
            185                 190                 195
ttc tgg cgg cgg att acc cag ggc ttt tgc gtg gtg acc tgagcggctc        679
Phe Trp Arg Arg Ile Thr Gln Gly Phe Cys Val Val Thr
        200                 205                 210
ggggcgtccc agcgacgcgg gaaggggcag ggcgctgacc tgctgctgag ctggctgggc        739

tggacccggt ccctaggctg tgaccgccga actccactgc aacagacggg cgccaccaaa        799

gccaggccct gaggcctggg agtcctggac tgagaaaggg ggttcctggg cccacctgct        859

ctgtgtaggg ctcgtcctgc ggtgcccgag aatcactcgc taacccctat gcccggtccc        919

ggaccgacat cctggagccg cctgtgcagc ctgatgcccc ctcgtggctg ctcccagggc        979

tgcacctgcc aggacctaat gttcttaggt ccctctggcc agaacccaca cccggcccct        1039

tcccacctgt catactggta actgtaacaa gaaaaacgac atcactt                    1086

<210> 344
<211> 210
<212> PRT
<213> Homo sapiens

<400> 344
Met Thr Ala Ala Gln Ala Ala Gly Glu Glu Ala Pro Pro Gly Val Arg
1               5                   10                  15
Ser Val Lys Val Val Leu Val Gly Asp Gly Gly Cys Gly Lys Thr Ser
            20                  25                  30
Leu Leu Met Val Phe Ala Asp Gly Ala Phe Pro Glu Ser Tyr Thr Pro
        35                  40                  45
Thr Val Phe Glu Arg Tyr Met Val Asn Leu Gln Val Lys Gly Lys Pro
    50                  55                  60
Val His Leu His Ile Trp Asp Thr Ala Gly Gln Asp Asp Tyr Asp Arg
65                  70                  75                  80
Leu Arg Pro Leu Phe Tyr Pro Asp Ala Ser Val Leu Leu Leu Cys Phe
                85                  90                  95
Asp Val Thr Ser Pro Asn Ser Phe Asp Asn Ile Phe Asn Arg Trp Tyr
            100                 105                 110
Pro Glu Val Asn His Phe Cys Lys Lys Val Pro Ile Ile Val Val Gly
        115                 120                 125
Cys Lys Thr Asp Leu Arg Lys Asp Lys Ser Leu Val Asn Lys Leu Arg
    130                 135                 140
Arg Asn Gly Leu Glu Pro Val Thr Tyr His Arg Gly Gln Glu Met Ala
145                 150                 155                 160
Arg Ser Val Gly Ala Val Ala Tyr Leu Glu Cys Ser Ala Arg Leu His
                165                 170                 175

Asp Asn Val His Ala Val Phe Gln Glu Ala Ala Glu Val Ala Leu Ser
        180                 185                 190
Ser Arg Gly Arg Asn Phe Trp Arg Arg Ile Thr Gln Gly Phe Cys Val
    195                 200                 205
Val Thr
    210

<210> 345
<211> 1166
<212> DNA
<213> Mus musculus

<220>
<221> CDS
<222> (85)..(717)

<400> 345
gccgccgggc cagccccgcg cccgccgcag ccagcccgcc gcgtaccgcc tgctgctccg      60

cgcaccgccg tccgccagcc aggg atg aac gcg tcc cag gtt gcg gga gaa        111
                           Met Asn Ala Ser Gln Val Ala Gly Glu
                           1               5
gag gcg ccg cag agc ggg cac tcg gtc aag gtg gtc ctg gtg ggc gac        159
Glu Ala Pro Gln Ser Gly His Ser Val Lys Val Val Leu Val Gly Asp
10                  15                  20                  25
ggg ggc tgc ggg aag acg tca ctg atg atg gtc ttc gcc aaa ggg gcc        207
Gly Gly Cys Gly Lys Thr Ser Leu Met Met Val Phe Ala Lys Gly Ala
                30                  35                  40
ttc cca gag agc tac agt ccc aca gtg ttt gag cgc tat aat gcc act        255
Phe Pro Glu Ser Tyr Ser Pro Thr Val Phe Glu Arg Tyr Asn Ala Thr
            45                  50                  55
ctg cag atg aag ggt aaa cct gtg cac ctc caa atc tgg gac aca gcc        303
Leu Gln Met Lys Gly Lys Pro Val His Leu Gln Ile Trp Asp Thr Ala
        60                  65                  70
ggg caa gat gac tat gac cgc ctc cgg ccc ttg ttc tat cct gat gcc        351
Gly Gln Asp Asp Tyr Asp Arg Leu Arg Pro Leu Phe Tyr Pro Asp Ala
    75                  80                  85
aat gtc ttg ctc ctc tgc ttc gat gtg acc aat cca aac agc ttt gac        399
Asn Val Leu Leu Leu Cys Phe Asp Val Thr Asn Pro Asn Ser Phe Asp
90                  95                  100                 105
aac gtc tcc aac cgg tgg tac cca gag gtg aca cat ttc tgc aag gga        447
Asn Val Ser Asn Arg Trp Tyr Pro Glu Val Thr His Phe Cys Lys Gly
                110                 115                 120
gtg ccc atc att gtt gtg ggc tgc aag ata gac ctg cgt aag gac aag        495
Val Pro Ile Ile Val Val Gly Cys Lys Ile Asp Leu Arg Lys Asp Lys
            125                 130                 135
gtg ctg gtg aac aac ctg cgg aag aaa aga ctg gag ccc gtg acc tac        543
Val Leu Val Asn Asn Leu Arg Lys Lys Arg Leu Glu Pro Val Thr Tyr
        140                 145                 150
cac agg ggc cac gat atg gca agg tct gtg gga gcg gtg gcc tat ctt        591
His Arg Gly His Asp Met Ala Arg Ser Val Gly Ala Val Ala Tyr Leu
    155                 160                 165
gag tgt tca gct cgg ctc cat gac aac gtg gaa gca gtc ttc cag gaa        639
Glu Cys Ser Ala Arg Leu His Asp Asn Val Glu Ala Val Phe Gln Glu
170                 175                 180                 185
gca gca gaa gtg gct ctc agc agt cgc aga cat aac ttt tgg cgg cgg        687
Ala Ala Glu Val Ala Leu Ser Ser Arg Arg His Asn Phe Trp Arg Arg
                190                 195                 200
att act cag aat tgt tgc ttg gcc acc tgactggctt ggaacccacc              734
Ile Thr Gln Asn Cys Cys Leu Ala Thr
            205                 210
ttgccaaccg gtttactccg ctgcagaaag acccagaagc agaacttgta ctctgttgac      794

tgggatggac ctaatcccta ggctgagctg gtagaaccac acatccatcc acagatgggc      854

tccaatgagg cctggcccct ggaaccgaat gaaacccggt aatgatagga aagaagtggt 914

cccaagaacc cttaagctct ggaaaccaat taatagaaca tcctgtgccc aaatcctgaa 974

cctgcgcctg aacggggtct gtgcggtctg tttggcgcca accttgtggc taattctaat 1034

tgaagttata tctacaggac ctaagggttc ccaagaacca acttgcccgg gctaataacc 1094

tgcgccgtgg ctcaacaacc ttaatttaat cccattgcta agtgtaacaa ctaaaatggc 1154

aagcacttgc ta 1166

<210> 346
<211> 210
<212> PRT
<213> Mus musculus

<400> 346
Met Asn Ala Ser Gln Val Ala Gly Glu Glu Ala Pro Gln Ser Gly His
1               5                   10                  15
Ser Val Lys Val Val Leu Val Gly Asp Gly Gly Cys Gly Lys Thr Ser
            20                  25                  30
Leu Met Met Val Phe Ala Lys Gly Ala Phe Pro Glu Ser Tyr Ser Pro
        35                  40                  45
Thr Val Phe Glu Arg Tyr Asn Ala Thr Leu Gln Met Lys Gly Lys Pro
    50                  55                  60
Val His Leu Gln Ile Trp Asp Thr Ala Gly Gln Asp Asp Tyr Asp Arg
65                  70                  75                  80
Leu Arg Pro Leu Phe Tyr Pro Asp Ala Asn Val Leu Leu Leu Cys Phe
                85                  90                  95
Asp Val Thr Asn Pro Asn Ser Phe Asp Asn Val Ser Asn Arg Trp Tyr
            100                 105                 110
Pro Glu Val Thr His Phe Cys Lys Gly Val Pro Ile Ile Val Val Gly
        115                 120                 125
Cys Lys Ile Asp Leu Arg Lys Asp Lys Val Leu Val Asn Asn Leu Arg
    130                 135                 140
Lys Lys Arg Leu Glu Pro Val Thr Tyr His Arg Gly His Asp Met Ala
145                 150                 155                 160
Arg Ser Val Gly Ala Val Ala Tyr Leu Glu Cys Ser Ala Arg Leu His
                165                 170                 175
Asp Asn Val Glu Ala Val Phe Gln Glu Ala Ala Glu Val Ala Leu Ser
            180                 185                 190
Ser Arg Arg His Asn Phe Trp Arg Arg Ile Thr Gln Asn Cys Cys Leu
        195                 200                 205
Ala Thr
    210

<210> 347
<211> 735
<212> DNA
<213> Sus scrofa

<220>
<221> CDS
<222> (1)..(735)

<400> 347
atg aag gag aga aga gcc agc cag aaa tta tcc agt aaa tct atc atg 48
Met Lys Glu Arg Arg Ala Ser Gln Lys Leu Ser Ser Lys Ser Ile Met
1               5                   10                  15

gat cct aat cag aac gtg aaa tgc aag ata gta gtg gtg gga gat agt 96
Asp Pro Asn Gln Asn Val Lys Cys Lys Ile Val Val Val Gly Asp Ser
            20                  25                  30
cag tgt ggg aga acc gcg ctg ctg cac gtc ttc gcc aaa gac tgc ttc 144
Gln Cys Gly Arg Thr Ala Leu Leu His Val Phe Ala Lys Asp Cys Phe
        35                  40                  45
ccc gag aat tac gtc cct aca gtg ttt gag aat tac acg gcc agt ttc 192
Pro Glu Asn Tyr Val Pro Thr Val Phe Glu Asn Tyr Thr Ala Ser Phe
    50                  55                  60
gaa atc gac aca caa aga ata gaa ttg agc ctg tgg gac act tcg ggt 240
Glu Ile Asp Thr Gln Arg Ile Glu Leu Ser Leu Trp Asp Thr Ser Gly
65                  70                  75                  80
tct cct tac tat gac aac gtc cgg ccc ctc tcc tac cca gac tca gac 288
Ser Pro Tyr Tyr Asp Asn Val Arg Pro Leu Ser Tyr Pro Asp Ser Asp
                85                  90                  95
gcc gtg ctg att tgc ttt gac atc agt aga cca gag act ctg gac agt 336
Ala Val Leu Ile Cys Phe Asp Ile Ser Arg Pro Glu Thr Leu Asp Ser
            100                 105                 110
gtc ctg aaa aag tgg aaa ggt gaa atc cag gag ttt tgt ccc aac acc 384
Val Leu Lys Lys Trp Lys Gly Glu Ile Gln Glu Phe Cys Pro Asn Thr
        115                 120                 125
aaa atg ctc ttg gtc ggc tgc aaa tct gat ctt cgg aca gat gtc agt 432
Lys Met Leu Leu Val Gly Cys Lys Ser Asp Leu Arg Thr Asp Val Ser
    130                 135                 140
aca tta gta gag ctc tca aac cac agg cag aca cca gtt tcc tac gac 480
Thr Leu Val Glu Leu Ser Asn His Arg Gln Thr Pro Val Ser Tyr Asp
145                 150                 155                 160
cag ggg gca aat atg gcc aaa cag att gga gca gcc act tac atc gaa 528
Gln Gly Ala Asn Met Ala Lys Gln Ile Gly Ala Ala Thr Tyr Ile Glu
                165                 170                 175
tgc tca gct tta cag tca gaa aat agc gtc aga gac att ttt cat gtt 576
Cys Ser Ala Leu Gln Ser Glu Asn Ser Val Arg Asp Ile Phe His Val
            180                 185                 190
gcc acc ttg gca tgt gta aat aag aca aat aaa aac gtt aag cgg aac 624
Ala Thr Leu Ala Cys Val Asn Lys Thr Asn Lys Asn Val Lys Arg Asn
        195                 200                 205
aaa tcg cag agg gca aca aag cgg att tca cac atg ccc agc aga ccg 672
Lys Ser Gln Arg Ala Thr Lys Arg Ile Ser His Met Pro Ser Arg Pro
    210                 215                 220
gaa ctc tcg gca gtg gct acg gac tta cga aag gac aaa gcc aag agc 720
Glu Leu Ser Ala Val Ala Thr Asp Leu Arg Lys Asp Lys Ala Lys Ser
225                 230                 235                 240
tgc act gtg atg tga 735
Cys Thr Val Met

<210> 348
<211> 244
<212> PRT
<213> Sus scrofa

<400> 348
Met Lys Glu Arg Arg Ala Ser Gln Lys Leu Ser Ser Lys Ser Ile Met
1               5                   10                  15
Asp Pro Asn Gln Asn Val Lys Cys Lys Ile Val Val Val Gly Asp Ser
            20                  25                  30
Gln Cys Gly Arg Thr Ala Leu Leu His Val Phe Ala Lys Asp Cys Phe
        35                  40                  45
Pro Glu Asn Tyr Val Pro Thr Val Phe Glu Asn Tyr Thr Ala Ser Phe
    50                  55                  60
Glu Ile Asp Thr Gln Arg Ile Glu Leu Ser Leu Trp Asp Thr Ser Gly
65                  70                  75                  80
Ser Pro Tyr Tyr Asp Asn Val Arg Pro Leu Ser Tyr Pro Asp Ser Asp
                85                  90                  95
Ala Val Leu Ile Cys Phe Asp Ile Ser Arg Pro Glu Thr Leu Asp Ser
            100                 105                 110
Val Leu Lys Lys Trp Lys Gly Glu Ile Gln Glu Phe Cys Pro Asn Thr

        115                 120                 125
Lys Met Leu Leu Val Gly Cys Lys Ser Asp Leu Arg Thr Asp Val Ser
    130                 135                 140
Thr Leu Val Glu Leu Ser Asn His Arg Gln Thr Pro Val Ser Tyr Asp
145                 150                 155                 160
Gln Gly Ala Asn Met Ala Lys Gln Ile Gly Ala Ala Thr Tyr Ile Glu
                165                 170                 175
Cys Ser Ala Leu Gln Ser Glu Asn Ser Val Arg Asp Ile Phe His Val
            180                 185                 190
Ala Thr Leu Ala Cys Val Asn Lys Thr Asn Lys Asn Val Lys Arg Asn
        195                 200                 205
Lys Ser Gln Arg Ala Thr Lys Arg Ile Ser His Met Pro Ser Arg Pro
    210                 215                 220
Glu Leu Ser Ala Val Ala Thr Asp Leu Arg Lys Asp Lys Ala Lys Ser
225                 230                 235                 240
Cys Thr Val Met


<210> 349
<211> 1017
<212> DNA
<213> Homo sapiens

<220>
<221> CDS
<222> (30)..(665)

<400> 349
gccgccgcca gtgctgcggg ctccgggca atg gat gcc ccc ggg gcc ctg gcc      53
                                Met Asp Ala Pro Gly Ala Leu Ala
                                1               5
cag acc gcc gcc ccc ggt ccg ggc agg aag gag ctg aag atc gtg atc     101
Gln Thr Ala Ala Pro Gly Pro Gly Arg Lys Glu Leu Lys Ile Val Ile
    10                  15                  20
gtg ggc gac ggc ggc tgc ggc aag acc tcg ctg ctc atg gtg tac agc     149
Val Gly Asp Gly Gly Cys Gly Lys Thr Ser Leu Leu Met Val Tyr Ser
25                  30                  35                  40
cag ggc tcc ttc ccc gag cac tac gcc cca tcg gtg ttc gag aag tac     197
Gln Gly Ser Phe Pro Glu His Tyr Ala Pro Ser Val Phe Glu Lys Tyr
                45                  50                  55
acg gcc agc gtg acc gtt ggc agc aag gag gtg acc ctg aac ctc tac     245
Thr Ala Ser Val Thr Val Gly Ser Lys Glu Val Thr Leu Asn Leu Tyr
            60                  65                  70
gac acg gcc ggg caa gaa gac tat gac cgg ctg cgg ccc ctg tcc tac     293
Asp Thr Ala Gly Gln Glu Asp Tyr Asp Arg Leu Arg Pro Leu Ser Tyr
        75                  80                  85
cag aac acc cac ctc gtg ctc atc tgc tat gac gtc atg aat ccc acc     341
Gln Asn Thr His Leu Val Leu Ile Cys Tyr Asp Val Met Asn Pro Thr
    90                  95                  100
agc tac gac aac gtc ctc atc aag tgg ttc cct gag gtc acg cat ttc     389
Ser Tyr Asp Asn Val Leu Ile Lys Trp Phe Pro Glu Val Thr His Phe
105                 110                 115                 120
tgc cgc ggg atc ccc atg gtg ctc atc ggc tgc aag aca gac ctg agg     437
Cys Arg Gly Ile Pro Met Val Leu Ile Gly Cys Lys Thr Asp Leu Arg
                125                 130                 135
aag gac aag gag cag ctg cgg aag ctc cgg gcc gcc cag ctg gag ccc     485
Lys Asp Lys Glu Gln Leu Arg Lys Leu Arg Ala Ala Gln Leu Glu Pro
            140                 145                 150
atc acc tac atg cag ggc ctg agc gcc tgc gaa cag atc cga gct gct     533
Ile Thr Tyr Met Gln Gly Leu Ser Ala Cys Glu Gln Ile Arg Ala Ala
        155                 160                 165
ctc tac ctg gaa tgt tcc gcc aag ttt cgg gag aat gtg gag gac gtc     581
Leu Tyr Leu Glu Cys Ser Ala Lys Phe Arg Glu Asn Val Glu Asp Val
    170                 175                 180
ttc cgg gag gcc gcc aag gtg gct ctc agc gct ctg aag aag gcg caa     629
Phe Arg Glu Ala Ala Lys Val Ala Leu Ser Ala Leu Lys Lys Ala Gln
185                 190                 195                 200
cgg cag aag aag cgc cgg ctc tgc ctg ctg ctc tgacccaggg cagacagacc   682

Arg Gln Lys Lys Arg Arg Leu Cys Leu Leu Leu
                205                 210
tcacgacagc actgacaggg cccgggggcc caggtgccga ttgcaccagg gaggctgccc 742

catcccgacc ctccagctca tggtgtctgg ggcctgcggc tagactcttg gaacattctg 802

gaactctctc ctttcctggc tggggctctg accacaaact cccctccagg ctgcccctgg 862

gacatggtgg tgatgtgggt gcaggagcca gtgtctgttg tcgggactcg caagtgccct 922

catcacagcc acccccacca cgagtgtctc cccagtgcag actcaagtta tgcttgaaat 982

gaaaaagtct atctggtagt gggtaaaaaa aaaaa 1017

<210> 350
<211> 211
<212> PRT
<213> Homo sapiens

<400> 350
Met Asp Ala Pro Gly Ala Leu Ala Gln Thr Ala Ala Pro Gly Pro Gly
1               5                   10                  15
Arg Lys Glu Leu Lys Ile Val Ile Val Gly Asp Gly Gly Cys Gly Lys
            20                  25                  30
Thr Ser Leu Leu Met Val Tyr Ser Gln Gly Ser Phe Pro Glu His Tyr
        35                  40                  45
Ala Pro Ser Val Phe Glu Lys Tyr Thr Ala Ser Val Thr Val Gly Ser
    50                  55                  60
Lys Glu Val Thr Leu Asn Leu Tyr Asp Thr Ala Gly Gln Glu Asp Tyr
65                  70                  75                  80
Asp Arg Leu Arg Pro Leu Ser Tyr Gln Asn Thr His Leu Val Leu Ile
                85                  90                  95
Cys Tyr Asp Val Met Asn Pro Thr Ser Tyr Asp Asn Val Leu Ile Lys
            100                 105                 110
Trp Phe Pro Glu Val Thr His Phe Cys Arg Gly Ile Pro Met Val Leu
        115                 120                 125
Ile Gly Cys Lys Thr Asp Leu Arg Lys Asp Lys Glu Gln Leu Arg Lys
    130                 135                 140
Leu Arg Ala Ala Gln Leu Glu Pro Ile Thr Tyr Met Gln Gly Leu Ser
145                 150                 155                 160
Ala Cys Glu Gln Ile Arg Ala Ala Leu Tyr Leu Glu Cys Ser Ala Lys
                165                 170                 175
Phe Arg Glu Asn Val Glu Asp Val Phe Arg Glu Ala Ala Lys Val Ala
            180                 185                 190
Leu Ser Ala Leu Lys Lys Ala Gln Arg Gln Lys Lys Arg Arg Leu Cys
        195                 200                 205
Leu Leu Leu
    210

<210> 351
<211> 684
<212> DNA
<213> Mus musculus

<220>
<221> CDS
<222> (1)..(684)

<400> 351
atg gag ggg cag agt ggc cgc tgc aag atc gta gtg gtg ggg gac gcg 48

Met Glu Gly Gln Ser Gly Arg Cys Lys Ile Val Val Val Gly Asp Ala
1               5                   10                  15
gag tgc ggc aag aca gcg ttg ctg cag gtg ttc gcc aag gac gcc tac      96
Glu Cys Gly Lys Thr Ala Leu Leu Gln Val Phe Ala Lys Asp Ala Tyr
            20                  25                  30
ccc ggg agt tat gtc ccc acg gtg ttt gag aac tac act gcc agc ttc      144
Pro Gly Ser Tyr Val Pro Thr Val Phe Glu Asn Tyr Thr Ala Ser Phe
        35                  40                  45
gag atc gac aag cgc cgc att gag ctc aac atg tgg gat act tca ggt      192
Glu Ile Asp Lys Arg Arg Ile Glu Leu Asn Met Trp Asp Thr Ser Gly
    50                  55                  60
tcc tct tac tat gac aat gtc cgg cct ctg gcc tac ccg gat tct gat      240
Ser Ser Tyr Tyr Asp Asn Val Arg Pro Leu Ala Tyr Pro Asp Ser Asp
65                  70                  75                  80
gct gtg ctt atc tgc ttt gac att agc cgg cca gaa aca ctg gac agt      288
Ala Val Leu Ile Cys Phe Asp Ile Ser Arg Pro Glu Thr Leu Asp Ser
                85                  90                  95
gtc ctc aag aag tgg caa gga gag act cag gag ttt tgc ccc aat gcc      336
Val Leu Lys Lys Trp Gln Gly Glu Thr Gln Glu Phe Cys Pro Asn Ala
            100                 105                 110
aag gtg gtg ctg gtt ggc tgt aag ctg gac atg cgg act gac ctg gcc      384
Lys Val Val Leu Val Gly Cys Lys Leu Asp Met Arg Thr Asp Leu Ala
        115                 120                 125
aca ctg agg gag cta tcc aag cag aga ctc atc cct gtc aca cat gag      432
Thr Leu Arg Glu Leu Ser Lys Gln Arg Leu Ile Pro Val Thr His Glu
    130                 135                 140
cag ggt act gtg ctg gcc aag caa gtg gga gct gtg tcc tac gtt gaa      480
Gln Gly Thr Val Leu Ala Lys Gln Val Gly Ala Val Ser Tyr Val Glu
145                 150                 155                 160
tgt tcc tcc cga tct tct gag cgc agt gtc cgg gat ytc ttc cat gtg      528
Cys Ser Ser Arg Ser Ser Glu Arg Ser Val Arg Asp Val Phe His Val
                165                 170                 175
gcc aca gtg gct tct ctc ggc cgt ggc cat agg cag cta cgt cgt act      576
Ala Thr Val Ala Ser Leu Gly Arg Gly His Arg Gln Leu Arg Arg Thr
            180                 185                 190
gac tct cgc cgg gga ctg cag cga tcc act caa ctg tcg gga cgg cca      624
Asp Ser Arg Arg Gly Leu Gln Arg Ser Thr Gln Leu Ser Gly Arg Pro
        195                 200                 205
gac cgg gga aat gag ggc gag atg cat aag gat cga gcc aag agc tgt      672
Asp Arg Gly Asn Glu Gly Glu Met His Lys Asp Arg Ala Lys Ser Cys
    210                 215                 220
aac ctc atg tga                                                      684
Asn Leu Met
225

<210> 352
<211> 227
<212> PRT
<213> Mus musculus

<400> 352
Met Glu Gly Gln Ser Gly Arg Cys Lys Ile Val Val Val Gly Asp Ala
1               5                   10                  15
Glu Cys Gly Lys Thr Ala Leu Leu Gln Val Phe Ala Lys Asp Ala Tyr
            20                  25                  30
Pro Gly Ser Tyr Val Pro Thr Val Phe Glu Asn Tyr Thr Ala Ser Phe
        35                  40                  45
Glu Ile Asp Lys Arg Arg Ile Glu Leu Asn Met Trp Asp Thr Ser Gly
    50                  55                  60
Ser Ser Tyr Tyr Asp Asn Val Arg Pro Leu Ala Tyr Pro Asp Ser Asp
65                  70                  75                  80
Ala Val Leu Ile Cys Phe Asp Ile Ser Arg Pro Glu Thr Leu Asp Ser
                85                  90                  95
Val Leu Lys Lys Trp Gln Gly Glu Thr Gln Glu Phe Cys Pro Asn Ala
            100                 105                 110
Lys Val Val Leu Val Gly Cys Lys Leu Asp Met Arg Thr Asp Leu Ala
        115                 120                 125

Thr Leu Arg Glu Leu Ser Lys Gln Arg Leu Ile Pro Val Thr His Glu
    130                 135                 140
Gln Gly Thr Val Leu Ala Lys Gln Val Gly Ala Val Ser Tyr Val Glu
145                 150                 155                 160
Cys Ser Ser Arg Ser Ser Glu Arg Ser Val Arg Asp Val Phe His Val
                165                 170                 175
Ala Thr Val Ala Ser Leu Gly Arg Gly His Arg Gln Leu Arg Arg Thr
            180                 185                 190
Asp Ser Arg Arg Gly Leu Gln Arg Ser Thr Gln Leu Ser Gly Arg Pro
        195                 200                 205
Asp Arg Gly Asn Glu Gly Glu Met His Lys Asp Arg Ala Lys Ser Cys
    210                 215                 220
Asn Leu Met
225

<210> 353
<211> 576
<212> DNA
<213> Brachydanio rerio

<220>
<221> CDS
<222> (1)..(576)

<400> 353
atg cag acg att aag tgt gtg gtg gtg gga gac ggt gca gta ggc aag      48
Met Gln Thr Ile Lys Cys Val Val Val Gly Asp Gly Ala Val Gly Lys
1               5                   10                  15
aca tgc ctt ctt att tct tat acg aca aat gcc ttt cca gag gag tac      96
Thr Cys Leu Leu Ile Ser Tyr Thr Thr Asn Ala Phe Pro Glu Glu Tyr
            20                  25                  30
att ccc aca gtg ttt gac aac tac agt gct cag atg agt gta gat ggg      144
Ile Pro Thr Val Phe Asp Asn Tyr Ser Ala Gln Met Ser Val Asp Gly
        35                  40                  45
cgc act gtc agc ctc aac ctc tgg gac acg gca ggg cag gag gag tat      192
Arg Thr Val Ser Leu Asn Leu Trp Asp Thr Ala Gly Gln Glu Glu Tyr
    50                  55                  60
gac cgt ctg cgc acg ctt tcc tac cca caa act aat gtt ttc atc att      240
Asp Arg Leu Arg Thr Leu Ser Tyr Pro Gln Thr Asn Val Phe Ile Ile
65                  70                  75                  80
tgc ttt tcc atc gga agc cct tca tca tta gct aat gtt cgc cac aag      288
Cys Phe Ser Ile Gly Ser Pro Ser Ser Leu Ala Asn Val Arg His Lys
                85                  90                  95
tgg cac ccg gag gtg tct cac cac tgt ccc aac gtg cca att ctt cta      336
Trp His Pro Glu Val Ser His His Cys Pro Asn Val Pro Ile Leu Leu
            100                 105                 110
gtt ggc acc aag aag gat ctg cgt tca gat aca gag aca att aag aag      384
Val Gly Thr Lys Lys Asp Leu Arg Ser Asp Thr Glu Thr Ile Lys Lys
        115                 120                 125
ttg aag gag caa ggg ctt gca ccc tct act caa cag cag ggt ggc acc      432
Leu Lys Glu Gln Gly Leu Ala Pro Ser Thr Gln Gln Gln Gly Gly Thr
    130                 135                 140
ctg tgc aag cag atc aat gct gtg agg tat ctg gag tgc tcg gcg ctc      480
Leu Cys Lys Gln Ile Asn Ala Val Arg Tyr Leu Glu Cys Ser Ala Leu
145                 150                 155                 160
cgc cag gaa ggc gtg cgg gat gtg ttt gta gat gct gtt cgt gct gtg      528
Arg Gln Glu Gly Val Arg Asp Val Phe Val Asp Ala Val Arg Ala Val
                165                 170                 175
ctc tac ccc atg acc aaa aag aat acc aag aag tgt gtt ctc tta          573
Leu Tyr Pro Met Thr Lys Lys Asn Thr Lys Lys Cys Val Leu Leu
            180                 185                 190
tag                                                                  576

<210> 354
<211> 191

<212> PRT
<213> Brachydanio rerio

<400> 354
Met Gln Thr Ile Lys Cys Val Val Val Gly Asp Gly Ala Val Gly Lys
1               5                   10                  15
Thr Cys Leu Leu Ile Ser Tyr Thr Thr Asn Ala Phe Pro Glu Glu Tyr
            20                  25                  30
Ile Pro Thr Val Phe Asp Asn Tyr Ser Ala Gln Met Ser Val Asp Gly
        35                  40                  45
Arg Thr Val Ser Leu Asn Leu Trp Asp Thr Ala Gly Gln Glu Glu Tyr
    50                  55                  60
Asp Arg Leu Arg Thr Leu Ser Tyr Pro Gln Thr Asn Val Phe Ile Ile
65                  70                  75                  80
Cys Phe Ser Ile Gly Ser Pro Ser Ser Leu Ala Asn Val Arg His Lys
                85                  90                  95
Trp His Pro Glu Val Ser His His Cys Pro Asn Val Pro Ile Leu Leu
            100                 105                 110
Val Gly Thr Lys Lys Asp Leu Arg Ser Asp Thr Glu Thr Ile Lys Lys
        115                 120                 125
Leu Lys Glu Gln Gly Leu Ala Pro Ser Thr Gln Gln Gln Gly Gly Thr
    130                 135                 140
Leu Cys Lys Gln Ile Asn Ala Val Arg Tyr Leu Glu Cys Ser Ala Leu
145                 150                 155                 160
Arg Gln Glu Gly Val Arg Asp Val Phe Val Asp Ala Val Arg Ala Val
                165                 170                 175
Leu Tyr Pro Met Thr Lys Lys Asn Thr Lys Lys Cys Val Leu Leu
            180                 185                 190

<210> 355
<211> 576
<212> DNA
<213> Xenopus laevis

<220>
<221> CDS
<222> (1)..(576)

<400> 355
atg cag agc att aag tgt gtg gtg gtg ggc gac gga gca gtg gga aag      48
Met Gln Ser Ile Lys Cys Val Val Val Gly Asp Gly Ala Val Gly Lys
1               5                   10                  15
acc tgt tta ctc atc tgc ttc aca acc aac gcc ttc cct aag gaa tac      96
Thr Cys Leu Leu Ile Cys Phe Thr Thr Asn Ala Phe Pro Lys Glu Tyr
            20                  25                  30
atc ccc aca gtg ttt gat aac tac agc gcc cag acg gca gtt gac ggg      144
Ile Pro Thr Val Phe Asp Asn Tyr Ser Ala Gln Thr Ala Val Asp Gly
        35                  40                  45
agg aca gtt agc ttg aac ctg tgg gac act gct ggc cag gaa gag tat      192
Arg Thr Val Ser Leu Asn Leu Trp Asp Thr Ala Gly Gln Glu Glu Tyr
    50                  55                  60
gac aga ctg cgt aca ctt tct tac cca cag acc aac gtc ttc atc att      240
Asp Arg Leu Arg Thr Leu Ser Tyr Pro Gln Thr Asn Val Phe Ile Ile
65                  70                  75                  80
tgc ttt tcc atc gcc agc cca acc tcc tat gag aat gta aag cac aag      288
Cys Phe Ser Ile Ala Ser Pro Thr Ser Tyr Glu Asn Val Lys His Lys
                85                  90                  95
tgg tac cct gag gtg ggt cac cat tgt ccc aac gtg ccc att ctc ttg      336
Trp Tyr Pro Glu Val Gly His His Cys Pro Asn Val Pro Ile Leu Leu
            100                 105                 110
gtg ggc acc aag aag gat ctt agg aac aac gca gat gtc ata aag aag      384
Val Gly Thr Lys Lys Asp Leu Arg Asn Asn Ala Asp Val Ile Lys Lys
        115                 120                 125
ctg aaa gag cag aac caa atg ccc atc act aac cat cag ggt gga aat      432
Leu Lys Glu Gln Asn Gln Met Pro Ile Thr Asn His Gln Gly Gly Asn
    130                 135                 140
ctg gcc aag cag atc cat gca gta aag tat atg gag tgc tcg gcg ctg      480
Leu Ala Lys Gln Ile His Ala Val Lys Tyr Met Glu Cys Ser Ala Leu

145                 150                 155                 160
aat caa gat ggc atc aag gaa gtg ttt gcg gat gct gtg cga gct gtt      528
Asn Gln Asp Gly Ile Lys Glu Val Phe Ala Asp Ala Val Arg Ala Val
                165                 170                 175
ctc aac ccg act ccc atc aag gac aaa aag agc tgc ttc att ttg          573
Leu Asn Pro Thr Pro Ile Lys Asp Lys Lys Ser Cys Phe Ile Leu
            180                 185                 190
tga                                                                  576

<210> 356
<211> 191
<212> PRT
<213> Xenopus laevis

<400> 356
Met Gln Ser Ile Lys Cys Val Val Val Gly Asp Gly Ala Val Gly Lys
1               5                   10                  15
Thr Cys Leu Leu Ile Cys Phe Thr Thr Asn Ala Phe Pro Lys Glu Tyr
            20                  25                  30
Ile Pro Thr Val Phe Asp Asn Tyr Ser Ala Gln Thr Ala Val Asp Gly
        35                  40                  45
Arg Thr Val Ser Leu Asn Leu Trp Asp Thr Ala Gly Gln Glu Glu Tyr
    50                  55                  60
Asp Arg Leu Arg Thr Leu Ser Tyr Pro Gln Thr Asn Val Phe Ile Ile
65                  70                  75                  80
Cys Phe Ser Ile Ala Ser Pro Thr Ser Tyr Glu Asn Val Lys His Lys
                85                  90                  95
Trp Tyr Pro Glu Val Gly His His Cys Pro Asn Val Pro Ile Leu Leu
            100                 105                 110
Val Gly Thr Lys Lys Asp Leu Arg Asn Asn Ala Asp Val Ile Lys Lys
        115                 120                 125
Leu Lys Glu Gln Asn Gln Met Pro Ile Thr Asn His Gln Gly Gly Asn
    130                 135                 140
Leu Ala Lys Gln Ile His Ala Val Lys Tyr Met Glu Cys Ser Ala Leu
145                 150                 155                 160
Asn Gln Asp Gly Ile Lys Glu Val Phe Ala Asp Ala Val Arg Ala Val
                165                 170                 175
Leu Asn Pro Thr Pro Ile Lys Asp Lys Lys Ser Cys Phe Ile Leu
            180                 185                 190

<210> 357
<211> 591
<212> DNA
<213> Xenopus tropicalis

<220>
<221> CDS
<222> (1)..(591)

<400> 357
atg gcc gcg ata cgc aag aag ctg gtg gtg gtc ggg gac gga gct tgc      48
Met Ala Ala Ile Arg Lys Lys Leu Val Val Val Gly Asp Gly Ala Cys
1               5                   10                  15
ggc aaa acc tgc ctc ctc atc gtc ttc agc aag gac gag ttc ccc gag      96
Gly Lys Thr Cys Leu Leu Ile Val Phe Ser Lys Asp Glu Phe Pro Glu
            20                  25                  30
gtg tac gtt ccc acc gtg ttc gag aac tac gtg gcc gat atc gag gtg      144
Val Tyr Val Pro Thr Val Phe Glu Asn Tyr Val Ala Asp Ile Glu Val
        35                  40                  45
gac gtg aag cag gtg gag ctg gcc ctg tgg gac acg gcg ggg cag gag      192
Asp Val Lys Gln Val Glu Leu Ala Leu Trp Asp Thr Ala Gly Gln Glu
    50                  55                  60
gac tac gac cgg ctg cgg cct ctc tcc tac ccc gac acg gac gtc atc      240
Asp Tyr Asp Arg Leu Arg Pro Leu Ser Tyr Pro Asp Thr Asp Val Ile
65                  70                  75                  80

ctc atg tgc ttc tcc gtg gac agc ccg gac tcg ctg gag aac atc ccg          288
Leu Met Cys Phe Ser Val Asp Ser Pro Asp Ser Leu Glu Asn Ile Pro
                85                  90                  95
gag aag tgg gtg ccc gag gtg aag cac ttc tgc ccc agc gtc ccc atc          336
Glu Lys Trp Val Pro Glu Val Lys His Phe Cys Pro Ser Val Pro Ile
            100                 105                 110
atc ctg gtg gcc aac aag aag gac ctg agg aac gac gag cac gtc cgc          384
Ile Leu Val Ala Asn Lys Lys Asp Leu Arg Asn Asp Glu His Val Arg
        115                 120                 125
aac gag ctg gcc cgc atg aag cag gag ccg gtc cgc acc gag gac ggc          432
Asn Glu Leu Ala Arg Met Lys Gln Glu Pro Val Arg Thr Glu Asp Gly
    130                 135                 140
agg gcc atg gcc gtg agg att aac gcc ttc gag tac ctg gag tgc tcg          480
Arg Ala Met Ala Val Arg Ile Asn Ala Phe Glu Tyr Leu Glu Cys Ser
145                 150                 155                 160
gcc aag acc aag gaa ggg gtc cgg gag gtg ttc gaa acg gcc act agg          528
Ala Lys Thr Lys Glu Gly Val Arg Glu Val Phe Glu Thr Ala Thr Arg
                165                 170                 175
gcg gcg ctg cag aag aaa cac ggc cgg agc ggt gaa tgt atg agc tgc          576
Ala Ala Leu Gln Lys Lys His Gly Arg Ser Gly Glu Cys Met Ser Cys
            180                 185                 190
tgt aag ctc ctc tga                                                      591
Cys Lys Leu Leu
        195


<210> 358
<211> 196
<212> PRT
<213> Xenopus tropicalis

<400> 358
Met Ala Ala Ile Arg Lys Lys Leu Val Val Val Gly Asp Gly Ala Cys
1               5                   10                  15
Gly Lys Thr Cys Leu Leu Ile Val Phe Ser Lys Asp Glu Phe Pro Glu
            20                  25                  30
Val Tyr Val Pro Thr Val Phe Glu Asn Tyr Val Ala Asp Ile Glu Val
        35                  40                  45
Asp Val Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Val Asp Ser Pro Asp Ser Leu Glu Asn Ile Pro
                85                  90                  95
Glu Lys Trp Val Pro Glu Val Lys His Phe Cys Pro Ser Val Pro Ile
            100                 105                 110
Ile Leu Val Ala Asn Lys Lys Asp Leu Arg Asn Asp Glu His Val Arg
        115                 120                 125
Asn Glu Leu Ala Arg Met Lys Gln Glu Pro Val Arg Thr Glu Asp Gly
    130                 135                 140
Arg Ala Met Ala Val Arg Ile Asn Ala Phe Glu Tyr Leu Glu Cys Ser
145                 150                 155                 160
Ala Lys Thr Lys Glu Gly Val Arg Glu Val Phe Glu Thr Ala Thr Arg
                165                 170                 175
Ala Ala Leu Gln Lys Lys His Gly Arg Ser Gly Glu Cys Met Ser Cys
            180                 185                 190
Cys Lys Leu Leu
        195

<210> 359
<211> 735
<212> DNA
<213> Xenopus laevis

<220>
<221> CDS
<222> (1)..(735)

<400> 359
atg aag gaa aga aga acg agt cag aaa ctg gcc agc aaa tca atg atg      48
Met Lys Glu Arg Arg Thr Ser Gln Lys Leu Ala Ser Lys Ser Met Met
1               5                   10                  15
gat ccc aac cag aat gtg aaa tgt aaa att gtg gtg gtg ggg gac agc      96
Asp Pro Asn Gln Asn Val Lys Cys Lys Ile Val Val Val Gly Asp Ser
            20                  25                  30
cag tgt ggc aaa act gcc ctg ctc cat gtc ttt gcc aag gac tcc ttc      144
Gln Cys Gly Lys Thr Ala Leu Leu His Val Phe Ala Lys Asp Ser Phe
        35                  40                  45
cca gag aac tat gtc ccc acc gta ttt gag aat tac acg gcc agt ttt      192
Pro Glu Asn Tyr Val Pro Thr Val Phe Glu Asn Tyr Thr Ala Ser Phe
    50                  55                  60
gaa att gac acg cag agg ata gaa ctg agt ctc tgg gat aca tct ggt      240
Glu Ile Asp Thr Gln Arg Ile Glu Leu Ser Leu Trp Asp Thr Ser Gly
65                  70                  75                  80
tcc cct tat tat gac aac gtc cga cct ctt agc tat cca gac tct gac      288
Ser Pro Tyr Tyr Asp Asn Val Arg Pro Leu Ser Tyr Pro Asp Ser Asp
                85                  90                  95
gcc gtt ttg ata tgt ttc gat atc agt cgc ccc gag acg ctt gac agt      336
Ala Val Leu Ile Cys Phe Asp Ile Ser Arg Pro Glu Thr Leu Asp Ser
            100                 105                 110
gtt ctt aaa aag tgg aaa ggt gaa att caa gaa ttc tgc ccc aat acc      384
Val Leu Lys Lys Trp Lys Gly Glu Ile Gln Glu Phe Cys Pro Asn Thr
        115                 120                 125
aaa atg ctg ctg gtt ggc tgc aaa tcc gac ctc cgt aca gat cta acc      432
Lys Met Leu Leu Val Gly Cys Lys Ser Asp Leu Arg Thr Asp Leu Thr
    130                 135                 140
aca tta gta gaa cta tct aac cac aga cag acg cca gtc tcc tat gat      480
Thr Leu Val Glu Leu Ser Asn His Arg Gln Thr Pro Val Ser Tyr Asp
145                 150                 155                 160
cag ggg gca aac atg gcc aag cag ata gga gca gcc acg tac atc gaa      528
Gln Gly Ala Asn Met Ala Lys Gln Ile Gly Ala Ala Thr Tyr Ile Glu
                165                 170                 175
tgc tct gcg tta cag tct gag aac agt gtc aga gac att ttc cat gta      576
Cys Ser Ala Leu Gln Ser Glu Asn Ser Val Arg Asp Ile Phe His Val
            180                 185                 190
gcc acc ctg gca tgt gtg aat aag act aac aaa aat ctt aaa agg aat      624
Ala Thr Leu Ala Cys Val Asn Lys Thr Asn Lys Asn Leu Lys Arg Asn
        195                 200                 205
aaa act caa aga gcc aca aag aga atc tcg cac atg ccg agt cgg cca      672
Lys Thr Gln Arg Ala Thr Lys Arg Ile Ser His Met Pro Ser Arg Pro
    210                 215                 220
gaa cta agt gct gtg gcc aca gac ttg agg aag gat aaa gca aag agc      720
Glu Leu Ser Ala Val Ala Thr Asp Leu Arg Lys Asp Lys Ala Lys Ser
225                 230                 235                 240
tgc agc atc atg tga                                                  735
Cys Ser Ile Met

<210> 360
<211> 244
<212> PRT
<213> Xenopus laevis

<400> 360
Met Lys Glu Arg Arg Thr Ser Gln Lys Leu Ala Ser Lys Ser Met Met
1               5                   10                  15
Asp Pro Asn Gln Asn Val Lys Cys Lys Ile Val Val Val Gly Asp Ser
            20                  25                  30
Gln Cys Gly Lys Thr Ala Leu Leu His Val Phe Ala Lys Asp Ser Phe
        35                  40                  45
Pro Glu Asn Tyr Val Pro Thr Val Phe Glu Asn Tyr Thr Ala Ser Phe
    50                  55                  60
Glu Ile Asp Thr Gln Arg Ile Glu Leu Ser Leu Trp Asp Thr Ser Gly
65                  70                  75                  80
Ser Pro Tyr Tyr Asp Asn Val Arg Pro Leu Ser Tyr Pro Asp Ser Asp

                85                  90                  95
Ala Val Leu Ile Cys Phe Asp Ile Ser Arg Pro Glu Thr Leu Asp Ser
            100                 105                 110
Val Leu Lys Lys Trp Lys Gly Glu Ile Gln Glu Phe Cys Pro Asn Thr
        115                 120                 125
Lys Met Leu Leu Val Gly Cys Lys Ser Asp Leu Arg Thr Asp Leu Thr
    130                 135                 140
Thr Leu Val Glu Leu Ser Asn His Arg Gln Thr Pro Val Ser Tyr Asp
145                 150                 155                 160
Gln Gly Ala Asn Met Ala Lys Gln Ile Gly Ala Ala Thr Tyr Ile Glu
                165                 170                 175
Cys Ser Ala Leu Gln Ser Glu Asn Ser Val Arg Asp Ile Phe His Val
            180                 185                 190
Ala Thr Leu Ala Cys Val Asn Lys Thr Asn Lys Asn Leu Lys Arg Asn
        195                 200                 205
Lys Thr Gln Arg Ala Thr Lys Arg Ile Ser His Met Pro Ser Arg Pro
    210                 215                 220
Glu Leu Ser Ala Val Ala Thr Asp Leu Arg Lys Asp Lys Ala Lys Ser
225                 230                 235                 240
Cys Ser Ile Met


<210> 361
<211> 1035
<212> DNA
<213> Pagrus major

<220>
<221> CDS
<222> (25)..(603)

<400> 361
cggcacgagg cgcagcggaa cagg atg cag gct atc aaa tgt gtg gtc gtg      51
                           Met Gln Ala Ile Lys Cys Val Val Val
                           1               5
gga gat gga gct gtg gga aaa aca tgt ctt ctc atc agc tac aca acc      99
Gly Asp Gly Ala Val Gly Lys Thr Cys Leu Leu Ile Ser Tyr Thr Thr
10                  15                  20                  25
aat gcc ttc ccc ggg gag tac att cct act gta ttt gac aac tac tca      147
Asn Ala Phe Pro Gly Glu Tyr Ile Pro Thr Val Phe Asp Asn Tyr Ser
                30                  35                  40
gct aat gtg atg gtg gac agc aag cca gtc aac ctg ggc ctc tgg gat      195
Ala Asn Val Met Val Asp Ser Lys Pro Val Asn Leu Gly Leu Trp Asp
            45                  50                  55
aca gct gga cag gaa gac tac gac agg ctc cgc cca ctg tcc tac cca      243
Thr Ala Gly Gln Glu Asp Tyr Asp Arg Leu Arg Pro Leu Ser Tyr Pro
        60                  65                  70
cag acg gat gtt ttc ctg atc tgt ttc tcc ctg gtg agc cca gcg tcc      291
Gln Thr Asp Val Phe Leu Ile Cys Phe Ser Leu Val Ser Pro Ala Ser
    75                  80                  85
tat gag aac gtc aga gct aag tgg tac cca gag gtt cgc cac cac tgc      339
Tyr Glu Asn Val Arg Ala Lys Trp Tyr Pro Glu Val Arg His His Cys
90                  95                  100                 105
ccc tcc aca cct atc atc ctg gtg ggc acc aag ctg gat ctg agg gac      387
Pro Ser Thr Pro Ile Ile Leu Val Gly Thr Lys Leu Asp Leu Arg Asp
                110                 115                 120
gag aag gaa acc att gag aag ctg aag gaa aaa aag ctg gcg ccg atc      435
Glu Lys Glu Thr Ile Glu Lys Leu Lys Glu Lys Lys Leu Ala Pro Ile
            125                 130                 135
acc tac ccc cag ggc ctg gct ctg gct aag gag atc gat gca gtg aag      483
Thr Tyr Pro Gln Gly Leu Ala Leu Ala Lys Glu Ile Asp Ala Val Lys
        140                 145                 150
tac ctg gag tgc tca gcc ttg acc cag cgt ggc ctg aag acc gtg ttc      531
Tyr Leu Glu Cys Ser Ala Leu Thr Gln Arg Gly Leu Lys Thr Val Phe
    155                 160                 165
gac gag gcc atc cgg gcc gtg ctc tgc cct cag ccc acc aag gtc aag      579
Asp Glu Ala Ile Arg Ala Val Leu Cys Pro Gln Pro Thr Lys Val Lys
170                 175                 180                 185

aag aag ccg tgc tca ctg ctg taaatgattc ttgcaagacg aagacgacga 630
Lys Lys Pro Cys Ser Leu Leu
                190
cgatgagaaa gcgtttgcga ctgatgaaaa ggtgaaaaga agacgatggg aaagttctgt 690

tatgtttaat ggtttgtgat agaagactgc gactagattt ccactaggtt agaaaaaaat 750

aatgcttgtg cggtgatgat aaatggtaaa tggtagcaaa ccctttctgt acagcatact 810

ctgagactga ttattcaagt gcattataga ccaggcagac atttcactgt aaaattgaac 870

aaagtatatc actagatgtg gaggctgaca cactgtacat tcatcctgtg ggcttaaatg 930

gtcagatcaa gagcagcaaa gcacctgcac aacggactct gctgtccctt ttggggctga 990

cagtatgtac acaggtgtct tgtgcggctt cacataatga taata 1035

<210> 362
<211> 192
<212> PRT
<213> Pagrus major

<400> 362
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Ser
        35                  40                  45
Lys Pro Val Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Ser Pro Ala Ser Tyr Glu Asn Val Arg Ala Lys
                85                  90                  95
Trp Tyr Pro Glu Val Arg His His Cys Pro Ser Thr Pro Ile Ile Leu
            100                 105                 110
Val Gly Thr Lys Leu Asp Leu Arg Asp Glu Lys Glu Thr Ile Glu Lys
        115                 120                 125
Leu Lys Glu Lys Lys Leu Ala Pro Ile Thr Tyr Pro Gln Gly Leu Ala
    130                 135                 140
Leu Ala Lys Glu Ile Asp Ala Val Lys Tyr Leu Glu Cys Ser Ala Leu
145                 150                 155                 160
Thr Gln Arg Gly Leu Lys Thr Val Phe Asp Glu Ala Ile Arg Ala Val
                165                 170                 175
Leu Cys Pro Gln Pro Thr Lys Val Lys Lys Lys Pro Cys Ser Leu Leu
            180                 185                 190

<210> 363
<211> 1090
<212> DNA
<213> Schizophyllum commune

<220>
<221> CDS
<222> (145)..(735)

<400> 363
gctgctcgcc ttgttcttcc attgtccatc cccttgcccg tgctcccgcg gtcgctgaac 60

gtacccaagc cctcgcctcc ccgtcgcgtc gtctgccttc ttcccctcgg tttccgatag 120

tcgctccgtt gcccagtccc cagt atg cag gcc atc aag tgt gtt gtc gta 171
Met Gln Ala Ile Lys Cys Val Val Val
1               5
gga gat ggt gcg gtc gga aag acc tgc ctg cta atc tcg tat acc acg 219
Gly Asp Gly Ala Val Gly Lys Thr Cys Leu Leu Ile Ser Tyr Thr Thr
10                  15                  20                  25
aac gcg ttc ccg gga gaa tat atc ccg acc gtg ttc gat aac tac tcc 267
Asn Ala Phe Pro Gly Glu Tyr Ile Pro Thr Val Phe Asp Asn Tyr Ser
                30                  35                  40
gcc aat gtc atg gtc gac ggc aag act atc tcc ctc ggg ctt tgg gat 315
Ala Asn Val Met Val Asp Gly Lys Thr Ile Ser Leu Gly Leu Trp Asp
            45                  50                  55
acc gct ggt caa gaa gat tac gac cgt ctc cgc ccg ctc tcc tac cct 363
Thr Ala Gly Gln Glu Asp Tyr Asp Arg Leu Arg Pro Leu Ser Tyr Pro
        60                  65                  70
cag acg gat gtc ttc ttg att tgt ttc tcg ctc gtc agc ccg cca agt 411
Gln Thr Asp Val Phe Leu Ile Cys Phe Ser Leu Val Ser Pro Pro Ser
    75                  80                  85
ttc gag aac gtc cgg acc aag tgg tac cct gaa ata tcg cat cac gca 459
Phe Glu Asn Val Arg Thr Lys Trp Tyr Pro Glu Ile Ser His His Ala
90                  95                  100                 105
ccg cag acg ccc gtc gtg ctc gtg ggc acc aag ctg gat ttg cga gag 507
Pro Gln Thr Pro Val Val Leu Val Gly Thr Lys Leu Asp Leu Arg Glu
                110                 115                 120
gac cct gcg acg ata gag aaa ctg cgt gac cgc cgc atg tcc ccc atc 555
Asp Pro Ala Thr Ile Glu Lys Leu Arg Asp Arg Arg Met Ser Pro Ile
            125                 130                 135
cag tac tcg cag ggt gtc gcg atg atg aag gac atc ggt gct gtg aag 603
Gln Tyr Ser Gln Gly Val Ala Met Met Lys Asp Ile Gly Ala Val Lys
        140                 145                 150
tac cta gag tgt tcc gcg ctg acg caa aag ggg ctc aag acc gtg ttt 651
Tyr Leu Glu Cys Ser Ala Leu Thr Gln Lys Gly Leu Lys Thr Val Phe
    155                 160                 165
gac gag gcg atc cgt gtt gtc ttg tac ccg tcc gcg cgg tcc gac aac 699
Asp Glu Ala Ile Arg Val Val Leu Tyr Pro Ser Ala Arg Ser Asp Asn
170                 175                 180                 185
aaa cgc agc aag ggc cgc tca tgc att gtc gca taagtggact ccagcgccgc 752
Lys Arg Ser Lys Gly Arg Ser Cys Ile Val Ala
                190                 195
tgtatgggtg gacgaaccac aggaagtgtt gtgcgcactg tatcatcacg cggcgcgcgg 812

cgctccctgc actatatcac gccatttatg tctttcttct agcactatct cctttttcgc 872

gccagctgct gatatccgca cggtttcctg ccaacacata ccagtggaac tcgaggatcc 932

tgaccatttt gctactcgtc acgaccaccc ttgcctcaca ccccctttgt gttgactctc 992

aggttctgta attcgacgac ctgctcttcg aatttcgctc ccctttgccc acatagcaca 1052

tatagtatca ccgtgacgaa ctgtatcctc cttgacga 1090

<210> 364
<211> 196
<212> PRT
<213> Schizophyllum commune

<400> 364
Met Gln Ala Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly
        35                  40                  45
Lys Thr Ile Ser Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Leu Val Ser Pro Pro Ser Phe Glu Asn Val Arg Thr Lys
                85                  90                  95
Trp Tyr Pro Glu Ile Ser His His Ala Pro Gln Thr Pro Val Val Leu
            100                 105                 110
Val Gly Thr Lys Leu Asp Leu Arg Glu Asp Pro Ala Thr Ile Glu Lys
        115                 120                 125
Leu Arg Asp Arg Arg Met Ser Pro Ile Gln Tyr Ser Gln Gly Val Ala
    130                 135                 140
Met Met Lys Asp Ile Gly Ala Val Lys Tyr Leu Glu Cys Ser Ala Leu
145                 150                 155                 160
Thr Gln Lys Gly Leu Lys Thr Val Phe Asp Glu Ala Ile Arg Val Val
                165                 170                 175
Leu Tyr Pro Ser Ala Arg Ser Asp Asn Lys Arg Ser Lys Gly Arg Ser
            180                 185                 190
Cys Ile Val Ala
        195

<210> 365
<211> 576
<212> DNA
<213> Paracoccidioides brasiliensis

<220>
<221> CDS
<222> (1)..(576)

<400> 365
atg gct gag att cgt cgt aaa ctt gtc att gtt ggt gat ggt gcc tgt      48
Met Ala Glu Ile Arg Arg Lys Leu Val Ile Val Gly Asp Gly Ala Cys
1               5                   10                  15
ggt aaa act tgt ctc ttg att gtc ttt tcc aag ggt acc ttc cct gag      96
Gly Lys Thr Cys Leu Leu Ile Val Phe Ser Lys Gly Thr Phe Pro Glu
            20                  25                  30
gtc tac gtc cca acc gtc ttc gag aac tat gtg gcc gac gtt gag gtc      144
Val Tyr Val Pro Thr Val Phe Glu Asn Tyr Val Ala Asp Val Glu Val
        35                  40                  45
gat gga aag cat gtc gag ctc gca ctt tgg gat acg gct ggc cag gaa      192
Asp Gly Lys His Val Glu Leu Ala Leu Trp Asp Thr Ala Gly Gln Glu
    50                  55                  60
gat tac gat cga ctc aga cct ctt tcc tac cct gat tca cat gtt att      240
Asp Tyr Asp Arg Leu Arg Pro Leu Ser Tyr Pro Asp Ser His Val Ile
65                  70                  75                  80
ctg atc tgt ttc gct atc gat tcc ccc gac tct ctc gac aac gtc cag      288
Leu Ile Cys Phe Ala Ile Asp Ser Pro Asp Ser Leu Asp Asn Val Gln
                85                  90                  95
gag aag tgg att tct gaa gtc ctt cat ttc tgc cag ggt cat ccc att      336
Glu Lys Trp Ile Ser Glu Val Leu His Phe Cys Gln Gly His Pro Ile
            100                 105                 110
atc ctc gtt ggt tgc aag aaa gat ctt cgt gac gac ccc aga acg att      384
Ile Leu Val Gly Cys Lys Lys Asp Leu Arg Asp Asp Pro Arg Thr Ile
        115                 120                 125
gag gag ctg cgc aag acg tct cag aag ccc gtg acc acc gaa cag ggt      432
Glu Glu Leu Arg Lys Thr Ser Gln Lys Pro Val Thr Thr Glu Gln Gly
    130                 135                 140
gag gag gtc cgc aag aag att ggc gct tac aag tat ctg gaa tgc tcc      480
Glu Glu Val Arg Lys Lys Ile Gly Ala Tyr Lys Tyr Leu Glu Cys Ser

145                 150                 155                 160
gcc cga aca aac gac gga gtt cgt gag gtg ttc gag tca gct act cga       528
Ala Arg Thr Asn Asp Gly Val Arg Glu Val Phe Glu Ser Ala Thr Arg
                165                 170                 175
gct gca ctg ctg gcg aag aag gag aaa aag aaa tgc aag atc ttg           573
Ala Ala Leu Leu Ala Lys Lys Glu Lys Lys Lys Cys Lys Ile Leu
            180                 185                 190
taa                                                                   576

<210> 366
<211> 191
<212> PRT
<213> Paracoccidioides brasiliensis

<400> 366
Met Ala Glu Ile Arg Arg Lys Leu Val Ile Val Gly Asp Gly Ala Cys
1               5                   10                  15
Gly Lys Thr Cys Leu Leu Ile Val Phe Ser Lys Gly Thr Phe Pro Glu
            20                  25                  30
Val Tyr Val Pro Thr Val Phe Glu Asn Tyr Val Ala Asp Val Glu Val
        35                  40                  45
Asp Gly Lys His Val Glu Leu Ala Leu Trp Asp Thr Ala Gly Gln Glu
    50                  55                  60
Asp Tyr Asp Arg Leu Arg Pro Leu Ser Tyr Pro Asp Ser His Val Ile
65                  70                  75                  80
Leu Ile Cys Phe Ala Ile Asp Ser Pro Asp Ser Leu Asp Asn Val Gln
                85                  90                  95
Glu Lys Trp Ile Ser Glu Val Leu His Phe Cys Gln Gly His Pro Ile
            100                 105                 110
Ile Leu Val Gly Cys Lys Lys Asp Leu Arg Asp Asp Pro Arg Thr Ile
        115                 120                 125
Glu Glu Leu Arg Lys Thr Ser Gln Lys Pro Val Thr Thr Glu Gln Gly
    130                 135                 140
Glu Glu Val Arg Lys Lys Ile Gly Ala Tyr Lys Tyr Leu Glu Cys Ser
145                 150                 155                 160
Ala Arg Thr Asn Asp Gly Val Arg Glu Val Phe Glu Ser Ala Thr Arg
                165                 170                 175
Ala Ala Leu Leu Ala Lys Lys Glu Lys Lys Lys Cys Lys Ile Leu
            180                 185                 190

<210> 367
<211> 591
<212> DNA
<213> Fucus distichus

<220>
<221> CDS
<222> (1)..(591)

<400> 367
atg cag aac ata aag tgc gtc gtc gtg ggt gac ggt gcg gtt ggt aaa       48
Met Gln Asn Ile Lys Cys Val Val Val Gly Asp Gly Ala Val Gly Lys
1               5                   10                  15
acc tgc ctc ctc atc tcc tac acg acg aac gcg ttt cct ggg gaa tac       96
Thr Cys Leu Leu Ile Ser Tyr Thr Thr Asn Ala Phe Pro Gly Glu Tyr
            20                  25                  30
atc cca act gtc ttc gac aac tat agc gct aat gta atg gtc gac gga       144
Ile Pro Thr Val Phe Asp Asn Tyr Ser Ala Asn Val Met Val Asp Gly
        35                  40                  45
aag ccg atc aac ctg ggc ctt tgg gat act gca ggg cag gaa gat tac       192
Lys Pro Ile Asn Leu Gly Leu Trp Asp Thr Ala Gly Gln Glu Asp Tyr
    50                  55                  60
gat cga ctc cgg cca ctg agc tac ccg caa acg gac gta ttt ctt gtg       240
Asp Arg Leu Arg Pro Leu Ser Tyr Pro Gln Thr Asp Val Phe Leu Val
65                  70                  75                  80

tgc ttc agc gtc gtt gac ccc acc agc ttt cac aac gtg aag ctc aag 288
Cys Phe Ser Val Val Asp Pro Thr Ser Phe His Asn Val Lys Leu Lys
                85                  90                  95
tgg ata ccc gag ctg caa cac cat gct ccg ggc atc cca ttt ata ctg 336
Trp Ile Pro Glu Leu Gln His His Ala Pro Gly Ile Pro Phe Ile Leu
            100                 105                 110
gtt ggt acg aag ctc gac ctg agg gat gat caa gac gcg atc aag cgt 384
Val Gly Thr Lys Leu Asp Leu Arg Asp Asp Gln Asp Ala Ile Lys Arg
        115                 120                 125
cta gca gaa cgt cgg cag acg ccc atc agc ttc agc gag gcg cag ggt 432
Leu Ala Glu Arg Arg Gln Thr Pro Ile Ser Phe Ser Glu Ala Gln Gly
    130                 135                 140
ctg tcg tct gac ctt gaa gct tac cgc tac ctt gag tgc agc gcg ctg 480
Leu Ser Ser Asp Leu Glu Ala Tyr Arg Tyr Leu Glu Cys Ser Ala Leu
145                 150                 155                 160
acg caa cac ggg tta aaa cag gtg ttt gac ggg gct atc cgg tgt gtt 528
Thr Gln His Gly Leu Lys Gln Val Phe Asp Gly Ala Ile Arg Cys Val
                165                 170                 175
cta gaa cag aac cag aga aag atg aaa aag aaa aag ggc aag aaa ggc 576
Leu Glu Gln Asn Gln Arg Lys Met Lys Lys Lys Lys Gly Lys Lys Gly
            180                 185                 190
tgc gtc atc tct tga 591
Cys Val Ile Ser
        195

<210> 368
<211> 196
<212> PRT
<213> Fucus distichus

<400> 368
Met Gln Asn Ile Lys Cys Val Val Val Gly Asp Gly Ala Val Gly Lys
1               5                   10                  15
Thr Cys Leu Leu Ile Ser Tyr Thr Thr Asn Ala Phe Pro Gly Glu Tyr
            20                  25                  30
Ile Pro Thr Val Phe Asp Asn Tyr Ser Ala Asn Val Met Val Asp Gly
        35                  40                  45
Lys Pro Ile Asn Leu Gly Leu Trp Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Val Asp Pro Thr Ser Phe His Asn Val Lys Leu Lys
                85                  90                  95
Trp Ile Pro Glu Leu Gln His His Ala Pro Gly Ile Pro Phe Ile Leu
            100                 105                 110
Val Gly Thr Lys Leu Asp Leu Arg Asp Asp Gln Asp Ala Ile Lys Arg
        115                 120                 125
Leu Ala Glu Arg Arg Gln Thr Pro Ile Ser Phe Ser Glu Ala Gln Gly
    130                 135                 140
Leu Ser Ser Asp Leu Glu Ala Tyr Arg Tyr Leu Glu Cys Ser Ala Leu
145                 150                 155                 160
Thr Gln His Gly Leu Lys Gln Val Phe Asp Gly Ala Ile Arg Cys Val
                165                 170                 175
Leu Glu Gln Asn Gln Arg Lys Met Lys Lys Lys Lys Gly Lys Lys Gly
            180                 185                 190
Cys Val Ile Ser
        195

<210> 369
<211> 636
<212> DNA
<213> Homo sapiens

<220>
<221> CDS
<222> (1)..(636)

<400> 369
atg cag gcc atc aag tgt gtg gtg gtg gga gac gga gct gta ggt aaa     48
act tgc cta ctg atc agt tac aca acc aat gca ttt cct gga gaa tat     96
atc cct act gtc ttt gac aat tat tct gcc aat gtt atg gta gat gga     144
aaa ccg gtg aat ctg ggc tta tgg gat aca gct gga caa gaa gat tat     192
gac aga tta cgc ccc cta tcc tat ccg caa aca gtt gga gaa acg tac     240
ggt aag gat ata acc tcc cgg ggc aaa gac aag ccg att gcc gat gtg     288
ttc tta att tgc ttt tcc ctt gtg agt cct gca tca ttt gaa aat gtc     336
cgt gca aag tgg tat cct gag gtg cgg cac cac tgt ccc aac act ccc     384
atc atc cta gtg gga act aaa ctt gat ctt agg gat gat aaa gac acg     432
atc gag aaa ctg aag gag aag aag ctg act ccc atc acc tat ccg cag     480
ggt cta gcc atg gct aag gag att ggt gct gta aaa tac ctg gag tgc     528
tcg gcg ctc aca cag cga ggc ctc aag aca gtg ttt gac gaa gcg atc     576
cga gca gtc ctc tgc ccg cct ccc gtg aag aag agg aag aga aaa tgc     624
ctg ctg ttg taa                                                     636

<210> 370
<211> 211
<212> PRT
<213> Homo sapiens

<400> 370

<210> 371
<211> 576
<212> DNA
<213> Pneumocystis carinii

<220>
<221> CDS
<222> (1)..(576)

<400> 371
atg caa act att aaa tgt gtc gtt gtt ggg gat ggc gcg gtt ggt aaa      48
Met Gln Thr Ile Lys Cys Val Val Val Gly Asp Gly Ala Val Gly Lys
1               5                   10                  15
acg tgt ctt tta ata tcc tat acg aca aac aaa ttt cct tca gaa tat      96
Thr Cys Leu Leu Ile Ser Tyr Thr Thr Asn Lys Phe Pro Ser Glu Tyr
            20                  25                  30
gtt cct act gta ttt gat aat tat gcg gtt acc gta atg att gga gaa      144
Val Pro Thr Val Phe Asp Asn Tyr Ala Val Thr Val Met Ile Gly Glu
        35                  40                  45
gaa cct tat act tta gga ctt ttt gat aca gca ggt caa gaa gat tat      192
Glu Pro Tyr Thr Leu Gly Leu Phe Asp Thr Ala Gly Gln Glu Asp Tyr
    50                  55                  60
gac aga ttg cgt ccc tta tca tat cca caa acg gat gtt ttt ctt att      240
Asp Arg Leu Arg Pro Leu Ser Tyr Pro Gln Thr Asp Val Phe Leu Ile
65                  70                  75                  80
tgc ttt tct gtt act agt cca gca agt ttt gaa aat gta aga gaa aag      288
Cys Phe Ser Val Thr Ser Pro Ala Ser Phe Glu Asn Val Arg Glu Lys
                85                  90                  95
tgg cat cca gag gtt cgt cat cat tgt cca gga aca ccc tgt ctt att      336
Trp His Pro Glu Val Arg His His Cys Pro Gly Thr Pro Cys Leu Ile
            100                 105                 110
gtt ggt aca caa atc gat tta cga gat gat cct atg att gta gag aaa      384
Val Gly Thr Gln Ile Asp Leu Arg Asp Asp Pro Met Ile Val Glu Lys
        115                 120                 125
ctc agt cga caa aga caa acc ccc att aca aaa gaa tta ggt gaa aaa      432
Leu Ser Arg Gln Arg Gln Thr Pro Ile Thr Lys Glu Leu Gly Glu Lys
    130                 135                 140
ctt tca aaa gaa ttg ggt gct gta aaa tat gtt gag tgc tca gct ttg      480
Leu Ser Lys Glu Leu Gly Ala Val Lys Tyr Val Glu Cys Ser Ala Leu
145                 150                 155                 160
act caa aaa gga tta aaa aac gtt ttt gat gaa gct ata gtt tgc gca      528
Thr Gln Lys Gly Leu Lys Asn Val Phe Asp Glu Ala Ile Val Cys Ala
                165                 170                 175
ctt gaa cca ccc gtt acg aag aag aaa act aaa tgt ctt att tta          573
Leu Glu Pro Pro Val Thr Lys Lys Lys Thr Lys Cys Leu Ile Leu
            180                 185                 190
taa                                                                  576

<210> 372
<211> 191
<212> PRT
<213> Pneumocystis carinii

<400> 372
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Glu Tyr
            20                  25                  30
Val Pro Thr Val Phe Asp Asn Tyr Ala Val Thr Val Met Ile Gly Glu
        35                  40                  45
Glu Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Ile
65                  70                  75                  80
Cys Phe Ser Val Thr Ser Pro Ala Ser Phe Glu Asn Val Arg Glu Lys
                85                  90                  95
Trp His Pro Glu Val Arg His His Cys Pro Gly Thr Pro Cys Leu Ile
            100                 105                 110
Val Gly Thr Gln Ile Asp Leu Arg Asp Asp Pro Met Ile Val Glu Lys
        115                 120                 125
Leu Ser Arg Gln Arg Gln Thr Pro Ile Thr Lys Glu Leu Gly Glu Lys
    130                 135                 140
Leu Ser Lys Glu Leu Gly Ala Val Lys Tyr Val Glu Cys Ser Ala Leu
145                 150                 155                 160
Thr Gln Lys Gly Leu Lys Asn Val Phe Asp Glu Ala Ile Val Cys Ala
                165                 170                 175
Leu Glu Pro Pro Val Thr Lys Lys Lys Thr Lys Cys Leu Ile Leu
            180                 185                 190

<210> 373
<211> 630
<212> DNA
<213> Ashbya gossypii

<220>
<221> CDS
<222> (1)..(630)

<400> 373
atg gcg tac cag aca ggc ggc aac ata cgc aag aag ttg gtt att gtc      48
Met Ala Tyr Gln Thr Gly Gly Asn Ile Arg Lys Lys Leu Val Ile Val
1               5                   10                  15
ggc gac ggg gcg tgt ggg aag acg tgt ctg ctc gtt gtg ttc tcc aaa      96
Gly Asp Gly Ala Cys Gly Lys Thr Cys Leu Leu Val Val Phe Ser Lys
            20                  25                  30
ggg cag ttc ccg gag atc cat gtg ccc acg gtg ttc gag aac tac gtg      144
Gly Gln Phe Pro Glu Ile His Val Pro Thr Val Phe Glu Asn Tyr Val
        35                  40                  45
gca gat gtg gac atc gac ggg cga cgc gta gag ctg gca ctg tgg gat      192
Ala Asp Val Asp Ile Asp Gly Arg Arg Val Glu Leu Ala Leu Trp Asp
    50                  55                  60
aca gcg ggc cag gag gac tac gac cgg ctg cgg cca ttg tcg tac ccg      240
Thr Ala Gly Gln Glu Asp Tyr Asp Arg Leu Arg Pro Leu Ser Tyr Pro
65                  70                  75                  80
gat tcc aat gtg gtg ctt atc tgt ttc tct gtg gac cta cct gac tcg      288
Asp Ser Asn Val Val Leu Ile Cys Phe Ser Val Asp Leu Pro Asp Ser
                85                  90                  95
ctg gac aat gtg cag gag aag tgg gtc agc gaa gtt ctg cac ttc tgc      336
Leu Asp Asn Val Gln Glu Lys Trp Val Ser Glu Val Leu His Phe Cys
            100                 105                 110
cag ggc gtg cca att tta ctg gta ggc tgc aag gtg gat ctg aga aac      384
Gln Gly Val Pro Ile Leu Leu Val Gly Cys Lys Val Asp Leu Arg Asn
        115                 120                 125
gac cct cag gtg ctc cag cag ctg cag gcc gag ggc cag cgc ccc gtg      432
Asp Pro Gln Val Leu Gln Gln Leu Gln Ala Glu Gly Gln Arg Pro Val
    130                 135                 140
acc gcc gca gag ggc tca gcc gtc gcc ggt aag ata ggc gcc gtc gcc      480
Thr Ala Ala Glu Gly Ser Ala Val Ala Gly Lys Ile Gly Ala Val Ala
145                 150                 155                 160
tac ctc gag tgc tct gcg cgc aca ggc cag ggt gtg aag gag gtt ttc      528
Tyr Leu Glu Cys Ser Ala Arg Thr Gly Gln Gly Val Lys Glu Val Phe

165                 170                 175
gac act gcg acc cgc gcc gca ctc tct ggc aag ccc gcc gcg tct gca      576
Asp Thr Ala Thr Arg Ala Ala Leu Ser Gly Lys Pro Ala Ala Ser Ala
        180                 185                 190
ggc aag aag aag gtc cac ggt gac aag aag aag aag aaa tgt ctg gtc      624
Gly Lys Lys Lys Val His Gly Asp Lys Lys Lys Lys Lys Cys Leu Val
        195                 200                 205
ctg tga                                                              630
Leu

<210> 374
<211> 209
<212> PRT
<213> Ashbya gossypii

<400> 374
Met Ala Tyr Gln Thr Gly Gly Asn Ile Arg Lys Lys Leu Val Ile Val
1               5                   10                  15
Gly Asp Gly Ala Cys Gly Lys Thr Cys Leu Leu Val Val Phe Ser Lys
            20                  25                  30
Gly Gln Phe Pro Glu Ile His Val Pro Thr Val Phe Glu Asn Tyr Val
        35                  40                  45
Ala Asp Val Asp Ile Asp Gly Arg Arg Val Glu Leu Ala Leu Trp Asp
    50                  55                  60
Thr Ala Gly Gln Glu Asp Tyr Asp Arg Leu Arg Pro Leu Ser Tyr Pro
65                  70                  75                  80
Asp Ser Asn Val Val Leu Ile Cys Phe Ser Val Asp Leu Pro Asp Ser
                85                  90                  95
Leu Asp Asn Val Gln Glu Lys Trp Val Ser Glu Val Leu His Phe Cys
            100                 105                 110
Gln Gly Val Pro Ile Leu Leu Val Gly Cys Lys Val Asp Leu Arg Asn
        115                 120                 125
Asp Pro Gln Val Leu Gln Gln Leu Gln Ala Glu Gly Gln Arg Pro Val
    130                 135                 140
Thr Ala Ala Glu Gly Ser Ala Val Ala Gly Lys Ile Gly Ala Val Ala
145                 150                 155                 160
Tyr Leu Glu Cys Ser Ala Arg Thr Gly Gln Gly Val Lys Glu Val Phe
                165                 170                 175
Asp Thr Ala Thr Arg Ala Ala Leu Ser Gly Lys Pro Ala Ala Ser Ala
            180                 185                 190
Gly Lys Lys Lys Val His Gly Asp Lys Lys Lys Lys Lys Cys Leu Val
        195                 200                 205
Leu

<210> 375
<211> 564
<212> DNA
<213> Ashbya gossypii

<220>
<221> CDS
<222> (1)..(564)

<400> 375
atg acg gtc aac gtt gtg aga cgg aag ttg gta atc ata ggg gat ggg      48
Met Thr Val Asn Val Val Arg Arg Lys Leu Val Ile Ile Gly Asp Gly
1               5                   10                  15
gca tgc ggc aag acg tcg tta cta cat gtg ttc acg ctg ggg aag ttc      96
Ala Cys Gly Lys Thr Ser Leu Leu His Val Phe Thr Leu Gly Lys Phe
            20                  25                  30
cct gag gaa tat ctg ccc acg gtt ttc gag aac tac gtt acg gat tgc      144
Pro Glu Glu Tyr Leu Pro Thr Val Phe Glu Asn Tyr Val Thr Asp Cys
        35                  40                  45
cgt gta gac ggc ata aaa gtg cag ttg gcg cta tgg gat act gct ggt      192
Arg Val Asp Gly Ile Lys Val Gln Leu Ala Leu Trp Asp Thr Ala Gly

50                  55                  60
cag gaa gaa tac gag cgt ctg cgc ccc atg tcc tac tcg aag gcg gac      240
Gln Glu Glu Tyr Glu Arg Leu Arg Pro Met Ser Tyr Ser Lys Ala Asp
65                  70                  75                  80
atc ata tta atc ggg ttt gcc ata gac gac ccg ggg tcg ttg tcc aat      288
Ile Ile Leu Ile Gly Phe Ala Ile Asp Asp Pro Gly Ser Leu Ser Asn
                85                  90                  95
gcg cgg gag aag tgg acg gtc gag gcg ctg cgc tac tgt ccc aac gcc      336
Ala Arg Glu Lys Trp Thr Val Glu Ala Leu Arg Tyr Cys Pro Asn Ala
            100                 105                 110
ccg atc atc ctc gtg ggg ctc aaa aag gac ctt cgc cgc ccc ggg acg      384
Pro Ile Ile Leu Val Gly Leu Lys Lys Asp Leu Arg Arg Pro Gly Thr
        115                 120                 125
cag tgc gcg atg gta gcg cct tcg cag gca caa gag gtg gtg cgc gcc      432
Gln Cys Ala Met Val Ala Pro Ser Gln Ala Gln Glu Val Val Arg Ala
    130                 135                 140
atc ggc gca aag aaa tac atg gag tgc agc gca ctt acg ggg gag ggc      480
Ile Gly Ala Lys Lys Tyr Met Glu Cys Ser Ala Leu Thr Gly Glu Gly
145                 150                 155                 160
gtg gac gat gtg ttc gag ctg gcc acg aga aca agt ctt ctg gtg aac      528
Val Asp Asp Val Phe Glu Leu Ala Thr Arg Thr Ser Leu Leu Val Asn
                165                 170                 175
aag gag ccg ggt caa ggc tgt tgc att atc tca tga                      564
Lys Glu Pro Gly Gln Gly Cys Cys Ile Ile Ser
            180                 185


<210> 376
<211> 187
<212> PRT
<213> Ashbya gossypii

<400> 376
Met Thr Val Asn Val Val Arg Arg Lys Leu Val Ile Ile Gly Asp Gly
1               5                   10                  15
Ala Cys Gly Lys Thr Ser Leu Leu His Val Phe Thr Leu Gly Lys Phe
            20                  25                  30
Pro Glu Glu Tyr Leu Pro Thr Val Phe Glu Asn Tyr Val Thr Asp Cys
        35                  40                  45
Arg Val Asp Gly Ile Lys Val Gln Leu Ala Leu Trp Asp Thr Ala Gly
    50                  55                  60
Gln Glu Glu Tyr Glu Arg Leu Arg Pro Met Ser Tyr Ser Lys Ala Asp
65                  70                  75                  80
Ile Ile Leu Ile Gly Phe Ala Ile Asp Asp Pro Gly Ser Leu Ser Asn
                85                  90                  95
Ala Arg Glu Lys Trp Thr Val Glu Ala Leu Arg Tyr Cys Pro Asn Ala
            100                 105                 110
Pro Ile Ile Leu Val Gly Leu Lys Lys Asp Leu Arg Arg Pro Gly Thr
        115                 120                 125
Gln Cys Ala Met Val Ala Pro Ser Gln Ala Gln Glu Val Val Arg Ala
    130                 135                 140
Ile Gly Ala Lys Lys Tyr Met Glu Cys Ser Ala Leu Thr Gly Glu Gly
145                 150                 155                 160
Val Asp Asp Val Phe Glu Leu Ala Thr Arg Thr Ser Leu Leu Val Asn
                165                 170                 175
Lys Glu Pro Gly Gln Gly Cys Cys Ile Ile Ser
            180                 185

<210> 377
<211> 1043
<212> DNA
<213> Tigriopus japonicus

<220>
<221> CDS
<222> (264)..(842)

<400> 377

aggaggagac gccttgtttg aaatccatcc aacaacctgg gactctctgc tccagcagca 60

agcatcacca cccccatcac catcatcttc atcctgatca acacccttga gatccagatc 120

cccatccatc gatccaccca tcctgaggat tgactcacac acctcgaaga ttgtgggctg 180

tgaaaaacca gtgtgggatc ggcccttgcg tgcgtgaggt gggtggttgt tgaaacccga 240

ctggcctgac cattgtcgtc tcg atg gcg gct att cgc aag aaa tta gtg att 293
Met Ala Ala Ile Arg Lys Lys Leu Val Ile
1               5                   10
gtg ggc gat ggc gcc tgc ggc aag acc tgc ctc ttg atc gtg ttc tcc 341
Val Gly Asp Gly Ala Cys Gly Lys Thr Cys Leu Leu Ile Val Phe Ser
                15                  20                  25
aag gat caa ttc ccc gag gtg tat gtg ccc aca gtg ttc gaa aac tac 389
Lys Asp Gln Phe Pro Glu Val Tyr Val Pro Thr Val Phe Glu Asn Tyr
            30                  35                  40
gtg gct gac atc gaa gtg gac ggc aag caa gtg gaa ctg gcc ttg tgg 437
Val Ala Asp Ile Glu Val Asp Gly Lys Gln Val Glu Leu Ala Leu Trp
        45                  50                  55
gat acg gcc ggt cag gag gac tac gat cga ctc cga ccc ctg tcc tat 485
Asp Thr Ala Gly Gln Glu Asp Tyr Asp Arg Leu Arg Pro Leu Ser Tyr
    60                  65                  70
ccg gat acg gat gtg atc ctg atg tgt ttc tcg atc gac tcg ccc gat 533
Pro Asp Thr Asp Val Ile Leu Met Cys Phe Ser Ile Asp Ser Pro Asp
75                  80                  85                  90
tcg ctc gag aac atc cct gag aag tgg acg ccc gaa gtc aaa cac ttc 581
Ser Leu Glu Asn Ile Pro Glu Lys Trp Thr Pro Glu Val Lys His Phe
                95                  100                 105
tgc cct aat gtg ccc att atc ctc gtg gga aac aaa aaa gac ttg cgc 629
Cys Pro Asn Val Pro Ile Ile Leu Val Gly Asn Lys Lys Asp Leu Arg
            110                 115                 120
aat gat ccc aat acc atc aag gag ttg ggc aag atg aaa caa gaa ccg 677
Asn Asp Pro Asn Thr Ile Lys Glu Leu Gly Lys Met Lys Gln Glu Pro
        125                 130                 135
gtc aag ccg gaa gag ggt cgc aca atg gcc gaa aag atc aac gcc ttc 725
Val Lys Pro Glu Glu Gly Arg Thr Met Ala Glu Lys Ile Asn Ala Phe
    140                 145                 150
gcc tac ctc gag tgt tcg gcc aag agc aag gaa ggc gtc aga gaa gtc 773
Ala Tyr Leu Glu Cys Ser Ala Lys Ser Lys Glu Gly Val Arg Glu Val
155                 160                 165                 170
ttt gag aca gcc acc cga gcg gca tta cag gtc aag aag aaa aag aag 821
Phe Glu Thr Ala Thr Arg Ala Ala Leu Gln Val Lys Lys Lys Lys Lys
                175                 180                 185
aag cct tgc gtt ctc ttt taatgtcgga aaccaaacca tttttcctat 869
Lys Pro Cys Val Leu Phe
            190
tcttattacc tatcttcatc atcaacatca acatccttca accacaaata caactacaac 929

tattactata cttctactac tatggacgcg atcttaattt agtcgcaatt atcaattggg 989

gaaatttacg gaataaaaca gaccctgaga acaaaaaaaa aaaaaaaaaa aaaa 1043

<210> 378
<211> 192
<212> PRT
<213> Tigriopus japonicus

<400> 378

Met Ala Ala Ile Arg Lys Lys Leu Val Ile Val Gly Asp Gly Ala Cys
1               5                   10                  15
Gly Lys Thr Cys Leu Leu Ile Val Phe Ser Lys Asp Gln Phe Pro Glu
            20                  25                  30
Val Tyr Val Pro Thr Val Phe Glu Asn Tyr Val Ala Asp Ile Glu Val
        35                  40                  45
Asp Gly Lys Gln Val Glu Leu Ala Leu Trp Asp Thr Ala Gly Gln Glu
    50                  55                  60
Asp Tyr Asp Arg Leu Arg Pro Leu Ser Tyr Pro Asp Thr Asp Val Ile
65                  70                  75                  80
Leu Met Cys Phe Ser Ile Asp Ser Pro Asp Ser Leu Glu Asn Ile Pro
                85                  90                  95
Glu Lys Trp Thr Pro Glu Val Lys His Phe Cys Pro Asn Val Pro Ile
            100                 105                 110
Ile Leu Val Gly Asn Lys Lys Asp Leu Arg Asn Asp Pro Asn Thr Ile
        115                 120                 125
Lys Glu Leu Gly Lys Met Lys Gln Glu Pro Val Lys Pro Glu Glu Gly
    130                 135                 140
Arg Thr Met Ala Glu Lys Ile Asn Ala Phe Ala Tyr Leu Glu Cys Ser
145                 150                 155                 160
Ala Lys Ser Lys Glu Gly Val Arg Glu Val Phe Glu Thr Ala Thr Arg
                165                 170                 175
Ala Ala Leu Gln Val Lys Lys Lys Lys Lys Lys Pro Cys Val Leu Phe
            180                 185                 190

<210> 379
<211> 1014
<212> DNA
<213> Rhopalosiphum padi

<220>
<221> CDS
<222> (197)..(772)

<400> 379
tcaattgcgt tctgccattt ttttctaccg acgtcgatcc gcgcgtgttg atctgccggg      60

tacactccgt acacaggttg gagatcgctg caggcagcgt tcgattgcga caacgacgac     120

cagcgagaac acttctctag tgctctgtgc tgcttattca ccagtgtaca cgtaggagtg     180

ttcccatagc ggcaac atg cag acc atc aag tgc gtt gtt gtt ggt gat gga     232
                  Met Gln Thr Ile Lys Cys Val Val Val Gly Asp Gly
                  1               5                   10
gct gtc ggt aag act tgt ctg ctc ata tcg tac aca aca aac aaa ttt     280
Ala Val Gly Lys Thr Cys Leu Leu Ile Ser Tyr Thr Thr Asn Lys Phe
        15                  20                  25
cct tca gaa tat gta ccg act gtt ttc gac aat tat gca gtg aca gtt     328
Pro Ser Glu Tyr Val Pro Thr Val Phe Asp Asn Tyr Ala Val Thr Val
    30                  35                  40
atg att ggt ggg gaa cca tac aca tta ggt tta ttt gat aca gca ggt     376
Met Ile Gly Gly Glu Pro Tyr Thr Leu Gly Leu Phe Asp Thr Ala Gly
45                  50                  55                  60
cag gaa gat tat gat cgc ctc aga cct ttg agt tat cca caa act gat     424
Gln Glu Asp Tyr Asp Arg Leu Arg Pro Leu Ser Tyr Pro Gln Thr Asp
                65                  70                  75
gtg ttt ctt gtt tgt ttc tct gtg gtt tta cca tct tca ttt gaa aat     472
Val Phe Leu Val Cys Phe Ser Val Val Leu Pro Ser Ser Phe Glu Asn
            80                  85                  90
gtc aaa gaa aaa tgg gtt ccg gag ata acg cat cac tgt caa aaa aca     520
Val Lys Glu Lys Trp Val Pro Glu Ile Thr His His Cys Gln Lys Thr
        95                  100                 105
cca ttc ctg ttg gtt ggc aca caa ata gac ctt aga gaa gat gcc aca     568
Pro Phe Leu Leu Val Gly Thr Gln Ile Asp Leu Arg Glu Asp Ala Thr
    110                 115                 120

act gtg gag aaa cta gcc aaa aat aaa caa aaa tca ata tca ttt gaa 616
Thr Val Glu Lys Leu Ala Lys Asn Lys Gln Lys Ser Ile Ser Phe Glu
125                 130                 135                 140
caa gga gag aag cta gct aaa gaa ctt aaa gct gtg aaa tat gtc gaa 664
Gln Gly Glu Lys Leu Ala Lys Glu Leu Lys Ala Val Lys Tyr Val Glu
                145                 150                 155
tgc tca gca ctt aca caa aaa gga cta aaa aat gta ttt gat gaa gct 712
Cys Ser Ala Leu Thr Gln Lys Gly Leu Lys Asn Val Phe Asp Glu Ala
            160                 165                 170
att ctt gca gct tta gag cct cct gaa cca gtt aag aag agg aag tgt 760
Ile Leu Ala Ala Leu Glu Pro Pro Glu Pro Val Lys Lys Arg Lys Cys
        175                 180                 185
gtt ata ttg taaggctgcg gataaataaa caggtgcgac aattatgtca taaaaatatt 819
Val Ile Leu
    190
taagataaaa caatttaaat catgatttag catggataca ataatgaaat aataattatt 879

ttgtttttac taatctataa atatatatat ataaataatt tattttatat tttacaagaa 939

aatatgtgca ttcattgaat aataaataaa taggtttttt atacgccaaa aaaaaaaaaa 999

aaaaaaaaaa aaaaa 1014

<210> 380
<211> 191
<212> PRT
<213> Rhopalosiphum padi

<400> 380
Met Gln Thr Ile Lys Cys Val Val Val Gly Asp Gly Ala Val Gly Lys
1               5                   10                  15
Thr Cys Leu Leu Ile Ser Tyr Thr Thr Asn Lys Phe Pro Ser Glu Tyr
            20                  25                  30
Val Pro Thr Val Phe Asp Asn Tyr Ala Val Thr Val Met Ile Gly Gly
        35                  40                  45
Glu Pro Tyr Thr Leu Gly Leu Phe Asp Thr Ala Gly Gln Glu Asp Tyr
    50                  55                  60
Asp Arg Leu Arg Pro Leu Ser Tyr Pro Gln Thr Asp Val Phe Leu Val
65                  70                  75                  80
Cys Phe Ser Val Val Leu Pro Ser Ser Phe Glu Asn Val Lys Glu Lys
                85                  90                  95
Trp Val Pro Glu Ile Thr His His Cys Gln Lys Thr Pro Phe Leu Leu
            100                 105                 110
Val Gly Thr Gln Ile Asp Leu Arg Glu Asp Ala Thr Thr Val Glu Lys
        115                 120                 125
Leu Ala Lys Asn Lys Gln Lys Ser Ile Ser Phe Glu Gln Gly Glu Lys
    130                 135                 140
Leu Ala Lys Glu Leu Lys Ala Val Lys Tyr Val Glu Cys Ser Ala Leu
145                 150                 155                 160
Thr Gln Lys Gly Leu Lys Asn Val Phe Asp Glu Ala Ile Leu Ala Ala
                165                 170                 175
Leu Glu Pro Pro Glu Pro Val Lys Lys Arg Lys Cys Val Ile Leu
            180                 185                 190

<210> 381
<211> 679
<212> DNA
<213> Hordeum vulgare var

<220>
<221> CDS
<222> (14)..(655)

<400> 381
cgcgcggcga gcc atg gcg tcc agc gcc tcc cgg ttc atc aag tgc gtc 49
Met Ala Ser Ser Ala Ser Arg Phe Ile Lys Cys Val
1               5                   10
acg gtg ggc gac ggc gcc gtc ggc aag acc tgc atg ctc atc tgc tac 97
Thr Val Gly Asp Gly Ala Val Gly Lys Thr Cys Met Leu Ile Cys Tyr
        15                  20                  25
acc agc aac aag ttc ccc acc gac tac ata ccc acg gtg ttc gac aat 145
Thr Ser Asn Lys Phe Pro Thr Asp Tyr Ile Pro Thr Val Phe Asp Asn
    30                  35                  40
ttc agc gcg aac gtg gtg gcg gac ggc acc acg gtg aat ttg ggc ctt 193
Phe Ser Ala Asn Val Val Ala Asp Gly Thr Thr Val Asn Leu Gly Leu
45                  50                  55                  60
tgg gac acc gcc ggg cag gag gat tac aac cgg ctg agg cct cta agc 241
Trp Asp Thr Ala Gly Gln Glu Asp Tyr Asn Arg Leu Arg Pro Leu Ser
                65                  70                  75
tac cgc ggc gcc gac gtt ttc gtg ctt gcc ttc tcc ctt gtg agc cga 289
Tyr Arg Gly Ala Asp Val Phe Val Leu Ala Phe Ser Leu Val Ser Arg
            80                  85                  90
gct agc tat gag aat atc atg aag aag tgg ata ccg gag ctt cag cat 337
Ala Ser Tyr Glu Asn Ile Met Lys Lys Trp Ile Pro Glu Leu Gln His
        95                  100                 105
tac gcg ccc ggc gta cct gtt gtg ctg gta ggc aca aaa ctg gat ctt 385
Tyr Ala Pro Gly Val Pro Val Val Leu Val Gly Thr Lys Leu Asp Leu
    110                 115                 120
cgt gaa gat aag cac tat ttg ctg gac cac cct ggg atg ata ccc gtt 433
Arg Glu Asp Lys His Tyr Leu Leu Asp His Pro Gly Met Ile Pro Val
125                 130                 135                 140
acc aca gca cag ggg gag gaa ctt cgt aag caa gtt ggt gct tta tat 481
Thr Thr Ala Gln Gly Glu Glu Leu Arg Lys Gln Val Gly Ala Leu Tyr
                145                 150                 155
tac ata gag tgc agc tca aag aca caa cag aat gtc aaa gct gtg ttt 529
Tyr Ile Glu Cys Ser Ser Lys Thr Gln Gln Asn Val Lys Ala Val Phe
            160                 165                 170
gat gct gct atc aag gta gta atc cag ccc cca act aaa caa aga gaa 577
Asp Ala Ala Ile Lys Val Val Ile Gln Pro Pro Thr Lys Gln Arg Glu
        175                 180                 185
aag aag aaa aag aaa cag cgt cgg gga tgt tct atg atg aac ttc agc 625
Lys Lys Lys Lys Lys Gln Arg Arg Gly Cys Ser Met Met Asn Phe Ser
    190                 195                 200
gga agg aaa atg cta tgc ttc aaa tcc tgaatgatga aagagaaggt 672
Gly Arg Lys Met Leu Cys Phe Lys Ser
205                 210
tccttgc 679

<210> 382
<211> 213
<212> PRT
<213> Hordeum vulgare var

<400> 382
Met Ala Ser Ser Ala Ser Arg Phe Ile Lys Cys Val Thr Val Gly Asp
1               5                   10                  15
Gly Ala Val Gly Lys Thr Cys Met Leu Ile Cys Tyr Thr Ser Asn Lys
            20                  25                  30
Phe Pro Thr Asp Tyr Ile Pro Thr Val Phe Asp Asn Phe Ser Ala Asn
        35                  40                  45
Val Val Ala Asp Gly Thr Thr Val Asn Leu Gly Leu Trp Asp Thr Ala
    50                  55                  60
Gly Gln Glu Asp Tyr Asn Arg Leu Arg Pro Leu Ser Tyr Arg Gly Ala
65                  70                  75                  80
Asp Val Phe Val Leu Ala Phe Ser Leu Val Ser Arg Ala Ser Tyr Glu
                85                  90                  95
Asn Ile Met Lys Lys Trp Ile Pro Glu Leu Gln His Tyr Ala Pro Gly

                100                 105                 110
Val Pro Val Val Leu Val Gly Thr Lys Leu Asp Leu Arg Glu Asp Lys
        115                 120                 125
His Tyr Leu Leu Asp His Pro Gly Met Ile Pro Val Thr Thr Ala Gln
    130                 135                 140
Gly Glu Glu Leu Arg Lys Gln Val Gly Ala Leu Tyr Tyr Ile Glu Cys
145                 150                 155                 160
Ser Ser Lys Thr Gln Gln Asn Val Lys Ala Val Phe Asp Ala Ala Ile
                165                 170                 175
Lys Val Val Ile Gln Pro Pro Thr Lys Gln Arg Glu Lys Lys Lys Lys
            180                 185                 190
Lys Gln Arg Arg Gly Cys Ser Met Met Asn Phe Ser Gly Arg Lys Met
        195                 200                 205
Leu Cys Phe Lys Ser
    210

<210> 383
<211> 762
<212> DNA
<213> Hordeum vulgare var

<220>
<221> CDS
<222> (33)..(689)

<400> 383
ggatccgctg gagaggagag gagaggagag ac atg agc ggc gga gcg ggg gcg      53
                                    Met Ser Gly Gly Ala Gly Ala
                                    1               5
gcg acg gcg gtg agc agg ttc atc aag tgc gtg gcc gtg ggc gac ggc      101
Ala Thr Ala Val Ser Arg Phe Ile Lys Cys Val Ala Val Gly Asp Gly
        10                  15                  20
gcc gtg ggc aag acc tgc atg ctc atc tgc tac acc tgc aac aag ttc      149
Ala Val Gly Lys Thr Cys Met Leu Ile Cys Tyr Thr Cys Asn Lys Phe
    25                  30                  35
ccc acc gac tac atc ccc acc gtg ttc gac aac ttc agc gcc aat gtc      197
Pro Thr Asp Tyr Ile Pro Thr Val Phe Asp Asn Phe Ser Ala Asn Val
40                  45                  50                  55
tcc gtg gac ggg agc atc gtc aac ctc ggc ctc tgg gac acc gca ggt      245
Ser Val Asp Gly Ser Ile Val Asn Leu Gly Leu Trp Asp Thr Ala Gly
                60                  65                  70
cag gag gat tac agc agg ctg agg cct ctg agc tac agg gga gcc gat      293
Gln Glu Asp Tyr Ser Arg Leu Arg Pro Leu Ser Tyr Arg Gly Ala Asp
            75                  80                  85
gtc ttc atc ctc tcc ttc tcc ctc acc agc aga gca agc tat gag aat      341
Val Phe Ile Leu Ser Phe Ser Leu Thr Ser Arg Ala Ser Tyr Glu Asn
        90                  95                  100
gtg cac aag aag tgg atg ccg gag ctt cgc cgg tac gcc ccc ggc att      389
Val His Lys Lys Trp Met Pro Glu Leu Arg Arg Tyr Ala Pro Gly Ile
    105                 110                 115
cct gta ctg ctt gtt gga acc aag ttg gat ctc cgg gag gat aga gct      437
Pro Val Leu Leu Val Gly Thr Lys Leu Asp Leu Arg Glu Asp Arg Ala
120                 125                 130                 135
tat ctt gct gat cat gca gct gat tcc atc ata aca act gag cag ggt      485
Tyr Leu Ala Asp His Ala Ala Asp Ser Ile Ile Thr Thr Glu Gln Gly
                140                 145                 150
gag gat ctt agg aga caa ata ggt gct gtg gca tac ata gaa tgc agc      533
Glu Asp Leu Arg Arg Gln Ile Gly Ala Val Ala Tyr Ile Glu Cys Ser
            155                 160                 165
tcc aag aca caa agg aac att aag gct gtt ttc gac acc gca atc aag      581
Ser Lys Thr Gln Arg Asn Ile Lys Ala Val Phe Asp Thr Ala Ile Lys
        170                 175                 180
gcg gtt ctt caa cct caa agg cac aag gag gta gcc aga aag gaa act      629
Ala Val Leu Gln Pro Gln Arg His Lys Glu Val Ala Arg Lys Glu Thr
    185                 190                 195
cgg aca cgc tct agt cgg tca gta agg cag tac ttc tgt ggg agt tct      677
Arg Thr Arg Ser Ser Arg Ser Val Arg Gln Tyr Phe Cys Gly Ser Ser
200                 205                 210                 215

tgt ttc gcg tagccgagaa gaacattctg gagtctttcc tcgacacgat gctcagatgg    736
Cys Phe Ala

cccgtggttg ttctccaaat ggggta                                          762

<210> 384
<211> 218
<212> PRT
<213> Hordeum vulgare var

<400> 384
Met Ser Gly Gly Ala Gly Ala Ala Thr Ala Val Ser Arg Phe Ile Lys
1               5                   10                  15
Cys Val Ala Val Gly Asp Gly Ala Val Gly Lys Thr Cys Met Leu Ile
            20                  25                  30
Cys Tyr Thr Cys Asn Lys Phe Pro Thr Asp Tyr Ile Pro Thr Val Phe
        35                  40                  45
Asp Asn Phe Ser Ala Asn Val Ser Val Asp Gly Ser Ile Val Asn Leu
    50                  55                  60
Gly Leu Trp Asp Thr Ala Gly Gln Glu Asp Tyr Ser Arg Leu Arg Pro
65                  70                  75                  80
Leu Ser Tyr Arg Gly Ala Asp Val Phe Ile Leu Ser Phe Ser Leu Thr
                85                  90                  95
Ser Arg Ala Ser Tyr Glu Asn Val His Lys Lys Trp Met Pro Glu Leu
            100                 105                 110
Arg Arg Tyr Ala Pro Gly Ile Pro Val Leu Leu Val Gly Thr Lys Leu
        115                 120                 125
Asp Leu Arg Glu Asp Arg Ala Tyr Leu Ala Asp His Ala Ala Asp Ser
    130                 135                 140
Ile Ile Thr Thr Glu Gln Gly Glu Asp Leu Arg Arg Gln Ile Gly Ala
145                 150                 155                 160
Val Ala Tyr Ile Glu Cys Ser Ser Lys Thr Gln Arg Asn Ile Lys Ala
                165                 170                 175
Val Phe Asp Thr Ala Ile Lys Ala Val Leu Gln Pro Gln Arg His Lys
            180                 185                 190
Glu Val Ala Arg Lys Glu Thr Arg Thr Arg Ser Ser Arg Ser Val Arg
        195                 200                 205
Gln Tyr Phe Cys Gly Ser Ser Cys Phe Ala
    210                 215

<210> 385
<211> 721
<212> DNA
<213> Hordeum vulgare

<220>
<221> CDS
<222> (38)..(676)

<400> 385
gtggaggcgc ggcgagagcg gcggaggcgg aggagag atg agc gtg acc aag ttc      55
                                         Met Ser Val Thr Lys Phe
                                         1               5
atc aag tgc gtc acg gtg ggg gac ggc gcc gtc ggc aag acc tgc atg      103
Ile Lys Cys Val Thr Val Gly Asp Gly Ala Val Gly Lys Thr Cys Met
            10                  15                  20
ctc atc tgc tac acc agc aac agg ttc ccc agt gat tac atc ccc acg      151
Leu Ile Cys Tyr Thr Ser Asn Arg Phe Pro Ser Asp Tyr Ile Pro Thr
        25                  30                  35
gtg ttc gac aac ttc agc gcc aac gtc tcc gtc gac ggc aac atc gtc      199
Val Phe Asp Asn Phe Ser Ala Asn Val Ser Val Asp Gly Asn Ile Val
    40                  45                  50
aac ctc ggc cta tgg gac acc gcc ggg caa gaa gac tac agc cgg ctg      247
Asn Leu Gly Leu Trp Asp Thr Ala Gly Gln Glu Asp Tyr Ser Arg Leu
55                  60                  65                  70

agg ccg ctg agc tac aga ggc gcc gac gtg ttc gtg ctc gcc ttc tcc 295
Arg Pro Leu Ser Tyr Arg Gly Ala Asp Val Phe Val Leu Ala Phe Ser
                75                  80                  85
ctc atc agc agc gcc agc tac gag aat gtt ctt aag aag tgg atg cca 343
Leu Ile Ser Ser Ala Ser Tyr Glu Asn Val Leu Lys Lys Trp Met Pro
            90                  95                  100
gag ctc cgc cgg ttc gcg ccg aat gtc ccc att gtt ctt gtt ggg acc 391
Glu Leu Arg Arg Phe Ala Pro Asn Val Pro Ile Val Leu Val Gly Thr
        105                 110                 115
aag cta gat ctg cgt gac cac aga gcc tac ctc gcc gac cac ccc ggt 439
Lys Leu Asp Leu Arg Asp His Arg Ala Tyr Leu Ala Asp His Pro Gly
    120                 125                 130
gct tca gca atc aca act gca cag ggt gaa gaa ctt agg aag cag atc 487
Ala Ser Ala Ile Thr Thr Ala Gln Gly Glu Glu Leu Arg Lys Gln Ile
135                 140                 145                 150
ggc gcc gcg gct tac atc gag tgc agc tcc aag aca cag cag aac gtc 535
Gly Ala Ala Ala Tyr Ile Glu Cys Ser Ser Lys Thr Gln Gln Asn Val
                155                 160                 165
aag gct gtg ttt gac acc gcc ata aag gtg gtc ctc cag ccg ccg agg 583
Lys Ala Val Phe Asp Thr Ala Ile Lys Val Val Leu Gln Pro Pro Arg
            170                 175                 180
aga agg gag gtg atg tcc gcc agg aag aaa acc agg cga agc tct gga 631
Arg Arg Glu Val Met Ser Ala Arg Lys Lys Thr Arg Arg Ser Ser Gly
        185                 190                 195
tgc tcc atc aag cac ttg atc tgc ggg agt acg tgc gct gct tgaattagca 683
Cys Ser Ile Lys His Leu Ile Cys Gly Ser Thr Cys Ala Ala
    200                 205                 210
ccatggaggc ctggactgac tatggagatg aagcatgg 721

<210> 386
<211> 212
<212> PRT
<213> Hordeum vulgare

<400> 386
Met Ser Val Thr Lys Phe Ile Lys Cys Val Thr Val Gly Asp Gly Ala
1               5                   10                  15
Val Gly Lys Thr Cys Met Leu Ile Cys Tyr Thr Ser Asn Arg Phe Pro
            20                  25                  30
Ser Asp Tyr Ile Pro Thr Val Phe Asp Asn Phe Ser Ala Asn Val Ser
        35                  40                  45
Val Asp Gly Asn Ile Val Asn Leu Gly Leu Trp Asp Thr Ala Gly Gln
    50                  55                  60
Glu Asp Tyr Ser Arg Leu Arg Pro Leu Ser Tyr Arg Gly Ala Asp Val
65                  70                  75                  80
Phe Val Leu Ala Phe Ser Leu Ile Ser Ser Ala Ser Tyr Glu Asn Val
                85                  90                  95
Leu Lys Lys Trp Met Pro Glu Leu Arg Arg Phe Ala Pro Asn Val Pro
            100                 105                 110
Ile Val Leu Val Gly Thr Lys Leu Asp Leu Arg Asp His Arg Ala Tyr
        115                 120                 125
Leu Ala Asp His Pro Gly Ala Ser Ala Ile Thr Thr Ala Gln Gly Glu
    130                 135                 140
Glu Leu Arg Lys Gln Ile Gly Ala Ala Ala Tyr Ile Glu Cys Ser Ser
145                 150                 155                 160
Lys Thr Gln Gln Asn Val Lys Ala Val Phe Asp Thr Ala Ile Lys Val
                165                 170                 175
Val Leu Gln Pro Pro Arg Arg Arg Glu Val Met Ser Ala Arg Lys Lys
            180                 185                 190
Thr Arg Arg Ser Ser Gly Cys Ser Ile Lys His Leu Ile Cys Gly Ser
        195                 200                 205
Thr Cys Ala Ala
    210

<210> 387

<211> 699
<212> DNA
<213> Hordeum vulgare

<220>
<221> CDS
<222> (67)..(660)

<400> 387
ggatcccgat tccatcagga aagcatatag actagcccag taaatagaaa taagnaaaga 60

tcggcg atg agc gca tct cgg ttc atc aag tgc gtg acg gtg ggg gac 108
Met Ser Ala Ser Arg Phe Ile Lys Cys Val Thr Val Gly Asp
1               5                   10
ggc gcc gtg gga aag aca tgc ctc ctc atc tca tac aca tcc aac acc 156
Gly Ala Val Gly Lys Thr Cys Leu Leu Ile Ser Tyr Thr Ser Asn Thr
15                  20                  25                  30
ttc ccc aca gac tat gtc cca aca gtt ttc gac aac ttc agc gct aac 204
Phe Pro Thr Asp Tyr Val Pro Thr Val Phe Asp Asn Phe Ser Ala Asn
                35                  40                  45
gtc gtg gtt gac ggc agc acc gtc aac ctc gga tta tgg gat act gca 252
Val Val Val Asp Gly Ser Thr Val Asn Leu Gly Leu Trp Asp Thr Ala
            50                  55                  60
gga caa gaa gac tat aat cga cta cgc cca cta agc tac cgt ggt gcc 300
Gly Gln Glu Asp Tyr Asn Arg Leu Arg Pro Leu Ser Tyr Arg Gly Ala
        65                  70                  75
gat gtc ttc ctg ctc gcc ttt tct ctc atc agc aaa gca agc tac gag 348
Asp Val Phe Leu Leu Ala Phe Ser Leu Ile Ser Lys Ala Ser Tyr Glu
    80                  85                  90
aat gtc act aag aag tgg att cca gag tta cgg cac tat gct cct ggc 396
Asn Val Thr Lys Lys Trp Ile Pro Glu Leu Arg His Tyr Ala Pro Gly
95                  100                 105                 110
gtg ccc ata att ctt gtt gga aca aag ctt gat ctg cgg gat gac aag 444
Val Pro Ile Ile Leu Val Gly Thr Lys Leu Asp Leu Arg Asp Asp Lys
                115                 120                 125
cag ttt ttt gtg gat cac cct ggg gcg gtt cct att tcc act gct cag 492
Gln Phe Phe Val Asp His Pro Gly Ala Val Pro Ile Ser Thr Ala Gln
            130                 135                 140
ggt gaa gag ctg aag aag gtg att ggc gcg act gcc tac atc gag tgc 540
Gly Glu Glu Leu Lys Lys Val Ile Gly Ala Thr Ala Tyr Ile Glu Cys
        145                 150                 155
agc tca aaa aca cag cag aac atc aag gcg gtg ttt gat gcg gcg atc 588
Ser Ser Lys Thr Gln Gln Asn Ile Lys Ala Val Phe Asp Ala Ala Ile
    160                 165                 170
aag gtg gtc ctc cag cct ccg aag cag aag cgg aag aag agg aag tca 636
Lys Val Val Leu Gln Pro Pro Lys Gln Lys Arg Lys Lys Arg Lys Ser
175                 180                 185                 190
cag aaa gga tgc agc atc ttg taaagctaaa atccctttg ttttgcagtg 687
Gln Lys Gly Cys Ser Ile Leu
                195
tctcgcgtcg ac 699

<210> 388
<211> 197
<212> PRT
<213> Hordeum vulgare

<400> 388
Met Ser Ala Ser Arg Phe Ile Lys Cys Val Thr Val Gly Asp Gly Ala
1               5                   10                  15
Val Gly Lys Thr Cys Leu Leu Ile Ser Tyr Thr Ser Asn Thr Phe Pro
            20                  25                  30
Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60
Glu Asp Tyr Asn Arg Leu Arg Pro Leu Ser Tyr Arg Gly Ala Asp Val
65                  70                  75                  80
Phe Leu Leu Ala Phe Ser Leu Ile Ser Lys Ala Ser Tyr Glu Asn Val
                85                  90                  95
Thr Lys Lys Trp Ile Pro Glu Leu Arg His Tyr Ala Pro Gly Val Pro
            100                 105                 110
Ile Ile Leu Val Gly Thr Lys Leu Asp Leu Arg Asp Asp Lys Gln Phe
        115                 120                 125
Phe Val Asp His Pro Gly Ala Val Pro Ile Ser Thr Ala Gln Gly Glu
    130                 135                 140
Glu Leu Lys Lys Val Ile Gly Ala Thr Ala Tyr Ile Glu Cys Ser Ser
145                 150                 155                 160
Lys Thr Gln Gln Asn Ile Lys Ala Val Phe Asp Ala Ala Ile Lys Val
                165                 170                 175
Val Leu Gln Pro Pro Lys Gln Lys Arg Lys Lys Arg Lys Ser Gln Lys
            180                 185                 190
Gly Cys Ser Ile Leu
        195

<210> 389
<211> 677
<212> DNA
<213> Hordeum vulgare

<220>
<221> CDS
<222> (27)..(668)

<400> 389
ggatccttct cgtccattta gccggc atg gcg tcc agc gcc tcc cgg ttc atc      53
                             Met Ala Ser Ser Ala Ser Arg Phe Ile
                             1               5
aag tgc gtc acc gtc ggg gac ggc gcc gtc ggc aag acc tgc atg ctc      101
Lys Cys Val Thr Val Gly Asp Gly Ala Val Gly Lys Thr Cys Met Leu
10                  15                  20                  25
atc tgc tac acc agc aac aag ttc ccc acc gac tac gtg ccc acc gtg      149
Ile Cys Tyr Thr Ser Asn Lys Phe Pro Thr Asp Tyr Val Pro Thr Val
                30                  35                  40
ttc gac aat ttc agc gcg aac gtg gtg gtg gac ggc acc acc gtg aac      197
Phe Asp Asn Phe Ser Ala Asn Val Val Val Asp Gly Thr Thr Val Asn
            45                  50                  55
ctg ggc ctc tgg gac act gca ggg cag gag gac tac aac aga ttg aga      245
Leu Gly Leu Trp Asp Thr Ala Gly Gln Glu Asp Tyr Asn Arg Leu Arg
        60                  65                  70
ccg ctg agc tac cgg gga gcc gac gtc ttc gtg ctc tcc ttc tcg ctc      293
Pro Leu Ser Tyr Arg Gly Ala Asp Val Phe Val Leu Ser Phe Ser Leu
    75                  80                  85
gtc agc cga gcc agc tac gag aat gtc atg aag aag tgg cta ccg gag      341
Val Ser Arg Ala Ser Tyr Glu Asn Val Met Lys Lys Trp Leu Pro Glu
90                  95                  100                 105
ctt cag cac cat gca ccc ggc gtg cca aca gtg ctg gtt ggt aca aag      389
Leu Gln His His Ala Pro Gly Val Pro Thr Val Leu Val Gly Thr Lys
                110                 115                 120
cta gat cta cgt gaa gac aag caa tac tta ctt gac cac ccc ggc gtg      437
Leu Asp Leu Arg Glu Asp Lys Gln Tyr Leu Leu Asp His Pro Gly Val
            125                 130                 135
gtg cct gtt act aca gct cag ggg gag gaa ctc cgc aag cac atc ggt      485
Val Pro Val Thr Thr Ala Gln Gly Glu Glu Leu Arg Lys His Ile Gly
        140                 145                 150
gca act tgt tat gtc gaa tgc agc tca aag aca cag cag aat gtc aaa      533
Ala Thr Cys Tyr Val Glu Cys Ser Ser Lys Thr Gln Gln Asn Val Lys
    155                 160                 165
gct gtg ttt gat gct gcc atc aag gta gtg atc aaa cct cca aca aag      581
Ala Val Phe Asp Ala Ala Ile Lys Val Val Ile Lys Pro Pro Thr Lys
170                 175                 180                 185
cag agg gaa agg agg aag aag aaa gca cgg caa gga tgt gca tca ttg      629

Gln Arg Glu Arg Arg Lys Lys Lys Ala Arg Gln Gly Cys Ala Ser Leu
                190                 195                 200
ggt acc ctg tca aga agg aag ctg gca tgc ttc aag tgatcagtcg          675
Gly Thr Leu Ser Arg Arg Lys Leu Ala Cys Phe Lys
            205                 210
ac                                                                  677

<210> 390
<211> 213
<212> PRT
<213> Hordeum vulgare

<400> 390
Met Ala Ser Ser Ala Ser Arg Phe Ile Lys Cys Val Thr Val Gly Asp
1               5                   10                  15
Gly Ala Val Gly Lys Thr Cys Met Leu Ile Cys Tyr Thr Ser Asn Lys
            20                  25                  30
Phe Pro Thr Asp Tyr Val Pro Thr Val Phe Asp Asn Phe Ser Ala Asn
        35                  40                  45
Val Val Val Asp Gly Thr Thr Val Asn Leu Gly Leu Trp Asp Thr Ala
    50                  55                  60
Gly Gln Glu Asp Tyr Asn Arg Leu Arg Pro Leu Ser Tyr Arg Gly Ala
65                  70                  75                  80
Asp Val Phe Val Leu Ser Phe Ser Leu Val Ser Arg Ala Ser Tyr Glu
                85                  90                  95
Asn Val Met Lys Lys Trp Leu Pro Glu Leu Gln His His Ala Pro Gly
            100                 105                 110
Val Pro Thr Val Leu Val Gly Thr Lys Leu Asp Leu Arg Glu Asp Lys
        115                 120                 125
Gln Tyr Leu Leu Asp His Pro Gly Val Val Pro Val Thr Thr Ala Gln
    130                 135                 140
Gly Glu Glu Leu Arg Lys His Ile Gly Ala Thr Cys Tyr Val Glu Cys
145                 150                 155                 160
Ser Ser Lys Thr Gln Gln Asn Val Lys Ala Val Phe Asp Ala Ala Ile
                165                 170                 175
Lys Val Val Ile Lys Pro Pro Thr Lys Gln Arg Glu Arg Arg Lys Lys
            180                 185                 190
Lys Ala Arg Gln Gly Cys Ala Ser Leu Gly Thr Leu Ser Arg Arg Lys
        195                 200                 205
Leu Ala Cys Phe Lys
    210

<210> 391
<211> 565
<212> DNA
<213> Nicotiana tabacum

<220>
<221> CDS
<222> (71)..(565)

<400> 391
ctttttccaa tttcaactcc ataaaactaa gaagctagtg ttcttctttc ttatctttct      60

aattgatgag atg aat act agt agt agt gcc agt aat agt gct tct act      109
           Met Asn Thr Ser Ser Ser Ala Ser Asn Ser Ala Ser Thr
           1               5                   10
gca aca gga aca aag ttc atc aaa tgt gtg aca gtt gga gat ggt gct      157
Ala Thr Gly Thr Lys Phe Ile Lys Cys Val Thr Val Gly Asp Gly Ala
    15                  20                  25
gtt ggc aag act tgc ctt ctc atc tcc tac act agc tgc ctt ctc atc      205
Val Gly Lys Thr Cys Leu Leu Ile Ser Tyr Thr Ser Cys Leu Leu Ile
30                  35                  40                  45
tcc tac act agc aac act ttt cca act gat tat gtg cca act gtt ttt      253

Ser Tyr Thr Ser Asn Thr Phe Pro Thr Asp Tyr Val Pro Thr Val Phe
                50                  55                  60
gac aat ttc agt gct aat gtc aat gtt gat ggg aag att gtg aat ttg 301
Asp Asn Phe Ser Ala Asn Val Asn Val Asp Gly Lys Ile Val Asn Leu
            65                  70                  75
ggt ctt tgg gat act gct ggt caa gag gat tat aac agg ctt agg cct 349
Gly Leu Trp Asp Thr Ala Gly Gln Glu Asp Tyr Asn Arg Leu Arg Pro
        80                  85                  90
ctt agt tat cga gga gct gac gtc ttc ttg ctt gca ttc tct ctc ata 397
Leu Ser Tyr Arg Gly Ala Asp Val Phe Leu Leu Ala Phe Ser Leu Ile
    95                  100                 105
agt agg cct agc ttt gaa aat ata tca aaa aag tgg gtt cct gag cta 445
Ser Arg Pro Ser Phe Glu Asn Ile Ser Lys Lys Trp Val Pro Glu Leu
110                 115                 120                 125
aga cat tat gcc cca tca gtg cct att gtt ctt gtg ggg act aaa ttg 493
Arg His Tyr Ala Pro Ser Val Pro Ile Val Leu Val Gly Thr Lys Leu
                130                 135                 140
gat tta aga gag gac aag cag ttt aga agg gac tac cct ggt gca tct 541
Asp Leu Arg Glu Asp Lys Gln Phe Arg Arg Asp Tyr Pro Gly Ala Ser
            145                 150                 155
aca att tca aca gaa cag ggc tag 565
Thr Ile Ser Thr Glu Gln Gly
        160

<210> 392
<211> 164
<212> PRT
<213> Nicotiana tabacum

<400> 392
Met Asn Thr Ser Ser Ser Ala Ser Asn Ser Ala Ser Thr Ala Thr Gly
1               5                   10                  15
Thr Lys Phe Ile Lys Cys Val Thr Val Gly Asp Gly Ala Val Gly Lys
            20                  25                  30
Thr Cys Leu Leu Ile Ser Tyr Thr Ser Cys Leu Leu Ile Ser Tyr Thr
        35                  40                  45
Ser Asn Thr Phe Pro Thr Asp Tyr Val Pro Thr Val Phe Asp Asn Phe
    50                  55                  60
Ser Ala Asn Val Asn Val Asp Gly Lys Ile Val Asn Leu Gly Leu Trp
65                  70                  75                  80
Asp Thr Ala Gly Gln Glu Asp Tyr Asn Arg Leu Arg Pro Leu Ser Tyr
                85                  90                  95
Arg Gly Ala Asp Val Phe Leu Leu Ala Phe Ser Leu Ile Ser Arg Pro
            100                 105                 110
Ser Phe Glu Asn Ile Ser Lys Lys Trp Val Pro Glu Leu Arg His Tyr
        115                 120                 125
Ala Pro Ser Val Pro Ile Val Leu Val Gly Thr Lys Leu Asp Leu Arg
    130                 135                 140
Glu Asp Lys Gln Phe Arg Arg Asp Tyr Pro Gly Ala Ser Thr Ile Ser
145                 150                 155                 160
Thr Glu Gln Gly

<210> 393
<211> 1057
<212> DNA
<213> Nicotiana tabacum

<220>
<221> CDS
<222> (176)..(808)

<400> 393
ggcacgagaa gctattaatt ccaagctatc tccacaccct ctccccaaac aagaaaatta 60

ttaaatgtaa aacgtactac tagaccccca gttacataca taaacacaca tcatctgcag 120

aattttggta tgcaatttta cgtactttct tgaggtattt ttttttttat gttca atg 178
Met
1
gcc tca agt gct tca aga ttc atc aaa tgt gtc acg gtt ggt gat ggt 226
Ala Ser Ser Ala Ser Arg Phe Ile Lys Cys Val Thr Val Gly Asp Gly
            5                   10                  15
gcc gtt gga aag act tgt atg ctt att tgc tat acc agt aac aag ttc 274
Ala Val Gly Lys Thr Cys Met Leu Ile Cys Tyr Thr Ser Asn Lys Phe
        20                  25                  30
ccc act gat tat gtt ccc aca gtg ttt gac aac ttc agt gcc aat gtg 322
Pro Thr Asp Tyr Val Pro Thr Val Phe Asp Asn Phe Ser Ala Asn Val
    35                  40                  45
gtt gtc gaa ggg acc aca gta aat tta ggt ctt tgg gat act gca ggc 370
Val Val Glu Gly Thr Thr Val Asn Leu Gly Leu Trp Asp Thr Ala Gly
50                  55                  60                  65
caa gaa gat tat aac aga tta agg cca ctg agc tac cga gga gca gat 418
Gln Glu Asp Tyr Asn Arg Leu Arg Pro Leu Ser Tyr Arg Gly Ala Asp
                70                  75                  80
gtt ttt gtc cta gcg ttc tcc ttg gtt agt cgc gca agc tac gag aac 466
Val Phe Val Leu Ala Phe Ser Leu Val Ser Arg Ala Ser Tyr Glu Asn
            85                  90                  95
ata ctt aaa aag tgg att cct gaa ctt cag cat tat gct cct gga ata 514
Ile Leu Lys Lys Trp Ile Pro Glu Leu Gln His Tyr Ala Pro Gly Ile
        100                 105                 110
ccg gtg gta tta gct ggc acc aaa ctt gat ctt cgt gag gat aag cac 562
Pro Val Val Leu Ala Gly Thr Lys Leu Asp Leu Arg Glu Asp Lys His
    115                 120                 125
ttc ttg gct gat cat cct gga tta gtt cct gtc acc acc gcy cag gga 610
Phe Leu Ala Asp His Pro Gly Leu Val Pro Val Thr Thr Ala Gln Gly
130                 135                 140                 145
gag gag cta cgg aaa caa att ggt gct gcc tat tac atc gaa tgt agc 658
Glu Glu Leu Arg Lys Gln Ile Gly Ala Ala Tyr Tyr Ile Glu Cys Ser
                150                 155                 160
tct aaa aca caa cag aat gtg aaa gct gtc ttt gat gct gca atc aag 706
Ser Lys Thr Gln Gln Asn Val Lys Ala Val Phe Asp Ala Ala Ile Lys
            165                 170                 175
gtc gtc atc aag cca ccg cag aag caa aag gag aag aag aaa caa cgt 754
Val Val Ile Lys Pro Pro Gln Lys Gln Lys Glu Lys Lys Lys Gln Arg
        180                 185                 190
cga gga tgt ctc atg aat gtg atg tgc gga agg aag ctc gtt tgt ttg 802
Arg Gly Cys Leu Met Asn Val Met Cys Gly Arg Lys Leu Val Cys Leu
    195                 200                 205
aag tgacatttca atgtacatat ttcccatctg ttcggcctct aacatgattt 855
Lys
210
atatccatgg ctttagcgtg ctgcctataa aacttacatt tctatcagaa cttttgtgac 915

gagcataatg tatttgtttt tccataaccc aacttactgc tggacggcct tgatttgtct 975

gtaaaatacg ttacttgcta ggtttatatt tcaagtacat gtattaattc tattaaacca 1035

acaaaaaaaa aaaaaaaaaa aa 1057

<210> 394
<211> 210
<212> PRT
<213> Nicotiana tabacum

<400> 394
Met Ala Ser Ser Ala Ser Arg Phe Ile Lys Cys Val Thr Val Gly Asp

1               5                   10                  15
Gly Ala Val Gly Lys Thr Cys Met Leu Ile Cys Tyr Thr Ser Asn Lys
            20                  25                  30
Phe Pro Thr Asp Tyr Val Pro Thr Val Phe Asp Asn Phe Ser Ala Asn
        35                  40                  45
Val Val Val Glu Gly Thr Thr Val Asn Leu Gly Leu Trp Asp Thr Ala
    50                  55                  60
Gly Gln Glu Asp Tyr Asn Arg Leu Arg Pro Leu Ser Tyr Arg Gly Ala
65                  70                  75                  80
Asp Val Phe Val Leu Ala Phe Ser Leu Val Ser Arg Ala Ser Tyr Glu
                85                  90                  95
Asn Ile Leu Lys Lys Trp Ile Pro Glu Leu Gln His Tyr Ala Pro Gly
            100                 105                 110
Ile Pro Val Val Leu Ala Gly Thr Lys Leu Asp Leu Arg Glu Asp Lys
        115                 120                 125
His Phe Leu Ala Asp His Pro Gly Leu Val Pro Val Thr Thr Ala Gln
    130                 135                 140
Gly Glu Glu Leu Arg Lys Gln Ile Gly Ala Ala Tyr Tyr Ile Glu Cys
145                 150                 155                 160
Ser Ser Lys Thr Gln Gln Asn Val Lys Ala Val Phe Asp Ala Ala Ile
                165                 170                 175
Lys Val Val Ile Lys Pro Pro Gln Lys Gln Lys Glu Lys Lys Lys Gln
            180                 185                 190
Arg Arg Gly Cys Leu Met Asn Val Met Cys Gly Arg Lys Leu Val Cys
        195                 200                 205
Leu Lys
    210

<210> 395
<211> 24
<212> DNA
<213> Primer

<400> 395
atgtctgaaa aggccgttag aagg                                          24

<210> 396
<211> 26
<212> DNA
<213> Primer

<400> 396
ttataaaatt atgcaacagt tagccc                                        26

<210>  397

<211>  20

<212>  PRT

<213>  consensus

<220>

<221>  MISC_FEATURE

<222>  (1)..(20)

<223>  Xaa in postion 2 is any amino acid, Xaa in postion 4 is Val, Thr,
       Ile or Leu, Xaa in position 5 is Val, Ile or Leu, Xaa in position
       9 is Ala or Gly, Xaa in position 10 is any amino acid, Xaa in pos
       ition 14 is Cys, Ser or Ala, Xaa in position 15 is Leu or Met, Xa
       a in position 16 is Leu or Met, Xaa in position 17 is any amino a
       cid, Xaa in position 18 is any amino acid, Xaa in position 19 is
       Tyr or Phe, Xaa in position 20 is Thr, Ser or Ala.

<400> 397

Lys Xaa Val Xaa Xaa Gly Asp Gly Xaa Xaa Gly Lys Thr Xaa Xaa Xaa
1               5                   10                  15

Xaa Xaa Xaa Xaa
            20


<210> 398

<211> 13

<212> PRT

<213> consensus



<220>

<221> MISC_FEATURE

<222> (1)..(13)

<223> Xaa in postion 1 is Phe or Tyr, Xaa in position 3 is any amino ac
      id, Xaa in position 4 is any amino acid, Xaa in position 6 is Val,
      Ile or Glu, Xaa in position 9 is Val or Ile, Xaa in position 11 is
       Asp or Glu, Xaa in position 12 is any amino acid, Xaa in position
      13 is Tyr or Phe.


<400> 398

Xaa Pro Xaa Xaa Tyr Xaa Pro Thr Xaa Phe Xaa Xaa Xaa
1               5                   10


<210> 399

<211> 22

<212> PRT

<213> consensus



<220>

<221> MISC_FEATURE

<222> (1)..(22)

<223> Xaa in postion 1 is Val, Ile or Tyr, Xaa in position 2 is any amin
      o acid, Xaa in position 3 is Leu, Val or Ile, Xaa in position 4 is
       any amino acid, Xaa in position 5 is Leu, Ile or Met, Xaa in posi
      tion 6 is Trp or Phe, Xaa in position 9 is Ala or Ser, Xaa in posi
      tion 12 is Glu or Asp, Xaa in position 13 is Asp or Glu, Xaa in po
      sition 14 is Tyr or Phe, Xaa in position 15 is any amino acid, Xaa
       in position 16 is Arg, Lys or Asn, Xaa in position 17 is Leu, Ile
       or Val, Xaa in position 19 is Pro, Ser or Thr, Xaa in position 20
       is Leu or Met, Xaa in position 21 is Ser, Ala or Phe.

<400>  399

Xaa Xaa Xaa Xaa Xaa Xaa Asp Thr Xaa Gly Gln Xaa Xaa Xaa Xaa Xaa
1               5                   10                  15

Xaa Arg Xaa Xaa Xaa Tyr
            20


<210>  400

<211>  143

<212>  PRT

<213>  consensus



<220>

<221>  MISC_FEATURE

<222>  (1)..(143)

<223>  Xaa in all postions is any amino acid.



<400>  400

Lys Xaa Val Xaa Val Gly Asp Gly Ala Xaa Gly Lys Thr Cys Leu Leu
1               5                   10                  15


Ile Xaa Xaa Xaa Xaa Xaa Xaa Xaa Xaa Xaa Xaa Xaa Xaa Xaa Xaa Phe
            20                  25                  25

Pro Xaa Xaa Tyr Xaa Pro Thr Val Phe Xaa Asn Xaa Xaa Xaa Xaa Xaa
        30                  35                  40

Xaa Xaa Xaa Xaa Xaa Xaa Xaa Xaa Xaa Xaa Leu Xaa Leu Trp Asp Thr
    45                  50                  55

Ala Gly Gln Glu Asp Tyr Xaa Arg Leu Arg Pro Leu Ser Tyr Xaa Xaa
60                  65                  70                  75

Xaa Xaa Xaa Xaa Xaa Xaa Xaa Xaa Xaa Xaa Xaa Xaa Xaa Xaa Xaa Xaa
                80                  85                  90

Xaa Xaa Xaa Xaa Asp Val Xaa Xaa Xaa Xaa Phe Ser Xaa Xaa Xaa Xaa
            95                  100                 105

Xaa Ser Xaa Xaa Asn Xaa Xaa Xaa Lys Trp Xaa Pro Glu Xaa Xaa Xaa
        110                 115                 120

Xaa Xaa Xaa Pro Xaa Xaa Pro Xaa Xaa Leu Val Gly Xaa Lys Xaa Asp
    125                 130                 135

Leu Arg Xaa Asp
140

## Description

The present invention relates to a process for the production of the fine chemical in a microorgansm, a plant cell, a plant, a plant tissue or in one or more parts thereof. The invention furthermore relates to nucleic acid molecules, polypeptides, nucleic acid constructs, vectors, antisense molecules, antibodies, host cells, plant tissue, propagtion material, harvested material, plants, microorganisms as well as agricultural compositions and to their use.

Certain products and by-products of naturally-occurring metabolic processes in cells have utility in a wide array of industries, including, but not limited to, the food, feed, cosmetics, and pharmaceutical industries and agriculture. These molecules, collectively termed 'fine chemicals' include molecules such as vitamins for example vitamin A, D, E, K, B₁, B₂, B₆. B₁₂, C, pantothenic acid, biotin or folic acid; substances with vitamin-like character for example vitamin F, lipoic acid, ubiquinones, choline, myoinsositiol, vitamin U (S-methylmethionine), flavours for example vanillin, coumarin, isoeugenol, eugenol, (R)-carvone, (S)-carvone, menthol, jasmone or farnesol; nutraceuticals for example phytosterols, flavonoids, anthocyanidins, isoflavons or isoprenoids; detergents; fatty acids such as saturated fatty acids, mono unsaturated fatty acids (singular MUFA, plural MUFAS), poly unsaturated fatty acids (singular PUFA, plural PUFAS), waxes or lipids containing said fatty acids; carbohydrates for example cellulose, starch, dextrin, pectin, xanthangum, carrageenan or alginate; sugars for example monosaccharides such as glucose, fructose, manose, sorbose, ribose, ribulose, xylose, xylulose or galactose, disaccharides such as lactose, sucrose, saccharose, maltose, isomaltose or cellobiose, trisaccharides such as raffinose or maltotriose; carboxylic acids for example citric acid, α-ketoglutaric acid, ferulic acid, sinapic acid or lactic acid; carotinoids for example α-carotene, β-carotene, zeaxanthine, lutein, astaxanthine, lycopene, phyotoene or phytofluene, amino acids for example lysine, threonine, methionine, tryptophane, phenylalanine or tyrosine, cofactors for example heme or quinines, enzymes for example lipases, esterases, proteases, amylases, glucosidases etc. and other compounds [as described e.g. in Kuninaka, A. (1996) Nucleotides and related compounds, p. 561-612, in Biotechnology vol. 6, Rehm et al., eds. VCH: Weinheim, in Industial Microbiology and Biotechnology, Demain et al., second edition, ASM Press Washington, D.C. 1999, in Ullmann's Encyclopedia of Industrial Chemistry, vol. A27, Vitamins, p. 443-613 (1996) VCH: Weinheim and Ong, A.S., Niki, E. & Packer, L. (1995) Nutrition, Lipids, Health, and Disease Proceedings of the UNESCO/Confederation of Scientific and Technological Associations in Malaysia, and the Society for Free Radical Research, Asia, held Sept. 1-3, 1994 at Penang, Malaysia, AOCS Press, (1995)), enzymes, and all other chemicals described in Gutcho (1983) Chemicals by Fermentation, Noyes Data Corporation, ISBN: 0818805086 and references contained therein]. Carotinoids are added for example to soft drinks, margarines or to animal feed for example to colour egg yolk or the flesh of fish. In the food industry polycarbohydrates are widely used as thickener. Polyunsaturated fatty acids are added for example to infant formulas to create a higher nutrition value of such formulas. PUFAs have for example a positive influence on the cholesterol level of the blood in humans and therefore are useful in the protection of heart diseases. Fine chemicals for example PUFAS can be isolated from animal sources such as for example fish or produced with microorganisms through the large-scale culture of microorganisms developed to produce and accumulate or secrete large quantities of one or more desired molecules.

In large scale fine chemicals are produced with microorganism in the fermentation industry, which is responsible for the manufacturing of at least five major ingredient categories: antibiotics, organic acids, amino acids, enzymes, vitamins and other related products. There are production facilities in all important areas of the world especially in Europe, the US and Asia. Companies continuously try to optimize the production processes, the organisms and thereby increasing the efficiency but, as in the case of amino acids and organic acids, with already high conversion rates based on feeded carbon source, the limitations of such work become evident. All fermentation processes depend on the efficient utilization of carbohydrates, supplied mainly in the form of oils, glucose or molasses. It is therefore the availability and pricing of these raw materials that influence the competitiveness of fermentation products versus production for example in plants. Amino acids, organic acids and vitamins are offered at very low prices. For such products the question is whether it is still economical to continue fermentation production in future. And that is frequently a question of comparing the availability and pricing of carbohydrates with the future markets.

Particularly useful organisms for the production of fine chemicals are microorganisms such as the algae, fungi, bacteria or plants. Through strain selection, a number of mutant strains of microorganisms have been developed which produce an array of desirable compounds including vitamins, amino acids, PUFAs etc. However, selection of strains improved for the production of a particular molecule is a time-consuming and difficult process.

Alternatively the production of fine chemicals can be most conveniently performed via the large scale production of plants developed to produce for example carotinoids, carbohydrates or PUFAS. For example for the production of carotinoids plants such as marigold are used. Particularly well-suited plants for this purpose are sugar producing plants such as sugar beet or sugar cane or oilseed plants containing high amounts of lipid compounds such as rapeseed, canola, linseed, soybean, sunflower, borage and evening primrose. But also other crop plants containing sugars, oils or lipids and fatty acids are well suited as mentioned in the detailed description of this invention. Through conventional breeding, a number of mutant plants have been developed which produce an array of desirable lipids and fatty acids, cofactors and enzymes. However, selection of new plant cultivars improved for the production of a particular molecule is a time-consuming and difficult process or even impossible if the compound does not naturally occur in the respective plant as for example in the case of C₂₀ and higher C-carbon chain polyunsaturated fatty acids.

Carbohydrates are an important dietary nutrient, which is mostly used to supply energy to the body, as well as, a carbon source for synthesis of other compounds such as fats or proteins. Furthermore mono- and disaccharides are widely used in the food and feed industry as sweetener. Saccharides have varying degrees of sweetness on a relative scale. Fructose is the sweetest. For example in the United States 22 million tons of sugar and other sweeteners were consumed in 1999. Said natural sweetener consumption includes the consumption of sugar, corn sweeteners such as high fructose corn syrup as main product and others such as honey or maple syrup. All natural sugar based sweeteners have a market shar of around 36 to 40 percent.

Whether unsaturated or saturated fatty acids are preferred in the food and feed industry depends on the intended purpose; thus, for example, lipids with unsaturated fatty acids, specifically polyunsaturated fatty acids, are preferred in human nutrition since they have a positive effect on the cholesterol level in the blood and thus on the possibility of heart disease. They are used in a variety of dietetic foodstuffs or medicaments. In addition PUFAs are commonly used in food, feed and in the cosmetic industry. Poly unsaturated ω-3- and/or ω-6-fatty acids are an important part of animal feed and human food. Because of the common composition of human food poly unsaturated ω-3-fatty acids, which are an essential component of fish oil, should be added to the food to increase the nutritional value of the food; thus, for example, poly unsaturated fatty acids such as docosahexaenoic acid or eicosapentaenoic acid are added as mentioned above to infant formula to increase its nutritional value.

Vitamins such as vitamin C, vitamin B12 or vitamin B2 are typically produced with microorganism as mentioned above in the fermentation industry. Vitamin C can be produced generally in a combined process using biotransformation steps in combination with classical chemical synthesis. In another production process vitamin C is produced by fermentation alone. In general organisms such as Arthrobacter, Gluconobacter, Corynebacterium, Brevibacterium or Erwinia are used for vitamin C production. Vitamin B2 and vitamin B12 are produced with organisms such as Bacillus, Streptomyces, Citrobacter, Klebsiella, Propionibacterium or Ashbya in large scale fermentation.

Commonly vitamin E and A are procuded in a classical chemical process or isolated from as natural vitamin E from plant oils. Vitamin E is an important natural fat-soluble antioxidants. As such, vitamin E protects cell membranes from the damage caused by free radicals. High doses of vitamin E have also been linked to a decreased ability of the blood to clot, which may be beneficial in those individuals at risk for heart disease by reducing the risk of heart attack. A vitamin E deficiency leads to pathophysiological situations in humans and animals. Of the different types of vitamin E, the alpha tocopherol form is typically considered the "gold standard" in terms of antioxidant activity - although the most recent research suggests that the other chemical forms may possess equivalent or superior antioxidant protection. Vitamin E compounds therefore are of high economical value as additives in the food and feed sectors, in pharmaceutical formulations and in cosmetic applications. Vitamin A is another fat-soluble vitamin that is part of a family of compounds including retinol, retinal and beta-carotene. Beta-carotene is also known as pro-vitamin A because it can be converted into vitamin A when additional levels are required. Vitamin A is needed by all of the body's tissues for general growth and repair processes and is especially important for bone formation, healthy skin/hair, night vision and function of the immune system. Vitamin A may help boost immune system function and resistance to infection. Vitamin A derivatives are widely used in cosmetics and dermatological treatments for skin preparations designed to combat skin aging and treat acne. Vitamin A has been used for decades as a treatment for various vision-related conditions, including night blindness, cataracts, conjunctivitis, retinopathy and macular degeneration.

An economical method for producing of vitamins such as vitamin C, B2, B12 or vitamin E and food- and feedstuffs with increased vitamin content are therefore very important. Particularly economical methods are biotechnological methods utilizing vitamin-producing organisms, which are either natural or optimized by genetic modification.

Carotenoids are a large family of compounds including over 600 members such as β-carotene, lycopene or lutein. Carotenoids are widely distributed in fruits and vegetables and are responsible, along with flavonoids, for contributing the color to many plants (a rule of thumb is the brighter, the better). In terms of nutrition, β-carotene's primary role is as mentioned above a precursor to vitamin A. β-carotene as most other carotenoids, is a powerful antioxidant - so it has been recommended to protect against a variety of diseases such as cancer, cataracts and heart disease.

The introduction of a new gene or new genes for the synthesis of fine chemicals into an organism or cell may not just increase the biosynthetic flux towards an end product it may also increase or create de novo a new compound composition. Similarly, other genes involved in the import of nutrients necessary for the biosynthesis of one or more fine chemicals (e.g., fatty acids, polar and neutral lipids, vitamins, enzymes etc.) may be increased in number or activity such that these precursors, cofactors, or intermediate compounds are increased in concentration within the cell or within the storing compartment thus increasing further the capability of the cell to produce the fine chemical as described herein.

Amino acids are used in many branches of industry, including the food, animal feed, cosmetics, pharmaceutical and chemical industries. Amino acids such as D,L-methionine, L-lysine or L-threonine are used in the animal feed industry. The essential amino acids valine, leucine, isoleucine, lysine, threonine, methionine, tyrosine, phenylalanine and tryptophan are particularly important for the nutrition of mammals especially humans and a number of livestock species. Glycine, L-methionine and tryptophan are all used in the pharmaceutical industry. Glutamine, valine, leucine, isoleucine, histidine, arginine, proline, serine and alanine are used in the pharmaceutical and cosmetics industries. Threonine, tryptophan and D,L-methionine are widely used feed additives (Leuchtenberger, W. (1996) Amino acids - technical production and use, pp. 466-502 in Rehm et al., (Ed.) Biotechnology vol. 6, chapter 14a, VCH Weinheim). Moreover, amino acids are suitable for the chemical industry as precursors for the synthesis of synthetic amino acids and proteins, such as N-acetylcysteine, S-carboxymethyl-L-cysteine, (S)-5-hydroxytryptophan and other subtances described in Ullmann's Encyclopedia of Industrial Chemistry, vol. A2, pp. 57-97, VCH Weinheim, 1985. To prefent physiological malnutritions the human body has a need for essentiall amino acids such as arginine, histidine, isoleucine, leucine, lysine, methionine, tyrosine, phenylalanine, threonine, tryptophan, and valine. Based on their content of amino acids, foods are often classified as complete, partially complete, or incomplete protein sources. In order for a protein to be complete, it must contain all of the essential amino acids. This is the reason that many nutritionists rank non-meat foods as being incomplete. The foods do contain all amino acids, but some may be in lower proportions than are required, and, therefore, should be combined with another food containing higher amounts of these amino acids or should be supplemented with said essential amino acids.

[0014.0.0.0] Over one million tonnes of amino acids are currently produced annually; their market value amounts to over 2.5 billion US dollars. They are currently produced by four competing processes: Extraction from protein hydrolysates, for example L-cystine, L-leucine or L-tyrosine, chemical synthesis, for example of D,L-methionine, conversion of chemical precursors in an enzyme or cell reactor, for example L-phenylalanine, and fermentative production by growing, on an industrial scale, bacteria which have been developed to produce and secrete large amounts of the desired molecule in question. An organism, which is particularly suitable for this purpose is Corynebacterium glutamicum, which is used for example for the production of L-lysine or L-glutamic acid. Other amino acids which are produced by fermentation are, for example, L-threonine, L-tryptophan, L-aspartic acid and L-phenylalanine.

The biosynthesis of the natural amino acids in organisms capable of producing them, for example bacteria, has been characterizied thoroughly; for a review of the bacterial amino acid biosynthesis and its regulation, see Umbarger, H.E. (1978) Ann. Rev. Biochem. 47: 533 - 606].

It is known that amino acids are produced by fermentation of strains of coryneform bacteria, in particular Corynebacterium glutamicum. Due to their great importance, the production processes are constantly being improved. Process improvements can relate to measures regarding technical aspects of the fermentation, such as, for example, stirring and oxygen supply, or the nutrient media composition, such as, for example, the sugar concentration during fermentation, or to the work-up to give the product, for example by ion exchange chromatography, or to the intrinsic performance properties of the microorganism itself. Bacteria from other genera such as Escherichia or Bacillus are also used for the production of amino acids. A number of mutant strains, which produce an assortment of desirable compounds from the group of the sulfur-containing fine chemicals have been developed via strain selection. The performance properties of said microorganisms are improved with respect to the production of a particular molecule by applying methods of mutagenesis, selection and mutant selection. Methods for the production of methionine have also been developed. In this manner, strains are obtained which are, for example, resistant to antimetabolites, such as, for example, the methionine analogues α-methylmethionine, ethionine, norleucine, N-acetylnorleucine, S-trifluoromethylhomocysteine, 2-amino-5-heprenoitic acid, selenomethionine, methionine sulfoximine, methoxine, 1-aminocyclopentanecarboxylic acid or which are auxotrophic for metabolites with regulatory importance and which produce sulfur-containing fine chemicals such as, for example, L-methionine. However, such processes developed for the production of methionine have the disadvantage that their yields are too low for being economically exploitable and that they are therefore not yet competitive with regard to chemical synthesis.

Zeh (Plant Physiol., Vol. 127, 2001: 792-802) describes increasing the methionine content in potato plants by inhibiting threonine synthase by what is known as antisense technology. This leads to a reduced threonine synthase activity without the threonine content in the plant being reduced. This technology is highly complex; the enzymatic activity must be inhibited in a very differentiated manner since otherwise auxotrophism for the amino acid occurs and the plant will no longer grow.

US 5,589,616 teaches the production of higher amounts of amino acids in plants by overexpressing a monocot storage protein in dicots. WO 96/38574, WO 97/07665, WO 97/28247, US 4,886,878, US 5,082,993 and US 5,670,635 are following this approach. That means in all the aforementioned intellectual property rights different proteins or polypeptides are expressed in plants. Said proteins or polypeptides should function as amino acid sinks. Other methods for increasing amino acids such as lysine are disclosed in WO 95/15392, WO 96/38574, WO 89/11789 or WO 93/19190. In this cases speziell enzymes in the amino acid biosynthetic pathway such as the diphydrodipicolinic acid synthase are deregulated. This leads to an increase in the production of lysine in the different plants. Another approach to increase the level of amino acids in plants is disclosed in EP-A-0 271 408. EP-A-0 271 408 teaches the mutagenensis of plant and selection afterwards with inhibitors of certain enzymes of amino acid biosynthetic pathway.

Methods of recombinant DNA technology have also been used for some years to improve Corynebacterium strains producing L-amino acids by amplifying individual amino acid biosynthesis genes and investigating the effect on amino acid production.

As described above, the essential amino acids are necessary for humans and many mammals, for example for livestock. L-methionine is important as methyl group donor for the biosynthesis of, for example, choline, creatine, adrenaline, bases and RNA and DNA, histidine, and for the transmethylation following the formation of S-adenosylmethionine or as a sulfhydryl group donor for the formation of cysteine. Moreover, L-methionine appears to have a positive effect in depression. [0021.0.0.0] improving the quality of foodstuffs and animal feeds is an important task of the food-and-feed industry. This is necessary since, for example, certain amino acids, which occur in plants are limited with regard to the supply of mammals. Especially advantageous for the quality of foodstuffs and animal feeds is as balanced as possible an amino acid profile since a great excess of an amino acid above a specific concentration in the food has no further positive effect on the utilization of the food since other amino acids suddenly become limiting. A further increase in quality is only possible via addition of further amino acids, which are limiting under these conditions. The targeted addition of the limiting amino acid in the form of synthetic products must be carried out with extreme caution in order to avoid amino acid imbalance. For example, the addition of an essential amino acid stimulates protein digestion, which may cause deficiency situations for the second or third limiting amino acid, in particular. In feeding experiments, for example casein feeding experiments, the additional provision of methionine, which is limiting in casein, has revealed the fatty degeneration of liver, which could only be alleviated after the additional provision of tryptophan.

To ensure a high quality of foods and animal feeds, it is therefore necessary to add fine chemicals that means a plurality of compounds such as amino acids, vitamins, organic acids, PUFAS etc. in a balanced manner to suit the organism. Such supplemented food is named as "functional foods" or "nutraceuticals". Nutraceuticals shall provide a health benefit to humans beyond basic nutrition. Functional foods have health-promoting or disease-preventing effects. Examples include omega-3 fatty acids (found in many fish, flaxseed oil, soybean oil, canola oil, and walnuts), which reduce risk of coronary heart disease and lycopene in tomatoes, which has been associated with reduced risk of certain cancers.

From a practical standpoint it would be of great advantage to produce an organism such as a microorganism or a plant containing a combination of different fine chemicals such as amino acids, vitamins, organic acids, carotenoids, PUFAS etc. at the same time in an sufficient amount to provide optimal growth and health benefit to animals or humans instead of combining different food or supplementing food or feed with different fine chemicals.

It is therefore an object of the present invention to develop an inexpensive process for the synthesis of a combination of fine chemicals such as amino acids like tryptophane, proline, arginine, phenylalanine, tyrosine, alanine, glycine, threonine, serine, valine, isoleucine or leucine especially essential amino acids such as tryptophane, arginine, phenylalanine, tyrosine, threonine, valine, isoleucine or leucine, carbohydrates such as raffinose, maltose or inositol, vitamins such as y-, α- or β-tocopherol, organic acids such as ferulic acid, malate or sinapic acid, carotenoids such as β-carotene etc. at the same time in an sufficient amount to provide optimal growth and health benefit to animals or humans

It was now found that this object is achieved by providing the process according to the invention described herein and the embodiments characterized in the claims.

Accordingly, in a first embodiment, the invention relates to a process for the production of a fine chemical, whereby the fine chemical is at least one compound selected from the group consisting of tryptophane, proline, arginine, phenylalanine, tyrosine, alanine, glycine, threonine, serine, valine, isoleucine, leucine, raffinose, maltose, inositol, ferulic acid, malate, γ- tocopherol, α- tocopherol, β-tocopherol, cerotic acid, lignoceric acid, putrescine, sinapic acid, 3,4-dihydroxyphenylaline (= DOPA), stearic acid and β-carotene. Accordingly, in the present invention, the term "the fine chemical" as used herein relates to an amino acid selected from the group consisting of tryptophane, proline, arginine, phenylalanine, tyrosine, alanine, glycine, threonine, serine, valine, isoleucine and leucine; a vitamin selected from the group consisting of γ-tocopherol, α- tocopherol and β-tocopherol, a fatty acid selected from the group consisting of cerotic acid, lignoceric acid and stearic acid, an organic acid selected from the group consisting of ferulic acid, malate and sinapic acid or a compound selected from the group consisting of raffinose, putrescine, 3,4-dihydroxyphenylaline (= DOPA) and β-carotene or mixtures thereof containing at least two, three, four or five compounds selected from the aforementioned groups, preferably 6, 7, 8 or 9 compounds selected from the aforementioned groups, most preferably 10, 11, 12, 13, 14, 15, 16, 17 or more compounds selected from the aforementioned groups. Further, the term "the fine chemicals" as used herein also relates to fine chemicals comprising at least one compound selected from the group consisting of tryptophane, proline, arginine, phenylalanine, tyrosine, alanine, glycine, threonine, serine, valine, isoleucine, leucine, raffinose, maltose, inositol, ferulic acid, malate, γ- tocopherol, α- tocopherol, β-tocopherol, cerotic acid, lignoceric acid, putrescine, sinapic acid, 3,4-dihydroxyphenylaline (= DOPA), stearic acid and β-carotene.

In one embodiment, the term "the fine chemical" or "fine chemical" means at least one compound selected from the group consisting of L-tryptophane, L-proline, L-arginine, L-phenylalanine, L-tyrosine, L-alanine, glycine, L-threonine, L-serine, L-valine, L-isoleucine, L-leucine, raffinose, maltose, inositol, ferulic acid, malate, γ-tocopherol, α- tocopherol, β-tocopherol, cerotic acid, lignoceric acid, putrescine, sinapic acid, 3,4-dihydroxyphenylaline (= DOPA), stearic acid and β-carotene. Throughout the specification the term "the fine chemical" means the aforementioned compounds, its salts, ester or amids in free form or bound to other chemical compounds especially proteins. In a preferred embodiment, the term "the fine chemical" or "fine chemical" means at least one compound selected from the group consisting of L-tryptophane, L-proline, L-arginine, L-phenylalanine, L-tyrosine, L-alanine, glycine, L-threonine, L-serine, L-valine, L-isoleucine and L-leucine in free form or its salts or bound to proteins.

Accordingly, the present invention relates to a process for the production of fine chemical comprising
(a) increasing or generating the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 in a non-human organism, or in one or more parts thereof; and
(b) growing the organism under conditions which permit the production of the fine chemical in said organism.

Comprises/comprising and grammatical variations thereof when used in this specification are to be taken to specify the presence of stated features, integers, steps or components or groups thereof, but not to preclude the presence or addition of one or more other features, integers, steps, components or groups thereof.

Preferably, this process further comprises the step of recovering the fine chemical, which is synthesized by the organism from the organism and/or from the culture medium used for the growth or maintainance of the organism. The term "recovering" means the isolation of the fine chemical in different purities, that means on the one hand harvesting of the biological material, which contains the fine chemical without further purification and on the other hand purities of the fine chemical between 5% and 100% purity, preferred purities are in the range of 10% and 99%. In one embodiment, the purities are 20%, 30%, 40%, 50%, 60%, 70%, 80%, 90%, 95% or 99% or more.

Advantageously the process for the production of the fine chemical leads to an enhanced production of the fine chemical. The terms "enhanced" or "increase" mean at least a 10%, 20%, 30%, 40% or 50%, preferably at least 60%, 70%, 80%, 90% or 100%, more preferably 150%, 200%, 300%. 400% or 500% higher production of the fine chemical in comparison to the reference as defined below, e.g. that means in comparison to an organism without the aforementioned modification of the activity of a protein having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394.

Surprisingly it was found, that the transgenic expression of the Kluyveromyces lactis, Cryptococcus neoformans, Neurospora crassa, Penicillium marneffei, Mucor rouxii, Schizophyllum commune, Paracoccidioides brasiliensis, Aspergillus fumigatus, Suillus bovinus, Candida albicans, Trichoderma reesei, Ashbya gossypii, Yarrowia lipolytica, Ustilago maydis, Emericella nidulans, Trichomonas vaginalis, Colletotrichum trifolii, Blumeria graminis, Dictyostelium discoideum, Saccaromyces cerevisiae, Schizosaccharomyces pombe, Entamoeba histolytica, Oryza sativa, Brassica napus, Glycine max, Beta vulgaris, Lotus japonicus, Zinnia elegans, Zea mays, Cicer arietinum, Arabidopsis thaliana, Hordeum vulgare, Nicotiana tabacum, Gossypium hirsutum, Physcomitrella patens, Fucus distichus, Medicago truncutula, Homo sapiens, Caenorhabditits elegans, Tigriopus japonicus, Rhopalosiphum padi, Mus musculus, Discopyge ommata, Canis lupus, Drosophila melanogaster, Anopheles gambiae, Aplysia california, Ciona savignyi, Ciona intestinalis, Hemicentrotus pulcherrimus, Giardia lamblia, Gallus gallus, Brachydanio rerio, Xenopus laevis, Xenopus tropicalis, Schistosoma japonicum, Schistosoma mansoni, Encephalitozoon cuniculi, Wuchereria bancrofti, Cavia porcellus, Sus scrofa, Rattus norvegicus, Pneumocystis carinii or Pagrus major proteins as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 for example in Arabidopsis thaliana conferred an increase in the fine chemical content of the transformed plants.

In accordance with the invention, the term "organism" as understood herein relates always to a non-human organism, in particular to an animal or plant organism or to a microorganism. Further, the term "animal" as understood herein relates always to a non-human animal.

The sequence depicted in SEQ ID NO: 1 (YNL090W) from Saccharomyces cerevisiae has been published in Madaule et al. [1987, "Characterization of two members of the rho gene family from the yeast Saccharomyces cerevisiae"; Proc. Natl. Acad. Sci., USA 84(3):779-83], and named as rho2. The gene encodes as all the other sequences mentioned above and use in the inventive process a non-essential GTPase of the rho/rac subfamily of the ras-like GTPases. The protein may play a role in the establishment of cell polarity or in microtubule assembly. Accordingly, in one embodiment, the process of the present invention comprises the use of a gene product "involved in in the establishment of cell polarity or in microtubule assembly" from Saccaromyces cerevisiae or its homolog, e.g. as shown herein, for the production of the fine chemical, meaning of preferably for the production of essential amino acids, in particular for increasing the amount of an essential amino acid in free or bound form in an organism or a part thereof, as mentioned herein. That means the increase of its biological activity by overexpression of the responsible gene leads to an increase of the fine chemical.

The term "biological activity" means the biological function of the protein of the invention. In contrast to the term "biological activity" the term "activity" means the increase in the production of the compound produced by the inventive process. The term "biological activity" preferably refers to for example the enzymatic function, transporter or carrier function, DNA-packaging function, heat shock protein function, recombination protein function or regulatory function of a peptide or protein in an organism, a tissue, a cell or a cell compartment. Suitable substrates are low-molecular-weight compounds and also the protein interaction partners of a protein. The term "increase" of the biological function refers, for example, to the increase in binding capacity or binding strength of a protein for at least one substrate in an organism, a tissue, a cell or a cell compartment - for example by one of the methods described herein below - in comparison with the wild type of the same genus and species to which this method has not been applied, under otherwise identical conditions (such as, for example, culture conditions, age of the plants and the like). Increase is also understood as meaning the modification of the substrate specificity as can be expressed for example, by the kcat/Km value. In this context, an increase of the function of at least 10%, advantageously of at least 20%, preferably at least 30%, especially preferably of at least 40%, 50%, 60 %, 70%, 80%, 90% or more, very especially preferably of at least 150%, 200%. 250%, 300% or more, in comparison with the untreated organism is advantageous.

Homologues (= homologs) of the present gene products can be derived from any organisms as long as the homologue confers the herein mentioned activity, in particular, confers an increase in the fine chemical amount or content. Further, in the present invention, the term "homologue" relates to the sequence of an organism having the highest sequence homology to the herein mentioned or listed sequences of all expressed sequences of said organism. However, the person skilled in the art knows, that preferably, the homologue has said the fine chemical increasing activity and, if known, the same biological function or activity in the organism as the YNL090W protein as depicted in SEQ ID NO: 2. In one embodiment, the homolog of the SEQ ID NO: 2 is a homolog having said biological acitivity and being derived from Eukaryot such as plants like the families Anacardiaceae, Asteraceae, Apiaceae, Betulaceae, Boraginaceae, Brassicaceae, Bromeliaceae, Caricaceae, Cannabaceae, Convolvulaceae, Chenopodiaceae, Cucurbitaceae, Elaeagnaceae, Ericaceae, Euphorbiaceae, Fabaceae, Geraniaceae, Gramineae, Juglandaceae, Lauraceae, Leguminosae or Linaceae; algae, fungi or mosses. In another embodiment, the homolog of SEQ ID NO: 2 is a homolog having said acitivity and being derived from bacteria. In a further embodiment, the homolog of the SEQ ID NO: 2 is a homolog having said acitivity and being derived from fungi. I

Further homologs of are described herein below.

In accordance with the invention, a protein or polypeptide has the "the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274. 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394" if its *de novo* activity, or its increased expression directly or indirectly leads to an increased the fine chemical level in the organism or a part thereof, preferably in a cell of said organism and the protein has the above mentioned activities of a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394. During the specification the activity or preferably the biological activity of such a protein or polypeptide or an nucleic acid molecule or sequence encoding such protein or polypeptide is identical or similar if it still has the biological or enzymatic activity of a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290; 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394, if it has at least 10% of the original biological or enzymatic activity, preferably 20%, particularly preferably 30%, most particularly preferably 40% in comparison to a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280. 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352; 354, 356, 358, 360, 362, 364, 366, 368, 370, 372, 374, 376, 378, 380, 382, 384, 386, 388, 390, 392 or 394 of Kluyveromyces lactis, Cryptococcus neoformans, Neurospora crassa, Penicillium marneffei, Mucor rouxii, Schizophyllum commune, Paracoccidioides brasiliensis, Aspergillus fumigatus, Suillus bovinus, Candida albicans, Trichoderma reesei, Ashbya gossypii, Yarrowia lipolytica, Ustilago maydis, Emericella nidulans, Trichomonas vaginalis, Colletotrichum trifolii, Blumeria graminis, Dictyostelium discoideum, Saccaromyces cerevisiae, Schizosaccharomyces pombe, Entamoeba histolytica, Oryza sativa, Brassica napus, Glycine max, Beta vulgaris, Lotus japonicus, Zinnia elegans, Zea mays, Cicer arietinum, Arabidopsis thaliana, Hordeum vulgare, Nicotiana tabacum, Gossypium hirsutum, Physcomitrella patens, Fucus distichus, Medicago truncutula, Homo sapiens, Caenorhabditits elegans, Tigriopus japonicus, Rhopalosiphum padi, Mus musculus, Discopyge ommata, Canis lupus, Drosophila melanogaster, Anopheles gambiae, Aplysia california, Ciona savignyi, Ciona intestinalis, Hemicentrotus pulcherrimus, Giardia lamblia, Gallus gallus, Brachydanio rerio, Xenopus laevis, Xenopus tropicalis, Schistosoma japonicum, Schistosoma mansoni, Encephalitozoon cuniculi, Wuchereria bancrofti, Cavia porcellus, Sus scrofa and/or Pagrus major.

The terms "increased", "rised", "extended", "enhanced", "improved" or "amplified" relate to a corresponding change of a property in an organism, a part of an organism such as a tissue, seed, root, leave, flower etc. or in a cell and are interchangeable. Preferrably, the overall activity in the volume is increased or enhanced in cases if the increase or enhancement is related to the increase or enhancement of an activity of a gene product, independent whether the amount of gene product or the specific activity of the gene product or both is increased or enhanced or whether the amount, stability or translation efficacy of the nucleic acid sequence or gene encoding for the gene product is increased or enhanced. The terms "reduction", "decrease" or "deletion" relate to a corresponding change of a property in an organism, a part of an organism such as a tissue, seed, root, leave, flower etc. or in a cell. Preferrably, the overall activity in the volume is reduced, decreased or deleted in cases if the reduction, decrease or deletion is related to the reduction, decrease or deletion of an activity of a gene product, independent whether the amount of gene product or the specific activity of the gene product or both is reduced, decreased or deleted or whether the amount, stability or translation efficacy of the nucleic acid sequence or gene encoding for the gene product is reduced, decreased or deleted.

The terms "increase" or "decrease" relate to a corresponding change of a property an organism or in a part of an organism, such as a tissue, seed, root, leave, flower etc. or in a cell. Preferrably, the overall activity in the volume is increased in cases the increase relates to the increase of an activity of a gene product, independent whether the amount of gene product or the specific activity of the gene product or both is increased or generated or whether the amount, stability or translation efficacy of the nucleic acid sequence or gene encoding for the gene product is increased.

Under "change of a property" it is understood that the activity, expression level or amount of a gene product or the metabolite content is changed in a specific volume relative to a corresponding volume of a control, reference or wild type, including the de novo creation of the activity or expression.

The terms "increase" or "decrease" include the change of said property in only parts of the subject of the present invention, for example, the modification can be found in compartment of a cell, like a organelle, or in a part of a plant, like tissue, seed, root, leave, flower etc. but is not detectable if the overall subject, i.e. complete cell or plant, is tested. Preferably, the increase or decrease is found cellular, thus the term "increase of an acitivity" or "increase of a metabolite content" relates to the cellular increase compared to the wild typ cell. Typically said increase is based on the higher content of the transcribed nucleic acid or the protein translated from said nucleic acid sequence in the modified cell in comparison to the wild type cell.

Accordingly, the term "increase" or "decrease" means that the specific activity of an enzyme as well as the amount of a compound or metabolite, e.g. of a polypeptide, a nucleic acid molelcule or of the fine chemical of the invention or an encoding mRNA or DNA, can be increased or decreased in a volume.

The terms "wild type", "control" or "reference" are exchangeable and can be a cell or a part of organisms such as an organelle or a tissue, or an organism, in particular a microorganism or a plant, which was not modified or treated according to the herein described process according to the invention. Accordingly, the cell or a part of organisms such as an organelle or a tissue, or an organism, in particular a microorganism or a plant used as wild typ, control or reference corresponds to the cell, organism or part thereof as much as possible and is in any other property but in the result of the process of the invention as identical to the subject matter of the invention as possible. Thus, the wild type, control or reference is treated identically or as identical as possible, saying that only conditions or properties might be different which do not influence the quality of the tested property.

Preferably, any comparison is carried out under analogous conditions. The term "analogous conditions" means that all conditions such as, for example, culture or growing conditions, assay conditions (such as buffer composition, temperature, substrates, pathogen strain, concentrations and the like) are kept identical between the experiments to be compared.

The "reference", "control", or "wild type" is preferably a subject, e.g. an organelle, a cell, a tissue, an organism, in particular a plant or a microorganism, which was not modified or treated according to the herein described process of the invention and is in any other property as similar to the subject matter of the invention as possible. The reference, control or wild type is in its genome, transcriptome, proteome or meta-bolome as similar as possible to the subject of the present invention. Preferably, the term "reference-" "control-" or "wild type-"-organelle, -cell, -tissue or -organism, in particular plant or microorganism, relates to an organelle, cell, tissue or organism, in particular plant or micororganism, which is nearly genetically identical to the organelle, cell, tissue or organism, in particular microorganism or plant, of the present invention or a part thereof preferably 95%, more peferred are 98%, even more preferred are 99,00%, in particular 99,10%, 99,30%, 99,50%, 99,70%, 99,90%, 99,99%, 99, 999% or more.. Most preferable the "reference", "control", or "wild type" is a subject, e.g. an organelle, a cell, a tissue, an organism, which is genetically identical to the organism, cell or organelle used according to the process of the invention except that the responsible or acvitivity conferring nucleic acid molecules or the gene product encoded by them are amended, manipulated, exchanged or introduced according to the inventive process.

Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention, e.g. by or in the expression level or activity of an protein having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs, its biochemical or genetical causes and the increased amount of the fine chemical.

In case, a control, reference or wild type differing from the subject of the present invention only by not being subject of the process of the invention can not be provided, a control, reference or wild type can be an organism in which the cause for the modulation of an activity conferring the increase of the fine chemical or expression of the nucleic acid molecule of the invention as described herein has been switched back or off, e.g. by knocking out the expression of responsible gene product, e.g. by antisense inhibition, by inactivation of an activator or agonist, by activation of an inhibitor or antagonist, by inhibition through adding inhibitory antibodies, by adding active compounds as e.g. hormones, by introducing negative dominant mutants, etc. A gene production can for example be knocked out by introducing inactivating point mutations, which lead to an enzymatic activity inhibition or a destabilization or an inhibition of the ability to bind to cofactors etc.

Accordingly, preferred reference subject is the starting subject of the present process of the invention. Preferably, the reference and the subject matter of the invention are compared after standardization and normalization, e.g. to the amount of total RNA, DNA, or protein or activity or expression of reference genes, like housekeeping genes, such as ubiquitin, actin or ribosomal proteins.

A series of mechanisms exists via which a modification of the a protein, e.g. the polypeptide of the invention can directly or indirectly affect the yield, production and/or production efficiency of the amino acid.

For example, the molecule number or the specific activity of the polypeptide or the nucleic acid molecule may be increased. Larger amounts of the fine chemical can be produced if the polypeptide or the nucleic acid of the invention is expressed *de novo* in an organism lacking the activity of said protein. However, it is also possible to increase the expression of the gene which is naturally present in the organisms, for example by modifying the regulation of the gene, or by increasing the stability of the corresponding mRNA or of the corresponding gene product encoded by the nucleic acid molecule of the invention, or by introducing homologous genes from other organisms which are differently regulated, eg. not feedback sensitive or not feedback regulated.

This also applies analogously to the combined increased expression of the nucleic acid molecule of the present invention or its gene product with that of further enzymes of the biochemical pathway of the fine chemical e.g. of the amino acid biosynthesis pathway, e.g. which are useful for the synthesis of the fine chemicals.

The increase, decrease or modulation according to this invention can be constitutive, e.g. due to a stable permanent transgenic expression or to a stable mutation in the corresponding endogenous gene encoding the nucleic acid molecule of the invention or to a modulation of the expression or of the behaviour of a gene conferring the expression of the polypeptide of the invention, or transient, e.g. due to an transient transformation or temporary addition of a modulator such as a agonist or antagonist or inducible, e.g. after transformation with a inducible construct carrying the nucleic acid molecule of the invention under control of a induceable promoter and adding the inducer, e.g. tetracycline or as described herein below.

The increase in activity of the polypeptide amounts in a cell, a tissue, a organelle, an organ or an organism or a part thereof preferably to at least 5%, preferably to at least 20% or at to least 50%, especially preferably to at least 70%, 80%, 90% or more, very especially preferably are to at least 200%, 300% or 400%, most preferably are to at least 500%, 600% or more in comparison to the control, reference or wild type.

The specific activity of a polypeptide encoded by a nucleic acid molecule of the present invention or of the polypeptide of the present invention can be tested as described in the examples. In particular, the expression of a protein in question in a cell, e.g. a plant cell or a microorganism and the detection of an increase the fine chemical level in comparison to a control is an easy test and can be performed as described in the state of the art.

The term "increase" includes, that a compound or an activity is introduced into a cell *de novo* or that the compound or the activity has not been detectable before, in other words it is "generated".

Accordingly, in the following, the term "increasing" also comprises the term "generating" or "stimulating". The increased activity manifests itself in an increase of the fine chemical.

In case the biological activity of the Saccaromyces cerevisiae protein YNL090W as depicted in SEQ ID NO: 2, or the biological activity of the of Kluyveromyces lactis, Cryptococcus neoformans, Neurospora crassa, Penicillium mameffei, Mucor rouxii, Schizophyllum commune, Paracoccidioides brasiliensis, Aspergillus fumigatus, Suillus bovinus, Candida albicans, Trichoderma reesei, Ashbya gossypii, Yarrowia lipolytica, Ustilago maydis, Emericella nidulans, Trichomonas vaginalis, Colletotrichum trifolii, Blumeria graminis, Dictyostelium discoideum, Schizosaccharomyces pombe, Entamoeba histolytica, Oryza sativa, Brassica napus, Glycine max, Beta vulgaris, Lotus japonicus, Zinnia elegans, Zea mays, Cicer arietinum, Arabidopsis thaliana, Hordeum vulgare, Nicotiana tabacum, Gossypium hirsutum, Physcomitrella patens, Fucus distichus, Medicago truncutula, Homo sapiens, Caenorhabditits elegans, Tigriopus japonicus, Rhopalosiphum padi, Mus musculus, Discopyge ommata, Canis lupus, Drosophila melanogaster, Anopheles gambiae, Aplysia california, Ciona savignyi, Ciona intestinalis, Hemicentrotus pulcherrimus, Giardia lamblia, Gallus gallus, Brachydanio rerio, Xenopus laevis, Xenopus tropicalis, Schistosoma japonicum, Schistosoma mansoni, Encephalitozoon cuniculi, Wuchereria bancrofti, Cavia porcellus, Sus scrofa, Rattus norvegicus, Pneumocystis carinii or Pagrus major proteins as depicted SEQ ID NO: 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs is increased, preferably, in one embodment an increase of the fine chemical of at least 100%, 150%, or preferably to at least 200%, 250%, 300%, 350% or 400%, especially prerferably to at least 450%, 500%, 550%, 600% or more is conferred.

In case the biological activity of the Saccaromyces cerevisiae protein YNL090W as depicted in SEQ ID NO: 2, or the biological activity of the Kluyveromyces lactis, Cryptococcus neoformans, Neurospora crassa. Penicillium mameffei, Mucor rouxii, Schizophyllum commune, Paracoccidioides brasiliensis, Aspergillus fumigatus, Suillus bovinus, Candida albicans, Trichoderma reesei, Ashbya gossypii, Yarrowia lipolytica, Ustilago maydis, Emericella nidulans, Trichomonas vaginalis, Colletotrichum trifolii, Blumeria graminis, Dictyostelium discoideum, Schizosaccharomyces pombe, Entamoeba histolytica, Oryza sativa, Brassica napus, Glycine max, Beta vulgaris, Lotus japonicus, Zinnia elegans, Zea mays, Cicer arietinum, Arabidopsis thaliana, Hordeum vulgare, Nicotiana tabacum, Gossypium hirsutum, Physcomitrella patens, Fucus distichus, Medicago truncutula, Homo sapiens, Caenorhabditits elegans, Tigriopus japonicus, Rhopalosiphum padi, Mus musculus, Discopyge ommata, Canis lupus, Drosophila melanogaster, Anopheles gambiae, Aplysia california, Ciona savignyi, Ciona intestinalis, Hemicentrotus pulcherrimus, Giardia lamblia, Gallus gallus, Brachydanio rerio, Xenopus laevis, Xenopus tropicalis, Schistosoma japonicum, Schistosoma mansoni, Encephalitozoon cuniculi, Wuchereria bancrofti, Cavia porcellus, Sus scrofa, Rattus norvegicus, Pneumocystis carinii or Pagrus major proteins as depicted SEQ ID NO: 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs is increased, preferably, an increase of the fine chemical in conferred, preferably of the fine chemical such as essential amino acids e.g. tryptophane, arginine, phenylalanine, tyrosine, threonine, valine, isoleucine and/or leucine, non-essential amino acids e.g. proline, alanine, glycine or serine, modified amino acids e.g. 3,4-dihydroxyphenylalanine, carbohydrates e.g. raffinose, inositol or iso-maltose, vitamins e.g. α-tocopherol, β-tocopherol or γ-tocopherol, organic acids e.g. ferulic acid, sinapic acid or malate, fatty acids e.g. cerotic acid, lignoceric acid, 2-hydroxy-palmitic acid or stearic acid, carotinoids e.g. β-carotene or mixtures thereof is conferred.

In this context, the fine chemical amount in a cell, preferably in a tissue, more preferred in a organism as a plant or a microorganim or part thereof, is increased by at least 3%, 4%, 5%, 6%, 7%, 8% or 9% or more, especially preferably are at least 10%, 20%, 40%, 50% or more, very especially preferably are more than 60%, 70%, 80%, 90%, 100% or more and most preferably are 150% or more, such as 200%, 250%, 300%, 350%, 400%, 450%, 500%, 550% or 600%.

The fine chemical can be contained in the organism either in its free form and/or bound to proteins, polypeptids or other compounds such as polysaccharides, lipids, glycoproteins or glycolipids etc. or mixtures thereof. Accordingly, in one embodiment, the amount of the free form in a cell, preferably in a tissue, more preferred in a organism as a plant or a microorganim or part thereof, is increased by at least 3%, 4%, 5%, 6%, 7%, 8% or 9% or more, especially preferably are at least 10%, 20%, 40%, 50% or more, very especially preferably are more than 60%, 70%, 80%, 90%, 100% or more and most preferably are 150% or more, such as 200%, 250%, 300%, 350%, 400%, 450%, 500%, 550% or 600%. Accordingly, in an other embodiment, the amount of the bound the fine chemical in a cell, preferably in a tissue, more preferred in a organism as a plant or a microorganism or part thereof, is increased by at least 3%, 4%, 5%, 6%, 7%, 8% or 9% or more, especially preferably are at least 10%, 20%, 40%, 50% or more, very especially preferably are more than 60%, 70%, 80%, 90%, 100% or more and most preferably are 150% or more, such as 200%, 250%, 300%, 350%, 400%, 450%, 500%, 550% or 600%.

A protein having an activity conferring an increase in the amount or level of the fine chemical preferably has the structure of the polypeptide described herein, in particular of the polypeptides comprising the consensus sequence shown in SEQ ID NO: 47, SEQ ID NO: 48, SEQ ID NO: 49, SEQ ID NO: 50, SEQ ID NO: 51, SEQ ID NO: 52, SEQ ID NO: 397, SEQ ID NO: 398, SEQ ID NO: 399 and/or SEQ ID NO: 400 as described herein, or is encoded by the nucleic acid molecule characterized herein or the nucleic acid molecule according to the invention, for example by the nucleic acid molecule as shown in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393 or its herein described functional homologues and has the herein mentioned activity.

For the purposes of the present invention, the terms "the fine chemical" or "fine chemical" such as essential amino acids e.g. tryptophane, arginine, phenylalanine, tyrosine, threonine, valine, isoleucine and/or leucine, non-essential amino acids e.g. proline, alanine, glycine or serine, modified amino acids e.g. 3,4-dihydroxyphenylalanine, carbohydrates e.g. raffinose, inositol or iso-maltose, vitamins e.g. α-tocopherol, β-tocopherol or γ-tocopherol, organic acids e.g. ferulic acid, sinapic acid or malate, fatty acids e.g. cerotic acid, lignoceric acid, 2-hydroxy-palmitic acid or stearic acid, carotinoids e.g. β-carotene or mixtures thereof also encompass the corresponding salts, such as, for example, tryptophane hydrochloride, arginine hydrochloride, phenylalanine hydrochloride, tyrosine hydrochloride, threonine hydrochloride, valine hydrochloride, isoleucine hydrochloride or leucine hydrochloride or tryptophane sulfate, arginine sulfate, phenylalanine sulfate, tyrosine sulfate, threonine sulfate, valine sulfate, isoleucine sulfate or leucine sulfate; ester or amids.

Owing to the biological activity of the proteins which are used in the process according to the invention and which are encoded by nucleic acid molecules according to the invention, it is possible to produce compositions comprising the fine chemical, i.e. an increased amount of the free chemical free or bound, e.g amino acid compositions. Depending on the choice of the organism used for the process according to the present invention, for example a microorganism or a plant, compositions or mixtures of various fine chemicals e.g. amino acids can be produced.

The term "expression" refers to the transcription and/or translation of a codogenic gene segment or gene. As a rule, the resulting product is an mRNA or a protein. However, expression products can also include functional RNAs such as, for example, antisense, nucleic acids, tRNAs, snRNAs, rRNAs, RNAi, siRNA, ribozymes etc. Expression may be systemic, local or temporal, for example limited to certain cell types, tissuesorgans or time periods.

In one embodiment, the process of the present invention comprises one or more of the following steps:
a) stabilizing a protein conferring the increased expression of a protein encoded by the nucleic acid molecule of the invention or of the protein of the invention, e.g. of a protein having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs having the fine chemical increasing activity;
b) stabilizing a mRNA conferring the increased expression of a protein encoded by the nucleic acid molecule of the invention, e.g. of a protein having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs or of an mRNA encoding the polypeptide of the present invention having the fine chemical increasing activity;
c) increasing the specific activity of a protein conferring the increased expression of a protein encoded by the nucleic acid molecule of the invention or of the protein of the invention having the fine chemical increasing activity, e.g. of a protein having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs, or decreasing the inhibitiory regulation of the protein of the invention;
d) generating or increasing the expression of an endogenous or artificial transcription factor mediating the expression of a protein conferring the increased expression of a protein encoded by the nucleic acid molecule of the invention or of the protein of the invention having the fine chemical increasing activity, e.g. of a polypeptide having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, .298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs;
e) stimulating activity of a protein conferring the increased expression of a protein encoded by the nucleic acid molecule of the present invention or a protein of the present invention having the fine chemical increasing activity, e.g. of a protein having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs, by adding one or more exogenous inducing factors to the organismus or parts thereof;
f) expressing a transgenic gene encoding a protein conferring the increased expression of a polypeptide (= protein) encoded by the nucleic acid molecule or the protein of the invention, having the fine chemical increasing activity, e.g. of a protein having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs;
g) increasing the copy number of a gene conferring the increased expression of a nucleic acid molecule encoding a protein encoded by the nucleic acid molecule of the invention or the protein of the invention the fine chemical increasing activity, e.g. of a protein having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs;
h) increasing the expression of the endogenous gene encoding the protein of the invention, e.g. a protein having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;
i) modulating growth conditions of an organism in such a manner, that the expression or activity of the gene encoding the protein of the invention or the protein itself is enhanced for example microorganisms or plants can be grown under a higher temperature regime leading to an enhanced expression of heat shock proteins, e.g. the heat shock protein of the invention, which can lead an enhanced the fine chemical production; and/or
j) selecting of organisms with expecially high activity of the protein of the invention from natural or from mutagenized resources and breeding them into the target organisms, eg the elite crops.

Preferably, said mRNA is the nucleic acid molecule of the invention and/or the protein conferring the increased expression of a protein encoded by the nucleic acid molecule of the invention or the polypeptide having the herein mentioned activity, e.g. conferring the increase of the fine chemical after increasing the expression or activity of the encoded polypeptide or having the activity of a polypeptide having biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or its homologs.

In general, the amount of mRNA, polynucleotide or nucleic acid molecule in a cell or a compartment of an organism correlates with the amount of encoded protein and thus with the overall activity of the encoded protein in said volume. Said correlation is not always linear, the activity in the volume is dependent on the stability of the molecules, the degradation of the molecules or the presence of activating or inhibiting co-factors. Further, product and educt inhibitions of enzymes are well known and described in textbooks, e.g. Stryer, Biochemistry or Zinser et al. "Enzyminhibitoren"/Enzyme inhibitors".

The activity of the abovementioned proteins and/or poylpeptide encoded by the nucleic acid molecule of the present invention can be increased in various ways. For example, the activity in an organism or in a part thereof, like a cell, is increased via increasing the gene product number, e.g. by increasing the expression rate, like introducing a stronger promoter, or by increasing the stability of the mRNA expressed, thus increasing the translation rate, and/or increasing the stability of the gene product, thus reducing the proteins decayed. Further, the activity or turnover of enzymes can be influenced in such a way that a reduction or increase of the reaction rate or a modification (reduction or increase) of the affinity to the substrate results, is reached. A mutation in the catalytic centre of a polypeptide of the invention, e.g. an enzyme, can modulate the turn over rate of the enzyme, e.g. a knock out of an essential amino acid can lead to a reduced or completely knock out activity of the enzyme, or the deletion or mutation of regulator binding sites can reduce a negative regulation like a feedback inhibition (or a substrate inhibition, if the substrate level is also increased). The specific activity of an enzyme of the present invention can be increased such that the turn over rate is increased or the binding of a co-factor is improved. Improving the stability of the encoding mRNA or the protein can also increase the activity of a gene product. The stimulation of the activity is also under the scope of the term "increased activity".

Moreover, the regulation of the abovementioned nucleic acid sequences may be modified so that gene expression is increased. This can be achieved advantageously by means of heterologous regulatory sequences or by modifying, for example mutating, the natural regulatory sequences which are present. The advantageous methods may also be combined with each other.

In general, an activity of a gene product in an organism or part thereof, in particular in a plant cell, a plant, or a plant tissue or a part thereof or in a microorganism can be increased by increasing the amount of the specific encoding mRNA or the corresponding protein in said organism or part thereof. "Amount of protein or mRNA" is understood as meaning the molecule number of polypeptides or mRNA molecules in an organism, a tissue, a cell or a cell compartment. "Increase" in the amount of a protein means the quantitative increase of the molecule number of said protein in an organism, a tissue, a cell or a cell compartment or part thereof - for example by one of the methods described herein below - in comparison to a wild type, control or reference.

The increase in molecule number amounts preferably to at least 1 %, preferably to more than 10%, more preferably to 30% or more, especially preferably to 50%, 70% or more, very especially preferably to 100%, most preferably to 500% or more. However, a de novo expression is also regarded as subject of the present invention.

[0073.0.0.0] A modification, i.e. an increase or decrease, can be caused by endogenous or exogenous factors. For example, an increase in activity in an organism or a part thereof can be caused by adding a gene product or a precursor or an activator or an agonist to the media or nutrition or can be caused by introducing said subjects into a organism, transient or stable.

In one embodiment the increase in the amount of the fine chemical in the organism or a part thereof, e.g. in a cell, a tissue, an organ, an organelle etc., is achived by increasing the endogenous level of the polypeptide of the invention. Accordingly, in an embodiment of the present invention, the present invention relates to a process wherein the gene copy number of a gene encoding the polynucleotide or nucleic acid molecule of the invention is increased. Further, the endogenous level of the polypeptide of the invention can for example be increased by modifying the transcriptional or translational regulation of the polypeptide.

In one embodiment the amount of the fine chemical in the organism or part thereof can be increase by targeted or random mutagenesis of the endogenous genes of the invention. For example homologous recombination can be used to either introduce positive regulatory elements like for plants the 35S enhancer into the promoter or to remove repressor elements form regulatory regions. In addition gene conversion like methods described by Kochevenko and Willmitzer (Plant Physiol. 2003 May;132(1):174-84) and citations therein can be used to disrupt repressor elements or to enhance to acitivty of positive regulatory elements.
Furthermore positive elements can be randomly introduced in (plant) genomes by T-DNA or transposon mutagenesis and lines can be screened for, in which the positive elements has be integrated near to a gene of the invention, the expression of which is thereby enhanced. The activation of plant genes by random integrations of enhancer elements has been described by Hayashi et al., 1992 (Science 258:1350-1353) or Weigel et al., 2000 (Plant Physiol. 122, 1003-1013) and others citated therein. Reverse genetic strategies to identify insertions (which eventually carrying the activation elements) near in genes of interest have been described for various cases eg. Krysan et al., 1999 (Plant Cell 1999, 11, 2283-2290); Sessions et al., 2002 (Plant Cell 2002, 14, 2985-2994); Young et al., 2001, (Plant Physiol. 2001, 125, 513-518); Koprek et al., 2000 (Plant J. 2000, 24, 253-263) ; Jeon et al., 2000 (Plant J. 2000, 22, 561-570) ; Tissier et al., 1999 (Plant Cell 1999, 11, 1841-1852); Speulmann et al., 1999 (Plant Cell 1999 ,11 , 1853-1866). Briefly material from all plants of a large T-DNA or transposon mutagenized plant population is harvested and genomic DNA prepared. Then the genomic DNA is pooled following specific architectures as described for example in Krysan et al., 1999 (Plant Cell 1999, 11, 2283-2290). Pools of genomics DNAs are then screened by specific multiplex PCR reactions detecting the combination of the insertional mutagen (eg T-DNA or Transposon) and the gene of interest. Therefore PCR reactions are run on the DNA pools with specific combinations of T-DNA or transposon border primers and gene specific primers. General rules for primer design can again be taken from Krysan et al., 1999 (Plant Cell 1999, 11, 2283-2290) Rescreening of lower levels DNA pools lead to the identifcation of individual plants in which the gene of interest is disrupted by the insertional mutagen.
The enhancement of positive regulatory elements or the disruption or weaking of negative regulatory elements can also be achieved through common mutagenesis techniques: The production of chemically or radiation mutated populations is a common technique and known to the skilled worker. Methods for plants are described by Koomeef et al. 1982 and the citations therein and by Lightner and Caspar in "Methods in Molecular Biology" Vol 82. These techniques usually induce pointmutations that can be identified in any known gene using methods such as TILLING (Colbert et al. 2001).

Accordingly, the expression level can be increased if the endogenous genes encoding a polypeptide conferring an increased expression of the polypeptide of the present invention, in particular genes comprising the nucleic acid molecule of the present invention, are modified via homologous recombination, Tilling approaches or gene conversion

Regulatory sequences can be operatively linked to the coding region of an endogenous protein and control its transcription and translation or the stability or decay of the encoding mRNA or the expressed protein. In order to modify and control the expression, promoter, UTRs, splicing sites, processing signals, polyadenylation sites, terminators, enhancers, repressors, post transcriptional or posttranslational modification sites can be changed, added or amended for example, the activation of plant genes by random integrations of enhancer elements has been described by Hayashi et al., 1992 (Science 258:1350-1353) or Weigel et al., 2000 (Plant Physiol. 122, 1003-1013) and others citated therein. For example, the expression level of the endogenous protein can be modulated by replacing the endogenous promoter with a stronger transgenic promoter or by replacing the endogenous 3'UTR with a 3'UTR, which provides more stablitiy without amending the coding region. Further, the transcriptional regulation can be modulated by introduction of an artifical transcription factor as described in the examples. Alternative promoters, terminators and UTR are described below.

The activation of an endogenous polypeptide having the fine chemical increasing activity, e.g.having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394, e.g. conferring the increase of the fine chemical after increase of expression or activity can also be increased by introducing a synthetic transcription factor, which binds close to the coding region of the protein of the invention encoding gene and activates its transcription. A chimeric zinc finger protein can be construed, which comprises a specific DNA-binding domain and an activation domain as e.g. the VP16 domain of Herpes Simplex virus. The specific binding domain can bind to the regulatory region of the nucleic acid sequence used in the inventive process. The expression of the chimeric transcription factor in an organism, in particular in a plant, leads to a specific expression of the protein of the invention, see e.g. in WO01/52620, Oriz, Proc. Natl. Acad. Sci. USA, 2002, Vol. 99, 13290 or Guan, Proc. Natl. Acad. Sci. USA, 2002, Vol. 99, 13296.

In one further embodiment of the process according to the invention, organisms are used in which one of the abovementioned genes, or one of the abovementioned nucleic acids, is mutated in a way that the activity of the encoded gene products is less influenced by cellular factors, or not at all, in comparison with the unmutated proteins. For example, well known regulation mechanism of enzymic activity are substrate inhibition or feed back regulation mechanisms. Ways and techniques for the introduction of substitutions, deletions and additions of one or more bases, nucleotides or amino acids of a corresponding sequence are described herein below in the corresponding paragraphs and the references listed there, e.g. in Sambrook et al., Molecular Cloning, Cold Spring Habour, NY, 1989. The person skilled in the art will be able to identify regulation domains and binding sites of regulators by comparing the sequence of the nucleic acid molecule of the present invention or the expression product thereof with the state of the art by computer software means which comprise algorithms for the identifying of binding sites and regulation domains or by introducing into a nucleic acid molecule or in a protein systematically mutations and assaying for those mutations which will lead to an increased specifiy activity or an increased activity per volume, in particular per cell.

It is therefore adavantageously to express in an organism a nucleic acid molecule of the invention or a polypeptide of the invention derived from a evolutionary distantly related organism, as e.g. using a prokaryotic gene in a eukaryotic host, as in these cases the regulation mechanism of the host cell may not weaken the activity (cellular or specific) of the gene or its expression product

The mutation is introduced in such a way that the production of the fine chemical is not adversely affected.

Less influence on the regulation of a gene or its gene product is understood as meaning a reduced regulation of the enzymatic activity leading to an increased specific or cellular activity of the gene or its product. An increase of the enzymatic activity is understood as meaning an enzymatic activity, which is increased by at least 10%, 20%, 30%, 40% or 50%, advantageously by at least 60%, 70%, 80%, 90% or 100%, especially advantageously by at least 150%, 200%, 300% or more in comparison with the starting organism. In the event the inventive nucleic acid sequences were introduced into an organism, which did not have the encoded protein activity, said new generated enzymatic activity shall also be embraced by the herein described invention. This leads to an increased productivity of the desired fine chemical.

Owing to the introduction of a gene or a plurality of genes conferring the expression of the nucleic acid molecule of the invention or the polypeptide of the invention as described below, for example the nucleic acid construct mentioned below, or e.g. encoding the protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 228, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 into an organism alone or in combination with other genes, it is possible not only to increase the biosynthetic flux towards the end product, but also to increase, modify or create *de novo* an advantageous, preferably novel metabolites composition in the organism, e.g. an advantageous amino acid composition comprising a higher content of (from a viewpoint of nutrional physiology limited) amino acids, for example essential amino acids like tryptophane, threonine, methionine or lysine.

Preferably the composition comprises further higher amounts of metabolites positively affecting or lower amounts of metabolites negatively affecting the nutrition or health of animals or humans provided with said compositions or organisms of the invention or parts thereof. Likewise, the number or activity of further genes which are required for the import or export of nutrients or metabolites, including for example amino acids or its precursors, required for the cell's biosynthesis of the fine chemical may be increased so that the concentration of necessary or relevant precursors, cofactors or intermediates within the cell(s) or within the corresponding storage compartments is increased. Owing to the increased or novel generated activity of the polypeptide of the invention or owing to the increased number of nucleic acid sequences of the invention and/or to the modulation of further genes which are involved in the biosynthesis of the fine chemical, e.g. by increasing the acitivty of enzymes synthizing precursors or by destroying the activity of one or more genes which are involved in the breakdown of the fine chemical, it is thereby possible to increase the yield, production and/or production efficiency of the fine chemical in the host organism, such as plants or microorganims.

Accordingly, in one embodiment, the process according to the invention relates to a process, which comprises:
a) providing a non-human organism, preferably a microorganism, non-human animal, plant or part, cell or tissue thereof;
b) increasing the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or of a polypeptide being encoded by the nucleic acid molecule of the invention and described below, i.e. conferring an increase of the fine chemical in the organism, preferably in the microorganism, the non-human animal, the plant or part, cell or tissue thereof,
c) growing the organism, preferably the microorganism, the non-human animal, the plant or part, cell or tissue thereof under conditions which permit the production of the fine chemical; and
d) if desired, revovering, optionally isolating, the free and/or bound fine chemical.

The organism, in particular the microorganism, non-human animal, the plant or animal parts, the plant or animal cell, the plant or animal tissue or the plant is advantageously grown in such a way that it is not only possible to recover, if desired isolate the free or bound fine chemical (Galili et al., Transgenic Res., 2000, 9, 2, 137-144).

After the above-described increasing (which as defined above also encompasses the generating of an activity in an organism, i.e. a de *novo* activity), for example after the introduction and the expression of the nucleic acid molecules of the invention or described in the methods or processes according to the invention, the organism according to the invention, advantageously, a microorganism, a non-human animal, a plant, plant or animal tissue or plant or animal cell, is grown and subsequently harvested.

Suitable organisms or host organisms (transgenic organism) for the nucleic acid molecule used according to the invention and for the inventive process, the nucleic acid construct or the vector (both as described below) are, in principle, all organisms which are capable of synthesizing the fine chemical, and which are suitable for the activation, introduction or stimulation genes. Examples which may be mentioned are plants, microorganisms such as fungi, bacteria, yeasts, alga or diatom, transgenic or obtained by site directed mutagenesis or random mutagenesis combined with specific selection procedures. Preferred organisms are those which are naturally capable of synthesizing the fine chemical in substantial amounts, like fungi, yeasts, bactria or plants. In principle, transgenic animals, for example Caenorhabditis elegans, are also suitable as host organisms.

In the event that the transgenic organism is a microorganism, such as a eukaryotic organism, for example a fungus, an alga, diatom or a yeast in particular a fungus, alga, diatom or yeast selected from the families Chaetomiaceae, Choanephoraceae, Cryptococcaceae, Cunninghamellaceae, Demetiaceae, Dipodascaceae, Moniliaceae, Mortierellaceae, Mucoraceae, Pythiaceae, Saccharomycetaceae, Saprolegniaceae, Schizosaccharomycetaceae, Sodariaceae, Sporobolomycetaceae Tuberculariaceae, Adelotheciaceae, Dinophyceae, Ditrichaceae or Prasinophyceae, or a fungus selected from the families Tremellaceae, Filobasidiaceae, Christianseniaceae, Cystofilobasidiaceae, Sordariaceae, Annulatascaceae, Cephalothecaceae, Chaetomiaceae, Coniochaetaceae, Lasiosphaeriaceae, Pleurotremataceae, Elaphomycetaceae, Trichocomaceae, Mucoraceae, Schizophyllaceae, Onygenaceae, Suillaceae, Hypocreaceae, Ustilaginaceae, Trichocomaceae, Phyllachoraceae, Erysiphaceae or a prokaryotic organism, for example a bacterium or blue alga, in particular a bacterium from the families Actinomycetaceae, Bacillaceae, Brevibacteriaceae, Corynebacteriaceae, Cyanophyceae, Enterobacteriacae, Gordoniaceae, Nocardiaceae, Micrococcaceae, Mycobacteriaceae, Pseudomonaceae, Rhizobiaceae or Streptomycetaceae, this microorganism is grown on a solid or in a liquid medium which is known to the skilled worker and suits the organism. After the growing phase, the organisms can be harvested.

The microorganisms or the recovered, and if desired isolated, fine chemical can then be processed further directly into foodstuffs or animal feeds or for other applications, for example according to the disclosures made in EP-B-0 533 039 or EP-A-0 615 693, which are expressly incorporated herein by reference. The fermentation broth or fermentation products can be purified in the customary manner by extraction and precipitation or via ion exchangers and other methods known to the person skilled in the art and described herein below. Products of these different work-up procedures are fine chemical e.g. amino acids or amino acid compositions which still comprise fermentation broth and cell components in different amounts, advantageously in the range of from 0 to 99% by weight, preferably below 80% by weight, especially preferably between below 50%, 40%, 30%, 20%, 10% or 5% by weight.

Preferred microorganisms are selected from the group consisting of Chaetomiaceae such as the genera Chaetomium e.g. the species *Chaetomidium fimeti;* Choanephoraceae such as the genera Blakeslea, Choanephora e.g. the species *Blakeslea trispora, Choanephora cucurbitarum or Choanephora infundibulifera* var. *cucurbitarum;* Cryptococcaceae such as the genera Candida, Crytococcus, Rhodotorula, Torulopsis e.g. the species *Candida albicans, Candida albomarginata, Candida antarctica, Candida bacarum, Candida bogoriensis, Candida boidinii, Candida bovina, Candida brumptii, Candida cacaoi, Candida cariosilignicola, Candida catenulata, Candida chalmersii, Candida ciferrii, Candida cylindracea, Candida edax, Candida emobii, Candida famata, Candida freyschussii, Candida friedrichii, Candida glabrata, Candida guilliermondii, Candida haemulonii, Candida humicola, Candida inconspicua, Candida ingens, Candida intermedia, Candida kefyr, Candida krusei, Candida lactiscondensi, Candida lambica, Candida lipolytica, Candida lusitaniae, Candida macedoniensis, Candida magnoliae, Candida membranaefaciens, Candida mesenterica, Candida multigemmis, Candida mycoderma, Candida nemodendra, Candida nitratophila, Candida norvegensis, Candida norvegica, Candida parapsilosis, Candida pelliculosa, Candida peltata, Candida pini, Candida pseudotropicalis, Candida pulcherrima, Candida punicea, Candida pustula, Candida ravautii, Candida reukaufii, Candida rugosa, Candida sake, Candida silvicola, Candida solani, Candida sp., Candida spandovensis, Candida succiphila, Candida tropicalis, Candida utilis, Candida valida, Candida versatilis, Candida vini, Candida zeylanoides, Cryptococcus albidus, Cryptococcus curvatus, Cryptococcus flavus, Cryptococcus humicola, Cryptococcus hungaricus, Cryptococcus kuetzingii, Cryptococcus laurentii, Cryptococcus macerans, Cryptococcus neoformans, Cryptococcus terreus, Cryptococcus uniguttulatus, Rhodotorula acheniorum, Rhodotorula bacarum, Rhodotorula bogoriensis, Rhodotorula flava, Rhodotorula glutinis, Rhodotorula macerans, Rhodotorula minuta, Rhodotorula mucilaginosa, Rhodotorula pilimanae, Rhodotorula pustula, Rhodotorula rubra, Rhodotorula tokyoensis, Torulopsis colliculosa, Torulopsis dattila or Torulopsis neoformans;* Cunninghamellaceae such as the genera Cunninghamella e.g. the species *Cunninghamella blakesleeana, Cunninghamella echinulata, Cunninghamella echinulata* var. *elegans, Cunninghamella elegans or Cunninghamella homothallica;* Demetiaceae such as the genera Altemaria, Bipolaris, Cercospora, Chalara, Cladosporium, Curvularia, Exophilia, Helicosporium, Helminthosporium, Orbimyces, Philalophora, Pithomyces, Spilocaea, Thielaviopsis, Wangiella e.g. the species *Curvularia affinis, Curvularia clavata, Curvularia fallax, Curvularia inaequalis, Curvularia indica, Curvularia lunata, Curvularia pallescens, Curvularia verruculosa or Helminothosporium sp.;* Moniliaceae such as the genera Arthrobotrys, Aspergillus, Epidermophyton, Geotrichum, Gliocladium, Histoplasma, Microsporum, Monilia, Oedocephalum, Oidium, Penicillium, Trichoderma, Trichophyton, Thrichoteclum, Verticillium e.g. the species *Aspergillus aculeatus, Aspergillus albus, Aspergillus alliaceus, Aspergillus asperescens , Aspergillus awamori, Aspergillus candidus, Aspergillus carbonarius, Aspergillus cameus, Aspergillus chevalieri, Aspergillus chevalieri* var. *intermedius, Aspergillus clavatus, Aspergillus ficuum, Aspergillus flavipes, Aspergillus flavus, Aspergillus foetidus, Aspergillus fumigatus, Aspergillus giganteus, Aspergillus humicola, Aspergillus intermedius, Aspergillus japonicus, Aspergillus nidulans, Aspergillus niger, Aspergillus niveus, Aspergillus ochraceus, Aspergillus oryzae, Aspergillus ostianus, Aspergillus parasiticus, Aspergillus parasiticus* var. *globosus, Aspergillus penicillioides, Aspergillus phoenicis, Aspergillus rugulosus, Aspergillus sclerotiorum, Aspergillus sojae* var. *gymnosardae, Aspergillus sydowi, Aspergillus tamarii, Aspergillus terreus, Aspergillus terricola, Aspergillus toxicarius, Aspergillus unguis, Aspergillus ustus, Aspergillus versicolor, Aspergillus vitricolae, Aspergillus wentii,·Penicillium adametzi, ·Penicillium albicans, Penicillium arabicum, Penicillium arenicola, Penicillium argillaceum, Penicillium arvense, Penicillium asperosporum, ·Penicillium aurantiogriseum, ·Penicillium avellaneum, ·Penicillium baarnense, ·Penicillium bacillisporum, ·Penicillium brasilianum, ·Penicillium brevicompactum, ·Penicillium camemberti, ·Penicillium canadense, ·Penicillium canescens, ·Penicillium caperatum, ·Penicillium capsulatum, ·Penicillium caseicolum, ·Penicillium chrysogenum, ·Penicillium citreonigrum, ·Penicillium citrinum, ·Penicillium claviforme, ·Penicillium commune, ·Penicillium corylophilum, ·Penicillium corymbiferum, ·Penicillium crustosum, ·Penicillium cyclopium, ·Penicillium daleae, ·Penicillium decumbens, ·Penicillium dierckxii, ·Penicillium digitatum, ·Penicillium digitatum var. latum, ·Penicillium divaricatum, ·Penicillium diversum, ·Penicillium duclauxii, ·Penicillium echinosporum, ·Penicillium expansum, ·Penicillium fellutanum, ·Penicillium frequentans, ·Penicillium funiculosum, ·Penicillium glabrum, ·Penicillium gladioli, ·Penicillium griseofulvum, ·Penicillium hirsutum, ·Penicillium hispanicum, ·Penicillium islandicum, ·Penicillium italicum, ·Penicillium italicum var. avellaneum, ·Penicillium janczewskii, ·Penicillium janthinellum, ·Penicillium japonicum, ·Penicillium lavendulum, ·Penicillium lilacinum, ·Penicillium lividum, ·Penicillium martensii, Penicillium megasporum, ·Penicillium miczynskii, ·Penicillium nalgiovense, ·Penicillium nigricans, ·Penicillium notatum, ·Penicillium ochrochloron, ·Penicillium odoratum, ·Penicillium oxalicum, ·Penicillium paraherquei, ·Penicillium patulum, ·Penicillium pinophilum, ·Penicillium piscarium, ·Penicillium pseudostromaticum, ·Penicillium puberulum, ·Penicillium purpurogenum, ·Penicillium raciborskii, ·Penicillium roqueforti, ·Penicillium rotundum, ·Penicillium rubrum, ·Penicillium sacculum, ·Penicillium simplicissimum, Penicillium sp., Penicillium spinulosum, Penicillium steckii, Penicillium stoloniferum, Penicillium striatisporum, Penicillium striatum, Penicillium tardum, Penicillium thomii, Penicillium turbatum, Penicillium variabile, Penicillium vermiculatum, Penicillium vermoesenii, Penicillium verrucosum, Penicillium verrucosum var. corymbiferum, Penicillium verrucosum var. cyclopium, Penicillium verruculosum, Penicillium vinaceum, Penicillium violaceum, Penicillium viridicatum, Penicillium vulpinum, Trichoderma hamatum, Trichoderma harzianum, Trichoderma koningii, Trichoderma longibrachiatum, Trichoderma polysporum, Trichoderma reesei, Trichoderma virens or Trichoderma viride;* Mortierellaceae such as the genera Mortierella e.g. the species *Mortierella isabellina, Mortierella polycephala, Mortierella ramanniana , Mortierella vinacea or Mortierella zonata;* Mucoraceae such as the genera Actinomucor, Mucor, Phycomyces, Rhizopus, Zygorhynchus e.g. the species *Mucor amphibiorum, Mucor circinelloides f. circinelloides, Mucor circinelloides var. griseocyanus, Mucor flavus, Mucor fuscus, Mucor griseocyanus, Mucor heterosporus, Mucor hiemalis, Mucor hiemalis f. hiemalis, Mucor inaequisporus, Mucor indicus, Mucor javanicus, Mucor mucedo, Mucor mucilagineus, Mucor piriformis, Mucor plasmaticus, Mucor plumbeus, Mucor racemosus, Mucor racemosus f*. *racemosus, Mucor racemosus f. sphaerosporus, Mucor rouxianus, Mucor rouxii, Mucor sinensis, Mucor sp., Mucor spinosus, Mucor tuberculisporus, Mucor variisporus, Mucor variosporus, Mucor wosnessenskii, Phycomyces blakesleeanus, Rhizopus achlamydosporus, Rhizopus arrhizus, Rhizopus chinensis, Rhizopus delemar, Rhizopus formosaensis, Rhizopus japonicus, Rhizopus javanicus, Rhizopus microsporus, Rhizopus microsporus var. chinensis, Rhizopus microsporus var. oligosporus, Rhizopus microsporus var. rhizopodiformis, Rhizopus nigricans, Rhizopus niveus, Rhizopus oligosporus, Rhizopus oryzae, Rhizopus pygmaeus, Rhizopus rhizopodiformis, Rhizopus semarangensis, Rhizopus sontii, Rhizopus stolonifer, Rhizopus thermosus, Rhizopus tonkinensis, Rhizopus tritici or Rhizopus usamii;* Pythiaceae such as the genera Phytium, Phytophthora e.g. the species *Pythium debaryanum, Pythium intermedium, Pythium irregulare, Pythium megalacanthum, Pythium paroecandrum, Pythium sylvaticum, Pythium ultimum, Phytophthora cactorum, Phytophthora cinnamomi, Phytophthora citricola, Phytophthora citrophthora, Phytophthora cryptogea, Phytophthora drechsleri, Phytophthora erythroseptica, Phytophthora lateralis, Phytophthora megasperma, Phytophthora nicotianae, Phytophthora nicotianae var. parasitica, Phytophthora palmivora, Phytophthora parasitica or Phytophthora syringae;* Saccharomycetaceae such as the genera Hansenula, Pichia, Saccharomyces, Saccharomycodes, Yarrowia e.g. the species *Hansenula anomala, Hansenula californica, Hansenula canadensis, Hansenula capsulata, Hansenula ciferrii, Hansenula glucozyma, Hansenula henricii, Hansenula holstii, Hansenula minuta, Hansenula nonfermentans, Hansenula philodendri, Hansenula polymorpha, Hansenula saturnus, Hansenula subpelliculosa, Hansenula wickerhamii, Hansenula wingei, Pichia alcoholophila, Pichia angusta, Pichia anomala, Pichia bispora, Pichia burtonii, Pichia canadensis, Pichia capsulata, Pichia carsonii, Pichia cellobiosa, Pichia ciferrii, Pichia farinosa, Pichia fermentans, Pichia finlandica, Pichia glucozyma, Pichia guilliermondii, Pichia haplophila, Pichia henricii, Pichia holstii, Pichia jadinii, Pichia lindnerii, Pichia membranaefaciens, Pichia methanolica, Pichia minuta var. minuta, Pichia minuta var. nonfermentans, Pichia norvegensis, Pichia ohmeri, Pichia pastoris, Pichia philodendri, Pichia pini, Pichia polymorpha, Pichia quercuum, Pichia rhodanensis, Pichia sargentensis, Pichia stipitis, Pichia strasburgensis, Pichia subpelliculosa, Pichia toletana, Pichia trehalophila, Pichia vini, Pichia xylosa, Saccharomyces aceti, Saccharomyces bailii, Saccharomyces bayanus, Saccharomyces bisporus, Saccharomyces capensis, Saccharomyces carlsbergensis, Saccharomyces cerevisiae, Saccharomyces cerevisiae var. ellipsoideus, Saccharomyces chevalieri, Saccharomyces delbrueckii, Saccharomyces diastaticus, Saccharomyces drosophilarum, Saccharomyces elegans, Saccharomyces ellipsoideus, Saccharomyces fermentati, Saccharomyces florentinus, Saccharomyces fragilis, Saccharomyces heterogenicus, Saccharomyces hienipiensis, Saccharomyces inusitatus, Saccharomyces italicus, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum, Saccharomycodes ludwigii or Yarrowia lipolytica;* Saprolegniaceae such as the genera Saprolegnia e.g. the species *Saprolegnia ferax;* Schizosaccharomycetaceae such as the genera Schizosaccharomyces e.g. the species *Schizosaccharomyces japonicus var. japonicus, Schizosaccharomyces japonicus var. versatilis, Schizosaccharomyces malidevorans, Schizosaccharomyces octosporus, Schizosaccharomyces pombe var. malidevorans or Schizosaccharomyces pombe var. pombe;* Sodariaceae such as the genera Neurospora, Sordaria e.g. the species *Neurospora africana, Neurospora crassa, Neurospora intermedia, Neurospora sitophila, Neurospora tetrasperma, Sordaria fimicola or Sordaria macrospora;* Tuberculariaceae such as the genera Epicoccum, Fusarium, Myrothecium, Sphacelia, Starkeyomyces, Tubercularia e.g. the species *Fusarium acuminatum, Fusarium anthophilum, Fusarium aquaeductuum, Fusarium aquaeductuum var. medium, Fusarium avenaceum, Fusarium buharicum, Fusarium camptoceras, Fusarium cerealis, Fusarium chlamydosporum, Fusarium ciliatum, Fusarium coccophilum, Fusarium coeruleum, Fusarium concolor, Fusarium crookwellense, Fusarium culmorum, Fusarium dimerum, Fusarium diversisporum, Fusarium equiseti, Fusarium equiseti var. bullatum, Fusarium eumartii, Fusarium flocciferum, Fusarium fujikuroi, Fusarium graminearum, Fusarium graminum, Fusarium heterosporum,Fusarium incamatum, Fusarium inflexum, Fusarium javanicum, Fusarium lateritium, Fusarium lateritium var. majus, Fusarium longipes, Fusarium melanochlorum, Fusarium merismoides, Fusarium merismoides var. chlamydosporale, Fusarium moniliforme, Fusarium moniliforme var. anthophilum, Fusarium moniliforme var. subglutinans, Fusarium nivale, Fusarium nivale var. majus, Fusarium oxysporum, Fusarium oxysporum f. sp. aechmeae, Fusarium oxysporum f. sp. cepae, Fusarium oxysporum f. sp. conglutinans, Fusarium oxysporum f. sp. cucumerinum, Fusarium oxysporum f. sp. cyclaminis, Fusarium oxysporum f. sp. dianthi, Fusarium oxysporum f. sp. lycopersici, Fusarium oxysporum f. sp. melonis, Fusarium oxysporum f. sp. passiflorae, Fusarium oxysporum f. sp. pisi, Fusarium oxysporum f. sp. tracheiphilum, Fusarium oxysporum f. sp. tuberosi, Fusarium oxysporum f. sp. tulipae, Fusarium oxysporum f. sp. vasinfectum, Fusarium pallidoroseum, Fusarium poae, Fusarium proliferatum, Fusarium proliferatum var. minus, Fusarium redolens, Fusarium redolens f. sp. dianthi, Fusarium reticulatum, Fusarium roseum, Fusarium sacchari var. elongatum, Fusarium sambucinum, Fusarium sambucinum var. coeruleum, Fusarium semitectum, Fusarium semitectum var. majus, Fusarium solani, Fusarium solani f. sp. pisi, Fusarium sporotrichioides, Fusarium sporotrichioides var. minus, Fusarium sublunatum, Fusarium succisae, Fusarium sulphureum, Fusarium tabacinum, Fusarium tricinctum, Fusarium udum, Fusarium ventricosum, Fusarium verticillioides, Fusarium xylarioides or Fusarium zonatum;* Sporobolomycetaceae such as the genera Bullera, Sporobolomyces, Itersonilia e.g. the species *Sporobolomyces holsaticus, Sporobolomyces odorus, Sporobolomyces puniceus, Sporobolomyces salmonicolor, Sporobolomyces singularis or Sporobolomyces tsugae;* Adelotheciaceae such as the genera e.g. the species *Physcomitrella patens;* Dinophyceae such as the genera Crypthecodinium, Phaeodactylum e.g. the species *Crypthecodinium cohnii or Phaeodactylum tricornutum;* Ditrichaceae such as the genera Ceratodon, Pleuridium, Astomiopsis, Ditrichum, Philibertiella, Ceratodon, Distichium, Skottsbergia e.g. the species *Ceratodon antarcticus, Ceratodon purpureus, Ceratodon purpureus ssp. convolutes or Ceratodon purpureus ssp. stenocarpus;* Prasinophyceae such as the genera Nephroselmis, Prasinococcus, Scherffelia, Tetraselmis, Mantoniella, Ostreococcus e.g. the species *Nephroselmis olivacea, Prasinococcus capsulatus, Scherffelia dubia, Tetraselmis chui, Tetraselmis suecica, Mantoniella squamata or Ostreococcus tauri;* Actinomycetaceae such as the genera Actinomyces, Actinobaculum, Arcanobacterium, Mobiluncus e.g. the species *Actinomyces bemardiae, Actinomyces bovis, Actinomyces bowdenii, Actinomyces canis, Actinomyces cardiffensis, Actinomyces catuli, Actinomyces coleocanis, Actinomyces denticolens, Actinomyces europaeus, Actinomyces funkei, Actinomyces georgiae, Actinomyces gerencseriae, Actinomyces hordeovulneris, Actinomyces howellii, Actinomyces humiferus, Actinomyces hyovaginalis, Actinomyces israelii, Actinomyces marimammalium, Actinomyces meyeri, Actinomyces naeslundii, Actinomyces nasicola, Actinomyces neuii* subsp. *anitratus, Actinomyces neuii* subsp. *neuii, Actinomyces odontolyticus, Actinomyces oricola, Actinomyces pyogenes, Actinomyces radicidentis, Actinomyces radingae, Actinomyces stackii, Actinomyces suimastitidis, Actinomyces suis, Actinomyces turicensis, Actinomyces urogenitalis, Actinomyces vaccimaxillae, Actinomyces viscosus, Actinobaculum schaalii, Actinobaculum suis, Actinobaculum urinale, Arcanobacterium bemardiae, Arcanobacterium haemolyticum, Arcanobacterium hippocoleae, Arcanobacterium phocae, Arcanobacterium pluranimalium, Arcanobacterium pyogenes, Mobiluncus curtisii* subsp. *curtisii, Mobiluncus curtisii* subsp. *holmesii or Mobiluncus mulieris;* Bacillaceae such as the genera Amphibacillus, Anoxybacillus, Bacillus, Exiguobacterium, Gracilibacillus, Holobacillus, Saccharococcus, Salibacillus, Virgibacillus e.g. the species *Amphibacillus fermentum, Amphibacillus tropicus, Amphibacillus xylanus, Anoxybacillus flavithermus, Anoxybacillus gonensis, Anoxybacillus pushchinoensis, Bacillus acidocaldarius, Bacillus acidoterrestris, Bacillus aeolius, Bacillus agaradhaerens, Bacillus agri, Bacillus alcalophilus, Bacillus alginolyticus, Bacillus alvei, Bacillus amyloliquefaciens, Bacillus amylolyticus, Bacillus aneurinilyticus, Bacillus aquimaris, Bacillus arseniciselenatis, Bacillus atrophaeus, Bacillus azotofixans, Bacillus azotoformans, Bacillus badius, Bacillus barbaricus, Bacillus benzoevorans, Bacillus borstelensis, Bacillus brevis, Bacillus carboniphilus, Bacillus centrosporus, Bacillus cereus, Bacillus chitinolyticus, Bacillus chondroitinus, Bacillus choshinensis, Bacillus circulans, Bacillus clarkii, Bacillus clausii, Bacillus coagulans, Bacillus cohnii, Bacillus curdlanolyticus, Bacillus cycloheptanicus, Bacillus decolorationis, Bacillus dipsosauri, Bacillus edaphicus, Bacillus ehimensis, Bacillus endophyticus, Bacillus fastidiosus, Bacillus firmus, Bacillus flexus, Bacillus formosus, Bacillus fumarioli, Bacillus funiculus, Bacillus fusiformis, Bacillus sphaericus subsp. fusiformis, Bacillus galactophilus, Bacillus globisporus, Bacillus globisporus subsp. marinus, Bacillus glucanolyticus, Bacillus gordonae, Bacillus halmapalus, Bacillus haloalkaliphilus, Bacillus halodenitriricans, Bacillus halodurans, Bacillus halophilus, Bacillus horikoshii, Bacillus horti, Bacillus infernos, Bacillus insolitus, Bacillus jeotgali, Bacillus kaustophilus, Bacillus kobensis, Bacillus krulwichiae; Bacillus laevolacticus, Bacillus.larvae, Bacillus laterosporus, Bacillus lautus, Bacillus lentimorbus, Bacillus lentus, Bacillus licheniformis, Bacillus luciferensis, Bacillus macerans, Bacillus macquariensis, Bacillus marinus, Bacillus marisflavi, Bacillus marismortui, Bacillus megaterium, Bacillus methanolicus, Bacillus migulanus, Bacillus mojavensis, Bacillus mucilaginosus, Bacillus mycoides, Bacillus naganoensis, Bacillus nealsonii, Bacillus neidei, Bacillus niacini, Bacillus okuhidensis, Bacillus oleronius, Bacillus pabuli, Bacillus pallidus, Bacillus pantothenticus, Bacillus parabrevis, Bacillus pasteurii, Bacillus peoriae, Bacillus polymyxa, Bacillus popilliae, Bacillus pseudalcaliphilus, Bacillus pseudofirmus, Bacillus pseudomycoides, Bacillus psychrodurans, Bacillus psychrophilus, Bacillus psychrosaccharolyticus, Bacillus psychrotolerans, Bacillus pulvifaciens, Bacillus pumilus, Bacillus pycnus, Bacillus reuszeri, Bacillus salexigens, Bacillus schlegelii, Bacillus selenitireducens, Bacillus silvestris, Bacillus simplex, Bacillus siralis, Bacillus smithii, Bacillus sonorensis, Bacillus sphaericus, Bacillus sporothermodurans, Bacillus stearothermophilus , Bacillus subterraneus, Bacillus subtilis subsp. spizizenii, Bacillus subtilis subsp. subtilis, Bacillus thermantarcticus, Bacillus thermoaerophilus, Bacillus thermoamylovorans, Bacillus thermoantarcticus, Bacillus thermocatenulatus, Bacillus thermocloacae, Bacillus thermodenitrificans, Bacillus thermoglucosidasius, Bacillus thermoleovorans, Bacillus thermoruber, Bacillus thermosphaericus, Bacillus thiaminolyticus, Bacillus thuringiensis, Bacillus tusciae, Bacillus validus, Bacillus vallismortis, Bacillus vedderi, Bacillus vulcani, Bacillus weihenstephanensis, Exiguobacterium acetylicum, Exiguobacterium antarcticum, Exiguobacterium aurantiacum, Exiguobacterium undae, Gracilibacillus dipsosauri, Gracilibacillus halotolerans, Halobacillus halophilus, Halobacillus karajensis, Halobacillus litoralis, Halobacillus salinus, Halobacillus trueperi, Saccharococcus caldoxylosilyticus, Saccharococcus thermophilus, Salibacillus marismortui, Salibacillus salexigens, Virgibacillus carmonensis, Virgibacillus marismortui, Virgibacillus necropolis, Virgibacillus pantothenticus, Virgibacillus picturae, Virgibacillus proomii or Virgibacillus salexigens,* Brevibacteriaceae such as the genera Brevibacterium e.g. the species *Brevibacterium acetylicum, Brevibacterium albidum, Brevibacterium ammoniagenes, Brevibacterium avium, Brevibacterium casei, Brevibacterium citreum, Brevibacterium divaricatum, Brevibacterium epidermidis, Brevibacterium fermentans, Brevibacterium frigoritolerans, Brevibacterium halotolerans, Brevibacterium imperiale, Brevibacterium incertum, Brevibacterium iodinum, Brevibacterium linens, Brevibacterium liquefaciens, Brevibacterium lutescens, Brevibacterium luteum, Brevibacterium lyticum, Brevibacterium mcbrellneri, Brevibacterium otitidis, Brevibacterium oxydans, Brevibacterium paucivorans, Brevibacterium protophormiae, Brevibacterium pusillum, Brevibacterium saperdae, Brevibacterium stationis, Brevibacterium testaceum or Brevibacterium vitaeruminis;* Corynebacteriaceae such as the genera Corynebacterium e.g. the species *Corynebacterium accolens, Corynebacterium afermentans* subsp. *afermentans, Corynebacterium afermentans* subsp. *lipophilum, Corynebacterium ammoniagenes, Corynebacterium amyco*/*atum, Corynebacterium appendicis, Corynebacterium aquilae, Corynebacterium argentoratense, Corynebacterium atypicum, Corynebacterium aurimucosum, Corynebacterium auris, Corynebacterium auriscanis, Corynebacterium betae, Corynebacterium beticola, Corynebacterium bovis, Corynebacterium callunae, Corynebacterium camporealensis, Corynebacterium capitovis, Corynebacterium casei, Corynebacterium confusum, Corynebacterium coyleae, Corynebacterium cystitidis, Corynebacterium durum, Corynebacterium efficiens, Corynebacterium equi, Corynebacterium falsenii, Corynebacterium fascians, Corynebacterium felinum, Corynebacterium flaccumfaciens, Corynebacterium flavescens, Corynebacterium freneyi, Corynebacterium glaucum, Corynebacterium glucuronolyticum, Corynebacterium glutamicum, Corynebacterium hoagii, Corynebacterium ilicis, Corynebacterium imitans, Corynebacterium insidiosum, Corynebacterium iranicum, Corynebacterium jeikeium, Corynebacterium kroppenstedtii, Corynebacterium kutscheri, Corynebacterium lilium, Corynebacterium lipophiloflavum, Corynebacterium macginleyi, Corynebacterium mastitidis, Corynebacterium matruchotii, Corynebacterium michiganense, Corynebacterium michiganense* subsp. *tessellarius, Corynebacterium minutissimum, Corynebacterium mooreparkense, Corynebacterium mucifaciens, Corynebacterium mycetoides, Corynebacterium nebraskense, Corynebacterium oortii, Corynebacterium paurometabolum, Corynebacterium phocae, Corynebacterium pilosum, Corynebacterium poinsettiae, Corynebacterium propinquum, Corynebacterium pseudodiphtheriticum, Corynebacterium pseudotuberculosis, Corynebacterium pyogenes, Corynebacterium rathayi, Corynebacterium renale, Corynebacterium riegelii, Corynebacterium seminale, Corynebacterium sepedonicum, Corynebacterium simulans, Corynebacterium singulare, Corynebacterium sphenisci, Corynebacterium spheniscorum, Corynebacterium striatum, Corynebacterium suicordis, Corynebacterium sundsvallense, Corynebacterium terpenotabidum, Corynebacterium testudinoris, Corynebacterium thomssenii, Corynebacterium tritici, Corynebacterium ulcerans, Corynebacterium urealyticum, Corynebacterium variabile, Corynebacterium vitaeruminis or Corynebacterium xerosis;* Enterobacteriacae such as the genera Alterococcus, Arsenophonus, Brenneria, Buchnera, Budvicia, Buttiauxella, Calymmatobacterium, Cedecea, Citrobacter, Edwardsiella, Enterobacter, Erwinia, Escherichia, Ewingella, Hafnia, Klebsiella, Kluyvera, Leclercia, Leminorella, Moellerella, Morganella, Obesumbacterium, Pantoea, Pectobacterium, Photorhabdus, Plesiomonas, Pragia, Proteus, Providencia, Rahnella, Saccharobacter, Salmonella, Shigella, Serratia, Sodalis, Tatumella, Trabulsiella, Wigglesworthia, Xenorhabdus,Yersinia and Yokenella e.g. the species *Arsenophonus nasoniae, Brenneria alni, Brenneria nigrifluens, Brenneria quercina, Brenneria rubrifaciens, Brenneria salicis, Budvicia aquatica, Buttiauxella agrestis, Buttiauxella brennerae, Buttiauxella ferragutiae, Buttiauxella gaviniae, Buttiauxella izardii, Buttiauxella noackiae, Buttiauxella warmboldiae, Cedecea davisae,* Cedecea *lapagei, Cedecea neteri, Citrobacter amalonaticus, Citrobacter diversus, Citrobacter freundii, Citrobacter genomospecies, Citrobacter gillenii, Citrobacter intermedium, Citrobacter koseri, Citrobacter murliniae, Citrobacter sp., Edwardsiella hoshinae, Edwardsiella ictaluri, Edwardsiella tarda, Erwinia alni, Erwinia amylovora, Erwinia ananatis, Erwinia aphidicola, Erwinia billingiae, Erwinia cacticida, Erwinia cancerogena, Erwinia camegieana, Erwinia carotovora* subsp. *atroseptica, Erwinia carotovora* subsp. *betavasculorum, Erwinia carotovora* subsp. *odorifera, Erwinia carotovora* subsp. *wasabiae, Erwinia chrysanthemi, Erwinia cypripedii, Erwinia dissolvens, Erwinia herbicola, Erwinia mallotivora, Erwinia milletiae, Erwinia nigrifluens, Erwinia nimipressuralis, Erwinia persicina, Erwinia psidii, Erwinia pyrifoliae, Erwinia quercina, Erwinia rhapontici, Erwinia rubrifaciens, Erwinia salicis, Erwinia stewartii, Erwinia tracheiphila, Erwinia uredovora, Escherichia adecarboxylata, Escherichia anindolica, Escherichia aurescens, Escherichia blattae, Escherichia coli, Escherichia coli var. communior, Escherichia coli-mutabile, Escherichia fergusonii, Escherichia hermannii, Escherichia sp., Escherichia vulneris, Ewingella americana, Hafnia alvei, Klebsiella aerogenes, Klebsiella edwardsii subsp. atlantae, Klebsiella ornithinolytica, Klebsiella oxytoca, Klebsiella planticola, Klebsiella pneumoniae, Klebsiella pneumoniae subsp. pneumoniae, Klebsiella sp., Klebsiella terrigena, Klebsiella trevisanii, Kluyvera ascorbata, Kluyvera citrophila, Kluyvera cochleae, Kluyvera cryocrescens, Kluyvera georgiana, Kluyvera noncitrophila, Kluyvera sp., Leclercia adecarboxylata, Leminorella grimontii, Leminorella richardii, Moellerella wisconsensis, Morganella morganii, Morganella morganii subsp. morganii, Morganella morganii subsp. sibonii, Obesumbaterium proteus, Pantoea agglomerans, Pantoea ananatis, Pantoea citrea, Pantoea dispersa, Pantoea punctata, Pantoea stewartii subsp. stewartii, Pantoea terrea, Pectobacterium atrosepticum, Pectobacterium carotovorum subsp. atrosepticum, Pectobacterium carotovorum subsp. carotovorum, Pectobacterium chrysanthemi, Pectobacterium cypripedii, Photorhabdus asymbiotica, Photorhabdus luminescens, Photorhabdus luminescens subsp. akhurstii, Photorhabdus luminescens subsp. laumondii, Photorhabdus luminescens subsp. luminescens, Photorhabdus sp., Photorhabdus temperata, Plesiomonas shigelloides, Pragia fontium, Proteus hauseri, Proteus ichthyosmius, Proteus inconstans, Proteus mirabilis, Proteus morganii, Proteus myxofaciens, Proteus penneri, Proteus rettgeri, Proteus shigelloides, Proteus vulgaris, Providencia alcalifaciens, Providencia friedericiana, Providencia heimbachae, Providencia rettgeri, Providencia rustigianii, Providencia stuartii, Rahnella aquatilis, Salmonella abony, Salmonella arizonae, Salmonella bongori, Salmonella choleraesuis subsp. arizonae, Salmonella choleraesuis subsp. bongori, Salmonella choleraesuis subsp. cholereasuis, Salmonella choleraesuis subsp. diarizonae, Salmonella choleraesuis subsp. houtenae, Salmonella choleraesuis subsp. indica, Salmonella choleraesuis subsp. salamae, Salmonella daressalaam, Salmonella enterica subsp. houtenae, Salmonella enterica subsp. salamae, Salmonella enteritidis, Salmonella gallinarum, Salmonella heidelberg, Salmonella panama, Salmonella senftenberg, Salmonella typhimurium, Serratia entomophila, Serratia ficaria, Serratia fonticola, Serratia grimesii, Serratia liquefaciens, Serratia marcescens, Serratia marcescens subsp. marcescens, Serratia marinorubra, Serratia odorifera, Serratia plymouthensis, Serratia plymuthica, Serratia proteamaculans, Serratia proteamaculans subsp. quinovora, Serratia quinivorans, Serratia rubidaea, Shigella boydii, Shigella flexneri, Shigella paradysenteriae, Shigella sonnei, Tatumella ptyseos, Xenorhabdus beddingii, Xenorhabdus bovienii, Xenorhabdus luminescens, Xenorhabdus nematophila, Xenorhabdus nematophila subsp. beddingii, Xenorhabdus nematophila subsp. bovienii, Xenorhabdus nematophila subsp. poinarii or Xenorhabdus poinarii;* Gordoniaceae such as the genera Gordonia, Skermania e.g. the species *Gordonia aichiensis, Gordonia alkanivorans, Gordonia amarae, Gordonia amicalis, Gordonia bronchialis, Gordonia desulfuricans, Gordonia hirsuta, Gordonia hydrophobica, Gordonia namibiensis, Gordonia nitida, Gordonia paraffinivorans, Gordonia polyisoprenivorans, Gordonia rhizosphera, Gordonia rubripertincta, Gordonia sihwensis, Gordonia sinesedis, Gordonia sputi, Gordonia terrae or Gordonia westfalica;* Micrococcaceae such as the genera Micrococcus, Arthrobacter, Kocuria, Nesterenkonia, Renibacterium, Rothia, Stomatococcus e.g. the species *Micrococcus agilis, Micrococcus antarcticus, Micrococcus halobius, Micrococcus kristinae, Micrococcus luteus, Micrococcus lylae, Micrococcus nishinomiyaensis, Micrococcus roseus, Micrococcus sedentarius, Micrococcus varians, Arthrobacter agilis, Arthrobacter albus, Arthrobacter atrocyaneus, Arthrobacter aurescens, Arthrobacter chlorophenolicus, Arthrobacter citreus, Arthrobacter creatinolyticus, Arthrobacter crystallopoietes, Arthrobacter cumminsii, Arthrobacter duodecadis, Arthrobacter flavescens, Arthrobacter flavus, Arthrobacter gandavensis, Arthrobacter globiformis, Arthrobacter histidinolovorans, Arthrobacter ilicis, Arthrobacter koreensis, Arthrobacter luteolus, Arthrobacter methylotrophus, Arthrobacter mysorens, Arthrobacter nasiphocae, Arthrobacter nicotianae, Arthrobacter nicotinovorans, Arthrobacter oxydans, Arthrobacter pascens, Arthrobacter picolinophilus, Arthrobacter polychromogenes, Arthrobacter protophormiae, Arthrobacter psychrolactophilus, Arthrobacter radiotolerans, Arthrobacter ramosus, Arthrobacter rhombi, Arthrobacter roseus, Arthrobacter siderocapsulatus, Arthrobacter simplex, Arthrobacter sulfonivorans, Arthrobacter sulfureus, Arthrobacter terregens, Arthrobacter tumescens, Arthrobacter uratoxydans, Arthrobacter ureafaciens, Arthrobacter variabilis, Arthrobacter viscosus, Arthrobacter woluwensis, Kocuria erythromyxa, Kocuria kristinae, Kocuria palustris, Kocuria polaris, Kocuria rhizophila, Kocuria rosea, Kocuria varians, Nesterenkonia halobia, Nesterenkonia lacusekhoensis, Renibacterium salmoninarum, Rothia amarae, Rothia dentocariosa, Rothia mucilaginosa, Rothia nasimurium or Stomatococcus mucilaginosus;* Mycobacteriaceae such as the genera Mycobacterium e.g. the species *Mycobacterium africanum, Mycobacterium agri, Mycobacterium aichiense, Mycobacterium alvei, Mycobacterium asiaticum, Mycobacterium aurum, Mycobacterium austroafricanum, Mycobacterium bohemicum, Mycobacterium botniense, Mycobacterium brumae, Mycobacterium chelonae* subsp. *abscessus, Mycobacterium chitae, Mycobacterium chlorophenolicum, Mycobacterium chubuense, Mycobacterium confluentis, Mycobacterium cookii, Mycobacterium diernhoferi, Mycobacterium doricum, Mycobacterium duvalii, Mycobacterium fallax, Mycobacterium farcinogenes, Mycobacterium flavescens, Mycobacterium frederiksbergense, Mycobacterium gadium, Mycobacterium gilvum, Mycobacterium gordonae, Mycobacterium hassiacum, Mycobacterium hibemiae, Mycobacterium hodleri, Mycobacterium holsaticum, Mycobacterium komossense, Mycobacterium lacus, Mycobacterium madagascariense, Mycobacterium mageritense, Mycobacterium montefiorense, Mycobacterium moriokaense, Mycobacterium murale, Mycobacterium neoaurum, Mycobacterium nonchromogenicum, Mycobacterium obuense, Mycobacterium palustre, Mycobacterium parafortuitum, Mycobacterium peregrinum, Mycobacterium phlei, Mycobacterium pinnipedii, Mycobacterium poriferae, Mycobacterium pulveris, Mycobacterium rhodesiae, Mycobacterium shottsii, Mycobacterium sphagni, Mycobacterium terrae, Mycobacterium thermoresistibile, Mycobacterium tokaiense, Mycobacterium triviale, Mycobacterium tusciae or Mycobacterium vanbaalenii;* Nocardiaceae such as the genera Nocardia, Rhodococcus e.g. the species *Nocardia abscessus, Nocardia africana, Nocardia amarae, Nocardia asteroides, Nocardia autotrophica, Nocardia beijingensis, Nocardia brasiliensis, Nocardia brevicatena, Nocardia caishijiensis, Nocardia calcarea, Nocardia camea, Nocardia cellulans, Nocardia cerradoensis, Nocardia coeliaca, Nocardia corynebacterioides, Nocardia crassostreae, Nocardia cummidelens, Nocardia cyriacigeorgica, Nocardia farcinica, Nocardia flavorosea, Nocardia fluminea, Nocardia globerula, Nocardia hydrocarbonoxydans, Nocardia ignorata, Nocardia mediterranei, Nocardia nova, Nocardia orientalis, Nocardia otitidis-caviarum, Nocardia otitidiscaviarum, Nocardia paucivorans, Nocardia petroleophila, Nocardia pinensis, Nocardia pseudobrasiliensis, Nocardia pseudovaccinii, Nocardia puris, Nocardia restricta, Nocardia rugosa, Nocardia salmonicida, Nocardia satumea, Nocardia seriolae, Nocardia soli, Nocardia sulphurea, Nocardia transvalensis, Nocardia uniformis, Nocardia vaccinii, Nocardia veterana or Nocardia vinacea;* Pseudomonaceae such as the genera Azomonas, Azotobacter, Cellvibrio, Chryseomonas, Flaviomonas, Lampropedia, Mesophilobacter, Morococcus, Oligella, Pseudomonas, Rhizobacter, Rugamonas, Serpens, Thermoleophilum, Xylophilus e.g. the species *Azomonas agilis, Azomonas insignis, Azomonas macrocytogenes, Azotobacter agilis, Azotobacter agilis subsp. armeniae, Azotobacter armeniacus, Azotobacter beijerinckii, Azotobacter chroococcum, Azotobacter indicum, Azotobacter macrocytogenes, Azotobacter miscellum, Azotobacter nigricans subsp. nigricans, Azotobacter paspali, Azotobacter salinestris, Azotobacter sp., Azotobacter vinelandii, Flavimonas oryzihabitans, Mesophilobacter marinus, Oligella urethralis, Pseudomonas acidovorans, Pseudomonas aeruginosa, Pseudomonas agarici, Pseudomonas alcaligenes, Pseudomonas aminovorans, Pseudomonas amygdali, Pseudomonas andropogonis, Pseudomonas anguilliseptica, Pseudomonas antarctica, Pseudomonas antimicrobica, Pseudomonas antimycetica, Pseudomonas aptata, Pseudomonas arvilla, Pseudomonas asplenii, Pseudomonas atlantica, Pseudomonas atrofaciens, Pseudomonas aureofaciens, Pseudomonas avellanae, Pseudomonas azelaica, Pseudomonas azotocolligans, Pseudomonas balearica, Pseudomonas barkeri, Pseudomonas bathycetes, Pseudomonas beijerinckii, Pseudomonas brassicacearum, Pseudomonas brenneri, Pseudomonas butanovora, Pseudomonas carboxydoflava, Pseudomonas carboxydohydrogena, Pseudomonas carboxydovorans, Pseudomonas carrageenovora, Pseudomonas caryophylli, Pseudomonas cepacia, Pseudomonas chloritidismutans, Pseudomonas chlororaphis, Pseudomonas cichorii, Pseudomonas citronellolis, Pseudomonas cocovenenans, Pseudomonas compransoris, Pseudomonas congelans, Pseudomonas coronafaciens, Pseudomonas corrugata, Pseudomonas dacunhae, Pseudomonas delafieldii, Pseudomonas delphinii, Pseudomonas denitrificans, Pseudomonas desmolytica, Pseudomonas diminuta, Pseudomonas doudoroffii, Pseudomonas echinoides, Pseudomonas elongata, Pseudomonas extorquens, Pseudomonas extremorientalis, Pseudomonas facilis, Pseudomonas ficuserectae, Pseudomonas flava, Pseudomonas flavescens, Pseudomonas fluorescens, Pseudomonas fragi, Pseudomonas frederiksbergensis, Pseudomonas fulgida, Pseudomonas fuscovaginae, Pseudomonas gazotropha, Pseudomonas gladioli, Pseudomonas glathei, Pseudomonas glumae, Pseudomonas graminis, Pseudomonas halophila, Pseudomonas helianthi, Pseudomonas huttiensis, Pseudomonas hydrogenothermophila, Pseudomonas hydrogenovora, Pseudomonas indica, Pseudomonas indigofera, Pseudomonas iodinum, Pseudomonas kilonensis, Pseudomonas lachrymans, Pseudomonas lapsa, Pseudomonas lemoignei, Pseudomonas lemonnieri, Pseudomonas lundensis, Pseudomonas luteola, Pseudomonas maltophilia, Pseudomonas marginalis, Pseudomonas marginata, Pseudomonas marina, Pseudomonas meliae, Pseudomonas mendocina, Pseudomonas mesophilica, Pseudomonas mixta, Pseudomonas monteilii, Pseudomonas morsprunorum, Pseudomonas multivorans, Pseudomonas natriegens, Pseudomonas nautica, Pseudomonas nitroreducens, Pseudomonas oleovorans, Pseudomonas oryzihabitans, Pseudomonas ovalis, Pseudomonas oxalaticus, Pseudomonas palleronii, Pseudomonas paucimobilis, Pseudomonas phaseolicola, Pseudomonas phenazinium, Pseudomonas pickettii, Pseudomonas pisi, Pseudomonas plantarii, Pseudomonas plecoglossicida, Pseudomonas poae, Pseudomonas primulae, Pseudomonas proteolytica, Pseudomonas pseudoalcaligenes, Pseudomonas pseudoalcaligenes* subsp. *konjaci, Pseudomonas pseudoalcaligenes* subsp. *pseudoalcaligenes, Pseudomonas pseudoflava, Pseudomonas putida, Pseudomonas putida* var. *naraensis, Pseudomonas putrefaciens, Pseudomonas pyrrocinia, Pseudomonas radiora, Pseudomonas reptilivora, Pseudomonas rhodesiae, Pseudomonas rhodos, Pseudomonas riboflavina, Pseudomonas rubescens, Pseudomonas rubrisubalbicans, Pseudomonas ruhlandii, Pseudomonas saccharophila, Pseudomonas savastanoi, Pseudomonas savastanoi* pvar. *glycinea, Pseudomonas savastanoi* pvar. *phaseolicola, Pseudomonas solanacearum, Pseudomonas* sp., *Pseudomonas spinosa, Pseudomonas stanieri, Pseudomonas stutzeri, Pseudomonas syringae, Pseudomonas syringae* pvar. *aptata, Pseudomonas syringae* pvar. *atrofaciens, Pseudomonas syringae* pvar. *coronafaciens, Pseudomonas syringae* pvar. *delphinii, Pseudomonas syringae* pvar. *glycinea, Pseudomonas syringae* pvar. *helianthi, Pseudomonas syringae* pvar. *lachrymans, Pseudomonas syringae* pvar. *lapsa, Pseudomonas syringae* pvar. *morsprunorum, Pseudomonas syringae* pvar. *phaseolicola, Pseudomonas syringae* pvar. *primulae, Pseudomonas syringae* pvar. *syringae, Pseudomonas syringae* pvar. *tabaci, Pseudomonas syringae* pvar. *tomato, Pseudomonas syringae* subsp. *glycinea, Pseudomonas syringae* subsp. *savastanoi, Pseudomonas syringae* subsp. *syringae, Pseudomonas syzygii, Pseudomonas tabaci, Pseudomonas taeniospiralis, Pseudomonas testosteroni, Pseudomonas thermocarboxydovorans, Pseudomonas thermotolerans, Pseudomonas thivervalensis, Pseudomonas tomato, Pseudomonas trivialis, Pseudomonas veronii, Pseudomonas vesicularis, Pseudomonas viridiflava, Pseudomonas viscogena, Pseudomonas woodsii, Rhizobacter dauci, Rhizobacter daucus or Xylophilus ampelinus;* Rhizobiaceae such as the genera Agrobacterium, Carbophilus, Chelatobacter, Ensifer, Rhizobium, Sinorhizobium e.g. the species *Agrobacterium atlanticum, Agrobacterium ferrugineum, Agrobacterium gelatinovorum, Agrobacterium larrymoorei, Agrobacterium meteori, Agrobacterium radiobacter, Agrobacterium rhizogenes, Agrobacterium rubi, Agrobacterium stellulatum, Agrobacterium tumefaciens, Agrobacterium vitis, Carbophilus carboxidus, Chelatobacter heintzii, Ensifer adhaerens, Ensifer arboris, Ensifer fredii, Ensifer kostiensis, Ensifer kummerowiae, Ensifer medicae, Ensifer meliloti, Ensifer saheli, Ensifer terangae, Ensifer xinjiangensis, Rhizobium ciceri Rhizobium etli, Rhizobium fredii, Rhizobium galegae, Rhizobium gallicum, Rhizobium giardinii, Rhizobium hainanense, Rhizobium huakuii, Rhizobium huautlense, Rhizobium indigoferae, Rhizobium japonicum, Rhizobium leguminosarum, Rhizobium loessense, Rhizobium loti, Rhizobium lupini, Rhizobium mediterraneum, Rhizobium meliloti, Rhizobium mongolense, Rhizobium phaseoli, Rhizobium radiobacter, Rhizobium rhizogenes, Rhizobium rubi, Rhizobium sullae, Rhizobium tianshanense, Rhizobium trifolii, Rhizobium tropici, Rhizobium undicola, Rhizobium vitis, Sinorhizobium adhaerens, Sinorhizobium arboris, Sinorhizobium fredii, Sinorhizobium kostiense, Sinorhizobium kummerowiae, Sinorhizobium medicae, Sinorhizobium meliloti, Sinorhizobium morelense, Sinorhizobium saheli or Sinorhizobium xinjiangense;* Streptomycetaceae such as the genera Kitasatosprora, Streptomyces, Streptoverticillium e.g. the species *Streptomyces abikoensis, Streptomyces aburaviensis, Streptomyces achromogenes subsp. achromogenes, Streptomyces achromogenes subsp. rubradiris, Streptomyces acidiscabies, Streptomyces acrimycini, Streptomyces aculeolatus, Streptomyces afghaniensis, Streptomyces alanosinicus, Streptomyces albaduncus, Streptomyces albiaxialis, Streptomyces albidochromogenes, Streptomyces albidoflavus, Streptomyces albireticuli, Streptomyces albofaciens, Streptomyces alboflavus, Streptomyces albogriseolus, Streptomyces albolongus, Streptomyces alboniger, Streptomyces albospinus, Streptomyces albosporeus subsp. albosporeus, Streptomyces albosporeus subsp. labilomyceticus, Streptomyces alboverticillatus, Streptomyces albovinaceus, Streptomyces alboviridis, Streptomyces albulus, Streptomyces albus subsp. albus, Streptomyces albus subsp. pathocidicus, Streptomyces almquistii, Streptomyces althioticus, Streptomyces amakusaensis, Streptomyces ambofaciens, Streptomyces aminophilus, Streptomyces anandii, Streptomyces anthocyanicus, Streptomyces antibioticus, Streptomyces antimycoticus, Streptomyces anulatus, Streptomyces arabicus, Streptomyces ardus, Streptomyces arenae, Streptomyces argenteolus, Streptomyces armeniacus, Streptomyces asiaticus, Streptomyces asterosporus, Streptomyces atratus, Streptomyces atroaurantiacus, Streptomyces atroolivaceus, Streptomyces atrovirens, Streptomyces aurantiacus, Streptomyces aurantiogriseus, Streptomyces aureocirculatus, Streptomyces aureofaciens, Streptomyces aureorectus, Streptomyces aureoversilis, Streptomyces aureoverticillatus, Streptomyces aureus, Streptomyces avellaneus, Streptomyces avermectinius, Streptomyces avermitilis, Streptomyces avidinii, Streptomyces azaticus, Streptomyces azureus, Streptomyces baarnensis, Streptomyces bacillaris, Streptomyces badius, Streptomyces baldaccii, Streptomyces bambergiensis, Streptomyces beijiangensis, Streptomyces bellus, Streptomyces bikiniensis, Streptomyces biverticillatus, Streptomyces blastmyceticus, Streptomyces bluensis, Streptomyces bobili, Streptomyces bottropensis, Streptomyces brasiliensis, Streptomyces bungoensis, Streptomyces cacaoi subsp. asoensis, Streptomyces cacaoi subsp. cacaoi, Streptomyces caelestis, Streptomyces caeru*/*eus, Streptomyces californicus, Streptomyces ca*/*vus, Streptomyces canaries, Streptomyces candidus, Streptomyces canescens, Streptomyces cangkringensis, Streptomyces caniferus, Streptomyces canus, Streptomyces capillispiralis, Streptomyces capoamus, Streptomyces carpaticus, Streptomyces carpinensis, Streptomyces catenulae, Streptomyces caviscabies, Streptomyces cavourensis subsp. cavourensis, Streptomyces cavourensis subsp. washingtonensis, Streptomyces cellostaticus, Streptomyces celluloflavus, Streptomyces cellulolyticus, Streptomyces cellulosae, Streptomyces champavatii, Streptomyces chartreuses, Streptomyces chattanoogensis, Streptomyces chibaensis, Streptomyces chrestomyceticus, Streptomyces chromofuscus, Streptomyces chryseus, Streptomyces chrysomallus subsp. chrysomallus, Streptomyces chrysomallus subsp. fumigatus, Streptomyces cinereorectus, Streptomyces cinereoruber subsp. cinereoruber, Streptomyces cinereoruber subsp. fructofermentans, Streptomyces cinereospinus, Streptomyces cinereus, Streptomyces cinerochromogenes, Streptomyces cinnabarinus, Streptomyces cinnamonensis, Streptomyces cinnamoneus, Streptomyces cinnamoneus subsp. albosporus, Streptomyces cinnamoneus subsp. cinnamoneus, Streptomyces cinnamoneus subsp. lanosus, Streptomyces cinnamoneus subsp. sparsus, Streptomyces cirratus, Streptomyces ciscaucasicus, Streptomyces citreofluorescens, Streptomyces clavifer, Streptomyces clavuligerus, Streptomyces cochleatus, Streptomyces coelescens, Streptomyces coelicoflavus, Streptomyces coelicolor, Streptomyces coeruleoflavus, Streptomyces coeruleofuscus, Streptomyces coeruleoprunus, Streptomyces coeruleorubidus, Streptomyces coerulescens, Streptomyces collinus, Streptomyces colombiensis, Streptomyces corchorusii, Streptomyces costaricanus, Streptomyces cremeus, Streptomyces crystallinus, Streptomyces curacoi, Streptomyces cuspidosporus, Streptomyces cyaneofuscatus, Streptomyces cyaneus, Streptomyces cyanoalbus, Streptomyces cystargineus, Streptomyces daghestanicus, Streptomyces diastaticus subsp. ardesiacus, Streptomyces diastaticus subsp. diastaticus, Streptomyces diastatochromogenes, Streptomyces distallicus, Streptomyces djakartensis, Streptomyces durhamensis, Streptomyces echinatus, Streptomyces echinoruber, Streptomyces ederensis, Streptomyces ehimensis, Streptomyces endus, Streptomyces enissocaesilis, Streptomyces erumpens, Streptomyces erythraeus, Streptomyces erythrogriseus, Streptomyces eurocidicus, Streptomyces europaeiscabiei, Streptomyces eurythermus, Streptomyces exfoliates, Streptomyces felleus, Streptomyces fervens, Streptomyces fervens subsp. fervens, Streptomyces fervens subsp. melrosporus, Streptomyces filamentosus, Streptomyces filipinensis, Streptomyces fimbriatus, Streptomyces fimicarius, Streptomyces finlayi, Streptomyces flaveolus, Streptomyces flaveus, Streptomyces flavidofuscus, Streptomyces flavidovirens, Streptomyces flaviscleroticus, Streptomyces flavofungini, Streptomyces flavofuscus, Streptomyces flavogriseus, Streptomyces flavopersicus, Streptomyces flavotricini, Streptomyces flavovariabilis, Streptomyces flavovirens, Streptomyces flavoviridis, Streptomyces flocculus, Streptomyces floridae, Streptomyces fluorescens, Streptomyces fradiae, Streptomyces fragilis, Streptomyces fulvissimus, Streptomyces fulvorobeus, Streptomyces fumanus, Streptomyces fumigatiscleroticus, Streptomyces galbus, Streptomyces galilaeus, Streptomyces gancidicus, Streptomyces gardneri, Streptomyces gelaticus, Streptomyces geysiriensis, Streptomyces ghanaensis, Streptomyces gibsonii, Streptomyces glaucescens, Streptomyces glaucosporus, Streptomyces glaucus, Streptomyces globisporus subsp. caucasicus, Streptomyces globisporus subsp. flavofuscus, Streptomyces globisporus subsp. globisporus, Streptomyces globosus, Streptomyces glomeratus, Streptomyces glomeroaurantiacus, Streptomyces gobitricini, Streptomyces goshikiensis, Streptomyces gougerotii, Streptomyces graminearus, Streptomyces graminofaciens, Streptomyces griseinus, Streptomyces griseoaurantiacus, Streptomyces griseobrunneus, Streptomyces griseocameus, Streptomyces griseochromogenes, Streptomyces griseoflavus, Streptomyces griseofuscus, Streptomyces griseoincarnatus, Streptomyces griseoloalbus, Streptomyces griseolosporeus, Streptomyces griseolus, Streptomyces griseoluteus, Streptomyces griseomycini, Streptomyces griseoplanus, Streptomyces griseorubens, Streptomyces griseoruber, Streptomyces griseorubiginosus, Streptomyces griseosporeus, Streptomyces griseostramineus, Streptomyces griseoverticillatus, Streptomyces griseoviridis, Streptomyces griseus subsp. alpha, Streptomyces griseus subsp. cretosus, Streptomyces griseus subsp. griseus, Streptomyces griseus subsp. solvifaciens, Streptomyces hachijoensis, Streptomyces halstedii, Streptomyces hawaiiensis, Streptomyces heliomycini, Streptomyces helvaticus, Streptomyces herbaricolor, Streptomyces hiroshimensis, Streptomyces hirsutus, Streptomyces humidus, Streptomyces humiferus, Streptomyces hydrogenans, Streptomyces hygroscopicus subsp. angustmyceticus, Streptomyces hygroscopicus subsp. decoyicus, Streptomyces hygroscopicus subsp. glebosus, Streptomyces hygroscopicus subsp. hygroscopicus, Streptomyces hygroscopicus subsp. ossamyceticus, Streptomyces iakyrus, Streptomyces indiaensis, Streptomyces indigoferus, Streptomyces indonesiensis, Streptomyces intermedius, Streptomyces inusitatus, Streptomyces ipomoeae, Streptomyces janthinus, Streptomyces javensis, Streptomyces kanamyceticus, Streptomyces kashmirensis, Streptomyces kasugaensis, Streptomyces katrae, Streptomyces kentuckensis, Streptomyces kifunensis, Streptomyces kishiwadensis, Streptomyces kunmingensis, Streptomyces kurssanovii, Streptomyces labedae, Streptomyces laceyi, Streptomyces ladakanum, Streptomyces lanatus, Streptomyces lateritius, Streptomyces laurentii, Streptomyces lavendofoliae, Streptomyces lavendulae subsp. grasserius, Streptomyces lavendulae subsp. lavendulae, Streptomyces lavenduligriseus, Streptomyces lavendulocolor, Streptomyces levis, Streptomyces libani* subsp. *libani, Streptomyces libani* subsp. *rufus, Streptomyces lienomycini, Streptomyces lilacinus, Streptomyces limosus, Streptomyces lincolnensis, Streptomyces lipmanii, Streptomyces litmocidini, Streptomyces lomondensis, Streptomyces longisporoflavus, Streptomyces longispororuber, Streptomyces longisporus, Streptomyces longwoodensis, Streptomyces lucensis, Streptomyces luridiscabiei, Streptomyces luridus, Streptomyces lusitanus, Streptomyces luteireticuli, Streptomyces luteogriseus, Streptomyces luteosporeus, Streptomyces luteoverticillatus, Streptomyces lydicus, Streptomyces macrosporus, Streptomyces malachitofuscus, Streptomyces malachitospinus, Streptomyces malaysiensis, Streptomyces mashuensis, Streptomyces massasporeus, Streptomyces matensis, Streptomyces mauvecolor, Streptomyces mediocidicus, Streptomyces mediolani, Streptomyces megasporus, Streptomyces melanogenes, Streptomyces melanosporofaciens, Streptomyces mexicanus, Streptomyces michiganensis, Streptomyces microflavus, Streptomyces minutiscleroticus, Streptomyces mirabilis, Streptomyces misakiensis, Streptomyces misionensis, Streptomyces mobaraensis, Streptomyces monomycini, Streptomyces morookaensis, Streptomyces murinus, Streptomyces mutabilis, Streptomyces mutomycini, Streptomyces naganishii, Streptomyces narbonensis, Streptomyces nashvillensis, Streptomyces netropsis, Streptomyces neyagawaensis, Streptomyces niger, Streptomyces nigrescens, Streptomyces nigrifaciens, Streptomyces nitrosporeus, Streptomyces niveiciscabiei, Streptomyces niveoruber, Streptomyces niveus, Streptomyces noboritoensis, Streptomyces nodosus, Streptomyces nogalater, Streptomyces nojiriensis, Streptomyces noursei, Streptomyces novaecaesareae, Streptomyces ochraceiscleroticus, Streptomyces odorifer, Streptomyces olivaceiscleroticus, Streptomyces olivaceoviridis, Streptomyces olivaceus, Streptomyces olivochromogenes, Streptomyces olivomycini, Streptomyces olivoreticuli, Streptomyces olivoreticuli subsp. cellulophilus, Streptomyces olivoreticuli subsp. olivoreticuli, Streptomyces olivoverticillatus, Streptomyces olivoviridis, Streptomyces omiyaensis, Streptomyces orinoci, Streptomyces pactum, Streptomyces paracochleatus, Streptomyces paradoxus, Streptomyces parvisporogenes, Streptomyces parvulus, Streptomyces parvus, Streptomyces peucetius, Streptomyces phaeochromogenes, Streptomyces phaeofaciens, Streptomyces phaeopurpureus, Streptomyces phaeoviridis, Streptomyces phosalacineus, Streptomyces pilosus, Streptomyces platensis, Streptomyces plicatus, Streptomyces pluricolorescens, Streptomyces polychromogenes, Streptomyces poonensis, Streptomyces praecox, Streptomyces prasinopilosus, Streptomyces prasinosporus, Streptomyces prasinus, Streptomyces prunicolor, Streptomyces psammoticus, Streptomyces pseudoechinosporeus, Streptomyces pseudogriseolus, Streptomyces pseudovenezuelae, Streptomyces pulveraceus, Streptomyces puniceus, Streptomyces puniciscabiei, Streptomyces purpeofuscus, Streptomyces purpurascens, Streptomyces purpureus, Streptomyces purpurogeneiscleroticus, Streptomyces racemochromogenes, Streptomyces rameus, Streptomyces ramulosus, Streptomyces rangoonensis, Streptomyces recifensis, Streptomyces rectiverticillatus, Streptomyces rectiviolaceus, Streptomyces regensis, Streptomyces resistomycificus, Streptomyces reticuliscabiei, Streptomyces rhizosphaericus, Streptomyces rimosus subsp. paromomycinus, Streptomyces rimosus subsp. rimosus, Streptomyces rishiriensis, Streptomyces rochei, Streptomyces roseiscleroticus, Streptomyces roseodiastaticus, Streptomyces roseoflavus, Streptomyces roseofulvus, Streptomyces roseolilacinus, Streptomyces roseolus, Streptomyces roseosporus, Streptomyces roseoverticillatus, Streptomyces roseoviolaceus, Streptomyces roseoviridis, Streptomyces rubber, Streptomyces rubiginosohelvolus, Streptomyces rubiginosus, Streptomyces rubrogriseus, Streptomyces rutgersensis subsp. castelarensis, Streptomyces rutgersensis subsp. rutgersensis, Streptomyces salmonis, Streptomyces sampsonii, Streptomyces sanglieri, Streptomyces sannanensis, Streptomyces sapporonensis, Streptomyces scabiei, Streptomyces sclerotialus, Streptomyces scopiformis, Streptomyces seoulensis, Streptomyces septatus, Streptomyces setae, Streptomyces setonii, Streptomyces showdoensis, Streptomyces sindenensis, Streptomyces sioyaensis, Streptomyces somaliensis, Streptomyces sparsogenes, Streptomyces spectabilis, Streptomyces speibonae, Streptomyces speleomycini, Streptomyces spheroids, Streptomyces spinoverrucosus, Streptomyces spiralis, Streptomyces spiroverticillatus, Streptomyces spitsbergensis, Streptomyces sporocinereus, Streptomyces sporoclivatus, Streptomyces spororaveus, Streptomyces sporoverrucosus, Streptomyces stelliscabiei, Streptomyces stramineus, Streptomyces subrutilus, Streptomyces sulfonofaciens, Streptomyces sulphurous, Streptomyces syringium, Streptomyces tanashiensis, Streptomyces tauricus, Streptomyces tendae, Streptomyces termitum, Streptomyces thermoalcalitolerans, Streptomyces thermoautotrophicus, Streptomyces thermocarboxydovorans, Streptomyces thermocarboxydus, Streptomyces thermocoprophilus, Streptomyces thermodiastaticus, Streptomyces thermogriseus, Streptomyces thermolineatus, Streptomyces thermonitrificans, Streptomyces thermospinosisporus, Streptomyces thermoviolaceus subsp. apingens, Streptomyces thermoviolaceus subsp. thermoviolaceus, Streptomyces thermovulgaris, Streptomyces thioluteus, Streptomyces torulosus, Streptomyces toxytricini, Streptomyces tricolor, Streptomyces tubercidicus, Streptomyces tuirus, Streptomyces turgidiscabies, Streptomyces umbrinus, Streptomyces variabilis, Streptomyces variegates, Streptomyces varsoviensis, Streptomyces vastus, Streptomyces venezuelae, Streptomyces vinaceus, Streptomyces vinaceusdrappus, Streptomyces violaceochromogenes, Streptomyces violaceolatus, Streptomyces violaceorectus, Streptomyces violaceoruber, Streptomyces violaceorubidus, Streptomyces violaceus, Streptomyces violaceusniger, Streptomyces violarus, Streptomyces violascens, Streptomyces violatus, Streptomyces violens, Streptomyces virens, Streptomyces virginiae, Streptomyces viridiflavus, Streptomyces viridiviolaceus, Streptomyces viridobrunneus, Streptomyces viridochromogenes, Streptomyces viridodiastaticus, Streptomyces viridosporus, Streptomyces vitaminophileus, Streptomyces vitaminophilus, Streptomyces wedmorensis, Streptomyces werraensis, Streptomyces willmorei, Streptomyces xanthochromogenes, Streptomyces xanthocidicus, Streptomyces xantholiticus, Streptomyces xanthophaeus, Streptomyces yatensis, Streptomyces yerevanensis, Streptomyces yogyakartensis, Streptomyces yokosukanensis, Streptomyces yunnanensis, Streptomyces zaomyceticus, Streptoverticillium abikoense, Streptoverticillium albireticuli, Streptoverticillium alboverticillatum, Streptoverticillium album, Streptoverticillium ardum, Streptoverticillium aureoversale, Streptoverticillium aureoversile, Streptoverticillium baldaccii , Streptoverticillium biverticillatum, Streptoverticillium blastmyceticum, Streptoverticillium cinnamoneum* subsp. *albosporum, Streptomyces cinnamoneus* subsp. *albosporus, Streptoverticillium cinnamoneum* subsp. *cinnamoneum, Streptoverticillium cinnamoneum* subsp. *lanosum, Streptoverticillium cinnamoneum* subsp. *sparsum, Streptoverticillium distallicum, Streptoverticillium ehimense, Streptoverticillium eurocidicum, Streptoverticillium fervens* subsp. *fervens, Streptoverticillium fervens* subsp. *melrosporus, Streptoverticillium flavopersicum, Streptoverticillium griseocarneum, Streptoverticillium griseoverticillatum, Streptoverticillium hachijoense, Streptoverticillium hiroshimense, Streptoverticillium kashmirense, Streptoverticillium kentuckense, Streptoverticillium kishiwadense, Streptoverticillium ladakanum, Streptoverticillium lavenduligriseum, Streptoverticillium lilacinum. Streptoverticillium luteoverticillatum, Streptoverticillium mashuense, Streptoverticillium mobaraense, Streptoverticillium morookaense, Streptoverticillium netropsis, Streptoverticillium olivomycini, Streptomyces olivomycini, Streptoverticillium olivoreticuli* subsp. *cellulophilum, Streptoverticillium olivoreticuli* subsp. *olivoreticuli, Streptoverticillium olivoreticulum, Streptoverticillium olivoreticulum* subsp. *cellulophilum, Streptoverticillium olivoverticillatum, Streptoverticillium orinoci, Streptoverticillium parvisporogenes, Streptoverticillium parvisporogenum, Streptoverticillium rectiverticillatum, Streptoverticillium reticulum* subsp. *protomycicum, Streptoverticillium roseoverticillatum, Streptoverticillium salmonis, Streptoverticillium sapporonense, Streptoverticillium septatum, Streptoverticillium syringium, Streptoverticillium thioluteum, Streptoverticillium verticillium* subsp. *quantum, Streptoverticillium verticillium* subsp. *tsukushiense or Streptoverticillium viridoflavum.*

Particular preferred strains are strains selected from the group consisting of Bacillaceae, Brevibacteriaceae, Corynebacteriaceae, Nocardiaceae, Mycobacteriaceae, Streptomycetaceae, Enterobacteriaceae such as *Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp., Nocardia rhodochrous (Rhodococcus rhodochrous), Mycobacterium rhodochrous, Streptomyces lividans* and *Escherichia coli* especially *Escherichia coli K12.*

In addition particular preferred strains are strains selected from the group consisting of Cryptococcaceae, Saccharomycetaceae, Schizosaccharomycetacease such as the genera Candida, Hansenula, Pichia, Saccharomyces and Schizosaccharomyces preferred are strains selected from the group consisting of the species *Rhodotorula rubra, Rhodotorula glutinis, Rhodotorula graminis, Yarrowia lipolytica, Sporobolomyces salmonicolor, Sporobolomyces shibatanus, Saccharomyces cerevisiae, Candida boidinii, Candida bombicola, Candida cylindracea, Candida parapsilosis, Candida rugosa, Candida tropicalis, Pichia methanolica and Pichia pastoris* especially *Saccharomyces cerevisiae.*

Anacardiaceae such as the genera Pistacia, Mangifera, Anacardium e.g. the species *Pistacia vera* [pistachios, Pistazie], *Mangifer indica* [Mango] or *Anacardium occidentale* [Cashew]; Asteraceae such as the genera Calendula, Carthamus, Centaurea, Cichorium, Cynara, Helianthus, Lactuca, Locusta, Tagetes, Valeriana e.g. the species *Calendula officinalis* [Marigold], *Carthamus tinctorius* [safflower], *Centaurea cyanus* [cornflower], *Cichorium intybus* [blue daisy], *Cynara scolymus* [Artichoke], *Helianthus annus* [sunflower], Lactuca sativa, *Lactuca crispa, Lactuca esculenta, Lactuca scariola L. ssp. sativa, Lactuca scariola L var. integrata, Lactuca scariola L. var. integrifolia, Lactuca sativa subsp. romana, Locusta communis, Valeriana locusta* [lettuce], *Tagetes lucida, Tagetes erecta or Tagetes tenuifolia* [Marigold]; Apiaceae such as the genera Daucus e.g. the species *Daucus carota* [carrot]; Betulaceae such as the genera Corylus e.g. the species *Corylus avellana* or *Corylus colurna* [hazelnut]; Boraginaceae such as the genera Borago e.g. the species *Borago officinalis* [borage]; Brassicaceae such as the genera Brassica, Melanosinapis, Sinapis, Arabadopsis e.g. the species *Brassica napus, Brassica rapa* ssp. [canola, oilseed rape, tumip rape], *Sinapis arvensis Brassica juncea, Brassica juncea var. juncea, Brassica juncea var. crispifolia, Brassica juncea var. foliosa, Brassica nigra, Brassica sinapioides, Melanosinapis communis* [mustard], *Brassica oleracea* [fodder beet] or Arabidopsis thaliana; Bromeliaceae such as the genera Anana, Bromelia e.g. the species *Anana comosus, Ananas ananas* or *Bromelia comosa* [pineapple]; Caricaceae such as the genera Carica e.g. the species *Carica papaya* [papaya]; Cannabaceae such as the genera Cannabis e.g. the species *Cannabis sative* [hemp], Convolvulaceae such as the genera Ipomea, Convolvulus e.g. the species *Ipomoea batatus, Ipomoea pandurata, Convolvulus batatas, Convolvulus tiliaceus, Ipomoea fastigiata, Ipomoea tiliacea, Ipomoea triloba* or *Convolvulus panduratus* [sweet potato, Man of the Earth, wild potato], Chenopodiaceae such as the genera Beta, i.e. the species *Beta vulgaris, Beta vulgaris var. altissima, Beta vulgaris var. Vulgaris, Beta maritima, Beta vulgaris var. perennis, Beta vulgaris var. conditiva* or *Beta vulgaris var. esculenta* [sugar beet]; Cucurbitaceae such as the genera Cucubita e.g. the species *Cucurbita maxima, Cucurbita mixta, Cucurbita pepo* or *Cucurbita moschata* [pumpkin, squash]; Elaeagnaceae such as the genera Elaeagnus e.g. the species *Olea europaea* [olive]; Ericaceae such as the genera Kalmia e.g. the species *Kalmia latifolia, Kalmia angustifolia, Kalmia microphylla, Kalmia polifolia, Kalmia occidentalis, Cistus chamaerhodendros* or *Kalmia lucida* [American laurel, broad-leafed laurel, calico bush, spoon wood, sheep laurel, alpine laurel, bog laurel, western bog-laurel, swamp-laurel]; Euphorbiaceae such as the genera Manihot, Janipha, Jatropha, Ricinus e.g. the species *Manihot utilissima, Janipha manihot,, Jatropha manihot., Manihot aipil, Manihot dulcis, Manihot manihot, Manihot melanobasis, Manihot esculenta* [manihot, arrowroot, tapioca, cassava] or *Ricinus communis* [castor bean, Castor Oil Bush, Castor Oil Plant, Palma Christi, Wonder Tree]; Fabaceae such as the genera Pisum, Albizia, Cathormion, Feuillea, Inga, Pithecolobium, Acacia, Mimosa, Medicajo, Glycine, Dolichos, Phaseolus, Soja e.g. the species *Pisum sativum, Pisum arvense, Pisum humile* [pea], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [bastard logwood, silk tree, East Indian Walnut], *Medicago sativa, Medicago falcata, Medicago varia* [alfalfa] *Glycine max Dolichos soja, Glycine gracilis, Glycine hispida, Phaseolus max, Soja hispida* or *Soja max* [soybean]; Geraniaceae such as the genera Pelargonium, Cocos, Oleum e.g. the species *Cocos nucifera, Pelargonium grossularioides* or *Oleum cocois* [coconut]; Gramineae such as the genera Saccharum e.g. the species *Saccharum officinarum;* Juglandaceae such as the genera Juglans, Wallia e.g. the species *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* or *Wallia nigra* [walnut, black walnut, common walnut, persian walnut, white walnut, butternut. black walnut]; Lauraceae such as the genera Persea, Laurus e.g. the species laurel *Laurus nobilis* [bay, laurel, bay laurel, sweet bay], *Persea americana Persea americana, Persea gratissima or Persea persea* [avocado]; Leguminosae such as the genera Arachis e.g. the species *Arachis hypogaea* [peanut]; Linaceae such as the genera Linum, Adenolinum e.g. the species *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* or *Linum trigynum* [flax, linseed]; Lythrarieae such as the genera Punica e.g. the species *Punica granatum* [pomegranate]; Malvaceae such as the genera Gossypium e.g. the species *Gossypium hirsutum, Gossypium arboreum, Gossypium barbadense, Gossypium herbaceum* or *Gossypium thurberi* [cotton]; Musaceae such as the genera Musa e.g. the species *Musa nana, Musa acuminata, Musa paradisiaca, Musa* spp. [banana]; Onagraceae such as the genera Camissonia, Oenothera e.g. the species *Oenothera biennis* or *Camissonia brevipes* [primrose, evening primrose]; Palmae such as the genera Elacis e.g. the species *Elaeis guineensis* [oil plam]; Papaveraceae such as the genera Papaver e.g. the species *Papaver orientale, Papaver rhoeas, Papaver dubium.* [poppy, oriental poppy, corn poppy, field poppy, shirley poppies, field poppy, long-headed poppy, long-pod poppy]; Pedaliaceae such as the genera Sesamum e.g. the species *Sesamum indicum* [sesame]; Piperaceae such as the genera Piper, Artanthe, Peperomia, Steffensia e.g. the species *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata.* [Cayenne pepper, wild pepper]; Poaceae such as the genera Hordeum, Secale, Avena, Sorghum, Andropogon, Holcus, Panicum, Oryza, Zea, Triticum e.g. the species *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [barley, pearl barley, foxtail barley, wall barley, meadow barley], *Secale cereale* [rye], Avena sativa, *Avena fatua, Avena byzantina, Avena fatua var. sativa, Avena hybrida* [oat], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cemuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum* millet, *Panicum militaceum [Sorghum, millet], Oryza sativa, Oryza latifolia* [rice], Zea *mays* [corn, maize] *Triticum aestivum, Triticum durum, Triticum turgidum, Triticum hybernum, Triticum macha, Triticum sativum* or *Triticum vulgare* [wheat, bread wheat, common wheat], Proteaceae such as the genera Macadamia e.g. the species *Macadamia intergrifolia* [macadamia]; Rubiaceae such as the genera Coffea e.g. the species Cofea spp., *Coffea arabica, Coffea canephora* or *Coffea liberica* [coffee]; Scrophulariaceae such as the genera Verbascum e.g. the species *Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* or *Verbascum thapsus* [mullein, white moth mullein, nettle-leaved mullein, dense-flowered mullein, silver mullein, long-leaved mullein, white mullein, dark mullein, greek mullein, orange mullein, purple mullein, hoary mullein, great mullein]; Solanaceae such as the genera Capsicum, Nicotiana, Solanum, Lycopersicon e.g. the species *Capsicum annuum, Capsicum annuum var. glabriusculum, Capsicum frutescens* [pepper], *Capsicum annuum* [paprika], *Nicotiana tabacum, Nicotiana alata, Nicotiana attenuata, Nicotiana glauca, Nicotiana langsdorffii, Nicotiana obtusifolia, Nicotiana quadrivalvis. Nicotiana repanda, Nicotiana rustica, Nicotiana sylvestris* [tobacco], *Solanum tuberosum* [potato], *Solanum melongena* [egg-plant] *(Lycopersicon esculentum, Lycopersicon lycopersicum., Lycopersicon pyriforme, Solanum integrifolium* or *Solanum lycopersicum [tomato];* Sterculiaceae such as the genera Theobroma e.g. the species *Theobroma cacao* [cacao]; Theaceae such as the genera Camellia e.g. the species *Camellia sinensis)* [tea]. All abovementioned organisms can in princible also function as host organisms.

[0095.0.0.0] Particular preferred plants are plants selected from the group consisting of Asteraceae such as the genera Helianthus, Tagetes e.g. the species *Helianthus annus* [sunflower], *Tagetes lucida, Tagetes erecta or Tagetes tenuifolia* [Marigold]; Brassicaceae such as the genera Brassica, Arabadopsis e.g. the species *Brassica napus, Brassica rapa* ssp., Brassica juncea [canola, oilseed rape, turnip rape] or Arabidopsis thaliana; Fabaceae such as the genera Glycine e.g. the species *Glycine max, Soja hispida* or *Soja max* [soybean]; Linaceae such as the genera Linum e.g. the species *Linum usitatissimum,* [flax, linseed]; Poaceae such as the genera Hordeum, Secale, Avena, Sorghum, Oryza, Zea, Triticum e.g. the species *Hordeum vulgare* [barley]; *Secale cereale* [rye], Avena sativa, *Avena fatua, Avena byzantina, Avena fatua var. sativa, Avena hybrida* [oat], *Sorghum bicolor* [Sorghum, millet], *Oryza sativa, Oryza latifolia* [rice], *Zea mays* [com, maize] *Triticum aestivum, Triticum durum, Triticum turgidum, Triticum hybemum, Triticum macha, Triticum sativum* or *Triticum vulgare* [wheat, bread wheat, common wheat]; Solanaceae such as the genera Solanum, Lycopersicon e.g. the species *Solanum tuberosum* [potato], *Lycopersicon esculentum, Lycopersicon lycopersicum., Lycopersicon pyriforme, Solanum integrifolium* or *Solanum lycopersicum* [*tomato*]*.*

All abovementioned organisms can in princible also function as host organisms.

With regard to the nucleic acid sequence as depicted below a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) the nucleic acid sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393 or a derivative thereof, or
b) a genetic regulatory element, for example a promoter, which is functionally linked to the nucleic acid sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

The use of the nucleic acid sequence according to the invention or of the nucleic acid construct according to the invention for the generation of transgenic plants is therefore also subject matter of the invention.

The fine chemical, which is synthesized in the organism, in particular the microorganism, the cell, the tissue or the plant, of the invention can be isolated if desired. Depending on the use of the fine chemical, different purities resulting from the purification may be advantageous as will be described herein below.

In an advantageous embodiment of the invention, the organism takes the form of a plant whose fine chemical content is modified advantageously owing to the nucleic acid molecule of the present invention expressed. This is important for plant breeders since, for example, the nutritional value of organisms such as a plant is very often limited by its amino acid, protein, co-factor and/or vitamin content to mention only a couple of them. For example in feed for monogastric animals a few essential amino acids such as lysine, threonine or methionine are very often limiting. After the biological activity of the nucleic acid and/or protein of the invention has been increased or generated, or after the expression of nucleic acid molecule or polypeptide according to the invention has been generated or increased, the transgenic plant generated thus is grown on or in a nutrient medium or else in the soil and subsequently harvested.

The plants or parts thereof, e.g. the leaves, roots, flowers, and/or stems and/or other harvestable material as described below, can then be used directly as foodstuffs or animal feeds or else be further processed. Again, the amino acids can be purified further in the customary manner via extraction and precipitation or via ion exchangers and other methods known to the person skilled in the art and described herein below. Products which are suitable for various applications and which result from these different processing procedures are for example amino acids or amino acid compositions which can still comprise further plant components in different amounts, advantageously in the range of from 0 to 99% by weight or more, preferably from below 90%, 80%, 70%, 60% or 50% by weight, especially preferably below 40%, 30%, 20% or 10% by weight. The plants can also advantageously be used directly without further processing, e.g. as feed or for extraction.

The chemically pure fine chemical or chemically pure compositions comprising the fine chemical may also be produced by the process described above. To this end, the fine chemical or the compositions are isolated in the known manner from an organism according to the invention, such as the microorganisms, non-human animal or the plants, and/or their culture medium in which or on which the organisms had been grown. These chemically pure fine chemical or said compositions are advantageous for applications in the field of the food industry, the cosmetics industry or the pharmaceutical industry.

Thus, the content of plant components and preferably also further impurities is as low as possible, and the abovementioned fine chemical are obtained in as pure form as possible. In these applications, the content of plant components advantageously amounts to less than 10% by weight, preferably 1% by weight, more preferably 0.1% by weight, very especially preferably 0.01 % by weight or less.

Accordingly, the fine chemical produced by the present invention is at least 0, 1 % by weight pure, preferably more than 1% by weight pure, more preferred 10% by weight pure, even more preferred are more than 50%, 60%, 70% or 80% by weight pure, even more preferred are more than 90%, 91%, 92%, 93%, 94% or 95% weight pure, most preferred are 96%, 97%, 98% or 99% by weight or more pure.

In this context, the amount of the fine chemical in a cell of the invention may be increased according to the process of the invention by at least a factor of 1.1, preferably at least a factor of 1.5; 2; or 5, especially preferably by at least a factor of 10 or 30, very especially preferably by at least a factor of 50, in comparison with the wild type, control or reference. Preferrably, said increase is found in a tissue of an organism, more preferred in the organism itself or in a harvestable part thereof.

In principle, the fine chemicals produced can be increased in two ways by the process according to the invention. The pool of free fine chemicals, in particular of the free fine chemical, and/or the content of bound for example protein-bound fine chemicals, in particular of the protein-bound fine chemical may advantageously be increased.

It may be advantageous to increase the pool of free fine chemical in the transgenic organisms by the process according to the invention in order to isolate high amounts of the pure fine chemical.

In another preferred embodiment of the invention a combination of the increased expression of the nucleic acid sequence or the protein of the invention together with the transformation of a protein or polypeptid, which functions as a sink for the desired fine chemical such as an amino acid for example methionine, lysine or threonine in the organism is useful to increase the production of the fine chemical (see US 5,589,616, WO 96/38574, WO 97/07665, WO 97/28247, US 4,886,878, US 5,082,993 and US 5,670,635). Galili et al. [Transgenic Res. 2000] showed, that enhancing the synthesis of threonine by a feed back insensitive aspertate kinase didnot lead only to in increase in free threonine but also in protein bound threonine.

In may also be advantageous to increase the content of the bound fine chemical.

In a preferred embodiment, the fine chemical is produced in accordance with the invention and, if desired, is isolated. The production of further fine chemicals for example amino acids such as lysine and of amino acid mixtures by the process according to the invention is advantageous.

In the case of the fermentation of microorganisms, the abovementioned fine chemical e.g. amino acid or mixtures of amino acids may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. This concentrated fermentation broth can subsequently be processed by lyophilization, spray drying, spray granulation or by other methods.

To purify a fine chemical such as an amino acid, a product-containing fermentation broth from which the biomass has been separated may be subjected to chromatography with a suitable resin such as ion exchange resin for example anion or cation exchange resin, hydrophobic resin or hydrophilic resin for example epoxy resin, polyurethane resin or polyacrylamid resin, or resin for separation according to the molecular weight of the compounds for example polyvinyl chloride homopolymer resin or resins composed for example of polymers of acrylic acid, crosslinked with polyalkenyl ethers or divinyl glycol such as Carbopol®, Pemulen® and Noveon®. If necessary these chromatography steps may be repeated using the same or other chromatography resins. The skilled worker is familiar with the choice of suitable chromatography resins and their most effective use. The purified product may be concentrated by filtration or ultrafiltration and stored at a temperature, which ensures the maximum stability of the product.

The identity and purity of the compound(s) isolated can be determined by prior-art techniques. They encompass high-performance liquid chromatography (HPLC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

Fine chemicals like amino acids can for excample be detected advantageously via HPLC separation in ethanolic extract as described by Geigenberger et al. (Plant Cell & Environ, 19, 1996: 43-55). Amino acids can be extracted with hot water. After filtration the extracts are diluted with water containing 20 mg/mL sodium acide. The separation and detection of the amino acids is performed using an anion exchange column and an electrochemical detector. Technical details can be taken from Y. Ding et al., 2002, Direct determination of free amino acids and sugars in green tea by anion-exchange chromatography with integrated pulsed amperometric detection, J Chromatogr A, (2002) 982; 237-244, or e.g. from Karchi et al., 1993 , Plant J. 3: 721-727; Matthews MJ, 1997 (Lysine, threonine and methionine biosynthesis. In BK Singh, ed, Plant Amino Acids: Biochemistry and Biotechnology. Dekker, New York, pp 205-225; H Hesse and R Hoefgen. (2003) Molecular aspects of methionine biosynthesis. TIPS 8(259-262.

In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or a fragment thereof, which confers an increase in the amount of the fine chemical in an organism or a part thereof;
b) nucleic acid molecule comprising, preferably at least the mature form, of the nucleic acid molecule as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287. 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof;
h) nucleic acid molecule comprising a nucleic acid molecule which is obtained by amplifying nucleic acid molecules from a cDNA library or a genomic library using the primers as depicted in SEQ ID NO: 53 or SEQ ID NO: 54 and conferring an increase in the amount of the fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the fine chemical in an organism or a part thereof;
j) nucleic acid molecule which encodes a polypeptide comprising the consensus sequence as depicted in SEQ ID NO: 47, SEQ ID NO: 48, SEQ ID NO: 49, SEQ ID NO: 50, SEQ ID NO: 51, SEQ ID NO: 52, SEQ ID NO: 397, SEQ ID NO: 398, SEQ ID NO: 399 and/or SEQ ID NO: 400 and conferring an increase in the amount of the fine chemical in an organism or a part thereof ; and/or
k) nucleic acid molecule which is obtainable by screening a suitable library under stringent conditions with a probe comprising one of the sequences of the nucleic acid molecule of (a) to (j), preferably to (a) to (c), or with a fragment of at least 15 nt, preferably 20 nt, 30 nt, 50 nt, 100 nt, 200 nt or 500 nt of the nucleic acid molecule characterized in (a) to (j), preferably to (a) to (c), and conferring an increase in the amount of the fine chemical in an organism or a part thereof; or which comprises a sequence which is complementary thereto.

In one embodiment, the nucleic acid molecule used in the process distinguishes over the sequence as depicted in SEQ ID NO: 1, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 by one or more nucleotides or does not consist of the sequence as depicted in SEQ ID NO: 1, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393. In one embodiment, the nucleic acid molecule of the present invention is less than 100%, 99,999%, 99,99%, 99,9% or 99% identical to the sequence as depicted in SEQ ID NO: 1, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393. In another embodiment, the nucleic acid molecule does not encode a polypeptide of the sequence as depicted in SEQ ID NO: 2, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394. In another embodiment, the nucleic acid molecule does not encode a polypeptide of the sequence of the present invention is less than 100%, 99,999%, 99,99%, 99,9% or 99% identical to the sequence as depicted in SEQ ID NO: 2, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394. In another embodiment, the nucleic acid molecule encodes a polypeptide of the sequence as depicted in SEQ ID NO: 2, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 and distinguishes over said sequence by one or more amino acids preferably by 1, 2, 3, 4, 5, 6, 7, 8, 9 or 10 amino acids. In further another advantageously embodiment, the nucleic acid molecule used in the process distinguishes over the sequence as depicted in SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 SEQ ID NO: 43 or SEQ ID NO: 45 by one or more nucleotides or does not consist of the sequence as depicted in SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 SEQ ID NO: 43 or SEQ ID NO: 45. In one advantageously embodiment, the nucleic acid molecule of the present invention is less than 100%, 99,999%, 99,99%, 99,9% or 99% identical to the sequence as depicted in SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 SEQ ID NO: 43 or SEQ ID NO: 45. In another advantageously embodiment, the nucleic acid molecule does not encode a polypeptide of the sequence as depicted in SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44 or SEQ ID NO: 46.

Unless otherwise specified, the terms "polynucleotides", "nucleic acid" and "nucleic acid molecule" are interchangeably in the present context. Unless otherwise specified, the terms "peptide", "polypeptide" and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)", "polynucleotide", "nucleic acid sequence", "nucleotide sequence", or "nucleic acid molecule(s)" as used herein refers to a polymeric form of nucleotides of any length, either ribonucleotides or deoxyribonucleotides. The terms refer only to the primary structure of the molecule.

Thus, the terms "gene(s)", "polynucleotide", "nucleic acid sequence", "nucleotide sequence", or "nucleic acid molecule(s)" as used herein include double-and single-stranded DNA and RNA. They also include known types of modifications, for example, methylation, "caps", substitutions of one or more of the naturally occurring nucleotides with an analog. Preferably, the DNA or RNA sequence of the invention comprises a coding sequence encoding the herein defined polypeptide.

A "coding sequence" is a nucleotide sequence, which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleotide sequences or genomic DNA, while introns may be present as well under certain circumstances.

Nucleic acid molecules with the sequence as depicted in SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41 SEQ ID NO: 43 or SEQ ID NO: 45, nucleic acid molecules which are derived from the amino acid sequences as depicted in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44 or SEQ ID NO: 46 or from polypeptides comprising the consensus sequence as depicted in SEQ ID NO: 47, SEQ ID NO: 48, SEQ ID NO: 49, SEQ ID NO: 50, SEQ ID NO: 51, SEQ ID NO: 52, SEQ ID NO: 397, SEQ ID NO: 398, SEQ ID NO: 399 and/or SEQ ID NO: 400, or their derivatives or homologues encoding polypeptides with the enzymatic or biological activity of a protein as depicted in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44 or SEQ ID NO: 46 and/or conferring a fine chemical increase after increasing its expression or activity are advantageously increased in the process according to the invention.

In one embodiment, said sequences are cloned into nucleic acid constructs, either individually or in combination. These nucleic acid constructs enable an optimal synthesis of the fine chemical produced in the process according to the invention.

Nucleic acid molecules, which are advantageous for the process according to the invention and which encode polypeptides with the biological activity of the protein of the invention can be determined from generally accessible databases.

Those, which must be mentioned, in particular in this context are general gene databases such as the EMBL database (Stoesser G. et al., Nucleic Acids Res 2001, Vol. 29, 17-21), the GenBank database (Benson D.A. et al., Nucleic Acids Res 2000, Vol. 28,15-18), or the PIR database (Barker W. C. et al., Nucleic Acids Res. 1999, Vol. 27, 39-43). It is furthermore possible to use organism-specific gene databases for determining advantageous sequences, in the case of yeast for example advantageously the SGD database (Cherry J. M. et al., Nucleic Acids Res. 1998, Vol. 26, 73-80) or the MIPS database (Mewes H.W. et al., Nucleic Acids Res. 1999, Vol. 27, 44-48), in the case of E. coli the GenProtEC database (http://web.bham.ac.uk/bcm4ght6/res.html), and in the case of Arabidopsis the TAIR-database (Huala, E. et al., Nucleic Acids Res. 2001 Vol. 29(1), 102-5) or the MIPS database.

[0124.0.0.0] The nucleic acid molecules used in the process according to the invention take the form of isolated nucleic acid sequences, which encode polypeptides having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394and conferring the fine chemical increase.

The nucleic acid sequence(s) used in the process for the production of the fine chemical in transgenic organisms originate advantageously from an eukaryote but may also originate from a prokaryote or an archebacterium, thus it can derived from e.g. a microorganism, an animal or a plant.

For the purposes of the invention, as a rule the plural is intended to encompass the singular and vice versa.

In order to improve the introduction of the nucleic acid sequences and the expression of the sequences in the transgenic organisms, which are used in the process, the nucleic acid sequences are incorporated into a nucleic acid construct and/or a vector. In addition to the herein described sequences which are used in the process according to the invention, further nucleic acid sequences, advantageously of biosynthesis genes of amino acids, carbohydrates, lipids, fatty acids, vitamins etc. produced in the process according to the invention, may additionally be present in the nucleic acid construct or in the vector and may be introduced into the organism together. However, these additional sequences may also be introduced into the organisms via other, separate nucleic acid constructs or vectors.

Using the herein mentioned cloning vectors and transformation methods such as those which are published and cited in: Plant Molecular Biology and Biotechnology (CRC Press, Boca Raton, Florida), chapter 6/7, pp. 71-119 (1993); F.F. White, Vectors for Gene Transfer in Higher Plants; in: Transgenic Plants, vol. 1, Engineering and Utilization, Ed.: Kung and R. Wu, Academic Press, 1993, 15-38; B. Jenes et al., Techniques for Gene Transfer, in: Transgenic Plants, vol. 1, Engineering and Utilization, Ed.: Kung and R. Wu, Academic Press (1993), 128-143; Potrykus, Annu. Rev. Plant. Physiol. Plant Molec. Biol. 42 (1991), 205-225)) and further cited below, the nucleic acids may be used for the recombinant modification of a wide range of organisms, in particular prokaryotic or eukaryotic microorganisms or plants, so that they become a better and more efficient producer of the fine chemical produced in the process according to the invention. This improved production, or production efficiency, of the fine chemical or products derived there from, such as modified proteins, can be brought about by a direct effect of the manipulation or by an indirect effect of this manipulation.

In one embodiment, the nucleic acid molecule according to the invention originates from a plant, such as a plant selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants and in particular plants mentioned herein above as host plants such as the families and genera mentioned above for example preferred the species *Anacardium occidentale, Calendula officinalis, Carthamus tinctorius, Cichorium intybus, Cynara scolymus, Helianthus annus, Tagetes lucida, Tagetes erecta, Tagetes tenuifolia; Daucus carota; Corylus avellana, Corylus columa, Borago officinalis; Brassica napus, Brassica rapa* ssp., *Sinapis arvensis Brassica juncea, Brassica juncea var. juncea, Brassica juncea var. crispifolia, Brassica juncea var. foliosa, Brassica nigra, Brassica sinapioides, Melanosinapis communis, Brassica oleracea,* Arabidopsis thaliana, *Anana comosus, Ananas ananas, Bromelia comosa, Carica papaya, Cannabis sative, Ipomoea batatus, Ipomoea pandurata, Convolvulus batatas, Convolvulus tiliaceus, Ipomoea fastigiata, Ipomoea tiliacea, Ipomoea triloba, Convolvulus panduratus, Beta vulgaris, Beta vulgaris var. altissima, Beta vulgaris var. vulgaris, Beta maritima, Beta vulgaris var. perennis, Beta vulgaris var. conditiva, Beta vulgaris var. esculenta, Cucurbita maxima, Cucurbita mixta, Cucurbita pepo, Cucurbita moschata, Olea europaea, Manihot utilissima, Janipha manihot,, Jatropha manihot., Manihot aipil, Manihot dulcis, Manihot manihot, Manihot melanobasis, Manihot esculenta, Ricinus communis, Pisum sativum, Pisum arvense, Pisum humile, Medicago sativa, Medicago falcata, Medicago varia, Glycine max Dolichos soja, Glycine gracilis, Glycine hispida, Phaseolus max, Soja hispida, Soja max, Cocos nucifera, Pelargonium grossularioides, Oleum cocoas, Laurus nobilis, Persea americana, Arachis hypogaea, Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense, Linum trigynum, Punica granatum, Gossypium hirsutum, Gossypium arboreum, Gossypium barbadense, Gossypium herbaceum, Gossypium thurberi, Musa nana, Musa acuminata, Musa paradisiaca, Musa* spp., *Elaeis guineensis, Papaver orientale, Papaver rhoeas, Papaver dubium, Sesamum indicum, Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata, , Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum,* Avena sativa, *Avena fatua, Avena byzantina, Avena fatua var. sativa, Avena hybrida, Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum* millet, *Panicum militaceum,* Zea *mays, Triticum aestivum, Triticum durum, Triticum turgidum, Triticum hybernum, Triticum macha, Triticum sativum* or *Triticum vulgare, Cofea spp., Coffea arabica, Coffea canephora, Coffea liberica, Capsicum annuum, Capsicum annuum var. glabriusculum, Capsicum frutescens, Capsicum annuum, Nicotiana tabacum, Solanum tuberosum, Solanum melongena, Lycopersicon esculentum, Lycopersicon lycopersicum., Lycopersicon pyriforme, Solanum integrifolium, Solanum lycopersicum Theobroma cacao* or *Camellia sinensis.*

In one embodiment, the nucleic acid molecule sequence originates advantageously from a microorganism as mentioned above under host organism such as a fungus for example the genera Aspergillus, Penicillium or Claviceps or from yeasts such as the genera Pichia, Torulopsis, Hansenula, Schizosaccharomyces, Candida, Rhodotorula or Saccharomyces, very especially advantageously from the yeast of the family Saccharomycetaceae, such as the advantageous genus Saccharomyces and the very advantageous genus and species Saccharomyces cerevisiae for the production of the fine chemical in microorganims.

The skilled worker knows other suitable sources for the production of fine chemicals, which present also useful nucleic acid molecule sources. They include in general all prokaryotic or eukaryotic cells, preferably unicellular microorganisms, such as fungi like the genus Claviceps or Aspergillus or gram-positive bacteria such as the genera Bacillus, Corynebacterium, Micrococcus, Brevibacterium, Rhodococcus, Nocardia, Caseobacter or Arthrobacter or gram-negative bacteria such as the genera Escherichia, Flavobacterium or Salmonella, or yeasts such as the genera Rhodotorula, Hansenula or Candida.

Production strains which are especially advantageously selected in the process according to the invention are microorganisms selected from the group of the families Actinomycetaceae, Bacillaceae, Brevibacteriaceae, Corynebacteriaceae, Enterobacteriacae, Gordoniaceae, Micrococcaceae, Mycobacteriaceae, Nocardiaceae, Pseudomonaceae, Rhizobiaceae, Streptomycetaceae, Chaetomiaceae, Choanephoraceae, Cryptococcaceae, Cunninghamellaceae, Demetiaceae, Moniliaceae, Mortierellaceae, Mucoraceae, Pythiaceae, Saccharomycetaceae, Saprolegniaceae, Schizosaccharomycetaceae, Sodariaceae, Sporobolomycetaceae, Tuberculariaceae, Adelotheciaceae, Dinophyceae, Ditrichaceae and Prasinophyceaeor of the genera and species consisting of Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Escherichia coli K12 and its described strains.

However, it is also possible to use artificial sequences, which differ in one or more bases (= nucleotides) from the nucleic acid sequences found in organisms, or in one or more amino acid molecules from polypeptide sequences found in organisms, in particular from the polypeptide sequences as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or the functional homologues thereof as described herein, preferably conferring above-mentioned biological activity, i.e. conferring the fine chemical increase after increasing its activity, e.g. having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394.

In the process according to the invention nucleic acid sequences can be used, which, if appropriate, contain synthetic, non-natural or modified nucleotide bases, which can be incorporated into DNA or RNA. Said synthetic, non-natural or modified bases can for example increase the stability of the nucleic acid molecule outside or inside a cell. The nucleic acid molecules of the invention can contain the same modifications as aforementioned.

As used in the present context the term "nucleic acid molecule" may also encompass the untranslated sequence located at the 3' and at the 5' end of the coding gene region, for example at least 500, preferably 200, especially preferably 100, nucleotides of the sequence upstream of the 5' end of the coding region and at least 100, preferably 50, especially preferably 20, nucleotides of the sequence downstream of the 3' end of the coding gene region. It is often advantageous only to choose the coding region for cloning and expression purposes.

Preferably, the nucleic acid molecule used in the process according to the invention or the nucleic acid molecule of the invention is an isolated nucleic acid molecule.

An "isolated" polynucleotide or nucleic acid molecule is separated from other polynucleotides or nucleic acid molecules, which are present in the natural source of the nucleic acid molecule. An isolated nucleic acid molecule may be a chromosomal fragment of several kb, or preferably, a molecule only comprising the coding region of the gene. Accordingly, an isolated nucleic acid molecule of the invention may comprise chromosomal regions, which are adjacent 5' and 3' or further adjacent chromosomal regions, but preferably comprises no such sequences which naturally flank the nucleic acid molecule sequence in the genomic or chromosomal context in the organism from which the nucleic acid molecule originates (for example sequences which are adjacent to the regions encoding the 5'- and 3'-UTRs of the nucleic acid molecule). In various embodiments, the isolated nucleic acid molecule used in the process according to the invention may, for example comprise less than approximately 5 kb, 4 kb, 3 kb, 2 kb, 1 kb, 0.5 kb or 0.1 kb nucleotide sequences which naturally flank the nucleic acid molecule in the genomic DNA of the cell from which the nucleic acid molecule originates.

The nucleic acid molecules used in the process, for example the polynucleotides of the invention or of a part thereof can be isolated using molecular-biological standard techniques and the sequence information provided herein. Also, for example a homologous sequence or homologous, conserved sequence regions at the DNA or amino acid level can be identified with the aid of comparison algorithms. The former can be used as hybridization probes under standard hybridization techniques (for example those described in Sambrook et al., Molecular Cloning: A Laboratory Manual. 2nd Ed., Cold Spring Harbor Laboratory, Cold Spring Harbor Laboratory Press, Cold Spring Harbor, NY, 1989) for isolating further nucleic acid sequences useful in this process.

A nucleic acid molecule encompassing a complete sequence of the nucleic acid molecules used in the process, for example the polynucleotide of the invention, or a part thereof may additionally be isolated by polymerase chain reaction, oligonucleotide primers based on this sequence or on parts thereof being used. For example, a nucleic acid molecule comprising the complete sequence or part thereof can be isolated by polymerase chain reaction using oligonucleotide primers which have been generated on the basis of this sequence for example, mRNA can be isolated from cells (for example by means of the guanidinium thiocyanate extraction method of Chirgwin et al. (1979) Biochemistry 18:5294-5299) and cDNA can be generated by means of reverse transcriptase (for example Moloney MLV reverse transcriptase, available from Gibco/BRL, Bethesda, MD, or AMV reverse transcriptase, obtainable from Seikagaku America, Inc., St.Petersburg, FL).

Synthetic oligonucleotide primers for the amplification, e.g. as shown in SEQ ID NO: 53, SEQ ID NO: 54, SEQ ID NO: 395 or SEQ ID NO: 396, by means of polymerase chain reaction can be generated on the basis of a sequence shown herein, for example the sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393.

Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved regions are those, which show a very little variation in the amino acid in one particular position of several homologs from different origin. The consenus sequences as depicted in SEQ ID NO: 47, SEQ ID NO: 48, SEQ ID NO: 49, SEQ ID NO: 50, SEQ ID NO: 51, SEQ ID NO: 52, SEQ ID NO: 397, SEQ ID NO: 398, SEQ ID NO: 399 and/or SEQ ID NO: 400 are derived from said aligments.

Degenerated primers can then be utilized by PCR for the amplification of fragments of novel proteins having above-mentioned activity, e.g. conferring the increase of the fine chemical after increasing the expression or activity or having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394or further functional homologs of the polypeptide of the invention from other organisms.

These fragments can then be utilized as hybridization probe for isolating the complete gene sequence. As an alternative, the missing 5' and 3' sequences can be isolated by means of RACE-PCR (rapid amplification of cDNA ends). A nucleic acid molecule according to the invention can be amplified using cDNA or, as an alternative, genomic DNA as template and suitable oligonucleotide primers, following standard PCR amplification techniques. The nucleic acid molecule amplified thus can be cloned into a suitable vector and characterized by means of DNA sequence analysis. Oligonucleotides, which correspond to one of the nucleic acid molecules used in the process can be generated by standard synthesis methods, for example using an automatic DNA synthesizer.

Nucleic acid molecules which are advantageously for the process according to the invention can be isolated based on their homology to the nucleic acid molecules disclosed herein using the sequences or part thereof as hybridization probe and following standard hybridization techniques under stringent hybridization conditions. In this context, it is possible to use, for example, isolated nucleic acid molecules of at least 15, 20, 25, 30, 35, 40, 50, 60 or more nucleotides, preferably of at least 15, 20 or 25 nucleotides in length which hybridize under stringent conditions with the above-described nucleic acid molecules, in particular with those which encompass a nucleotide sequence of the nucleic acid molecule used in the process of the invention or encoding a protein used in the invention or of the nucleic acid molecule of the invention. Nucleic acid molecules with 30, 50, 100, 250 or more nucleotides may also be used.

The term "homology" means that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other plant varieties or species, or mutations. These mutations may occur naturally or may be obtained by mutagenesis techniques. The allelic variations may be naturally occurring allelic variants as well as synthetically produced or genetically engineered variants. Structurally equivalents can, for example, be identified by testing the binding of said polypeptide to antibodies or computer based predictions. Structurally equivalent have the similar immunological characteristic, e.g. comprise similar epitopes.

By "hybridizing" it is meant that such nucleic acid molecules hybridize under conventional hybridization conditions, preferably under stringent conditions such as described by, e.g., Sambrook (Molecular Cloning; A Laboratory Manual, 2nd Edition, Cold Spring Harbor Laboratory Press, Cold Spring Harbor, NY (1989)) or in Current Protocols in Molecular Biology, John Wiley & Sons, N. Y. (1989), 6.3.1-6.3.6.

According to the invention, DNA as well as RNA molecules of the nucleic acid of the invention can be used as probes. Further, as template for the identification of functional homologues Northern blot assays as well as Southern blot assays can be performed. The Northern blot assay advantageously provides further informations about the expressed gene product: e.g. expression pattern, occurance of processing steps, like splicing and capping, etc. The Southern blot assay provides additional information about the chromosomal localization and organization of the gene encoding the nucleic acid molecule of the invention.

A preferred, nonlimiting example of stringent hybridization conditions are hybridizations in 6 x sodium chloride/sodium citrate (= SSC) at approximately 45°C, followed by one or more wash steps in 0.2 x SSC, 0.1 % SDS at 50 to 65°C, for example at 50°C, 55°C or 60°C. The skilled worker knows that these hybridization conditions differ as a function of the type of the nucleic acid and, for example when organic solvents are present, with regard to the temperature and concentration of the buffer. The temperature under "standard hybridization conditions" differs for example as a function of the type of the nucleic acid between 42°C and 58°C, preferably between 45°C and 50°C in an aqueous buffer with a concentration of 0.1 x 0.5 x, 1 x, 2x, 3x, 4x or 5 x SSC (pH 7.2). If organic solvent(s) is/are present in the abovementioned buffer, for example 50% formamide, the temperature under standard conditions is approximately 40°C, 42°C or 45°C. The hybridization conditions for DNA:DNA hybrids are preferably for example 0.1 x SSC and 20°C, 25°C, 30°C, 35°C, 40°C or 45°C, preferably between 30°C and 45°C. The hybridization conditions for DNA:RNA hybrids are preferably for example 0.1 x SSC and 30°C, 35°C, 40°C, 45°C, 50°C or 55°C, preferably between 45°C and 55°C. The abovementioned hybridization temperatures are determined for example for a nucleic acid approximately 100 bp (= base pairs) in length and a G + C content of 50% in the absence of formamide. The skilled worker knows to determine the hybridization conditions required with the aid of textbooks, for example the ones mentioned above, or from the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford.

A further example of one such stringent hybridization condition is hybridization at 4XSSC at 65°C, followed by a washing in 0.1XSSC at 65°C for one hour. Alternatively, an exemplary stringent hybridization condition is in 50 % formamide, 4XSSC at 42°C. Further, the conditions during the wash step can be selected from the range of conditions delimited by low-stringency conditions (approximately 2X SSC at 50°C) and high-stringency conditions (approximately 0.2X SSC at 50°C, preferably at 65°C) (20X SSC: 0.3M sodium citrate, 3M NaCl, pH 7.0). In addition, the temperature during the wash step can be raised from low-stringency conditions at room temperature, approximately 22°C, to higher-stringency conditions at approximately 65°C. Both of the parameters salt concentration and temperature can be varied simultaneously, or else one of the two parameters can be kept constant while only the other is varied. Denaturants, for example formamide or SDS, may also be employed during the hybridization. In the presence of 50% formamide, hybridization is preferably effected at 42°C. Relevant factors like i) length of treatment, ii) salt conditions, iii) detergent conditions, iv) competitor DNAs, v) temperature and vi) probe selection can be combined case by case so that not all possibilities can be mentioned herein.
Thus, in a preferred embodiment, Northern blots are prehybridized with Rothi-Hybri-Quick buffer (Roth, Karlsruhe) at 68°C for 2h. Hybridzation with radioactive labelled probe is done overnight at 68°C. Subsequent washing steps are performed at 68°C with 1xSSC.
For Southern blot assays the membrane is prehybridized with Rothi-Hybri-Quick buffer (Roth, Karlsruhe) at 68°C for 2h. The hybridzation with radioactive labelled probe is conducted over night at 68°C. Subsequently the hybridization buffer is discarded and the filter shortly washed using 2xSSC; 0,1 % SDS. After discarding the washing buffer new 2xSSC; 0,1% SDS buffer is added and incubated at 68°C for 15 minutes. This washing step is performed twice followed by an additional washing step using 1xSSC; 0.1% SDS at 68°C for 10 min.

Some further examples of conditions for DNA hybridization (Southern blot assays) and wash step are shown hereinbelow:
(1) Hybridization conditions can be selected, for example, from the following conditions:
   a) 4X SSC at 65°C,
   b) 6X SSC at 45°C,
   c) 6X SSC, 100 mg/ml denatured fragmented fish sperm DNA at 68°C,
   d) 6X SSC, 0.5% SDS, 100 mg/ml denatured salmon sperm DNA at 68°C,
   e) 6X SSC, 0.5% SDS, 100 mg/ml denatured fragmented salmon sperm DNA, 50% formamide at 42°C,
   f) 50% formamide, 4X SSC at 42°C,
   g) 50% (vol/vol) formamide, 0.1 % bovine serum albumin, 0.1 % Ficoll, 0.1% polyvinylpyrrolidone, 50 mM sodium phosphate buffer pH 6.5, 750 mM NaCl, 75 mM sodium citrate at 42°C,
   h) 2X or 4X SSC at 50°C (low-stringency condition), or
   i) 30 to 40% formamide, 2X or 4X SSC at 42°C (low-stringency condition).
(2) Wash steps can be selected, for example, from the following conditions:
   a) 0.015 M NaCl/0.0015 M sodium citrate/0.1% SDS at 50°C.
   b) 0.1X SSC at 65°C.
   c) 0.1X SSC, 0.5 % SDS at 68°C.
   d) 0.1X SSC, 0.5% SDS, 50% formamide at 42°C.
   e) 0.2X SSC, 0.1% SDS at 42°C.
   f) 2X SSC at 65°C (low-stringency condition).

Polypeptides having above-mentioned biological activity, i.e. conferring the fine chemical increase, derived from other organisms, can be encoded by other DNA sequences which hybridize to the sequences shown in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393under relaxed hybridization conditions and which code on expression for peptides having the fine chemical increasing activity.

Further, some applications have to be performed at low stringency hybridisation conditions, without any consequences for the specificity of the hybridisation. For example, a Southern blot analysis of total DNA could be probed with a nucleic acid molecule of the present invention and washed at low stringency (55°C in 2xSSPE0, 1 % SDS). The hybridisation analysis could reveal a simple pattern of only genes encoding polypeptides of the present invention or used in the process of the invention, e.g. having herein-mentioned activity of increasing the fine chemical. A further example of such low-stringent hybridization conditions is 4XSSC at 50°C or hybridization with 30 to 40% formamide at 42°C. Such molecules comprise those which are fragments, analogues or derivatives of the polypeptide of the invention or used in the process of the invention and differ, for example, by way of amino acid and/or nucleotide deletion(s), insertion(s), substitution (s), addition(s) and/or recombination (s) or any other modification(s) known in the art either alone or in combination from the above-described amino acid sequences or their underlying nucleotide sequence(s). However, it is preferred to use high stringency hybridisation conditions.

Hybridization should advantageously be carried out with fragments of at least 5, 10, 15, 20, 25, 30, 35 or 40 bp, advantageously at least 50, 60, 70 or 80 bp, preferably at least 90, 100 or 110 bp. Most preferably are fragments of at least 15, 20, 25 or 30 bp. Preferably are also hybridizations with at least 100 bp or 200, very especially preferably at least 400 bp in length. In an especially preferred embodiment, the hybridization should be carried out with the entire nucleic acid sequence with conditions described above.

The terms "fragment", "fragment of a sequence" or "part of a sequence" mean a truncated sequence of the original sequence referred to. The truncated sequence (nucleic acid or protein sequence) can vary widely in length; the minimum size being a sequence of sufficient size to provide a sequence with at least a comparable function and/or biological activity of the original sequence referred to or hybidizing with the nucleic acid molecule of the invention or used in the process of the invention under stringend conditions, while the maximum size is not critical. In some applications, the maximum size usually is not substantially greater than that required to provide the desired activity and/or function(s) of the original sequence.

Typically, the truncated amino acid sequence will range from about 5 to about 260 amino acids in length. More typically, however, the sequence will be a maximum of about 220 amino acids in length, preferably a maximum of about 215 or 100 amino acids. It is usually desirable to select sequences of at least about 100, 120 or 150 amino acids, up to a maximum of about 200 or 250 amino acids.

The term "epitope" relates to specific immunoreactive sites within an antigen, also known as antigenic determinates. These epitopes can be a linear array of monomers in a polymeric composition - such as amino acids in a protein - or consist of or comprise a more complex secondary or tertiary structure. Those of skill will recognize that immunogens (i.e., substances capable of eliciting an immune response) are antigens; however, some antigen, such as haptens, are not immunogens but may be made immunogenic by coupling to a carrier molecule. The term "antigen" includes references to a substance to which an antibody can be generated and/or to which the antibody is specifically immunoreactive.

In one embodiment the present invention relates to a epitope of the polypeptide of the present invention or used in the process of the present invention and conferring above mentioned biological activity, preferably conferring an increase in the fine chemical.

The term "one or several amino acids" relates to at least one amino acid but not more than that number of amino acids, which would result in a homology of below 50% identity. Preferably, the identity is more than 70% or 80%, more preferred are 85%, 90%, 91%, 92%, 93%, 94% or 95%, even more preferred are 96%, 97%, 98%, or 99% identity.

Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393 is one which is sufficiently complementary to one of the nucleotide sequences as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 such that it can hybridize to one of the nucleotide sequences as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% most preferably at least about 99,1%; 99,2%, 99,3%; 99,4%; 99,5%; 99,6%; 99,7%; 99,8% or 99,9% or more homologous to a nucleotide sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, :131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393, or a portion thereof and preferably has above mentioned biological activity, in particular having a fine chemical increasing activity after increasing the biological acitivity of a protein having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, .108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 and their respective gene products.

The nucleic acid molecule of the invention comprises a nucleotide sequence which hybridizes, preferably hybridizes under stringent conditions as defined herein, to one of the nucleotide sequences as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393, or a portion thereof and encodes a protein having above-mentioned biological activity, e.g. conferring the fine chemical increase, and optionally, having the biological activity of YNL090W.

Moreover, the nucleic acid molecule of the invention can comprise only a portion of the coding region of one of the sequences in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393, for example a fragment which can be used as a probe or primer or a fragment encoding a biologically active portion of the polypeptide of the present invention or of a polypeptide used in the process of the present invention, i.e. having above-mentioned activity, e.g. conferring an increase of methionine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393, an anti-sense sequence of one of the sequences, e.g., set forth in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primers shown in SEQ ID NO: 53, SEQ ID NO: 54, SEQ ID NO: 395 or SEQ ID NO: 396 will at least result in a fragment of gene products as depicted in the sequences SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393.

Primer sets are interchangable. The person skilled in the art knows to combine said primers to result in the desired product, e.g. in a full-length clone or a partial sequence. Probes based on the sequences of the nucleic acid molecule of the invention or used in the process of the present invention can be used to detect transcripts or genomic sequences encoding the same or homologous proteins. The probe can further comprise a label group attached thereto, e.g. the label group can be a radioisotope, a fluorescent compound, an enzyme, or an enzyme co-factor. Such probes can be used as a part of a genomic marker test kit for identifying cells which express an polypepetide of the invention or used in the process of the present invention, such as by measuring a level of an encoding nucleic acid molecule in a sample of cells, e.g., detecting mRNA levels or determining, whether a genomic gene comprising the sequence of the polynucleotide of the invention or used in the processs of the present invention has been mutated or deleted.

The nucleic acid molecule of the invention encodes a polypeptide or portion thereof which includes an amino acid sequence which is sufficiently homologous to the amino acid sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 such that the protein or portion thereof maintains the ability to participate in the fine chemical production, in particular an amino acid increasing activity as mentioned above or as described in the examples in plants or microorganisms is comprised.

As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 such that the protein or portion thereof is able to participate in the increase of the fine chemical production. For examples having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 are described herein.

In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%. 80%, 85%. 90%, 91%, 92%, 93% or 94% and most preferably at least about 95%, 97%, 98%, 99% or more homologous to an entire amino acid sequence of SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 and having above-mentioned activity, e.g.conferring preferably the increase of the fine chemical.

Portions of proteins encoded by the nucleic acid molecule of the invention are preferably biologically active, preferably having above-mentioned annotated activity, e.g. conferring an increase of the fine chemical after increase of activity.

As mentioned herein, the term "biologically active portion" is intended to include a portion, e.g., a domain/motif, that confers increase of the fine chemical or has an immunological activity such that it is binds to an antibody binding specifially to the polypeptide of the present invention or a polypeptide used in the process of the present invention for producing the fine chemical;

The invention further relates to nucleic acid molecules that differ from one of the nucleotide sequences as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 (and portions thereof) due to degeneracy of the genetic code and thus encode a polypeptide of the present invention, in particular a polypeptide having above mentioned activity, e.g. conferring an increase in the fine chemical in a organism. Advantageously, the nucleic acid molecule of the invention comprises, or in an other embodiment has, a nucleotide sequence encoding a protein comprising, or in an other embodiment having, an amino acid sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or the functional homologues. In a still further embodiment, the nucleic acid molecule of the invention encodes a full length protein which is substantially homologous to an amino acid sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or the functional homologues. However, in a preferred embodiment, the nucleic acid molecule of the present invention does not consist of the sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393.

In addition, it will be appreciated by those skilled in the art that DNA sequence polymorphisms that lead to changes in the amino acid sequences may exist within a population. Such genetic polymorphism in the gene encoding the polypeptide of the invention or comprising the nucleic acid molecule of the invention may exist among individuals within a population due to natural variation.

As used herein, the terms "gene" and "recombinant gene" refer to nucleic acid molecules comprising an open reading frame encoding the polypeptide of the invention or comprising the nucleic acid molecule of the invention or encoding the polypeptide used in the process of the present invention, preferably from a crop plant or from a microorgansim useful for the production of fine chemicals, in particular for the production of the fine chemical. Such natural variations can typically result in 1-5% variance in the nucleotide sequence of the gene. Any and all such nucleotide variations and resulting amino acid polymorphisms in genes encoding a polypeptide of the invention or comprising a the nucleic acid molecule of the invention that are the result of natural variation and that do not alter the functional activity as described are intended to be within the scope of the invention.

Nucleic acid molecules corresponding to natural variants homologues of a nucleic acid molecule of the invention, which can also be a cDNA, can be isolated based on their homology to the nucleic acid molecules disclosed herein using the nucleic acid molecule of the invention, or a portion thereof, as a hybridization probe according to standard hybridization techniques under stringent hybridization conditions.

Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising the sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393. The nucleic acid molecule is preferably at least 20, 30, 50, 100, 250 or more nucleotides in length.

The term "hybridizes under stringent conditions" is defined above. In one embodiment, the term "hybridizes under stringent conditions" is intended to describe conditions for hybridization and washing under which nucleotide sequences at least 30 %, 40 %, 50 % or 65% identical to each other typically remain hybridized to each other. Preferably, the conditions are such that sequences at least about 70%, more preferably at least about 75% or 80%, and even more preferably at least about 85%, 90% or 95% or more identical to each other typically remain hybridized to each other.

Preferably, nucleic acid molecule of the invention that hybridizes under stringent conditions to a sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the fine chemical increase after increasing the expression or activity thereof or the activity of a protein of the invention or used in the process of the invention.

In addition to naturally-occurring variants of the sequences of the polypeptide or nucleic acid molecule of the invention as well as of the polypeptide or nucleic acid molecule used in the process of the invention that may exist in the population, the skilled artisan will further appreciate that changes can be introduced by mutation into a nucleotide sequence of the nucleic acid molecule encoding the polypeptide of the invention or used in the process of the present invention, thereby leading to changes in the amino acid sequence of the encoded polypeptide, without altering the functional ability of the polypeptide, preferably not decreasing said activity.

For example, nucleotide substitutions leading to amino acid substitutions at "non-essential" amino acid residues can be made in a sequence of the nucleic acid molecule of the invention or used in the process of the invention, e.g. in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323. 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393.

A"non-essential" amino acid residue is a residue that can be altered from the wild-type sequence of one without altering the activity of said polypeptide, whereas an "essential" amino acid residue is required for an activity as mentioned above, e.g. leading to an increase in the fine chemical in an organism after an increase of activity of the polypeptide. Other amino acid residues, however, (e.g., those that are not conserved or only semi-conserved in the domain having said activity) may not be essential for activity and thus are likely to be amenable to alteration without altering said activity.

Further, a person skilled in the art knows that the codon usage between organisms can differ. Therefore, he may adapt the codon usage in the nucleic acid molecule of the present invention to the usage of the organism in which the polynuclestide or polypeptide is expressed.

Accordingly, the invention relates to nucleic acid molecules encoding a polypeptide having above-mentioned biological activity, e.g. conferring an increase in the the fine chemical in an organism or part thereof that contain changes in amino acid residues that are not essential for said activity. Such polypeptides differ in amino acid sequence from a sequence contained in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 yet retain said activity described herein. The nucleic acid molecule can comprise a nucleotide sequence encoding a polypeptide, wherein the polypeptide comprises an amino acid sequence at least about 50% identical to an amino acid sequence of SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394and is capable of participation in the increase of production of the fine chemical after increasing its activity, e.g. its expression. Preferably, the protein encoded by the nucleic acid molecule is at least about 60% identical to the sequence in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394, more preferably at least about 70% identical to one of the sequences in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394, even more preferably at least about 80%, 90%, 95% homologous to the sequence in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394, and most preferably at least about 96%, 97%, 98%, or 99% identical to the sequence in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394.

To determine the percentage homology (= identity) of two amino acid sequences or of two nucleic acid molecules, the sequences are written one underneath the other for an optimal comparison (for example gaps may be inserted into the sequence of a protein or of a nucleic acid in order to generate an optimal alignment with the other protein or the other nucleic acid).

The amino acid residues or nucleic acid molecules at the corresponding amino acid positions or nucleotide positions are then compared. If a position in one sequence is occupied by the same amino acid residue or the same nucleic acid molecule as the corresponding position in the other sequence, the molecules are homologous at this position (i.e. amino acid or nucleic acid "homology" as used in the present context corresponds to amino acid or nucleic acid "identity". The percentage homology between the two sequences is a function of the number of identical positions shared by the sequences (i.e. % homology = number of identical positions/total number of positions x 100). The terms "homology" and "identity" are thus to be considered as synonyms.

For the determination of the percentage homology (=identity) of two or more amino acids or of two or more nucleotide sequences several computer software programs have been developed. The homology of two or more sequences can be calculated with for example the software fasta, which presently has been used in the version fasta 3 (W. R. Pearson and D. J. Lipman (1988), Improved Tools for Biological Sequence Comparison.PNAS 85:2444- 2448; W. R. Pearson (1990) Rapid and Sensitive Sequence Comparison with FASTP and FASTA, Methods in Enzymology 183:63 - 98; W. R. Pearson and D. J. Lipman (1988) Improved Tools for Biological Sequence Comparison.PNAS 85:2444- 2448; W. R. Pearson (1990); Rapid and Sensitive Sequence Comparison with FASTP and FASTAMethods in Enzymology 183:63 - 98). Another useful program for the calculation of homologies of different sequences is the standard blast program, which is included in the Biomax pedant software (Biomax, Munich, Federal Republic of Germany). This leads unfortunately sometimes to suboptimal results since blast does not always include complete sequences of the subject and the querry. Nevertheless as this program is very efficient it can be used for the comparison of a huge number of sequences. The following settings are typically used for such a comparisons of sequences: -p Program Name [String]; -d Database [String]; default = nr; -i Query File [File In]; default = stdin; -e Expectation value (E) [Real]; default = 10.0; -m alignment view options: 0 = pairwise; 1 = query-anchored showing identities; 2 = query-anchored no identities; 3 = flat query-anchored, show identities; 4 = flat query-anchored, no identities; 5 = query-anchored no identities and blunt ends; 6 = flat query-anchored, no identities and blunt ends; 7 = XML Blast output; 8 = tabular; 9 tabular with comment lines [Integer]; default = 0; -o BLAST report Output File [File Out] Optional; default = stdout; -F Filter query sequence (DUST with blastn, SEG with others) [String]; default = T; -G Cost to open a gap (zero invokes default behavior) [Integer]; default = 0; -E Cost to extend a gap (zero invokes default behavior) [Integer]; default = 0; -X X dropoff value for gapped alignment (in bits) (zero invokes default behavior); blastn 30, megablast 20, tblastx 0, all others 15 [Integer]; default = 0; -I Show GI's in deflines [T/F]; default = F; -q Penalty for a nucleotide mismatch (blastn only) [Integer]; default = -3; -r Reward for a nucleotide match (blastn only) [Integer]; default = 1; -v Number of database sequences to show one-line descriptions for (V) [Integer]; default = 500; -b Number of database sequence to show alignments for (B) [Integer]; default = 250; -f Threshold for extending hits, default if zero; blastp 11, blastn 0, blastx 12, tblastn 13; tblastx 13, megablast 0 [Integer]; default = 0; -g Perfom gapped alignment (not available with tblastx) [T/F]; default = T; -Q Query Genetic code to use [Integer]; default = 1; -D DB Genetic code (for tblast[nx] only) [Integer]; default = 1; -a Number of processors to use [Integer]; default = 1; -O SeqAlign file [File Out] Optional; -J Believe the query defline [T/F]; default = F; -M Matrix [String]; default = BLOSUM62; - W Word size, default if zero (blastn 11, megablast 28, all others 3) [Integer]; default = 0; -z Effective length of the database (use zero for the real size) [Real]; default = 0; -K Number of best hits from a region to keep (off by default, if used a value of 100 is recommended) [Integer]; default = 0; -P 0 for multiple hit, 1 for single hit [Integer]; default = 0; -Y Effective length of the search space (use zero for the real size) [Real]; default = 0; -S Query strands to search against database (for blast[nx], and tblastx); 3 is both, 1 is top, 2 is bottom [Integer]; default = 3; -T Produce HTML output [T/F]; default = F; -I Restrict search of database to list of GI's [String] Optional; -U Use lower case filtering of FASTA sequence [T/F] Optional; default = F; -y X dropoff value for ungapped extensions in bits (0.0 invokes default behavior); blastn 20, megabtast 10, all others 7 [Real]; default = 0.0; -Z X dropoff value for final gapped alignment in bits (0.0 invokes default behavior); blastn/megablast 50, tblastx 0, all others 25 [Integer]; default = 0; -R PSI-TBLASTN checkpoint file [File In] Optional; -n MegaBlast search [T/F]; default = F; -L Location on query sequence [String] Optional; -A Multiple Hits window size, default if zero (blastn/megablast 0, all others 40 [Integer]; default = 0; -w Frame shift penalty (OOF algorithm for blastx) [Integer]; default = 0; -t Length of the largest intron allowed in tblastn for linking HSPs (0 disables linking) [Integer]; default = 0.

[0184.0.0.0] Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351-360, 1987, Higgins et al., CABIOS, 5 1989: 151-153) or preferably with the programs Gap and BestFit, which are respectively based on the algorithms of Needleman and Wunsch [J. Mol. Biol. 48; 443-453 (1970)] and Smith and Waterman [Adv. Appl. Math. 2; 482-489 (1981)]. Both programs are part of the GCG software-package [Genetics Computer Group, 575 Science Drive, Madison, Wisconsin, USA 53711 (1991); Altschul et al. (1997) Nucleic Acids Res. 25:3389 et seq.]. Therefore preferably the calculations to determine the perentages of sequence homology are done with the program Gap over the whole range of the sequences. The following standard adjustments for the comparison of nucleic acid sequences were used: gap weight: 50, length weight: 3, average match: 10.000, average mismatch: 0.000.

For example a sequence which has a 80% homology with sequence SEQ ID NO: 1 at the nucleic acid level is understood as meaning a sequence which, upon comparison with the sequence SEQ ID NO: 1 by the above Gap program algorithm with the above parameter set, has a 80% homology.

In the state of the art, homology between two polypeptides is also understood as meaning the identity of the amino acid sequence over in each case the entire sequence length which is calculated by comparison with the aid of the program algorithm GAP (Wisconsin Package Version 10.0, University of Wisconsin, Genetics Computer Group (GCG), Madison, USA), setting the following parameters:

| | | | |
|---|---|---|---|
| Gap weight: | 8 | Length weight: | 2 |
| Average match: | 2,912 | Average mismatch: | -2,003 |

For example a sequence which has a 80% homology with sequence SEQ ID NO: 2 at the protein level is understood as meaning a sequence which, upon comparison with the sequence SEQ ID NO: 2 by the above program algorithm with the above parameter set, has a 80% homology.

Functional equivalents derived from one of the polypeptides as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as shown in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 according to the invention and are distinguished by essentially the same properties as the polypeptide as shown in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394.

Functional equivalents derived from the nucleic acid sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 203, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 according to the invention and encode polypeptides having essentially the same properties as the polypeptide as shown in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394.

"Essentially the same properties" of a functional equivalent is above all understood as meaning that the functional equivalent has above mentioned acitivty, e.g conferring an increase in the fine chemical amount while increasing the amount of protein, activity or function of said functional equivalent in an organism, e.g. a microorgansim, a plant or plant or animal tissue, plant or animal cells or a part of the same.

A nucleic acid molecule encoding an homologous to a protein sequence of SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 can be created by introducing one or more nucleotide substitutions, additions or deletions into a nucleotide sequence of the nucleic acid molecule of the present invention, in particular of SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393such that one or more amino acid substitutions, additions or deletions are introduced into the encoded protein. Mutations can be introduced into the encoding sequences of SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 by standard techniques, such as site-directed mutagenesis and PCR-mediated mutagenesis.

Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Thus, a predicted nonessential amino acid residue in a polypeptide of the invention or a polypeptide used in the process of the invention is preferably replaced with another amino acid residue from the same family. Alternatively, in another embodiment, mutations can be introduced randomly along all or part of a coding sequence of a nucleic acid molecule of the invention or used in the process of the invention, such as by saturation mutagenesis, and the resultant mutants can be screened for activity described herein to identify mutants that retain or even have increased above mentioned activity, e.g. conferring an increase in content of the fine chemical.

Following mutagenesis of one of the sequences of shown herein, the encoded protein can be expressed recombinantly and the activity of the protein can be determined using, for example, assays described herein (see Examples).

The highest homology of the nucleic acid molecule used in the process according to the invention was found for the following database entries by Gap search.

Homologues of the nucleic acid sequences used, with the sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393, or from the derived nucleic acid sequences, the intention being, however, that the enzyme activity or the biological activity of the resulting proteins synthesized is advantageously retained or increased.

In one embodiment of the present invention, the nucleic acid molecule of the invention or used in the process of the invention comprises the sequences shown in any of the sequences SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393. It is preferred that the nucleic acid molecule comprises as little as possible other nucleotides not shown in any one of SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, the nucleic acid molecule use in the process of the invention is identical to the sequences shown in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393.

Also preferred is that the nucleic acid molecule used in the process of the invention encodes a polypeptide comprising the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment used in the inventive process, the encoded polypeptide is identical to the sequences as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394.

In one embodiment, the nucleic acid molecule of the invention or used in the process encodes a polypeptide comprising the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 and comprises less than 100 further nucleotides. In a further embodiment, said nucleic acid molecule comprises less than 30 further nucleotides. In one embodiment, the nucleic acid molecule used in the process is identical to a coding sequence of the sequences as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 256, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393.

Polypeptides (= proteins), which still have the essential enzymatic activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 expressed under identical conditions.

Homologues of as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241. 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

In a further embodiment, the process according to the present invention comprises the following steps:
(a) selecting an organism or a part thereof expressing the polypeptide of this invention;
(b) mutagenizing the selected organism or the part thereof;
(c) comparing the activity or the expression level of said polypeptide in the mutagenized organism or the part thereof with the activity or the expression of said polypeptide in the selected organisms or the part thereof;
(d) selecting the mutagenized organisms or parts thereof, which comprise an increased activity or expression level of said polypeptide compared to the selected organism (a) or the part thereof;
(e) optionally, growing and cultivating the organisms or the parts thereof; and
(f) recovering, and optionally isolating, the free or bound the fine chemical produced by the selected mutated organisms or parts thereof.

The organisms or part thereof produce according to the herein mentioned process of the invention an increased level of free and/or protein-bound fine chemical compared to said control or selected organisms or parts thereof.

Advantageously the seclected organisms are mutagenized according to the invention. According to the invention mutagenesis is any change of the genetic information in the genom of an organism, that means any structural or compositional change in the nucleic acid preferably DNA of an organism that is not caused by normal segregation or genetic recombiantion processes. Such mutations may occur spontaneously, or may be induced by mutagens as described below. Such change can be induced either randomly or selectivly. In both cases the genetic information of the organism is modified. In general this lead to the situation that the activity of the gene product of the relevant genes inside the cells or inside the organism is increased.

In case of the specific or so called site directed mutagenesis a distinct gene is mutated and thereby its activity and/or the activity or the encoded gene product is repressed, reduced or increased, preferably increased. In the event of a random mutagenesis one or more genes are mutated by chance and their activities and/or the activities of their gene productes are repressed, reduced or increased, preferably increased.

For the purpose of a mutagenesis of a huge population of organisms, such population can be transformed with a DNA construct, which is useful for the activation of as much as possible genes of an organism, preferably all genes. For example the construct can contain a strong promoter or one or more enhancers, which are capable of transcriptionally activate genes in the vicinity of their integration side. With this method it is possible to statistically mutagenize, eg activate nearly all genes of an organism by the random integration of an activation construct. Afterwards the skilled worker can identify those mutagenized lines in which a gene of the invention has been activated, which in turns leads to the desired increase in the fine chemical production.

The genes of the invention can also be activated by mutagensis, either of regulatory or coding regions. In the event of a random mutagenesis a huge number of organisms are treated with a mutagenic agent. The amount of said agent and the intensity of the treatment will be chosen in such a manner that statistically nearly every gene is mutated once. The process for the random mutagensis as well as the respective agens is well known by the skilled person. Such methods are disclosed for example by A.M. van Harten [(1998), "Mutation breeding: theory and practical applications", Cambridge University Press, Cambridge, UK], E Friedberg, G Walker, W Siede [(1995), "DNA Repair and Mutagenesis", Blackwell Publishing], or K. Sankaranarayanan, J. M. Gentile, L. R. Ferguson [(2000) "Protocols in Mutagenesis", Elsevier Health Sciences]. As the skilled worker knows the spontaneous mutation rate in the cells of an organism is very low and that a large numer of chemical, physical or biological agents are available for the mutagenesis of organisms. These agents are named as mutagens or mutagenic agents. As mentioned before three different kinds of mutagens (chemical, physical or biological agents) are available.

There are different classes of chemical mutagens, which can be separated by their mode of action. For example base analogues such as 5-bromouracil, 2-amino purin. Other chemical mutagens are interacting with the DNA such as sulphuric acid, nitrous acid, hydroxylamine; or other alkylating agents such as monofunctional agents like ethyl methanesulfonate, dimethylsulfate, methyl methanesulfonate), bifunctional like dichloroethyl sulphide, Mitomycin, Nitrosoguanidine - dialkylnitrosamine, N-Nitrosoguanidin derivatives, N-alkyl-N-nitro-N-nitroso-guanidine-), ntercalating dyes like Acridine, ethidium bromide).

Physical mutagens are for example ionizing irradiation (X ray), UV irradiation. Different forms of irradiation are available and they are strong mutagens. Two main classes of irradiation can be distinguished: a) non-ionizing irradiation such as UV light or ionizing irradiation such as X ray. Biological mutagens are for example transposable elements for example IS elements such as IS100, transposons such as Tn5, Tn10, Tn916 or Tn1000 or phages like Mu^{amplac}, P1, T5, λplac etc. Methods for introducing this phage DNA into the appropriate microorganism are well known to the skilled worker (see Microbiology, Third Edition, Eds. Davis, B.D., Dulbecco, R., Eisen, H.N. and Ginsberg, H.S., Harper International Edition, 1980). The common procedure of a transposon mutagenesis is the insertion of a transposable element within a gene or nearby for example in the promotor or terminator region and thereby leading to a loss of the gene function. Procedures to localize the transposon within the genome of the organisms are well known by a person skilled in the art.

Preferably a chemical or biochemical procedure is used for the mutagenesis of the organisms. A preferred chemical method is the mutagensis with N-methyl-N-nitro-nitrosoguanidine.

Other biological methods are disclosed by Spee et al. (Nucleic Acids Research, Vol. 21, No. 3, 1993: 777 - 778). Spee et al. teaches a PCR method using dITP for the random mutagenesis. This method described by Spee et al. was further improved by Rellos et al. (Protein Expr. Purif., 5, 1994: 270 - 277). The use of an in vitro recombination technique for molecular mutagenesis is described by Stemmer (Proc. Natl. Acad. Sci. USA, Vol. 91, 1994: 10747 - 10751). Moore et al. (Nature Biotechnology Vol. 14, 1996: 458 - 467) describe the combination of the PCR and recombination methods for increasing the enzymatic activity of an esterase toward a para-nitrobenzyl ester. Another route to the mutagenesis of enzymes is described by Greener et al. in Methods in Molecular Biology (Vol. 57, 1996: 375 - 385). Greener et al. use the specific Escherichia coli strain XL1-Red to generate Escherichia coli mutants, which have increased antibiotic resistance.

In one embodiment, the protein according to the invention or the nucleic acid molecule characterized herein originates from a eukaryotic or prokaryotic organism such as a non-human animal, a plant, a microorganism such as a fungus, yeast, an alga, a diatom or a bacterium. Nucleic acid molecules, which advantageously can be used in the process of the invention originate from yeasts, for example the family Saccharomycetaceae, in particular the genus Saccharomyces, or yeast genera such as Candida, Hansenula, Pichia, Yarrowia, Rhodotorula or Schizosaccharomyces and the especially advantageous from the species Saccharomyces cerevisiae.

In one embodiment, nucleic acid molecules, which advantageously can be used in the process of the invention orginate from yeast, for example from Saccharomycetaceae, particularly from the genus Saccharomyces advantageously form the species Saccharomyces cerevisiae.

If, in the process according to the invention, plants are selected as the donor organism, this plant may, in principle, be in any phylogenetic relation of the recipient plant. Donor and recipient plant may belong to the same family, genus, species, variety or line, resulting in an increasing homology between the nucleic acids to be integrated and corresponding parts of the genome of the recipient plant. This also applies analogously to microorganisms as donor and recipient organism. It might also be advantageously to use nuclei acids molecules from very distinct species, since these might exhibit reduced sensitivity against endogenous regulatory mechanisms and such sequences might not be recognized by endogenous silencing mechanisms.

Accordingly, one embodiment of the application relates to the use of nucleic acid molecules in the process of the invention from plants, e.g. crop plants, e.g. from: B. napus; O. sativa, Glycine max; B. vulgaris, L. japonicus, Z. elegans, Z. mays, C. arietinum, A. thaliana, H. vulgare, N. tabacum, G. hirsutum, P. patens, F. distichus, sunflower linseed or maize or their homologues.

Accordingly, in one embodiment, the invention relates to a nucleic acid molecule, which comprises a nucleic acid molecule selected from the group consisting of:
a) nucleic acid molecule encoding of the polypeptide as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394or a fragment thereof, which confers an increase in the amount of fine chemical in an organism or a part thereof;
b) nucleic acid molecule comprising of the nucleic acid molecule as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 or a fragment thereof, which confers an increase in the amount of fine chemical in an organism or a part thereof;
c) nucleic acid molecule whose sequence can be deduced from a polypeptide sequence encoded by a nucleic acid molecule of (a) or (b) as a result of the degeneracy of the genetic code and conferring an increase in the amount of fine chemical in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of fine chemical in an organism or a part thereof;
e) nucleic acid molecule which hybridizes with a nucleic acid molecule of (a) to (c) under stringent hybridization conditions and conferring an increase in the amount of fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule which encompasses a nucleic acid molecule which is obtained by amplifying nucleic acid molecules from a cDNA library or a genomic library using the primers in SEQ ID NO: 53, SEQ ID NO: 54, SEQ ID NO: 395 or SEQ ID NO: 396 and conferring an increase in the amount of the fine chemical in an organism or a part thereof;
i) nucleic acid molecule encoding a polypeptide which is isolated, e.g. from a expression library, with the aid of monoclonal and/or polyclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule encoding a polypeptide comprising the consensus sequence as depicted in SEQ ID NO: 47, SEQ ID NO: 48, SEQ ID NO: 49, SEQ ID NO: 50, SEQ ID NO: 51, SEQ ID NO: 52, SEQ ID NO: 397, SEQ ID NO: 398, SEQ ID NO: 399 and/or SEQ ID NO: 400 and conferring an increase in the amount of the fine chemical in an organism or a part thereof; and/or
k) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of the nucleic acid molecule as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 or a nucleic acid molecule encoding, preferably at least the mature form of, the polypeptide as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394, and conferring an increase in the amount of the fine chemical in an organism or a part thereof;
or which encompasses a sequence which is complementary thereto;

whereby, preferably, the nucleic acid molecule according to (a) to (k) distinguishes over the sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 by one or more nucleotides. In one embodiment, the nucleic acid molecule of the invention does not consist of the sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137. 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393. In an other embodiment, the nucleic acid molecule of the present invention is at least 30 % identical and less than 100%, 99,999%, 99,99%, 99,9% or 99% identical to the sequence as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393. In a further embodiment the nucleic acid molecule does not encode the polypeptide sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394. Accordingly, in one embodiment, the nucleic acid molecule of the present invention encodes in one embodiment a polypeptide which differs at least in one or more amino acids from the polypeptide as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394. In another embodiment, the nucleic acid molecule as depicted in SEQ ID NO: 1, 3, 5; 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 does not encode a protein of the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394. Accordingly, in one embodiment, the protein encoded by a sequences of a nucleic acid accoriding to (a) to (k) does not consist of the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394. In a further embodiment, the protein of the present invention is at least 30 % identical to protein sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 216, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 and less than 100%, preferably less than 99,999%, 99,99% or 99,9%, more preferably less than 99%, 98%, 97%, 96% or 95% identical to the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394.

The nucleic acid sequences used in the process are advantageously introduced in a nucleic acid construct, preferably an expression cassette, which makes the expression of the nucleic acid molecules in an organism, advantageously a plant or a microorganism possible.

Accordingly, the invention also relates to a nucleic acid construct, preferably to an expression construct, comprising the nucleic acid molecule of the present invention functionally linked to one or more regulatory elements or signals.

As described herein, the nucleic acid construct can also comprise further genes, which are introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes are genes of the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also,possible, for example by increasing mRNA stability or by inserting a translation enhancer sequence.

In principle, the nucleic acid construct can comprise the herein described regulator sequences and further sequences relevant for the expression of the comprised genes. Thus, the nucleic acid construct of the invention can be used as expression cassette and thus can be used directly for introduction into the plant, or else they may be introduced into a vector. Accordingly in one embodiment the nucleic acid construct is an expression cassette comprising a microorganism promoter or a microorganism terminator or both. In another embodiment the expression cassette encompasses a plant promoter or a plant terminator or both.

Accordingly, in one embodiment, the process according to the invention comprises the following steps:
(a) introducing of a nucleic acid construct comprising the nucleic acid molecule of the invention or used in the process of the invention or encoding the polypeptide of the present invention or used in the process of the invention; or
(b) introducing of a nucleic acid molecule, including regulatory sequences or factors, which expression increases the expression of the nucleic acid molecule of the invention or used in the process of the invention or encoding the polypeptide of the present invention or used in the process of the invention in a cell, or an organism or a part thereof, preferably in a plant, plant cell or a microorganism, and
(c) expressing of the gene product encoded by the nucleic acid construct or the nucleic acid molecule mentioned under (a) or (b) in the cell or the organism.

After the introduction and expression of the nucleic acid construct the transgenic organism or cell is advantageously cultured and subsequently harvested. The transgenic organism or cell may be a prokaryotic or eukaryotic organism such as a microorganism, a non-human animal and plant for example a plant or animal cell, a plant or animal tissue, preferably a crop plant, or a part thereof.

To introduce a nucleic acid molecule into a nucleic acid construct, e.g. as part of an expression cassette, the codogenic gene segment is advantageously subjected to an amplification and ligation reaction in the manner known by a skilled person. It is preferred to follow a procedure similar to the protocol for the Pfu DNA polymerase or a Pfu/Taq DNA polymerase mixture. The primers are selected according to the sequence to be amplified. The primers should expediently be chosen in such a way that the amplificate comprise the codogenic sequence from the start to the stop codon. After the amplification, the amplificate is expediently analyzed. For example, the analysis may consider quality and quantity and be carried out following separation by gel electrophoresis. Thereafter, the amplificate can be purified following a standard protocol (for example Qiagen). An aliquot of the purified amplificate is then available for the subsequent cloning step. The skilled worker generally knows suitable cloning vectors.

They include, in particular, vectors which are capable of replication in easy to handle cloning systems like as bacterial yeast or insect cell based (e.g. baculovirus expression) systems, that is to say especially vectors which ensure efficient cloning in E. coli, and which make possible the stable transformation of plants. Vectors, which must be mentioned, in particular are various binary and cointegrated vector systems, which are suitable for the T-DNA-mediated transformation. Such vector systems are generally characterized in that they contain at least the vir genes, which are required for the Agrobacterium-mediated transformation, and the T-DNA border sequences.

In general, vector systems preferably also comprise further cis-regulatory regions such as promoters and terminators and/or selection markers by means of which suitably transformed organisms can be identified. While vir genes and T-DNA sequences are located on the same vector in the case of cointegrated vector systems, binary systems are based on at least two vectors, one of which bears vir genes, but no T-DNA, while a second one bears T-DNA, but no vir gene. Owing to this fact, the last-mentioned vectors are relatively small, easy to manipulate and capable of replication in E. coli and in Agrobacterium. These binary vectors include vectors from the series pBIB-HYG, pPZP, pBecks, pGreen. Those, which are preferably used in accordance with the invention, are Bin19, pBl101, pBinAR, pGPTV and pCAMBIA. An overview of binary vectors and their use is given by Hellens et al, Trends in Plant Science (2000) 5, 446-451.

For a vector preparation, vectors may first be linearized using restriction endonuclease(s) and then be modified enzymatically in a suitable manner. Thereafter, the vector is purified, and an aliquot is employed in the cloning step. In the cloning step, the enzyme-cleaved and, if required, purified amplificate is cloned together with similarly prepared vector fragments, using ligase. In this context, a specific nucleic acid construct, or vector or plasmid construct, may have one or else more codogenic gene segments. The codogenic gene segments in these constructs are preferably linked operably to regulatory sequences. The regulatory sequences include, in particular, plant sequences like the above-described promoters and terminators. The constructs can advantageously be propagated stably in microorganisms, in particular Escherichia coli and/or Agrobacterium tumefaciens, under selective conditions and enable the transfer of heterologous DNA into plants or other microorganisms. In accordance with a particular embodiment, the constructs are based on binary vectors (overview of a binary vector: Hellens et al., 2000). As a rule, they contain prokaryotic regulatory sequences, such as replication origin and selection markers, for the multiplication in microorganisms such as Escherichia coli and Agrobacterium tumefaciens. Vectors can further contain agrobacterial T-DNA sequences for the transfer of DNA into plant genomes or other eukaryotic regulatory sequences for transfer into other eukaryotic cells, e.g. Saccharomyces sp. or other prokaryotic regulatory sequences for the transfer into other prokaryotic cells, e.g. Corynebacterium sp. or Bacillus sp. For the transformation of plants, the right border sequence, which comprises approximately 25 base pairs, of the total agrobacterial T-DNA sequence is advantageously included. Usually, the plant transformation vector constructs according to the invention contain T-DNA sequences both from the right and from the left border region, which contain expedient recognition sites for site-specific acting enzymes, which, in turn, are encoded by some of the vir genes.

Suitable host organisms are known to the skilled worker. Advantageous organisms are described further above in the present application. They include in particular eukaryotes or eubacteria, e.g. prokaryotes or archae bacteria. Advantageously host organisms are microorganisms selected from the group consisting of Actinomycetaceae, Bacillaceae, Brevibacteriaceae, Corynebacteriaceae, Enterobacteriacae, Gordoniaceae, Micrococcaceae, Mycobacteriaceae, Nocardiaceae, Pseudomonaceae, Rhizobiaceae, Streptomycetaceae, Chaetomiaceae, Choanephoraceae, Cryptococcaceae, Cunninghamellaceae, Demetiaceae, Moniliaceae, Mortierellaceae, Mucoraceae, Pythiaceae, Saccharomycetaceae, Saprolegniaceae, Schizosaccharomycetaceae, Sodariaceae, Sporobolomycetaceae, Tuberculariaceae, Adelotheciaceae, Dinophyceae, Ditrichaceae and Prasinophyceae. Preferably are unicellular, microorganisms, e.g. fungi, bacteria or protoza, such as fungi like the genus Claviceps or Aspergillus or gram-positive bacteria such as the genera Bacillus, Corynebacterium, Micrococcus, Brevibacterium, Rhodococcus, Nocardia, Caseobacter or Arthrobacter or gram-negative bacteria such as the genera Escherichia, Flavobacterium or Salmonella, or yeasts such as the genera Rhodotorula, Hansenula, Pichia, Yerrowia, Saccharomyces, Schizosaccharomyces or Candida.

Host organisms which are especially advantageously selected in the process according to the invention are microorganisms selected from the group of the genera and species consisting of Hansenula anomala, Saccharomyces cerevisiae, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Saccharomyces cerevisiae or Escherichia coli K12 and its described strains

Advantageously preferred in accordance with the invention are host organisms of the genus Agrobacterium tumefaciens or plants. Preferred plants are selected from among the families Aceraceae, Anacardiaceae, Apiaceae, Asteraceae, Apiaceae, Betulaceae, Boraginaceae, Brassicaceae, Bromeliaceae, Cactaceae, Caricaceae, Caryophyllaceae, Cannabaceae, Convolvulaceae, Chenopodiaceae, Elaeagnaceae, Geraniaceae, Gramineae, Juglandaceae, Lauraceae, Leguminosae, Linaceae, Cucurbitaceae, Cyperaceae, Euphorbiaceae, Fabaceae, Malvaceae, Nymphaeaceae, Papaveraceae, Rosaceae, Salicaceae, Solanaceae, Arecaceae, Iridaceae, Liliaceae, Orchidaceae, Gentianaceae, Labiaceae, Magnoliaceae, Ranunculaceae, Carifolaceae, Rubiaceae, Scrophulariaceae, Ericaceae, Polygonaceae, Violaceae, Juncaceae, Poaceae, perennial grass, fodder crops, vegetables and ornamentals.

Especially preferred are plants selected from the groups of the families Apiaceae, Asteraceae, Brassicaceae, Cucurbitaceae, Fabaceae, Papaveraceae, Rosaceae, Solanaceae, Liliaceae or Poaceae. Especially advantageous are, in particular, crop plants. Accordingly, an advantageous plant preferably belongs to the group of the genus peanut, oilseed rape, canola, sunflower, safflower, olive, sesame, hazelnut, almond, avocado, bay, pumpkin/squash, linseed, soya, pistachio, borage, maize, wheat, rye, oats, sorghum and millet, triticale, rice, barley, cassava, potato, sugarbeet, fodder beet, egg plant, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, alfalfa, dwarf bean, lupin, clover and lucerne.

In order to introduce, into a plant, the nucleic acid molecule of the invention or used in the process according to the invention, it has proved advantageous first to transfer them into an intermediate host, for example a bacterium or a eukaryotic unicellular cell. The transformation into E. coli, which can be carried out in a manner known per se, for example by means of heat shock or electroporation, has proved itself expedient in this context. Thus, the transformed E. coli colonies can be analysed for their cloning efficiency. This can be carried out with the aid of a PCR. Here, not only the identity, but also the integrity, of the plasmid construct can be verified with the aid of a defined colony number by subjecting an aliquot of the colonies to said PCR. As a rule, universal primers which are derived from vector sequences are used for this purpose, it being possible, for example, for a forward primer to be arranged upstream of the start ATG and a reverse primer to be arranged downstream of the stop codon of the codogenic gene segment. The amplificates are separated by electrophoresis and assessed with regard to quantity and quality.

The nucleic acid constructs, which are optionally verified, are subsequently used for the transformation of the plants or other hosts, e.g. other eukaryotic cells or other prokaryotic cells. To this end, it may first be necessary to obtain the constructs from the intermediate host. For example, the constructs may be obtained as plasmids from bacterial hosts by a method similar to conventional plasmid isolation.

The nucleic acid molecule of the invention or used in the process according to the invention can also be introduced into modified viral vectors like baculovirus vectors for expression in insect cells or plant viral vectors like tobacco mosaic virus or potato virus X-based vectors. Approaches leading to the expression of proteins from the modified viral genome including the the nucleic acid molecule of the invention or used in the process according to the invention involve for example the inoculation of tobacco plants with infectious RNA transcribed in vitro from a cDNA copy of the recombinant viral genome. Another approach utilizes the transfection of whole plants from wounds inoculated with Agrobacterium tumefaciens containing cDNA copies of recombinant plus-sense RNA viruses. Different vectors and virus are known to the skilled worker for expression in different target eg. production plants.

A large number of methods for the transformation of plants are known. Since, in accordance with the invention, a stable integration of heterologous DNA into the genome of plants is advantageous, the T-DNA-mediated transformation has proved expedient in particular. For this purpose, it is first necessary to transform suitable vehicles, in particular agrobacteria, with a codogenic gene segment or the corresponding plasmid construct comprising the nucleic acid molecule of the invention. This can be carried out in a manner known per se. For example, said nucleic acid construct of the invention, or said expression construct or said plasmid construct, which has been generated in accordance with what has been detailed above, can be transformed into competent agrobacteria by means of electroporation or heat shock. In principle, one must differentiate between the formation of cointegrated vectors on the one hand and the transformation with binary vectors on the other hand. In the case of the first alternative, the constructs, which comprise the codogenic gene segment or the nucleic acid molecule of the invention have no T-DNA sequences, but the formation of the cointegrated vectors or constructs takes place in the agrobacteria by homologous recombination of the construct with T-DNA. The T-DNA is present in the agrobacteria in the form of Ti or Ri plasmids in which exogenous DNA has expediently replaced the oncogenes. If binary vectors are used, they can be transferred to agrobacteria either by bacterial conjugation or by direct transfer. These agrobacteria expediently already comprise the vector bearing the vir genes (currently referred to as helper Ti(Ri) plasmid).

One or more markers may expediently also be used together with the nucleic acid construct, or the vector of the invention and, if plants or plant cells shall be transformed together with the T-DNA, with the aid of which the isolation or selection of transformed organisms, such as agrobacteria or transformed plant cells, is possible. These marker genes enable the identification of a successful transfer of the nucleic acid molecules according to the invention via a series of different principles, for example via visual identification with the aid of fluorescence, luminescence or in the wavelength range of light which is discernible for the human eye, by a resistance to herbicides or antibiotics, via what are known as nutritive markers (auxotrophism markers) or antinutritive markers, via enzyme assays or via phytohormones. Examples of such markers which may be mentioned are GFP (= green fluorescent protein); the luciferin/luceferase system, the β-galactosidase with its colored substrates, for example X-Gal, the herbicide resistances to, for example, imidazolinone, glyphosate, phosphinothricin or sulfonylurea, the antibiotic resistances to, for example, bleomycin, hygromycin, streptomycin, kanamycin, tetracyclin, chloramphenicol, ampicillin, gentamycin, geneticin (G418), spectinomycin or blasticidin, to mention only a few, nutritive markers such as the utilization of mannose or xylose, or antinutritive markers such as the resistance to 2-deoxyglucose. This list is a small number of possible markers. The skilled worker is very familiar with such markers. Different markers are preferred, depending on the organism and the selection method.

As a rule, it is desired that the plant nucleic acid constructs are flanked by T-DNA at one or both sides of the codogenic gene segment. This is particularly useful when bacteria of the species Agrobacterium tumefaciens or Agrobacterium rhizogenes are used for the transformation. A method, which is preferred in accordance with the invention, is the transformation with the aid of Agrobacterium tumefaciens. However, biolistic methods may also be used advantageously for introducing the sequences in the process according to the invention, and the introduction by means of PEG is also possible. The transformed agrobacteria can be grown in the manner known per se and are thus available for the expedient transformation of the plants. The plants or plant parts to be transformed are grown or provided in the customary manner. The transformed agrobacteria are subsequently allowed to act on the plants or plant parts until a sufficient transformation rate is reached. Allowing the agrobacteria to act on the plants or plant parts can take different forms. For example, a culture of morphogenic plant cells or tissue may be used. After the T-DNA transfer, antibiotics as a rule eliminate the bacteria, and the regeneration of plant tissue is induced. This is done in particular using suitable plant hormones in order to initially induce callus formation and then to promote shoot development.

The transfer of foreign genes into the genome of a plant is called transformation. In doing this the methods described for the transformation and regeneration of plants from plant tissues or plant cells are utilized for transient or stable transformation. An advantageous transformation method is the transformation in *planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least the flower primordia. The plant is subsequently grown on until the seeds of the treated plant are obtained (Clough and Bent, Plant J. (1998) 16, 735-743). To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Further advantageous transformation methods, in particular for plants, are known to the skilled worker and are described hereinbelow.

Further advantageous and suitable methods are protoplast transformation by poly (ethylene glycol)-induced DNA uptake, the "biolistic" method using the gene cannon - referred to as the particle bombardment method, electroporation, the incubation of dry embryos in DNA solution, microinjection and gene transfer mediated by Agrobacterium. Said methods are described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, in particular of crop plants such as by way of example tobacco plants, for example by bathing bruised leaves or chopped leaves in an agrobacterial solution and then culturing them in suitable media. The transformation of plants by means of Agrobacterium tumefaciens is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.

In addition to the sequence mentioned in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 or its derivatives, it is advantageous additionally to express and/or mutate further genes in the organisms. Especially advantageously, additionally at least one further gene of the fine chemical biosynthetic pathway e.g. the amino acid biosynthetic pathway such as for L-tryptophane, L-isoleucine, L-leucine, L-lysine, L-threonine and/or L-methionine to mention only a couple of them is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals e.g. the amino acids desired since, for example, feedback regulations no longer exist to the same extent or not at all. In addition it might be advantageously to combine the sequences shown in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393 with genes which generally support or enhances to growth or yield of the target organismen, for example genes which lead to faster growth rate of microorganisms or genes which produces stress-, pathogen, or herbicide resistant plants.

In a further embodiment of the process of the invention, therefore, organisms are grown, in which there is simultaneous overexpression of at least one nucleic acid or one of the genes which code for proteins involved in the fine chemical metabolism e.g. the amino acid metabolism, in particular in amino acid synthesis.

A further advantageous nucleic acid sequence which can be expressed in combination with the sequences used in the process and/or the abovementioned biosynthesis genes is the sequence of the ATP/ADP translocator as described in WO 01/20009. This ATP/ADP translocator leads to an increased synthesis of the essential amino acids lysine and/or methionine. Furthermore, an advantageous nucleic acid sequence coexpressed can be threonine adlolase and/or lysine decarboxylase as described in the state of the art.

In a further advantageous embodiment of the process of the invention, the organisms used in the process are those in which simultaneously at least one of the aforementioned genes or one of the aforementioned nucleic acids is mutated so that the activity of the corresponding proteins is influenced by metabolites to a smaller extent compared with the unmutated proteins, or not at all, and that in particular the production according to the invention of the fine chemical for example of the amino acids is not impaired, or so that their specific enzymatic activity is increased. Less influence means in this connection that the regulation of the enzymic activity is less by at least 10%, advantageously at least 20, 30 or 40%, particularly advantageously by at least 50, 60 or 70%, compared with the starting organism, and thus the activity of the enzyme is increased by these figures mentioned compared with the starting organism. An increase in the enzymatic activity means an enzymatic activity which is increased by at least 10%, advantageously at least 20, 30 or 40%, particularly advantageously by at least 50, 60 or 70%, compared with the starting organism. This leads to an increased productivity of the fine chemical.

In a further advantageous embodiment of the process of the invention, the organisms used in the process are those in which simultaneously the fine chemical degrading protein is attenuated, in particular by reducing the rate of expression of the corresponding gene.

In another embodiment of the process of the invention, the organisms used in the process are those in which simultaneously at least one of the aforementioned nucleic acids or of the aforementioned genes is mutated in such a way that the enzymatic activity of the corresponding protein is partially reduced or completely blocked. A reduction in the enzymatic activity means an enzymatic activity, which is reduced by at least 10%, advantageously at least 20, 30 or 40%, particularly advantageously by at least 50, 60 or 70%, preferably more, compared with the starting organism.

If it is intended to transform the host cell, in particular the plant cell, with several constructs or vectors, the marker of a preceding transformation must be removed or a further marker employed in a following transformation. The markers can be removed from the host cell, in particular the plant cell, as described hereinbelow via methods with which the skilled worker is familiar. In particular plants without a marker, in particular without resistance to antibiotics, are an especially preferred embodiment of the present invention.

In the process according to the invention, the nucleic acid sequences used in the process according to the invention are advantageously linked operably to one or more regulatory signals in order to increase gene expression. These regulatory sequences are intended to enable the specific expression of the genes and the expression of protein. Depending on the host organism for example plant or microorganism, this may mean, for example, that the gene is expressed and/or overexpressed after induction only, or that it is expressed and/or overexpressed constitutively. These regulatory sequences are, for example, sequences to which the inductors or repressors bind and which thus regulate the expression of the nucleic acid. In addition to these novel regulatory sequences, or instead of these sequences, the natural regulation of these sequences may still be present before the actual structural genes and, if appropriate, may have been genetically modified so that the natural regulation has been switched off and gene expression has been increased. However, the nucleic acid construct of the invention suitable as expression cassette (= expression construct = gene construct) can also be simpler in construction, that is to say no additional regulatory signals have been inserted before the nucleic acid sequence or its derivatives, and the natural promoter together with its regulation has not been removed. Instead, the natural regulatory sequence has been mutated in such a way that regulation no longer takes place and/or gene expression is increased. These modified promoters can also be introduced on their own before the natural gene in the form of part sequences (= promoter with parts of the nucleic acid sequences according to the invention) in order to increase the activity. Moreover, the gene construct can advantageously also comprise one or more of what are known as enhancer sequences in operable linkage with the promoter, and these enable an increased expression of the nucleic acid sequence. Also, it is possible to insert additional advantageous sequences at the 3' end of the DNA sequences, such as, for example, further regulatory elements or terminators.

The nucleic acid molecules, which encode proteins according to the invention and nucleic acid molecules, which encode other polypeptides may be present in one nucleic acid construct or vector or in several ones. Advantageously, only one copy of the nucleic acid molecule of the invention or its encoding genes is present in the nucleic acid construct or vector. Several vectors or nucleic acid construct or vector can be expressed together in the host organism. The nucleic acid molecule or the nucleic acid construct or vector according to the invention can be inserted in a vector and be present in the cell in a free form. If a stable transformation is preferred, a vector is used, which is stably duplicated over several generations or which is else be inserted into the genome. In the case of plants, integration into the plastid genome or, in particular, into the nuclear genome may have taken place. For the insertion of more than one gene in the host genome the genes to be expressed are present together in one gene construct, for example in above-described vectors bearing a plurality of genes.

As a rule, regulatory sequences for the expression rate of a gene are located upstream (5'), within, and/or downstream (3') relative to to the coding sequence of the nucleic acid molecule of the invention or another codogenic gene segment. They control in particular transcription and/or translation and/or the transcript stability. The expression level is dependent on the conjunction of further cellular regulatory systems, such as the protein biosynthesis and degradation systems of the cell.

Regulatory sequences include transcription and translation regulating sequences or signals, e.g. sequences located upstream (5'), which concern in particular the regulation of transcription or translation initiation, such as promoters or start codons, and sequences located downstream (3'), which concern in particular the regulation of transcription or translation termination and transcript stability, such as polyadenylation signals or stop codons. Regulatory sequences can also be present in transcribed coding regions as well in transcribed non-coding regions, e.g. in introns, as for example splicing sites. Promoters for the regulation of expression of the nucleic acid molecule according to the invention in a cell and which can be employed are, in principle, all those which are capable of stimulating the transcription of genes in the organisms in question, such as microorganisms or plants. Suitable promoters, which are functional in these organisms, are generally known. They may take the form of constitutive or inducible promoters. Suitable promoters can enable the development-and/or tissue-specific expression in multi-celled eukaryotes; thus, leaf-, root-, flower-, seed-, stomata-, tuber- or fruit-specific promoters may advantageously be used in plants.

The regulatory sequences or factors can, as described above, have a positive effect on, the expression of the genes introduced, thus increasing their expression. Thus, an enhancement of the expression can advantageously take place at the transcriptional level by using strong transcription signals such as strong promoters and/or strong enhancers. In addition, enhancement of expression on the translational level is also possible, for example by introducing translation enhancer sequences, e.g., the Ω enhancer e.g. improving the ribosomal binding to the transcript, or by increasing the stability of the mRNA, e.g. by replacing the 3'UTR coding region by a region encoding a 3'UTR known as conferring an high stability of the transcript or by stabilization of the transcript through the elimination of transcript instability, so that the mRNA molecule is translated more often than the wild type. For example in plants AU-rich elements (AREs) and DST (downstream) elements destabilized transcripts. Mutagenesis studies have demonstrated that residues within two of the conserved domains, the ATAGAT and the GTA regions, are necessary for instability function. Therefore removal or mutation of such elements would obviously lead to more stable transcripts, higher transcript rates and higher protein acitivity.Translation enhancers are also the "overdrive sequence", which comprises the tobacco mosaic virus 5'-untranslated leader sequence and which increases the protein/RNA ratio (Gallie et al., 1987, Nucl. Acids Research 15:8693-8711)

Enhancers are generally defined as cis active elements, which can stimulate gene transcription independent of position and orientation. Different enhancers have been identified in plants, which can either stimulate transcription constitutively or tissue or stimuli specific. Well known examples for constitutive enhancers are the enhancer from the 35S promoter (Odell et al., 1985, Nature 313:810-812) or the ocs enhancer (Fromm et al., 1989, Plant Cell 1: 977:984) Another examples are the G-Box motif tetramer which confers high-level constitutive expression in dicot and monocot plants (Ishige et al., 1999, Plant Journal, 18, 443-448) or the petE, a A/T-rich sequence which act as quantitative enhancers of gene expression in transgenic tobacco and potato plants (Sandhu et al., 1998; Plant Mol Biol. 37(5):885-96). Beside that, a large variety of cis-active elements have been described which contribute to specific expression pattern, like organ specific expression or induced expression in response to biotic or abiotic stress. Examples are elements, which provide pathogen or wound-induced expression (Rushton,-2002, Plant Cell, 14, 749-762) or guard cell-specific expression (Plesch, 2001, Plant Journal 28, 455-464).

Advantageous regulatory sequences for the expression of the nucleic acid molecule according to the invention in microorganisms are present for example in promoters such as the cos, tac, rha, trp, tet, trp-tet, Ipp, lac, Ipp-lac, lacl^{q-}, T7, T5, T3, gal, trc, ara, SP6, λ-P_{R} or λ-P_{L} promoter, which are advantageously used in Gram-negative bacteria. Further advantageous regulatory sequences are present for example in the Gram-positive promoters amy, dnaK, xylS and SPO2, in the yeast or fungal promoters ADC1, MFα, AC, P-60, UASH, MCB, PHO, CYC1, GAPDH, TEF, rp28, ADH. Promoters, which are particularly advantageous, are constitutive, tissue or compartment specific and inducible promoters. In general, "promoter" is understood as meaning, in the present context, a regulatory sequence in a nucleic acid molecule, which mediates the expression of a coding sequence segment of a nucleic acid molecule. In general, the promoter is located upstream to the coding sequence segment. Some elements, for example expression-enhancing elements such as enhancer may, however, also be located downstream or even in the transcribed region.

In principle, it is possible to use natural promoters together with their regulatory sequences, such as those mentioned above, for the novel process. It is also possible advantageously to use synthetic promoters, either additionally or alone, in particular when they mediate seed-specific expression such as described in, for example, WO 99/16890.

The expression of the nucleic acid molecules used in the process may be desired alone or in combination with other genes or nucleic acids. Multiple nucleic acid molecules conferring the expression of advantageous genes can be introduced via the simultaneous transformation of several individual suitable nucleic acid constructs, i.e. expression constructs, or, preferably, by combining several expression cassettes on one construct. It is also possible to transform several vectors with in each case several expression cassettes stepwise into the recipient organisms.

As described above, the transcription of the genes introduced should advantageously be terminated by suitable terminators at the 3' end of the biosynthesis genes introduced (behind the stop codon). A terminator, which may be used for this purpose is, for example, the OCS1 terminator, the nos3 terminator or the 35S terminator. As is the case with the promoters, different terminator sequences should be used for each gene. Terminators, which are useful in microorganism, are for example the fimA terminator, txn terminator or trp terminator. Such terminators can be rho-dependent or rho-independent.

Different plant promoters such as, for example, the USP, the LegB4-, the DC3 promoter or the ubiquitin promoter from parsley or other herein mentioned promoter and different terminators may advantageously be used in the nucleic acid construct.

In order to ensure the stable integration, into the transgenic plant, of nucleic acid molecules used in the process according to the invention in combination with further biosynthesis genes over a plurality of generations, each of the coding regions used in the process should be expressed under the control of its own, preferably unique, promoter since repeating sequence motifs may lead to recombination events or to silencing or, in plants, to instability of the T-DNA.

The nucleic acid construct is advantageously constructed in such a way that a promoter is followed by a suitable cleavage site for insertion of the nucleic acid to be expressed, advantageously in a polylinker, followed, if appropriate, by a terminator located behind the polylinker. If appropriate, this order is repeated several times so that several genes are combined in one construct and thus can be introduced into the transgenic plant in order to be expressed. The sequence is advantageously repeated up to three times. For the expression, the nucleic acid sequences are inserted via the suitable cleavage site, for example in the polylinker behind the promoter. It is advantageous for each nucleic acid sequence to have its own promoter and, if appropriate, its own terminator, as mentioned above. However, it is also possible to insert several nucleic acid sequences behind a promoter and, if appropriate, before a terminator if a polycistronic transcription is possible in the host or target cells. In this context, the insertion site, or the sequence of the nucleic acid molecules inserted, in the nucleic acid construct is not decisive, that is to say a nucleic acid molecule can be inserted in the first or last position in the cassette without this having a substantial effect on the expression. However, it is also possible to use only one promoter type in the construct. However, this may lead to undesired recombination events or silencing effects, as said.

Accordingly, in a preferred embodiment, the nucleic acid construct according to the invention confers expression of the nucleic acid molecule of the invention, and, optionally further genes, in a plant and comprises one or more plant regulatory elements. Said nucleic acid construct according to the invention advantageously encompasses a plant promoter or a plant terminator or a plant promoter and a plant terminator.

A "plant" promoter comprises regulatory elements, which mediate the expression of a coding sequence segment in plant cells. Accordingly, a plant promoter need not be of plant origin, but may originate from viruses or microorganisms, in particular for example from viruses which attack plant cells.

The plant promoter can also originates from a plant cell, e.g. from the plant, which is transformed with the nucleic acid construct or vector as described herein. This also applies to other "plant" regulatory signals, for example in "plant" terminators.

A nucleic acid construct suitable for plant expression preferably comprises regulatory elements which are capable of controlling the expression of genes in plant cells and which are operably linked so that each sequence can fulfill its function. Accordingly, the nucleic acid construct can also comprise transcription terminators. Examples for transcriptional termination are polyadenylation signals. Preferred polyadenylation signals are those which originate from Agrobacterium tumefaciens T-DNA, such as the gene 3 of the Ti plasmid pTiACH5, which is known as octopine synthase (Gielen et al., EMBO J. 3 (1984) 835 et seq.) or functional equivalents thereof, but all the other terminators which are functionally active in plants are also suitable.

The nucleic acid construct suitable for plant expression preferably also comprises other operably linked regulatory elements such as translation enhancers, for example the overdrive sequence, which comprises the tobacco mosaic virus 5'-untranslated leader sequence, which increases the protein/RNA ratio (Gallie et al., 1987, Nucl. Acids Research 15:8693-8711).

Other preferred sequences for use in operable linkage in gene expression constructs are targeting sequences, which are required for targeting the gene product into specific cell compartments (for a review, see Kermode, Crit. Rev. Plant Sci. 15, 4 (1996) 285-423 and references cited therein), for example into the vacuole, the nucleus, all types of plastids, such as amyloplasts, chloroplasts, chromoplasts, the extracellular space, the mitochondria, the endoplasmic reticulum, elaioplasts, peroxisomes, glycosomes, and other compartments of cells or extracellular. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell. Targeting sequences are also known for eukaryotic and to a lower extent for prokaryotic organisms and can advantageously be operable linked with the nucleic acid molecule of the present invention to achieve an expression in one of said compartments or extracellular.

For expression in plants, the nucleic acid molecule must, as described above, be linked operably to or comprise a suitable promoter which expresses the gene at the right point in time and in a cell- or tissue-specific manner. Usable promoters are constitutive promoters (Benfey et al., EMBO J. 8 (1989) 2195-2202), such as those which originate from plant viruses, such as 35S CAMV (Franck et al., Cell 21 (1980) 285-294), 19S CaMV (see also US 5352605 and WO 84/02913), 34S FMV (Sanger et al., Plant. Mol. Biol., 14, 1990: 433-443), the parsley ubiquitin promoter, or plant promoters such as the Rubisco small subunit promoter described in US 4,962,028 or the plant promoters PRP1 [Ward et al., Plant. Mol. Biol. 22 (1993)], SSU, PGEL1, OCS [Leisner (1988) Proc Natl Acad Sci USA 85(5): 2553-2557], lib4, usp, mas [Comai (1990) Plant Mol Biol 15 (3):373-381], STLS1, ScBV (Schenk (1999) Plant Mol Biol 39(6):1221-1230), B33, SAD1 or SAD2 (flax promoters, Jain et al., Crop Science, 39 (6), 1999: 1696-1701) or nos [Shaw et al. (1984) Nucleic Acids Res. 12(20):7831-7846]. Stable, constitutive expression of the proteins according to the invention a plant can be advantageous. However, inducible expression of the polypeptide of the invention is advantageous, if a late expression before the harvest is of advantage, as metabolic manipulation may lead to a plant growth retardation.

The expression of plant genes can also be facilitated as described above via a chemical inducible promoter (for a review, see Gatz 1997, Annu. Rev. Plant Physiol. Plant Mol. Biol., 48:89-108). Chemically inducible promoters are particularly suitable when it is desired to express the gene in a time-specific manner. Examples of such promoters are a salicylic acid inducible promoter (WO 95/19443), and abscisic acid-inducible promoter (EP 335 528), a tetracyclin-inducible promoter (Gatz et al. (1992) Plant J. 2, 397-404), a cyclohexanol- or ethanol-inducible promoter (WO 93/21334) or others as described herein.

Other suitable promoters are those which react to biotic or abiotic stress conditions, for example the pathogen-induced PRP1 gene promoter (Ward et al., Plant. Mol. Biol. 22 (1993) 361-366), the tomato heat-inducible hsp80 promoter (US 5,187,267), the potato chill-inducible alpha-amylase promoter (WO 96/12814) or the wound-inducible pinll promoter (EP-A-0 375 091) or others as described herein.

Preferred promoters are in particular those which bring about gene expression in tissues and organs in which the biosynthesis of fine chemical takes place, in seed cells, such as endosperm cells and cells of the developing embryo. Suitable promoters are the oilseed rape napin gene promoter (US 5,608,152), the Vicia faba USP promoter (Baeumlein et al., Mol Gen Genet, 1991, 225 (3):459-67), the Arabidopsis oleosin promoter (WO 98/45461), the Phaseolus vulgaris phaseolin promoter (US 5,504,200), the Brassica Bce4 promoter (WO 91/13980), the bean arc5 promoter, the carrot DcG3 promoter, or the Legumin B4 promoter (LeB4; Baeumlein et al., 1992, Plant Journal, 2 (2):233-9), and promoters which bring about the seed-specific expression in monocotyledonous plants such as maize, barley, wheat, rye, rice and the like. Advantageous seed-specific promoters are the sucrose binding protein promoter (WO 00/26388), the phaseolin promoter and the napin promoter. Suitable promoters which must be considered are the barley Ipt2 or Ipt1 gene promoter (WO 95/15389 and WO 95/23230), and the promoters described in WO 99/16890 (promoters from the barley hordein gene, the rice glutelin gene, the rice oryzin gene, the rice prolamin gene, the wheat gliadin gene, the wheat glutelin gene, the maize zein gene, the oat glutelin gene, the sorghum kasirin gene and the rye secalin gene). Further suitable promoters are Amy32b, Amy 6-6 and Aleurain [US 5,677,474], Bce4 (oilseed rape) [US 5,530,149], glycinin (soya) [EP 571 741], phosphoenolpyruvate carboxylase (soya) [JP 06/62870], ADR12-2 (soya) (WO 98/08962], isocitrate lyase (oilseed rape) [US 5,689,040] or α-amylase (barley) [EP 781 849]. Other promoters which are available for the expression of genes in plants are leaf-specific promoters such as those described in DE-A 19644478 or light-regulated promoters such as, for example, the pea petE promoter.

Further suitable plant promoters are the cytosolic FBPase promoter or the potato ST-LSI promoter (Stockhaus et al., EMBO J. 8, 1989, 2445), the Glycine max phosphoribosylpyrophosphate amidotransferase promoter (GenBank Accession No. U87999) or the node-specific promoter described in EP-A-0 249 676.

Other promoters, which are particularly suitable, are those, which bring about plastid-specific expression. Suitable promoters such as the viral RNA polymerase promoter are described in WO 95/16783 and WO 97/06250, and the Arabidopsis clpP promoter, which is described in WO 99/46394.

Other promoters, which are used for the strong expression of heterologous sequences in as many tissues as possible, in particular also in leaves, are, in addition to several of the abovementioned viral and bacterial promoters, preferably, plant promoters of actin or ubiquitin genes such as, for example, the rice actin1 promoter. Further examples of constitutive plant promoters are the sugarbeet V-ATPase promoters (WO 01/14572). Examples of synthetic constitutive promoters are the Super promoter (WO 95/14098) and promoters derived from G-boxes (WO 94/12015). If appropriate, chemical inducible promoters may furthermore also be used, compare EP-A 388186, EP-A 335528, WO 97/06268.

As already mentioned herein, further regulatory sequences, which may be expedient, if appropriate, also include sequences, which target the transport and/or the localization of the expression products. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell.

Preferred recipient plants are, as described above, in particular those plants, which can be transformed in a suitable manner. These include monocotyledonous and dicotyledonous plants. Plants which must be mentioned in particular are agriculturally useful plants such as cereals and grasses, for example Triticum spp., Zea mays, Hordeum vulgare, oats, Secale cereale, Oryza sativa, Pennisetum glaucum, Sorghum bicolor, Triticale, Agrostis spp., Cenchrus ciliaris, Dactylis glomerata, Festuca arundinacea, Lolium spp., Medicago spp. and Saccharum spp., legumes and oil crops, for example Brassica juncea, Brassica napus, Glycine max, Arachis hypogaea, Gossypium hirsutum, Cicer arietinum, Helianthus annuus, Lens culinaris, Linum usitatissimum, Sinapis alba, Trifolium repens and Vicia narbonensis, vegetables and fruits, for example bananas, grapes, Lycopersicon esculentum, asparagus, cabbage, watermelons, kiwi fruit, Solanum tuberosum, Beta vulgaris, cassava and chicory, trees, for example Coffea species, Citrus spp., Eucalyptus spp., Picea spp., Pinus spp. and Populus spp., medicinal plants and trees, and flowers..

One embodiment of the present invention also relates to a method for generating a vector, which comprises the insertion, into a vector, of the nucleic acid molecule characterized herein, the nucleic acid molecule according to the invention or the expression cassette according to the invention. The vector can, for example, be introduced in to a cell, e.g. a microorganism or a plant cell, as described herein for the nucleic acid construct, or below under transformation or transfection or shown in the examples. A transient or stable transformation of the host or target cell is possible, however, a stable transformation is preferred. The vector according to the invention is preferably a vector, which is suitable for expressing the polypeptide according to the invention in a plant. The method can thus also encompass one or more steps for integrating regulatory signals into the vector, in particular signals, which mediate the expression in microorganisms or plants.

Accordingly, the present invention also relates to a vector comprising the nucleic acid molecule characterized herein as part of a nucleic acid construct 5 suitable for plant expression or the nucleic acid molecule according to the invention.

The advantageous vectors of the invention comprise the nucleic acid molecules which encode proteins according to the invention, nucleic acid molecules which are used in the process, or nucleic acid construct suitable for plant expression comprising the nucleic acid molecules used, either alone or in combination with further genes such as the biosynthesis or regulatory genes of the fine chemical metabolism e.g. with the genes mentioned herein above. In accordance with the invention, the term "vector" refers to a nucleic acid molecule, which is capable of transporting another nucleic acid to which it is linked. One type of vector is a "plasmid", which means a circular double-stranded. DNA loop into which additional DNA segments can be ligated. A further type of vector is a viral vector, it being possible to ligate additional nucleic acids segments into the viral genome. Certain vectors are capable of autonomous replication in a host cell into which they have been introduced (for example bacterial vectors with bacterial replication origin). Other preferred vectors are advantageously completely or partly integrated into the genome of a host cell when they are introduced into the host cell and thus replicate together with the host genome. Moreover, certain vectors are capable of controlling the expression of genes with which they are in operable linkage. In the present context, these vectors are referred to as "expression vectors". As mentioned above, they are capable of autonomous replication or may be integrated partly or completely into the host genome. Expression vectors, which are suitable for DNA recombination techniques usually, take the form of plasmids. In the present description, "plasmid" and "vector" can be used interchangeably since the plasmid is the most frequently used form of a vector. However, the invention is also intended to encompass these other forms of expression vectors, such as viral vectors, which exert similar functions. The term vector is furthermore also to encompass other vectors which are known to the skilled worker, such as phages, viruses such as SV40, CMV, TMV, transposons, IS elements, phasmids, phagemids, cosmids, and linear or circular DNA.

The recombinant expression vectors which are advantageously used in the process comprise the nucleic acid molecules according to the invention or the nucleic acid construct according to the invention in a form which is suitable for expressing, in a host cell, the nucleic acid molecules according to the invention or described herein. Accordingly, the the recombinant expression vectors comprise one or more regulatory signals selected on the basis of the host cells to be used for the expression, in operable linkage with the nucleic acid sequence to be expressed. [0279.0.0.0] In a recombinant expression vector, "operable linkage" means that the nucleic acid molecule of interest is linked to the regulatory signals in such a way that expression of the nucleic acid molecule is possible: they are linked to one another in such a way that the two sequences fulfill the predicted function assigned to the sequence (for example in an in-vitro transcription/translation system, or in a host cell if the vector is introduced into the host cell).

The term "regulatory sequence" is intended to comprise promoters, enhancers and other expression control elements (for example polyadenylation signalsThese regulatory sequences are described, for example, in Goeddel: Gene Expression Technology: Methods in Enzymology 185, Academic Press, San Diego, CA (1990), or see: Gruber and Crosby, in: Methods in Plant Molecular Biology and Biotechnolgy, CRC Press, Boca Raton, Florida, Ed.: Glick and Thompson, chapter 7, 89-108, including the references cited therein. Regulatory sequences encompass those, which control the constitutive expression of a nucleotide sequence in many types of host cells and those which control the direct expression of the nucleotide sequence in specific host cells only, and under specific conditions. The skilled worker knows that the design of the expression vector may depend on factors such as the selection of the host cell to be transformed, the extent to which the desired protein is expressed, and the like. A preferred selection of regulatory sequences is described above, for example promoters, terminators, enhancers and the like. The term regulatory sequence is to be considered as being encompassed by the term regulatory signal. Several advantageous regulatory sequences, in particular promoters and terminators are described above. In general, the regulatory sequences described as advantageous for nucleic acid construct suitable for expression are also applicable for vectors.

The recombinant expression vectors used can be designed specifically for the expression, in prokaryotic and/or eukaryotic cells, of nucleic acid molecules used in the process. This is advantageous since intermediate steps of the vector construction are frequently carried out in microorganisms for the sake of simplicity. For example, the genes according to the invention and other genes can be expressed in bacterial cells, insect cells (using baculovirus expression vectors), yeast cells and other fungal cells [Romanos (1992), Yeast 8:423-488; van den Hondel, (1991), in: More Gene Manipulations in Fungi, J.W. Bennet & L.L. Lasure, Ed., pp. 396-428: Academic Press: San Diego; and van den Hondel, C.A.M.J.J. (1991), in: Applied Molecular Genetics of Fungi, Peberdy, J.F., et al., Ed., pp. 1-28, Cambridge University Press: Cambridge], algae [Falciatore et al., 1999, Marine Biotechnology.1, 3:239-251] using vectors and following a transformation method as described in WO 98/01572, and preferably in cells of multi-celled plants [see Schmidt, R. and Willmitzer, L. (1988) Plant Cell Rep.:583-586; Plant Molecular Biology and Biotechnology, C Press, Boca Raton, Florida, chapter 6/7, pp.71-119 (1993); F.F. White, in: Transgenic Plants, Bd. 1, Engineering and Utilization, Ed.: Kung and R. Wu, Academic Press (1993), 128-43; Potrykus, Annu. Rev. Plant Physiol. Plant Molec. Biol. 42 (1991), 205-225 (and references cited therein)]. Suitable host cells are furthermore discussed in Goeddel, Gene Expression Technology: Methods in Enzymology 185, Academic Press, San Diego, CA (1990). As an alternative, the sequence of the recombinant expression vector can be transcribed and translated in vitro, for example using T7 promotor-regulatory sequences and T7 polymerase.

Proteins can be expressed in prokaryotes using vectors comprising constitutive or inducible promoters, which control the expression of fusion proteins or nonfusion proteins. Typical fusion expression vectors are, inter alia, pGEX (Pharmacia Biotech Inc; Smith, D.B., and Johnson, K.S. (1988) Gene 67:31-40), pMAL (New England Biolabs, Beverly, MA) and pRIT5 (Pharmacia, Piscataway, NJ), in which glutathione-S-transferase (GST), maltose-E-binding protein or protein A is fused with the recombinant target protein. Examples of suitable inducible nonfusion E. coli expression vectors are, inter alia, pTrc (Amann et al. (1988) Gene 69:301-315) and pET 11d [Studier et al., Gene Expression Technology: Methods in Enzymology 185, Academic Press, San Diego, California (1990) 60-89]. The target gene expression of the pTrc vector is based on the transcription of a hybrid trp-lac fusion promoter by the host RNA polymerase. The target gene expression from the pET 11d vector is based on the transcription of a T7-gn10-lac fusion promoter, which is mediated by a coexpressed viral RNA polymerase (T7 gn1). This viral polymerase is provided by the host strains BL21 (DE3) or HMS174 (DE3) by a resident λ-prophage, which harbors a T7 gn1 gene under the transcriptional control of the lacUV 5 promoter.

Other vectors which are suitable in prokaryotic organisms are known to the skilled worker; these vectors are for example in E. coli pLG338, pACYC184, the pBR series, such as pBR322, the pUC series such as pUC18 or pUC19, the M113mp series, pKC30, pRep4, pHS1, pHS2, pPLc236, pMBL24, pLG200, pUR290, pIN-III¹¹³-B1, λgt11 or pBdCl, in Streptomyces pIJ101, pIJ364, pIJ702 or pIJ361, in Bacillus pUB110, pC194 or pBD214, in Corynebacterium pSA77 or pAJ667.

In a further embodiment, the expression vector is a yeast expression vector. Examples of vectors for expression in the yeasts S. cerevisiae encompass pYeDesaturasec1 (Baldari et al. (1987) Embo J. 6:229-234), pMFa (Kurjan and Herskowitz (1982) Cell 30:933-943), pJRY88 (Schultz et al. (1987) Gene 54:113-123) and pYES2 (Invitrogen Corporation, San Diego, CA). Vectors and methods for the construction of vectors which are suitable for use in other fungi, such as the filamentous fungi, encompass those which are described in detail in: van den Hondel, C.A.M.J.J. [(1991), J.F. Peberdy, Ed., pp. 1-28, Cambridge University Press: Cambridge; or in: More Gene Manipulations in Fungi; J.W. Bennet & L.L. Lasure, Ed., pp. 396-428: Academic Press: San Diego]. Examples of other suitable yeast vectors are 2□M, pAG-1, YEp6, YEp13 or pEMBLYe23.

Further vectors, which may be mentioned by way of example, are pALS1, pIL2 or pBB116 in fungi or pLGV23, pGHlac⁺, pBIN19, pAK2004 or pDH51 in plants.

As an alternative, the nucleic acid sequences can be expressed in insect cells using baculovirus expression vectors. Baculovirus vectors which are available for expressing proteins in cultured insect cells (for example Sf9 cells) encompass the pAc series (Smith et al. (1983) Mol. Cell Biol. 3:2156-2165) and the pVL series (Lucklow and Summers (1989) Virology 170:31-39).

The abovementioned vectors are only a small overview of potentially suitable vectors. Further plasmids are known to the skilled worker and are described, for example, in: Cloning Vectors (Ed. Pouwels, P.H., et al., Elsevier, Amsterdam-New York-Oxford, 1985, ISBN 0 444 904018). Further suitable expression systems for prokaryotic and eukaryotic cells, see the chapters 16 and 17 by Sambrook, J., Fritsch, E.F., and Maniatis, T., Molecular Cloning: A Laboratory Manual, 2nd Edition, Cold Spring Harbor Laboratory, Cold Spring Harbor Laboratory Press, Cold Spring Harbor, NY, 1989.

Accordingly, one embodiment of the invention relates to a vector where the nucleic acid molecule according to the invention is linked operably to regulatory sequences which permit the expression in a prokaryotic or eukaryotic or in a prokaryotic and eukaryotic host.

Accordingly, one embodiment of the invention relates to a host cell, which has been transformed stably or transiently with the vector according to the invention or the nucleic acid molecule according to the invention or the nucleic acid construct according to the invention.

Depending on the host organism, the organisms used in the process according to the invention are cultured or grown in a manner with which the skilled worker is familiar. As a rule, microorganisms are grown in a liquid medium comprising a carbon source, usually in the form of sugars, a nitrogen source, usually in the form of organic nitrogen sources such as yeast extract or salts such as ammonium sulfate, trace elements such as iron salts, manganese salts, magnesium salts, and, if appropriate, vitamins, at temperatures between 0°C and 100°C, preferably between 10°C and 60°C, while passing in oxygen. In the event the microorganism is anaerobe, no oxygen is blown through the culture medium. The pH value of the liquid nutrient medium may be kept constant, that is to say regulated during the culturing phase, or not. The organisms may be cultured batchwise, semibatchwise or continuously. Nutrients may be provided at the beginning of the fermentation or fed in semicontinuously or continuously.

The fine chemical produced can be isolated from the organism by methods with which the skilled worker is familiar. For example via extraction, salt precipitation and/or ion-exchange chromatography etc. To this end, the organisms may be disrupted beforehand. The process according to the invention can be conducted batchwise, semibatchwise or continuously. A summary of known culture and isolation techniques can be found in the textbook by Chmiel [Bioprozeßtechnik 1, Einführung in die Bioverfahrenstechnik (Gustav Fischer Verlag, Stuttgart, 1991)], Demain et al. (Industrial Microbiology and Biotechnology, second edition, ASM Press, Washington, D.C., 1999, ISBN 1-55581-128-0] or in the textbook by Storhas [Bioreaktoren und periphere Einrichtungen (Vieweg Verlag, Braunschweig/Wiesbaden, 1994)].

In one embodiment, the present invention relates to a polypeptide encoded by the nucleic acid molecule according to the present invention, preferably conferring an increase in the fine chemical content in an organism or cell after increasing the expression or activity.

The present invention also relates to a process for the production of a polypeptide according to the present invention, the polypeptide being expressed in a host cell according to the invention, preferably in a microorganism or a transgenic plant cell.

In one embodiment, the nucleic acid molecule used in the process for the production of the polypeptide is derived from a microorganism, preferably from a prokaryotic or protozoic cell with a eukaryotic organism as host cell. E.g., in one embodiment the polypeptide is produced in a plant cell or plant with a nucleic acid molecule derived from a prokaryote or a fungus or an alga or another microorganismus but not from plant.

The skilled worker knows that protein and DNA expressed in different organisms differ in many respects and properties, e.g. DNA modulation and imprinting, such as methylation or post-translational modification, as for example glucosylation, phosphorylation, acetylation, myristoylation, ADP-ribosylation, farnesylation, carboxylation, sulfation, ubiquination, etc. though having the same coding sequence. Preferably, the cellular expression control of the corresponding protein differs accordingly in the control mechanisms controlling the activity and expression of an endogenous protein or another eukaryotic protein. One major difference between proteins expressed in prokaryotic or eukaryotic organisms is the amount and pattern of glycosylation. For example in E. coli there are no glycosylated proteins. Proteins expressed in yeasts have high mannose content in the glycosylated proteins, whereas in plants the glycosylation pattern is complex.

The polypeptide of the present invention is preferably produced by recombinant DNA techniques. For example, a nucleic acid molecule encoding the protein is cloned into a vector (as described above), the vector is introduced into a host cell (as described above) and said polypeptide is expressed in the host cell. Said polypeptide can then be isolated from the cells by an appropriate purification scheme using standard protein purification techniques. Alternative to recombinant expression, the polypeptide or peptide of the present invention can be synthesized chemically using standard peptide synthesis techniques.

Moreover, native polypeptides conferring the increase of the fine chemical in an organism or part thereof can be isolated from cells (e.g., endothelial cells), for example using the antibody of the present invention as described below, in particular, an anti-YNL090W protein antibody or an antibody against polypeptides as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394, which can be produced by standard techniques utilizing the polypeptid of the present invention or fragment thereof, i.e., the polypeptide of this invention. Preferred are monoclonal antibodies.

In one embodiment, the present invention relates to a polypeptide having the amino acid sequence encoded by a nucleic acid molecule of the invention or obtainable by a process of the invention. Said polypeptide confers preferably the aforementioned activity, in particular, the polypeptide confers the increase of the fine chemical in a cell or an organsim or a part thereof after increasing the cellular activity, e.g. by increasing the expression or the specific activity of the polypeptide.

In one embodiment, the present invention relates to a polypeptide having the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or as coded by the nucleic acid molecule as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393or functional homologues thereof.

In one advantageous embodiment, in the method of the present invention the activity of a polypeptide is increased comprising or consisting of the consensus sequence as depicted in SEQ ID NO: 47, SEQ ID NO: 48, SEQ ID NO: 49, SEQ ID NO: 50, SEQ ID NO: 51, SEQ ID NO: 52, SEQ ID NO: 397, SEQ ID NO: 398, SEQ ID NO: 399 and/or SEQ ID NO: 400 and in one another embodiment, the present invention relates to a polypeptide comprising or consisting of the consensus sequence as depicted in SEQ ID NO: 47, SEQ ID NO: 48, SEQ ID NO: 49, SEQ ID NO: 50, SEQ ID NO: 51, SEQ ID NO: 52, SEQ ID NO: 397, SEQ ID NO: 398, SEQ ID NO: 399 and/or SEQ ID NO: 400 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid.

In one embodiment not more than 15%, preferably 10%, even more preferred 5%, 4%, 3%, or 2%, most preferred 1% or 0% of the amino acid position indicated by a letter are/is replaced another amino acid.

In one embodiment 20 or less, preferably 15 or 10, preferably 9, 8, 7, or 6, more preferred 5 or 4, even more preferred 3, even more preferred 2, even more preferred 1, most preferred 0 amino acids are inserted into the consensus sequence.

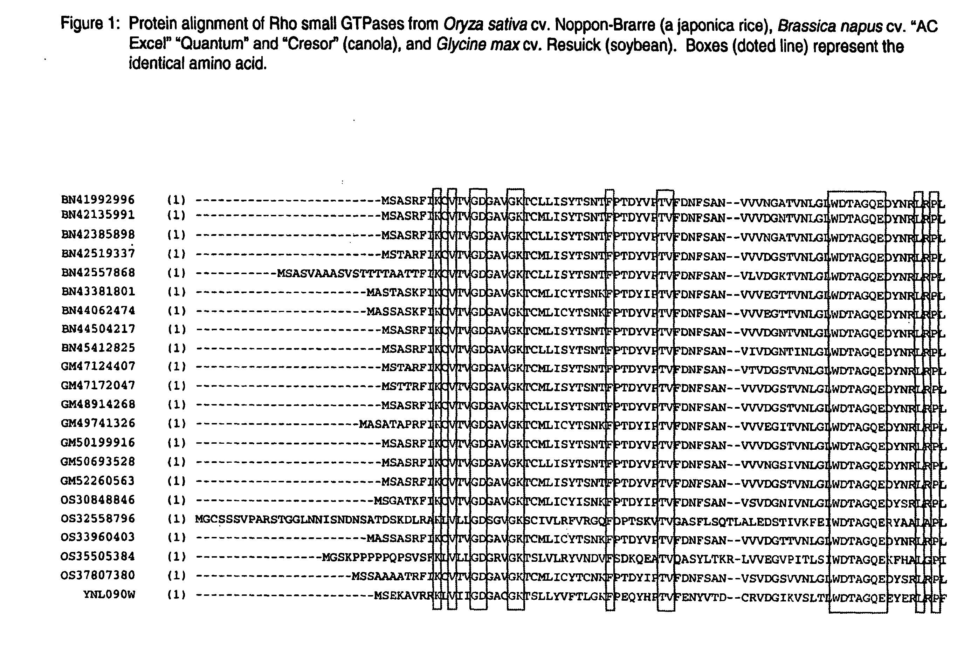

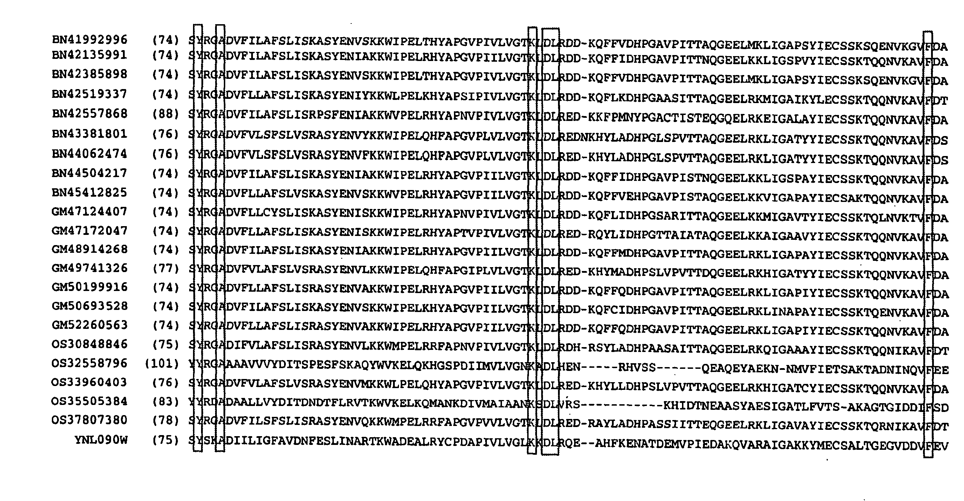

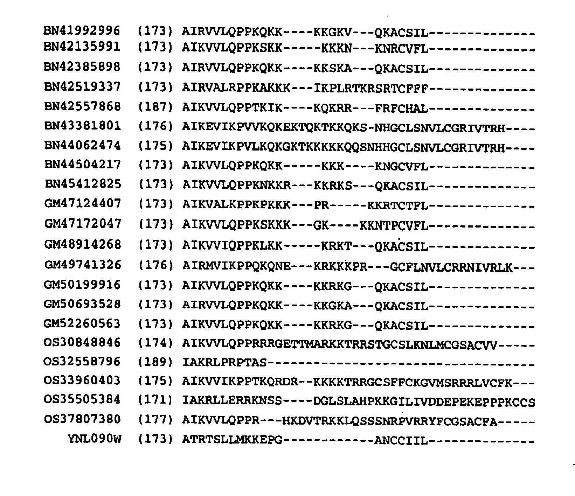

The consensus sequences of specified domains were derived from a multiple alignment of all sequences. The consensus sequences are disclosed under SEQ ID NO: 47, SEQ ID NO: 48, SEQ ID NO: 49, SEQ ID NO: 50, SEQ ID NO: 51, SEQ ID NO: 52, SEQ ID NO: 397, SEQ ID NO: 398, SEQ ID NO: 399 and/or SEQ ID NO: 400. The letters represent the three letter amino acid code and indicate that the amino acids are conserved in all aligned proteins. The letter Xaa stands for amino acids, which are not conserved in all sequences. In some cases of the sequences as depicted in SEQ ID NO: 47, SEQ ID NO: 48, SEQ ID NO: 49, SEQ ID NO: 50, SEQ ID NO: 51, SEQ ID NO: 52, SEQ ID NO: 397, SEQ ID NO: 398, SEQ ID NO: 399 and/or SEQ ID NO: 400 preferred amino acids are mentioned in the sequence protocol in others all natural amino acids are possible. Figur 1 shows an alignment with part of the sequences in the one letter amino acid code. The conserved sequences are framed.

The alignment was performed either with the Software AlignX (sept 25, 2002) a component of Vector NTI Suite 8.0 , InforMax™ ,Invitrogen™ life science software, U.S. Main Office, 7305 Executive Way,Frederick, MD 21704,USA with the following settings: For pairwise alignments: gap opening penality: 10,0; gap extension penality 0,1. For multiple alignments: Gap opening penalty: 10,0; Gap extension penalty: 0,1; Gap separation penalty range: 8; Residue substitution matrix: blosum62; Hydrophilic residues: G P S N D Q E K R; Transition weighting: 0,5; Consensus calculation options: Residue fraction for consensus: 1 or preferably the percent sequence identity between two nucleic acid or polypeptide sequences was determined using the Vector NTI 6.0 (PC) software package (InforMax, 7600 Wisconsin Ave., Bethesda, MD 20814). A gap opening penalty of 15 and a gap extension penalty of 6.66 are used for determining the percent identity of two nucleic acids. A gap opening penalty of 10 and a gap extension penalty of 0.1 are used for determining the percent identity of two polypeptides. All other parameters are set at the default settings. For purposes of a multiple alignment (Clustal W algorithm), the gap opening penalty is 10, and the gap extension penalty is 0.05 with blosum62 matrix.

In one advantageous embodiment, the method of the present invention comprises the increasing of a polypeptide comprising or consisting of plant or microorganism specific consensus sequences.
In one embodiment, said polypeptide of the invention distinguishes over the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 by one or more amino acids. In one embodiment, polypeptide distinguishes form the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 by more than 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 by not more than 80% or 70% of the amino acids, preferably not more than 60% or 50%, more preferred not more than 40% or 30%, even more preferred not more than 20% or 10%. In an other embodiment, said polypeptide of the invention does not consist of the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394.

In one embodiment, the polypeptide of the invention comprises any one of the sequences not known to the public before. In one embodiment, the polypeptide of the invention orginates from a non-plant cell, in particular from a microorganism, and was expressed in a plant cell. In one embodiment, the present invention relates to a polypeptide encoded by the nucleic acid molecule of the invention or used in the process of the invention for which an activity has not been described yet.

In one embodiment, the invention relates to polypeptide conferring an increase in the fine chemical in an organism or part being encoded by the nucleic acid molecule of the invention or used in the process of the invention and having a sequence which distinguishes from the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 by one or more amino acids. In an other embodiment, said polypeptide of the invention does not consist of the sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394. In a further embodiment, said polypeptide of the present invention is less than 100%, 99,999%, 99,99%, 99,9% or 99% identical. In one embodiment, said polypeptide does not consist of the sequence encoded by the nucleic acid molecules as depicted in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393.

In one embodiment, the present invention relates to a polypeptide having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394and which distinguishes over the aforementioned sequences by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

The terms "protein" and "polypeptide" used in this application are interchangeable. "Polypeptide" refers to a polymer of amino acids (amino acid sequence) and does not refer to a specific length of the molecule. Thus peptides and oligopeptides are included within the definition of polypeptide. This term does also refer to or include post-translational modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring.

Preferably, the polypeptide is isolated. An "isolated" or "purified" protein or nucleic acid molecule or biologically active portion thereof is substantially free of cellular material when produced by recombinant DNA techniques or chemical precursors or other chemicals when chemically synthesized.

The language "substantially free of cellular material" includes preparations of the polypeptide of the invention in which the protein is separated from cellular components of the cells in which it is naturally or recombinantly produced. In one embodiment, the language "substantially free of cellular material" includes preparations having less than about 30% (by dry weight) of "contaminating protein", more preferably less than about 20% of "contaminating protein", still more preferably less than about 10% of "contaminating protein", and most preferably less than about 5% "contaminating protein". The term "Contaminating protein" relates to polypeptides, 5 which are not polypeptides of the present invention. When the polypeptide of the present invention or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations in which the polypeptide of the present invention is separated from chemical precursors or other chemicals, which are involved in the synthesis of the protein. The language "substantially free of chemical precursors or other chemicals" includes preparations having less than about 30% (by dry weight) of chemical precursors or non-polypeptide of the inventionchemicals, more preferably less than about 20% chemical precursors or non-polypeptide of the inventionchemicals, still more preferably less than about 10% chemical precursors or non-polypeptide of the invention-chemicals, and most preferably less than about 5% chemical precursors or non- polypeptide of the invention-chemicals. In preferred embodiments, isolated proteins or biologically active portions thereof lack contaminating proteins from the same organism from which the polypeptide of the present invention is derived. Typically, such proteins are produced by recombinant techniques.

A polypeptide of the invention can participate in the process of the present invention. The polypeptide or a portion thereof comprises preferably an amino acid sequence which is sufficiently homologous to an amino acid sequence as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 such that the protein or portion thereof maintains the ability to confer the activity of the present invention. The portion of the protein is preferably a biologically active portion as described herein. Preferably, the polypeptide used in the processof the invention has an amino acid sequence identical as shown in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394.

Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences of SEQ ID NO: 2, 4, 6, 8, 10, 12, 14, 16, 18, 20, 22, 24, 26, 28, 30, 32, 34, 36, 38, 40, 42, 44, 46, 56. 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 362, 384, 386, 388, 390, 392 or 394. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of SEQ ID NO: 1, 3, 5, 7, 9, 11, 13, 15, 17, 19, 21, 23, 25, 27, 29, 31, 33. 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393 or which is homologous thereto, as defined above.

Accordingly the polypeptide of the present invention can vary from SEQ ID NO: 2, 4, 6, 8, 10, 12, 14, 16, 18, 20, 22, 24, 26, 28, 30, 32, 34, 36. 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366. 368, 370, 372, 374, 376, 378, 380, 382, 384, 386, 388, 390, 392 or 394 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394.

For the comparison of amino acid sequences the same algorithms as described above or nucleic acid sequences can be used. Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351-360, 1987, Higgins et al., CABIOS, 5 1989: 151-153) or preferably with the programs Gap and BestFit, which are respectively based on the algorithms of Needleman and Wunsch [J. Mol. Biol. 48; 443-453 (1970)] and Smith and Waterman [Adv. Appl. Math. 2; 482-489 (1981)]. Both programs are part of the GCG software-package [Genetics Computer Group, 575 Science Drive, Madison, Wisconsin, USA 53711 (1991); Altschul et al. (1997) Nucleic Acids Res. 25:3389 et seq.]. Therefore preferably the calculations to determine the perentages of sequence homology are done with the program Gap over the whole range of the sequences. The following standard adjustments for the comparison of amino acid sequences were used: gap weight: 8, length weight: 2, average match: 2.912, average mismatch: -2.003.

Biologically active portions of an polypeptide of the present invention include peptides comprising amino acid sequences derived from the amino acid sequence of the polypeptide of the present invention or used in the process of the present invention, e.g., the amino acid sequence shown in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

Typically, biologically (or immunologically) active portions i.e. peptides, e.g., peptides which are, for example, 5, 10, 15, 20, 30, 35, 36, 37, 38; 39, 40, 50, 100 or more amino acids in length comprise a domain or motif with at least one activity or epitope of a polypeptide of the present invention or used in the process of the present invention. Moreover, other biologically active portions, in which other regions of the polypeptide are deleted, can be prepared by recombinant techniques and evaluated for one or more of the activities described herein.

Manipulation of the nucleic acid molecule of the invention may result in the production of proteins having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394 and having differences from said aforementioned wild-type proteins. These proteins may be improved in efficiency or activity, may be present in greater numbers in the cell than is usual, or may be decreased in efficiency or activity in relation to the wild type protein.

Any mutagenesis strategies for the polypeptide of the present invention or the polypeptide used in the process of the present invention to result in increasing said activity are not meant to be limiting; variations on these strategies will be readily apparent to one skilled in the art. Using such strategies, and incorporating the mechanisms disclosed herein, the nucleic acid molecule and polypeptide of the invention may be utilized to generate plants or parts thereof, expressing wildtype proteins of the invention or mutated protein encoding nucleic acid molecules and polypeptide molecules of the invention such that the yield, production, and/or efficiency of production of a desired compound is improved.

This desired compound may be any natural product of plants, which includes the final products of biosynthesis pathways and intermediates of naturally-occurring metabolic pathways, as well as molecules which do not naturally occur in the metabolism of said cells, but which are produced by a said cells of the invention. Preferrably, the compound is a composition of amino acids or a recovered amino acid, in particular, the fine chemical, free or in protein-bound form.

The invention also provides chimeric or fusion proteins.

As used herein, an "chimeric protein" or "fusion protein" comprises an polypeptide operatively linked to a polypeptide which does not confer above-mentioned activity, in particulare, which does not confer an increase of content of the fine chemical in a cell or an organism or a part thereof, if its activity is increased.

In one embodiment, a protein (= polypeptide)" having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394refers to a polypeptide having an amino acid sequence corresponding to the polypeptide of the invention or used in the process of the invention, whereas a "non- polypeptide of the invention" or "other polypeptide" refers to a polypeptide having an amino acid sequence corresponding to a protein which is not substantially homologous a polypeptide of the invention, preferably which is not substantially homologous to a polypeptide having the biological activity represented by a protein as depicted in SEQ ID NO: 2, 4, 6, 8, 10, 12, 14, 16, 18, 20, 22, 24, 26, 28, 30, 32, 34, 36, 38, 40, 42, 44, 46, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384, 386, 388, 390, 392 or 394, e.g., a protein which does not confer the activity described herein and which is derived from the same or a different organism.

Within the fusion protein, the term "operatively linked" is intended to indicate that the polypeptide of the invention or a polypeptide used in the process of the invention and the "other polypeptide" or a part thereof are fused to each other so that both sequences fulfil the proposed function addicted to the sequence used. The "other polypeptide" can be fused to the N-terminus or C-terminus of the polypeptide of the invention or used in the process of the invention. For example, in one embodiment the fusion protein is a GST-LMRP fusion protein in which the sequences of the polypeptide of the invention or the polypeptide used in the process of the invention are fused to the C-terminus of the GST sequences. Such fusion proteins can facilitate the purification of recombinant polypeptides of the invention or a poylpeptide usefull in the process of the invention.

In another embodiment, the fusion protein is a polypeptide of the invention or a polypeptide used in the process of the invention containing a heterologous signal sequence at its N-terminus. In certain host cells (e.g., mammalian host cells), expression and/or secretion of a polypeptide of the invention or a poylpeptide used in the process of the invention can be increased through use of a heterologous signal sequence. As already mentioned above, targeting sequences, are required for targeting the gene product into specific cell compartment (for a review, see Kermode, Crit. Rev. Plant Sci. 15, 4 (1996) 285-423 and references cited therein), for example into the vacuole, the nucleus, all types of plastids, such as amyloplasts, chloroplasts, chromoplasts, the extracellular space, the mitochondria, the endoplasmic reticulum, elaioplasts, peroxisomes, glycosomes, and other compartments of cells or extracellular. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell. Targeting sequences are also known for eukaryotic and to a lower extent for prokaryotic organisms and can advantageously be operable linked with the nucleic acid molecule of the present invention to achieve an expression in one of said compartments or extracellular.

Preferably, a chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. The fusion gene can be synthesized by conventional techniques including automated DNA synthesizers. Alternatively, PCR amplification of gene fragments can be carried out using anchor primers, which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (e.g., a GST polypeptide). The nucleic acid molecule of the invention can be cloned into such an expression vector such that the fusion moiety is linked in-frame to the encoded protein.

Furthermore, folding simulations and computer redesign of structural motifs of the protein of the invention can be performed using appropriate computer programs (Olszewski, Proteins 25 (1996), 286-299; Hoffman, Comput. Appl. Biosci. 11 (1995), 675-679). Computer modeling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). The appropriate programs can be used for the identification of interactive sites the polypeptide of the invention or polypeptides used in the process of the invention and its substrates or binding factors or other interacting proteins by computer assistant searches for complementary peptide sequences (Fassina, Immunomethods (1994), 114-120). Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used for, e.g., the preparation of peptidomimetics of the protein of the invention or fragments thereof. Such pseudopeptide analogues of the, natural amino acid sequence of the protein may very efficiently mimic the parent protein (Benkirane, J. Biol. Chem. 271 (1996), 33218-33224). For example, incorporation of easily available achiral Q-amino acid residues into a protein of the invention or a fragment thereof results in the substitution of amide bonds by polymethylene units of an aliphatic chain, thereby providing a convenient strategy for constructing a peptidomimetic (Banerjee, Biopolymers 39 (1996), 769-777).

Superactive peptidomimetic analogues of small peptide hormones in other systems are described in the prior art (Zhang, Biochem. Biophys. Res. Commun. 224 (1996), 327-331). Appropriate peptidomimetics of the protein of the present invention can also be identified by the synthesis of peptidomimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Domer, Bioorg. Med. Chem. 4 (1996), 709-715.

Furthermore, a three-dimensional and/or crystallographic structure of the protein of the invention can be used for the design of peptidomimetic inhibitors of the biological activity of the protein of the invention (Rose, Biochemistry 35 (1996), 12933-12944; Rutenber, Bioorg. Med. Chem. 4 (1996), 1545-1558).

Furthermore, a three-dimensional and/or crystallographic structure of the protein of the invention and the identification of interactive sites the polypeptide of the invention and its substrates or binding factors can be used for design of mutants with modulated binding or turn over activities. For example, the active center of the polypeptide of the present invention can be modelled and amino acid residues participating in the catalytic reaction can be modulated to increase or decrease the binding of the substrate to activate or improve the polypeptide. The identification of the active center and the amino acids involved in the catalytic reaction facilitates the screening for mutants having an increased activity.

One embodiment of the invention also relates to an antibody, which binds specifically to the polypeptide according to the invention or parts, i.e. specific fragments or epitopes of such a protein.

The antibodies of the invention can be used to identify and isolate the polypeptide according to the invention and encoding genes in any organism, preferably plants, prepared in plants described herein. These antibodies can be monoclonal antibodies, polyclonal antibodies or synthetic antibodies as well as fragments of antibodies, such as Fab, Fv or scFv fragments etc. Monoclonal antibodies can be prepared, for example, by the techniques as originally described in Köhler and Milstein, Nature 256 (1975), 495, and Galfr6, Meth. Enzymol. 73 (1981), 3, which comprise the fusion of mouse myeloma cells to spleen cells derived from immunized mammals.

Furthermore, antibodies or fragments thereof to the aforementioned peptides can be obtained by using methods, which are described, e.g., in Harlow and Lane "Antibodies, A Laboratory Manual", CSH Press, Cold Spring Harbor, 1988. These antibodies can be used, for example, for the immunoprecipitation and immunolocalization of proteins according to the invention as well as for the monitoring of the synthesis of such proteins, for example, in recombinant organisms, and for the identification of compounds interacting with the protein according to the invention. For example, surface plasmon resonance as employed in the BIAcore system can be used to increase the efficiency of phage antibodies selections, yielding a high increment of affinity from a single library of phage antibodies, which bind to an epitope of the protein of the invention (Schier, Human Antibodies Hybridomas 7 (1996), 97-105; Malmborg, J. Immunol. Methods 183 (1995), 7-13). In many cases, the binding phenomena of antibodies to antigens are equivalent to other ligand/anti-ligand binding.

In one embodiment, the present invention relates to an antisense nucleic acid molecule comprising the complementary sequence of the nucleic acid molecule of the present invention.

Methods to modify the expression levels and/or the activity are known to persons skilled in the art and include for instance overexpression, co-suppression, the use of ribozymes, sense and anti-sense strategies or other gene silencing approaches like RNA interference (RNAi) or promoter methylation."Sense strand" refers to the strand of a double-stranded DNA molecule that is homologous to an mRNA transcript thereof. The "anti-sense strand" contains an inverted sequence, which is complementary to that of the "sense strand".
In addition the expression levels and/or the activity can be modified by the introduction of mutations in the regulatory or coding regions of the nucleic acids of the invention. Furthermore antibodies can be expressed which specifically binds to a polypeptide of interest and thereby blocks it acitivity. The protein-binding factors can, for example, also be aptamers [Famulok M and Mayer G (1999) Curr. Top Microbiol. Immunol. 243: 123-36] or antibodies or antibody fragments or single-chain antibodies. Obtaining these factors has been described, and the skilled worker is familiar therewith. For example, a cytoplasmic scFv antibody has been employed for modulating activity of the phytochrome A protein in genetically modified tobacco plants [Owen M et al. (1992) Biotechnology (NY) 10(7): 790-794; Franken E et al. (1997) Curr. Opin. Biotechnol. 8(4): 411-416; Whitelam (1996) Trend Plant Sci. 1: 286-272].

An "antisense" nucleic acid molecule comprises a nucleotide sequence, which is complementary to a "sense" nucleic acid molecule encoding a protein, e.g., complementary to the coding strand of a double-stranded cDNA molecule or complementary to an encoding mRNA sequence. Accordingly, an antisense nucleic acid molecule can bond via hydrogen bonds to a sense nucleic acid molecule. The antisense nucleic acid molecule can be complementary to an entire coding strand of a nucleic acid molecule conferring the expression of the polypeptide of the invention or used in the process of the present invention, as the nucleic acid molecule of the invention coding strand, or to only a portion thereof. Accordingly, an antisense nucleic acid molecule can be antisense to a "coding region" of the coding strand of a nucleotide sequence of a nucleic acid molecule of the present invention. The term "coding region" refers to the region of the nucleotide sequence comprising codons, which are translated into amino acid residues. Further, the antisense nucleic acid molecule is antisense to a "noncoding region" of the coding strand of a nucleotide sequence encoding the polypeptide of the invention or a polypeptide used in the process of the invention. The term "noncoding region" refers to 5' and 3' sequences which flank the coding region that are not translated into a polypeptide, i.e., also referred to as 5' and 3' untranslated regions (5'-UTR or 3'-UTR).

Given the coding strand sequences encoding the polypeptide of the present invention antisense nucleic acid molecules of the invention can be designed according to the rules of Watson and Crick base pairing.

The antisense nucleic acid molecule can be complementary to the entire coding region of the mRNA encoding the nucleic acid molecule ot the invention or used in the process of the present invention, but can also be an oligonucleotide which is antisense to only a portion of the coding or noncoding region of said mRNA. For example, the antisense oligonucleotide can be complementary to the region surrounding the translation start site of said mRNA. An antisense oligonucleotide can be, for example, about 5, 10, 15, 20, 25, 30, 35, 40, 45, 50, 100 or 200 nucleotides in length. An antisense nucleic acid molecule of the invention can be constructed using chemical synthesis and enzymatic ligation reactions using procedures known in the art. For example, an antisense nucleic acid molecule (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid molecule has been subcloned in an antisense orientation (i.e., RNA transcribed from the inserted nucleic acid molecule will be of an antisense orientation to a target nucleic acid molecule of interest, described further in the following subsection).

The antisense nucleic acid molecules of the invention are typically administered to a cell or generated *in situ* such that they hybridize with or bind to cellular mRNA and/or genomic DNA encoding a polypeptide of the invention having aforementioned the fine chemical increasing activity to thereby inhibit expression of the protein, e.g., by inhibiting transcription and/or translation.

The hybridization can be by conventional nucleotide complementarity to form a stable duplex, or, for example, in the case of an antisense nucleic acid molecule which binds to DNA duplexes, through specific interactions in the major groove of the double helix. The antisense nucleic acid molecule can also be delivered to cells using the vectors described herein. To achieve sufficient intracellular concentrations of the antisense molecules, vector in which the antisense nucleic acid molecule is placed under the control of a strong prokaryotic, viral, or eukaryotic including plant promoters are preferred.

In a further embodiment, the antisense nucleic acid molecule of the invention can be an α-anomeric nucleic acid molecule. A α-anomeric nucleic acid molecule forms specific double-stranded hybrids with complementary RNA in which, contrary to the usual units, the strands run parallel to each other (Gaultier et al. (1987) Nucleic Acids. Res. 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) FEBS Lett. 215:327-330).

Further the antisense nucleic acid molecule of the invention can be also a ribozyme. Ribozymes are catalytic RNA molecules with ribonuclease activity, which are capable of cleaving a single-stranded nucleic acid, such as an mRNA, to which they have a complementary region. Thus, ribozymes (e.g., hammerhead ribozymes (described in Haselhoff and Gerlach (1988) Nature 334:585-591)) can be used to catalytically cleave mRNA transcripts encoding the polypeptide of the invention to thereby inhibit translation of said mRNA. A ribozyme having specificity for a nucleic acid molecule encoding the polypeptide of the invention or used in the process of the invention can be designed based upon the nucleotide sequence of the nucleic acid molecule of the invention or coding a protein used in the process of the invention or on the basis of a heterologous sequence to be isolated according to methods taught in this invention. For example, a derivative of a *Tetrahymena* L-19 IVS RNA can be constructed in which the nucleotide sequence of the active site is complementary to the nucleotide sequence to be cleaved in an encoding mRNA. See, e.g., Cech et al. U.S. Patent No. 4,987,071 and Cech et al. U.S. Patent No. 5,116,742. Alternatively, mRNA encoding the polypeptide of the invention or a polypeptide used in the process of the invention can be used to select a catalytic RNA having a specific ribonuclease activity from a pool of RNA molecules. See, e.g., Bartel, D. and Szostak, J.W. (1993) Science 261:1411-1418.

The antisense molecule of the present invention comprises also a nucleic acid molecule comprising a nucleotide sequences complementary to the regulatory region of an nucleotide sequence encoding the natural occurring polypeptide of the invention, e.g. the polypeptide sequences shown in the sequence listing, or identified according to the methods described herein, e.g., its promoter and/or enhancers, e.g. to form triple helical structures that prevent transcription of the gene in target cells. See generally, Helene, C. (1991) Anticancer Drug Des. 6(6): 569-84; Helene, C. et al. (1992) Ann. N.Y. Acad. Sci. 660:27-36; and Maher, L.J. (1992) Bioassays 14(12):807-15.

Furthermore the present invention relates to a double stranded RNA molecule capable for the reduction or inhibition of the activity of the gene product of a gene encoding the polypeptide of the invention, a polypeptide used in the process of the invention, the nucleic acid molecule of the invention or a nucleic acid molecule used in the process of the invention encoding.

The method of regulating genes by means of double-stranded RNA ("double-stranded RNA interference"; dsRNAi) has been described extensively for animal, yeast, fungi and plant organisms such as Neurospora, zebrafish, Drosophila, mice, planaria, humans, Trypanosoma, petunia or Arabidopsis (for example Matzke MA et al. (2000) Plant Mol. Biol. 43: 401-415; Fire A. et al. (1998) Nature 391: 806-811; WO 99/32619; WO 99/53050; WO 00/68374; WO 00/44914; WO 00/44895; WO 00/49035; WO 00/63364). In addition RNAi is also documented as an advantageously tool for the repression of genes in bacteria such as E. coli for example by Tchurikov et al. [J. Biol. Chem., 2000, 275 (34): 26523 - 26529]. Fire et al. named the phenomenon RNAi for "RNA interference". The techniques and methods described in the above references are expressly referred to. Efficient gene suppression can also be observed in the case of transient expression or following transient transformation, for example as the consequence of a biolistic transformation (Schweizer P et al. (2000) Plant J 2000 24: 895-903). dsRNAi methods are based on the phenomenon that the simultaneous introduction of complementary strand and counterstrand of a gene transcript brings about highly effective suppression of the expression of the gene in question. The resulting phenotype is very similar to that of an analogous knock-out mutant (Waterhouse PM et al. (1998) Proc. Natl. Acad. Sci. USA 95: 13959-64).

Tuschl et al. [Gens Dev., 1999, 13 (24): 3191 - 3197] was able to show that the efficiency of the RNAi method is a function of the length of the duplex, the length of the 3'-end overhangs, and the sequence in these overhangs. Based on the work of Tuschl et al. the following guidelines can be given to the skilled worker: To achieve good results the 5' and 3' untranslated regions of the used nucleic acid sequence and regions close to the start codon should be avoided as this regions are richer in regulatory protein binding sites and interactions between RNAi sequences and such regulatory proteins might lead to undesired interactions. Preferably a region of the used mRNA is selected, which is 50 to 100 nt (= nucleotides or bases) downstream of the AUG start codon. Only dsRNA (= double-stranded RNA) sequences from exons are useful for the method, as sequences from introns have no effect. The G/C content in this region should be greater than 30% and less than 70% ideally around 50%. A possible secondary structure of the target mRNA is less important for the effect of the RNAi method.

The dsRNAi method has proved to be particularly effective and advantageous for reducing the expression of the nucleic acid sequences of the SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of the nucleic acid sequences of the SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 and/or homologs thereof. In a double-stranded RNA molecule for reducing the expression of an protein encoded by a nucleic acid sequence of one of the SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 and/or homologs thereof, one of the two RNA strands is essentially identical to at least part of a nucleic acid sequence, and the respective other RNA strand is essentially identical to at least part of the complementary strand of a nucleic acid sequence.

The term "essentially identical" refers to the fact that the dsRNA sequence may also include insertions, deletions and individual point mutations in comparison to the target sequence while still bringing about an effective reduction in expression. Preferably, the homology as defined above amounts to at least 30%, preferably at least 40%, 50%, 60%, 70% or 80%, very especially preferably at least 90%, most preferably 100%, between the "sense" strand of an inhibitory dsRNA and a part-segment of a nucleic acid sequence of the invention (or between the "antisense" strand and the complementary strand of a nucleic acid sequence, respectively). The part-segment amounts to at least 10 bases, preferably at least 17, 18, 19, 20, 21, 22, 23, 24, 25, 26, 27, 28, 29 or 30 bases, especially preferably at least 40, 50, 60, 70, 80 or 90 bases, very especially preferably at least 100, 200, 300 or 400 bases, most preferably at least 500, 600, 700, 800, 900 or more bases or at least 1000 or 2000 bases or more in length. In another preferred embodiment of the invention the part-segment amounts to 17, 18, 19, 20, 21, 22, 23, 24, 25, 26 or 27 bases, preferably to 20, 21, 22, 23, 24 or 25 bases. These short sequences are preferred in animals and plants. The longer sequences preferably between 200 and 800 bases are preferred in nonmammalian animals, preferably in invertebrates, in yeast, fungi or bacteria, but they are also useable in plants. Long double-stranded RNAs are processed in the organisms into many siRNAs (= small/short interfering RNAs) for example by the protein Dicer, which is a ds-specific Rnase III enzyme. As an alternative, an "essentially identical" dsRNA may also be defined as a nucleic acid sequence, which is capable of hybridizing with part of a gene transcript (for example in 400 mM NaCl, 40 mM PIPES pH 6.4, 1 mM EDTA at 50°C or 70°C for 12 to 16 h).

The dsRNA may consist of one or more strands of polymerized ribonucleotides. Modification of both the sugar-phosphate backbone and of the nucleosides may furthermore be present. For example, the phosphodiester bonds of the natural RNA can be modified in such a way that they encompass at least one nitrogen or sulfur heteroatom. Bases may undergo modification in such a way that the activity of, for example, adenosine deaminase is restricted. These and other modifications are described herein below in the methods for stabilizing antisense RNA.

The dsRNA can be prepared enzymatically; it may also be synthesized chemically, either in full or in part.

The double-stranded structure can be formed starting from a single, self-complementary strand or starting from two complementary strands. In a single, self-complementary strand, "sense" and "antisense" sequence can be linked by a linking sequence ("linker") and form for example a hairpin structure. Preferably, the linking sequence may take the form of an intron, which is spliced out following dsRNA synthesis. The nucleic acid sequence encoding a dsRNA may contain further elements such as, for example, transcription termination signals or polyadenylation signals. If the two strands of the dsRNA are to be combined in a cell or an organism advantageously in a plant, this can be brought about in a variety of ways.

Formation of the RNA duplex can be initiated either outside the cell or within the cell. As shown in WO 99/53050, the dsRNA may also encompass a hairpin structure, by linking the "sense" and "antisense" strands by a "linker" (for example an intron). The self-complementary dsRNA structures are preferred since they merely require the expression of a construct and always encompass the complementary strands in an equimolar ratio.

The expression cassettes encoding the "antisense" or the "sense" strand of the dsRNA or the self-complementary strand of the dsRNA are preferably inserted into a vector and stably inserted into the genome of a plant, using the methods described herein below (for example using selection markers), in order to ensure permanent expression of the dsRNA.

The dsRNA can be introduced using an amount which makes possible at least one copy per cell. A larger amount (for example at least 5, 10, 100, 500 or 1 000 copies per cell) may bring about more efficient reduction.

As has already been described, 100 % sequence identity between the dsRNA and a gene transcript of a nucleic acid sequence of one of the SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391 or 393 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 or homologs thereof of the one organism, may be used to suppress the corresponding expression in another organism.

Due to the high degree of sequence homology between sequences from various organisms (e. g. plants), allows the conclusion that these proteins may be conserved to a high degree within, for example other, plants, it is optionally possible that the expression of a dsRNA derived from one of the disclosed sequences as shown herein or homologs thereof should also have has an advantageous effect in other plant species. Preferably the consensus sequences shown herein can be used for the construction of useful dsRNA molecules.

The dsRNA can be synthesized either in vivo or in vitro. To this end, a DNA sequence encoding a dsRNA can be introduced into an expression cassette under the control of at least one genetic control element (such as, for example, promoter, enhancer, silencer, splice donor or splice acceptor or polyadenylation signal). Suitable advantageous constructs are described herein below. Polyadenylation is not required, nor do elements for initiating translation have to be present.

A dsRNA can be synthesized chemically or enzymatically. Cellular RNA polymerases or bacteriophage RNA polymerases (such as, for example T3, T7 or SP6 RNA polymerase) can be used for this purpose. Suitable methods for the in-vitro expression of RNA are described (WO 97/32016; US 5,593,874; US 5,698,425, US 5,712,135, US 5,789,214, US 5,804,693). Prior to introduction into a cell, tissue or organism, a dsRNA which has been synthesized in vitro either chemically or enzymatically can be isolated to a higher or lesser degree from the reaction mixture, for example by extraction, precipitation, electrophoresis, chromatography or combinations of these methods. The dsRNA can be introduced directly into the cell or else be applied extracellularly (for example into the interstitial space).

Advantageously the RNAi method leads to only a partial loss of gene function and therefore enables the skilled worker to study a gene dose effect in the disered organism and to fine tune the process of the invention. Futhermore it enables a person skilled in the art to study multiple functions of a gene.

Stable transformation of the plant with an expression construct, which brings about the expression of the dsRNA is preferred, however. Suitable methods are described herein below.

A further embodiment of the invention also relates to a method for the generation of a transgenic host or host cell, e.g. a eukaryotic or prokaryotic cell, preferably a transgenic microorganism, a transgenic plant cell or a transgenic plant tissue or a transgenic plant, which comprises introducing, into the plant, the plant cell or the plant tissue, the nucleic acid construct according to the invention, the vector according to the invention, or the nucleic acid molecule according to the invention.

A further embodiment of the invention also relates to a method for the transient generation of a host or host cell, eukaryotic or prokaryotic cell, preferably a transgenic microorganism, a transgenic plant cell or a transgenic plant tissue or a transgenic plant, which comprises introducing, into the plant, the plant cell or the plant tissue, the nucleic acid construct according to the invention, the vector according to the invention, the nucleic acid molecule characterized herein as being contained in the nucleic acid construct of the invention or the nucleic acid molecule according to the invention, whereby the introduced nucleic acid molecules, nucleic acid construct and/or vector is not integrated into the genome of the host or host cell. Therefore the transformants are not stable during the propagation of the host in respect of the introduced nucleic acid molecules, nucleic acid construct and/or vector.

In the process according to the invention, transgenic organisms are also to be understood as meaning - if they take the form of plants - plant cells, plant tissues, plant organs such as root, shoot, stem, seed, flower, tuber or leaf, or intact plants which are grown for the production of the fine chemical.

Growing is to be understood as meaning for example culturing the transgenic plant cells, plant tissue or plant organs on or in a nutrient medium or the intact plant on or in a substrate, for example in hydroponic culture, potting compost or on a field soil.

In a further advantageous embodiment of the process, the nucleic acid molecules can be expressed in single-celled plant cells (such as algae), see Falciatore et al., 1999, Marine Biotechnology 1 (3): 239-251 and references cited therein, and plant cells from higher plants (for example spermatophytes such as crops). Examples of plant expression vectors encompass those which are described in detail herein or in: Becker, D. [(1992) Plant Mol. Biol. 20:1195-1197] and Bevan, M.W. [(1984), Nucl. Acids Res. 12:8711-8721; Vectors for Gene Transfer in Higher Plants; in: Transgenic Plants, Vol. 1, Engineering and Utilization, Ed.: Kung and R. Wu, Academic Press, 1993, pp. 15-38]. An overview of binary vectors and their use is also found in Hellens, R. [(2000), Trends in Plant Science, Vol. 5 No.10, 446-451.

Vector DNA can be introduced into prokaryotic or eukaryotic cells via conventional transformation or transfection techniques. The terms "transformation" and "transfection" include conjugation and transduction and, as used in the present context, are intended to encompass a multiplicity of prior-art methods for introducing foreign nucleic acid molecules (for example DNA) into a host cell, including calcium phosphate coprecipitation or calcium chloride coprecipitation, DEAE-dextran-mediated transfection, PEG-mediated transfection, lipofection, natural competence, chemically mediated transfer, electroporation or particle bombardment. Suitable methods for the transformation or transfection of host cells, including plant cells, can be found in Sambrook et al. (Molecular Cloning: A Laboratory Manual., 2nd Ed., Cold Spring Harbor Laboratory, Cold Spring Harbor Laboratory Press, Cold Spring Harbor, NY, 1989) and in other laboratory handbooks such as Methods in Molecular Biology, 1995, Vol. 44, Agrobacterium protocols, Ed.: Gartland and Davey, Humana Press, Totowa, New Jersey.

The above-described methods for the transformation and regeneration of plants from plant tissues or plant cells are exploited for transient or stable transformation of plants. Suitable methods are the transformation of protoplasts by polyethylene-glycol-induced DNA uptake, the biolistic method with the gene gun - known as the particle bombardment method -, electroporation, the incubation of dry embryos in DNA-containing solution, microinjection and the Agrobacterium-mediated gene transfer. The abovementioned methods are described for example in B. Jenes, Techniques for Gene Transfer, in: Transgenic Plants, Vol. 1, Engineering and Utilization, edited by S.D. Kung and R. Wu, Academic Press (1993) 128-143 and in Potrykus Annu. Rev. Plant Physiol. Plant Molec. Biol. 42 (1991) 205-225. The construct to be expressed is preferably cloned into a vector, which is suitable for transforming Agrobacterium tumefaciens, for example pBin19 (Bevan, Nucl. Acids Res. 12 (1984) 8711). Agrobacteria transformed with such a vector can then be used in the known manner for the transformation of plants, in particular crop plants, such as, for example, tobacco plants, for example by bathing scarified leaves or leaf segments in an agrobacterial solution and subsequently culturing them in suitable media. The transformation of plants with Agrobacterium tumefaciens is described for example by Höfgen and Willmitzer in Nucl. Acid Res. (1988) 16, 9877 or known from, inter alia, F.F. White, Vectors for Gene Transfer in Higher Plants; in Transgenic Plants, Vol. 1, Engineering and Utilization, edited by S.D. Kung and R. Wu, Academic Press, 1993, pp. 15-38.

To select for the successful transfer of the nucleic acid molecule, vector or nucleic acid construct of the invention according to the invention into a host organism, it is advantageous to use marker genes as have already been described above in detail. It is known of the stable or transient integration of nucleic acids into plant cells that only a minority of the cells takes up the foreign DNA and, if desired, integrates it into its genome, depending on the expression vector used and the transfection technique used. To identify and select these integrants, a gene encoding for a selectable marker (as described above, for example resistance to antibiotics) is usually introduced into the host cells together with the gene of interest. Preferred selectable markers in plants comprise those, which confer resistance to an herbicide such as glyphosate or gluphosinate. Other suitable markers are, for example, markers, which encode genes involved in biosynthetic pathways of, for example, sugars or amino acids, such as ß-galactosidase, ura3 or ilv2. Markers, which encode genes such as luciferase, gfp or other fluorescence genes, are likewise suitable. These markers and the aforementioned markers can be used in mutants in whom these genes are not functional since, for example, they have been deleted by conventional methods. Furthermore, nucleic acid molecules, which encode a selectable marker, can be introduced into a host cell on the same vector as those, which encode the polypeptides of the invention or used in the process or else in a separate vector. Cells which have been transfected stably with the nucleic acid introduced can be identified for example by selection (for example, cells which have integrated the selectable marker survive whereas the other cells die).

Since the marker genes, as a rule specifically the gene for resistance to antibiotics and herbicides, are no longer required or are undesired in the transgenic host cell once the nucleic acids have been introduced successfully, the process according to the invention for introducing the nucleic acids advantageously employs techniques which enable the removal, or excision, of these marker genes. One such a method is what is known as cotransformation. The cotransformation method employs two vectors simultaneously for the transformation, one vector bearing the nucleic acid according to the invention and a second bearing the marker gene(s). A large proportion of transformants receives or, in the case of plants, comprises (up to 40% of the transformants and above), both vectors. In case of transformation with Agrobacteria, the transformants usually receive only a part of the vector, the sequence flanked by the T-DNA which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase resource or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases, the marker gene must be eliminated by performing crosses. In microbiology, techniques were developed which make possible, or facilitate, the detection of such events. A further advantageous method relies on what are known as recombination systems, whose advantage is that elimination by crossing can be dispensed with. The best-known system of this type is what is known as the Cre/lox system. Cre1 is a recombinase, which removes the sequences located between the loxP sequences. If the marker gene is integrated between the loxP sequences, it is removed, once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Agrobacteria transformed with an expression vector according to the invention may also be used in the manner known per se for the transformation of plants such as experimental plants like Arabidopsis or crop plants, such as, for example, cereals, maize, oats, rye, barley, wheat, soya, rice, cotton, sugarbeet, canola, sunflower, flax, hemp, potato, tobacco, tomato, carrot, bell peppers, oilseed rape, tapioca, cassava, arrow root, tagetes, alfalfa, lettuce and the various tree, nut, and grapevine species, in particular oil-containing crop plants such as soya, peanut, castor-oil plant, sunflower, maize, cotton, flax, oilseed rape, coconut, oil palm, safflower (Carthamus tinctorius) or cocoa beans, for example by bathing scarified leaves or leaf segments in an agrobacterial solution and subsequently growing them in suitable media.

In addition to the transformation of somatic cells, which then has to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic (Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289). Alternative methods are based on the repeated removal of the influorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension (Bechthold, N (1993). C R Acad Sci Paris Life Sci, 316: 1194-1199), while in the case of the"floral dip" method the developing floral tissue is incubated briefly with a surfactant-treated agrobacterial suspension (Clough, SJ und Bent, AF (1998). The Plant J. 16, 735-743). A certain proportion of transgenic seeds are harvested in both cases, and these seeds can be distinguished from nontransgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process, which has been schematically displayed in Klaus et al., 2004 (Nature Biotechnology 22(2), 225-229). Briefly the sequences to be transformed are cloned together with a selectable marker gene between flanking sequences homologous to the chloroplast genome. These homologous flanking sequences direct site-specific integration into the plastome. Plastidal transformation has been described for many different plant species and an overview can be taken from Bock (2001) Transgenic plastids in basic research and plant biotechnology. J Mol Biol. 2001 Sep 21; 312(3): 425-38 or Maliga, P (2003) Progress towards commercialization of plastid transformation technology. Trends Biotechnol. 21, 20-28. Further biotechnological progress has recently been reported in form of marker free plastid transformants, which can be produced by a transient cointegrated maker gene (Klaus et al., 2004, Nature Biotechnology 22(2), 225-229).

The genetically modified plant cells can be regenerated via all methods with which the skilled worker is familiar. Suitable methods can be found in the abovementioned publications of S.D. Kung and R. Wu, Potrykus or Höfgen and Willmitzer.

Accordingly, the present invention thus also relates to a plant cell comprising the nucleic acid construct according to the invention, the nucleic acid molecule according to the invention or the vector according to the invention.

Accordingly the present invention relates to any cell transgenic for any nucleic acid characterized as part of the invention, e.g. conferring the increase of the fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. encoding a polypeptide having the biological activity of the protein of the invention. Due to the above mentioned activity the fine chemical content in a cell or an organism is increased. For example, due to modulation or manupulation, the cellular activity is increased, e.g. due to an increased expression or specific activity of the subject matters of the invention in a cell or an organism or a part thereof. Transgenic for a polypeptide having biological activity of the protein of the invention or activity means herein that due to modulation or manipulation of the genome, the biological activity of the protein of the invention is increased in the cell or organism or part thereof. Examples are described above in context with the process of the invention

"Transgenic", for example regarding a nucleic acid molecule, an nucleic acid construct or a vector comprising said nucleic acid molecule or an organism transformed with said nucleic acid molecule, nucleic acid construct or vector, refers to all those subjects originating by recombinant methods in which either
a) the nucleic acid sequence, or
b) a genetic control sequence linked operably to the nucleic acid sequence, for example a promoter, or
c) (a) and (b)
are not located in their natural genetic environment or have been modified by recombinant methods, an example of a modification being a substitution, addition, deletion, inversion or insertion of one or more nucleotide residues. Natural genetic environment refers to the natural chromosomal locus in the organism of origin, or to the presence in a genomic library. In the case of a genomic library, the natural genetic environment of the nucleic acid sequence is preferably retained, at least in part. The environment flanks the nucleic acid sequence at least at one side and has a sequence of at least 50 bp, preferably at least 500 bp, especially preferably at least 1000 bp, very especially preferably at least 5000 bp, in length.

A naturally occurring expression cassette - for example the naturally occurring combination of the promoter of the protein of the invention with the corresponding protein gene - becomes a transgenic expression cassette when it is modified by non-natural, synthetic "artificial" methods such as, for example, mutagenization. Such methods have been described (US 5,565,350; WO 00/15815; also see above).

Further, the plant cell, plant tissue or plant can also be transformed such that further enzymes and proteins are (over)expressed which expression supports an increase of the fine chemical.

However, transgenic also means that the nucleic acids according to the invention are located at their natural position in the genome of an organism, but that the sequence has been modified in comparison with the natural sequence and/or that the regulatory sequences of the natural sequences have been modified. Preferably, transgenic/recombinant is to be understood as meaning the transcription of the nucleic acids used in the process according to the invention occurs at a non-natural position in the genome, that is to say the expression of the nucleic acids is homologous or, preferably, heterologous. This expression can be transiently or of a sequence integrated stably into the genome.

The term "transgenic plants" used in accordance with the invention also refers to the progeny of a transgenic plant, for example the T₁, T₂, T₃ and subsequent plant generations or the BC₁, BC₂, BC₃ and subsequent plant generations. Thus, the transgenic plants according to the invention can be raised and selfed or crossed with other individuals in order to obtain further transgenic plants according to the invention. Transgenic plants may also be obtained by propagating transgenic plant cells vegetatively. The present invention also relates to transgenic plant material, which can be derived from a transgenic plant population according to the invention. Such material includes plant cells and certain tissues, organs and parts of plants in all their

manifestations, such as seeds, leaves, anthers, fibers, tubers, roots, root hairs, stems, embryo, calli, cotelydons, petioles, harvested material, plant tissue, reproductive tissue and cell cultures, which are derived from the actual transgenic plant and/or can be used for bringing about the transgenic plant.

Any transformed plant obtained according to the invention can be used in a conventional breeding scheme or in in vitro plant propagation to produce more transformed plants with the same characteristics and/or can be used to introduce the same characteristic in other varieties of the same or related species. Such plants are also part of the invention. Seeds obtained from the transformed plants genetically also contain the same characteristic and are part of the invention. As mentioned before, the present invention is in principle applicable to any plant and crop that can be transformed with any of the transformation method known to those skilled in the art. Another embodiment of the invention is the use of the nucleic acid molecule as claimed in above in mapping and breeding processes for the identification of plant varieties having and increased capacity for production of the fine chemical.

In an especially preferred embodiment, the organism, the host cell, plant cell, plant, microorganism or plant tissue according to the invention is transgenic.

Accordingly, the invention therefore relates to transgenic organisms transformed with at least one nucleic acid molecule, nucleic acid construct or vector according to the invention, and to cells, cell cultures, tissues, parts - such as, for example, in the case of plant organisms, plant tissue, for example leaves, roots and the like - or propagation material derived from such organisms, or intact plants. The terms "recombinant (host)", and "transgenic (host)" are used interchangeably in this context. Naturally, these terms refer not only to the host organism or target cell in question, but also to the progeny, or potential progeny, of these organisms or cells. Since certain modifications may occur in subsequent generations owing to mutation or environmental effects, such progeny is not necessarily identical with the parental cell, but still comes within the scope of the term as used herein.

Suitable organisms for the process according to the invention or as hosts are all these eukaryotic or prokaryotic organisms, which are capable of synthesizing the fine chemcial. The organisms used as hosts are microorganisms, such as bacteria, fungi, yeasts or algae, non-human animals, or plants, such as dictotyledonous or monocotyledonous plants.

In principle all plants can be used as host organism, especially the plants mentioned above as source organism. Preferred transgenic plants are, for example, selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants such as plants advantageously selected from the group of the genus peanut, oilseed rape, canola, sunflower, safflower, olive, sesame, hazelnut, almond, avocado, bay, pumpkin/squash, linseed, soya, pistachio, borage, maize, wheat, rye, oats, sorghum and millet, triticale, rice, barley, cassava, potato, sugarbeet, egg plant, alfalfa, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, dwarf bean, lupin, clover and Luceme for mentioning only some of them.

Preferred plant cells, plant organs, plant tissues or parts of plants originate from the under source organism mentioned plant families, preferably from the abovementioned plant genus, more preferred from abovementioned plants spezies.

Transgenic plants comprising the fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. The fine chemical produced in the process according to the invention may, however, also be isolated from the plant in free or bound form. The fine chemical produced by this process can be harvested by harvesting the organisms either from the culture in which they grow or from the field. This can be done via expressing, grinding and/or extraction, salt precipitation and/or ion-exchange chromatography of the plant parts, preferably the plant seeds, plant fruits, plant tubers and the like.

In a further embodiment, the present invention relates to a process for the generation of a microorganism, comprising the introduction, into the microorganism or parts thereof, of the nucleic acid construct of the invention, or the vector of the invention or the nucleic acid molecule of the invention.

In another embodiment, the present invention relates also to a transgenic microorganism comprising the nucleic acid molecule of the invention, the nucleic acid construct of the invention or the vector as of the invention. Appropriate microorganisms have been described herein before under source organism, preferred are in particular aforementioned strains suitable for the production of fine chemicals.

Accordingly, the present invention relates also to a process according to the present invention whereby the produced fine chemical or fine chemical composition is isolated.

In this manner, more than 50% by weight, advantageously more than 60% by weight, preferably more than 70% by weight, especially preferably more than 80% by weight, very especially preferably more than 90% by weight, of the fine chemical produced in the process can be isolated. The resulting fine chemical can, if appropriate, subsequently be further purified, if desired mixed with other active ingredients such as vitamins, amino acids, carbohydrates, antibiotics and the like, and, if appropriate, formulated.

The fine chemical obtained in the process is suitable as starting material for the synthesis of further products of value. For example, they can be used in combination with other ingredients or alone for the production of pharmaceuticals, foodstuffs, animal feeds or cosmetics. Accordingly, the present invention relates a method for the production of a pharmaceuticals, food stuff, animal feeds, nutrients or cosmetics comprising the steps of the process according to the invention, including the isolation of the fine chemical or fine chemical composition produced and if desired formulating the product with a pharmaceutical acceptable carrier or formulating the product in a form acceptable for an application in agriculture. A further embodiment according to the invention is the use of the fine chemical produced in the process or of the transgenic organisms in animal feeds, foodstuffs, medicines, food supplements, cosmetics or pharmaceuticals.

In principle all microorganisms can be used as host organism especially the ones mentioned under source organism above. It is advantageous to use in the process of the invention transgenic microorganisms such as fungi such as the genus Claviceps or Aspergillus or Gram-positive bacteria such as the genera Bacillus, Corynebacterium, Micrococcus, Brevibacterium, Rhodococcus, Nocardia, Caseobacter or Arthrobacter or Gram-negative bacteria such as the genera Escherichia, Flavobacterium or Salmonella or yeasts such as the genera Rhodotorula, Saccharomyces, Hansenula or Candida. Particularly advantageous organisms are selected from the group of genera Corynebacterium, Brevibacterium, Escherichia, Bacillus, Rhodotorula, Saccharomyces, Hansenula, Candida, Claviceps or Flavobacterium. It is very particularly advantageous to use in the process of the invention microorganisms selected from the group of genera and species consisting of Saccharomyces cerevisiae, Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Saccharomyces cerevisiae, Escherichia coli K12 and its described strains.

The process of the invention is, when the host organisms are microorganisms, advantageously carried out at a temperature between 0°C and 95°C, preferably between 10°C and 85°C, particularly preferably between 15°C and 75°C, very particularly preferably between 15°C and 45°C. The pH is advantageously kept at between pH 4 and 12, preferably between pH 6 and 9, particularly preferably between pH 7 and 8, during this. The process of the invention can be operated batchwise, semibatchwise or continuously. A summary of known cultivation methods is to be found in the textbook by Chmiel (Bioprozeßtechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)). The culture medium to be used must meet the requirements of the respective strains in a suitable manner. Descriptions of culture media for various microorganisms are present in the handbook "Manual of Methods for General Bacteriology" of the American Society for Bacteriology (Washington D. C., USA, 1981). These media, which can be employed according to the invention include, as described above, usually one or more carbon sources, nitrogen sources, inorganic salts, vitamins and/or trace elements. Preferred carbon sources are sugars such as mono-, di- or polysaccharides. Examples of very good carbon sources are glucose, fructose, mannose, galactose, ribose, sorbose, ribulose, lactose, maltose, sucrose, raffinose, starch or cellulose. Sugars can also be added to the media via complex compounds such as molasses, or other byproducts of sugar refining. It may also be advantageous to add mixtures of various carbon sources. Other possible carbon sources are oils and fats such as, for example, soybean oil, sunflower oil, peanut oil and/or coconut fat, fatty acids such as, for example, palmitic acid, stearic acid and/or linoleic acid, alcohols and/or polyalcohols such as, for example, glycerol, methanol and/or ethanol and/or organic acids such as, for example, acetic acid and/or lactic acid. Nitrogen sources are usually organic or inorganic nitrogen compounds or materials, which contain these compounds. Examples of nitrogen sources include ammonia in liquid or gaseous form or ammonium salts such as ammonium sulfate, ammonium chloride, ammonium phosphate, ammonium carbonate or ammonium nitrate, nitrates, urea, amino acids or complex nitrogen sources such as corn steep liquor, soybean meal, soybean protein, yeast extract, meat extract and others. The nitrogen sources may be used singly or as a mixture. Inorganic salt compounds, which may be present in the media include the chloride, phosphorus or sulfate salts of calcium, magnesium, sodium, cobalt, molybdenum, potassium, manganese, zinc, copper and iron.

For preparing sulfur-containing fine chemicals, in particular the fine chemical, it is possible to use as sulfur source inorganic sulfur-containing compounds such as, for example, sulfates, sulfites, dithionites, tetrathionates, thiosulfates, sulfides or else organic sulfur compounds such as mercaptans and thiols.

It is possible to use as phosphorus source phosphoric acid, potassium dihydrogenphosphate or dipotassium hydrogenphosphate or the corresponding sodium-containing salts. Chelating agents can be added to the medium in order to keep the metal ions in solution. Particularly suitable chelating agents include dihydroxyphenols such as catechol or protocatechuate, or organic acids such as citric acid. The fermentation media employed according to the invention for cultivating microorganisms normally also contain other growth factors such as vitamins or growth promoters, which include, for example, biotin, riboflavin, thiamine, folic acid, nicotinic acid, pantothenate and pyridoxine. Growth factors and salts are often derived from complex media components such as yeast extract, molasses, corn steep liquor and the like. Suitable precursors can moreover be added to the culture medium. The exact composition of the media compounds depends greatly on the particular experiment and is chosen individually for each specific case. Information about media optimization is obtainable from the textbook "Applied Microbiol. Physiology, A Practical Approach" (editors P.M. Rhodes, P.F. Stanbury, IRL Press (1997) pp. 53-73, ISBN 0 19 963577 3). Growth media can also be purchased from commercial suppliers such as Standard 1 (Merck) or BHI (Brain heart infusion, DIFCO) and the like. All media components are sterilized either by heat (1.5 bar and 121°C for 20 min) or by sterilizing filtration. The components can be sterilized either together or, if necessary, separately. All media components can be present at the start of the cultivation or optionally be added continuously or batchwise. The temperature of the culture is normally between 15°C and 45°C, preferably at 25°C to 40°C, and can be kept constant or changed during the experiment. The pH of the medium should be in the range from 5 to 8.5, preferably around 7. The pH for the cultivation can be controlled during the cultivation by adding basic compounds such as sodium hydroxide, potassium hydroxide, ammonia or aqueous ammonia or acidic compounds such as phosphoric acid or sulfuric acid. Foaming can be controlled by employing antifoams such as, for example fatty acid polyglycol esters. The stability of plasmids can be maintained by adding to the medium suitable substances having a selective effect, for example antibiotics. Aerobic conditions are maintained by introducing oxygen or oxygen-containing gas mixtures such as, for example ambient air into the culture. The temperature of the culture is normally from 20°C to 45°C and preferably from 25°C to 40°C. The culture is continued until formation of the desired product is at a maximum. This aim is normally achieved within 10 hours to 160 hours.

The fermentation broths obtained in this way, containing the fine chemical in particular for example amino acids such as L-methionine, L-threonine and/or L-lysine, normally have a dry matter content of from 7.5 to 25% by weight. Sugar-limited fermentation is additionally advantageous, at least at the end, but especially over at least 30% of the fermentation time. This means that the concentration of utilizable sugar in the fermentation medium is kept at, or reduced to, ≥ 0 to 3 g/l during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

However, it is also possible to purify the fine chemical produced further. For this purpose, the product-containing composition is subjected to a chromatography on a suitable resin, in which case the desired product or the impurities are retained wholly or partly on the chromatography resin. These chromatography steps can be repeated if necessary, using the same or different chromatography resins. The skilled worker is familiar with the choice of suitable chromatography resins and their most effective use. The purified product can be concentrated by filtration or ultrafiltration and stored at a temperature at which the stability of the product is a maximum.

The identity and purity of the isolated compound(s) can be determined by prior art techniques. These include high performance liquid chromatography (HPLC), spectroscopic methods, mass spectrometry (MS), staining methods, thin-layer chromatography, NIRS, enzyme assay or microbiological assays. These analytical methods are summarized in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Vol. A27, VCH: Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Vol. 17.

In yet another aspect, the invention also relates to harvestable parts and to propagation material of the transgenic plants according to the invention which either contain transgenic plant cells expressing a nucleic acid molecule according to the invention or which contains cells which show an increased cellular activity of the polypeptide of the invention, e.g. an increased expression level or higher activity of the described protein.

Harvestable parts can be in principle any useful parts of a plant, for example, flowers, pollen, seedlings, tubers, leaves, stems, fruit, seeds, roots etc. Propagation material includes, for example, seeds, fruits, cuttings, seedlings, tubers, rootstocks etc. Preferred are seeds, fruits, seedlings or tubers as harvestable or propagation material.

The invention furthermore relates to the use of the transgenic organisms according to the invention and of the cells, cell cultures, parts - such as, for example, roots, leaves and the like as mentioned above in the case of transgenic plant organisms - derived from them, and to transgenic propagation material such as seeds or fruits and the like as mentioned above, for the production of foodstuffs or feeding stuffs, pharmaceuticals or fine chemicals.

Accordingly in another embodiment, the present invention relates to the use of the nucleic acid molecule, the organism, e.g. the microorganism, the plant, plant cell or plant tissue, the vector, or the polypeptide of the present invention for making fine chemicals such as fatty acids, carotenoids, isoprenoids, vitamins, lipids, wax esters, (poly)saccharides and/or polyhydroxyalkanoates, and/or its metabolism products, in particular, steroid hormones, cholesterol, prostaglandin, triacylglycerols, bile acids and/or ketone bodies producing cells, tissues and/or plants. There are a number of mechanisms by which the yield, production, and/or efficiency of production of fatty acids, carotenoids, isoprenoids, vitamins, wax esters, lipids, (poly)saccharides and/or polyhydroxyalkanoates, and/or its metabolism products, in particular, steroid hormones, cholesterol, triacylglycerols, prostaglandin, bile acids and/or ketone bodies or further of above defined fine chemicals incorporating such an altered protein can be affected. In the case of plants, by e.g. increasing the expression of acetyl-CoA which is the basis for many products, e.g., fatty acids, carotenoids, isoprenoids, vitamines, lipids, (poly)saccharides, wax esters, and/or polyhydroxyalkanoates, and/or its metabolism products, in particular, prostaglandin, steroid hormones, cholesterol, triacylglycerols, bile acids and/or ketone bodies in a cell, it may be possible to increase the amount of the produced said compounds thus permitting greater ease of harvesting and purification or in case of plants more efficient partitioning. Further, one or more of said metabolism products, increased amounts of the cofactors, precursor molecules, and intermediate compounds for the appropriate biosynthetic pathways maybe required. Therefore, by increasing the number and/or activity of transporter proteins involved in the import of nutrients, such as carbon sources (i.e., sugars), nitrogen sources (i.e., amino acids, ammonium salts), phosphate, and sulfur, it may be possible to improve the production of acetyl CoA and its metabolism products as mentioned above, due to the removal of any nutrient supply limitations on the biosynthetic process. In particular, it may be possible to increase the yield, production, and/or efficiency of production of said compounds, e.g. fatty acids, carotenoids, isoprenoids, vitamins, was esters, lipids, (poly)saccharides, and/or polyhydroxyalkanoates, and/or its metabolism products, in particular, steroid hormones, cholesterol, prostaglandin, triacylglycerols, bile acids and/or ketone bodies molecules etc. in plants.

Furthermore preferred is a method for the recombinant production of pharmaceuticals or fine chemicals in host organisms, wherein a host organism is transformed with one of the above-described nucleic acid constructs comprising one or more structural genes which encode the desired fine chemical or catalyze the biosynthesis of the desired fine chemical, the transformed host organism is cultured, and the desired fine chemical is isolated from the culture medium. This method can be applied widely to fine chemicals such as enzymes, vitamins, amino acids, sugars, fatty acids, and natural and synthetic flavorings, aroma substances and colorants or compositions comprising these. Especially preferred is the additional production of further amino acids, tocopherols and tocotrienols and carotenoids or compositions comprising said compounds. The transformed host organisms are cultured and the products are recovered from the host organisms or the culture medium by methods known to the skilled worker or the organism itself servers as food or feed supplement. The production of pharmaceuticals such as, for example, antibodies or vaccines, is described by Hood EE, Jilka JM. Curr Opin Biotechnol. 1999 Aug; 10(4): 382-6; Ma JK, Vine ND. Curr Top Microbiol Immunol. 1999; 236:275-92.

In one embodiment, the present invention relates to a method for the identification of a gene product conferring an increase in the fine chemical production in a cell, comprising the following steps:
(a) contacting, e.g. hybridising, the nucleic acid molecules of a sample, e.g. cells, tissues, plants or microorganisms or a nucleic acid library, which can contain a candidate gene encoding a gene product conferring an increase in the fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence shown in SEQ ID NO: 1, 3, 5, 7, 9, 11, 13, 15, 17, 19, 21, 23, 25, 27, 29, 31, 33, 35, 37, 39, 41 43, 45, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, 385, 387, 389, 391or 393 and, optionally, isolating the full length cDNA clone or complete genomic clone;
(c) introducing the candidate nucleic acid molecules in host cells, preferably in a plant cell or a microorganism, appropriate for producing the fine chemical;
(d) expressing the identified nucleic acid molecules in the host cells;
(e) assaying the the fine chemical level in the host cells; and
(f) identifying the nucleic acid molecule and its gene product which expression confers an increase in the the fine chemical level in the host cell after expression compared to the wild type.

Relaxed hybridisation conditions are: After standard hybridisation procedures washing steps can be performed at low to medium stringency conditions usually with washing conditions of 40°-55°C and salt conditions between 2xSSC and 0,2x SSC with 0,1% SDS in comparison to stringent washing conditions as e.g. 60°-68°C with 0,1% SDS. Further examples can be found in the references listed above for the stringend hybridization conditions. Usually washing steps are repeated with increasing stringency and length until a useful signal to noise ratio is detected and depend on many factors as the target, e.g. its purity, GC-content, size etc, the probe, e.g.its length, is it a RNA or a DNA probe, salt conditions, washing or hybridisation temperature, washing or hybridisation time etc.

In another embodiment, the present invention relates to a method for the identification of a gene product conferring an increase in the fine chemical production in a cell, comprising the following steps:
(a) identifiying nucleic acid molecules of an organism; which can contain a candidate gene encoding a gene product conferring an increase in the fine chemical after expression, which are at least 20%, preferably 25%, more preferably 30%, even more preferred are 35%. 40% or 50%, even more preferred are 60%, 70% or 80%, most preferred are 90% or 95% or more homology to the nucleic acid molecule of the present invention, for example via homology search in a data bank;
(b) introducing the candidate nucleic acid molecules in host cells, preferably in a plant cells or microorganisms, appropriate for producing the fine chemical;
(c) expressing the identified nucleic acid molecules in the host cells;
(d) assaying the the fine chemcial level in the host cells; and
(e) identifying the nucleic acid molecule and its gene product which expression confers an increase in the the fine chemical level in the host cell after expression compared to the wild type.

The nucleic acid molecules identified can then be used for the production of the fine chemical in the same way as the nucleic acid molecule of the present invention. Accordingly, in one embodiment, the present invention relates to a process for the production of the fine chemical, comprising (a) identifying a nucleic acid molecule according to aforementioned steps (a) to (f) or (a) to (e) and recovering the free or bound fine chemical from a organism having an increased cellular activity of a polypeptide encoded by the isolated nucleic acid molecule compared to a wild type.

Furthermore, in one embodiment, the present invention relates to a method for the identification of a compound stimulating production of the fine chemical to said plant comprising:
a) contacting cells which express the polypeptide of the present invention or its mRNA with a candidate compound under cell cultivation conditions;
b) assaying an increase in expression of said polypeptide or said mRNA;
c) comparing the expression level to a standard response made in the absence of said candidate compound; whereby, an increased expression over the standard indicates that the compound is stimulating production of the fine chemical.

Furthermore, in one embodiment, the present invention relates to a method for the screening for agonists or an antagonist of the activity of the polypeptide of the present invention or used in the process of the present invention, e.g. a polypeptide conferring an increase of the fine chemical in an organism or a part therof after increasing the activity in an organism or a part thereof, comprising:
(a) contacting cells, tissues , plants or microorganisms which express the polypeptide according to the invention with a candidate compound or a sample comprising a plurality of compounds under conditions which permit the expression the polypeptide of the present invention or used in the process of the present invention;
(b) assaying the fine chemical level or the polypeptide expression level in the cell, tissue, plant or microorganism or the media the cell, tissue, plant or microorganisms is cultured or maintained in; and
(c) identifying a agonist or antagonist by comparing the measured the fine chemical level or polypeptide of the invention or used in the invention expression level with a standard the fine chemical or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.

Furthermore, in one embodiment, the present invention relates to process for the identification of a compound conferring increased the fine chemical production in a plant or microorganism, comprising the steps:
(a) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(b) identifying if the compound is an effective agonist by detecting the presence or absence or increase of a signal produced by said readout system.

The screen for a gene product or an agonist conferring an increase in the fine chemical production can be performed by growth of an organism for example a microorganism in the presence of growth reducing amounts of an inhibitor of the synthesis of the fine chemical. Better growth, eg higher dividing rate or high dry mass in comparison to the control under such conditions would identify a gene or gene product or an agonist conferring an increase in fine chemical production.

One can think to screen for increased fine chemical production by for example resistance to a drug blocking the fine chemical synthesis and looking whether this effect is dependent on the protein of the invention e.g. comparing near identical organisms with low and high biological acitivity of the protein of the invention.

Said compound may be chemically synthesized or microbiologically produced and/or comprised in, for example, samples, e.g., cell extracts from, e.g., plants, animals or microorganisms, e.g. pathogens. Furthermore, said compound(s) may be known in the art but hitherto not known to be capable of suppressing or activating the polypeptide of the present invention. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994), in particular Chapter 17. The compounds may be, e.g., added to the reaction mixture, culture medium, injected into the cell or sprayed onto the plant.

If a sample containing a compound is identified in the method of the invention, then it is either possible to isolate the compound from the original sample identified as containing the compound capable of activating or increasing the content of the fine chemical in an organism or part thereof, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. Preferably, the compound identified according to the above-described method or its derivative is further formulated in a form suitable for the application in plant breeding or plant cell and tissue culture.

The compounds which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Said compounds can also be functional derivatives or analogues of known inhibitors or activators. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art. Furthermore, peptidomimetics and/or computer aided design of appropriate derivatives and analogues can be used, for example, according to the methods described above. The cell or tissue that may be employed in the method of the invention preferably is a host cell, plant cell or plant tissue of the invention described in the embodiments hereinbefore.

Thus, in a further embodiment the invention relates to a compound obtained or identified according to the method for identifiying an agonist of the invention said compound being an agonist of the polypeptide of the present invention or used in the process of the present invention.

Accordingly, in one embodiment, the present invention further relates to a compound identified by the method for identifying a compound of the present invention.

Said compound is, for example, a homologous of the polypeptide of the present invention. Homologues of the polypeptid of the present invention can be generated by mutagenesis, e.g., discrete point mutation or truncation of the polypeptide of the present invenion. As used herein, the term "homologue" refers to a variant form of the protein, which acts as an agonist of the activity of the polypeptide of the present invention. An agonist of said protein can retain substantially the same, or a subset, of the biological activities of the polypeptide of the present invention. In particular, said agonist confers the increase of the expression level of the polypeptide of the present invention and/or the expression of said agonist in an organisms or part thereof confers the increase of free and/or bound the fine chemical in the organism or part thereof.

In one embodiment, the invention relates to an antibody specifically recognizing the compound or agonist of the present invention.

The invention also relates to a diagnostic composition comprising at least one of the aforementioned nucleic acid molecules, vectors, proteins, antibodies or compounds of the invention and optionally suitable means for detection.

The diagnostic composition of the present invention is suitable for the isolation of mRNA from a cell and contacting the mRNA so obtained with a probe comprising a nucleic acid probe as described above under hybridizing conditions, detecting the presence of mRNA hybridized to the probe, and thereby detecting the expression of the protein in the cell. Further methods of detecting the presence of a protein according to the present invention comprise immunotechniques well known in the art, for example enzyme linked immunosorbent assay. Furthermore, it is possible to use the nucleic acid molecules according to the invention as molecular markers or primer in plant breeding. Suitable means for detection are well known to a person skilled in the art, e.g. buffers and solutions for hydridization assays, e.g. the aforementioned solutions and buffers, further and means for Southern-, Westem-, Northern- etc. -blots, as e.g. described in Sambrook et al. are known.

In another embodiment, the present invention relates to a kit comprising the nucleic acid molecule, the vector, the host cell, the polypeptide, the antisense nucleic acid, the antibody, plant cell, the plant or plant tissue, the harvestable part, the propagation material and/or the compound or agonist or antagonists identified according to the method of the invention.

The compounds of the kit of the present invention may be packaged in containers such as vials, optionally with/in buffers and/or solution. If appropriate, one or more of said components might be packaged in one and the same container. Additionally or alternatively, one or more of said components might be adsorbed to a solid support as, e.g. a nitrocellulose filter, a glas plate, a chip, or a nylon membrane or to the well of a micro titerplate. The kit can be used for any of the herein described methods and embodiments, e.g. for the production of the host cells, transgenic plants, pharmaceutical compositions, detection of homologous sequences, identification of antagonists or agonists, as food or feed or as a supplement thereof, as supplement for the treating of plants, etc.

Further, the kit can comprise instructions for the use of the kit for any of said embodiments, in particular for the use for producing organisms or part thereof having an increased free or bound the fine chemcial content.

In one embodiment said kit comprises further a nucleic acid molecule encoding one or more of the aforementioned protein, and/or an antibody, a vector, a host cell, an antisense nucleic acid, a plant cell or plant tissue or a plant.

In a further embodiment, the present invention relates to a method for the production of a agricultural composition providing the nucleic acid molecule, the vector or the polypeptide of the invention or comprising the steps of the method according to the invention for the identification of said compound, agonist or antagonist; and formulating the nucleic acid molecule, the vector or the polypeptide of the invention or the agonist, or compound identified according to the methods or processes of the present invention or with use of the subject matters of the present invention in a form applicable as plant agricultural composition.

In another embodiment, the present invention relates to a method for the production of a "the fine chemical"-production supporting plant culture composition comprising the steps of the method for of the present invention; and formulating the compound identified in a form acceptable as agricultural composition.

Under "acceptable as agricultural composition" is understood, that such a composition is in agreement with the laws regulating the content of fungicides, plant nutrients, herbizides, etc. Preferably such a composition is without any harm for the protected plants and the animals (humans included) fed therewith.

The present invention also pertains to several embodiments relating to further uses and methods. The nucleic acid molecule, polypeptide, protein homologues, fusion proteins, primers, vectors, host cells, described herein can be used in one or more of the following methods: identification of plants useful for fine chemical production as mentioned and related organisms; mapping of genomes; identification and localization of sequences of interest; evolutionary studies; determination of regions required for function; modulation of an activity.

The nucleic acid molecule of the invention, the vector of the invention or the nucleic acid construct of the invention may also be useful for the production of organisms resistant to inhibitors of the fine chemical e.g. the amino acid production biosynthesis pathways. In particular, the overexpression of the polypeptide of the present invention may protect plants against herbicides, which block the amino acid, in particular the fine chemical, synthesis in said plant. Examples of herbicides blocking the fine chemical synthesis e.g. the amino acid synthesis in plants are for example sulfonylurea and imidazolinone herbicides which catalyze the first step in branched-chain amino acid biosynthesis

Accordingly, the nucleic acid molecules of the present invention have a variety of uses. First, they may be used to identify an organism or a close relative thereof. Also, they may be used to identify the presence thereof or a relative thereof in a mixed population of microorganisms or plants. By probing the extracted genomic DNA of a culture of a unique or mixed population of plants under stringent conditions with a probe spanning a region of the gene of the present invention which is unique to this, one can ascertain whether the present invention has been used or whether it or a close relative is present.

Further, the nucleic acid molecule of the invention may be sufficiently homologous to the sequences of related species such that these nucleic acid molecules may serve as markers for the construction of a genomic map in related organism.

Accordingly, the present invention relates to a method for breeding plants for the production of the fine chemical, comprising
(a) providing a first plant variety produced according to the process of the invention preferably (over)expressing the nucleic acid molecule of the invention;
(b) crossing the first plant variety with a second plant variety; and
(c) selecting the offspring plants which overproduce the fine chemical by means of analysis the distribution of a molecular marker in the offspring representing the first plant variety and its capability to (over)produce the fine chemical.

Details about the use of molecular markers in breeding can be found in Kumar et al., 1999 (Biotech Adv., 17:143-182) and Peleman and van der Voort 2003 (Trends Plant Sci. 2003 Jul;8(7):330-334). The molecular marker can e.g. relate to the nucleic acid molecule of the invention and/or its expression level. Accordingly, the molecular marker can be a probe or a PCR primer set useful for identification of the genomic existence or genomic localisation of the nucleic acid molecule of the invention, e.g. in a Southern blot analysis or a PCR or its expression level, i.g. in a Northern Blot analysis or a quantitative PCR. Accordingly, in one embodiment, the present invention relates to the use of the nucleic acid molecule of the present invention or encoding the polypeptide of the present invention as molecular marker for breeding.

The nucleic acid molecules of the invention are also useful for evolutionary and protein structural studies. By comparing the sequences of the invention or used in the process of the invention to those encoding similar enzymes from other organisms, the evolutionary relatedness of the organisms can be assessed. Similarly, such a comparison permits an assessment of which regions of the sequence are conserved and which are not, which may aid in determining those regions of the protein which are essential for the functioning of the enzyme. This type of determination is of value for protein engineering studies and may give an indication of what the protein can tolerate in terms of mutagenesis without losing function.

Accordingly, the nucleic acid molecule of the invention can be used for the identification of other nucleic acids conferring an increase of the fine chemical after expression.

Further, the nucleic acid molecule of the invention or a fragment of a gene conferring the expression of the polypeptide of the invention, preferably comprising the nucleic acid molecule of the invention, can be used for marker assisted breeding or association mapping of the fine chemical derived traits

Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the fine chemical or of the fine chemical and one or more other ingredients such as amino acids, in particular Threoinine, Alanine, Glutamin, Glutamic acid, Valine, Aspargine, Phenylalanine, Leucine, Proline , Tryptophan Tyrosine, Valine, Isoleucine and Arginine.
Accordingly, the nucleic acid of the invention, or the nucleic acid molecule identified with the method of the present invention or the complement sequences thereof, the polypeptide of the invention, the nucleic acid construct of the invention, the organisms, the host cell, the microorgansims, the plant, plant tissue, plant cell, or the part thereof of the invention, the vector of the invention, the antagonist identified with the method of the invention, the antibody of the present invention, the antisense molecule of the present invention, can be used for the reduction of the fine chemical in a organism or part thereof, e.g. in a cell.

Further, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the antagonist or the agonist identified with the method of the invention, the antibody of the presen invention, the antisense molecule of the present invention or the nucleic acid molecule identified with the method of the present invention, can be used for the preparation of an agricultural composition.

Furthermore, the nucleic acid of the invention, the polypeptide of the invention, the nucleic acid construct of the invention, the organisms, the host cell, the microorgansims, the plant, plant tissue, plant cell, or the part thereof of the invention, the vector of the invention, antagonist or the agonist identified with the method of the invention, the antibody of the presen invention, the antisense molecule of the present invention or the nucleic acid molecule identified with the method of the present invention, can be used for the identification and production of compounds capable of conferring a modulation of the fine chemical levels in an organism or parts thereof, preferably to identify and produce compounds conferring an increase of the fine chemical levels in an organism or parts thereof, if said identified compound is applied to the organism or part thereof, i.e. as part of its food, or in the growing or culture media.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under hftp://www.ncbi.nlm.nih.gov/PubMed/medline.html. Further databases and addresses, such as hftp://www.ncbi.nlm.nih.gov/, hftp://www.infobiogen. fr/, hftp://www.fmi.ch/biology/research-tools.html, hftp://www.tigr.org/, are known to the person skilled in the art and can also be obtained using, e.g., hftp://www.lycos.com. An overview of patent information in biotechnology and a survey of relevant sources of patent information useful for retrospective searching and for current awareness are given in Berks, TIBTECH 12 (1994), 352-364. Table 1 gives an overview about the fine chemicals produced by SEQ ID NO: 1 of the present invention.

**Table 1: Fine chemicals produced with SEQ ID NO: 1 (YNL090W)**

| **Metabolite** | **Method** | **Min** | **Max** |
|---|---|---|---|
| Tryptophane | LC | 1,26 | 4,39 |
| Proline | LC | 1,73 | 6,29 |
| Arginine | LC | 1,54 | 4,23 |
| Raffinose | LC | 2,57 | 16,05 |
| Ferulic Acid | LC | 1,40 | 2,41 |
| Phenylalanine | LC | 1,34 | 2,75 |
| Tyrosine | LC | 1,44 | 2,55 |
| gamma+beta-Tocopherol | LC | 0,36 | 0,66 |
| Cerotic Acid (C26:0) | GC | 1,41 | 3,78 |
| Lignoceric Acid (C24:0) | GC | 1,34 | 2,14 |
| Alanine | GC | 1,21 | 1,57 |
| Glycine | GC | 1,46 | 1,67 |
| Threonine | GC | 1,21 | 2,02 |
| Putrescine | GC | 0,18 | 0,42 |
| Serine | GC | 1,29 | 1,96 |
| Valine | GC | 1,20 | 2,31 |
| Isoleucine | GC | 1,30 | 4,35 |
| Leucine | GC | 1,51 | 5,01 |
| Sinapic Acid | GC | 1,28 | 2,08 |
| 3,4-Dihydroxyphenylalanine (=DOPA) | GC | 1,37 | 1,87 |
| Stearic Acid (C18:0) | GC | 1,16 | 2,13 |
| beta-Carotene | LC | 1,77 | 2,68 |

Column 1 shows the metabolite produced. Column 2 mirrors the analytic method and column 3 and 4 shows the minimum and maximum production of the respective fine chemical as x-fold.

The present invention is illustrated by the examples, which follow. The present examples illustrate the basic invention without being intended as limiting the subject of the invention. The content of all of the references, patent applications, patents and published patent applications cited in the present patent application is herewith incorporated by reference.

Examples

Example 1: Cloning SEQ ID NO: 1 in Escherichia coli

SEQ ID NO: 1 was cloned into the plasmids pBR322 (Sutcliffe, J.G. (1979) Proc. Natl Acad. Sci. USA, 75: 3737-3741); pACYC177 (Change & Cohen (1978) J. Bacteriol. 134: 1141-1156); plasmids of the pBS series (pBSSK+, pBSSK-and others; Stratagene, LaJolla, USA) or cosmids such as SuperCos1 (Stratagene, LaJolla, USA) or Lorist6 (Gibson, T.J. Rosenthal, A., and Waterson, R.H. (1987) Gene 53: 283-286) for expression in E. coli using known, well-established procedures (see, for example, Sambrook, J. et al. (1989) "Molecular Cloning: A Laboratory Manual". Cold Spring Harbor Laboratory Press or Ausubel, F.M. et al. (1994) "Current Protocols in Molecular Biology", John Wiley & Sons).

Example 2: DNA sequencing and computerized functional analysis [0448.0.0.0] The DNA was sequenced by standard procedures, in particular the chain determination method, using ABI377 sequencers (see, for example, Fleischman, R.D. et al. (1995) "Whole-genome Random Sequencing and Assembly of Haemophilus Influenzae Rd., Science 269; 496-512)".

Example 3: In-vivo mutagenesis

An *in vivo* mutagenesis of Corynebacterium glutamicum for the production of amino acids can be carried out by passing a plasmid DNA (or another vector DNA) through E. coli and other microorganisms (for example Bacillus spp. or yeasts such as Saccharomyces cerevisiae), which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.

Example 4: DNA transfer between Escherichia coli and Corynebacterium glutamicum

Several Corynebacterium and Brevibacterium species comprise endogenous plasmids (such as, for example, pHM1519 or pBL1), which replicate autonomously (for a review, see, for example, Martin, J.F. et al. (1987) Biotechnology 5: 137-146). Shuttle vectors for Escherichia coli and Corynebacterium glutamicum can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons), which have a replication origin for, and suitable marker from, Corynebacterium glutamicum added. Such replication origins are preferably taken from endogenous plasmids, which have been isolated from Corynebacterium and Brevibacterium species. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology, VCH, Weinheim). There are many examples in the literature of the preparation of a large multiplicity of shuttle vectors which are replicated in E. coli and C. glutamicum and which can be used for various purposes including the overexpression of genes (see, for example, Yoshihama, M. et al. (1985) J. Bacteriol. 162: 591-597, Martin, J.F. et al., (1987) Biotechnology, 5: 137-146 and Eikmanns, B.J. et al. (1992) Gene 102: 93-98). Suitable vectors which replicate in coryneform bacteria are for example, pZ1 (Menkel et al., Appl. Environ. Microbiol., 64, 1989: 549 - 554) pEkEx1 (Eikmanns et al., Gene 102, 1991: 93 - 98) or pHS2-1 (Sonnen et al, Gene 107, 1991: 69 - 74). These vectors are based on the cryptic plasmids pHM1519, pBL1 or pGA1. Other plasmid vectors such as, for example, those based on pCG4 (US 4,489,160), pNG2 (Serwold-Davis et al., FEMS Microbiol. Lett., 66, 1990: 119 - 124) or pAG1 (US 5,158,891) can be used in the same manner.

Using standard methods, it is possible to clone a gene of interest into one of the above-described shuttle vectors and to introduce such hybrid vectors into Corynebacterium glutamicum strains. The transformation of C. glutamicum can be achieved by protoplast transformation (Kastsumata, R. et al., (1984) J. Bacteriol. 159, 306-311), electroporation (Liebl, E. et al., (1989) FEMS Microbiol. Letters, 53: 399-303) and in those cases where specific vectors are used also by conjugation (such as, for example, described in Schäfer, A., et al. (1990) J. Bacteriol. 172: 1663-1666). Likewise, it is possible to transfer the shuttle vectors for C. glutamicum to E. coli by preparing plasmid DNA from C. glutamicum (using standard methods known in the art) and transforming it into E. coli. This transformation step can be carried out using standard methods, but preferably using a Mcr-deficient E. coli strain, such as NM522 (Gough & Murray (1983) J. Mol. Biol. 166: 1-19).

If the transformed sequence(s) is/are to be integrated advantageously into the genome of the coryneform bacteria, standard techniques known to the skilled worker also exist for this purpose. Examples, which are used for this purpose are plasmid vectors as they have been described by Remscheid et al. (Appl. Environ. Microbiol., 60, 1994: 126-132) for the duplication and amplification of the hom-thrB operon. In this method, the complete gene is cloned into a plasmid vector, which is capable of replication in a host such as E. coli, but not in C. glutamicum. Suitable vectors are, for example, pSUP301 (Simon et al., Bio/Technology 1, 1983: 784-791), pKIBmob or pK19mob (Schäfer et al., Gene 145, 1994: 69-73), pGEM-T (Promega Corp., Madison, WI, USA), pCR2.1-TOPO (Schuman, J. Biol. Chem., 269, 1994: 32678-32684, US 5,487,993), pCR^{®}Blunt (Invitrogen, Groningen, the Netherlands) or pEM1 (Schrumpf et al., J. Bacteriol., 173, 1991: 4510-4516).

Example 5: Determining the expression of the mutant/transgenic protein

The observations of the acivity of a mutated, or transgenic, protein in a transformed host cell are based on the fact that the protein is expressed in a similar manner and in a similar quantity as the wild-type protein. A suitable method for determining the transcription quantity of the mutant, or transgenic, gene (a sign for the amount of mRNA which is available for the translation of the gene product) is to carry out a Northern blot (see, for example, Ausubel et al., (1988) Current Protocols in Molecular Biology, Wiley: New York), where a primer which is designed in such a way that it binds to the gene of interest is provided with a detectable marker (usually a radioactive or chemiluminescent marker) so that, when the total RNA of a culture of the organism is extracted, separated on a gel, applied to a stable matrix and incubated with this probe, the binding and quantity of the binding of the probe indicates the presence and also the amount of mRNA for this gene. Another method is a quantitative PCR. This information detects the extent to which the gene has been transcribed. Total cell RNA can be isolated from Corynebacterium glutamicum by a variety of methods, which are known in the art, as described in Bormann, E.R. et al., (1992) Mol. Microbial. 6: 317-326.

Standard techniques, such as Western blot, may be employed to determine the presence or relative amount of protein translated from this mRNA (see, for example, Ausubel et al. (1988) "Current Protocols in Molecular Biology", Wiley, New York). In this method, total cell proteins are extracted, separated by gel electrophoresis, transferred to a matrix such as nitrocellulose and incubated with a probe, such as an antibody, which binds specifically to the desired protein. This probe is usually provided directly or indirectly with a chemiluminescent or colorimetric marker, which can be detected readily. The presence and the observed amount of marker indicate the presence and the amount of the sought mutant protein in the cell. However, other methods are also known.

Example 6: Growth of genetically modified Corynebacterium glutamicum: media and culture conditions

Genetically modified Corynebacteria are grown in synthetic or natural growth media. A number of different growth media for Corynebacteria are known and widely available (Lieb et al. (1989) Appl. Microbiol. Biotechnol. 32: 205-210; von der Osten et al. (1998) Biotechnology Letters 11: 11-16; Patent DE 4 120 867; Liebl (1992) "The Genus Corynebacterium", in: The Procaryotes, Vol. II, Balows, A., et al., Ed. Springer-Verlag).

Said media which can be used according to the invention usually consist of one or more carbon sources, nitrogen sources, inorganic salts, vitamins and trace elements. Preferred carbon sources are sugars such as mono-, di- or polysaccharides. Examples of very good carbon sources are glucose, fructose, mannose, galactose, ribose, sorbose, ribulose, lactose, maltose, sucrose, raffinose, starch or cellulose. Sugars may also be added to the media via complex compounds such as molasses or other by-products of sugar refining. It may also be advantageous to add mixtures of various carbon sources. Other possible carbon sources are alcohols and/or organic acids such as methanol, ethanol, acetic acid or lactic acid. Nitrogen sources are usually organic or inorganic nitrogen compounds or materials containing said compounds. Examples of nitrogen sources include ammonia gas, aqueous ammonia solutions or ammonium salts such as NH₄Cl, or (NH₄)₂SO₄, NH₄OH, nitrates, urea, amino acids or complex nitrogen sources such as comsteep liquor, soybean flour, soybean protein, yeast extract, meat extract and others. Mixtures of the above nitrogen sources may be used advantageously.

Inorganic salt compounds, which may be included in the media comprise the chloride, phosphorus or sulfate salts of calcium, magnesium, sodium, cobalt, molybdenum, potassium, manganese, zinc, copper and iron. Chelating agents may be added to the medium in order to keep the metal ions in solution. Particularly suitable chelating agents include dihydroxyphenols such as catechol or protocatechulate or organic acids such as citric acid. The media usually also contain other growth factors such as vitamins or growth promoters, which include, for example, biotin, riboflavin, thiamine, folic acid, nicotinic acid, panthothenate and pyridoxine. Growth factors and salts are frequently derived from complex media components such as yeast extract, molasses, comsteep liquor and the like. The exact composition of the compounds used in the media depends heavily on the particular experiment and is decided upon individually for each specific case. Information on the optimization of media can be found in the textbook "Applied Microbiol. Physiology, A Practical Approach" (Ed. P.M. Rhodes, P.F. Stanbury, IRL Press (1997) S. 53-73, ISBN 0 19 963577 3). Growth media can also be obtained from commercial suppliers, for example Standard 1 (Merck) or BHI (Brain heart infusion, DIFCO) and the like.

All media components are sterilized, either by heat (20 min at 1.5 bar und 121°C) or by filter sterilization. The components may be sterilized either together or, if required, separately. All media components may be present at the start of the cultivation or added continuously or batchwise, as desired.

The culture conditions are defined separately for each experiment. The temperature is normally between 15°C and 45°C and may be kept constant or may be altered during the experiment. The pH of the medium should be in the range from 5 to 8.5, preferably around 7.0, and can be maintained by adding buffers to the media. An example of a buffer for this purpose is a potassium phosphate buffer. Synthetic buffers such as MOPS, HEPES, ACES and the like may be used as an alternative or simultaneously. The culture pH value may also be kept constant during the culture period by addition of, for example, NaOH or NH₄OH. If complex media components such as yeast extract are used, additional buffers are required less since many complex compounds have a high buffer capacity. When using a fermenter for the culture of microorganisms, the pH value can also be regulated using gaseous ammonia.

The incubation period is generally in a range of from several hours to several days. This time period is selected in such a way that the maximum amount of product accumulates in the fermentation broth. The growth experiments, which are disclosed can be carried out in a multiplicity of containers such as microtiter plates, glass tubes, glass flasks or glass or metal fermenters of various sizes. To screen a large number of clones, the microorganisms should be grown in microtiter plates, glass tubes or shake flasks, either using simple flasks or baffle flasks. 100 ml shake flasks filled with 10% (based on the volume) of the growth medium required are preferably used. The flasks should be shaken on an orbital shaker (amplitude 25 mm) at a rate ranging from 100 to 300 rpm. Evaporation losses can be reduced by maintaining a humid atmosphere; as an alternative, a mathematical correction should be carried out for the evaporation losses.

If genetically modified clones are examined, an unmodified control clone, or a control clone, which contains the basic plasmid without insertion, should also be included in the tests. If a transgenic sequence is expressed, a control clone should advantageously again be included in these tests. The medium is advantageously inoculated to an OD600 of 0.5 to 1.5 using cells which have been grown on agar plates, such as CM plates (10 g/l glucose, 2.5 g/l NaCl, 2 g/l urea, 10 g/l polypeptone, 5 g/l yeast extract, 5 g/l meat extract, 22 g/l agar, pH value 6.8 established with 2M NaOH), which have been incubated at 30°C. The media are inoculated either by introducing a saline solution of C. glutamicum cells from CM plates or by addition of a liquid preculture of this bacterium.

Example 7: In-vitro analysis of the function of the proteins encoded by the transformed sequences

The determination of the activities and kinetic parameters of enzymes is well known in the art. Experiments for determining the activity of a specific modified enzyme must be adapted to the specific activity of the wild-enzyme type, which is well within the capabilities of the skilled worker. Overviews of enzymes in general and specific details regarding the structure, kinetics, principles, methods, applications and examples for the determination of many enzyme activities can be found for example in the following literature: Dixon, M., and Webb, E.C: (1979) Enzymes, Longmans, London; Fersht (1985) Enzyme Structure and Mechanism, Freeman, New York; Walsh (1979) Enzymatic Reaction Mechanisms. Freeman, San Francisco; Price, N.C., Stevens, L. (1982) Fundamentals of Enzymology. Oxford Univ. Press: Oxford; Boyer, P.D: Ed. (1983) The Enzymes, 3rd Ed. Academic Press, New York; Bisswanger, H. (1994) Enzymkinetik, 2nd Ed. VCH, Weinheim (ISBN 3527300325); Bergmeyer, H.U., Bergmeyer, J., Graßl, M. Ed. (1983-1986) Methods of Enzymatic Analysis, 3rd Ed. Vol. I-XII, Verlag Chemie: Weinheim; and Ullmann's Encyclopedia of Industrial Chemistry (1987) Vol. A9, "Enzymes", VCH, Weinheim, pp. 352-363.

Example 8: Analysis of the effect of the nucleic acid molecule on the production of the amino acids

The effect of the genetic modification in C. glutamicum on the production of an amino acid can be determined by growing the modified microorganisms under suitable conditions (such as those described above) and analyzing the medium and/or the cellular components for the increased production of the amino acid. Such analytical techniques are well known to the skilled worker and encompass spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, pp. 89-90 and pp. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", pp. 469-714, VCH: Weinheim; Belter, P.A. et al. (1988) Bioseparations: downstream processing for Biotechnology, John Wiley and Sons; Kennedy, J.F. and Cabral, J.M.S. (1992) Recovery processes for biological Materials, John Wiley and Sons; Shaeiwitz, J.A. and Henry, J.D. (1988) Biochemical Separations, in Ullmann's Encyclopedia of Industrial Chemistry, Vol. B3; chapter 11, pp. 1-27, VCH: Weinheim; and Dechow, F.J. (1989) Separation and purification techniques in biotechnology, Noyes Publications).

In addition to the determination of the fermentation end product, other components of the metabolic pathways which are used for the production of the desired compound, such as intermediates and by-products, may also be analyzed in order to determine the total productivity of the organism, the yield and/or production efficiency of the compound. The analytical methods encompass determining the amounts of nutrients in the medium (for example sugars, hydrocarbons, nitrogen sources, phosphate and other ions), determining biomass composition and growth, analyzing the production of ordinary metabolites from biosynthetic pathways and measuring gases generated during the fermentation. Standard methods for these are described in Applied Microbial Physiology; A Practical Approach, P.M. Rhodes and P.F. Stanbury, Ed. IRL Press, pp. 103-129; 131-163 and 165-192 (ISBN: 0199635773) and the references cited therein.

Example 9: Purification of the amino acid

The amino acid can be recovered from cells or from the supernatant of the above-described culture by a variety of methods known in the art. For example, the culture supernatant is recovered first. To this end, the cells are harvested from the culture by slow centrifugation. Cells can generally be disrupted or lysed by standard techniques such as mechanical force or sonication. The cell debris is removed by centrifugation and the supernatant fraction, if appropriate together with the culture supernatant, is used for the further purification of the amino acid. However, it is also possible to process the supernatant alone if the amino acid is present in the supernatant in sufficiently high a concentration. In this case, the amino acid, or the amino acid mixture, can be purified further for example via extraction and/or salt precipitation or via ion-exchange chromatography.

If required and desired, further chromatography steps with a suitable resin may follow, the amino acid, but not many contaminants in the sample, being retained on the chromatography resin or the contaminants, but not the sample with the product (amino acid), being retained on the resin. If necessary, these chromatography steps may be repeated, using identical or other chromatography resins. The skilled worker is familiar with the selection of suitable chromatography resin and the most effective use for a particular molecule to be purified. The purified product can be concentrated by filtration or ultrafiltration and stored at a temperature at which maximum product stability is ensured. Many purification methods, which are not limited to the above purification method are known in the art. They are described, for example, in Bailey, J.E. & Ollis, D.F. Biochemical Engineering Fundamentals, McGraw-Hill: New York (1986).

Identity and purity of the amino acid isolated can be determined by standard techniques of the art. They encompass high-performance liquid chromatography (HPLC), spectroscopic methods, mass spectrometry (MS), staining methods, thin-layer chromatography, NIRS, enzyme assay or microbiological assays. These analytical methods are compiled in: Patek et al. (1994) Appl. Environ. Microbiol. 60: 133-140; Malakhova et al. (1996) Biotekhnologiya 11: 27-32; and Schmidt et al. (1998) Bioprocess Engineer. 19: 67-70. Ulmann's Encyclopedia of Industrial Chemistry (1996) Vol. A27, VCH: Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Vol. 17.

Example 10: Cloning SEQ ID NO: 1 for the expression in plants

Unless otherwise specified, standard methods as described in Sambrook et al., Molecular Cloning: A laboratory manual, Cold Spring Harbor 1989, Cold Spring Harbor Laboratory Press are used.

SEQ ID NO: 1 is amplified by PCR as described in the protocol of the Pfu Turbo or DNA Herculase polymerase (Stratagene).

The composition for the protocol of the *Pfu* Turbo DNA polymerase was as follows: 1x PCR buffer (Stratagene), 0.2 mM of each dNTP, 100 ng genomic DNA of *Saccharomyces cerevisiae* (strain S288C; Research Genetics, Inc., now Invitrogen) or *Escherichia coli* (strain MG1655; *E.coli* Genetic Stock Center), 50 pmol forward primer, 50 pmol reverse primer, 2.5 u *Pfu* Turbo DNA polymerase. The amplification cycles were as follows:
1 cycle of 3 minutes at 94-95°C, followed by 25-36 cycles of in each case 1 minute at 95°C or 30 seconds at 94°C, 45 seconds at 50°C, 30 seconds at 50°C or 30 seconds at 55°C and 210-480 seconds at 72°C, followed by 1 cycle of 8 minutes at 72°C, then 4°C.

The composition for the protocol of the Herculase polymerase was as follows: 1x PCR buffer (Stratagene), 0.2 mM of each dNTP, 100 ng genomic DNA of *Saccharomyces cerevisiae* (strain S288C; Research Genetics, Inc., now Invitrogen) or *Escherichia coli* (strain MG1655; *E.coli* Genetic Stock Center), 50 pmol forward primer, 50 pmol reverse primer, 2.5 u Herculase polymerase. The amplification cycles were as follows:

1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

The following primer sequences were selected for the gene

SEQ ID NO: 1:
i) forward primer (SEQ ID NO: 53)
   5'-ATGTCTGAAAAGGCCGTTAGAAGG-3'
ii) reverse primer (SEQ ID NO: 54)
   5'-TTATAAAATTATGCAACAGTTAGCCC-3'

Thereafter, the amplificate was purified over QIAquick columns following the standard protocol (Qiagen).

For the cloning of PCR-products, produced by *Pfu* Turbo DNA polymerase, the vector DNA (30 ng) was restricted with *Sma*l following the standard protocol (MBI Fermentas)and stopped by addition of high-salt buffer. The restricted vector fragments were purified via Nucleobond columns using the standard protocol (Macherey-Nagel). Thereafter, the linearized vector was dephosphorylated following the standard protocol (MBI Fermentas).

The PCR-products, produced by *Pfu* Turbo DNA polymerase, were directly cloned into the processed binary vector.

The DNA termini of the PCR-products, produced by Herculase DNA polymerase, were blunted in a second synthesis reaction using *Pfu* Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were cloned into the processed vector as well.

A binary vector comprising a selection cassette (promoter, selection marker, terminator) and an expression cassette with promoter, cloning cassette and terminator sequence between the T-DNA border sequences was used. In addition to those within the cloning cassette, the binary vector has no *Sma*l cleavage site. Binary vectors which can be used are known to the skilled worker, an overview of binary vectors and their use can be found in Hellens, R., Mullineaux, P. and Klee H., [(2000) "A guide to Agrobacterium binary vectors", Trends in Plant Science, Vol. 5 No.10, 446-451. Depending on the vector used, cloning may advantageously also be carried out via other restriction enzymes. Suitable advantageous cleavage sites can be added to the ORF by using suitable primers for the PCR amplification.

Approximately 30 ng of prepared vector and a defined amount of prepared amplificate were mixed and ligated by addition of ligase.

The ligated vectors were transformed in the same reaction vessel by addition of competent E. *coli* cells (strain DH5alpha) and incubation for 20 minutes at 1°C followed by a heat shock for 90 seconds at 42°C and cooling to 4°C. Then, complete medium (SOC) was added and the mixture was incubated for 45 minutes at 37°C. The entire mixture was subsequently plated onto an agar plate with antibiotics (selected as a function of the binary vector used) and incubated overnight at 37°C.

The outcome of the cloning step was verified by amplification with the aid of primers which bind upstream and downstream of the integration site, thus allowing the amplification of the insertion. In addition combinations of the above mentioned gene specific primers and upstream and downstream primers were used in PCR reactions to identify clones with the correct insert orientation. The amplifications were carried as described in the protocol of *Taq* DNA polymerase (Gibco-BRL).

The amplification cycles were as follows: 1 cycle of 5 minutes at 94°C, followed by 35 cycles of in each case 15 seconds at 94°C, 15 seconds at 50-66°C and 5 minutes at 72°C, followed by 1 cycle of 10 minutes at 72°C, then 4°C.

Several colonies were checked, but only one colony for which a PCR product of the expected size was detected was used in the following steps.

A portion of this positive colony was transferred into a reaction vessel filled with complete medium (LB) and incubated overnight at 37°C. The LB medium contained an antibiotic chosen to suit the binary vector (see above) used and the resistance gene present therein in order to select the clone.

The plasmid preparation was carried out as specified in the Qiaprep standard protocol (Qiagen).

Example 11: Generation of transgenic plants which express SEQ ID NO: 1

1 ng of the plasmid DNA isolated was transformed by electroporation into competent cells of *Agrobacterium tumefaciens, of* strain GV 3101 pMP90 (Koncz and Schell, Mol. Gen. Gent. 204, 383-396, 1986). The choice of the agrobacterial strain depends on the choice of the binary vector. An overview of possible strains and their properties is found in Hellens, R., Mullineaux, P. and Klee H., (2000) "A guide to Agrobacterium binary vectors, Trends in Plant Science, Vol. 5 No. 10, 446-451. Thereafter, complete medium (YEP) was added and the mixture was transferred into a fresh reaction vessel for 3 hours at 28°C. Thereafter, all of the reaction mixture was plated onto YEP agar plates supplemented with the respective antibiotics, for example rifampicin and gentamycin for GV3101 pMP90, and a further antibiotic for the selection onto the binary vector, was plated, and incubated for 48 hours at 28°C.

The agrobacteria generated in Example 10, which contains the plasmid construct were then used for the transformation of plants.

A colony was picked from the agar plate with the aid of a pipette tip and taken up in 3 ml of liquid TB medium, which also contained suitable antibiotics, depending on the agrobacterial strain and the binary plasmid. The preculture was grown for 48 hours at 28°C and 120 rpm.

400 ml of LB medium containing the same antibiotics as above were used for the main culture. The preculture was transferred into the main culture. It was grown for 18 hours at 28°C and 120 rpm. After centrifugation at 4 000 rpm, the pellet was resuspended in infiltration medium (MS medium, 10% sucrose).

In order to grow the plants for the transformation, dishes (Piki Saat 80, green, provided with a screen bottom, 30 x 20 x 4.5 cm, from Wiesauplast, Kunststofftechnik, Germany) were half-filled with a GS 90 substrate (standard soil, Werkverband E.V., Germany). The dishes were watered overnight with 0.05% Proplant solution (Chimac-Apriphar, Belgium). *Arabidopsis thaliana* C24 seeds (Nottingham Arabidopsis Stock Centre, UK; NASC Stock N906) were scattered over the dish, approximately 1 000 seeds per dish. The dishes were covered with a hood and placed in the stratification facility (8 h, 110 µ µmol/m²/s⁻¹, 22°C; 16 h, dark, 6°C). After 5 days, the dishes were placed into the short-day controlled environment chamber (8 h 130 µmol/m²/s⁻¹, 22°C; 16 h, dark 20°C), where they remained for approximately 10 days until the first true leaves had formed.

The seedlings were transferred into pots containing the same substrate (Teku pots, 7 cm, LC series, manufactured by Pöppelmann GmbH & Co, Germany). Five plants were pricked out into each pot. The pots were then returned into the short-day controlled environment chamber for the plant to continue growing.

After 10 days, the plants were transferred into the greenhouse cabinet (supplementary illumination, 16 h, 340 µE, 22°C; 8 h, dark, 20°C), where they were allowed to grow for further 17 days.

For the transformation, 6-week-old Arabidopsis plants which had just started flowering were immersed for 10 seconds into the above-described agrobacterial suspension which had previously been treated with 10 µl Silwett L77 (Crompton S.A., Osi Specialties, Switzerland). The method in question is described in Clough and Bent, 1998 (Clough, JC and Bent, AF. 1998 Floral dip: a simplified method for Agrobacterium-mediated transformation of Arabidopsis thaliana, Plant J. 16:735-743.

The plants were subsequently placed for 18 hours into a humid chamber. Thereafter, the pots were returned to the greenhouse for the plants to continue growing. The plants remained in the greenhouse for another 10 weeks until the seeds were ready for harvesting.

Depending on the resistance marker used for the selection of the transformed plants the harvested seeds were planted in the greenhouse and subjected to a spray selection or else first sterilized and then grown on agar plates supplemented with the respective selection agent.. In case of BASTA®-resistance, plantlets were sprayed four times at an interval of 2 to 3 days with 0.02 % BASTA® and transformed plants were allowed to set seeds. The seeds of the transgenic *A. thaliana* plants were stored in the freezer (at -20°C).

Example 12: Plant culture for bioanalytical analyses

For the bioanalytical analyses of the transgenic plants, the latter were grown uniformly a specific culture facility. To this end the GS-90 substrate as the compost mixture was introduced into the potting machine (Laible System GmbH, Singen, Germany) and filled into the pots. Thereafter, 35 pots were combined in one dish and treated with Previcur. For the treatment, 25 ml of Previcur were taken up in 10 I of tap water. This amount was sufficient for the treatment of approximately 200 pots. The pots were placed into the Previcur solution and additionally irrigated overhead with tap water without Previcur. They were used within four days..

For the sowing, the seeds, which had been stored in the refrigerator (at - 20°C), were removed from the Eppendorf tubes with the aid of a toothpick and transferred into the pots with the compost. In total, approximately 5 to 12 seeds were distributed in the middle of the pot.

After the seeds had been sown, the dishes with the pots were covered with matching plastic hood and placed into the stratification chamber for 4 days in the dark at 4°C. The humidity was approximately 90%. After the stratification, the test plants were grown for 22 to 23 days at a 16-h-light, 8-h-dark rhythm at 20°C, an atmospheric humidity of 60% and a CO₂ concentration of approximately 400 ppm. The light sources used were Powerstar HQI-T 250 W/D Daylight lamps from Osram, which generate a light resembling the solar color spectrum with a light intensity of approximately 220 µE/m2/s-1.

When the plants were 8, 9 and 10 days old, they were subjected to selection for the resistance marker Approximately 1400 pots with transgenic plants were treated with 1l 0,015% vol/vol of Basta® (Glufosinate-ammonium) solution in water (Aventis Cropsience, GermanyAfter a further 3 to 4 days, the transgenic, resistant seedlings (plantlets in the 4-leaf stage) could be distinguished clearly from the untransformed plantlets. The nontransgenic seedlings were bleached or dead. The transgenic resistance plants were thinned when they had reached the age of 14 days. The plants, which had grown best in the center of the pot were considered the target plants. All the remaining plants were removed carefully with the aid of metal tweezers and discarded.

During their growth, the plants received overhead irrigation with distilled water (onto the compost) and bottom irrigation into the placement grooves. Once the grown plants had reached the age of 23 days, they were harvested.

Example 13: Metabolic analysis of transformed plants

The modifications identified in accordance with the invention, in the content of above-described metabolites, were identified by the following procedure.

### a) sampling and storage of the samples

Sampling was performed directly in the controlled-environment chamber. The plants were cut using small laboratory scissors, rapidly weighed on laboratory scales, transferred into a pre-cooled extraction sleeve and placed into an aluminum rack cooled by liquid nitrogen. If required, the extraction sleeves can be stored in the freezer at -80°C. The time elapsing between cutting the plant to freezing it in liquid nitrogen amounted to not more than 10 to 20 seconds.

### b) Lyophilization

During the experiment, care was taken that the plants either remained in the deep-frozen state (temperatures < -40°C) or were freed from water by lyophilization until the first contact with solvents.

The aluminum rack with the plant samples in the extraction sleeves was placed into the pre-cooled (-40°C) lyophilization facility. The initial temperature during the main drying phase was-35°C and the pressure was 0.120 mbar. During the drying phase, the parameters were altered following a pressure and temperature program. The final temperature after 12 hours was +30°C and the final pressure was 0.001 to 0.004 mbar. After the vacuum pump and the refrigerating machine had been switched off, the system was flushed with air (dried via a drying tube) or argon.

### c) Extraction

Immediately after the lyophilization apparatus had been flushed, the extraction sleeves with the lyophilized plant material were transferred into the 5 ml extraction cartridges of the ASE device (Accelerated Solvent Extractor ASE 200 with Solvent Controller and AutoASE software (DIONEX)).

The 24 sample positions of an ASE device (Accelerated Solvent Extractor ASE 200 with Solvent Controller and AutoASE software (DIONEX)) were filled with plant samples, including some samples for testing quality control.

The polar substances were extracted with approximately 10 ml of methanol/water (80/20, v/v) at T = 70°C and p = 140 bar, 5 minutes heating-up phase, 1 minute static extraction. The more lipophilic substances were extracted with approximately 10 ml of methanol/dichloromethane (40/60, v/v) at T = 70°C and p = 140 bar, 5 minute heating-up phase, 1 minute static extraction. The two solvent mixtures were extracted into the same glass tubes (centrifuge tubes, 50 ml, equipped with screw cap and pierceable septum for the ASE (DIONEX)).

The solution was treated with internal standards: ribitol, L-glycine-2,2-d₂, L-alanine-2,3,3,3-d₄, methionine-methyl-d₃, and α-methylglucopyranoside and methyl nonadecanoate, methyl undecanoate, methyl tridecanoate, methyl pentadecanoate, methyl nonacosanoate.

The total extract was treated with 8 ml of water. The solid residue of the plant sample and the extraction sleeve were discarded.

The extract was shaken and then centrifuged for 5 to 10 minutes at at least 1 400 g in order to accelerate phase separation. 1 ml of the supernatant methanol/water phase ("polar phase", colorless) was removed for the further GC analysis, and 1 ml was removed for the LC analysis. The remainder of the methanol/water phase was discarded. 0.5 ml of the organic phase ("lipid phase", dark green) was removed for the further GC analysis and 0.5 ml was removed for the LC analysis. All the portions removed were evaporated to dryness using the IR Dancer infrared vacuum evaporator (Hettich). The maximum temperature during the evaporation process did not exceed 40°C. Pressure in the apparatus was not less than 10 mbar.

### d) Processing the lipid phase for the LC/MS or LC/MS/MS analysis

The lipid extract, which had been evaporated to dryness was taken up in mobile phase. The HPLC was run with gradient elution.

The polar extract, which had been evaporated to dryness was taken up in mobile phase. The HPLC was run with gradient elution.

### e) Derivatization of the lipid phase for the GC/MS analysis

For the transmethanolysis, a mixture of 140 µl of chloroform, 37 µl of hydrochloric acid (37% by weight HCl in water), 320 µl of methanol and 20 µl of toluene was added to the evaporated extract. The vessel was sealed tightly and heated for 2 hours at 100°C, with shaking. The solution was subsequently evaporated to dryness. The residue was dried completely.

The methoximation of the carbonyl groups was carried out by reaction with methoxyamine hydrochloride (5 mg/ml in pyridine, 100 µl for 1.5 hours at 60°C) in a tightly sealed vessel. 20 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 100 µl of N-methyl-N-(trimethylsilyl)-2,2,2-trifluoroacetamide (MSTFA) was carried out for 30 minutes at 60°C, again in the tightly sealed vessel. The final volume before injection into the GC was 220 µl.

### f) Derivatization of the polar phase for the GC/MS analysis

The methoximation of the carbonyl groups was carried out by reaction with methoxyamine hydrochloride (5 mg/ml in pyridine, 50 µl for 1.5 hours at 60°C) in a tightly sealed vessel. 10 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 50 µl of N-methyl-N-(trimethylsilyl)-2,2,2-trifluoroacetamide (MSTFA) was carried out for 30 minutes at 60°C, again in the tightly sealed vessel. The final volume before injection into the GC was 110 µl.

### g) Analysis of the various plant samples

The samples were measured in individual series of 20 plant samples each (also referred to as sequences), each sequence containing at least 5 wild-type plants as controls. The peak area of each analyte was divided by the peak area of the respective internal standard. The data were standardized for the fresh weight established for the plant. The values calculated thus were related to the wild-type control group by being divided by the mean of the corresponding data of the wild-type control group of the same sequence. The values obtained were referred to as ratio_by_WT, they are comparable between sequences and indicate how much the analyte concentration in the mutant differs in relation to the wild-type control. Appropiate controls were done before to proof that the vector and transformation procedure itself has no significant influence on the metabolic composition of the plants. Therefore the desribed changes in comparison with wildtypes were caused by the introduced genes.

As an alternative, the amino acids can be detected advantageously via HPLC separation in ethanolic extract as described by Geigenberger et al. (Plant Cell & Environ, 19, 1996: 43-55).
The results of the different plant analyses can be seen from the table 1.

Column 1 in Table 1 shows the amino acid analyzed. Columns 3 and 4 shows the ratio of the analyzed amino acid between the transgenic plants and the wild type; Increase of the metabolites: Max: maximal x-fold (normalised to wild type)-Min: minimal x-fold (normalised to wild type). Decrease of the metabolites: Max: maximal x-fold (normalised to wild type) (minimal decrease), Min: minimal x-fold (normalised to wild type) (maximal decrease). Column 2 indicates the analytical method.

When the analyses were repeated independently, all results proved to be significant.

Example 14a: Engineering ryegrass plants by over-expressing YNL090W, e.g. from Saccharomyces cerevisiae or plants

Seeds of several different ryegrass varieties can be used as explant sources for transformation, including the commercial variety Gunne available from Svalof Weibull seed company or the variety Affinity. Seeds are surface-sterilized sequentially with 1% Tween-20 for 1 minute, 100 % bleach for 60 minutes, 3 rinses with 5 minutes each with de-ionized and distilled H2O, and then germinated for 3-4 days on moist, sterile filter paper in the dark. Seedlings are further sterilized for 1 minute with 1% Tween-20, 5 minutes with 75% bleach, and rinsed 3 times with ddH₂O 5 min each.

Surface-sterilized seeds are placed on the callus induction medium containing Murashige and Skoog basal salts and vitamins, 20 g/l sucrose, 150 mg/l asparagine, 500 mg/l casein hydrolysate, 3 g/l Phytagel, 10 mg/l BAP, and 5 mg/l dicamba. Plates are incubated in the dark at 25°C for 4 weeks for seed germination and embryogenic callus induction.

After 4 weeks on the callus induction medium, the shoots and roots of the seedlings are trimmed away, the callus is transferred to fresh media, is maintained in culture for another 4 weeks, and is then transferred to MSO medium in light for 2 weeks. Several pieces of callus (11-17 weeks old) are either strained through a 10 mesh sieve and put onto callus induction medium, or are cultured in 100 ml of liquid ryegrass callus induction media (same medium as for callus induction with agar) in a 250 ml flask. The flask is wrapped in foil and shaken at 175 rpm in the dark at 23°C for 1 week. Sieving the liquid culture with a 40-mesh sieve is collected the cells. The fraction collected on the sieve is plated and is cultured on solid ryegrass callus induction medium for 1 week in the dark at 25°C. The callus is then transferred to and is cultured on MS medium containing 1 % sucrose for 2 weeks.

Transformation can be accomplished with either Agrobacterium or with particle bombardment methods. An expression vector is created containing a constitutive plant promoter and the cDNA of the gene in a pUC vector. The plasmid DNA is prepared from E. coli cells using with Qiagen kit according to manufacturer's instruction. Approximately 2 g of embryogenic callus is spread in the center of a sterile filter paper in a Petri dish. An aliquot of liquid MSO with 10 g/l sucrose is added to the filter paper. Gold particles (1.0 µm in size) are coated with plasmid DNA according to method of Sanford et al., 1993 and are delivered to the embryogenic callus with the following parameters: 500 µg particles and 2 µg DNA per shot, 1300 psi and a target distance of 8.5 cm from stopping plate to plate of callus and 1 shot per plate of callus.

After the bombardment, calli are transferred back to the fresh callus development medium and maintained in the dark at room temperature for a 1-week period. The callus is then transferred to growth conditions in the light at 25°C to initiate embryo differentiation with the appropriate selection agent, e.g. 250 nM Arsenal, 5 mg/l PPT or 50 mg/L Kanamycin. Shoots resistant to the selection agent are appearing and once rooted are transferred to soil.

Samples of the primary transgenic plants (TO) are analyzed by PCR to confirm the presence of T-DNA. These results are confirmed by Southern hybridization in which DNA is electrophoresed on a 1% agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

Transgenic T0 ryegrass plants are propagated vegetatively by excising tillers. The transplanted tillers are maintained in the greenhouse for 2 months until well established. The shoots are defoliated and allowed to grow for 2 weeks.

Example 14b: Engineering soybean plants by over-expressing YNL090W, e.g. from Saccharomyces cerevisiae or plants [0541.0.0.0] Soybean can be transformed according to the following modification of the method described in the Texas A&M patent US 5,164,310. Several commercial soybean varieties are amenable to transformation by this method. The cultivar Jack (available from the Illinois Seed Foundation) is commonly used for transformation. Seeds are sterilized by immersion in 70% (v/v) ethanol for 6 min and in 25 % commercial bleach (NaOCl) supplemented with 0.1% (v/v) Tween for 20 min, followed by rinsing 4 times with sterile double distilled water. Removing the radicle, hypocotyl and one cotyledon from each seedling propagates seven-day seedlings. Then, the epicotyl with one cotyledon is transferred to fresh germination media in petri dishes and incubated at 25°C under a 16-hr photoperiod (approx. 100 µE-m-2s-1) for three weeks. Axillary nodes (approx. 4 mm in length) are cut from 3 - 4 week-old plants. Axillary nodes are excised and incubated in Agrobacterium LBA4404 culture.

Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used as described above, including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription as described above. In this example, the 34S promoter (GenBank Accession numbers M59930 and X16673) is used to provide constitutive expression of the trait gene.

After the co-cultivation treatment, the explants are washed and transferred to selection media supplemented with 500 mg/L timentin. Shoots are excised and placed on a shoot elongation medium. Shoots longer than 1 cm are placed on rooting medium for two to four weeks prior to transplanting to soil.

The primary transgenic plants (TO) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and is used as recommended by the manufacturer.

Example 14c: Engineering corn plants by over-expressing YNL090W, e.g. from Saccharomyces cerevisiae or plants [0546.0.0.0] Transformation of maize (Zea Mays L.) is performed with a modification of the method described by Ishida et al. (1996. Nature Biotech 14745-50). Transformation is genotype-dependent in corn and only specific genotypes are amenable to transformation and regeneration. The inbred line A188 (University of Minnesota) or hybrids with A188 as a parent are good sources of donor material for transformation (Fromm et al. 1990 Biotech 8:833-839), but other genotypes can be used successfully as well. Ears are harvested from corn plants at approximately 11 days after pollination (DAP) when the length of immature embryos is about 1 to 1.2 mm. Immature embryos are co-cultivated with Agrobacterium tumefaciens that carry "super binary" vectors and transgenic plants are recovered through organogenesis. The super binary vector system of Japan Tobacco is described in WO patents WO94/00977 and WO95/06722. Vectors can be constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. In this example, the 34S promoter (GenBank Accession numbers M59930 and X16673) is used to provide constitutive expression of the trait gene.

Excised embryos are grown on callus induction medium, then maize regeneration medium, containing imidazolinone as a selection agent. The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the imidazolinone herbicides and which are PCR positive for the transgenes.

The T1 generation of single locus insertions of the T-DNA can segregate for the transgene in a 3:1 ratio. Those progeny containing one or two copies of the transgene are tolerant of the imidazolinone herbicide. Homozygous T2 plants can exhibited similar phenotypes as the T1 plants. Hybrid plants (F1 progeny) of homozygous transgenic plants and non-transgenic plants can also exhibited increased similar phenotyps.

Example 14d: Engineering wheat plants by over-expressing YNL090W, e.g. from Saccharomyces cerevisiae or plants

Transformation of wheat is performed with the method described by Ishida et al. (1996 Nature Biotech. 14745-50). The cultivar Bobwhite (available from CYMMIT, Mexico) is commonly used in transformation. Immature embryos are co-cultivated with Agrobacterium tumefaciens that carry "super binary" vectors, and transgenic plants are recovered through organogenesis. The super binary vector system of Japan Tobacco is described in WO patents WO94/00977 and WO95/06722. Vectors were constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. In this example, the 34S promoter (GenBank Accession numbers M59930 and X16673) can be used to provide constitutive expression of the trait gene.

After incubation with Agrobacterium, the embryos are grown on callus induction medium, then regeneration medium, containing imidazolinone as a selection agent. The Petri plates are incubated in the light at 25°C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the imidazolinone herbicides and which are PCR positive for the transgenes.

The T1 generation of single locus insertions of the T-DNA can segregate for the transgene in a 3:1 ratio. Those progeny containing one or two copies of the transgene are tolerant of the imidazolinone herbicide. Homozygous T2 plants exhibited similar phenotypes.

Example 14e: Engineering Rapeseed/Canola plants by over-expressing YNL090W, e.g. from Saccharomyces cerevisiae or plants

Cotyledonary petioles and hypocotyls of 5-6 day-old young seedlings are used as explants for tissue culture and transformed according to Babic et al. (1998, Plant Cell Rep 17: 183-188). The commercial cultivar Westar (Agriculture Canada) is the standard variety used for transformation, but other varieties can be used.

Agrobacterium tumefaciens LBA4404 containing a binary vector are used for canola transformation. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription. In this example, the 34S promoter (GenBank Accession numbers M59930 and X16673) can be used to provide constitutive expression of the trait gene.

Canola seeds are surface-sterilized in 70% ethanol for 2 min., and then in 30% Clorox with a drop of Tween-20 for 10 min, followed by three rinses with sterilized distilled water. Seeds are then germinated in vitro 5 days on half strength MS medium without hormones, 1% sucrose, 0.7% Phytagar at 23oC, 16 hr. light. The cotyledon petiole explants with the cotyledon attached are excised from the in vitro seedlings, and are inoculated with Agrobacterium by dipping the cut end of the petiole explant into the bacterial suspension. The explants are then cultured for 2 days on MSBAP-3 medium containing 3 mg/l BAP, 3% sucrose, 0.7 % Phytagar at 23°C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MSO) for root induction.

Samples of the primary transgenic plants (TO) are analyzed by PCR to confirm the presence of T-DNA. These results are confirmed by Southern hybridization in which DNA is electrophoresed on a 1 % agarose gel and are transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

Example 14f: Engineering alfalfa plants by over-expressing YNL090W genes, e.g. from Saccharomyces cerevisiae or E. coli or plants

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659).

Petiole explants are cocultivated with an overnight culture of Agrobacterium tumefaciens C58C1 pMP90 (McKersie et al., 1999 Plant Physiol 119: 839-847) or LBA4404 containing a binary vector. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene that provides constitutive, developmental, tissue or environmental regulation of gene transcription. In this example, the 34S promoter (GenBank Accession numbers M59930 and X16673) can be used to provide constitutive expression of the trait gene.

The explants are cocultivated for 3 d in the dark on SH induction medium containing 288 mg/ L Pro, 53 mg/L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings are transplanted into pots and grown in a greenhouse.

The TO transgenic plants are propagated by node cuttings and rooted in Turface growth medium. The plants are defoliated and grown to a height of about 10 cm (approximately 2 weeks after defoliation).

Example 14g: Engineering alfalfa plants by over-expressing YLR375W genes, e.g. from Saccharomyces cerevisiae or plants

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659).

Petiole explants are cocultivated with an overnight culture of Agrobacterium tumefaciens C58C1 pMP90 (McKersie et al., 1999 Plant Physiol 119: 839-847) or LBA4404 containing a binary vector. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene that provides constitutive, developmental, tissue or environmental regulation of gene transcription. In this example, the 34S promoter (GenBank Accession numbers M59930 and X16673) can be used to provide constitutive expression of the trait gene.

The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings are transplanted into pots and grown in a greenhouse.

The T0 transgenic plants are propagated by node cuttings and rooted in Turface growth medium. The plants are defoliated and grown to a height of about 10 cm (approximately 2 weeks after defoliation).

Equivalents

Those of ordinary skill in the art will recognize, or will be able to ascertain using no more than routine experimentation, many equivalents to the specific embodiments of the invention described herein. Such equivalents are intended to be encompassed by the claims.

## Claims

1. A process for the production of fine chemical, which comprises
a) increasing or generating the biological activity represented by a protein as depicted in SEQ ID NO: 376 in a non-human organism, or in one or more parts thereof; and
b) growing the organism under conditions which permit the production of the fine chemical in said organism.

2. A process for the production of fine chemical, comprising the increasing or generating in an organism or a part thereof the expression of at least one nucleic acid molecule comprising a nucleic acid molecule selected from the group consisting of:
a) nucleic acid molecule encoding of the polypeptide as depicted in SEQ ID NO: 376 or a fragment thereof, which confers an increase in the amount of fine chemical in an organism or a part thereof;
b) nucleic acid molecule comprising of the nucleic acid molecule as depicted in SEQ ID NO: 375;
c) nucleic acid molecule whose sequence can be deduced from a polypeptide sequence encoded by a nucleic acid molecule of (a) or (b) as a result of the degeneracy of the genetic code and conferring an increase in the amount of fine chemical in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of fine chemical in an organism or a part thereof;
e) nucleic acid molecule which hybridizes with a nucleic acid molecule of (a) to (c) under stringent hybridization conditions and conferring an increase in the amount of fine chemical in an organism or a part thereof;
f) nucleic acid molecule which encompasses a nucleic acid molecule which is obtained by amplifying nucleic acid molecules from a cDNA library or a genomic library using the primers in SEQ ID NO: 53 or SEQ ID NO: 54 and conferring an increase in the amount of the fine chemical in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of fine chemical in an organism or a part thereof;
and
h) nucleic acid molecule which is obtainable by screening a suitable nucleic acid library under stringent hybridization conditions with a probe comprising one of the sequences of the nucleic acid molecule of (a) to (g) or with a fragment thereof having at least 15 nt, preferably 20 nt, 30 nt, 50 nt, 100 nt, 200 nt or 500 nt of the nucleic acid molecule **characterized in** (a) to (g) and conferring an increase in the amount of the fine chemical in an organism or a part thereof.
or comprising a sequence which is complementary thereto.

3. The process of claim 1 or 2, comprising recovering of the free or bound fine chemical.

4. The process of any one of claim 1 to 3, comprising the following steps:
(a) selecting an organism or a part thereof expressing a polypeptide encoded by the nucleic acid molecule **characterized in** claim 2;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound fine chemical produced by the selected mutated organisms or parts thereof.

5. The process of any one of claims 1 to 4, wherein the activity of said protein or the expression of said nucleic acid molecule is increased or generated transiently or stably.

6. An isolated nucleic acid molecule comprising a nucleic acid molecule selected from the group consisting of:
(a) nucleic acid molecule encoding of the polypeptide as depicted in SEQ ID NO: 376 or a fragment thereof, which confers an increase in the amount of fine chemical in an organism or a part thereof;
(b) nucleic acid molecule comprising of the nucleic acid molecule as depicted in SEQ ID NO: 375 or a fragment thereof, which confers an increase in the amount of fine chemical in an organism or a part thereof;
(c) nucleic acid molecule whose sequence can be deduced from a polypeptide sequence encoded by a nucleic acid molecule of (a) or (b) as a result of the degeneracy of the genetic code and conferring an increase in the amount of fine chemical in an organism or a part thereof;
(d) nucleic acid molecule which encodes a polypeptide which has at least 50% identity with the amino acid sequence of the polypeptide encoded by the nucleic acid molecule of (a) to (c) and conferring an increase in the amount of fine chemical in an organism or a part thereof;
(e) nucleic acid molecule which hybridizes with a nucleic acid molecule of (a) to (c) under stringent hybridization conditions and conferring an increase in the amount of fine chemical in an organism or a part thereof;
(f) nucleic acid molecule which encompasses a nucleic acid molecule which is obtained by amplifying nucleic acid molecules from a cDNA library or a genomic library using the primers in SEQ ID NO: 53 or SEQ ID NO: 54 and conferring an increase in the amount of the fine chemical in an organism or a part thereof;
(g) nucleic acid molecule encoding a polypeptide which is isolated with the aid of monoclonal and/or polyclonal antibodies against a polypeptide encoded by one of the nucleic acid molecules of (a) to (f) and conferring an increase in the amount of fine chemical in an organism or a part thereof;
and
(h) nucleic acid molecule which is obtainable by screening a suitable nucleic acid library under stringent hybridization conditions with a probe comprising one of the sequences of the nucleic acid molecule of (a) to (g) or with a fragment thereof having at least 15 nt, preferably 20 nt, 30 nt, 50 nt, 100 nt, 200 nt or 500 nt of the nucleic acid molecule **characterized in** (a) to (g) and conferring an increase in the amount of the fine chemical in an organism or a part thereof,
whereby the nucleic acid molecule distinguishes over the sequence as depicted in SEQ ID NO: 375 by one or more nucleotides.

7. A nucleic acid construct which confers the expression of the nucleic acid molecule of claim 6, comprising one or more regulatory elements.

8. A vector comprising the nucleic acid molecule as claimed in claim 6 or the nucleic acid construct of claim 7.

9. The vector as claimed in claim 8, wherein the nucleic acid molecule is in operable linkage with regulatory sequences for the expression in a prokaryotic or eukaryotic, or in a prokaryotic and eukaryotic, host.

10. A host cell, which has been transformed stably or transiently with the vector as claimed in claim 8 or 9 or with the vector, wherein said vector comprises the nucleic acid molecule as described in claim 2, or the nucleic acid molecule as claimed in claim 6 or with the nucleic acid construct of claim 7 or with the nucleic acid construct, which confers the expression of the nucleic acid molecule as described in claim 2, comprising one or more regulatory elements, or produced as described in claim any one of claims 2 to 4.

11. The host cell of claim 10, which is a transgenic host cell.

12. The host cell of claim 10 or 11, which is a plant cell, an animal cell, a microorganism, or a yeast cell, a fungus cell, a prokaryotic cell, an eukaryotic cell or an archaebacterium.

13. A process for producing a polypeptide, wherein the polypeptide is expressed in a host cell as claimed in any one of claims 9 to 11.

14. A polypeptide produced by the process as claimed in claim 13 or encoded by the nucleic acid molecule as claimed in claim 6 whereby the polypeptide distinguishes over the sequence as depicted in SEQ ID NO: 376 by one or more amino acids.

15. An antibody, which binds specifically to the polypeptide encoded by a nucleic acid sequence as claimed in claim 6 a).

16. A plant tissue, propagation material, harvested material or a plant comprising the host cell as claimed in claim 12, which is plant cell or an Agrobacterium.

17. Food or feed composition comprising the nucleic acid molecule of claim 6, the polypeptide of claim 14, the nucleic acid construct of claim 7, the vector of claim 8 or 9, the antibody of claim 15, the plant or plant tissue of claim 16, the harvested material of claim 16 or the host cell of claim 10 to 12.

18. A method for the identification of a compound stimulating production of the fine chemical in a plant comprising:
(a) contacting cells which express the a polypeptide encoded by the nucleic acid molecule of claim 6 or as decribed in claim 2 or its mRNA with a candidate compound under cell cultivation conditions;
(b) assaying an increase in expression of said polypoeptide or said mRNA; and
(c) identifying said stimulating compound by comparing the expression level to a standard response made in the absence of said candidate compound, whereby an increased expression over the standard indicates that the compound is stimulating production of the fine chemical.
